(19) Europäisches Patentamt
European Patent Office
Office européen des brevets

(11) EP 2 103 619 A1

(12) **EUROPEAN PATENT APPLICATION**
published in accordance with Art. 153(4) EPC

(43) Date of publication:
**23.09.2009 Bulletin 2009/39**

(21) Application number: **07850481.8**

(22) Date of filing: **12.12.2007**

(51) Int Cl.:
*C07D 513/04* (2006.01) *A61K 31/429* (2006.01)
*A61K 31/4439* (2006.01) *A61K 31/454* (2006.01)
*A61K 31/496* (2006.01) *A61K 31/4985* (2006.01)
*A61K 31/5377* (2006.01) *A61K 31/551* (2006.01)
*A61P 35/00* (2006.01) *A61P 43/00* (2006.01)
*C07D 513/10* (2006.01) *C07D 519/00* (2006.01)

(86) International application number:
**PCT/JP2007/073930**

(87) International publication number:
**WO 2008/072655 (19.06.2008 Gazette 2008/25)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **14.12.2006 JP 2006336662**

(71) Applicant: **Daiichi Sankyo Company, Limited**
**Chuo-ku**
**Tokyo 103-0023 (JP)**

(72) Inventors:
- **KAJINO, Hisaki**
  **Kanagawa 254-0014 (JP)**
- **NIHEI, Satoru**
  **Kanagawa 254-0014 (JP)**
- **IKEUCHI, Yutaka**
  **Kanagawa 254-0014 (JP)**
- **MIYAMOTO, Hiroshi**
  **Kanagawa 254-0014 (JP)**
- **NUMAGAMI, Eiji**
  **Kanagawa 254-0014 (JP)**

(74) Representative: **Fairbairn, Angus Chisholm**
**Marks & Clerk LLP**
**90 Long Acre**
**London**
**WC2E 9RA (GB)**

(54) **IMIDAZOTHIAZOLE DERIVATIVES**

(57) There is provided a novel compound that inhibits interaction between murine double minute 2 (Mdm2) protein and p53 protein and exhibits anti-tumor activity. The present invention provides an imidazothiazole derivative represented by the following formula (1) having various substituents that inhibits interaction between Mdm2 protein and p53 protein and exhibits anti-tumor activity:

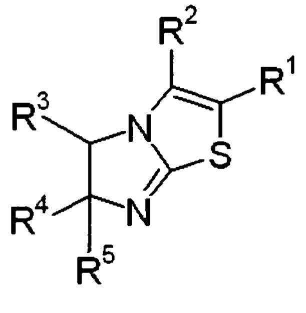

(1)

wherein $R^1$, $R^2$, $R^3$, $R^4$, and $R^5$ in the formula (1) each has the same meaning as defined in the specification.

## Description

### Technical Field

The present invention relates to imidazothiazole derivatives having anti-tumor activity by inhibition of murine double minute 2 (Mdm2).

### Background Art

p53 is known as an important factor for inhibiting canceration of cells. p53 is a transcription factor that induces the expression of genes involved in the cell cycle and cellular apoptosis in response to various stresses. p53 is thought to inhibit canceration of cells by a transcription regulating function thereof. In fact, deletion or mutation of the p53 gene is observed in about half of human cancer cases.

Meanwhile, overexpression of murine double minute 2 (Mdm2), a type of E3 ubiquitin ligase, is known as a factor for canceration of cells that are cancerated in spite of the presence of normal p53. Mdm2 is a protein of which expression is induced by p53. Mdm2 negatively regulates p53 by mediating degradation of p53 by binding to the transcription activity domain of p53 to decrease the transcription activity of p53, exporting p53 out of the nucleus, and further acting as a ubiquitination ligase against p53. Therefore, it is thought that inactivation of functions of and degradation of p53 are promoted in cells in which Mdm2 is overexpressed, resulting in canceration (Non-Patent Document 1).

Paying attention to such functions of Mdm2, many approaches have been attempted using substances that inhibits the suppression of p53 functions by Mdm2, as candidate anti-tumor agents. As substances that inhibit the suppression of p53 functions by Mdm2, for example, various anti-Mdm2 antisense oligonucleotides (for example, refer to Patent Document 1), low molecular weight compounds (for example, refer to Non-Patent Documents 1 to 14 and Patent Documents 2 to 17), and the like have been reported. Recently, Mdm2 inhibitors targeting the Mdm2-p53 binding site have been explored using the results of crystal structure analyses of Mdm2 and p53 (for example, refer to Non-Patent Documents 1, 2, and 4 to 14). Examples of the Mdm2 inhibitors targeting the Mdm2-p53 binding site include imidazoline derivatives having two sites substituted with halogenobenzene (for example, refer to Non-Patent Documents 1 and 2 and Patent Documents 5 to 11), benzodiazepine derivatives containing two halogenobenzene moieties in the structure thereof (for example, refer to Non-Patent Documents 4 to 11 and Patent Documents 12 to 16) or spiro oxindole derivatives containing two halogenobenzene moieties in the structure thereof (for example, refer to Non-Patent Documents 12 to 14 and Patent Document 17), and so forth. However, no report has demonstrated that these compounds actually showed efficacy in clinical practice.

Non-Patent Document 1: Science, 2004, 303, 844-848
Non-Patent Document 2: Proceedings of the National Academy of Sciences of the United States of America, 2006, 103, 1888-1893
Non-Patent Document 3: Analytical Biochemistry, 2004, 331, 138-146
Non-Patent Document 4: Bioorganic & Medicinal Chemistry Letters, 2005, 15, 765-770
Non-Patent Document 5: Journal of Medicinal Chemistry, 2005, 48, 909-912
Non-Patent Document 6: Chemical Biology & Drug Design, 2006, 67, 201-205
Non-Patent Document 7: Bioorganic & Medicinal Chemistry Letters, 2005, 15, 1857-1861
Non-Patent Document 8: Molecular Cancer Therapeutics, 2006, 5(1), 160-169
Non-Patent Document 9: Bioorganic & Medicinal Chemistry Letters, 2006, 16, 3115-3120
Non-Patent Document 10: Bioorganic & Medicinal Chemistry Letters, 2006, 16, 3310-3314
Non-Patent Document 11: Bioorganic & Medicinal Chemistry Letters, 2006, 16, 3463-3468
Non-Patent Document 12: Journal of the American Chemical Society, 2005, 127, 10130-10131
Non-Patent Document 13: Tetrahedron Letters, 2005, 46, 5949-5951
Non-Patent Document 14: Journal of Medicinal Chemistry, 2006, 49, 3432-3435
Patent Document 1: WO1999/49065
Patent Document 2: WO2000/15657
Patent Document 3: WO2006/24837
Patent Document 4: WO2004/80460
Patent Document 5: WO2003/51359
Patent Document 6: WO2003/51360
Patent Document 7: WO2005/3097
Patent Document 8: WO2005/2575
Patent Document 9: WO2005/110996

Patent Document 10: WO2005/123691
Patent Document 11: WO2006/97261
Patent Document 12: WO2003/41715
Patent Document 13: WO2003/95625
Patent Document 14: U.S. Patent Application Publication No. 2004/197893
Patent Document 15: U.S. Patent Application Publication No. 2004/220179
Patent Document 16: WO2004/96134
Patent Document 17: WO2006/91646

Disclosure of the Invention

Problems to be Solved by the Invention

The present invention provides an Mdm2 inhibiting compound having a novel skeleton. Furthermore, the present invention provides an anti-tumor agent containing the Mdm2 inhibiting compound.

Means for Solving the Problems

The inventors of the present invention conducted various researches. As a result, they found that a novel compound having an imidazothiazole structure represented by formula (1) had potent Mdm2 inhibiting activity and anti-tumor activity, and accomplished the present invention.

Specifically, the present invention relates to the following [1] to [47].

[1] A compound represented by formula (1), a salt of the compound, or a solvate of the compound or the salt:

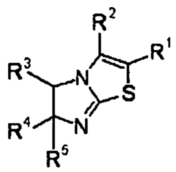

(1)

wherein

$R^1$ represents a hydrogen atom, $-V_1-V_2$, -CO-W, or $-COX_1-CO-X_2$, wherein

$V_1$ represents a $C_1-C_6$ alkylene group,

$V_2$, W and $X_2$ each independently represents a group selected from the group consisting of a hydrogen atom, a hydroxy group, a $C_1-C_6$ alkoxy group, an amino group which may be substituted with one or two 4- to 7-membered saturated or unsaturated heterocyclic groups (said 4- to 7-membered saturated or unsaturated heterocyclic group may have one or more $C_1-C_6$ alkyl groups and/or oxo groups), a $C_1-C_6$ alkylamino group which may have one or more substituents selected from the following Group A, an amino $C_1-C_6$ alkylamino group which may have one or more substituents selected from the following Group B, a 4- to 7-membered saturated or unsaturated nitrogen-containing heterocyclic group which may have one or more substituents selected from the following Group C, and an 8- to 11-membered bicyclic condensed nitrogen-containing heterocyclic group which may have one or more substituents selected from the following Group D,

$X_1$ represents a group selected from the group consisting of an NH-$C_1-C_6$ alkylene group (said NH-$C_1-C_6$ alkylene group may be substituted on NH with a $C_1-C_6$ alkyl group), a divalent 4- to 7-membered saturated or unsaturated nitrogen-containing heterocyclic group which may have one or more substituents selected from the following Group E, and a divalent 8- to 11-membered bicyclic condensed nitrogen-containing heterocyclic group which may have one or more substituents selected from the following Group F,

$R^2$ represents a group selected from the group consisting of a hydrogen atom, a phenyl group, and a $C_1-C_6$ alkyl group which may have one or more substituents selected from the following Group G,

$R^3$ and $R^4$ each independently represents a group selected from the group consisting of a $C_1-C_6$ alkyl group which may have one or more substituents selected from the following Group H, a phenyl group which may have one or more substituents selected from the following Group I, and a 5- to 6-membered aromatic heterocyclic group which may have one or more substituents selected from the following Group I (provided that $R^3$ and $R^4$ do not both represent

a C1-C6 alkyl group which may have a substituent(s)),
$R^5$ represents a hydrogen atom or a $C_1$-$C_6$ alkyl group, and
furthermore, $R^4$ and $R^5$ together with the carbon atom on the ring to which $R^4$ and $R^5$ are bonded may form a 3-to 7-membered spiro ring:

Group A: a $C_1$-$C_6$ alkylamino $C_1$-$C_6$ alkoxy group, a hydroxy $C_1$-$C_6$ alkoxy group, a $C_1$-$C_6$ alkoxy group, a $C_1$-$C_6$ alkoxycarbonyl group, a $C_1$-$C_6$ alkylamino group, a 4- to 7-membered saturated or unsaturated heterocyclic group (said 4- to 7-membered saturated or unsaturated heterocyclic group may have one or more substituents selected from the group consisting of a $C_1$-$C_6$ alkyl group which may be substituted with one or more hydroxy groups, a $C_2$-$C_6$ alkanoyl group, a hydroxy group, a $C_1$-$C_6$ alkoxycarbonyl group, a $C_1$-$C_6$ alkylamino $C_1$-$C_6$ alkyl group, a $C_1$-$C_6$ alkylaminocarbonyl group, a $C_1$-$C_6$ alkylamino $C_1$-$C_6$ alkylcarbonyl group, and an oxo group), a hydroxy group, and a carboxy group;
Group B: a $C_1$-$C_6$ alkyl group (said $C_1$-$C_6$ alkyl group being a substituent on an amino group of the amino $C_1$-$C_6$ alkylamino group), a formyl group, and a $C_2$-$C_6$ alkanoyl group;
Group C: a halogen atom, a hydroxy group, a $C_1$-$C_6$ alkyl group which may be substituted with one or more hydroxy groups, a $C_1$-$C_6$ alkoxy group, a $C_1$-$C_6$ alkoxy $C_1$-$C_6$ alkyl group (said $C_1$-$C_6$ alkoxy $C_1$-$C_6$ alkyl group may have one or more substituents selected from the group consisting of a hydroxyphenyl group, a carboxy group, and a 4- to 7-membered saturated or unsaturated heterocyclic group which may have a substituent(s)), a halogeno $C_1$-$C_6$ alkyl group, a carboxy $C_1$-$C_6$ alkyl group, a $C_1$-$C_6$ alkoxycarbonyl $C_1$-$C_6$ alkyl group, a $C_1$-$C_6$ alkylamino $C_1$-$C_6$ alkyl group, a carbamoyl $C_1$-$C_6$ alkyl group, a carboxy group, a formyl group, a $C_2$-$C_6$ alkanoyl group, a $C_1$-$C_6$ alkoxycarbonyl group, an amino group, a $C_1$-$C_6$ alkylamino group, a $C_1$-$C_6$ alkylsulfonyl group, an oxo group, a phenyl group, a 4- to 7-membered saturated or unsaturated heterocyclic group (said 4- to 7-membered saturated or unsaturated heterocyclic group may have one or more substituents selected from a $C_1$-$C_6$ alkyl group, a $C_2$-$C_6$ alkanoyl group, and an oxo group), and a $C_1$-$C_6$ alkylene-4- to 7-membered saturated or unsaturated heterocyclic group (said 4- to 7-membered saturated or unsaturated heterocyclic group may have one or more substituents selected from a $C_1$-$C_6$ alkyl group, a $C_2$-$C_6$ alkanoyl group, and an oxo group);
Group D: a $C_1$-$C_6$ alkyl group and an oxo group;
Group E: a halogen atom, a hydroxy group, a $C_1$-$C_6$ alkyl group, a $C_1$-$C_6$ alkoxy group, an amino group, a $C_1$-$C_6$ alkylamino group, a cyano group, a $C_1$-$C_6$ alkylamino $C_1$-$C_6$ alkyl group, and an oxo group;
Group F: a $C_1$-$C_6$ alkyl group and an oxo group;
Group G: a $C_1$-$C_6$ alkoxy group, an oxo group, a 4- to 7-membered saturated or unsaturated heterocyclic group (said 4- to 7-membered saturated or unsaturated heterocyclic group may be substituted with a $C_1$-$C_6$ alkyl group and/or an oxo group), a hydroxy group, and a $C_3$-$C_8$ cycloalkyl group;
Group H: a phenyl group and a $C_3$-$C_8$ cycloalkyl group; and
Group I: a halogen atom, an amino group, a $C_1$-$C_6$ alkyl group, a $C_1$-$C_6$ alkoxy group, a $C_1$-$C_6$ alkylamino group, a halogeno $C_1$-$C_6$ alkyl group, and a cyano group.

[2] The compound according to [1], a salt of the compound, or a solvate of the compound or the salt,
wherein
$R^1$ in the formula (1) represents $-V_1-V_2$, wherein
$V_1$ represents a $C_1$-$C_6$ alkylene group, and
$V_2$ represents a group selected from the group consisting of a hydrogen atom, a hydroxy group, and a 4-to 7-membered saturated or unsaturated nitrogen-containing heterocyclic group which may have one or more substituents selected from Group C (where, Group C has the same meaning as defined above).
[3] The compound according to [2], a salt of the compound, or a solvate of the compound or the salt, wherein
$V_1$ represents a methylene group or an ethylidene group ($-CH(CH_3)-$), and
$V_2$ represents a 4- to 7-membered saturated or unsaturated nitrogen-containing heterocyclic group which may have one or more substituents selected from Group C (where, Group C has the same meaning as defined above).
[4] The compound according to [3], a salt of the compound, or a solvate of the compound or the salt, wherein
$V_1$ represents a methylene group or an ethylidene group ($-CH(CH_3)-$), and
$V_2$ represents a piperazinyl group or a pyrrolidinyl group (said piperazinyl group or pyrrolidinyl group may have one or more substituents selected from the group consisting of an oxo group, a methyl group, and an ethyl group).
[5] The compound according to [1], a salt of the compound, or a solvate of the compound or the salt, wherein
$R^1$ in the formula (1) represents -CO-W, wherein
W represents a group selected from the group consisting of a hydroxy group, a $C_1$-$C_6$ alkoxy group, an amino group which may be substituted with one or two 4-to 7-membered saturated or unsaturated heterocyclic groups (said 4- to 7-membered saturated or unsaturated heterocyclic group may have one or more $C_1$-$C_6$ alkyl groups and/or oxo groups), a $C_1$-$C_6$ alkylamino group which may have one or more substituents selected from Group A, an amino $C_1$-$C_6$ alkylamino group which may have one or more substituents selected from Group B, a 4- to 7-membered saturated or unsaturated nitrogen-containing heterocyclic group which may have one or more substituents selected from Group C, and an 8- to 11-membered bicyclic condensed nitrogen-containing heterocyclic group which may have one or more substituents selected from Group D (where, Groups A, B, C, and D have the same meanings as defined above).

[6] The compound according to [5], a salt of the compound, or a solvate of the compound or the salt, wherein W represents a $C_1$-$C_6$ alkylamino group which may have one or more substituents selected from Group A (where, Group A has the same meaning as defined above).

[7] The compound according to [6], a salt of the compound, or a solvate of the compound or the salt, wherein W represents a methylamino group, a dimethylamino group, an ethylmethylamino group, or an isopropylmethylamino group (these groups may be substituted with an azetidinyl group, a pyrrolidinyl group, or a cyclobutyl group (said azetidinyl group, pyrrolidinyl group, or cyclobutyl group may have one or more $C_1$-$C_6$ alkyl groups and/or oxo groups)).

[8] The compound according to [5], a salt of the compound, or a solvate of the compound or the salt, wherein W represents an amino $C_1$-$C_6$ alkylamino group which may have one or more substituents selected from Group B (where, Group B has the same meaning as defined above).

[9] The compound according to [8], a salt of the compound, or a solvate of the compound or the salt, wherein W represents a (2-aminoethyl) amino group, a (1-aminopropyl-2-yl)amino group, or a (2-aminopropyl)amino group (said (2-aminoethyl)amino group, (1-aminopropyl-2-yl)amino group, or (2-aminopropyl)amino group may be substituted with one or more methyl groups (however, said methyl group being a substituent on an amino group of each group) or acetyl groups).

[10] The compound according to [5], a salt of the compound, or a solvate of the compound or the salt, wherein W represents a 4- to 7-membered saturated or unsaturated nitrogen-containing heterocyclic group which may have one or more substituents selected from Group C (where, Group C has the same meaning as defined above).

[11] The compound according to [10], a salt of the compound, or a solvate of the compound or the salt, wherein W represents a piperazinyl group or a pyrrolidinyl group (said piperazinyl group or pyrrolidinyl group may have one or more substituents selected from the group consisting of an oxo group, a methyl group, an ethyl group, an acetyl group, a dimethylaminomethyl group, a (morpholin-4-yl)methyl group, and a carbamoyl methyl group).

[12] The compound according to [1], a salt of the compound, or a solvate of the compound or the salt, wherein $R^1$ in the formula (1) represents $-CO-X_1-CO-X_2$, wherein

$X_1$ represents a group selected from the group consisting of an NH-$C_1$-$C_6$ alkylene group (said NH-$C_1$-$C_6$ alkylene group may be substituted on NH with a $C_1$-$C_6$ alkyl group), a divalent 4- to 7-membered saturated or unsaturated nitrogen-containing heterocyclic group which may have one or more substituents selected from Group E, and a divalent 8- to 11-membered bicyclic condensed nitrogen-containing heterocyclic group which may have one or more substituents selected from Group F (where, Groups E and F have the same meanings as defined above), and

$X_2$ represents a group selected from the group consisting of a hydroxy group, a $C_1$-$C_6$ alkoxy group, an amino group which may be substituted with one or two 4-to 7-membered saturated or unsaturated heterocyclic groups (said 4- to 7-membered saturated or unsaturated heterocyclic group may have one or more $C_1$-$C_6$ alkyl groups and/or oxo groups), a $C_1$-$C_6$ alkylamino group which may have one or more substituents selected from Group A, an amino $C_1$-$C_6$ alkylamino group which may have one or more substituents selected from Group B, a 4- to 7-membered saturated or unsaturated nitrogen-containing heterocyclic group which may have one or more substituents selected from Group C, and an 8- to 11-membered bicyclic condensed nitrogen-containing heterocyclic group which may have one or more substituents selected from Group D (where, Groups A, B, C, and D have the same meanings as defined above).

[13] The compound according to [12], a salt of the compound, or a solvate of the compound or the salt, wherein $X_1$ represents an NH-$C_1$-$C_6$ alkylene group (said NH-$C_1$-$C_6$ alkylene group may be substituted on NH with a $C_1$-$C_6$ alkyl group).

[14] The compound according to [13], a salt of the compound, or a solvate of the compound or the salt, wherein $X_1$ represents an NH-methylene group (said NH-methylene group may be substituted on NH with a substituent selected from the group consisting of an ethyl group, a propyl group, an isopropyl group, a butyl group, an isobutyl group, and a sec-butyl group).

[15] The compound according to [12], a salt of the compound, or a solvate of the compound or the salt, wherein $X_1$ represents a divalent 4- to 7-membered saturated or unsaturated nitrogen-containing heterocyclic group which may have one or more substituents selected from Group E (where, Group E has the same meaning as defined above).

[16] The compound according to [15], a salt of the compound, or a solvate of the compound or the salt, wherein $X_1$ represents a pyrrolidin-1,2-diyl group which may have one or more substituents selected from the group consisting of a $C_1$-$C_3$ alkyl group, a hydroxy group, and a methoxy group.

[17] The compound according to [12], a salt of the compound, or a solvate of the compound or the salt, wherein $X_2$ represents a $C_1$-$C_6$ alkylamino group which may have one or more substituents selected from Group A (where, Group

A has the same meaning as defined above).

[18] The compound according to [17], a salt of the compound, or a solvate of the compound or the salt, wherein $X_2$ represents a dimethylamino group, an ethylmethylamino group, or a diethylamino group (said dimethylamino group, ethylmethylamino group, or diethylamino group may have one or more substituents selected from the group consisting of a hydroxy group, a methoxy group, and a carboxy group).

[19] The compound according to [12], a salt of the compound, or a solvate of the compound or the salt, wherein $X_2$ represents an amino $C_1$-$C_6$ alkylamino group which may have one or more substituents selected from Group B (where, Group B has the same meaning as defined above).

[20] The compound according to [19], a salt of the compound, or a solvate of the compound or the salt, wherein $X_2$ represents a (2-aminoethyl)amino group, a (1-aminopropyl-2-yl)amino group, or a (2-aminopropyl)amino group (said (2-aminoethyl)amino group, (1-aminopropyl-2-yl)amino group, or (2-aminopropyl)amino group may be substituted with one or more methyl groups (however, said methyl group being a substituent on an amino group of each group) or acetyl groups).

[21] The compound according to [12], a salt of the compound, or a solvate of the compound or the salt, wherein $X_2$ represents a 4- to 7-membered saturated or unsaturated nitrogen-containing heterocyclic group which may have one or more substituents selected from Group C (where, Group C has the same meaning as defined above).

[22] The compound according to [21], a salt of the compound, or a solvate of the compound or the salt, wherein $X_2$ represents a piperazinyl group, a pyrrolidinyl group, or a morpholino group (said piperazinyl group, pyrrolidinyl group, or morpholino group may have one or more substituents selected from the group consisting of an oxo group, a methyl group, an ethyl group, a cyclopropyl group, an acetyl group, a hydroxy group, a carboxy group, a carbamoyl group, a dimethylamino group, a hydroxymethyl group, a hydroxyethyl group, an amino group, a fluoro group, and a fluoromethyl group).

[23] The compound according to [12], a salt of the compound, or a solvate of the compound or the salt, wherein $X_2$ represents an 8- to 11-membered bicyclic condensed nitrogen-containing heterocyclic group which may have one or more substituents selected from Group D (where, Group D has the same meaning as defined above).

[24] The compound according to [23], a salt of the compound, or a solvate of the compound or the salt, wherein $X_2$ represents an octahydropyrrolopyrazyl group (said octahydropyrrolopyrazyl group may have one or more methyl groups or oxo groups).

[25] The compound according to [12], a salt of the compound, or a solvate of the compound or the salt, wherein $X_1$ represents an NH-$C_1$-$C_6$ alkylene group (said NH-$C_1$-$C_6$ alkylene group may be substituted on NH with a $C_1$-$C_6$ alkyl group) or a divalent 4- to 7-membered saturated or unsaturated nitrogen-containing heterocyclic group which may have one or more substituents selected from Group E (where, Group E has the same meaning as defined above), and

$X_2$ represents a $C_1$-$C_6$ alkylamino group which may have one or more substituents selected from Group A, an amino $C_1$-$C_6$ alkylamino group which may have one or more substituents selected from Group B, a 4- to 7-membered saturated or unsaturated nitrogen-containing heterocyclic group which may have one or more substituents selected from Group C, or an 8- to 11-membered bicyclic condensed nitrogen-containing heterocyclic group which may have one or more substituents selected from Group D (where, Groups A, B, C, and D each have the same meanings as defined above).

[26] The compound according to any one of [1] to [25], a salt of the compound, or a solvate of the compound or the salt, wherein

$R^2$ in the formula (1) represents an alkyl group which may have one or more substituents selected from Group G (where, Group G has the same meaning as defined above).

[27] The compound according to any one of [1] to [26], a salt of the compound, or a solvate of the compound or the salt, wherein

$R^2$ in the formula (1) represents a $C_1$-$C_4$ alkyl group.

[28] The compound according to any one of [1] to [27], a salt of the compound, or a solvate of the compound or the salt, wherein

$R^3$ in the formula (1) represents a phenyl group which may have one or more substituents selected from Group I or a 5- to 6-membered aromatic heterocyclic group which may have one or more substituents selected from Group I (where, Group I has the same meaning as defined above).

[29] The compound according to any one of [1] to [28], a salt of the compound, or a solvate of the compound or the salt, wherein

$R^3$ in the formula (1) represents a 4-chlorophenyl group, a 6-chloropyridin-3-yl group, a 3-fluoro-4-chlorophenyl group, a 2-fluoro-4-chlorophenyl group, a 5-bromopyridin-2-yl group, a 4-trifluoromethylphenyl group, or a 4-bromophenyl group.

[30] The compound according to any one of [1] to [29], a salt of the compound, or a solvate of the compound or the salt, wherein

$R^4$ in the formula (1) represents a phenyl group which may have one or more substituents selected from Group I or a 5- to 6-membered aromatic heterocyclic group which may have one or more substituents selected from Group I (where, Group I has the same meaning as defined above).

[31] The compound according to any one of [1] to [30], a salt of the compound, or a solvate of the compound or the salt, wherein

$R^4$ in the formula (1) represents a 4-chlorophenyl group, a 6-chloropyridin-3-yl group, a 4-chloro-3-methylaminophenyl group, a 3-fluoro-4-chlorophenyl group, a 2-fluoro-4-chlorophenyl group, or a 3,4-difluoro-phenyl group.

[32] The compound according to any one of [1] to [31], a salt of the compound, or a solvate of the compound or the salt, wherein,

in the formula (1), both $R^3$ and $R^4$ represent a 4-chlorophenyl group, $R^3$ represents 3-fluoro-4-chlorophenyl group and $R^4$ represents 4-chlorophenyl group, or $R^3$ represents a 3-fluoro-4-chlorophenyl group and $R^4$ represents a 6-chloropyridin-3-yl group.

[33] The compound according to any one of [1] to [32], a salt of the compound, or a solvate of the compound or the salt, wherein

$R^5$ in the formula (1) represents a $C_1$-$C_3$ alkyl group.

[34] A compound represented by formula (1-A), a salt of the compound, or a solvate of the compound or the salt:

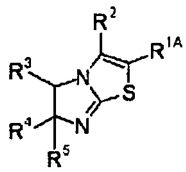

(1-A)

wherein

$R^{1A}$ represents -$V_1$-$V_2$, wherein

$V_1$ represents a $C_1$-$C_6$ alkylene group,

$V_2$ represents a group selected from the group consisting of a hydrogen atom, a hydroxy group, and a 4-to 7-membered saturated or unsaturated nitrogen-containing heterocyclic group which may have one or more substituents selected from the following Group C,

$R^2$ represents a $C_1$-$C_6$ alkyl group which may have one or more substituents selected from the following Group G,

$R^3$ and $R^4$ each independently represents a phenyl group which may have one or more substituents selected from the following Group I, and

$R^5$ represents a hydrogen atom or a $C_1$-$C_6$ alkyl group:

Group C: a halogen atom, a hydroxy group, a $C_1$-$C_6$ alkyl group which may be substituted with one or more hydroxy groups, a $C_1$-$C_6$ alkoxy group, a $C_1$-$C_6$ alkoxy $C_1$-$C_6$ alkyl group (said $C_1$-$C_6$ alkoxy $C_1$-$C_6$ alkyl group may have one or more substituents selected from the group consisting of a hydroxyphenyl group, a carboxy group, and a 4- to 7-membered saturated or unsaturated heterocyclic group which may have a substituent(s)), a halogeno $C_1$-$C_6$ alkyl group, a carboxy $C_1$-$C_6$ alkyl group, a $C_1$-$C_6$ alkoxycarbonyl $C_1$-$C_6$ alkyl group, a $C_1$-$C_6$ alkylamino $C_1$-$C_6$ alkyl group, a carbamoyl $C_1$-$C_6$ alkyl group, a carboxy group, a formyl group, a $C_2$-$C_6$ alkanoyl group, a $C_1$-$C_6$ alkoxycarbonyl group, an amino group, a $C_1$-$C_6$ alkylamino group, a $C_1$-$C_6$ alkylsulfonyl group, an oxo group, a phenyl group, a 4- to 7-membered saturated or unsaturated heterocyclic group (said 4- to 7-membered saturated or unsaturated heterocyclic group may have one or more substituents selected from a $C_1$-$C_6$ alkyl group, a $C_2$-$C_6$ alkanoyl group, and an oxo group), and a $C_1$-$C_6$ alkylene-4- to 7-membered saturated or unsaturated heterocyclic group (said 4- to 7-membered saturated or unsaturated heterocyclic group may have one or more substituents selected from a $C_1$-$C_6$ alkyl group, a $C_2$-$C_6$ alkanoyl group, and an oxo group);

Group G: a $C_1$-$C_6$ alkoxy group, an oxo group, a 4- to 7-membered saturated or unsaturated heterocyclic group (said 4- to 7-membered saturated or unsaturated heterocyclic group may have one or more $C_1$-$C_6$ alkyl groups and/or oxo groups), a hydroxy group, and a $C_3$-$C_8$ cycloalkyl group; and

Group I: a halogen atom, an amino group, a $C_1$-$C_6$ alkyl group, a $C_1$-$C_6$ alkoxy group, a $C_1$-$C_6$ alkylamino group, a halogeno $C_1$-$C_6$ alkyl group, and a cyano group.

[35] A compound represented by formula (1-B), a salt of the compound, or a solvate of the compound or the salt:

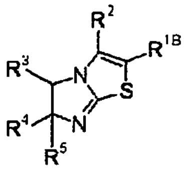

(1-B)

wherein

$R^{1B}$ represents -CO-W, wherein

W represents a group selected from the group consisting of a hydroxy group, a $C_1$-$C_6$ alkoxy group, an amino group which may be substituted with one or two 4-to 7-membered saturated or unsaturated heterocyclic groups (said 4- to 7-membered saturated or unsaturated heterocyclic group may have one or more $C_1$-$C_6$ alkyl groups and/or oxo groups), a $C_1$-$C_6$ alkylamino group which may have one or more substituents selected from the following Group A, an amino $C_1$-$C_6$ alkylamino group which may have one or more substituents selected from the following Group B, a 4- to 7-membered saturated or unsaturated nitrogen-containing heterocyclic group which may have one or more substituents selected from the following Group C, and an 8- to 11-membered bicyclic condensed nitrogen-containing heterocyclic group which may have one or more substituents selected from the following Group D,

$R^2$ represents a $C_1$-$C_6$ alkyl group which may have one or more substituents selected from the following Group G,

$R^3$ and $R^4$ each independently represents a phenyl group which may have one or more substituents selected from the following Group I or a 5- to 6-membered aromatic heterocyclic group which may have one or more substituents selected from the following Group I, and

$R^5$ represents a hydrogen atom or a $C_1$-$C_6$ alkyl group:

Group A: a $C_1$-$C_6$ alkylamino $C_1$-$C_6$ alkoxy group, a hydroxy $C_1$-$C_6$ alkoxy group, a $C_1$-$C_6$ alkoxy group, a $C_1$-$C_6$ alkoxycarbonyl group, a $C_1$-$C_6$ alkylamino group, a 4- to 7-membered saturated or unsaturated heterocyclic group (said 4- to 7-membered saturated or unsaturated heterocyclic group may have one or more substituents selected from the group consisting of a $C_1$-$C_6$ alkyl group which may be substituted with one or more hydroxy groups, a $C_2$-$C_6$ alkanoyl group, a hydroxy group, a $C_1$-$C_6$ alkoxycarbonyl group, a $C_1$-$C_6$ alkylamino $C_1$-$C_6$ alkyl group, a $C_1$-$C_6$ alkylaminocarbonyl group, a $C_1$-$C_6$ alkylamino $C_1$-$C_6$ alkylcarbonyl group, and an oxo group), a hydroxy group, and carboxy group;

Group B: a $C_1$-$C_6$ alkyl group (said $C_1$-$C_6$ alkyl group being a substituent on an amino group of the amino $C_1$-$C_6$ alkylamino group), a formyl group, and a $C_2$-$C_6$ alkanoyl group;

Group C: a halogen atom, a hydroxy group, a $C_1$-$C_6$ alkyl group which may be substituted with one or more hydroxy groups, a $C_1$-$C_6$ alkoxy group, a $C_1$-$C_6$ alkoxy $C_1$-$C_6$ alkyl group (said $C_1$-$C_6$ alkoxy $C_1$-$C_6$ alkyl group may have one or more substituents selected from the group consisting of a hydroxyphenyl group, a carboxy group, and a 4- to 7-membered saturated or unsaturated heterocyclic group which may have a substituent(s)), a halogeno $C_1$-$C_6$ alkyl group, a carboxy $C_1$-$C_6$ alkyl group, a $C_1$-$C_6$ alkoxycarbonyl $C_1$-$C_6$ alkyl group, a $C_1$-$C_6$ alkylamino $C_1$-$C_6$ alkyl group, a carbamoyl $C_1$-$C_6$ alkyl group, a carboxy group, a formyl group, a $C_2$-$C_6$ alkanoyl group, a $C_1$-$C_6$ alkoxycarbonyl group, an amino group, a $C_1$-$C_6$ alkylamino group, a $C_1$-$C_6$ alkylsulfonyl group, an oxo group, a phenyl group, a 4- to 7-membered saturated or unsaturated heterocyclic group (said 4- to 7-membered saturated or unsaturated heterocyclic group may have one or more substituents selected from a $C_1$-$C_6$ alkyl group, a $C_2$-$C_6$ alkanoyl group, and an oxo group), and a $C_1$-$C_6$ alkylene-4- to 7-membered saturated or unsaturated heterocyclic group (said 4- to 7-membered saturated or unsaturated heterocyclic group may have one or more substituents selected from a $C_1$-$C_6$ alkyl group, a $C_2$-$C_6$ alkanoyl group, and an oxo group);

Group D: a $C_1$-$C_6$ alkyl group and an oxo group;

Group G: a $C_1$-$C_6$ alkoxy group, an oxo group, a 4- to 7-membered saturated or unsaturated heterocyclic group (said 4- to 7-membered saturated or unsaturated heterocyclic group may have one or more $C_1$-$C_6$ alkyl groups and/or oxo groups), a hydroxy group, and a $C_3$-$C_8$ cycloalkyl group; and

Group I: a halogen atom, an amino group, a $C_1$-$C_6$ alkyl group, a $C_1$-$C_6$ alkoxy group, a $C_1$-$C_6$ alkylamino group, a halogeno $C_1$-$C_6$ alkyl group, and a cyano group.

[36] A compound represented by formula (1-C), a salt of the compound, or a solvate of the compound or the salt:

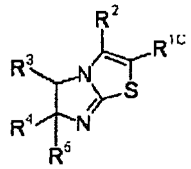

(I-C)

wherein
$R^{1C}$ represents -CO-$X_1$-CO-$X_2$, wherein
$X_1$ represents a group selected from the group consisting of an NH-$C_1$-$C_6$ alkylene group (said NH-$C_1$-$C_6$ alkylene group may be substituted on NH with a $C_1$-$C_6$ alkyl group), a divalent 4- to 7-membered saturated or unsaturated nitrogen-containing heterocyclic group which may have one or more substituents selected from the following Group E, and a divalent 8- to 11-membered bicyclic condensed nitrogen-containing heterocyclic group which may have one or more substituents selected from the following Group F,
$X_2$ represents a group selected from the group consisting of a hydroxy group, a $C_1$-$C_6$ alkoxy group, an amino group which may be substituted with one or two 4-to 7-membered saturated or unsaturated heterocyclic groups (said 4- to 7-membered saturated or unsaturated heterocyclic group may have one or more $C_1$-$C_6$ alkyl groups and/or oxo groups), a $C_1$-$C_6$ alkylamino group which may have one or more substituents selected from the following Group A, an amino $C_1$-$C_6$ alkylamino group which may have one or more substituents selected from the following Group B, a 4- to 7-membered saturated or unsaturated nitrogen-containing heterocyclic group which may have one or more substituents selected from the following Group C, and an 8- to 11-membered bicyclic condensed nitrogen-containing heterocyclic group which may have one or more substituents selected from the following Group D,
$R^2$ represents a group selected from the group consisting of a $C_1$-$C_6$ alkyl group which may have one or more substituents selected from the following Group G,
$R^3$ represents a group selected from the group consisting of a phenyl group which may have one or more substituents selected from the following Group I and a 5-to 6-membered aromatic heterocyclic group which may have one or more substituents selected from the following Group I,
$R^4$ represents a group selected from the group consisting of a $C_1$-$C_6$ alkyl group which may have one or more substituents selected from the following Group H, a phenyl group which may have one or more substituents selected from the following Group I, and a 5- to 6-membered aromatic heterocyclic group which may have one or more substituents selected from the following Group I,
$R^5$ represents a hydrogen atom or a $C_1$-$C_6$ alkyl group, and
furthermore, $R^4$ and $R^5$ together with the carbon atom on the ring to which $R^4$ and $R^5$ are bonded may form a 3-to 7-membered spiro ring:
Group A: a $C_1$-$C_6$ alkylamino $C_1$-$C_6$ alkoxy group, a hydroxy $C_1$-$C_6$ alkoxy group, a $C_1$-$C_6$ alkoxy group, a $C_1$-$C_6$ alkoxycarbonyl group, a $C_1$-$C_6$ alkylamino group, a 4- to 7-membered saturated or unsaturated heterocyclic group (said 4- to 7-membered saturated or unsaturated heterocyclic group may have one or more substituents selected from the group consisting of a $C_1$-$C_6$ alkyl group which may be substituted with one or more hydroxy groups, a $C_2$-$C_6$ alkanoyl group, a hydroxy group, a $C_1$-$C_6$ alkoxycarbonyl group, a $C_1$-$C_6$ alkylamino $C_1$-$C_6$ alkyl group, a $C_1$-$C_6$ alkylaminocarbonyl group, a $C_1$-$C_6$ alkylamino $C_1$-$C_6$ alkylcarbonyl group, and an oxo group), a hydroxy group, and a carboxy group;
Group B: a $C_1$-$C_6$ alkyl group (said $C_1$-$C_6$ alkyl group being a substituent on an amino group of the amino $C_1$-$C_6$ alkylamino group), a formyl group, and a $C_2$-$C_6$ alkanoyl group;
Group C: a halogen atom, a hydroxy group, a $C_1$-$C_6$ alkyl group which may be substituted with one or more hydroxy groups, a $C_1$-$C_6$ alkoxy group, a $C_1$-$C_6$ alkoxy $C_1$-$C_6$ alkyl group (said $C_1$-$C_6$ alkoxy $C_1$-$C_6$ alkyl group may have one or more substituents selected from the group consisting of a hydroxyphenyl group, a carboxy group, and a 4- to 7-membered saturated or unsaturated heterocyclic group which may have a substituent(s)), a halogeno $C_1$-$C_6$ alkyl group, a carboxy $C_1$-$C_6$ alkyl group, a $C_1$-$C_6$ alkoxycarbonyl $C_1$-$C_6$ alkyl group, a $C_1$-$C_6$ alkylamino $C_1$-$C_6$ alkyl group, a carbamoyl $C_1$-$C_6$ alkyl group, a carboxy group, a formyl group, a $C_2$-$C_6$ alkanoyl group, a $C_1$-$C_6$ alkoxycarbonyl group, an amino group, a $C_1$-$C_6$ alkylamino group, a $C_1$-$C_6$ alkylsulfonyl group, an oxo group, a phenyl group, a 4- to 7-membered saturated or unsaturated heterocyclic group (said 4- to 7-membered saturated or unsaturated heterocyclic group may have one or more substituents selected from a $C_1$-$C_6$ alkyl group, a $C_2$-$C_6$ alkanoyl group, and an oxo group), and a $C_1$-$C_6$ alkylene-4- to 7-membered saturated or unsaturated heterocyclic group (said 4- to 7-membered saturated or unsaturated heterocyclic group may have one or more substituents selected from a $C_1$-$C_6$ alkyl group, a $C_2$-$C_6$ alkanoyl group, and an oxo group);

Group D: a $C_1$-$C_6$ alkyl group and an oxo group;
Group E: a halogen atom, a hydroxy group, a $C_1$-$C_6$ alkyl group, a $C_1$-$C_6$ alkoxy group, an amino group, a $C_1$-$C_6$ alkylamino group, a cyano group, a $C_1$-$C_6$ alkylamino $C_1$-$C_6$ alkyl group, and an oxo group;
Group F: a $C_1$-$C_6$ alkyl group and an oxo group;
Group G: a $C_1$-$C_6$ alkoxy group, an oxo group, a 4- to 7-membered saturated or unsaturated heterocyclic group (said 4- to 7-membered saturated or unsaturated heterocyclic group may have one or more $C_1$-$C_6$ alkyl groups and/or oxo groups), a hydroxy group, and a $C_3$-$C_8$ cycloalkyl group;
Group H: a phenyl group and a $C_3$-$C_8$ cycloalkyl group; and
Group I: a halogen atom, an amino group, a $C_1$-$C_6$ alkyl group, a $C_1$-$C_6$ alkoxy group, a $C_1$-$C_6$ alkylamino group, a halogeno $C_1$-$C_6$ alkyl group, and a cyano group.

[37] An inhibitor of Mdm2 comprising the compound according to any one of [1] to [36], a salt of the compound, or a solvate of the compound or the salt.

[38] An inhibitor of Mdm2 ubiquitin ligase comprising the compound according to any one of [1] to [36], a salt of the compound, or a solvate of the compound or the salt.

[39] An inhibitor of p53-Mdm2 binding comprising the compound according to any one of [1] to [36], a salt of the compound, or a solvate of the compound or the salt.

[40] An inhibitor of suppression of p53 transcription activity comprising the compound according to any one of [1] to [36], a salt of the compound, or a solvate of the compound or the salt.

[41] An inhibitor of p53 degradation comprising the compound according to any one of [1] to [36], a salt of the compound, or a solvate of the compound or the salt.

[42] A medicament comprising the compound according to any one of [1] to [36], a salt of the compound, or a solvate of the compound or the salt as an active ingredient.

[43] An anti-tumor agent comprising the compound according to any one of [1] to [36], a salt of the compound, or a solvate of the compound or the salt as an active ingredient.

[44] A pharmaceutical composition comprising the compound according to any one of [1] to [36], a salt of the compound, or a solvate of the compound or the salt and a pharmaceutically acceptable carrier.

[45] A method for treating cancer, characterized by administering the compound according to any one of [1] to [36], a salt of the compound, or a solvate of the compound or the salt.

[46] Use of the compound according to any one of [1] to [36], a salt of the compound, or a solvate of the compound or the salt for the manufacture of a medicament.

[47] Use of the compound according to any one of [1] to [36], a salt of the compound, or a solvate of the compound or the salt for the manufacture of an anti-tumor agent.

## Advantage of the Invention

The present invention provides a novel imidazothiazole derivative represented by formula (1), which has Mdm2 inhibiting activity. Such a novel compound is useful as an anti-tumor agent.

## Best Mode for Carrying Out the Invention

In the present invention, "Mdm2" means a protein encoded by the murine double minute 2 gene. "Mdm2" includes Mdm2 proteins encoded by a complete length of the Mdm2 gene, Mdm2 proteins encoded by mutated Mdm2 genes (including deletion mutants, substitution mutants, and addition mutants), and so forth. In the present invention, "Mdm2" also includes homologues derived from various animal species such as, for example, human Mdm2 homologue (HDM2).

In the present invention, "p53" means a protein encoded by the p53 gene. "p53" means the p53 protein encoded by a full length p53 gene or a p53 protein that has a mutation (including mutations by deletion, substitution, and addition), but functions normally.

In the present invention, "Mdm2 inhibitor" means a factor that restores p53 functions suppressed by Mdm2 by acting on Mdm2 or p53, or on both p53 and Mdm2. The p53 functions are not particularly limited so long as they are functions which p53 normally has. Examples thereof include inhibition of canceration of cells by inducing the expression of genes involved in the cell cycle or cellular apoptosis. Examples of Mdm2 inhibitors include factors that inhibit binding of Mdm2 to p53 (hereinafter, referred to as p53-Mdm2 binding inhibitors) or factors that inhibit ubiquitination of p53 by Mdm2 (hereinafter, referred to as Mdm2 ubiquitin ligase inhibitors).

In the present invention, "inhibitor of suppression of p53 transcription activity" means a factor that restores the functions of p53 as a transcription factor suppressed by Mdm2.

In the present invention, "inhibitor of p53 degradation" means a factor that inhibits degradation of p53 in proteasomes by inhibiting ubiquitination of p53 by Mdm2.

In the present invention, the terms "tumor" and "cancer" are used interchangeably. Furthermore, in the present invention, tumor, malignant tumor, cancer, malignant neoplasm, carcinoma, sarcoma, and the like may be collectively referred to as "tumor" or "cancer."

Hereafter, each substituent in the formula (1) will be explained.

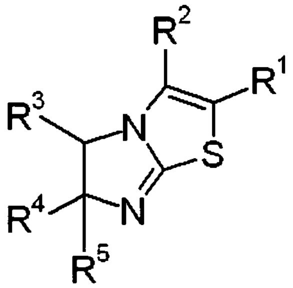

(1)

In the present invention, "$C_1$-$C_6$ alkyl group" means a straight, branched, or cyclic alkyl group having 1 to 6 carbon atoms unless otherwise specified. Examples of the $C_1$-$C_6$ alkyl group include a methyl group, an ethyl group, a propyl group, an isopropyl group, a cyclopropyl group, a butyl group, a pentyl group, and a hexyl group.

In the present invention, "$C_1$-$C_6$ alkoxy group" means an alkoxy group containing a straight, branched, or cyclic alkyl group having 1 to 6 carbon atoms as a component unless otherwise specified. Examples of the $C_1$-$C_6$ alkoxy group include a methoxy group, an ethoxy group, a propoxy group, an isopropoxy group, a butoxy group, an isobutoxy group, a tert-butoxy group, and a pentoxy group.

In the present invention, "halogen atom" means a chlorine atom, a fluorine atom, a bromine atom, or an iodine atom unless otherwise specified.

In the present invention, "oxo group" means a group represented by "=0" unless otherwise specified.

[I] Regarding $R^1$

$R^1$ represents a hydrogen atom, -$V_1$-$V_2$, -CO-W, or -CO$X_1$-CO-$X_2$.

[1-1] Regarding $V_1$

$V_1$ represents a $C_1$-$C_6$ alkylene group.

In $V_1$, the "$C_1$-$C_6$ alkylene group" means an unsubstituted straight, branched, or cyclic alkylene group having 1 to 6 carbon atoms. Examples of the straight, branched, or cyclic alkylene group having 1 to 6 carbon atoms include a methylene group, an ethylene group, and ethylidene group (-CH($CH_3$)-).

[1-2] Regarding $V_2$, W, and $X_2$

$V_2$, W, and $X_2$ each independently represents a substituent selected from the group consisting of a hydrogen atom, a hydroxy group, a $C_1$-$C_6$ alkoxy group, an amino group that may be substituted with one or two 4- to 7-membered saturated or unsaturated heterocyclic groups (the 4- to 7-membered saturated or unsaturated heterocyclic group may have one or more $C_1$-$C_6$ alkyl groups and/or oxo groups), a $C_1$-$C_6$ alkylamino group that may have one or more substituents selected from the following Group A, an amino $C_1$-$C_6$ alkylamino group that may have one or more substituents selected from the following Group B, a 4- to 7-membered saturated or unsaturated nitrogen-containing heterocyclic group that may have one or more substituents selected from the following Group C, and an 8- to 11-membered bicyclic condensed nitrogen-containing heterocyclic group that may have one or more substituents selected from the following Group D.

Group A: a $C_1$-$C_6$ alkylamino $C_1$-$C_6$ alkoxy group, a hydroxy $C_1$-$C_6$ alkoxy group, a $C_1$-$C_6$ alkoxy group, a $C_1$-$C_6$ alkoxycarbonyl group, a $C_1$-$C_6$ alkylamino group, a 4- to 7-membered saturated or unsaturated heterocyclic group (the 4- to 7-membered saturated or unsaturated heterocyclic group may have one or more substituents selected from the group consisting of a $C_1$-$C_6$ alkyl group that may be substituted with one or more hydroxy groups, a $C_2$-$C_6$ alkanoyl group, a hydroxy group, a $C_1$-$C_6$ alkoxycarbonyl group, a $C_1$-$C_6$ alkylamino C1-$C_6$ alkyl group, a $C_1$-$C_6$ alkylaminocarbonyl group, a $C_1$-$C_6$ alkylamino $C_1$-$C_6$ alkylcarbonyl group, and an oxo group), a hydroxy group, and

a carboxy group.

Group B: a $C_1$-$C_6$ alkyl group (the $C_1$-$C_6$ alkyl group being substituted on an amino group of the amino $C_1$-$C_6$ alkylamino group), a formyl group, and a $C_2$-$C_6$ alkanoyl group.

Group C: a halogen atom, a hydroxy group, a $C_1$-$C_6$ alkyl group that may be substituted with one or more hydroxy groups, a $C_1$-$C_6$ alkoxy group, and a $C_1$-$C_6$ alkoxy $C_1$-$C_6$ alkyl group (the $C_1$-$C_6$ alkoxy $C_1$-$C_6$ alkyl group may have one or more substituents selected from the group consisting of a hydroxyphenyl group, a carboxy group, and a 4- to 7-membered saturated or unsaturated heterocyclic group that may have a substituent(s)), a halogeno $C_1$-$C_6$ alkyl group, a carboxy $C_1$-$C_6$ alkyl group, a $C_1$-$C_6$ an alkoxycarbonyl $C_1$-$C_6$ alkyl group, a $C_1$-$C_6$ alkylamino $C_1$-$C_6$ alkyl group, a carbamoyl $C_1$-$C_6$ alkyl group, a carboxy group, a formyl group, a $C_2$-$C_6$ alkanoyl group, a $C_1$-$C_6$ alkoxy-carbonyl group, an amino group, a $C_1$-$C_6$ alkylamino group, a $C_1$-$C_6$ alkylsulfonyl group, an oxo group, a phenyl group, a 4- to 7-membered saturated or unsaturated heterocyclic group (the 4- to 7-membered saturated or unsaturated heterocyclic group may have one or more $C_1$-$C_6$ alkyl groups, $C_2$-$C_6$ alkanoyl groups, or oxo groups), and a $C_1$-$C_6$ alkylene-4- to 7-membered saturated or unsaturated heterocyclic group (the 4- to 7-membered saturated or unsaturated heterocyclic group may have one or more substituents selected from a $C_1$-$C_6$ alkyl group, a $C_2$-$C_6$ alkanoyl group, and an oxo group).

Group D: a $C_1$-$C_6$ alkyl group and an oxo group.

In $V_2$, W, and $X_2$, the "amino group that may be substituted with one or two 4- to 7-membered saturated or unsaturated heterocyclic groups (the 4- to 7-membered saturated or unsaturated heterocyclic group may have one or more $C_1$-$C_6$ alkyl groups and/or oxo groups)" means an unsubstituted amino group or an amino group substituted with a 4- to 7-membered saturated or unsaturated heterocyclic group at one or two positions. Here, the 4-to 7-membered saturated or unsaturated heterocyclic group means a group derived from a 4- to 7-membered, saturated or unsaturated heterocyclic compound containing one or more oxygen atoms, nitrogen atoms, or sulfur atoms as constituent atom(s) of the ring structure. The 4- to 7-membered saturated or unsaturated heterocyclic group may be bonded at any position. Examples of the 4- to 7-membered saturated heterocyclic group include groups derived from azetidine, pyrrolidine, imidazolidine, triazolidine, tetrahydrofuran, oxazolidine, thiazolidine, piperidine, piperazine, tetrahydropyrane, dioxane, morpholine, thiomorpholine, homomorpholine, homopiperazine, and the like. Examples of the 4- to 7-membered unsaturated heterocyclic group include groups derived from pyrrole, dihydropyrrole, pyrazole, imidazole, thiophene, furan, pyridine, dihydropyridine, tetrahydro pyridine, pyridazine, pyrimidine, thiazole, oxadiazole, tetrazole, dihydrooxadiazole, and the like. These 4- to 7-membered saturated or unsaturated heterocyclic groups may have one or more $C_1$-$C_6$ alkyl groups and/or oxo groups. Here, the $C_1$-$C_6$ alkyl group means a straight, branched, or cyclic alkyl group having 1 to 6 carbon atoms, and examples thereof include a methyl group, an ethyl group, a propyl group, an isopropyl group, a cyclopropyl group, a butyl group, a pentyl group, and a hexyl group.

In $V_2$, W, and $X_2$, the "$C_1$-$C_6$ alkylamino group that may have one or more substituents selected from Group A" means a mono-$C_1$-$C_6$ alkylamino group that is substituted with one $C_1$-$C_6$ alkyl group, or a di-$C_1$-$C_6$ alkylamino group that is substituted with two $C_1$-$C_6$ alkyl groups, and the $C_1$-$C_6$ alkyl group moiety or the amino group moiety may be substituted with one or more substituents selected from the above-mentioned Group A. The $C_1$-$C_6$ alkyl group moiety may be straight, branched, or cyclic. When the $C_1$-$C_6$ alkylamino group is a di-$C_1$-$C_6$ alkylamino group, the two $C_1$-$C_6$ alkyl groups may be identical to or different from each other. Therefore, examples of the $C_1$-$C_6$ alkylamino group include a methylamino group, a dimethylamino group, an ethylamino group, a methyl(ethyl)amino group, an isopropyl(methyl) amino group, and so forth.

The $C_1$-$C_6$ alkylamino $C_1$-$C_6$ alkoxy group that can be present as a substituent on a "$C_1$-$C_6$ alkylamino group that may have one or more substituents selected from Group A" means a $C_1$-$C_6$ alkoxy group containing a $C_1$-$C_6$ alkylamino group having the same meaning as that of the above-mentioned $C_1$-$C_6$ alkylamino group as a component. Examples of the $C_1$-$C_6$ alkylamino $C_1$-$C_6$ alkoxy group include a dimethylaminomethyloxy group.

The hydroxy $C_1$-$C_6$ alkoxy group that can be present as a substituent on a "$C_1$-$C_6$ alkylamino group that may have one or more substituents selected from Group A" means a $C_1$-$C_6$ alkoxy group containing a $C_1$-$C_6$ alkyl group having the same meaning as that of the above-mentioned $C_1$-$C_6$ alkyl group substituted with one or two hydroxy groups as a component. Examples of the hydroxy $C_1$-$C_6$ alkoxy group include a hydroxymethyl group, a hydroxyethyl group, and so forth.

The $C_1$-$C_6$ alkylamino group that can be present as a substituent on a "$C_1$-$C_6$ alkylamino group that may have one or more substituents selected from Group A" has the same definition as that of the above-mentioned $C_1$-$C_6$ alkylamino group, and examples thereof include a dimethylamino group.

The $C_1$-$C_6$ alkoxycarbonyl group that can be present as a substituent on a "$C_1$-$C_6$ alkylamino group that may have one or more substituents selected from Group A" means a $C_1$-$C_6$ alkoxycarbonyl group containing a $C_1$-$C_6$ alkoxy group having the same meaning as that of the above-mentioned $C_1$-$C_6$ alkoxy group as a component. Examples of the $C_1$-$C_6$ alkoxycarbonyl group include a methoxycarbonyl group, an ethoxycarbonyl group, and an isopropoxycarbonyl group.

The 4- to 7-membered saturated or unsaturated heterocyclic group in the 4- to 7-membered saturated or unsaturated heterocyclic group (the 4- to 7-membered saturated or unsaturated heterocyclic group may have one or more substituents selected from the group consisting of a $C_1$-$C_6$ alkyl group that may be substituted with one or more hydroxy groups, a $C_2$-$C_6$ alkanoyl group, a hydroxy group, a $C_1$-$C_6$ alkoxycarbonyl group, a $C_1$-$C_6$ alkylamino $C_1$-$C_6$ alkyl group, a $C_1$-$C_6$ alkylaminocarbonyl group, a $C_1$-$C_6$ alkylamino $C_1$-$C_6$ alkylcarbonyl group, and an oxo group) that can be present as a substituent on a "$C_1$-$C_6$ alkylamino group that may have one or more substituents selected from Group A" has the same meaning as that of the heterocyclic group in the above-mentioned 4- to 7-membered saturated or unsaturated heterocyclic group (the 4- to 7-membered saturated or unsaturated heterocyclic group may have one or more $C_1$-$C_6$ alkyl groups and/or oxo groups) explained as a substituent on an amino group in $V_2$, W, and $X_2$. The substituent that can be present as a substituent may have one or more substituents selected from the group consisting of a $C_1$-$C_6$ alkyl group substituted with a hydroxy group (may be one or more), a $C_2$-$C_6$ alkanoyl group, a hydroxy group, a $C_1$-$C_6$ alkoxycarbonyl group, a $C_1$-$C_6$ alkylamino $C_1$-$C_6$ alkyl group, a $C_1$-$C_6$ alkylaminocarbonyl group, and $C_1$-$C_6$ alkylamino $C_1$-$C_6$ alkylcarbonyl group in addition to the above-mentioned alkyl groups and oxo group.

Here, the $C_1$-$C_6$ alkyl group substituted with a hydroxy group (may be one or more) means a hydroxy $C_1$-$C_6$ alkyl group containing a $C_1$-$C_6$ alkyl group having the same meaning as that of the above-mentioned $C_1$-$C_6$ alkyl group as a component. Examples of the hydroxy $C_1$-$C_6$ alkyl group include a hydroxymethyl group, a hydroxyethyl group, a hydroxypropyl group, a hydroxybutyl group, and so forth. The $C_2$-$C_6$ alkanoyl group means a straight or branched alkanoyl group having 2 to 6 carbon atoms that contains an alkyl group having 1 to 5 carbon atoms as a component, and examples thereof include an acetyl group, a propionyl group, a butyryl group, a valeryl group, and a hexanoyl group. The $C_1$-$C_6$ alkoxycarbonyl group means a $C_1$-$C_6$ alkoxycarbonyl group containing the above-mentioned $C_1$-$C_6$ alkoxy group as a component. Examples of the $C_1$-$C_6$ alkoxycarbonyl group include a methoxycarbonyl group, an ethoxycarbonyl group, an isopropoxycarbonyl group, a tertiary butoxycarbonyl group, and so forth. The $C_1$-$C_6$ alkylamino $C_1$-$C_6$ alkyl group means a $C_1$-$C_6$ alkyl group substituted with a $C_1$-$C_6$ alkylamino group defined as above, and examples thereof include a dimethylaminomethyl group. The $C_1$-$C_6$ alkylaminocarbonyl group means a carbonyl group substituted with $C_1$-$C_6$ alkylamino group defined as above, and examples thereof include a dimethylaminocarbonyl group. The $C_1$-$C_6$ alkylamino $C_1$-$C_6$ alkylcarbonyl group means a $C_1$-$C_6$ alkylcarbonyl group substituted with a $C_1$-$C_6$ alkylamino group defined as above, and examples thereof include a dimethylaminomethylcarbonyl group.

In $V_2$, W, and $X_2$, the "amino $C_1$-$C_6$ alkylamino group that may have one or more substituents selected from Group B" means an unsubstituted amino $C_1$-$C_6$ alkylamino group or an amino $C_1$-$C_6$ alkylamino group substituted with one or more substituents selected from the above-mentioned Group B. Examples of the amino $C_1$-$C_6$ alkylamino group include an aminoethylamino group and an aminopropyl amino group.

Here, the $C_1$-$C_6$ alkyl group that can be present as a substituent on an "amino $C_1$-$C_6$ alkylamino group that may have one or more substituents selected from Group B" (the amino $C_1$-$C_6$ alkylamino group is substituted on an amino group with the $C_1$-$C_6$ alkyl group) means a straight, branched, or cyclic alkyl group having 1 to 6 carbon atoms (for example, a methyl group, an ethyl group, a propyl group, an isopropyl group, a cyclopropyl group, a butyl group, a pentyl group, and a hexyl group) which is present as a substituent on either amino group of the amino $C_1$-$C_6$ alkylamino group.

The $C_2$-$C_6$ alkanoyl group that can be present as a substituent on an "amino $C_1$-$C_6$ alkylamino group that may have one or more substituents selected from Group B" means an alkanoyl group having 2 to 6 carbon atoms that contains a straight or branched alkyl group having 1 to 5 carbon atoms as a component, and examples thereof include an acetyl group, a propionyl group, a butyryl group, a valeryl group, and hexanoyl group.

In $V_2$, W, and $X_2$, the "4- to 7-membered saturated or unsaturated nitrogen-containing heterocyclic group that may have one or more substituents selected from Group C" means an unsubstituted saturated or unsaturated 4- to 7-membered nitrogen-containing heterocyclic group or a saturated or unsaturated 4- to 7-membered nitrogen-containing heterocyclic group having one or more substituents selected from the above-mentioned Group C. Here, the saturated or unsaturated 4- to 7-membered nitrogen-containing heterocyclic group means a group derived from a saturated or unsaturated 4- to 7-membered heterocyclic compound containing at least one nitrogen atom as a constituent atom of the ring structure. The 4-to 7-membered nitrogen-containing heterocyclic group may be bonded at any position. Examples of the 4- to 7-membered nitrogen-containing saturated heterocyclic group include groups derived from azetidine, pyrrolidine, imidazolidine, triazolidine, oxazolidine, thiazolidine, piperidine, piperazine, morpholine, thiomorpholine, homomorpholine, and homopiperazine. Examples of the 4-to 7-membered nitrogen-containing unsaturated heterocyclic group include groups derived from pyrrole, pyrazole, imidazole, triazole, thiazole, isothiazole, oxazole, isoxazole, pyridine, pyridazine, and pyrimidine.

Here, the $C_1$-$C_6$ alkyl group that may be substituted with one or more hydroxy groups that can be present as a substituent on a "4- to 7-membered nitrogen-containing heterocyclic group that may have one or more substituents selected from Group C" means a hydroxy $C_1$-$C_6$ alkyl group containing an unsubstituted $C_1$-$C_6$ alkyl group having the same meaning as that of the above-mentioned $C_1$-$C_6$ alkyl group or a $C_1$-$C_6$ alkyl group having the same meaning as that of the above-mentioned $C_1$-$C_6$ alkyl group as a component. Examples of the hydroxy $C_1$-$C_6$ alkyl group include a

hydroxymethyl group, a hydroxyethyl group, a hydroxypropyl group, a hydroxybutyl group, a hydroxypentyl group, and a hydroxyhexyl group. One or more hydroxy groups may be contained.

The $C_1$-$C_6$ alkoxy $C_1$-$C_6$ alkyl group that can be present as a substituent on a "4- to 7-membered nitrogen-containing heterocyclic group that may have one or more substituents selected from Group C" means a $C_1$-$C_6$ alkoxy $C_1$-$C_6$ alkyl group containing a $C_1$-$C_6$ alkoxy group having the same meaning as that of the above-mentioned $C_1$-$C_6$ alkoxy group and a $C_1$-$C_6$ alkyl group having the same meaning as that of the above-mentioned $C_1$-$C_6$ alkyl group as components. Examples of the $C_1$-$C_6$ alkoxy $C_1$-$C_6$ alkyl group include a methoxymethyl group, an ethoxymethyl group, a methoxyethyl group, and an isopropoxymethyl group. Furthermore, the $C_1$-$C_6$ alkoxy $C_1$-$C_6$ alkyl group may have one or more substituents selected from the group consisting of a hydroxyphenyl group, a carboxy group, and a 4- to 7-membered saturated or unsaturated heterocyclic group that may have a substituent(s). Here, examples of the substituent that can be present as a substituent on a 4- to 7-membered saturated or unsaturated heterocyclic group that may have a substituent(s) include a $C_1$-$C_6$ alkyl group and a hydroxy group, and examples of the 4-to 7-membered saturated or unsaturated heterocyclic group include a pyrrolidinyl group, a piperidinyl group, a pyridyl group, and so forth.

The halogeno $C_1$-$C_6$ alkyl group that can be present as a substituent on a "4- to 7-membered nitrogen-containing heterocyclic group that may have one or more substituents selected from Group C" means a straight, branched, or cyclic alkyl group having 1 to 6 carbon atoms that has one or more halogen atoms selected from the group consisting of a chlorine atom, a fluorine atom, a bromine atom, and an iodine atom. When the $C_1$-$C_6$ alkyl group is substituted with two or more halogen atoms, these halogen atoms may be identical to or different from each other. Examples of the halogeno $C_1$-$C_6$ alkyl group include a fluoromethyl group, a difluoromethyl group, a trifluoromethyl group, a chloromethyl group, a chloroethyl group, and a chlorobutyl group.

The carboxy $C_1$-$C_6$ alkyl group that can be present as a substituent on a "4- to 7-membered nitrogen-containing heterocyclic group that may have one or more substituents selected from Group C" means a carboxy $C_1$-$C_6$ alkyl group containing a $C_1$-$C_6$ alkyl group having the same meaning as that of the above-mentioned $C_1$-$C_6$ alkyl group as a component. Examples of the carboxy $C_1$-$C_6$ alkyl group include a carboxymethyl group, a carboxyethyl group, a carboxypropyl group, a carboxybutyl group, a carboxypentyl group, and a carboxyhexyl group.

The $C_1$-$C_6$ alkoxycarbonyl $C_1$-$C_6$ alkyl group that can be present as a substituent on a "4- to 7-membered nitrogen-containing heterocyclic group that may have one or more substituents selected from Group C" means a $C_1$-$C_6$ alkoxycarbonyl $C_1$-$C_6$ alkyl group containing a $C_1$-$C_6$ alkoxy group having the same meaning as that of the above-mentioned $C_1$-$C_6$ alkoxy group and a $C_1$-$C_6$ alkyl group having the same meaning as that of the above-mentioned $C_1$-$C_6$ alkyl group as a component. Examples of the $C_1$-$C_6$ alkoxycarbonyl $C_1$-$C_6$ alkyl group include a methoxycarbonylmethyl group, a methoxycarbonylethyl group, an ethoxycarbonylmethyl group, and an isopropoxycarbonylmethyl group.

The $C_1$-$C_6$ alkylamino $C_1$-$C_6$ alkyl group that can be present as a substituent on a "4- to 7-membered nitrogen-containing heterocyclic group that may have one or more substituents selected from Group C" means a mono-$C_1$-$C_6$ alkylamino $C_1$-$C_6$ alkyl group or a di-$C_1$-$C_6$ alkylamino $C_1$-$C_6$ alkyl group. Here, the $C_1$-$C_6$ alkyl group in the $C_1$-$C_6$ alkylamino $C_1$-$C_6$ alkyl group means a straight, branched, or cyclic alkyl group having 1 to 6 carbon atoms as with the above-mentioned $C_1$-$C_6$ alkyl group. The $C_1$-$C_6$ alkyl groups may be identical to or different from each other. Therefore, examples of the $C_1$-$C_6$ alkylamino $C_1$-$C_6$ alkyl group include a methylaminomethyl group, a dimethylaminomethyl group, a methylaminoethyl group, an ethyl(methyl)aminomethyl group, an ethyl(methyl)aminopropyl group, an isopropylaminomethyl group, an isopropyl(methyl)aminomethyl group, and a dimethylaminoethyl group.

The carbamoyl $C_1$-$C_6$ alkyl group that can be present as a substituent on a "4- to 7-membered nitrogen-containing heterocyclic group that may have one or more substituents selected from Group C" means a carbamoylalkyl group containing a $C_1$-$C_6$ alkyl group having the same meaning as that of the above-mentioned $C_1$-$C_6$ alkyl group as a component. Examples of the carbamoyl $C_1$-$C_6$ alkyl group include a carbamoylmethyl group, a carbamoylethyl group, a carbamoylpropyl group, a carbamoylbutyl group, a carbamoylpentyl group, and a carbamoylhexyl group.

The $C_2$-$C_6$ alkanoyl group that can be present as a substituent on a "4- to 7-membered nitrogen-containing heterocyclic group that may have one or more substituents selected from Group C" means an alkanoyl group having 2 to 6 carbon atoms that contains a straight or branched alkyl group having 1 to 5 carbon atoms as a component, and examples thereof include an acetyl group, a propionyl group, a butyryl group, a valeryl group, and a hexanoyl group.

The $C_1$-$C_6$ alkoxycarbonyl group that can be present as a substituent on a "4- to 7-membered nitrogen-containing heterocyclic group that may have one or more substituents selected from Group C" means a $C_1$-$C_6$ alkoxycarbonyl group containing a $C_1$-$C_6$ alkoxy group having the same meaning as that of the above-mentioned $C_1$-$C_6$ alkoxy group as a component. Examples of the $C_1$-$C_6$ alkoxycarbonyl group include a methoxycarbonyl group, an ethoxycarbonyl group, and an isopropoxycarbonyl group.

The $C_1$-$C_6$ alkylamino group that can be present as a substituent on a "4- to 7-membered nitrogen-containing heterocyclic group that may have one or more substituents selected from Group C" means a mono-$C_1$-$C_6$ alkylamino group or a di-$C_1$-$C_6$ alkylamino group. When the $C_1$-$C_6$ alkylamino group is a di-$C_1$-$C_6$ alkylamino group, the two $C_1$-$C_6$ alkyl groups may be identical to or different from each other. Therefore, examples of the $C_1$-$C_6$ alkylamino group include a methylamino group, an ethylamino group, an isopropyl amino group, a dimethylamino group, a diethylamino group,

an ethyl(methyl)amino group, and an isopropyl(methyl)amino group.

The $C_1$-$C_6$ alkylsulfonyl group that can be present as a substituent on a "4- to 7-membered nitrogen-containing heterocyclic group that may have one or more substituents selected from Group C" means an alkylsulfonyl group containing a $C_1$-$C_6$ alkyl group having the same meaning as that of the above-mentioned $C_1$-$C_6$ alkyl group as a component. Examples of the $C_1$-$C_6$ alkylsulfonyl group include a methylsulfonyl group, an ethylsulfonyl group, a propylsulfonyl group, and an isopropylsulfonyl group.

The 4- to 7-membered saturated or unsaturated heterocyclic group (the 4- to 7-membered saturated or unsaturated heterocyclic group may have one or more substituents selected from a $C_1$-$C_6$ alkyl group, a $C_2$-$C_6$ alkanoyl group, and an oxo group) that can be present as a substituent on a "4- to 7-membered nitrogen-containing heterocyclic group that may have one or more substituents selected from Group C" has the same meaning as that of the 4- to 7-membered saturated or unsaturated heterocyclic group explained as the substituent moiety on an amino group in $V_2$, W, and $X_2$. The $C_1$-$C_6$ alkyl group and the $C_2$-$C_6$ alkanoyl group also have the same meanings as defined above.

The $C_1$-$C_6$ alkylene-4- to 7-membered saturated or unsaturated heterocyclic group (the 4- to 7-membered saturated or unsaturated heterocyclic group may have one or more substituents selected from a $C_1$-$C_6$ alkyl group, a $C_2$-$C_6$ alkanoyl group, and an oxo group) that can be present as a substituent on "4- to 7-membered nitrogen-containing heterocyclic group that may have one or more substituents selected from Group C" means a group containing a straight, branched, or cyclic alkylene group having 1 to 6 carbon atoms and a 4- to 7-membered saturated or unsaturated heterocyclic group having the same meaning as that of the 4- to 7-membered saturated or unsaturated heterocyclic group explained as a substituent on a $C_1$-$C_6$ amino group in $V_2$, W, and $X_2$. Examples of the substituent that can be present as a substituent on the heterocyclic group include a $C_1$-$C_6$ alkyl group, an acetyl group, an oxo group, and so forth. Examples of the $C_1$-$C_6$ alkylene-4- to 7-membered saturated or unsaturated heterocyclic group (the 4- to 7-membered saturated or unsaturated heterocyclic group may have one or more substituents selected from a $C_1$-$C_6$ alkyl group, a $C_2$-$C_6$ alkanoyl group, and an oxo group) include methylene-morpholine, ethylene-morpholine, methylene-pyrrolidine, methylene-piperazine, methylene-methyl acetyl piperazine, and so forth.

In $V_2$, W, and $X_2$, the "8- to 11-membered bicyclic condensed nitrogen-containing heterocyclic group that may have one or more substituents selected from Group D" means an unsubstituted 8- to 11-membered bicyclic condensed nitrogen-containing heterocyclic group or an 8-to 11-membered bicyclic condensed nitrogen-containing heterocyclic group having one or more substituents selected from the above-mentioned Group D. Examples of the 8- to 11-membered bicyclic condensed nitrogen-containing heterocyclic group include furopyrazine (for example, hexahydrofuro[3,4-b]pyrazine) or pyrrolopyrazine (for example, octahydropyrrolo[3,4-b]pyrazine).

[1-3] Regarding $X_1$

$X_1$ represents a substituent selected from the group consisting of a NH-$C_1$-$C_6$ alkylene group (the NH-$C_1$-$C_6$ alkylene group may be substituted on NH with a $C_1$-$C_6$ alkyl group), a divalent 4- to 7-membered saturated or unsaturated nitrogen-containing heterocyclic group that may have one or more substituents selected from the following Group E, and a divalent 8- to 11-membered bicyclic condensed nitrogen-containing heterocyclic group that may have one or more substituents selected from the following Group F.

Group E: a halogen atom, a hydroxy group, a $C_1$-$C_6$ alkyl group, a $C_1$-$C_6$ alkoxy group, an amino group, a $C_1$-$C_6$ alkylamino group, a cyano group, a $C_1$-$C_6$ alkylamino $C_1$-$C_6$ alkyl group, and an oxo group.
Group F: $C_1$-$C_6$ alkyl group and an oxo group.

In $X_1$, the "NH-$C_1$-$C_6$ alkylene group (the NH-$C_1$-$C_6$ alkylene group may be substituted on NH with a $C_1$-$C_6$ alkyl group)" means an unsubstituted NH-$C_1$-$C_6$ alkylene group or an NH-$C_1$-$C_6$ alkylene group having the NH moiety substituted with a $C_1$-$C_6$ alkyl group. The $C_1$-$C_6$ alkylene group means a straight, branched, or cyclic alkylene group having 1 to 6 carbon atoms.

In $X_1$, the "divalent 4- to 7-membered saturated or unsaturated nitrogen-containing heterocyclic group that may have one or more substituents selected from Group E" means an unsubstituted divalent 4- to 7-membered saturated or unsaturated nitrogen-containing heterocyclic group or a divalent saturated or unsaturated 4- to 7-membered nitrogen-containing heterocyclic group having one or more substituents selected from the above-mentioned Group E. Here, the divalent 4- to 7-membered nitrogen-containing heterocyclic group means a divalent group derived from a saturated or unsaturated heterocyclic compound containing at least one nitrogen atom as a constituent atom of the ring structure. This divalent 4- to 7-membered nitrogen-containing heterocyclic group may be bonded at any position. Examples of the divalent 4- to 7-membered nitrogen-containing saturated heterocyclic group include divalent groups derived from azetidine, pyrrolidine, imidazolidine, triazolidine, oxazolidine, thiazolidine, piperidine, piperazine, morpholine, thiomorpholine, homomorpholine, and homopiperazine. Examples of the divalent 4- to 7-membered nitrogen-containing unsaturated

heterocyclic group include divalent groups derived from pyrrole, pyrazole, imidazole, triazole, thiazole, isothiazole, oxazole, isoxazole, pyridine, pyridazine, and pyrimidine.

The $C_1$-$C_6$ alkylamino group that can be present as a substituent on a "divalent 4- to 7-membered nitrogen-containing heterocyclic group that may have one or more substituents selected from Group E" has the same meaning as that of the $C_1$-$C_6$ alkylamino group explained as a substituent on a 4- to 7-membered nitrogen-containing saturated heterocyclic group in $V_2$, W, and $X_2$.

The $C_1$-$C_6$ alkylamino $C_1$-$C_6$ alkyl group that can be present as a substituent on a "divalent 4- to 7-membered nitrogen-containing heterocyclic group that may have one or more substituents selected from Group E" has the same meaning as that of the $C_1$-$C_6$ alkylamino $C_1$-$C_6$ alkyl group explained as a substituent on a 4- to 7-membered nitrogen-containing saturated heterocyclic group in $V_2$, W, and $X_2$.

In $X_1$, the "divalent 8- to 11-membered bicyclic condensed nitrogen-containing heterocyclic group that may have one or more substituents selected from Group F" means an unsubstituted divalent 8- to 11-membered bicyclic condensed nitrogen-containing heterocyclic group or a divalent 8- to 11-membered bicyclic condensed nitrogen-containing heterocyclic group having one or more substituents selected from the above-mentioned Group F. Here, examples of the bicyclic condensed nitrogen-containing heterocyclic group include groups induced from furopyrrole, pyrrolopyrrole, and cyclopentapyrrole.

[II] Regarding $R^2$

$R^2$ represents a substituent selected from the group consisting of a hydrogen atom, a phenyl group and a $C_1$-$C_6$ alkyl group that may have one or more substituents selected from the following Group G.

Group G: a $C_1$-$C_6$ alkoxy group, an oxo group, a 4- to 7-membered saturated or unsaturated heterocyclic group (the 4- to 7-membered saturated or unsaturated heterocyclic group may have one or more $C_1$-$C_6$ alkyl groups and/or oxo groups), a hydroxy group, a $C_3$-$C_8$ cycloalkyl group.

In $R^2$, the "$C_1$-$C_6$ alkyl group that may have one or more substituents selected from Group G" means an unsubstituted $C_1$-$C_6$ alkyl group or a $C_1$-$C_6$ alkyl group that may have the above-mentioned one or more substituents selected from Group G.

Here, the 4- to 7-membered saturated or unsaturated heterocyclic group (the 4- to 7-membered saturated or unsaturated heterocyclic group may have one or more $C_1$-$C_6$ alkyl groups and/or oxo groups) that can be present as a substituent on a "$C_1$-$C_6$ alkyl group that may have one or more substituents selected from Group G" has the same meaning as that of the 4- to 7-membered saturated or unsaturated heterocyclic group explained as a substituent on an amino group in $V_2$, W, and $X_2$.

The $C_3$-$C_8$ cycloalkyl group that can be present as a substituent on a "$C_1$-$C_6$ alkyl group that may have one or more substituents selected from Group G" means a cycloalkyl group having 3 to 8 carbon atoms. Examples of the cycloalkyl group having 3 to 8 carbon atoms include a cyclopropyl group, a cyclobutyl group, a cyclopentyl group, and a cyclohexyl group.

[III] Regarding $R^3$, $R^4$, and $R^5$

$R^3$ and $R^4$ each independently represent a substituent selected from the group consisting of a $C_1$-$C_6$ alkyl group that may have one or more substituents selected from the following Group H, a phenyl group that may have one or more substituents selected from the following Group I, and a 5- or 6-membered aromatic heterocyclic group that may have one or more substituents selected from the following Group I (provided that $R^3$ and $R^4$ do not both represent a $C_1$-$C_6$ alkyl group that may have a substituent(s)). $R^5$ represents a hydrogen atom or a $C_1$-$C_6$ alkyl group. Furthermore, $R^9$ and $R^5$ together with the carbon atom on the ring to which $R^4$ and $R^5$ are bonded may form a 3- to 7-membered spiro ring.

Group H: a $C_3$-$C_8$ cycloalkyl group and a phenyl group.

Group I: a halogen atom, an amino group, a $C_1$-$C_6$ alkyl group, a $C_1$-$C_6$ alkoxy group, a $C_1$-$C_6$ alkylamino group, a halogeno $C_1$-$C_6$ alkyl group, and a cyano group.

In $R^3$ and $R^4$, the "$C_1$-$C_6$ alkyl group that may have one or more substituents selected from Group H" means an unsubstituted $C_1$-$C_6$ alkyl group or a $C_1$-$C_6$ alkyl group that may have one or more substituents selected from the above-mentioned Group H.

Here, the $C_3$-$C_8$ cycloalkyl group that can be present as a substituent on a "$C_1$-$C_6$ alkyl group that may have one or more substituents selected from Group H" means a cycloalkyl group having 3 to 8 carbon atoms. Examples of the cycloalkyl group having 3 to 8 carbon atoms include a cyclopropyl group, a cyclopentyl group, and a cyclohexyl group.

In $R^3$ and $R^4$, the "phenyl group that may have one or more substituents selected from Group I" means an

unsubstituted phenyl group or a phenyl group that may have one or more substituents selected from the above-mentioned Group I.

Here, a $C_1$-$C_6$ alkylamino group that can be present as a substituent on a "phenyl group that may have one or more substituents selected from Group I" has the same meaning as that of the $C_1$-$C_6$ alkylamino group explained as a substituent on a 4- to 7-membered nitrogen-containing saturated heterocyclic group in $V_2$, W, and $X_2$.

The halogeno $C_1$-$C_6$ alkyl group that can be present as a substituent on a "phenyl group that may have one or more substituents selected from Group I" means a straight, branched, or cyclic alkyl group having 1 to 6 carbon atoms that contains one or more halogen atoms selected from the group consisting of a chlorine atom, a fluorine atom, a bromine atom, and an iodine atom. When a $C_1$-$C_6$ alkyl group contains two or more halogen atoms as substituents, these halogen atoms may be identical to or different from each other. Examples of the halogeno $C_1$-$C_6$ alkyl group include a fluoromethyl group, a difluoromethyl group, a trifluoromethyl group, a chloromethyl group, a chloroethyl group, and a chlorobutyl group.

In $R^3$ and $R^4$, the "5- or 6-membered aromatic heterocyclic group that may have one or more substituents selected from Group I" means an unsubstituted 5- or 6-membered aromatic heterocyclic group or a 5- or 6-membered aromatic heterocyclic group that may have one or more substituents selected from the above-mentioned Group I. Here, the 5- or 6-membered aromatic heterocyclic group means a group derived from a 5- or 6-membered aromatic heterocyclic compound that contains one or more oxygen atoms, nitrogen atoms, or sulfur atoms as constituent atoms of the ring structure. The 5- or 6-membered aromatic heterocyclic group may be bonded at any position. Examples of the 5- or 6-membered aromatic heterocyclic group include groups derived from pyridine, pyrimidine, pyrrole, furan, thiophene, imidazole, pyrazole, oxazole, thiazole, isothiazole, oxadiazole, and triazole.

Here, the $C_1$-$C_6$ alkylamino group and the halogeno $C_1$-$C_6$ alkyl group that can be present as a substituent on a "5- or 6-membered aromatic heterocyclic group that may have one or more substituents selected from Group I" each has the same meaning as that of the group explained as a substituent on a phenyl group in $R^3$ and $R^4$.

The "3- to 7-membered spiro ring" formed by $R^4$, $R^5$, and the carbon atom on the ring to which $R^4$ and $R^5$ are bonded means a ring formed by an alkylene group having 2 to 6 carbon atoms with the carbon atom at the 6th position of the imidazothiazole ring in the formula (1) to form a spiro form.

In one embodiment of the present invention, $R^1$ is preferably $-V_1-V_2$, -CO-W, or $-CO-X_1-CO-X_2$, more preferably $-CO-X_1-CO-X_2$.

$V_1$ is preferably a methylene group or an ethylidene group ($-CH(CH_3)-$), more preferably a methylene group.

$V_2$ is preferably a hydrogen atom, a hydroxy group or a 4- to 7-membered nitrogen-containing heterocyclic group that may have one or more substituents selected from Group C, more preferably a 4- to 7-membered nitrogen-containing heterocyclic group that may have one or more substituents selected from Group C. The 4- to 7-membered nitrogen-containing heterocyclic group is preferably a piperazinyl group or a pyrrolidinyl group. The substituent selected from Group C is preferably an oxo group, a methyl group, or an ethyl group.

W is preferably a hydroxy group, a $C_1$-$C_6$ alkoxy group, an amino group that may be substituted with one or two 4-to 7-membered saturated or unsaturated heterocyclic groups (the 4- to 7-membered saturated or unsaturated heterocyclic group may have one or more $C_1$-$C_6$ alkyl groups and/or oxo groups), a $C_1$-$C_6$ alkylamino group that may have one or more substituents selected from Group A, an amino $C_1$-$C_6$ alkylamino group that may have one or more substituents selected from Group B, a 4- to 7-membered nitrogen-containing heterocyclic group that may have one or more substituents selected from Group C, or an 8- to 11-membered bicyclic condensed nitrogen-containing heterocyclic group that may have one or more substituents selected from Group D. W is more preferably a $C_l$-$C_6$ alkylamino group that may have one or more substituents selected from Group A, an amino $C_1$-$C_6$ alkylamino group that may have one or more substituents selected from Group B, or a 4- to 7-membered nitrogen-containing heterocyclic group that may have one or more substituents selected from Group C. W is yet more preferably a 4- to 7-membered nitrogen-containing heterocyclic group that may have one or more substituents selected from Group C.

The $C_1$-$C_6$ alkyl moiety of a $C_1$-$C_6$ alkylamino group that may have one or more substituents selected from Group A is preferably a $C_2$-$C_3$ alkyl group, and the $C_1$-$C_6$ alkylamino group is preferably a methylamino group, a dimethylamino group, an ethylmethylamino group, or an isopropylmethylamino group. The substituent selected from Group A is preferably a 4- to 7-membered saturated or unsaturated heterocyclic group that may have one or more $C_1$-$C_6$ alkyl groups and/or oxo groups, more preferably an azetidinyl group, a pyrrolidinyl group, or a cyclobutyl group that may have one or more $C_1$-$C_6$ alkyl groups and/or oxo groups.

The amino $C_1$-$C_6$ alkylamino group of an amino $C_1$-$C_6$ alkylamino group that may have one or more substituents selected from Group B is preferably a (2-aminoethyl)amino group, a (1-aminopropyl-2-yl)amino group, or a (2-aminopropyl)amino group. The substituent selected from Group B is preferably a methyl group or an acetyl group.

The 4- to 7-membered nitrogen-containing heterocyclic group of a 4- to 7-membered nitrogen-containing heterocyclic group that may have one or more substituents selected from Group C is preferably a group derived from piperazine or pyrrolidine. The substituent selected from Group C is preferably an oxo group, a methyl group, an ethyl group, an acetyl group, a dimethylaminomethyl group, a (morpholin-4-yl)methyl group, or a carbamoylmethyl group.

Furthermore, when the 4- to 7-membered nitrogen-containing heterocyclic group of a 4-to 7-membered nitrogen-containing heterocyclic group that may have one or more substituents selected from Group C is a pyrrolidinyl group, a pyrrolidinyl group having a substituent at the 2nd position is preferred.

$X_1$ is preferably an NH-$C_1$-$C_6$ alkylene group (the NH-$C_1$-$C_6$ alkylene group may be substituted on NH with a $C_1$-$C_6$ alkyl group), a divalent 4- to 7-membered saturated or unsaturated nitrogen-containing heterocyclic group that may have one or more substituents selected from Group E, or a divalent 8- to 11-membered bicyclic condensed nitrogen-containing heterocyclic group that may have one or more substituents selected from Group F, more preferably an NH-$C_1$-$C_6$ alkylene group (the NH-$C_1$-$C_6$ alkylene group may be substituted on NH with a $C_1$-$C_6$ alkyl group) or a divalent 4- to 7-membered nitrogen-containing heterocyclic group that may have one or more substituents selected from Group E, yet more preferably a divalent 4- to 7-membered nitrogen-containing heterocyclic group that may have one or more substituents selected from Group E.

The NH-$C_1$-$C_6$ alkylene group is preferably an NH-methylene group. The substituent on nitrogen is preferably a $C_2$-$C_4$ alkyl group, more preferably an ethyl group, a propyl group, an isopropyl group, a butyl group, an isobutyl group, or a sec-butyl group.

The divalent 4- to 7-membered nitrogen-containing heterocyclic group of a divalent 4- to 7-membered nitrogen-containing heterocyclic group that may have one or more substituents selected from Group E is a pyrrolidine-1,2-diyl group. The substituent selected from Group E is preferably a $C_1$-$C_3$ alkyl group, a hydroxy group, or a methoxy group, more preferably an ethyl group or a methyl group.

$X_2$ is preferably a hydroxy group, a $C_1$-$C_6$ alkoxy group, an amino group that may be substituted with one or two 4- to 7-membered saturated or unsaturated heterocyclic groups (the 4- to 7-membered saturated or unsaturated heterocyclic group may have one or more $C_1$-$C_6$ alkyl groups and/or oxo groups), a $C_1$-$C_6$ alkylamino group that may have one or more substituents selected from Group A, an amino $C_1$-$C_6$ alkylamino group that may have one or more substituents selected from Group B, a 4- to 7-membered saturated or unsaturated nitrogen-containing heterocyclic group that may have one or more substituents selected from Group C, or an 8- to 11-membered bicyclic condensed nitrogen-containing heterocyclic group that may have one or more substituents selected from Group D. $X_2$ is more preferably a $C_1$-$C_6$ alkylamino group that may have one or more substituents selected from Group A, an amino $C_1$-$C_6$ alkylamino group that may have one or more substituents selected from Group B, a 4- to 7-membered nitrogen-containing heterocyclic group that may have one or more substituents selected from Group C, or an 8- to 11-membered bicyclic condensed nitrogen-containing heterocyclic group that may have one or more substituents selected from Group D. $X_2$ is yet more preferably a $C_1$-$C_6$ alkylamino group that may have one or more substituents selected from Group A or a 4- to 7-membered nitrogen-containing heterocyclic group that may have one or more substituents selected from Group C.

The $C_1$-$C_6$ alkylamino group of the $C_1$-$C_6$ alkylamino group that may have one or more substituents selected from Group A is preferably a dimethylamino group, an ethylmethylamino group, or a diethylamino group, more preferably a dimethylamino group. The substituent selected from Group A is preferably a hydroxy group, a methoxy group, or a carboxy group.

The amino $C_1$-$C_6$ alkylamino group of the amino $C_1$-$C_6$ alkylamino group that may have one or more substituents selected from Group B is preferably a (2-aminoethyl)amino group, a (1-aminopropyl-2-yl)amino group, or a (2-aminopropyl)amino group. The substituent selected from Group B is preferably a methyl group or an acetyl group.

The 4- to 7-membered nitrogen-containing heterocyclic group of a 4- to 7-membered nitrogen-containing heterocyclic group that may have one or more substituents selected from Group C is preferably a piperazinyl group, a pyrrolidinyl group, or a morpholino group. The substituent selected from Group C is preferably an oxo group, a methyl group, an ethyl group, a cyclopropyl group, an acetyl group, a hydroxy group, a carboxy group, a carbamoyl group, a dimethylamino group, a hydroxymethyl group, a hydroxyethyl group, an amino group, a fluoro group, or a fluoromethyl group.

The 8- to 11-membered bicyclic condensed nitrogen-containing heterocyclic group of an 8- to 11-membered bicyclic condensed nitrogen-containing heterocyclic group that may have one or more substituents selected from Group D is preferably a group derived from octahydropyrrolopyrazine. The substituent selected from Group D is preferably a methyl group or an oxo group.

$R^2$ is preferably a $C_1$-$C_4$ alkyl group, more preferably an isopropyl group or a sec-butyl group.

$R^3$ and $R^4$ each is preferably a phenyl group that may have one or more substituents selected from Group I or a 5- or 6-membered aromatic heterocyclic group that may have one or more substituents selected from Group I, more preferably a phenyl group or a pyridyl group that may have one or more substituents selected from Group I. The substituent selected from Group I is preferably a halogen atom, a halogeno $C_1$-$C_6$ alkyl group, or a $C_1$-$C_6$ alkylamino group, more preferably a chlorine atom, a fluorine atom, a bromine atom, a fluoromethyl group, a trifluoromethyl group, or a methylamino group.

$R^3$ is preferably a phenyl group or a pyridyl group that may have one or more substituents selected from Group I. The substituent selected from Group I is preferably a halogen atom or a halogeno $C_1$-$C_6$ alkyl group, more preferably a chlorine atom, a bromine atom, or a trifluoromethyl group. The phenyl group or the pyridyl group that may have one

or more substituents selected from Group I is preferably a 4-chlorophenyl group, a 3-fluoro-4-chlorophenyl group, a 2-fluoro-4-chlorophenyl group, a 4-trifluoromethylphenyl group, a 4-bromophenyl group, a 6-chloropyridin-3-yl group, or a 5-bromopyridin-2-yl group.

$R^4$ is preferably a phenyl group or a pyridyl group that may have one or more substituents selected from Group I. The substituent selected from Group I is preferably a halogen atom or a methylamino group, and the halogen atom is more preferably a chlorine atom or a fluorine atom. The phenyl group or the pyridyl group that may have one or more substituents selected from Group I is preferably a 4-chlorophenyl group, a 6-chloropyridin-3-yl group, a 4-chloro-3-methylaminophenyl group, a 3-fluoro-4-chlorophenyl group, a 2-fluoro-4-chlorophenyl group, or a 3,4-difluoro-phenyl group.

Furthermore, preferably, $R^3$ and $R^4$ are both a 4-chlorophenyl group, $R^3$ is a 3-fluoro-4-chlorophenyl group and $R^4$ is a 4-chlorophenyl group, or $R^3$ is a 3-fluoro-4-chlorophenyl group and $R^4$ is a 6-chloropyridin-3-yl group.

$R^5$ is preferably a $C_1$-$C_6$ alkyl group, more preferably a $C_1$-$C_3$ alkyl group, yet more preferably a methyl group.

Furthermore, $R^1$ to $R^5$ are preferably combined as follows.

[A] When $R^1$ represents -$V_1$-$V_2$ ($V_1$ and $V_2$ have the same meanings as defined above.)
$R^2$ is preferably a $C_1$-$C_6$ alkyl group that may have one or more substituents selected from Group G, more preferably a $C_1$-$C_4$ alkyl group, yet more preferably an isopropyl group or a sec-butyl group.

$R^3$ is preferably a phenyl group that may have one or more substituents selected from Group I. The substituent selected from Group I is preferably a halogen atom, a chlorine atom, a fluorine atom, or a bromine atom. The phenyl group that may have one or more substituents selected from Group I is preferably a 4-chlorophenyl group, a 3-fluoro-4-chlorophenyl group, a 2-fluoro-4-chlorophenyl group, or a 4-bromophenyl group.

$R^4$ is preferably a phenyl group that may have one or more substituents selected from Group I. The substituent selected from Group I is preferably a halogen atom, more preferably a chlorine atom, a fluorine atom, or a bromine atom. The phenyl group that may have one or more substituents selected from Group I is preferably a 4-chlorophenyl group, a 3-fluoro-4-chlorophenyl group, a 2-fluoro-4-chlorophenyl group, or a 4-bromophenyl group.

$R^5$ is preferably a hydrogen atom or a $C_1$-$C_6$ alkyl group, more preferably a $C_1$-$C_3$ alkyl group, yet more preferably a methyl group.

[B] When $R^1$ is -CO-W (W has the same meaning as defined above.)
$R^2$ is preferably a $C_1$-$C_6$ alkyl group that may have one or more substituents selected from Group G, more preferably a $C_1$-$C_4$ alkyl group, yet more preferably an isopropyl group or a sec-butyl group.

$R^3$ is preferably a phenyl group that may have one or more substituents selected from Group I or a 5- or 6-membered aromatic heterocyclic group that may have one or more substituents selected from Group I. The 5- or 6-membered aromatic heterocyclic group is particularly preferably a pyridyl group. The substituent selected from Group I is preferably a halogen atom or a halogeno $C_1$-$C_6$ alkyl group, more preferably a chlorine atom, a bromine atom, or a trifluoromethyl group. The phenyl group that may have one or more substituents selected from Group I or the 5- or 6-membered aromatic heterocyclic group that may have one or more substituents selected from Group I is preferably a 4-chlorophenyl group, a 3-fluoro-4-chlorophenyl group, a 2-fluoro-4-chlorophenyl group, a 4-trifluoromethylphenyl group, a 4-bromophenyl group, a 6-chloropyridin-3-yl group, or a 5-bromopyridin-2-yl group.

$R^4$ is preferably a phenyl group that may have one or more substituents selected from Group I or a 5- or 6-membered aromatic heterocyclic group that may have one or more substituents selected from Group I. The 5- or 6-membered aromatic heterocyclic group is particularly preferably a pyridyl group. The substituent selected from Group I is preferably a halogen atom or a methylamino group, and the halogen atom is preferably a chlorine atom or a fluorine atom. The phenyl group that may have one or more substituents selected from Group I or the 5- or 6-membered aromatic heterocyclic group that may have one or more substituents selected from Group I is preferably a 4-chlorophenyl group, a 6-chloropyridin-3-yl group, a 4-chloro-3-methylaminophenyl group, a 3-fluoro-4-chlorophenyl group, a 2-fluoro-4-chlorophenyl group, or a 3,4-difluoro-phenyl group.

Furthermore, preferably, $R^3$ and $R^4$ are both a 4-chlorophenyl group, $R^3$ is a 3-fluoro-4-chlorophenyl group and $R^4$ is a 4-chlorophenyl group, or $R^3$ is a 3-fluoro-4-chlorophenyl group and $R^4$ is a 6-chloropyridin-3-yl group.

$R^5$ is preferably a hydrogen atom or a $C_1$-$C_6$ alkyl group, more preferably a $C_1$-$C_3$ alkyl group, yet more preferably a methyl group.

[C] When $R^1$ is -CO-$X_1$-CO-$X_2$ ($X_1$ and $X_2$ have the same meanings as defined above.)
$R^2$ is preferably a $C_1$-$C_6$ alkyl group that may have one or more substituents selected from Group G, more preferably a $C_1$-$C_4$ alkyl group, yet more preferably an isopropyl group or a sec-butyl group.

$R^3$ is preferably a phenyl group that may have one or more substituents selected from Group I or a 5- or 6-membered aromatic heterocyclic group that may have one or more substituents selected from Group I. The 5- or 6-membered aromatic heterocyclic group is particularly preferably a pyridyl group. The substituent selected from Group I is preferably a halogen atom or a halogeno $C_1$-$C_6$ alkyl group, more preferably a chlorine atom, a bromine atom, or a trifluoromethyl group. The phenyl group that may have one or more substituents selected from Group I or the 5- or 6-membered aromatic heterocyclic group that may have one or more substituents selected from Group I is preferably a

4-chlorophenyl group, a 3-fluoro-4-chlorophenyl group, a 2-fluoro-4-chlorophenyl group, a 4-trifluoromethylphenyl group, a 4-bromophenyl group, a 6-chloropyridin-3-yl group, or a 5-bromopyridin-2-yl group.

$R^9$ is preferably a $C_1$-$C_6$ alkyl group that may have one or more substituents selected from Group H, a phenyl group that may have one or more substituents selected from Group I, or a 5- or 6-membered aromatic heterocyclic group that may have one or more substituents selected from Group I, more preferably a phenyl group that may have one or more substituents selected from Group I or a 5- or 6-membered aromatic heterocyclic group that may have one or more substituents selected from Group I, yet more preferably a phenyl group or a pyridyl group that may have one or more substituents selected from Group I. The substituent selected from Group I is preferably a halogen atom or a methylamino group, and the halogen atom is more preferably a chlorine atom or a fluorine atom. The phenyl group or the pyridyl group that may have one or more substituents selected from Group I is preferably a 4-chlorophenyl group, a 6-chloropyridin-3-yl group, a 4-chloro-3-methylaminophenyl group, a 3-fluoro-4-chlorophenyl group, a 2-fluoro-4-chlorophenyl group, or a 3,4-difluoro-phenyl group.

Furthermore, preferably, $R^3$ and $R^4$ are both a 4-chlorophenyl group, $R^3$ is a 3-fluoro-4-chlorophenyl group and $R^4$ is a 4-chlorophenyl group, or $R^3$ is a 3-fluoro-4-chlorophenyl group and $R^4$ is a 6-chloropyridin-3-yl group.

$R^5$ is preferably a hydrogen atom or a $C_1$-$C_6$ alkyl group, more preferably a $C_1$-$C_3$ alkyl group, yet more preferably a methyl group.

Alternatively, preferably, $R^9$ and $R^5$ together with the carbon atom on the ring to which $R^4$ and $R^5$ are bonded form a 3- to 7-membered spiro ring.

The compound represented by the formula (1) of the present invention may have stereoisomers or optical isomers due to asymmetric carbon atoms, and all these stereoisomers, optical isomers, and mixtures thereof are included in the present invention.

In one embodiment of the present invention, a compound having an absolute configuration represented by formula (2) is preferred:

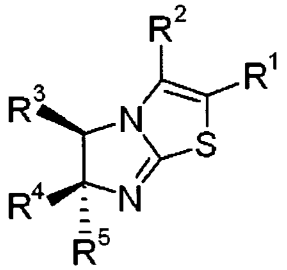

(2)

wherein $R^1$ to $R^5$ have the same meanings as defined above.

The imidazoline derivative of the present invention may remain a free compound or be in the form of a salt. Salts of the compound represented by the general formula (1) of the present invention are not particularly limited so long as they are pharmaceutically acceptable salts, and examples thereof include acid addition salts and salts of a carboxy group.

Examples of acid addition salts include inorganic acid salts such as hydrochlorides, sulfates, nitrates, hydrobromides, hydroiodides, and phosphates, and organic acid salts such as acetates, methanesulfonates, benzenesulfonates, toluenesulfonates, citrates, maleates, fumarates, and lactates.

Furthermore, examples of salts of a carboxy group include alkali metal salts such as lithium salts, sodium salts, and potassium salts, alkaline earth metal salts such as magnesium salts and calcium salts, and inorganic or organic salts such as ammonium salts, triethylamine salts, N-methylglucamine salts, and tris-(hydroxylmethyl)aminomethane salts.

A representative method for producing the compound represented by the formula (1) of the present invention will be explained below. The following compounds (1a) to (1u) are also compounds of the present invention and fall within the scope of the compound (1) of the present invention.

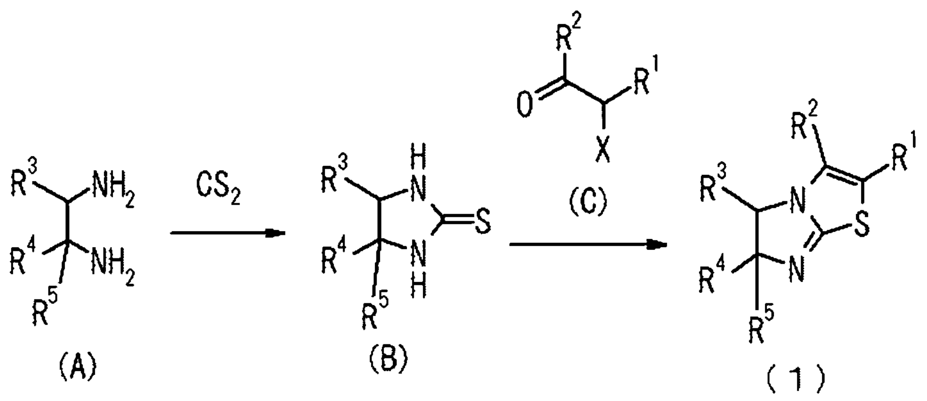

(in each formula, $R^1$ to $R^5$ have the same meanings as defined above, and X represents a halogen atom such a chlorine atom or a bromine atom.)

The imidazothiazole derivative (1) of the present invention can be obtained by reacting a diamine compound (A) with carbon disulfide to obtain an imidazolin-2-thione compound (B) and then reacting the compound (B) with an α-halogenoketone derivative (C).

The solvent in the reaction in the 2 steps illustrated above is not particularly limited and is preferably a solvent that dissolves reaction raw materials and reagents, particularly preferably an alcohol solvent such as ethanol. The reaction temperature is preferably from room temperature to the boiling point of the solvent.

In the above-mentioned production method, a compound (1) in which $R^1$ is an ester group can be synthesized by using an α-halogeno-β-keto ester (D) for the compound B, and an amide derivative (1b can be derived by hydrolyzing the ester group and then reacting the product with an amine such as, for example, morpholine. The amide derivative (1b can be further converted to an aminomethyl derivative (1c) by reduction.

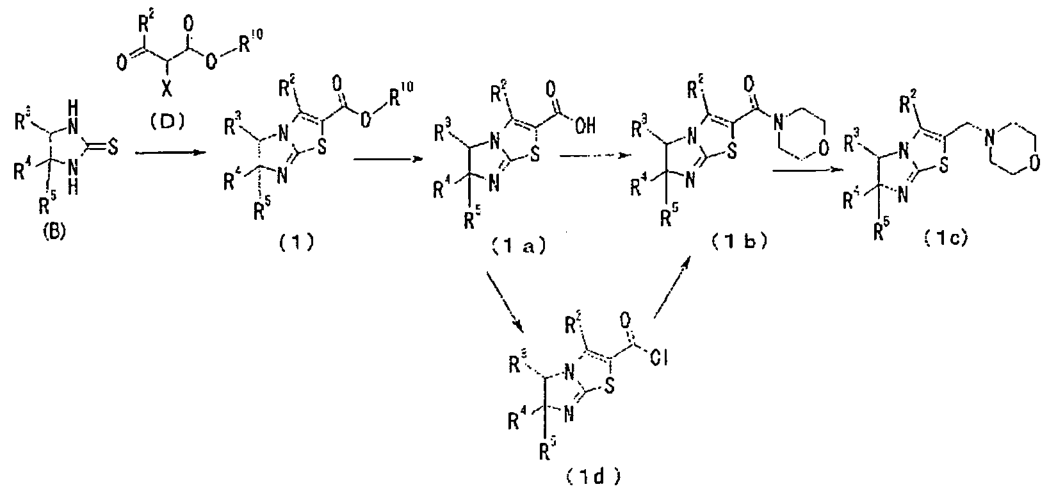

(in each formula, $R^2$ to $R^5$ and X have the same meanings as defined above, and $R^{10}$ represents a $C_1$-$C_6$ alkyl group.)

The above-mentioned compound (1) in which $R^1$ is an ester group can be hydrolyzed to a carboxylic acid (1a) by, for example, treatment with an alkali such as sodium hydroxide or potassium hydroxide. The solvent is preferably a mixed solution of water and an organic solvent, and the organic solvent is preferably a solvent that is miscible in water, such as ethanol or tetrahydrofuran. Furthermore, the reaction temperature is preferably from 0 to 100°C, and it is recommended to adjust the temperature as required.

When other amines are used instead of morpholine used in the above example, various corresponding amide derivatives (1b) can be synthesized by conversion of a carboxylic acid (1a) to an amide (1b). In the reaction from a carboxylic acid (1a) from an amide (1b) an equimolar or excess molar amine can be allowed to act on the carboxylic

acid (1a) in a solvent in the presence of a condensing agent. The reaction temperature can be from -50°C to the boiling point of a solvent used in the reaction, preferably from 0 to 30°C. The reaction time is from 10 min to 72 h, preferably 30 min to approx. 12 h. Examples of the condensing agent include N,N'-dicyclohexylcarbodiimide, 1-ethyl-3-(3-dimethylaminopropyl)carbodiimide, diethyl cyanophosphate, benzotriazolyl oxy-tris[pyrrolidino]-phosphonium hexafluorophosphate, 2-(1H-benzotriazol-1-yl)-1,1,3,3-tetramethyluronium tetrafluoroborate, and so forth. The condensing agent is used in an equal mole or an excess mole to the carboxylic acid (1a), and 1 to 5 moles is preferred. Examples of the solvent used for the reaction include dichloromethane, N,N-dimethylformamide, tetrahydrofuran, and ethyl acetate, and mixtures thereof. Furthermore, the reaction can be performed in the presence of a base such as triethylamine, diisopropylethylamine, N-methylmorpholine, or 4-dimethylaminopyridine, if necessary. Furthermore, an N-hydroxy compound such as 1-hydroxybenzotriazole, N-hydroxysuccinimide, or N-hydroxyphthalimide or a phenol compound such as 4-nitrophenol, 2,4-dinitrophenol, 2,4,5-trichlorophenol, or pentachlorophenol can be added as a reaction accelerator.

Alternatively, various amide derivatives (1b can be synthesized by reacting a carboxylic acid (1a) with an acid halogenating agent in a solvent or in the absence of a solvent for conversion to an acyl halide (1d) and reacting the product with a corresponding amine compound in the presence of a base. In the acid halogenation reaction, the reaction temperature is from -50°C to the boiling point of a solvent used in the reaction, preferably from -20 to 80°C. The reaction time is from 10 min to 24 h, preferably 30 min to approx. 12 h. Examples of the acid halogenating reagent include thionyl chloride, oxalyl chloride, phosphorus trichloride, phosphorus pentachloride, and so forth. As a reaction accelerator, a catalytic amount of N,N-dimethylformamide can be added. It is used in an equal mole or an excess mole thereof to the carboxylic acid (1a), and 1 to 5 moles is preferred. Examples of the solvent used for the reaction include dichloromethane, chloroform, benzene, and toluene or mixed solvents thereof. In the subsequent amidation reaction with an amine compound, the reaction temperature is from -50°C to the boiling point of the solvent used in the reaction, preferably from 0 to 50°C. The reaction time is from 10 min to 72 h, preferably from 30 min to approx. 12 h. Examples of the base used include organic bases such as pyridine, 2,6-lutidine, 4-dimethylaminopyridine, triethylamine, N-methylmorpholine, diisopropylethylamine, and diazabicyclo[5.4.0]undec-7-ene and inorganic bases such as potassium carbonate, sodium carbonate, and sodium bicarbonate. Examples of the solvent used for the reaction include dichloromethane, chloroform, tetrahydrofuran, benzene, and toluene or mixed solvents thereof.

A compound (1c) can be produced by reducing the above-mentioned amide derivative (1b). In this reduction reaction, a reducing agent such as lithium aluminium hydride can be allowed to act on an amide derivative (1b) at approx. 0°C in a solvent such as, for example, tetrahydrofuran.

Furthermore, a hydroxymethyl derivative (1d) can be produced by reducing a compound (1) in which $R^1$ is an ester group as shown below. Furthermore, an aminomethyl derivative 1e) can be produced by reacting a hydroxymethyl derivative (1d) and an amine such as, for example, piperazin-2-one.

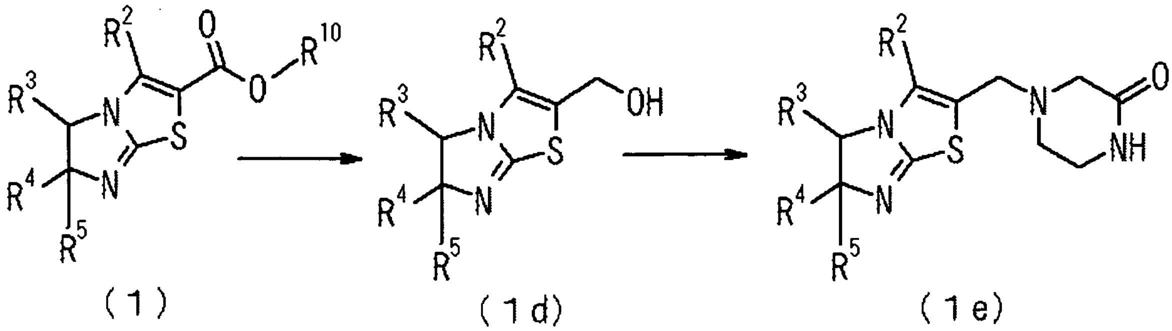

(1) (1d) (1e)

(in each formula, $R^2$ to $R^5$ and $R^{10}$ have the same meanings as defined above.)
In the above-mentioned reduction reaction of the compound (1) in which $R^1$ is an ester group to a hydroxymethyl derivative (1d), a reducing agent such as lithium aluminium hydride can be allowed to act on a compound (1) in which $R^1$ is an ester group in a solvent such as, for example, tetrahydrofuran at approx. 0°C.

An aminomethyl derivative (1e) can be produced by synthesizing a sulfonyloxy derivative by allowing a sulfonyl chloride derivative such as methanesulfonyl chloride to act on the resulting hydroxymethyl derivative (1d) in the presence of a tertiary amine such as triethylamine in a solvent such as methylene chloride and then reacting the product with various amines in the presence of a tertiary amine such as triethylamine. The reaction solvent is preferably methylene chloride, chloroform, or the like. It is sufficient that the reaction temperature is from -10°C to approx. room temperature.

By using other amines instead of piperazin-2-one used in the above example in the conversion of a hydroxymethyl derivative (1d) to an aminomethyl derivative (1e), an aminomethyl derivative (1e) corresponding to each amine can be obtained.

Furthermore, as shown in the following reaction formula, a ketone derivative (1g) can be produced from a carboxylic acid (1a), and a reduced compound (1h) or an aminomethyl derivative (1j) thereof can be further produced.

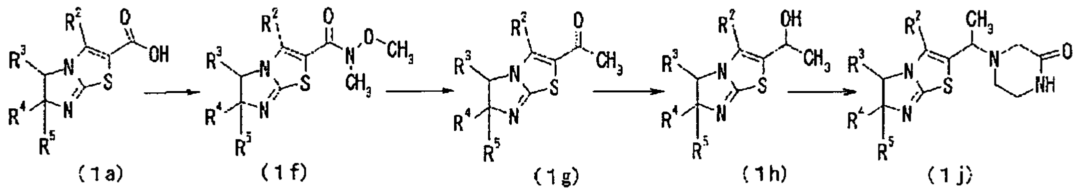

(1a) (1f) (1g) (1h) (1j)

(in each formula, $R^2$ to $R^5$ and $R^{10}$ have the same meanings as defined above.)

In the conversion reaction of a carboxylic acid (1a) to a ketone derivative (1g), a methyl ketone derivative (1g) can be produced by synthesizing N-methyl-N-methoxyamide (so-called Weinreb amide) (1f) and allowing methylmagnesium bromide to act thereon. By using other Grignard reagents instead of methylmagnesium bromide in this reaction, various ketone derivatives corresponding to those Grignard reagents can be produced. The reaction solvent is preferably an ether solvent such as tetrahydrofuran or diethyl ether. The reaction temperature is preferably from room temperature to the boiling points of these solvents.

An alcohol derivative ([1h] in the above-mentioned example) can be produced by reducing a ketone derivative obtained as described above with a reducing agent such as sodium borohydride. The reaction solvent is preferably an alcohol solvent such as ethanol. It is sufficient that the reaction temperature is from room temperature to the boiling point of the solvent. The alcohol derivative can be converted to an aminomethyl derivative ([1j] in the above-mentioned example) by reacting with an amine as described above in the conversion of a hydroxymethyl derivative (1d) to an aminomethyl derivative (1e). The reaction conditions are the same as described above.

Furthermore, an aldehyde derivative (1n) can be synthesized from a compound (1k) in which $R^2$ is a methyl group as shown below, and can be further converted to a hydroxymethyl derivative (1o).

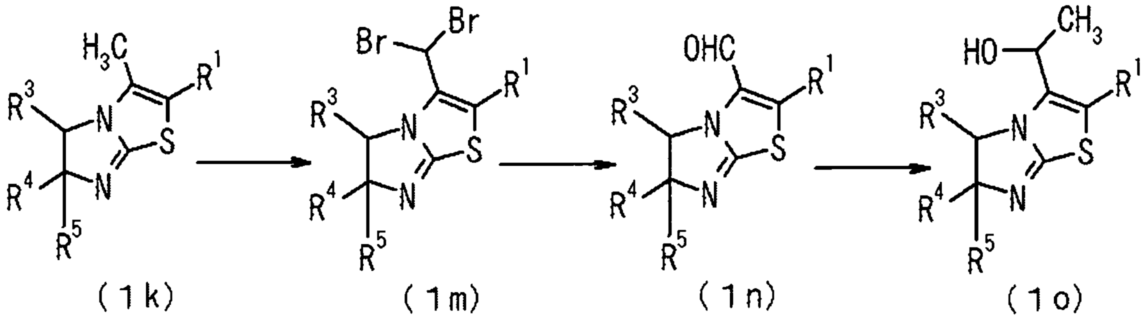

(1k) (1m) (1n) (1o)

(in each formula, $R^1$ and $R^3$ to $R^5$ have the same meanings as defined above.)

A dibromomethyl derivative (1m) can be synthesized by reacting a compound (1k) with N-bromosuccinimide in carbon tetrachloride in the presence of 2,2'-azobis(isobutyronitrile). An aldehyde derivative (1n) can be synthesized by reacting this dibromomethyl derivative (1m) with silver nitrate in a mixed solvent of acetone and water. Subsequently, a hydroxymethyl derivative (1o) can be synthesized by reacting this aldehyde derivative (1n) with methylmagnesium bromide in tetrahydrofuran. By using other Grignard reagents instead of methylmagnesium bromide, various ketone derivatives corresponding to those Grignard reagents can be produced. The reaction conditions are the same as described above.

Furthermore, an amide derivative (1q) can be produced by oxidizing an aldehyde derivative (1n) to obtain a carboxylic acid derivative (1p) and then reacting the product with amines.

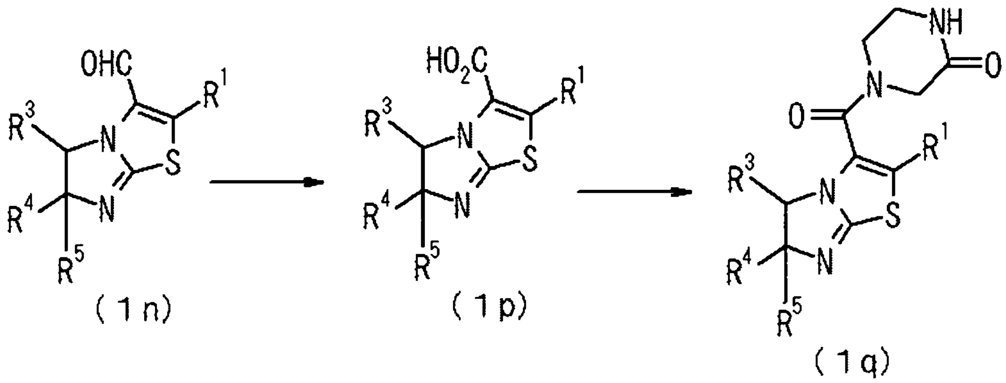

(in each formula, $R^1$ and $R^3$ to $R^5$ have the same meanings as defined above.)
In the oxidation reaction of an aldehyde derivative (1n) to a carboxylic acid derivative (1p), an oxidizing agent commonly used in organic synthetic chemistry can be used. Furthermore, the reaction conditions and condensing agents in the conversion of a carboxylic acid derivative (1p) to an amide derivative (1q) are the same as those for the above-mentioned amide derivative (1b)

Furthermore, the above-mentioned carboxylic acid derivative (1p) can also be produced by the following reaction. Specifically, a carboxylic acid derivative (1p) can be produced by synthesizing an imidazothiazole derivative (1r) using a reagent (E) and hydrolyzing the ester.

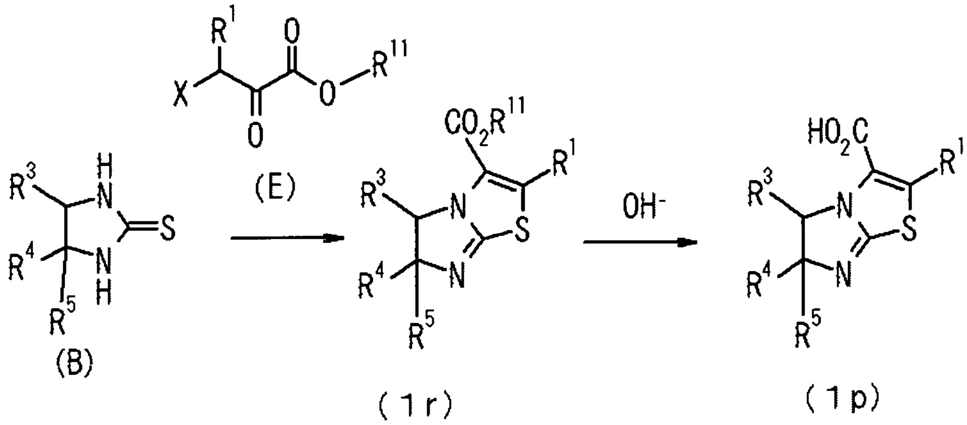


(in each formula, $R^1$ and $R^3$ to $R^5$ have the same meanings as defined as above, and $R^{11}$ represents a $C_1$-$C_6$ alkyl group.)
The reaction conditions and the like for producing an imidazothiazole derivative (1r) are the same as the production conditions for the above-mentioned imidazothiazole derivative (1). Furthermore, the conditions for ester hydrolysis are the same as those for the carboxylic acid derivative (1a).

Furthermore, a compound (1s) in which $R^3$ and $R^4$ in the imidazothiazole derivative (1) are a chlorophenyl group can be converted as shown below.

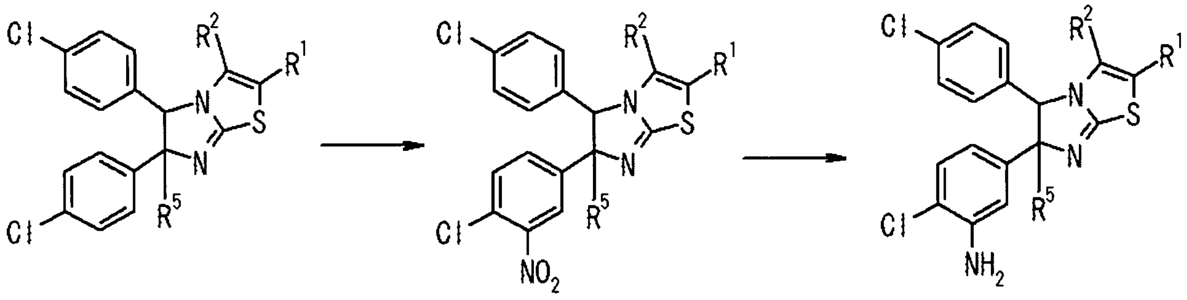

(1s) (1t) (1u)

(in each formula, $R^1$, $R^2$, and $R^5$ have the same meanings as defined above.)
A compound (1t) in which $R^4$ is a 4-chloro-3-nitrophenyl group can be synthesized by reacting a compound (1s) with potassium nitrate in concentrated sulfuric acid. A compound (1u) in which $R^9$ is a 3-amino-4-chlorophenyl group can be synthesized by reacting a compound (1t) with an iron powder in a mixed solution of ethanol and acetic acid.

Furthermore, a compound (1u) can also be converted to various acylamino derivatives by allowing various acylating agents to act thereon.

Furthermore, a compound in which $R^4$ is a 3-(tert-butoxycarbonylamino)-4-chlorophenyl group can be synthesized by reacting a compound (1u) with di-tert-butyl dicarbonate in acetonitrile in the presence of 4-(N,N-dimethylamino) pyridine, and a compound in which $R^4$ is a 3-[(alkyl)(tert-butoxycarbonyl)amino]-4-chlorophenyl group can be synthesized by reacting this compound with lithium bis(trimethylsilyl)amide in tetrahydrofuran and then reacting the product with an alkyl halide. Subsequently, a compound in which $R^4$ is a 3-alkylamino-4-chlorophenyl group can be synthesized by reacting this compound in trifluoroacetic acid.

To stereoselectively synthesize a compound represented by the formula (1), a starting compound (A) or (B) having a required configuration can be used (in each formula, $R^1$ to $R^5$ have the same meanings as defined above, and X represents a halogen atom such as a chlorine atom or a bromine atom).
A compound (A) having a required configuration can be synthesized according to the method described in Synlett, 1998, p.623. Furthermore, the compound can also be synthesized by the following method.

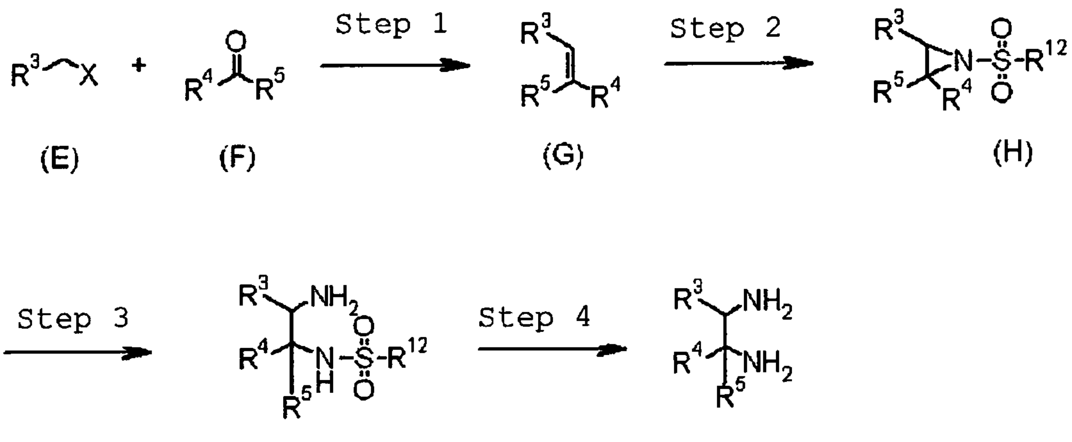


(E) (F) (G) (H)

(I) (A)

A compound (A) can also be synthesized by the following method.

Synthesis of compound (G)

An alcohol derivative can be obtained by reacting a compound (E) and a Grignard reagent produced from

magnesium with a ketone (F). The solvent used in this reaction is not particularly limited, and examples thereof include diethyl ether, tetrahydrofuran, toluene, and so forth or mixed solvents thereof. The reaction temperature is usually in the range from -78 to 100°C or the boiling point of the solvent, preferably in the range from 50 to 80°C.

A compound (G) can be synthesized by dehydrating this alcohol compound in the presence of a strongly acidic compound such as p-toluenesulfonic acid or (±)-camphor-10-sulfonic acid, a Lewis acid such as titanium tetrachloride or a boron trifluoride ether complex, or an acidic catalyst such as sulfuric acid using a device such as a dehydration tube. The solvent used in the reaction is not particularly limited, and examples thereof include tetrahydrofuran, dioxane, benzene, toluene, and so forth or mixed solvents thereof. However, solvents that can remove water by azeotropical removal are preferred. The reaction temperature is usually from -78 to 100°C or the boiling point of the solvent, preferably in the range from 80 to 100°C.

Furthermore, a compound (G) can also be synthesized by the following method. A compound (G) can be obtained by treating a phosphonium salt or a phosphonic acid ester obtained from a reaction of a compound (E) and an organic phosphorous compound such as triphenylphosphine or triethyl phosphite with a base such as alkyl lithium, lithium diisopropylamide, lithium hexamethyldisilazane, sodium hexamethyldisilazane, sodium hydride, or potassium tert-butoxide and then adding a compound (F). The solvent used in this reaction is not particularly limited, and examples thereof include diethyl ether, tetrahydrofuran, toluene, dimethyl sulfoxide, and so forth or mixed solvents thereof. However, dried solvents are preferred. The reaction temperature is usually in the range from -78 to 100°C or the boiling point of a solvent, preferably in the range from -78°C to room temperature.

Synthesis of compound (H)

A compound (H) can be obtained by dissolving a compound (G) in a solvent and reacting the mixture with iodosobenzene acetate and a sulfamate ester in the presence of a metal catalyst that can form a carbenoid complex (particularly preferably rhodium or copper) according to a known method (Tetrahedron Lett., 2005, vol.46, p.4031; J. Am. Chem. Soc., 2002, vol.124, p.136672; and J. Am. Chem. Soc., 2001, vol.123, p.7707). The solvent used in this reaction is not particularly limited, and examples thereof include diethyl ether, tetrahydrofuran, benzene, toluene, acetonitrile, and so forth or mixed solvents thereof. However, dried solvents are preferred. The reaction temperature is usually in the range from -78 to 100°C or the boiling point of a solvent, preferably in the range from -20 to 80°C ($R^{12}$ represents a trichloroethyloxy group, a p-tolyl group, or a p-nitroaryl group).

Synthesis of compound (I)

A compound (I) can be obtained by dissolving a compound (G) in a solvent and treating the mixture with ammonia. The solvent used in this reaction is not particularly limited, and examples thereof include methanol, ethanol, water, tetrahydrofuran, dioxane, and so forth or mixed solvents thereof. However, organic solvents that can be mixed with water in an arbitrary ratio are preferred. The reaction temperature is usually in the range from 0 to 100°C or the boiling point of a solvent, preferably in the range from room temperature to 80°C.

Synthesis of compound (A)

A compound (A) can be produced by dissolving a compound (I) in a solvent and treating the mixture with hydrochloric acid, sulfuric acid, trifluoroacetic acid, or the like for deprotection. The solvent used in this reaction is not particularly limited, and examples thereof include methanol, ethanol, water, tetrahydrofuran, dioxane, and so forth or mixed solvents thereof. The reaction temperature is usually in the range from 0 to 100°C or the boiling point of a solvent, preferably in the range from room temperature to 80°C.

Furthermore, this reaction can also be implemented by using a zinc-copper alloy in a mixed solvent of acetic acid and an alcohol solvent such as methanol. The reaction temperature is usually in the range from -10 to 100°C, preferably to the boiling point of a solvent.

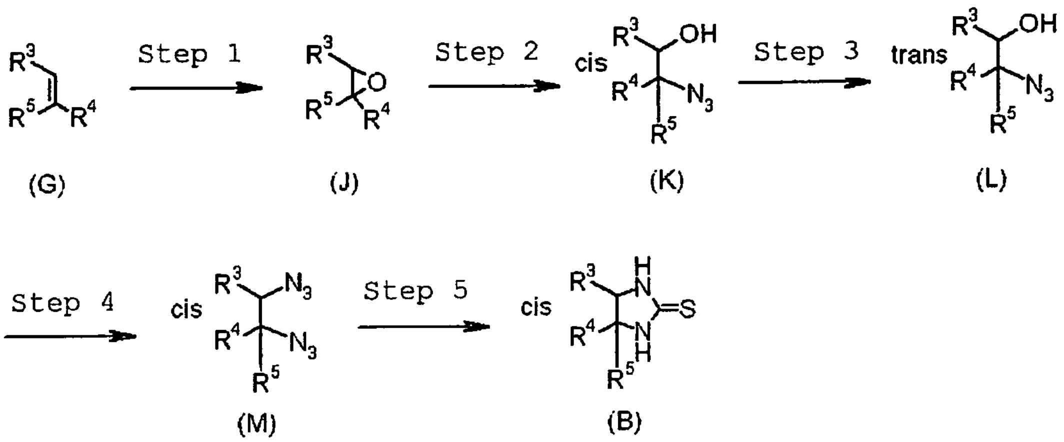


(G) (J) (K) (L) (M) (B)

A compound (B) can also be synthesized by the following method.

Synthesis of compound (J)

A compound (J) can be synthesized by dissolving a compound (G) in a solvent and then treating the mixture with a peroxide such as an organic peroxide such as m-chloroperbenzoic acid or tert-butyl hydroperoxide, aqueous hydrogen peroxide, or oxone (these peroxides and a catalytic amount of a metal such as vanadium, molybdenum, or tungsten may be used). The solvent used in this reaction is not particularly limited, and examples thereof include dichloromethane, chloroform, diethyl ether, toluene, acetone, acetonitrile, and so forth or mixed solvents thereof. The reaction temperature is usually in the range from -78 to 100°C or the boiling point of the solvent, preferably in the range from -20 to 60°C.

Synthesis of compound (K)

A compound (K) in which $R^3$ and $R^4$ are in a *cis*-configuration can be synthesized by dissolving a compound (J) in a solvent and then reacting the mixture with sodium azide or the like in the presence of a weak acidic inorganic compound such as ammonium chloride. In this step, a target compound (K) is obtained as the main product by a substituent of a compound (J) which is a raw material, and a positional isomer thereof may be contained as a byproduct. In this case, the mixture can be used as it is in the following step. The solvent used in this reaction is not particularly limited, and examples thereof include dimethylformamide, dimethyl sulfoxide, ethanol, methanol, tetrahydrofuran, diethyl ether, and so forth or mixed solvents thereof. The reaction temperature is usually in the range from -78 to 150°C or the boiling point of the solvent, preferably in the range from room temperature to 120°C or the boiling point.

Synthesis of compound (L)

An ester derivative in which $R^3$ and $R^4$ are in a trans-configuration can be obtained by dissolving a compound (K) in a solvent and then allowing a Mitsunobu reaction reagent such as, for example, diethyl azodicarboxylate to act on a mixture solution with triphenylphosphine and various organic carboxylic acids such as, acetic acid, and benzoic acid. The solvent used in this reaction is not particularly limited, and examples thereof include dichloromethane, chloroform, diethyl ether, tetrahydrofuran, benzene, toluene, and so forth or mixed solvents thereof. However, dried solvents are preferred. The reaction temperature is usually from -78 to 100°C or the boiling point of the solvent, preferably in the range from -10 to 60°C or the boiling point. Subsequently, a compound (L) can be synthesized by treating the ester derivative synthesized by the above-mentioned procedure with a base such as sodium hydroxide, potassium hydroxide, or lithium hydroxide. The solvent used in this reaction is not particularly limited, and examples thereof include methanol, ethanol, water, tetrahydrofuran, dioxane, and so forth or mixed solvents thereof. However, organic solvents that can be mixed with water in an arbitrary ratio are preferred. The reaction temperature is usually in the range from -10 to 100°C or the boiling point of the solvent.

Synthesis of compound (M)

A compound (M) in which $R^3$ and $R^4$ are in a *cis*-configuration can be obtained by dissolving a compound (L)

in a solvent and then allowing a Mitsunobu reaction reagent such as, for example, diethyl azodicarboxylate, triphenylphosphine and diphenylphosphoryl azide to act on the mixture. The solvent used in this reaction is not particularly limited, and examples thereof include dichloromethane, chloroform, diethyl ether, tetrahydrofuran, benzene, toluene, and so forth or mixed solvents thereof. However, dried solvents are preferred. The reaction temperature is usually in the range from -78 to 100°C or the boiling point of the solvent, preferably in the range from -10 to 60°C or the boiling point. Furthermore, this compound can also be synthesized by the following method. This compound can be obtained by allowing sodium azide to act on a sulfonate ester obtained by treating in a solvent such as, for example, dimethylformamide with methanesulfonyl chloride, p-toluenesulfonyl chloride, or anhydrous trifluoromethanesulfonic acid in a solvent such as, for example, dichloromethane at 0°C or below in the presence of a nitrogen-containing heterocyclic group having a base such as pyridine or a tertiary amine such as triethylamine. The reaction temperature is usually in the range from -78 to 100°C or the boiling point of a solvent, preferably in the range from -10 to 80°C.

Synthesis of compound (B)

A compound (B) can be obtained by dissolving a compound (M) in a solvent and then treating the mixture with a reducing agent such as lithium aluminium hydride, sodium borohydride, or diisobutyl aluminium hydride. The solvent used in this reaction is not particularly limited, and examples thereof include diethyl ether, tetrahydrofuran, toluene, and so forth or mixed solvents thereof. However, dried solvents are preferred. The reaction temperature is usually in the range from -78 to 100°C or the boiling point of the solvent, preferably in the range from -78°C to room temperature.

Furthermore, in another synthesis method, a compound (B) can be obtained by a hydrogenation reaction using a catalyst such as palladium on carbon or platinum on carbon. The solvent used in this reaction is not particularly limited, and examples thereof include methanol, ethanol, tetrahydrofuran, ethyl acetate, and so forth or mixed solvents thereof. The reaction temperature is usually from -78 to 100°C or the boiling point of the solvent, preferably room temperature to 50°C.

Furthermore, in another method, a compound (B) can also be synthesized by treating with triphenylphosphine in a hydrous solvent. The solvent used in this reaction is not particularly limited, and examples thereof include methanol, ethanol, tetrahydrofuran, dioxane, ethyl acetate, toluene, and so forth or mixed solvents thereof. The reaction temperature is usually in the range from -78 to 100°C or the boiling point of the solvent, preferably in the range from room temperature to 50°C.

A compound (B) can be obtained according to the method described above using a compound (M) that can be synthesized as described above. When a compound (K) is a mixture of isomers, a compound (B) having a required three-dimensional structure can be obtained as a single product according to a known separation purification method such as column chromatography using a compound synthesized by the above-mentioned procedures.
Furthermore, a compound (B) can also be synthesized by the following method.

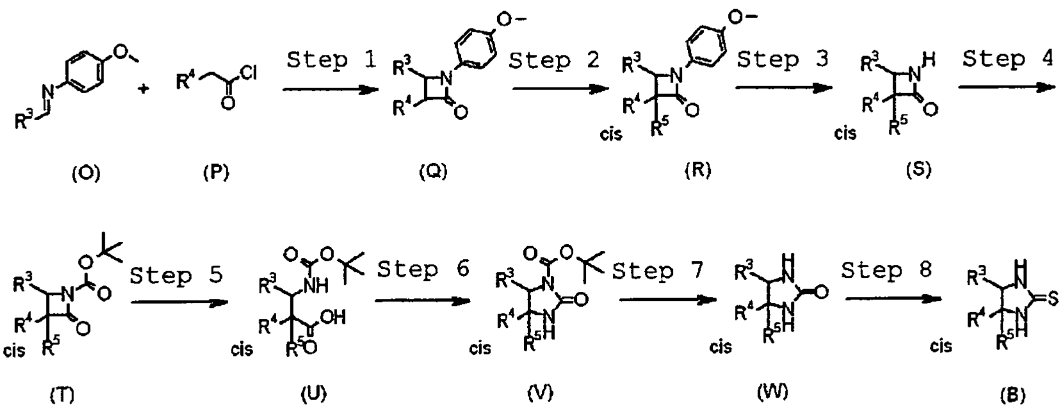

(O) (P) (Q) (R) (S)
(T) (U) (V) (W) (B)

Synthesis of compound (Q)

A compound (Q) can be obtained by reacting a imine (O) which is obtained by mixing various aldehydes and anisidine in a solvent or in the absence of a solvent with addition of a dehydrating agent such as anhydrous sodium sulfate, anhydrous magnesium sulfate, or a molecular sieve, and various acid chlorides (P) in the presence of a base such as the tributylamine with heating (preferably at 70°C). The solvent used in this reaction is not particularly limited,

and examples thereof include carbon tetrachloride, benzene, toluene, and so forth or mixed solvents thereof. However, dried solvents are preferred. The reaction temperature is usually in the range from 50 to 100°C or the boiling point of the solvent.

Synthesis of compound (R)

A compound (R) in which $R^3$ and $R^4$ are in a *cis*-configuration can be obtained by dissolving a compound (Q) in a solvent, treating the mixture with a base such as alkyl lithium, lithium diisopropyl amide, lithium hexamethyldisilazane, or sodium hexamethyldisilazane at - 60°C or below, and adding a methyl halide (for example, methyl iodide). The solvent used in this reaction is not particularly limited, and examples thereof include diethyl ether, tetrahydrofuran, toluene, n-hexane, and so forth or mixed solvents thereof. However, dried solvents are preferred. The reaction temperature is usually in the range from -78 to 100°C or the boiling point of the solvent, preferably in the range from -78°C to room temperature.

Synthesis of compound (S)

A compound (S) can be obtained by dissolving a compound (R) in a solvent and adding an oxidizing agent such as ceric ammonium nitrate or an aqueous solution thereof. Here, examples of the solvent used in this reaction include acetonitrile, tetrahydrofuran, water, acetone, and so forth or mixed solvents thereof. However, organic solvents that can be mixed with water in an arbitrary ratio are preferred. The reaction temperature is usually in the range from -78 to 100°C or range to the boiling point of the solvent, preferably in the range from -20°C to room temperature.

Synthesis of compound (T)

A compound (T) can be obtained by dissolving a compound (S) in a solvent and adding di-tert-butyl dicarbonate in the presence of a base such as 4-dimethylaminopyridine. Here, examples of the solvent used in this reaction include methylene chloride, tetrahydrofuran, acetonitrile, and the like and mixed solvents thereof, but are not particularly limited. However, dried solvents are preferred. The reaction temperature is usually -78 to 100°C or the boiling point of the solvent, preferably in the range from 0 to 60°C.

Synthesis of compound (U)

A compound (U) can be obtained by dissolving a compound (T) in a solvent and treating the mixture with a base such as sodium hydroxide, potassium hydroxide, or lithium hydroxide. Examples of the solvent used in this reaction include ethanol, tetrahydrofuran, water, dioxane, and so forth or mixed solvents thereof. However, organic solvents that can be mixed with water in an arbitrary ratio are preferred. The reaction temperature is usually in the range from -78 to 100°C or the boiling point of the solvent, preferably in the range from 50 to 100°C.

Synthesis of compound (V)

A compound (V) can be obtained by dissolving a compound (U) in a solvent, adding diphenylphosphoryl azide in the presence of a tertiary amine such as triethylamine, and reacting the mixture with tert-butanol. The solvent used in this reaction is not particularly limited, and examples thereof include tert-butanol, tetrahydrofuran, dichloromethane, dioxane, toluene, and so forth or mixed solvents thereof. The reaction temperature is usually in the range from 0 to 100°C or the boiling point of the solvent, preferably in the range from 50 to 100°C.

Synthesis of compound (W)

A compound (W) can be obtained by dissolving a compound (V) in a solvent and adding trifluoroacetic acid, hydrochloric acid, or the like. The solvent used in this reaction is not particularly limited, and examples thereof include dichloromethane, dioxane, ethanol, tetrahydrofuran, and so forth or mixed solvents thereof. The reaction temperature is usually in the range from 0 to 100°C or the boiling point of the solvent, preferably in the range from 0 to 30°C.

Synthesis of compound (B)

A compound (B) can be obtained by dissolving a compound (W) in a solvent and reacting the mixture with diphosphorus pentasulfide, Lawesson's reagent, or the like. The solvent used in this reaction is not particularly limited, and examples thereof include chloroform, tetrahydrofuran, dioxane, benzene, toluene, and forth or mixed solvents

thereof. The reaction temperature is usually in the range from 0 to 100°C or the boiling point of the solvent, preferably in the range from 50 to 100°C.

In one embodiment of the present invention, the compound of the present invention can be used as a p53-Mdm2 binding inhibitor and/or an Mdm2 ubiquitin ligase inhibitor because it inhibits the binding of p53 with Mdm2 and the ubiquitination of p53 by Mdm2.

The condition of the p53-Mdm2 binding can be examined by a method usually used by those skilled in the art to examine binding conditions between proteins (for example, immunological techniques, surface plasmon resonance techniques, etc.). Examples of methods for examining the condition of the Mdm2-p53 binding using an immunological technique include an immuno-sedimentation method and enzyme-linked-immuno-sorbent assay (ELISA). An antibody used in such immunological techniques may be an anti-Mdm2 antibody and/or an anti-p53 antibody that can directly detect Mdm2 and/or p53. When Mdm2 and/or p53 is labeled with a tag (for example, a GST tag or a histidine tag) or the like, an antibody suitable for labeling (for example, an anti-GST antibody or an anti-histidine antibody) can be used. Methods for examining the condition of the Mdm2-p53 binding using an immunological technique are described in, for example, WO2003/51359, WO2003/51360, U.S. Patent Application No. 2004/259867 or 2004/259884, and WO2005/110996. Methods for examining the condition of the Mdm2-p53 binding using a surface plasmon resonance technique are described in, for example, Science, vol.303, p.844-848, 2004.

Ubiquitin ligase activity of Mdm2 against p53 can be examined by an ubiquitin ligase assay usually used by those skilled in the art. The ubiquitin ligase activity can be detected by, for example, comparing ubiquitination of p53 by ubiquitin activation enzyme (E1), ubiquitin binding enzyme (E2), and ubiquitin ligase (E3) (Mdm2) in the presence and absence of a test compound (for example, refer to WO2001/75145 and W02003/76608) .

In another embodiment, the compound of the present invention can be used as an inhibitor of suppression of the p53 transcription activity because it restores functions of p53 as a transcription factor that is suppressed by Mdm2 by inhibiting the binding of Mdm2 to the p53 transcription activation domain. The inhibitor of suppression of the p53 transcription activity can be obtained by, for example, measuring the mRNA level or the protein level of a protein whose transcription is regulated by p53 (for example, $p21^{waf1/Cip1}$) in the presence or absence of a test compound by an mRNA measuring method (for example, Northern blot) or a protein measuring method (for example, Western blot) usually used by those skilled in the art and selecting the test compound as an inhibitor of suppression of the p53 transcription activity when the mRNA level or the protein level is increased in the presence of the test compound as compared with that in the absence of the test compound. Furthermore, the inhibitor of suppression of the p53 transcription activity can also be identified by a reporter assay using the reporter activity of a reporter gene including a p53 responsive element as an indicator.

In another embodiment, the compound of the present invention can be used as a p53 degradation inhibitor because it inhibits ubiquitination of p53 by Mdm2 and thereby prevents the degradation of p53 in proteasomes. The p53 degradation inhibitor can be obtained by, for example, measuring the mRNA level or the protein level of p53 in the presence or absence of a test compound by an mRNA measuring method (for example, Northern blot) or a protein measuring method (for example, Western blot) usually used by those skilled in the art and selecting the test compound as a p53 degradation inhibitor when the mRNA level or the protein level is increased in the presence of the test compound as compared with that in the absence of the test compound.

In another embodiment, the compound of present invention can be used as an anti-tumor agent because it normalizes functions of p53 as a cancer-restraining gene by inhibition of the Mdm2-p53 binding and/or ubiquitination of p53 by Mdm2.

Cellular growth inhibiting activity can be examined by methods for testing growth inhibition usually used by those skilled in the art. The cell growth inhibition activity can be determined by, for example, comparing the levels of cellular growth (for example, tumor cells) in the presence or absence of a test compound as described in the following Test Example 2. The levels of cellular growth can be examined by using, for example, a test system for measuring living cells. Examples of the method for measuring living cells include the [$^3$H]-thymidine uptake test, the BrdU method, the MTT assay, and so forth.

The compound of the present invention can be used for the treatment of tumors or cancers such as, for example, lung cancer, digestive system cancer, ovary cancer, uterine cancer, breast cancer, liver cancer, head/neck region cancer, blood cancer, renal cancer, and testicular tumor.

The pharmaceutical composition of the present invention can contain the compound of the present invention and a pharmaceutically acceptable carrier and can be administered as various injections such as intravenous injection, intramuscular injection, and subcutaneous injection or by various methods such as oral administration or percutaneous administration. The pharmaceutically acceptable carrier means a pharmacologically acceptable material that is involved in transport of the compound of the present invention or a composition containing the compound of present invention (for example, excipient, diluent, additive, solvent, etc.) from a given organ to another organ.

A formulation can be prepared by selecting a suitable formulation form (for example, oral formulation or injection) depending on the administration method and using various usually used methods for preparing a formulation. Examples

of oral formulations include tablet, powder, granule, capsule, pill, lozenge, solution, syrup, elixir, emulsion, oily or aqueous suspension, and so forth. In oral administration, the free compound or a salt form may be used. An aqueous formulation can be prepared by forming an acid adduct with a pharmacologically acceptable acid or by forming an alkali metal salt such as sodium. As an injection, a stabilizer, a preservative, a dissolving aid, and the like can be used in the formulation. After filling a solution that may contain these aids and the like in a vessel, a formulation for use may be prepared as a solid formulation by lyophilization or the like. Furthermore, one dose may be filled in one vessel, or two or more doses may be filled in a vessel.

Examples of solid formulations include tablet, powder, granule, capsule, pill, and lozenge. These solid formulations may contain pharmaceutically acceptable additives together with the compound of the present invention. Examples of additives include filler, extender, binder, disintegrating agent, dissolution promoting agent, skin wetting agent, and lubricant, and these can be selected and mixed as required to prepare a formulation.

Examples of liquid formulations include solution, syrup, elixir, emulsions, and suspension. These liquid formulations may contain pharmaceutically acceptable additives together with the compound of the present invention. Examples of additives include suspending agents and emulsifiers, and these are selected and mixed as required to prepare a formulation.

The compound of the present invention can be used in cancer treatment of mammals, in particular, humans. The dose and the administration interval can be suitably selected depending on the site of a disease, the patient's height, body weight, sex, or medical history, according to a physician's judgment. When the compound of the present invention is administered to a human, the dose range is approx. 0.01 to 500 mg/kg body weight per day, preferably, approx 0.1 to 100 mg/kg body weight. Preferably, the compound of the present invention is administered to a human once a day, or the dose is divided into two to four times, and administration is repeated at an appropriate interval. Furthermore, the daily dose may exceed the above-mentioned dose at a physician's discretion, if necessary.

Hereafter, the present invention will be specifically explained with reference to the following examples. However, the scope of the present invention is not limited to these examples, and they should not be construed in any limitative way. Furthermore, reagents, solvents, and starting materials in the specification can be readily obtained from commercially available supply sources unless otherwise specified.

[Examples]

Example 1

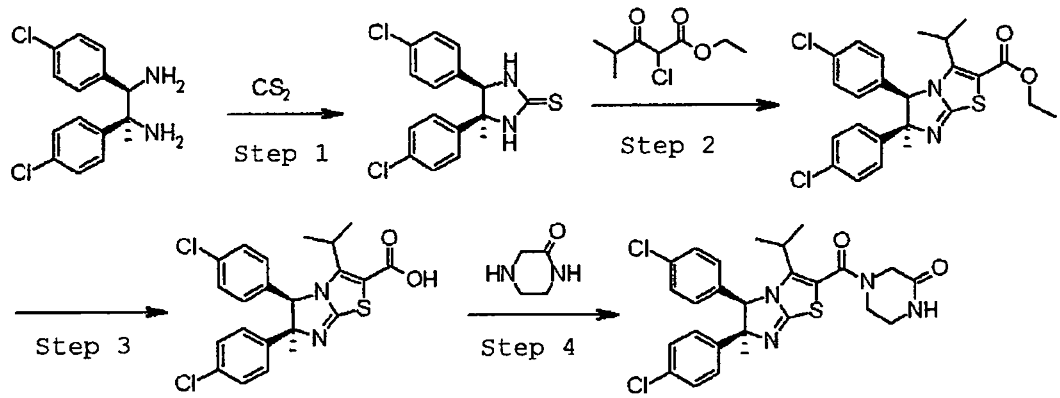


Step 1: (4S,5R)-4,5-Bis(4-chlorophenyl)-4-methylimidazolidine-2-thione

Carbon disulfide (2.04 ml, 33.9 mmol) was added to an ethanol (20 ml) solution of (1R,2S)-1,2-bis(4-chlorophenyl)propane-1,2-diamine (2.00 g, 6.77 mmol) and the resulting mixture was heated to reflux for 4 hours. The solvent was evaporated under reduced pressure and isopropanol and diisopropyl ether were added to the residue. The resulting precipitate was collected by filtration to give the title compound (1.91 g, 84%) as a colorless solid.
$^1$H-NMR (DMSO-$d_6$) δ: 1.71 (3H, s), 4.94 (1H, s), 6.89 (2H, dt, J=8.9, 2.1 Hz), 6.97 (2H, dt, J=8.9, 2.1 Hz), 7.17-7.12 (4H, m), 8.74 (1H, s), 8.92 (1H, s).

Step 2: Ethyl (5R,6S)-5,6-Bis(4-chlorophenyl)-3-isopropyl-6-methyl-5,6-dihydroimidazo[2,1-b][1,3]thiazole-2-carboxylate

Ethyl 2-chloro-4-methyl-3-oxopentanoate (1.42 g, 7.36 mmol) was added to an ethanol (20 ml) solution of the compound (1.91 g, 5.66 mmol) obtained in Step 1 above and the resulting mixture was heated to reflux for 18 hours. The solvent was evaporated under reduced pressure and isopropanol and diisopropyl ether were added to the residue. The resulting precipitate was collected by filtration to give the title compound (2.11 g, 78%) as a colorless solid.
$^1$H-NMR ($CDCl_3$) δ: 1.03 (3H, d, J=7.0 Hz), 1.03 (3H, d, J=7.0 Hz), 1.37 (3H, t, J=7.1 Hz), 2.10 (3H, s), 3.28-3.47 (1H, m), 4.33 (2H, q, J=7.1 Hz), 5.57 (1H, s), 6.45-7.18 (8H, m).

Step 3: (5R,6S)-5,6-Bis(4-chlorophenyl)-3-isopropyl-6-methyl-5,6-dihydroimidazo[2,1-b][1,3]thiazole-2-carboxylic acid

1 N aqueous sodium hydroxide solution (20 ml, 20 mmol) was added to an ethanol (20 ml) solution of the compound (2.11 g, 4.44 mmol) obtained in Step 2 above and the resulting mixture was heated to reflux for 4 hours. 1 N aqueous hydrochloric acid solution (22 ml) was added to the reaction mixture, the resulting mixture was diluted with water and stirred, and then the deposited insoluble matter was collected by filtration to give the title compound (1.54 g, 78%) as a colorless solid.
$^1$H-NMR (DMSO-$d_6$) δ: 0.83 (3H, d, J=7.1 Hz), 0.93 (3H, d, J=7.1 Hz), 1.78 (3H, s), 2.99-3.67 (1H, m), 5.79 (1H, s), 6.44-7.43 (8H, m).
MS (ESI) m/z: 447, 449.

Step 4: 4-{[(5R,6S)-5,6-Bis(4-chlorophenyl)-3-isopropyl-6-methyl-5,6-dihydroimidazo[2,1-b][1,3]thiazol-2-yl]carbonyl} piperazin-2-one

A mixture of the compound (0.200 g, 0.447 mmol) obtained in Step 3 above, piperazin-2-one (53.7 mg, 0.536 mmol), 1-(3-dimethylaminopropyl)-3-ethylcarbodiimide hydrochloride (0.129 g, 0.671 mmol) and 1-hydroxybenzotriazole (72.5 mg, 0.536 mmol) in N,N-dimethylformamide (10 ml) was stirred at room temperature for 18 hours. The reaction mixture was diluted with ethyl acetate, washed with water and brine, and dried over anhydrous sodium sulfate. The solvent was evaporated under reduced pressure, the residue was separated and purified by silica gel column chromatography (ethyl acetate → ethyl acetate:ethanol = 4:1), and the solvent was evaporated from the desired fraction under reduced pressure. Diethyl ether and hexane were added to the residue and the resulting precipitate was collected by filtration to give the title compound (0.150 g, 63%) as a colorless solid.
$^1$H-NMR ($CDCl_3$) δ: 0.89 (3H, d, J=7.1 Hz), 1.00 (3H, d, J=7.1 Hz), 1.83 (3H, s), 2.41-2.62 (1H, m), 3.44-3.51 (2H, m), 3.79-3.87 (2H, m), 4.28 (2H, s), 4.97 (1H, s), 6.15 (1H, brs), 6.67-6.77 (2H, m), 7.00-7.11 (6H, m).
MS (ESI) m/z: 529, 531.

Example 2

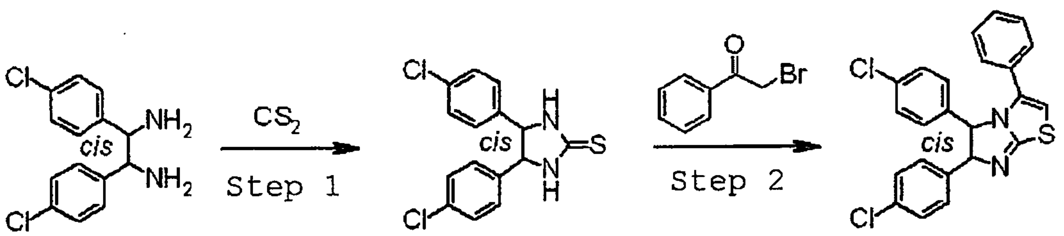


Step 1: 4,5-cis-4,5-Bis(4-chlorophenyl)imidazolidine-2-thione

meso-1,2-Bis(4-chlorophenyl)ethane-1,2-diamine (1.16 g, 4.13 mmol) was dissolved in ethanol (20 ml) and followed by the dropwise addition of carbon disulfide (373 μl, 8.11 mmol) and the resulting mixture was heated to reflux for 12 hours. After cooling, the solvent was evaporated under reduced pressure, and diethyl ether was added to the residue for trituration and the powder was collected by filtration thus to give the title compound (1.08 g, 81%) as a colorless solid.
$^1$H-NMR ($CDCl_3$) δ: 5.33 (2H, s), 6.25 (2H, brs), 6.86 (4H, d, J=8.5 Hz), 7.12 (4H, d, J=8.5 Hz).

Step 2: (5R*,6S*)-5,6-Bis(4-chlorophenyl)-3-phenyl-5,6-dihydroimidazo[2,1-b][1,3]thiazole hydrobromide

The compound (150 mg, 0.46 mmol) obtained in Step 1 above was virtually dissolved in ethanol (15 ml) followed by the addition of 2-bromoacetophenone (101.6 mg, 0.51 mmol) and the resulting mixture was heated to reflux for 14 hours. After cooling, deposited matter was collected by filtration and washed with diethyl ether to give the title compound (16.6 mg, 84%) as a colorless solid racemic mixture.
$^1$H-NMR (DMSO-$d_6$) δ: 6.38 (1H, d, J=10.2 Hz), 6.69 (2H, d, J=8.8 Hz), 6.75 (1H, d, J=10.2 Hz), 7.03 (2H, d, J=8.5 Hz), 7.16 (2H, d, J=8.5 Hz), 7.23 (1H, s), 7.26 (2H, d, J=8.5 Hz), 7.31-7.36 (3H, m), 7.44-7.50 (2H, m), 10.76 (1H, brs).
MS (FAB) m/z: 423, 425.

Example 3

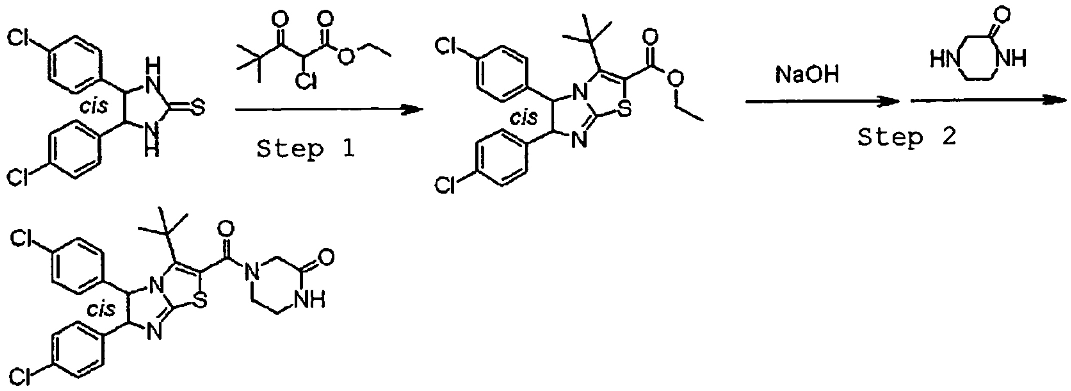


Step 1: Ethyl (5R*,6S*)-3-tert-Butyl-5,6-bis(4-chlorophenyl)-5,6-dihydroimidazo[2,1-b][1,3]thiazole-2-carboxylate

The same reaction was performed as in Step 2 of Example 1 using the compound obtained in Step 1 of Example 2 instead of the compound obtained in Step 1 of Example 1, and ethyl 2-chloro-4,4-dimethyl-3-oxopentanoate instead of ethyl 2-chloro-4-methyl-3-oxopentanoate. Purification was performed using silica gel thin layer chromatography (chloroform:methanol = 30:1 and then hexane:ethyl acetate = 3:1) to give the title compound as a colorless solid racemic mixture.
$^1$H-NMR ($CDCl_3$) δ: 1.27 (9H, s), 1.36 (3H, t, J=7.3 Hz), 4.27 (2H, q, J=7.3 Hz), 5.68 (1H, d, J=8.7 Hz), 5.75 (1H, d, J=8.7 Hz), 6.52 (2H, brd, J=7.6 Hz), 6.92 (2H, d, J=8.3 Hz), 7.04-7.12 (4H, m).
MS (FAB) m/z: 475, 477.

Step 2: 4-{[(5R*,6S*)-3-tert-Butyl-5,6-bis(4-chlorophenyl)-5,6-dihydroimidazo[2,1-b][1,3]thiazol-2-yl]carbonyl}piperazin-2-one

The compound obtained in Step 1 above was reacted in the same way as in Step 3 of Example 1 to give the corresponding carboxylic acid. This was reacted with piperazin-2-one in the same way as in Step 4 of Example 1, and after purified by silica gel thin layer chromatography (chloroform: methanol = 10:1), lyophilized with dioxane to give the title compound as a colorless solid racemic mixture.
$^1$H-NMR ($CDCl_3$) δ: 1.07 (9H, s), 3.40-3.57 (2H, m), 3.96-4.11 (1H, m), 4.20 (1H, d, J=18.2 Hz), 4.36 (1H, d, J=18.2 Hz), 5.46 (1H, d, J=8.5 Hz), 5.80 (1H, d, J=8.5 Hz), 6.09 (1H, s), 6.58 (1H, brs), 6.94 (2H, d, J=8.3 Hz), 7.02-7.14 (4H, m).
MS (EI) m/z: 528.

Example 4

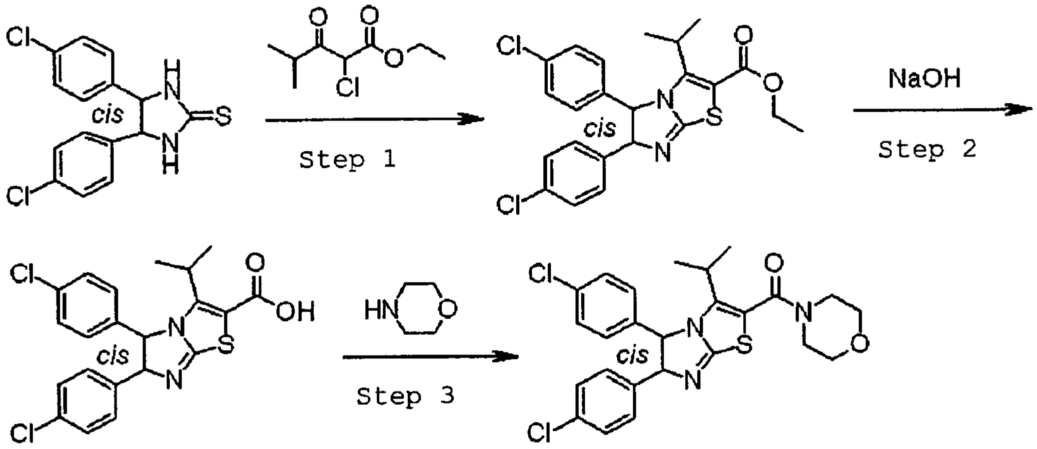


Step 1: Ethyl (5R*,6S*)-5,6-Bis(4-chlorophenyl)-3-isopropyl-5,6-dihydroimidazo[2,1-b][1,3]thiazole-2-carboxylate

Ethyl 2-chloro-4-methyl-3-oxopentanoate (11.90 g, 61.8 mmol) was dissolved in ethanol (500 ml) followed by the addition of the compound (15.36 g, 47.5 mmol) obtained in Step 1 of Example 2 and the resulting mixture was heated to reflux for 15 hours. After cooling, the solvent was evaporated under reduced pressure followed by the addition of saturated aqueous sodium bicarbonate solution and extracted with chloroform. After washing with brine and drying over anhydrous sodium sulfate, the solvent was evaporated under reduced pressure. The residue was purified by silica gel column chromatography (chloroform → chloroform:methanol = 100:1 → 50:1) to give the title compound (19.0 g, 87%) as a pale yellow solid racemic mixture.
$^1$H-NMR ($CDCl_3$) δ: 0.89 (3H, d, J=7.2 Hz), 1.05 (3H, d, J=7.2 Hz), 1.34 (3H, t, J=7.2 Hz), 3.33-3.43 (1H, m), 4.26 (2H, q, J=7.2 Hz), 5.44 (1H, d, J=9.3 Hz), 5.89 (1H, d, J=9.3 Hz), 6.65 (2H, brd, J=7.8 Hz), 6.96 (2H, d, J=8.3 Hz), 7.04-7.11 (4H, m).

Step 2: (5R*,6S*)-5,6-Bis(4-chlorophenyl)-3-isopropyl-5,6-dihydroimidazo[2,1-b][1,3]thiazole-2-carboxylic acid

The compound obtained in Step 1 above was reacted in the same way as in Step 3 of Example 1 to give the title compound as a racemic mixture.
$^1$H-NMR ($CDCl_3$-$CD_3OD$ (10:1) ) δ: 1.00 (3H, d, J=7.1 Hz), 1.10 (3H, d, J=7.1 Hz), 3.37-3.47 (1H, m), 6.01 (1H, d, J=9.5 Hz), 6.17 (1H, d, J=9.5 Hz), 6.64-6.71 (2H, m), 6.97-7.04 (2H, m), 7.09-7.19 (4H, m).

Step 3: (5R*,6S*)-5,6-Bis(4-chlorophenyl)-3-isopropyl-2-(morpholin-4-ylcarbonyl)-5,6-dihydroimidazo[2,1-b][1,3]thiazole

The compound obtained in Step 2 above was reacted in the same way as in Step 4 of Example 1 using morpholine instead of piperazin-2-one to give the title compound as a racemic mixture.
$^1$H-NMR ($CDCl_3$) δ: 0.97 (3H, d, J=7.1 Hz), 1.01 (3H, d, J=7.1 Hz), 2.40-2.60 (1H, m), 3.62-3.67 (4H, m), 3.69-3.74 (4H, m), 5.34 (1H, d, J=9.3 Hz), 5.89 (1H, d, J=9.3 Hz), 6.65 (2H, d, J=8.3 Hz), 6.96 (2H, d, J=8.3 Hz), 7.11-7.04 (4H, m).
MS (ESI) m/z: 502, 504.

Example 5

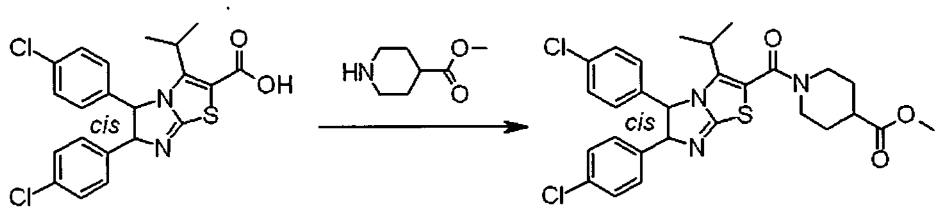

Methyl 1-{[(5R*,6S*)-5,6-Bis(4-chlorophenyl)-3-isopropyl-5,6-dihydroimidazo[2,1-b][1,3]thiazol-2-yl]carbonyl}piperidine-4-carboxylate

The compound obtained in Step 2 of Example 4 was reacted in the same way as in Step 4 of Example 1 using methyl piperidine-4-carboxylate instead of piperazin-2-one to give the title compound as a racemic mixture.
$^1$H-NMR ($CDCl_3$) δ: 0.96-0.99 (6H, m), 1.72-1.74 (2H, m), 2.00-2.03 (2H, m), 2.51-2.60 (2H, m), 3.10-3.12 (2H, m), 3.70-3.72 (3H, m), 4.15-4.19 (2H, m), 5.32 (1H, d, J=9.3 Hz), 5.89 (1H, d, J=9.3 Hz), 6.65 (2H, d, J=8.5 Hz), 6.96 (2H, d, J=8.5 Hz), 7.06 (2H, d, J=8.5 Hz), 7.08 (2H, d, J=8.5 Hz).
MS (FAB) m/z: 558.

Example 6

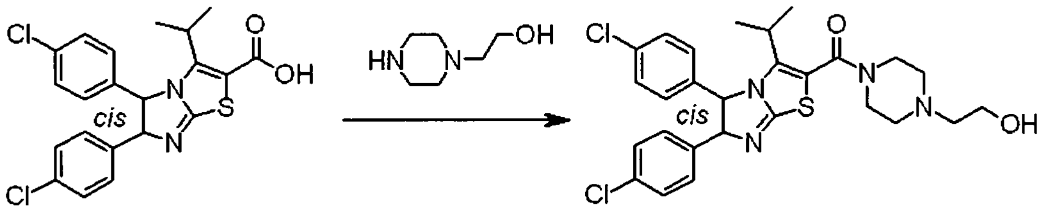

2-(4-{[(5R*,6S*)-5,6-Bis(4-chlorophenyl)-3-isopropyl-5,6-dihydroimidazo[2,1-b][1,3]thiazol-2-yl]carbonyl}piperazin-1-yl) ethanol

The compound obtained in Step 2 of Example 4 was reacted in the same way as in Step 4 of Example 1 using 2-(piperazin-1-yl)ethanol instead of piperazin-2-one to give the title compound as a racemic mixture.
$^1$H-NMR ($CDCl_3$) δ: 0.97 (3H, d, J=7.1 Hz), 1.00 (3H, d, J=7.1 Hz), 2.47-2.65 (8H, m), 3.59-3.72 (6H, m), 5.34 (1H, d, J=9.5 Hz), 5.89 (1H, d, J=9.5 Hz), 6.65 (2H, d, J=8.3 Hz), 6.96 (2H, d, J=8.3 Hz), 7.04-7.11 (4H, m).
MS (ESI) m/z: 545, 547.

Example 7

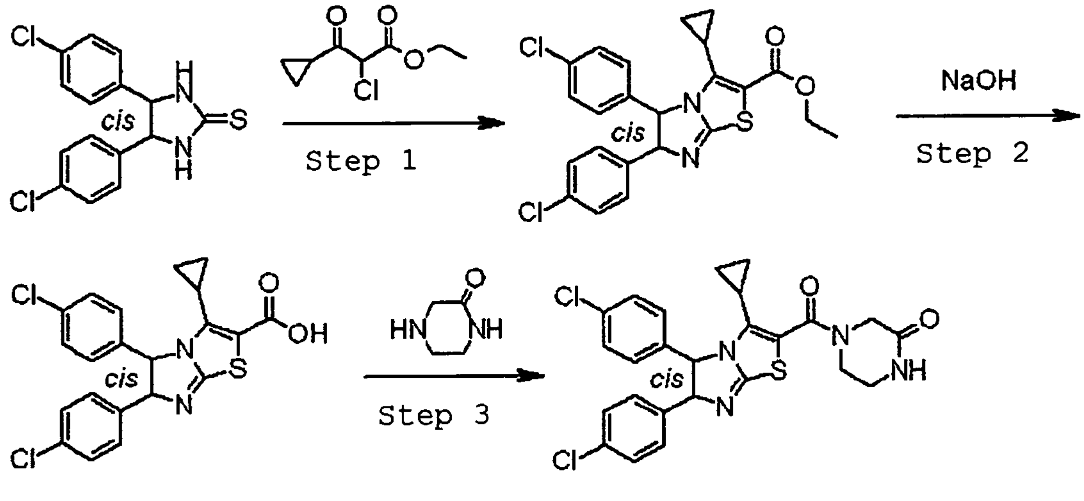


Step 1: Ethyl (5R*,6S*)-5,6-Bis(4-chlorophenyl)-3-cyclopropyl-5,6-dihydroimidazo[2,1-b][1,3]thiazole-2-carboxylate

The compound obtained in Step 1 of Example 2 was reacted in the same way as in Step 1 of Example 4 using ethyl 2-chloro-3-cyclopropyl-3-oxopropanoate instead of ethyl 2-chloro-4-methyl-3-oxopentanoate to give the title compound as a racemic mixture.
$^1$H-NMR ($CDCl_3$) δ: 0.63-0.67 (1H, m), 0.69-0.74 (1H, m), 0.80-0.86 (1H, m), 0.93-1.02 (1H, m), 1.26-1.37 (1H, m), 1.33 (3H, t, J=7.1 Hz), 4.21-4.30 (2H, m), 5.44 (1H, d, J=9.8 Hz), 5.87 (1H, d, J=9.5 Hz), 6.68 (2H, d, J=8.3 Hz), 6.96 (2H, d, J=8.3 Hz), 7.06 (2H, d, J=8.5 Hz), 7.07 (2H, d, J=8.5 Hz).
MS (ESI) m/z: 459.

Step 2: (5R*,6S*)-5,6-Bis(4-chlorophenyl)-3-cyclopropyl-5,6-dihydroimidazo[2,1-b][1,3]thiazole-2-carboxylic acid

The compound obtained in Step 1 above was reacted in the same way as in Step 3 of Example 1 to give the title compound as a racemic mixture.
$^1$H-NMR (DMSO-$d_6$) δ: 0.52-0.59 (1H, m), 0.75-0.80 (1H, m), 0.82-0.90 (2H, m), 1.55-1.63 (1H, m), 5.87 (2H, s), 6.79 (2H, d, J=8.3 Hz), 7.06 (2H, d, J=8.5 Hz), 7.13 (2H, d, J=8.3 Hz), 7.18 (2H, d, J=8.3 Hz).
MS (ESI) m/z: 431.

Step 3: 4-{[(5R*,6S*)-5,6-Bis(4-chlorophenyl)-3-cyclopropyl-5,6-dihydroimidazo[2,1-b][1,3]thiazol-2-yl]carbonyl}piperazin-2-one

The compound obtained in Step 2 above was reacted with piperazin-2-one in the same way as in Step 4 of Example 1 to give the title compound as a racemic mixture. $^1$H-NMR ($CDCl_3$) δ: 0.35-0.39 (1H, m), 0.58-0.63 (3H, m), 0.95-1.01 (1H, m), 3.43-3.51 (2H, m), 3.69-3.74 (1H, m), 3.98-4.03 (1H, m), 4.25 (2H, s), 5.45 (1H, d, J=10.0 Hz), 5.90 (1H, d, J=10.0 Hz), 6.10 (1H, brs), 6.76 (2H, d, J=8.3 Hz), 6.95 (2H, d, J=8.3 Hz), 7.07 (2H, d, J=8.3 Hz), 7.09 (2H, d, J=8.1 Hz).
MS (ESI) m/z: 513.

Example 8

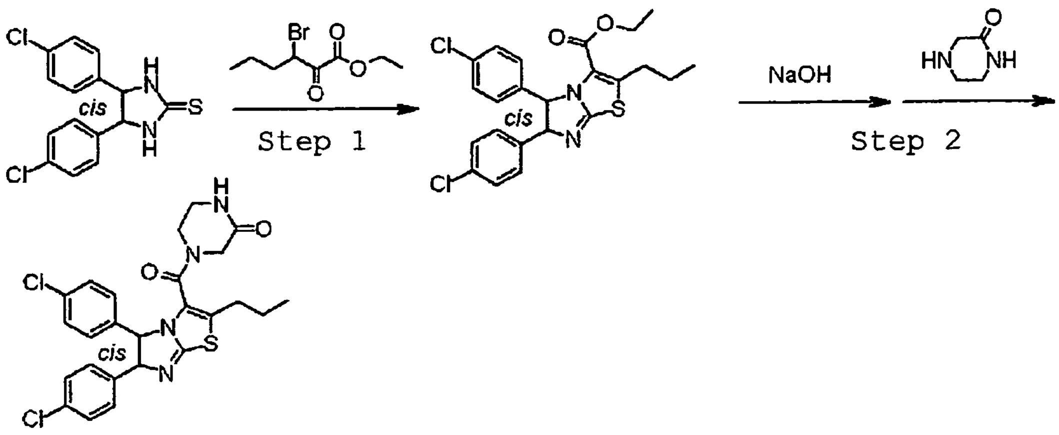


Step 1: Ethyl (5R*,6S*)-5,6-Bis(4-chlorophenyl)-2-propyl-5,6-dihydroimidazo[2,1-b][1,3]thiazole-3-carboxylate

The same reaction was performed as in Step 1 of Example 4 using ethyl 3-bromo-2-oxohexanoate instead of ethyl 2-chloro-4-methyl-3-oxopentanoate. Purification was performed using silica gel column chromatography (chloroform:methanol = 50:1 → 30:1 and then hexane:ethyl acetate = 3:1 → 2:1 → 1:1 → 1:2) to give the title compound as a racemic pale orange oily substance.

$^1$H-NMR ($CDCl_3$) δ: 0.99 (3H, t, J=7.4 Hz), 1.14 (3H, t, J=7.2 Hz), 1.60-1.71 (2H, m), 2.87 (2H, t, J=7.6 Hz), 4.05 (2H, q, J=7.1 Hz), 5.67 (1H, d, J=9.3 Hz), 5.81 (1H, d, J=9.3 Hz), 6.60 (2H, d, J=8.5 Hz), 6.97-7.07 (6H, m). Step 2: 4-{[(5R*, 6S*)-5,6-Bis(4-chlorophenyl)-2-propyl-5,6-dihydroimidazo[2,1-b][1,3]thiazole-3-yl]carbonyl}piperazin-2-one

The compound obtained in Step 1 above was reacted in the same way as in Step 3 of Example 1 to give the corresponding carboxylic acid. This was reacted with piperazin-2-one in the same way as in Step 4 of Example 1 to give the title compound as a racemic colorless solid. mixture.

$^1$H-NMR ($CDCl_3$) δ: 0. 96 (3H, t, J=7.3 Hz), 1.49-1.77 (3H, m), 2.20-2.55 (3H, m), 2.85-3.16 (2H, m), 3.79 (1H, d, J=17.5 Hz), 3.88 (1H, d, J=17.5 Hz), 4.22 (1H, d, J=12.2 Hz), 5.49 (1H, d, J=10.2 Hz), 5.94 (1H, d, J=10.2 Hz), 6.74 (2H, d, J=8.1 Hz), 6.99-7.09 (6H, m).

MS (FAB) m/z: 515, 517.

Example 9

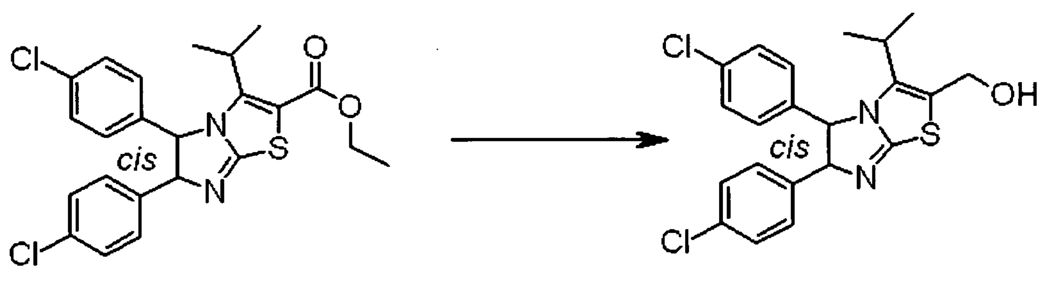

[(5R*,6S*)-5,6-Bis(4-chlorophenyl)-3-isopropyl-5,6-dihydroimidazo[2,1-b][1,3]thiazol-2-yl]methanol

The compound (0.39 g, 0.85 mmol) obtained in Step 1 of Example 4 was dissolved in tetrahydrofuran (10 ml) followed by the gradual addition of lithium aluminium hydride (64 mg, 1.69 mmol) under ice cooling and stirred at the same temperature for one hour. Water (64 μl), a 15% aqueous sodium hydroxide solution (64 μl) and water (192 μl) were sequentially added further followed by the addition of anhydrous sodium sulfate and stirred at room temperature. After insoluble matter was filtered off, the solvent was evaporated under reduced pressure. The residue was purified by

silica gel thin layer chromatography (chloroform:methanol = 10:1) to give the title compound (193 mg, 54%) as a racemic colorless solid mixture.
$^1$H-NMR ($CDCl_3$) δ: 0.92 (3H, d, J=7.1 Hz), 0.93 (3H, d, J=7.1 Hz), 2.47-2.58 (1H, m), 3.65 (1H, brs), 4.42 (1H, d, J=13.7 Hz), 4.47 (1H, d, J=13.7 Hz), 5.31 (1H, d, J=9.3 Hz), 5.84 (1H, d, J=9.3 Hz), 6.61 (2H, d, J=7.8 Hz), 6.97 (2H, d, J=8.3 Hz), 7.03-7.09 (4H, m).
MS (FAB) m/z: 419, 421.

Example 10

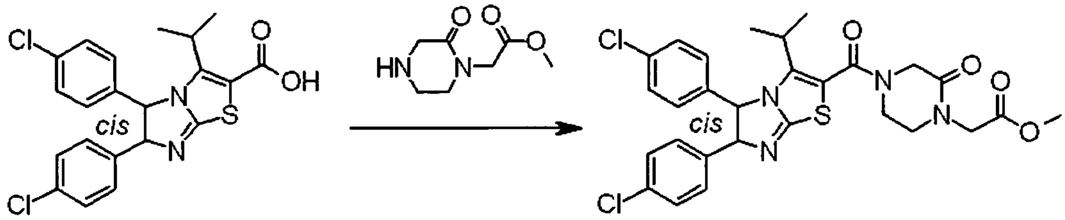

Ethyl (4-{[(5R*,6S*)-5,6-Bis(4-chlorophenyl)-3-isopropyl-5,6-dihydroimidazo[2,1-b][1,3]thiazol-2-yl]carbonyl}-2-oxopiperazin-1-yl)acetate

The compound obtained in Step 2 of Example 4 was reacted in the same way as in Step 4 of Example 1 using methyl (2-oxopiperazin-1-yl)acetate instead of piperazin-2-one to give the title compound as a racemic mixture.
$^1$H-NMR ($CDCl_3$) δ: 0.96 (3H, d, J=7.1 Hz), 1.00 (3H, d, J=7.1 Hz), 2.53-2.62 (1H, m), 3.51 (2H, t, J=5.6 Hz), 3.76 (3H, s), 3.93 (2H, t, J=5.4 Hz), 4.19 (2H, d, J=2.2 Hz), 4.33 (2H, d, J=3.2 Hz), 5.39 (1H, d, J=9.5 Hz), 5.93 (1H, d, J=9.5 Hz), 6.65 (2H, d, J=8.5 Hz), 6.96 (2H, d, J=8.3 Hz), 7.05-7.13 (4H, m).
MS (FAB) m/z: 587, 589.

Example 11

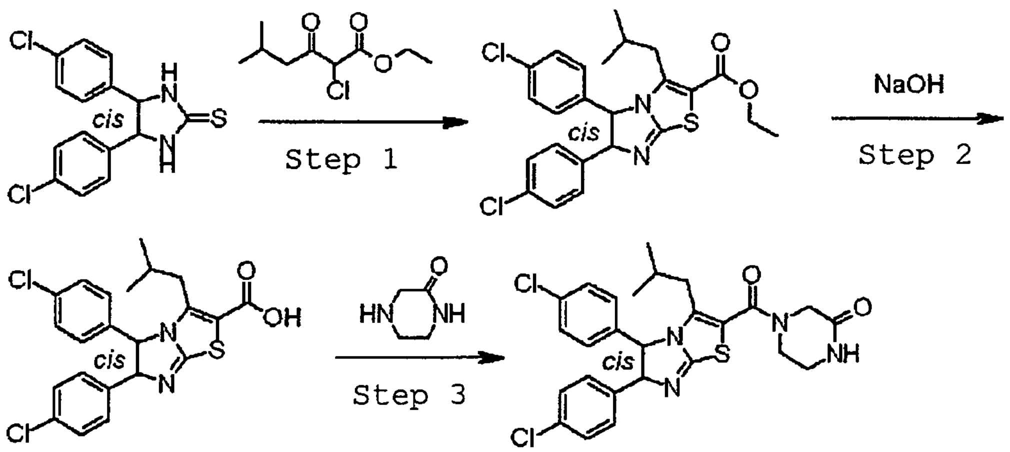


Step 1: Ethyl (5R*,6S*)-5,6-Bis(4-chlorophenyl)-3-isobutyl-5,6-dihydroimidazo[2,1-b][1,3]thiazole-2-carboxylate

The same reaction was performed as in Step 1 of Example 4 using ethyl 2-chloro-5-methyl-3-oxohexanoate instead of ethyl 2-chloro-4-methyl-3-oxopentanoate to give the title compound as a racemic mixture.
$^1$H-NMR ($CDCl_3$) δ: 0.91 (3H, d, J=6.6 Hz), 0.92 (3H, d, J=6.6 Hz), 1.52 (9H, s), 1.56-1.59 (1H, m), 1.87-1.94 (1H, m),

2.99 (1H, dd, J=13.1, 7.4 Hz), 5.31 (1H, d, J=9.8 Hz), 5.89 (1H, d, J=9.5 Hz), 6.65 (2H, d, J=8.5 Hz), 6.95 (2H, d, J=8.3 Hz), 7.06 (2H, d, J=8.5 Hz), 7.08 (2H, d, J=8.5 Hz).
MS (ESI) m/z: 503.

Step 2: (5R*,6S*)-5,6-Bis(4-chlorophenyl)-3-isobutyl-5,6-dihydroimidazo[2,1-b][1,3]thiazole-2-carboxylic acid

The compound obtained in Step 1 above was reacted in the same way as in Step 3 of Example 1 to give the title compound as a racemic mixture.
$^1$H-NMR (DMSO-$d_6$) δ: 0.82 (3H, d, J=6.6 Hz), 0.85 (3H, d, J=6.6 Hz), 1.56 (1H, dd, J=13.9, 5.9 Hz), 1.87-1.94 (1H, m), 2.91 (1H, dd, J=13.3, 8.7 Hz), 5.94 (1H, d, J=9.8 Hz), 6.00 (1H, d, J=9.8 Hz), 6.85 (2H, d, J=7.6 Hz), 7.09 (2H, d, J=8.5 Hz), 7.15 (2H, d, J=8.5 Hz), 7.20 (2H, d, J=8.8 Hz).
MS (ESI) m/z: 447.

Step 3: 4-{[(5R*,6S*)-5,6-Bis(4-chlorophenyl)-3-isobutyl-5,6-dihydroimidazo[2,1-b][1,3]thiazol-2-yl]carbonyl}piperazin-2-one

The compound obtained in Step 2 above was reacted with piperazin-2-one in the same way as in Step 4 of Example 1 to give the title compound as a racemic mixture.
$^1$H-NMR ($CDCl_3$) δ: 0.84 (3H, d, J=6.3 Hz), 0.84 (3H, d, J=6.6 Hz), 1.69 (1H, dd, J=14.0, 5.4 Hz), 1.76-1.83 (1H, m), 2.41 (1H, dd, J=14.0, 9.2 Hz), 3.45-3.49 (2H, m), 3.73-3.78 (1H, m), 3.87-3.93 (1H, m), 4.28 (2H, d, J=1.7 Hz), 5.36 (1H, d, J=9.8 Hz), 5.92 (1H, d, J=9.5 Hz), 6.18 (1H, s), 6.68 (2H, d, J=8.5 Hz), 6.95 (2H, d, J=8.5 Hz), 7.07 (2H, d, J=8.5 Hz), 7.09 (2H, d, J=8.5 Hz).
MS (ESI) m/z: 529.

Example 12

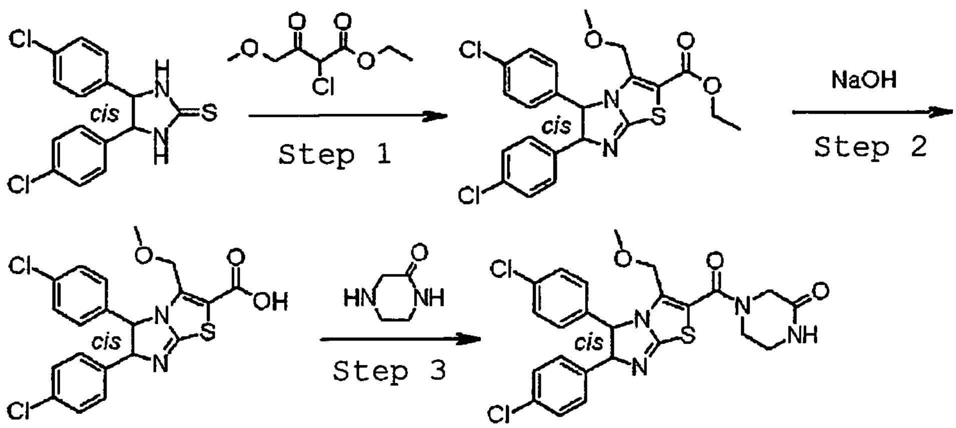


Step 1: Ethyl (5R*,6S*)-5,6-Bis(4-chlorophenyl)-3-methoxymethyl-5,6-dihydroimidazo[2,1-b][1,3]thiazole-2-carboxylate

The compound obtained in Step 1 of Example 2 was reacted in the same way as in Step 1 of Example 4 using ethyl 2-chloro-4-methoxy-3-oxobutanoate instead of ethyl 2-chloro-4-methyl-3-oxopentanoate to give the title compound as a racemic mixture.
$^1$H-NMR ($CDCl_3$) δ: 1.34 (3H, t, J=7.2 Hz), 3.20 (3H, s), 3.50 (1H, d, J=13.4 Hz), 4.24-4.29 (2H, m), 4.94 (1H, d, J=13.4 Hz), 5.60 (1H, d, J=10.0 Hz), 5.94 (1H, d, J=10.0 Hz), 6.71 (2H, d, J=8.5 Hz), 6.96 (2H, d, J=8.5 Hz), 7.06 (2H, d, J=8.3 Hz), 7.07 (2H, d, J=8.5 Hz).
MS (ESI) m/z: 463.

Step 2: (5R*, 6S*)-5,6-Bis(4-chlorophenyl)-3-methoxymethyl-5,6-dihydroimidazo[2,1-b][1,3]thiazole-2-carboxylic acid

The compound obtained in Step 1 above was reacted in the same way as in Step 3 of Example 1 to give the

title compound as a racemic mixture.
$^1$H-NMR (DMSO-$d_6$) δ: 3.07 (3H, s), 3.45 (1H, d, J=13.2 Hz), 4.81 (1H, d, J=12.9 Hz), 5.86 (1H, d, J=10.0 Hz), 5.99 (1H, d, J=10.0 Hz), 6.83 (2H, d, J=7.8 Hz), 7.09 (2H, d, J=8.5 Hz), 7.13 (2H, d, J=8.5 Hz), 7.17 (2H, d, J=8.5 Hz).
MS (ESI) m/z: 435.

Step 3:4-{[(5R*,6S*)-5,6-Bis(4-chlorophenyl)-3-methoxymethyl-5,6-dihydroimidazo[2,1-b][1,3]thiazol-2-yl]carbonyl}piperazin-2-one

The compound obtained in Step 2 above was reacted with piperazin-2-one in the same way as in Step 4 of Example 1 to give the title compound as a racemic mixture.
$^1$H-NMR ($CDCl_3$) δ: 3.12 (3H, s), 3.42 (1H, d, J=12.9 Hz), 3.44-3.50 (2H, m), 3.75-3.81 (1H, m), 3.87-3.94 (1H, m), 4.21 (1H, d, J=12.9 Hz), 4.22-4.31 (2H, m), 5.56 (1H, d, J=10.0 Hz), 5.94 (1H, d, J=10.0 Hz), 6.41 (1H, brs), 6.74 (2H, d, J=8.3 Hz), 6.95 (2H, d, J=8.5 Hz), 7.07 (2H, d, J=8.5 Hz), 7.09 (2H, d, J=8.5 Hz).
MS (ESI) m/z: 517.

Example 13

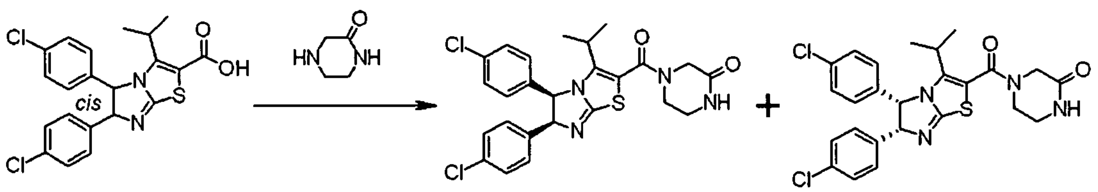

4-{[(5R,6S)-5,6-Bis(4-chlorophenyl)-3-isopropyl-5,6-dihydroimidazo[2,1-b][1,3]thiazol-2-yl]carbonyl}piperazin-2-one and 4-{[(5S,6R)-5,6-Bis(4-chlorophenyl)-3-isopropyl-5,6-dihydroimidazo[2,1-b][1,3]thiazol-2-yl]carbonyl}piperazin-2-one
The compound obtained in Step 2 of Example 4 was reacted with piperazin-2-one in the same way as in Step 4 of Example 1 to give the racemic title compounds. Subsequently, the title compounds were resolved by an optically active column (CHIRALCEL OD (Daicel Chemical Industries, ltd.), 2 cmϕ × 25 cm, eluting solvent; hexane:2-propanol = 70:30) to give isomer A (eluted earlier) and isomer B.
Isomer A
$^1$H-NMR ($CDCl_3$) δ: 0.96 (3H, d, J=7.1 Hz), 1.00 (3H, d, J=7.1 Hz), 2.52-2.62 (1H, m), 3.46-3.53 (2H, m), 3.81-3.91 (2H, m), 4.29 (2H, brs), 5.36 (1H, d, J=9.5 Hz), 5.91 (1H, d, J=9.5 Hz), 6.04 (1H, brs), 6.66 (2H, d, J=8.3 Hz), 6.96 (2H, d, J=8.3 Hz), 7.08 (4H, dd, J=11.0, 8.3 Hz).
MS (FAB) m/z: 515, 517.

Example 14

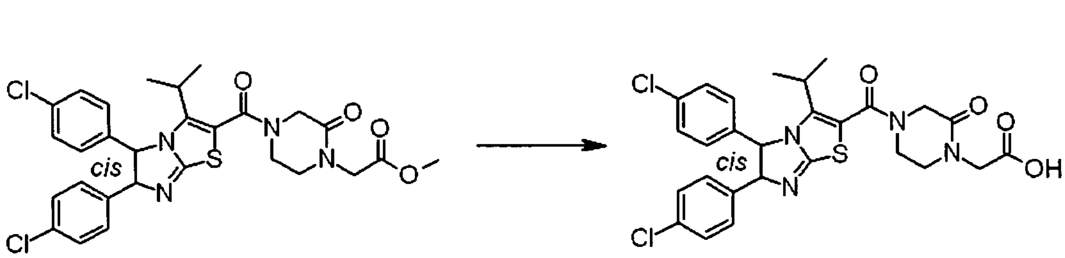

(4-{([(5R*,6S*)-5,6-Bis(4-chlorophenyl)-3-isopropyl-5,6-dihydroimidazo[2,1-b][1,3]thiazol-2-yl]carbony}-2-oxopiperazin-1-yl)acetic acid

The compound (169 mg, 0.29 mmol) obtained in Example 10 was dissolved in methanol (10 ml) followed by the dropwise addition of 1 N aqueous sodium hydroxide solution (432 μl, 0.43 mmol) and the resulting mixture was heated under an argon atmosphere at about 60°C for two hours. After cooling, 1 N aqueous hydrochloric acid solution (432 μl) was added for neutralization and the solvent was evaporated under reduced pressure. After the residue was purified by silica gel thin layer chromatography (chloroform:methanol:water = 8:3:0.5), the residue was repurified by HPLC (this column was Develosil Combi-PR-5 manufactured by Nomura Chemical Company, eluting solvent; water: acetonitrile =84:16 → 46:53 (containing 0.1% formic acid)) and lyophilized with dioxane to give the title compound (51.8 mg, 31%) as a racemic colorless solid mixture.
$^1$H-NMR ($CDCl_3$) δ: 0.95 (3H, d, J=7.1 Hz), 1.04 (3H, d, J=7.1 Hz), 2.52-2.62 (1H, m), 3.56-3.66 (2H, m), 3.85-3.93 (2H, m), 4.12-4.19 (2H, m), 4.26-4.30 (2H, m), 5.49 (1H, d, J=9.7 Hz), 5.99 (1H, d, J=9.7 Hz), 6.63 (2H, d, J=8.3 Hz), 6.95 (2H, d, J=8.3 Hz), 7.08-7.16 (4H, m).
MS (FAB) m/z: 573, 575.

Example 15

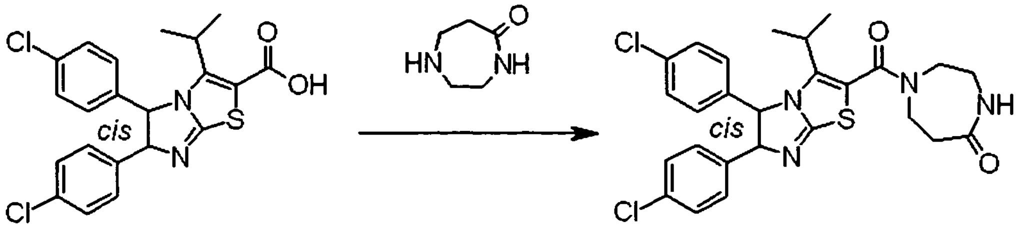

1-{[(5R*,6S*)-5,6-Bis(4-chlorophenyl)-3-isopropyl-5,6-dihydroimidazo[2,1-b][1,3]thiazol-2-yl]carbonyl}-1,4-diazepan-5-one

The compound obtained in Step 2 of Example 4 was reacted in the same way as in Step 4 of Example 1 using 1,4-diazepan-5-one instead of piperazin-2-one to give the title compound as a racemic mixture.
$^1$H-NMR ($CDCl_3$) δ: 0.97 (3H, d, J=7.1 Hz), 1.01 (3H, d, J=7.1 Hz), 2.50-2.59 (1H, m), 2.67-2.72 (2H, m), 3.33-3.40 (2H, m), 3.76-3.83 (4H, m), 5.38 (1H, d, J=9.4 Hz), 5.92 (1H, d, J=9.4 Hz), 6.02 (1H, brs), 6.65 (2H, d, J=8.4 Hz), 6.96 (2H, d, J=8.4 Hz), 7.04-7.11 (4H, m).
MS (ESI) m/z: 529, 531.

Example 16

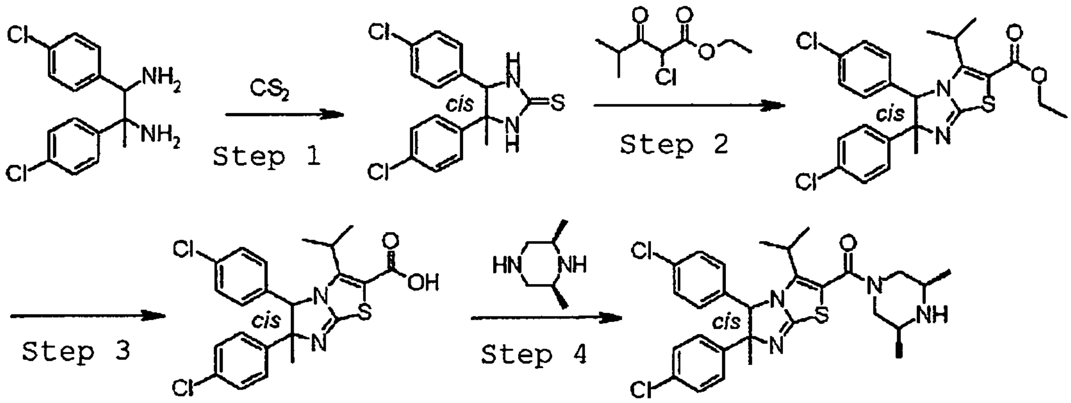


Step 1: (4R*,5S*)-4,5-Bis(4-chlorophenyl)-4-methylimidazolidine-2-thione

The same reaction was performed as in Step 1 of Example 1 using racemic (1R*,2S*)-1,2-bis(4-chlorophenyl) propane-1,2-diamine instead of optically active (1R,2S)-1,2-bis(4-chlorophenyl)propane-1,2-diamine to give the title compound as a racemic mixture.

Step 2: Ethyl (5R*,6S*)-5,6-Bis(4-chlorophenyl)-3-isopropyl-6-methyl-5,6-dihydroimidazo[2,1-b][1,3]thiazole-2-carboxylate

The compound obtained in Step 1 above was reacted in the same way as in Step 2 of Example 1 to give the title compound as a racemic mixture.

Step 3: (5R*,6S*)-5,6-Bis(4-chlorophenyl)-3-isopropyl-6-methyl-5,6-dihydroimidazo[2,1-b][1,3]thiazole-2-carboxylic acid

The compound obtained in Step 2 above was reacted in the same way as in Step 3 of Example 1 to give the title compound as a racemic mixture.

Step 4: (5R*,6S*)-5,6-Bis(4-chlorophenyl)-2-{[(3R,5S)-3,5-dimethylpiperazin-1-yl]carbonyl}-3-isopropyl-6-methyl-5,6-dihydroimidazo[2,1-b][1,3]thiazole

The compound obtained in Step 3 above was reacted in the same way as in Step 4 of Example 1 using cis-2,6-dimethylpiperazine instead of piperazin-2-one to give the title compound as a racemic mixture.
$^1$H-NMR ($CDCl_3$) δ: 0.92 (3H, d, J=7.1 Hz), 1.03 (3H, d, J=7.1 Hz), 1.15 (6H, brs), 1.84 (3H, s), 2.40-2.62 (3H, m), 2.72-2.92 (2H, m), 4.11-4.25 (2H, m), 4.97 (1H, s), 6.74 (2H, brd, J=7.3 Hz), 7.04-7.07 (4H, m), 7.13 (2H, d, J=8.8 Hz).
MS (EI) m/z: 542, 544.

Example 17

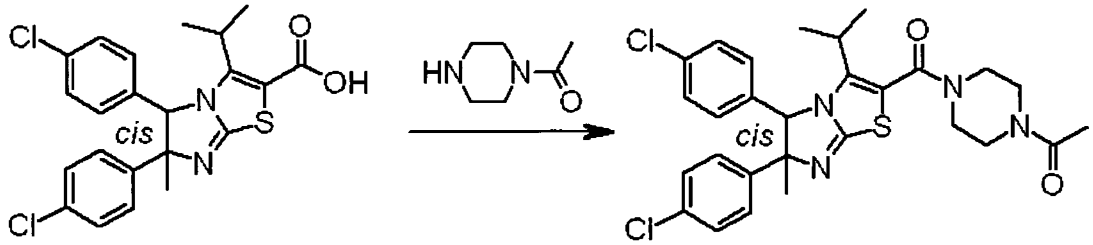

(5R*,6S*)-2-[(4-Acetylpiperazin-1-yl)carbonyl]-5,6-bis(4-chlorophenyl)-3-isopropyl-6-methyl-5,6-dihydroimidazo[2,1-b][1,3]thiazole

The compound obtained in Step 3 of Example 16 was reacted in the same way as in Step 4 of Example 1 using 1-acetylpiperazine instead of piperazin-2-one to give the title compound as a racemic mixture.
$^1$H-NMR ($CDCl_3$) δ: 0.90 (3H, d, J=7.1 Hz), 1.02 (3H, d, J=7.1 Hz), 1.83 (3H, s), 2.15 (3H, s), 2.50 (1H, m), 3.49-3.54 (2H, m), 3.58-3.69 (6H, m), 4.98 (1H, s), 6.73 (2H, brd, J=6.8 Hz), 7.02-7.11 (6H, m).
MS (FAB) m/z: 557, 559.

Example 18

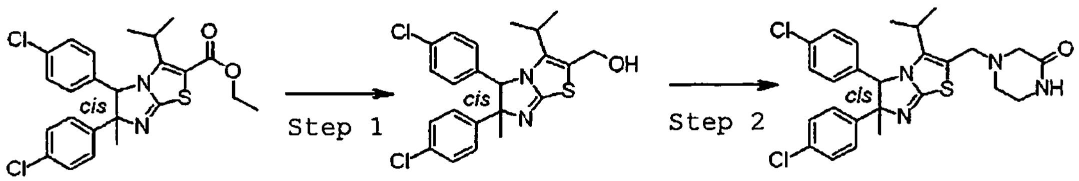


Step 1: [(5R*,6S*)-5,6-Bis(4-chlorophenyl)-3-isopropyl-6-methyl-5,6-dihydroimidazo[2,1-b][1,3]thiazol-2-yl]methanol

The compound obtained in Step 2 of Example 16 was reacted in the same way as in Example 9 to give the title compound as a racemic mixture.
$^1$H-NMR ($CDCl_3$) δ: 0.87 (3H, d, J=7.1 Hz), 0.96 (3H, d, J=7.1 Hz), 1.81 (3H, s), 2.43-2.57 (2H, m), 4.46 (1H, d, J=13.2 Hz), 4.52 (1H, d, J=13.2 Hz), 4.93 (1H, s), 6.64-6.74 (2H, m), 6.99-7.05 (4H, m), 7.11 (2H, d, J=8.5 Hz).

Step 2: 4-{[(5R*,6S*)-5,6-Bis(4-chlorophenyl)-3-isopropyl-6-methyl-5,6-dihydroimidazo[2,1-b][1,3]thiazol-2-yl]methyl}piperazin-2-one

The compound (225 mg, 0.52 mmol) obtained in Step 1 above was dissolved in methylene chloride (60 ml) followed by the dropwise addition of triethylamine (115 μl, 1.04 mmol) and methanesulfonyl chloride (48 μl, 0.62 mmol) under ice cooling. After stirring at the same temperature for 15 minutes, piperazin-2-one (260 mg, 2.59 mmol) was added and stirred at the same temperature for two hours and then stirred at room temperature for 13 hours. An aqueous sodium bicarbonate solution was added to the reaction mixture, followed by extraction with chloroform. The extract was washed with brine and then dried over anhydrous sodium sulfate. The solvent was evaporated under reduced pressure. The residue was purified by silica gel thin layer chromatography (chloroform:methanol = 10:1, then ethyl acetate:methanol = 10:1) and then lyophilized with dioxane to give the title compound (49.7 mg, 19%) as a racemic colorless solid mixture.
$^1$H-NMR ($CDCl_3$) δ: 0.84 (3H, d, J=7.1 Hz), 0.94 (3H, d, J=7.1 Hz), 1.84 (3H, s), 2.45-2.54 (1H, m), 2.70-2.75 (2H, m), 3.23 (2H, s), 3.40-3.46 (4H, m), 4.94 (1H, s), 5.79 (1H, s), 6.64-6.75 (2H, m), 6.99-7.06 (4H, m), 7.10-7.14 (2H, m).
MS (ESI) m/z: 515, 517.

Example 19

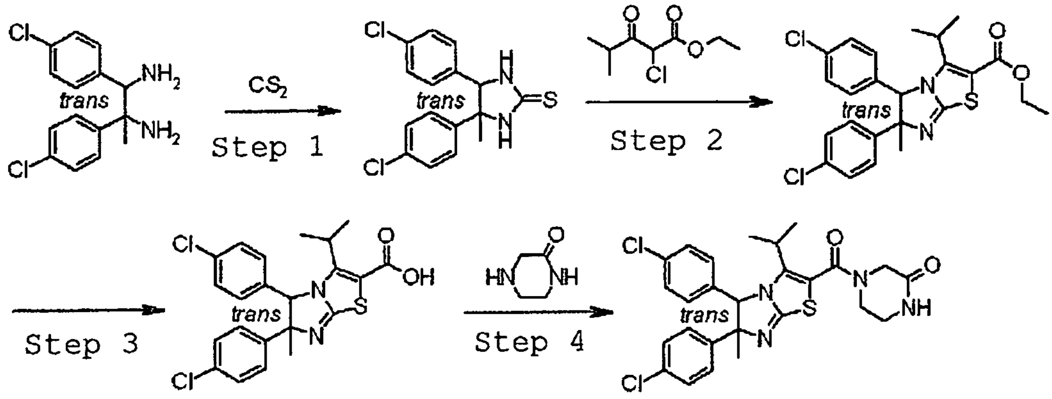


Step 1: (4R*, 5R*)-4,5-Bis(4-chlorophenyl)-4-methylimidazolidine-2-thione

The same reaction was performed as in Step 1 of Example 1 using (1R*,2R*)-1,2-bis(4-chlorophenyl)propane-1,2-diamine instead of (1R,2S)-1,2-bis(4-chlorophenyl)propane-1,2-diamine to give the title compound as a racemic mixture.
$^1$H-NMR ($CDCl_3$) δ: 1.23 (3H, s), 4.89 (1H, s), 6.27 (1H, brs), 6.41 (1H, brs), 7.13 (2H, d, J=8.1 Hz), 7.31-7.41 (6H, m).
MS (ESI) m/z: 337, 339.

Step 2: Ethyl (5R*,6R*)-5,6-Bis(4-chlorophenyl)-3-isopropyl-6-methyl-5,6-dihydroimidazo[2,1-b][1,3]thiazole-2-carboxylate

The compound obtained in Step 1 above was reacted in the same way as in Step 2 of Example 1 to give the title compound as a racemic mixture.
$^1$H-NMR ($CDCl_3$) δ: 0.94 (3H, d, J=7.1), 0.95 (3H, d, J=7.1), 1.36 (3H, t, J=7.1 Hz), 1.40 (3H, s), 3.27 (1H, m), 4.32 (2H, q, J=7.1 Hz), 5.58 (1H, s), 6.77 (1H, brd, J=5.9 Hz), 7.34 (1H, brd, J=3.9 Hz), 7.44-7.53 (6H, m).
MS (FAB) m/z: 475, 477.

Step 3: (5R*,6R*)-5,6-Bis(4-chlorophenyl)-3-isopropyl-6-methyl-5,6-dihydroimidazo[2,1-b][1,3]thiazole-2-carboxylic acid

The compound obtained in Step 2 above was reacted in the same way as in Step 3 of Example 1 to give the title compound as a racemic mixture.
$^1$H-NMR (DMSO-$d_6$) δ: 0.79 (6H, d, J=7.1 Hz), 1.02 (3H, s), 3.15 (1H, m), 5.51 (1H, s), 6.90 (1H, brd, J=7.8 Hz), 7.43 (2H, d, J=8.1 Hz), 7.50-7.57 (4H, m), 7.64 (1H, brs).
MS (EI) m/z: 446, 448.

Step 4: 4-[(5R*,6R*)-5,6-Bis(4-chlorophenyl)-3-isopropyl-6-methyl-5,6-dihydroimidazo[2,1-b][1,3]thiazol-2-yl]carbonyl} piperazin-2-one

The compound obtained in Step 3 above was reacted with piperazin-2-one in the same way as in Step 4 of Example 1 to give the title compound as a racemic mixture. $^1$H-NMR ($CDCl_3$) δ: 0.87 (3H, d, J=7.1 Hz), 0.91 (3H, d, J=7.1 Hz), 1.21 (3H, s), 2.39-2.46 (1H, m), 3.46-3.51 (2H, m), 3.81-3.87 (2H, m), 4.29 (2H, s), 5.07 (1H, s), 6.02 (1H, s), 7.09 (1H, brd, J=6.6 Hz), 7.20 (1H, brd, J=6.6 Hz), 7.38-7.46 (6H, m).
MS (EI) m/z: 528, 530.

Example 20

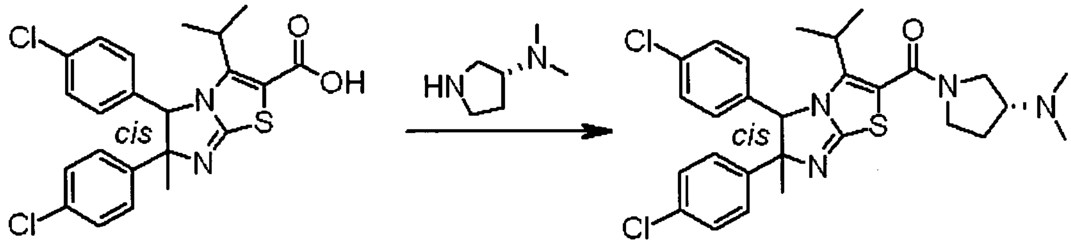

(3R)-1-{[(5R*,6S*)-5,6-Bis(4-chlorophenyl)-3-isopropyl-6-methyl-5,6-dihydroimidazo[2,1-b][1,3]thiazol-2-yl]carbonyl}-N,N-dimethylaminopyrrolidin-3-amine

The compound obtained in Step 3 of Example 16 was reacted in the same way as in Step 4 of Example 1 using (3R)-N,N-dimethylpyrrolidin-3-amine instead of piperazin-2-one to give the title compound as a mixture of diastereomers. $^1$H-NMR ($CDCl_3$) δ: 0.90-1.03 (6H, m), 1.81 (3H, s), 1.84-1.89 (1H, m), 2.11-2.20 (1H, m), 2.30 (6H, s), 2.51-2.54 (1H, m), 2.69-2.79 (1H, m), 3.26-3.34 (1H, m), 3.44-3.60 (1H, m), 3.65-3.85 (2H, m), 4.97 and 4.95 (total 1H, each s), 6.66-6.72 (2H, m), 7.00-7.05 (4H, m), 7.07-7.12 (2H, m) .
MS (FAB) m/z: 543, 545.

Example 21

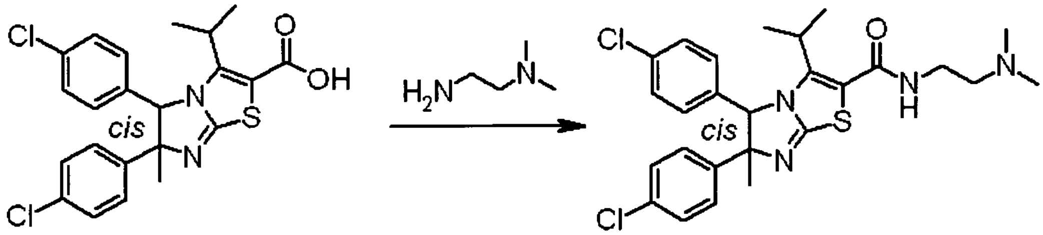

(5R*, 6S*)-5,6-Bis(4-chlorophenyl)-N-[2-(dimethylamino)ethyl]-3-isopropyl-6-methyl-5,6-dihydroimidazo[2,1-b][1,3]thiazole-2-carboxamide

The compound obtained in Step 3 of Example 16 was reacted in the same way as in Step 4 of Example 1 using N,N-dimethylethane-1,2-diamine instead of piperazin-2-one to give the title compound as a racemic mixture. $^1$H-NMR ($CDCl_3$) δ: 0.82 (3H, d, J=7.1 Hz), 1.03 (3H, d, J=7.1 Hz), 1.80 (3H, s), 2.27 (6H, s), 2.45-2.49 (2H, m), 3.31-3.42 (3H, m), 5.04 (1H, s), 6.19 (1H, brs), 6.65-6.73 (2H, m), 7.00-7.05 (4H, m), 7.10 (2H, d, J=8.5 Hz).
MS (FAB) m/z: 517, 519.

Example 22

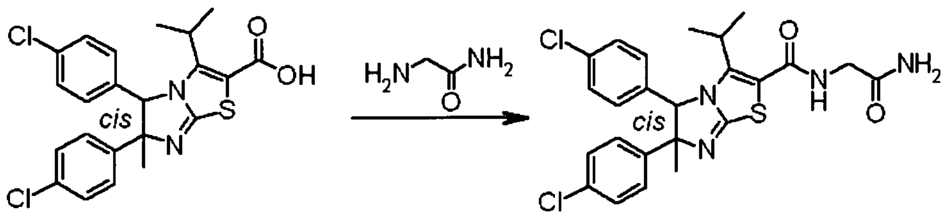

(5R*,6S*)-N-(2-Amino-2-oxoethyl)-5,6-Bis(4-chlorophenyl)-3-isopropyl-6-methyl-5,6-dihydroimidazo[2,1-b][1,3]thiazole-2-carboxamide

The compound obtained in Step 3 of Example 16 was reacted in the same way as in Step 4 of Example 1 using glycinamide instead of piperazin-2-one to give the title compound as a racemic mixture.
$^1$H-NMR ($CDCl_3$) δ: 0.84 (3H, d, J=7.1 Hz), 1.03 (3H, d, J=7.1 Hz), 1.82 (3H, s), 3.31-3.38 (1H, m), 3.98-4.07 (2H, m), 5.07 (1H, s), 5.48 (1H, brs), 5.94 (1H, brs), 6.26 (1H, brs), 6.57-6.75 (2H, m), 7.01-7.06 (4H, m), 7.09-7.11 (2H, m).
MS (EI) m/z: 502, 504.

Example 23

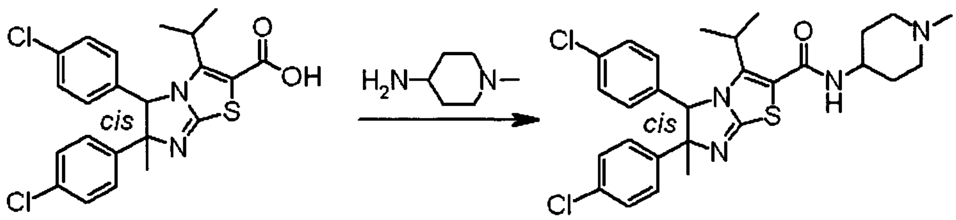

(5R*,6S*)-5,6-Bis(4-chlorophenyl)-3-isopropyl-6-methyl-N-(1-methylpiperidin-4-yl)-5,6-dihydroimidazo[2,1-b][1,3]thiazole-2-carboxamide

The compound obtained in Step 3 of Example 16 was reacted in the same way as in Step 4 of Example 1 using 1-methylpiperidin-4-amine instead of piperazin-2-one to give the title compound as a racemic mixture.
$^1$H-NMR ($CDCl_3$) δ: 0.84 (3H, d, J=7.1 Hz), 1.00 (3H, d, J=7.1 Hz), 1.18-1.27 (1H, m), 1.47-1.52 (1H, m), 1.80 (3H, s), 1.94-2.01 (2H, m), 2.01-2.07 (2H, m), 2.28 (3H, s), 2.75-2.85 (2H, m), 3.26-3.33 (1H, m), 3.77-3.87 (1H, m), 5.04 (1H, s), 5.26 (1H, brd, J=7.3 Hz), 6.55-6.73 (2H, brs), 6.99-7.04 (4H, m), 7.09-7.11 (2H, m).
MS (FAB) m/z: 543, 545.

Example 24

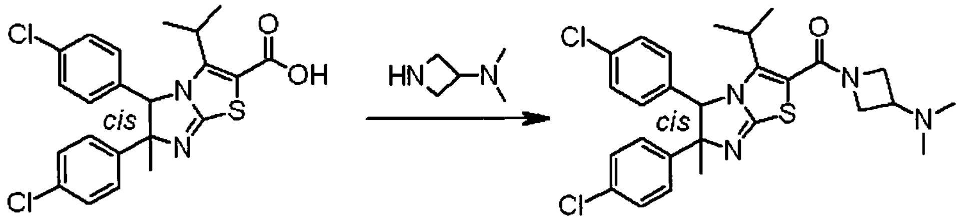

1-{[(5R*,6S*)-5,6-Bis(4-chlorophenyl)-3-isopropyl-6-methyl-5,6-dihydroimidazo[2,1-b][1,3]thiazol-2-yl]carbonyl}-N,N-dimethylazetidin-3-amine

The compound obtained in Step 3 of Example 16 was reacted in the same way as in Step 4 of Example 1 using 3-dimethylaminoazetidine instead of piperazin-2-one to give the title compound as a racemic mixture.
$^1$H-NMR ($CDCl_3$) δ: 0.83 (3H, d, J=7.1 Hz), 1.00 (3H, d, J=7.1 Hz), 1.80 (3H, s), 2.19 (6H, s), 3.05-3.12 (1H, m), 3.14-3.22 (1H, m), 3.95-4.04 (2H, m), 4.14-4.25 (2H, m), 5.02 (1H, s), 6.61-6.73 (2H, m), 7.00-7.03 (4H, m), 7.08-7.12 (2H, m).
MS (FAB) m/z: 529, 531.

Example 25

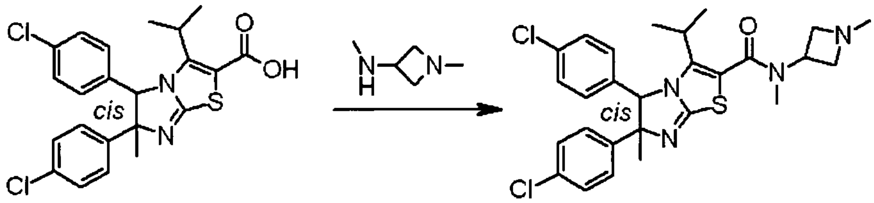

(5R*,6S*)-5,6-Bis(4-chlorophenyl)-3-isopropyl-N,6-dimethyl-N-(1-methylazetidine-3-yl)-5,6-dihydroimidazo[2,1-b][1,3]thiazole-2-carboxamide

The compound obtained in Step 3 of Example 16 was reacted in the same way as in Step 4 of Example 1 using N,1-dimethylazetidin-3-amine instead of piperazin-2-one to give the title compound as a racemic mixture.
$^1$H-NMR ($CDCl_3$) δ: 0.86 (3H, d, J=7.1 Hz), 0.97 (3H, d, J=7.1 Hz), 1.81 (3H, s), 2.37 (3H, s), 2.42-2.49 (1H, m), 3.11 (3H, s), 3.13-3.19 (2H, m), 3.58-3.66 (2H, m), 4.67-4.73 (1H, m), 4.94 (1H, s), 6.70-6.72 (2H, m), 6.99-7.11 (6H, m).
MS (FAB) m/z: 529, 531.

Example 26

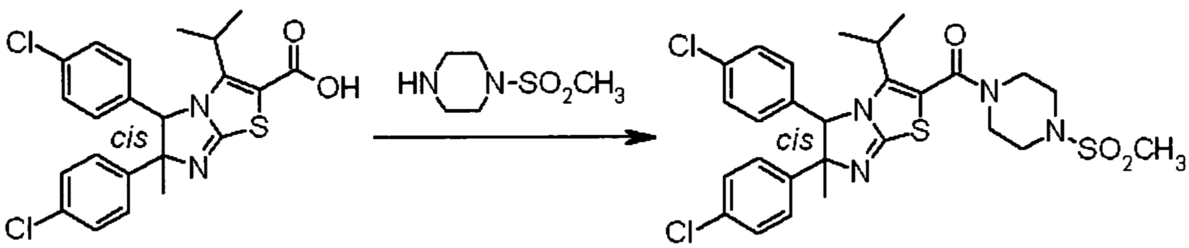

(5R*,6S*)-5,6-Bis(4-chlorophenyl)-3-isopropyl-6-methyl-2[(4-methylsulfonylpiperazin-1-yl)carbonyl]-5,6-dihydroimidazo[2,1-b][1,3]thiazole

The compound obtained in Step 3 of Example 16 was reacted in the same way as in Step 4 of Example 1 using 1-methanesulfonylpiperazine instead of piperazin-2-one to give the title compound as a racemic mixture.
$^1$H-NMR ($CDCl_3$) δ : 0.90 (3H, d, J=7.1 Hz), 1.02 (3H, d, J=7.1 Hz), 1.84 (3H, s), 2.46-2.55 (1H, m), 2.83 (3H, s), 3.28 (4H, t, J=4.9 Hz), 3.72 (4H, t, J=4.9 Hz), 5.00 (1H, s), 6.66-6.76 (2H, m), 7.02-7.11 (6H, m).
MS (ESI) m/z: 593, 595.

Example 27

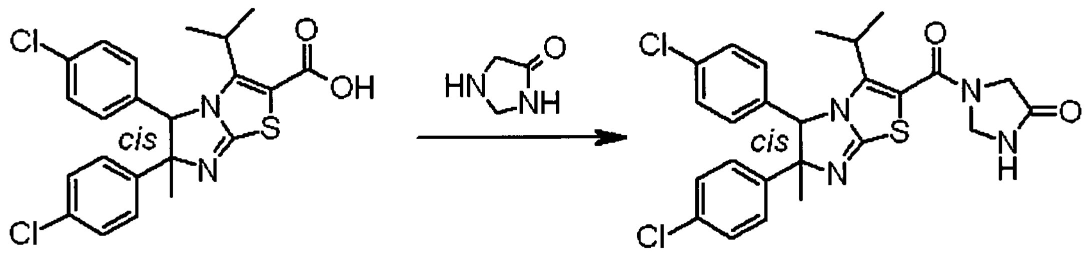

1-{[(5R*,6S*)-5,6-Bis(4-chlorophenyl)-3-isopropyl-6-methyl-5,6-dihydroimidazo[2,1-b][1,3]thiazol-2-yl]carbonyl}imidazolidin-4-one

The compound obtained in Step 3 of Example 16 was reacted in the same way as in Step 4 of Example 1 using imidazolidin-4-one instead of piperazin-2-one to give the title compound as a racemic mixture.
$^1$H-NMR ($CDCl_3$) δ: 0.95 (3H, d, J=7.1 Hz), 0.97 (3H, d, J=7.1 Hz), 1.85 (3H, s), 2.72-2.81 (1H, m), 4.13 (1H, d, J=16.4 Hz), 4.20 (1H, d, J=16.4 Hz), 4.99-5.06 (3H, m), 6.08 (1H, brs), 6.66-6.75 (2H, m), 7.02-7.12 (6H, m).
MS (ESI) m/z: 515, 517.

Example 28

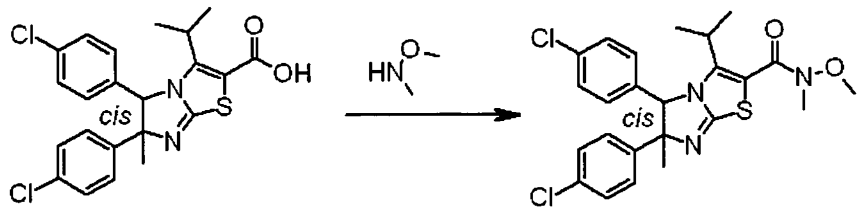

(5R*,6S*)-5,6-Bis(4-chlorophenyl)-3-isopropyl-N-methoxy-N,6-dimethyl-5,6-dihydroimidazo[2,1-b][1,3]thiazole-2-carboxamide

The compound obtained in Step 3 of Example 16 was reacted in the same way as in Step 4 of Example 1 using N,O-dimethylhydroxyamine hydrochloride instead of piperazin-2-one to give the title compound as a racemic mixture.
$^1$H-NMR ($CDCl_3$) δ: 0.82 (3H, d, J=7.1 Hz), 1.04 (3H, d, J=7.1 Hz), 1.81 (3H, s), 3.25 (3H, s), 3.38 (1H, sept, J=7.1 Hz), 3.75 (3H, s), 5.07 (1H, s), 6.68 (2H, brd, J=7.6 Hz), 7.01-7.03 (4H, m), 7.12 (2H, d, J=9.0 Hz).
MS (FAB) m/z: 490, 492.

Example 29

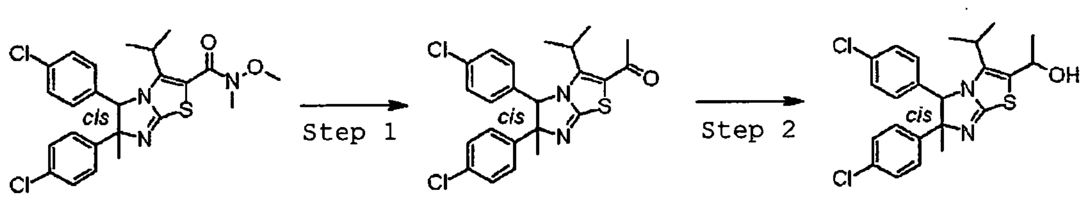


Step 1: 1-[(5R*,6S*)-5,6-Bis(4-chlorophenyl)-3-isopropyl-6-methyl-5,6-dihydroimidazo[2,1-b][1,3]thiazol-2-yl]ethanone

After methylmagnesium bromide (2.1 ml, 1.89 mmol; 0.89 M tetrahydrofuran solution) was added dropwise to a tetrahydrofuran (6 ml) solution of the compound (310 mg, 0.63 mmol) obtained in Example 28 under ice cooling, and the temperature was slowly warmed to room temperature. After stirring for one hour, the mixture was ice cooled followed by the addition of saturated ammonium chloride aqueous solution and stirred at room temperature. The reaction mixture was extracted with ethyl acetate and dried over anhydrous sodium sulfate after washing with brine. After the solvent was evaporated under reduced pressure, the residue was purified by silica gel column chromatography to give the title compound (241 mg, 86%) as a yellow solid racemic mixture.
$^1$H-NMR ($CDCl_3$) δ: 0.81 (3H, d, J=7.1 Hz), 1.00 (3H, d, J=7.1 Hz), 1.81 (3H, s), 2.32 (3H, s), 3.39 (1H, sept, J=7.1 Hz), 5.10 (1H, s), 6.69 (2H, brs), 7.02-7.06 (4H, m), 7.09-7.12 (2H, m).
MS (FAB) m/z: 445, 447.

Step 2: 1-[(5R*,6S*)-5,6-Bis(4-chlorophenyl)-3-isopropyl-6-methyl-5,6-dihydroimidazo[2,1-b][1,3]thiazol-2-yl]ethanol

Sodium borohydride (18 mg, 0.48 mmol) was slowly added to a methanol (5 ml) solution of the compound (214 mg, 0.48 mmol) obtained in Step 1 above at room temperature. The solvent was evaporated under reduced pressure after the reaction was completed. Water was added to the residue and stirred and deposited solid was collected by filtration. The obtained solid was purified by silica gel column chromatography to give a low polarity isomer (58 mg, 27%) and a high polarity isomer (95 mg, 44%) as a colorless solid each.
Low polarity isomer:
$^1$H-NMR ($CDCl_3$) δ: 0.89 (3H, d, J=7.1 Hz), 0.94 (3H, d, J=7.1 Hz), 1.46 (3H, d, J=6.3 Hz), 1.81 (3H, s), 2.47 (1H, sept, J=7.1 Hz), 4.89 (1H, s), 5.04 (1H, q, J=6.3 Hz), 6.69 (2H, brd, J=6.8 Hz), 6.99-7.03 (4H, m), 7.11 (2H, d, J=8.8 Hz).
MS (FAB) m/z: 447, 449.
High polarity isomer:
$^1$H-NMR ($CDCl_3$) δ: 0.85 (3H, d, J=7.1 Hz) , 0.92 (3H, d, J=7.1 Hz), 1.46 (3H, d, J=6.3 Hz), 1.79 (3H, s), 2.54 (1H, sept, J=7.1 Hz), 4.91 (1H, s), 5.02 (1H, q, J=6.3 Hz), 6.68 (2H, brs), 6.99-7.04 (4H, td, J=5.1, 2.9 Hz), 7.12 (2H, d, J=8.8 Hz) .
MS (FAB) m/z: 447, 449.

Example 30

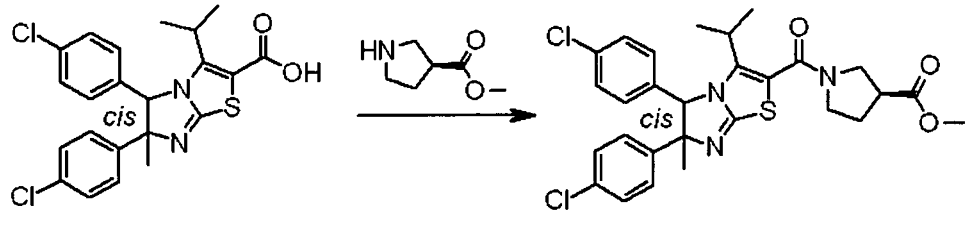

Methyl (3S)-1-{[(5R*,6S*)-5,6-Bis(4-chlorophenyl)-3-isopropyl-6-methyl-5,6-dihydroimidazo[2, 1-b][1,3]thiazol-2-yl]carbonyl}pyrrolidine-3-carboxylate

The compound obtained in Step 3 of Example 16 was reacted in the same way as in Step 4 of Example 1 using methyl pyrrolidine-3-carboxylate instead of piperazin-2-one to give the title compound as a mixture of the diastereomers.
$^1$H-NMR ($CDCl_3$) δ: 0.90-1.00 (6H, m), 1.82 (3H, s), 2.20-2.25 (2H, m), 2.54-2.61 (1H, m), 3.09-3.17 (1H, m), 3.53-3.86

(7H, m), 4.97 (1H, s), 6.66-6.72 (2H, m), 7.00-7.06 (4H, m), 7.10 (2H, d, J=8.5 Hz).
MS (FAB) m/z: 558, 560.

Example 31

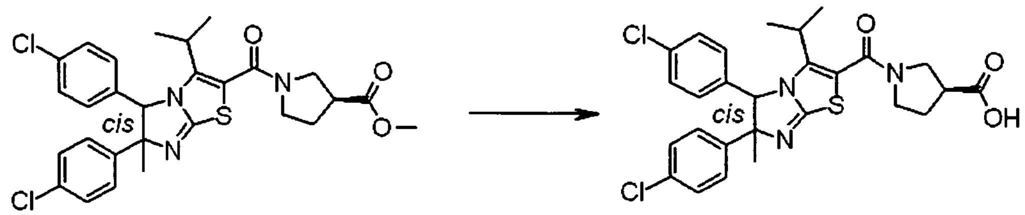

(3S)-1-{[(5R*,6S*)-5,6-Bis(4-chlorophenyl)-3-isopropyl-6-methyl-5,6-dihydroimidazo[2,1-b][1,3]thiazol-2-yl]carbonyl} pyrrolidine-3-carboxylic acid

A 0.25 N aqueous sodium hydroxide solution (2.90 ml, 0.71 mmol) was added at room temperature to a dioxane (3 ml) solution of the compound (266 mg, 0.47 mmol) obtained in Example 30. After stirring at the same temperature for six hours, the reaction mixture was concentrated under reduced pressure, and then the residue was diluted with water and 1 N aqueous hydrochloric acid solution was added for acidification. Deposited solid was collected by filtration, washed with water and then dried to give the title compound (130 mg, 50%) as a colorless solid diastereomer mixture.
$^1$H-NMR (DMSO-$d_6$) δ: 0.75-0.81 (3H, m), 0.94-0.99 (3H, m), 2.00 (3H, s), 2.04-2.19 (2H, m), 2.55-2.62 (1H, m), 3.09-3.17 (1H, m), 3.41-3.53 (2H, m), 3.58-3.70 (2H, m), 6.03 (1H, s), 6.78-7.26 (8H, m).
MS (FAB) m/z: 544, 546.

Example 32

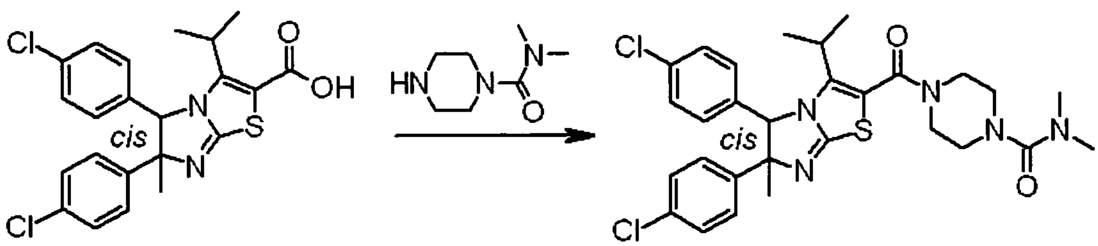

4-{[(5R*,6S*)-5,6-Bis(4-chlorophenyl)-3-isopropyl-6-methyl-5,6-dihydroimidazo[2,1-b][1,3]thiazol-2-yl]carbonyl}-N,N-dimethylpiperazine-1-carboxamide

The compound obtained in Step 3 of Example 16 was reacted in the same way as in Step 4 of Example 1 using N,N-dimethylpiperazine-1-carboxamide instead of piperazin-2-one to give the title compound as a racemic mixture.
$^1$H-NMR ($CDCl_3$) δ: 0.89 (3H, d, J=7.1 Hz), 1.02 (3H, d, J=7.1 Hz), 1.84 (3H, s), 2.42-2.51 (1H, m), 2.87 (6H, s), 3.24-3.30 (4H, m), 3.61-3.66 (4H, m), 4.98 (1H, s), 6.67-6.77 (2H, m), 7.01-7.12 (6H, m).
MS (ESI) m/z: 586.

Example 33

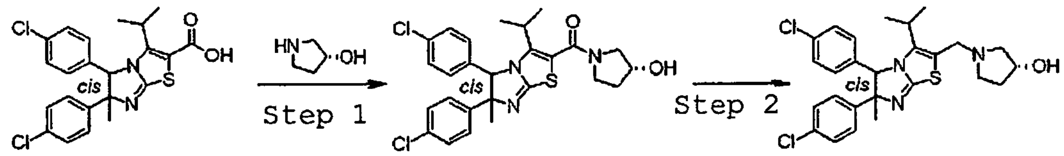


Step 1: (3R)-1-{[(5R*, 6S*)-5,6-Bis(4-chlorophenyl)-3-isopropyl-6-methyl-5,6-dihydroimidazo[2,1-b][1,3]thiazol-2-yl]carbonyl}pyrrolidin-3-ol

The compound obtained in Step 3 of Example 16 was reacted in the same way as in Step 4 of Example 1 using (3R)-pyrrolidin-3-ol instead of piperazin-2-one to give the title compound as a mixture of diastereomers.
$^1$H-NMR ($CDCl_3$) δ: 0.92-1.02 (6H, m), 1.81 (3H, s), 1.97-2.10 (2H, m), 2.54-2.65 (1H, m), 3.50-3.81 (5H, m), 4.55 (1H, brs), 4.95and4.97 (total 1H, each s), 6.66-6.73 (2H, m), 6.99-7.12 (6H, m).
MS (FAB) m/z: 516, 518.

Step 2: (3R)-1-{[(5R*,6S*)-5,6-Bis(4-chlorophenyl)-3-isopropyl-6-methyl-5,6-dihydroimidazo[2,1-b][1,3]thiazol-2-yl]methyl}pyrrolidin-3-ol

The compound obtained in Step 1 above was reacted in the same way as in Example 9 to give the title compound as a mixture of diastereomers.
$^1$H-NMR ($CDCl_3$) δ: 0.83-0.90 (6H, m), 1.80 (3H, s), 2.15-2.64 (6H, m), 2.70-2.74 (1H, m), 2.89-2.96 (1H, m), 3.41-3.54 (2H, m), 4.35 (1H, brs), 4.89 (1H, s), 6.65-6.71 (2H, m), 6.99-7.04 (4H, m), 7.10-7.14 (2H, m).
MS (FAB) m/z: 502, 504.

Example 34

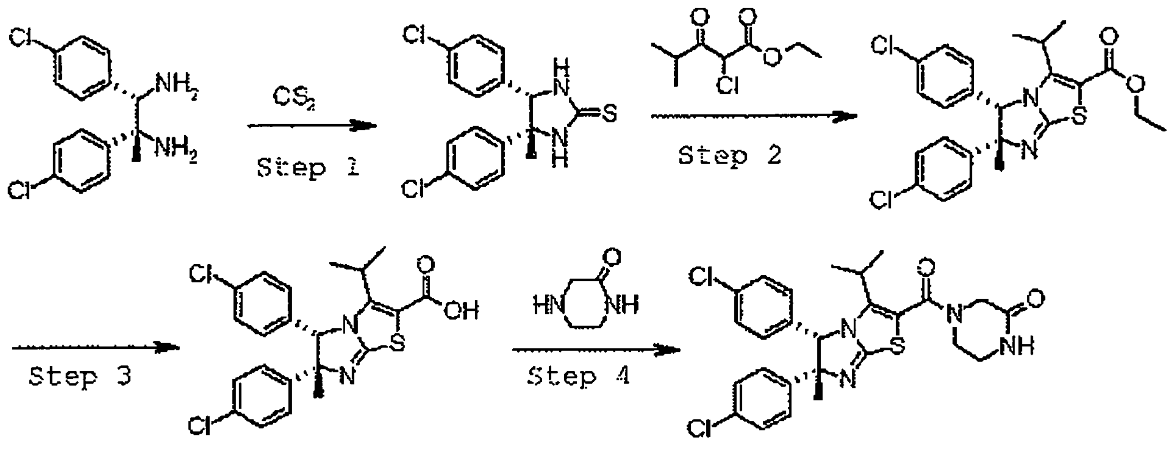


4-{[(5S,6R)-5,6-Bis(4-chlorophenyl)-3-isopropyl-6-methyl-5,6-dihydroimidazo[2,1-b][1,3]thiazol-2-yl]carbonyl}piperazin-2-one

In the series of steps of Example 1, the starting material, (1R,2S)-1,2-bis(4-chlorophenyl)propane-1,2-diamine was substituted with the optical isomer thereof, (1S,2R)-1,2-bis(4-chlorophenyl)propane-1,2-diamine, and the series of reaction operation was performed in the same way to give the title compound.
$^1$H-NMR ($CDCl_3$) δ: 0.89 (3H, d, J=7.1 Hz), 1.00 (3H, d, J=7.1 Hz), 1.83 (3H, s), 2.48-2.55 (1H, m), 3.44-3.52 (2H, m), 3.84 (2H, td, J=5.4, 2.1 Hz), 4.28 (2H, s), 4.97 (1H, s), 6.03 (1H, brs), 6.67-6.77 (2H, m), 7.02-7.10 6H, m). MS (ESI) m/z: 529, 531.

Example 35

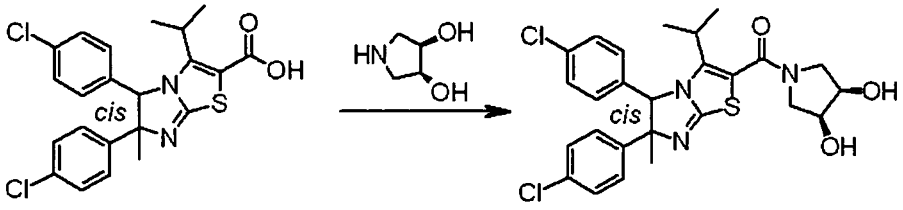

(3R,4S)-1-{[(5R*,6S*)-5,6-Bis(4-chlorophenyl)-3-isopropyl-6-methyl-5,6-dihydroimidazo[2,1-b][1,3]thiazol-2-yl]carbonyl}pyrrolidine-3,4-diol

The compound obtained in Step 3 of Example 16 was reacted in the same way as in Step 4 of Example 1 using cis-pyrrolidine-3,4-diol instead of piperazin-2-one to give the title compound as a racemic mixture.
$^1$H-NMR ($CDCl_3$) δ: 0.94 (3H, d, J=7.1 Hz), 0.98 (3H, d, J=7.1 Hz), 1.83 (3H, s), 2.55-2.68 (1H, m), 3.54-3.62 (2H, m), 3.72-3.85 (2H, m), 4.26-4.34 (2H, m), 4.98 (1H, s), 6.64-6.73 (2H, m), 7.00-7.09 (6H, m).
MS (ESI) m/z: 532, 534.

Example 36

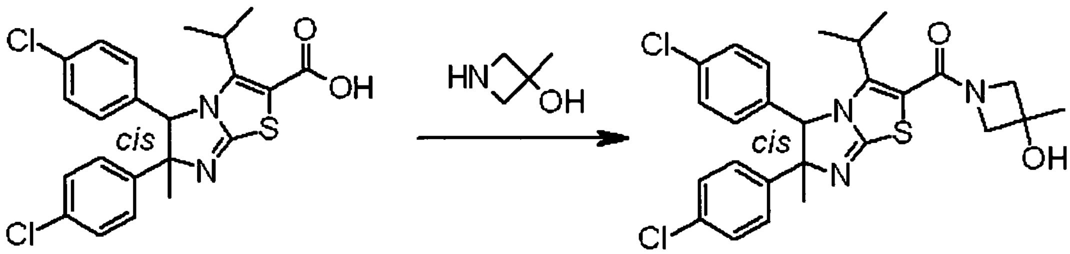

1-([(5R*,6S*)-5,6-Bis(4-chlorophenyl)-3-isopropyl-6-methyl-5,6-dihydroimidazo[2,1-b][1,3]thiazol-2-yl]carbonyl}-3-methylazetidin-3-ol

The compound obtained in Step 3 of Example 16 was reacted in the same way as in Step 4 of Example 1 using 3-methylazetidin-3-ol instead of piperazin-2-one to give the title compound as a racemic mixture.
$^1$H-NMR ($CDCl_3$) δ: 0.84 (3H, d, J=7.1 Hz), 1.02 (3H, d, J=7.1 Hz), 1.53 (3H, s), 1.83 (3H, s), 3.18-3.32 (1H, m), 4.04-4.23 (4H, m), 5.06 (1H, s), 6.60-6.77 (2H, m), 7.00-7.12 (6H, m).
MS (ESI) m/z: 516.

Example 37

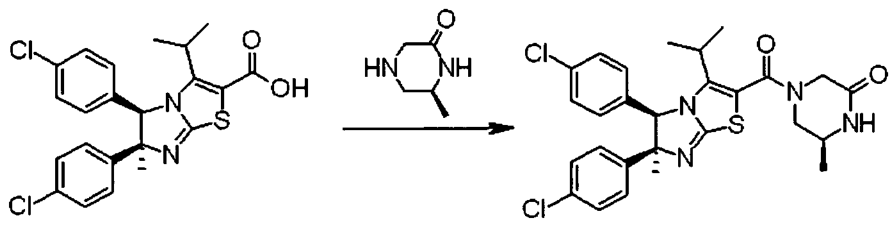

(6S)-4-{[(5R,6S)-5,6-Bis(4-chlorophenyl)-3-isopropyl-6-methyl-5,6-dihydroimidazo[2,1-b][1,3]thiazol-2-yl]carbonyl}-6-methylpiperazin-2-one

The same reaction as in Step 4 of Example 1 was performed using (6S)-6-methylpiperazin-2-one instead of piperazin-2-one to give the title compound.
$^1$H-NMR ($CDCl_3$) δ: 0.90 (3H, d, J=7.1 Hz), 1.04 (3H, d, J=7.1 Hz), 1.27 (3H, d, J=6.6 Hz), 1.83 (3H, s), 2.46-2.54 (1H, m), 3.08 (1H, dd, J=13.5, 8.9 Hz), 3.71-3.75 (1H, m), 4.05 (1H, d, J=18.1 Hz), 4.20 (1H, dd, J=13.5, 3.8 Hz), 4.44 (1H, d, J=18.3 Hz), 4.97 (1H, s), 6.01 (1H, brs), 6.71 (2H, d, J=7.8 Hz), 7.03 (2H, d, J=8. Hz) , 7.06 (2H, d, J=8.8 Hz), 7.09 (2H, d, J=8.8 Hz).
MS (ESI) m/z: 543.

Example 38

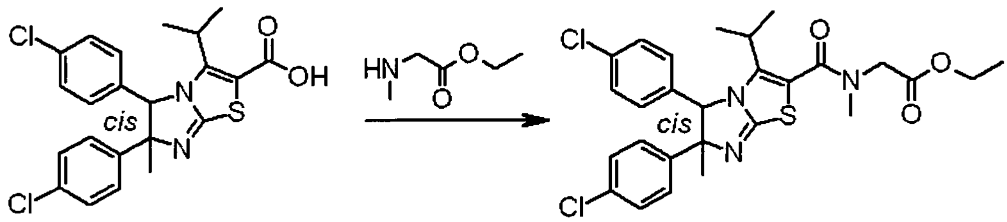

Ethyl N-{[(5R*,6S*)-5,6-bis(4-chlorophenyl)-3-isopropyl-6-methyl-5,6-dihydroimidazo[2,1-b][1,3]thiazol-2-yl]carbonyl}-N-methylglycinate

The compound obtained in Step 3 of Example 16 was reacted in the same way as in Step 4 of Example 1 using ethyl N-methylglycinate instead of piperazin-2-one to give the title compound as a racemic mixture.
$^1$H-NMR ($CDCl_3$) δ: 0.93 (3H, d, J=7.1 Hz), 1.01 (3H, d, J=7.1 Hz), 1.29 (3H, t, J=7.1 Hz), 1.82 (3H, s), 2.46-2.54 (1H, m), 3.15 (3H, s), 4.09-4.18 (2H, m), 4.21 (2H, q, J=7.2 Hz), 4.96 (1H, s), 6.71 (2H, d, J=7.6 Hz), 7.02 (2H, d, J=8.3 Hz), 7.04 (2H, d, J=8.1 Hz), 7.10 (2H, d, J=8.5 Hz).
MS (ESI) m/z: 546.

Example 39

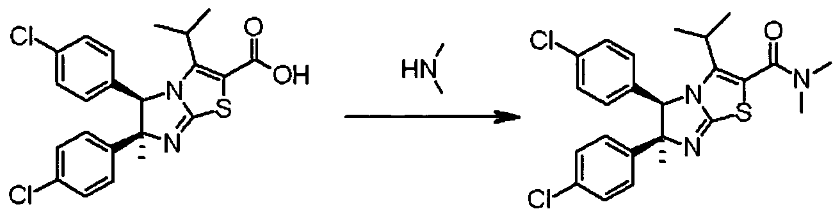

(5R,6S)-5,6-Bis(4-chlorophenyl)-3-isopropyl-N,N,6-trimethyl-5,6-dihydroimidazo[2,1-b][1,3]thiazole-2-carboxamide

The same reaction as in Step 4 of Example 1 was performed using dimethylamine instead of piperazin-2-one to give the title compound.
$^1$H-NMR ($CDCl_3$) δ: 0.90 (3H, d, J=7.1 Hz), 1.00 (3H, d, J=7.1 Hz), 1.81 (3H, s), 2.41-2.48 (1H, m), 3.07 (6H, s), 4.94 (1H, s), 6.71 (2H, d, J=7.6 Hz), 7.02 (2H, d, J=8.5 Hz), 7.04 (2H, d, J=8.5 Hz), 7.11 (2H, d, J=8.5 Hz).
MS (ESI) m/z: 474.

Example 40

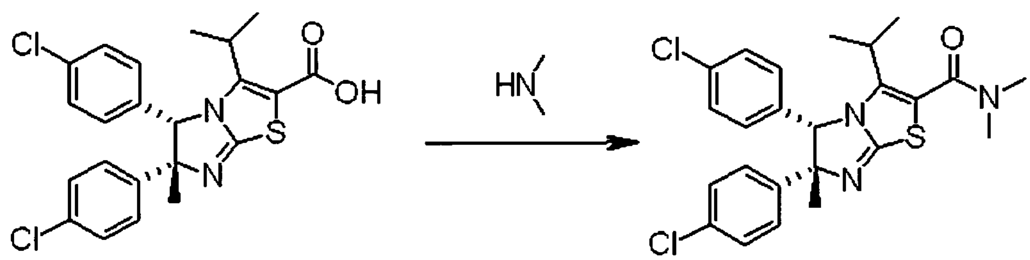

(5S,6R)-5,6-Bis(4-chlorophenyl)-3-isopropyl-N,N,6-trimethyl-5,6-dihydroimidazo[2,1-b][1,3]thiazole-2-carboxamide

The compound obtained in Step 3 of Example 34 was reacted in the same way as in Step 4 of Example 1 using dimethylamine instead of piperazin-2-one to give the title compound.
$^1$H-NMR ($CDCl_3$) δ: 0.90 (3H, d, J=7.1 Hz), 1.00 (3H, d, J=7.1 Hz), 1.81 (3H, s), 2.39-2.48 (1H, m), 3.07 (6H, s), 4.94 (1H, s), 6.71 (2H, d, J=7.1 Hz), 7.01-7.05 (4H, m), 7.11 (2H, d, J=8.1 Hz).
MS (ESI) m/z: 474.

Example 41

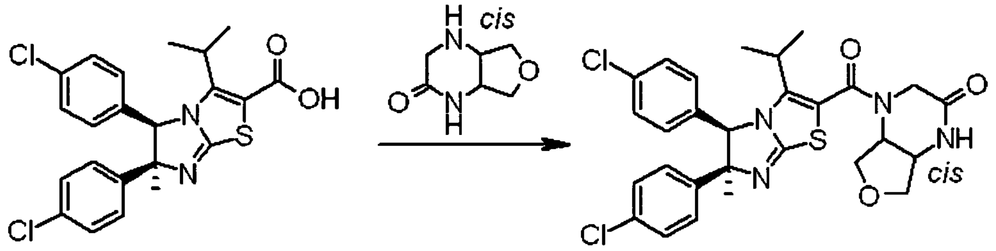

(4aR*,7aS*)-4-{[(5R,6S)-5,6-Bis(4-chlorophenyl)-3-isopropyl-6-methyl-5,6-dihydroimidazo[2,1-b][1,3]thiazol-2-yl]carbonyl}hexahydrofuro[3,4-b]pyrazin-2(1H)-one

The same reaction as in Step 4 of Example 1 was performed using (4aR*,7aS*)-hexahydrofuro[3,4-b]pyrazin-2(1H)-one instead of piperazin-2-one to give the title compound as a mixture of diastereomers.
$^1$H-NMR ($CDCl_3$) δ: 0.83-0.91 (3H, m), 1.00 (3H, dd, J=12.0, 7.1 Hz), 1.83 (3H, s), 2.42-2.52 (1H, m), 3.85-4.06 (5H, m), 4.14-4.21 (1H, m), 4.58 (1H, dd, J=17.6, 5.1 Hz), 4.97 (1H, d, J=3.4 Hz), 5.17-5.28 (1H, m), 6.07 (1H, brs), 6.64-6.77 (2H, m), 7.02-7.09 (6H, m).
MS (ESI) m/z: 571, 573.

Example 42

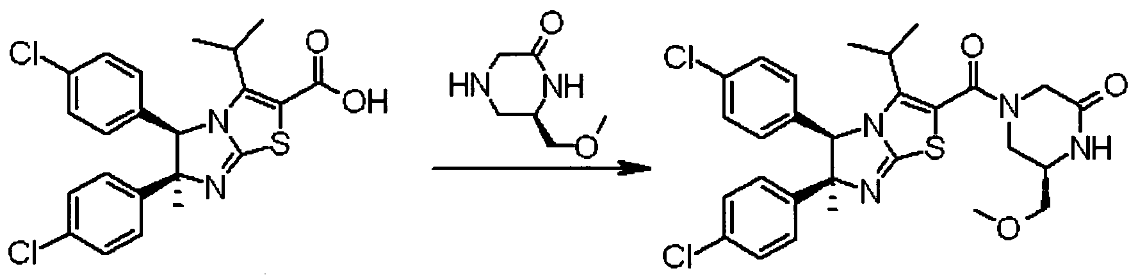

(6R)-4-{[(5R,6S)-5,6-Bis(4-chlorophenyl)-3-isopropyl-6-methyl-5,6-dihydroimidazo[2,1-b][1,3]thiazol-2-yl]carbonyl}-6-(methoxymethyl)piperazin-2-one

The same reaction as in Step 4 of Example 1 was performed using (6R)-6-(methoxymethyl)piperazin-2-one instead of piperazin-2-one to give the title compound.
$^1$H-NMR ($CDCl_3$) δ: 0.90 (3H, d, J=7.1 Hz), 1.03 (3H, d, J=7.1 Hz), 1.83 (3H, s), 2.46-2.54 (1H, m), 3.27-3.36 (2H, m), 3.38 (3H, s), 3.50 (1H, dd, J=9.4, 4.3 Hz), 3.75-3.80 (1H, m), 4.09 (1H, dd, J=13.3, 4.3 Hz), 4.14 (1H, d, J=18.1 Hz), 4.38 (1H, d, J=18.1 Hz), 4.98 (1H, s), 6.34 (1H, brs), 6.70 (2H, d, J=7.8 Hz), 7.04 (2H, d, J=8.8 Hz), 7.06 (2H, d, J=8.5 Hz), 7.09 (2H, d, J=8.8 Hz).
MS (ESI) m/z: 573.

Example 43

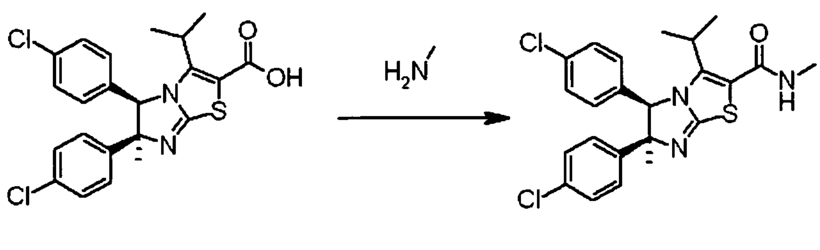

(5R,6S)-5,6-Bis(4-chlorophenyl)-3-isopropyl-N,6-dimethyl-5,6-dihydroimidazo[2,1-b][1,3]thiazole-2-carboxamide

The same reaction as in Step 4 of Example 1 was performed using methylamine instead of piperazin-2-one to give the title compound.
$^1$H-NMR ($CDCl_3$) δ: 0.83 (3H, d, J=7.1 Hz), 1.03 (3H, d, J=7.1 Hz), 1.81 (3H, s), 2.89 (3H, d, J=4.9 Hz), 3.35-3.42 (1H, m), 5.06 (1H, s), 5.45 (1H, brs), 6.67-6.70 (2H, m), 7.03 (2H, d, J=8.5 Hz), 7.03 (2H, d, J=8.8 Hz), 7.11 (2H, d, J=8.5 Hz).

Example 44

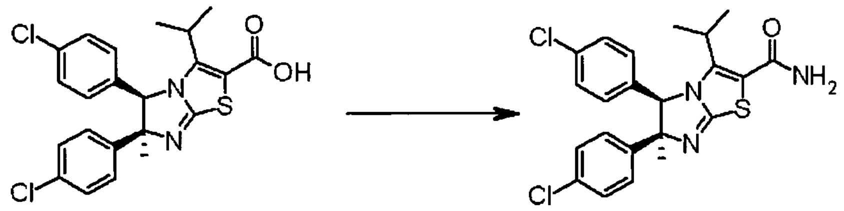

(5R,6S)-5,6-Bis(4-chlorophenyl)-3-isopropyl-6-methyl-5,6-dihydroimidazo[2,1-b][1,3]thiazole-2-carboxamide

The same reaction as in Step 4 of Example 1 was performed using ammonium chloride instead of piperazin-2-one to give the title compound.
$^1$H-NMR ($CDCl_3$) δ: 0.82 (3H, d, J=7.3 Hz), 1.04 (3H, d, J=7.1 Hz), 1.82 (3H, s), 3.39-3.46 (1H, m), 5.08 (1H, s), 5.37 (2H, brs), 6.68-6.73 (2H, m), 7.03 (2H, d, J=8.5 Hz), 7.04 (2H, d, J=8.5 Hz), 7.11 (2H, d, J=8.8 Hz).
MS (ESI) m/z: 446.

Example 45

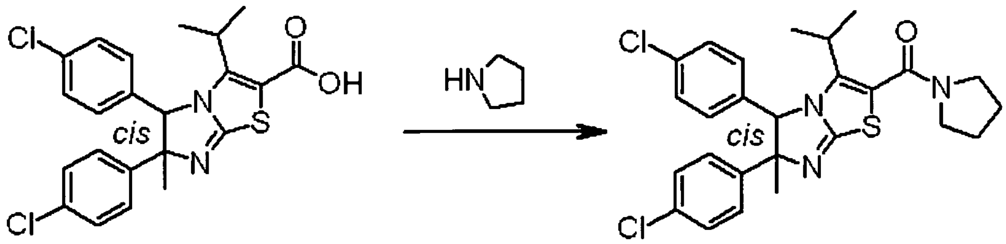

(5R*,6S*)-5,6-Bis(4-chlorophenyl)-3-isopropyl-6-methyl-2(pyrrolidin-1-ylcarbonyl)-5,6-dihydroimidazo[2,1-b][1,3]thiazole

The compound obtained in Step 3 of Example 16 was reacted in the same way as in Step 4 of Example 1 using pyrrolidine instead of piperazin-2-one to give the title compound as a racemic mixture.
$^1$H-NMR ($CDCl_3$) δ: 0.93 (3H, d, J=7.1 Hz), 0.99 (3H, d, J=7.1 Hz), 1.83 (3H, s), 1.90-1.98 (4H, m), 2.53-2.65 (1H, m), 3.47-3.61 (4H, m), 4.98 (1H, s), 6.65-6.75 (2H, m), 7.00-7.07 (4H, m), 7.09-7.13 (2H, m).
MS (ESI) m/z: 500.

Example 46

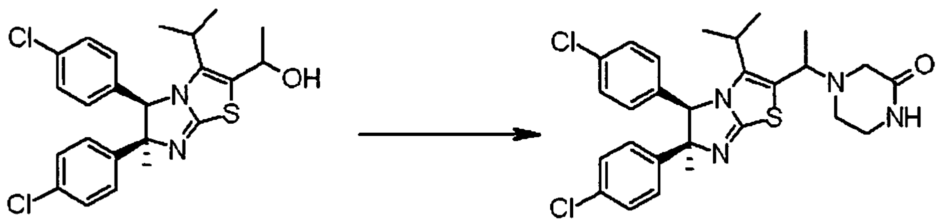

4-{1-[(5R,6S)-5,6-Bis(4-chlorophenyl)-3-isopropyl-6-methyl-5,6-dihydroimidazo[2,1-b][1,3]thiazol-2-yl]ethyl}piperazin-2-one

The high polarity isomer of the compound obtained in Step 2 of Example 29 was reacted with piperazin-2-one in the same way as in Example 18 to give the title compound. Low polarity isomer
$^1$H-NMR ($CDCl_3$) δ: 0.84 (3H, d, J=7.1 Hz), 0.91 (3H, d, J=7.1 Hz), 1.37 (3H, d, J=6.6 Hz), 1.81 (3H, s), 2.47-2.60 (2H, m), 2.94 (1H, m), 3.13 (1H, d, J=16.4 Hz), 3.33 (1H, m), 3.49-3.53 (3H, m), 4.89 (1H, s), 6.12 (1H, brs), 6.69 (2H, brs), 6.99-7.03 (4H, m), 7.12 (2H, d, J=9.0 Hz). MS (EI) m/z: 528, 530.
High polarity isomer
$^1$H-NMR ($CDCl_3$) δ: 0.83 (3H, d, J=7.1 Hz), 0.91 (3H, d, J=7.1 Hz), 1.36 (3H, d, J=6.3 Hz), 1.80 (3H, s), 2.46-2.53 (2H, m), 2.96 (1H, m), 3.07 (1H, d, J=16.4 Hz), 3.31 (1H, m), 3.41-3.50 (3H, m), 4.89 (1H, s), 6.01 (1H, brs), 6.70 (2H, brs), 7.00-7.03 (4H, m), 7.11-7.13 (2H, m).
MS (EI) m/z: 528, 530.

Example 47

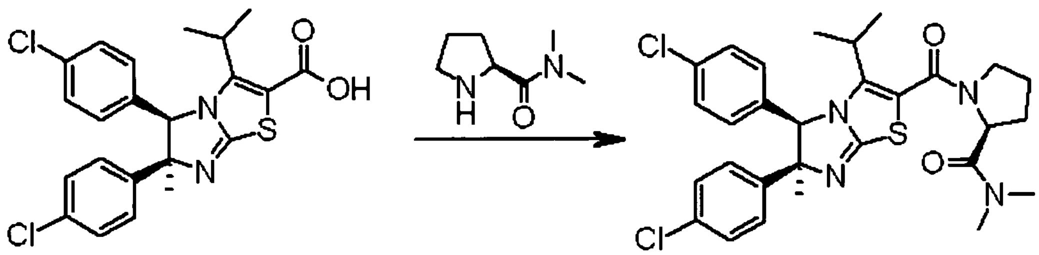

1-{[(5R,6S)-5,6-Bis(4-chlorophenyl)-3-isopropyl-6-methyl-5,6-dihydroimidazo[2,1-b][1,3]thiazol-2-yl]carbonyl}-N,N-dimethyl-L-prolinamide

The same reaction as in Step 4 of Example 1 was performed using N,N-dimethyl-L-prolinamide instead of piperazin-2-one to give the title compound.
$^1$H-NMR ($CDCl_3$) δ: 0.97 (6H, d, J=6.6 Hz), 1.83 (3H, s), 1.88-2.00 (2H, m), 2.08-2.29 (2H, m), 2.60-2.72 (1H, m), 2.95 (3H, s), 3.12 (3H, s), 3.61-3.84 (2H, m), 4.86-4.94 (1H, m), 4.99 (1H, s), 6.65-6.74 (2H, m), 7.08-7.13 (4H, m), 7.09-7.12 (2H, m).
MS (ESI) m/z: 571.

Example 48

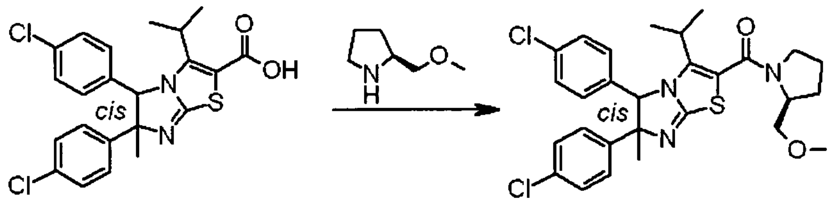

(5R*,6S*)-5,6-Bis (4-chlorophenyl)-3-isopropyl-2-{[(2S)-2-(methoxymethyl)pyrrolidin-1-yl]carbonyl}-6-methyl-5,6-dihydroimidazo[2,1-b][1,3]thiazole

The compound obtained in Step 3 of Example 16 was reacted in the same way as in Step 4 of Example 1 using (2S)-2-(methoxymethyl)pyrrolidine instead of piperazin-2-one to give the title compound as a mixture of diastereomers.
$^1$H-NMR ($CDCl_3$) δ: 0.92-1.04 (6H, m), 1.81 (3H, s), 1.82-1.87 (1H, m), 1.97-2.04 (3H, m), 2.50-2.61 (1H, m), 3.32 (3H, s), 3.42-3.68 (4H, m), 4.24-4.30 (1H, m), 4.96 (1H, m), 6.70 (2H, d, J=7.8 Hz), 7.01-7.04 (4H, m), 7.09-7.12 (2H, m).
MS (FAB) m/z: 544.

Example 49

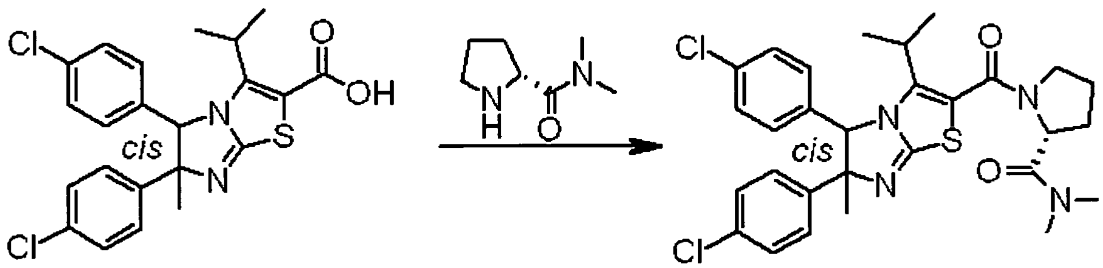

1-{[(5R*,6S*)-5,6-Bis(4-chlorophenyl)-3-isopropyl-6-methyl-5,6-dihydroimidazo[2,1-b][1,3]thiazol-2-yl]pyrrolidine}-N,N-dimethyl-D-prolinamide

The compound obtained in Step 3 of Example 16 was reacted in the same way as in Step 4 of Example 1 using N,N-dimethyl-D-prolinamide instead of piperazin-2-one to give the title compound as a mixture of diastereomers.
$^1$H-NMR ($CDCl_3$) δ: 0.91-0.99 (6H, m), 1.81 (3H, s), 1.89-1.98 (2H, m), 2.13-2.26 (2H, m), 2.65-2.74 (1H, m), 2.95 (3H, s), 3.12 (3H, m), 3.69-3.83 (2H, m), 4.89 (1H, m), 4.97 (1H, m), 6.68-6.71 (2H, m), 7.01-7.06 (4H, m), 7.10-7.13 (2H, m).
MS (FAB) m/z: 571.

Example 50

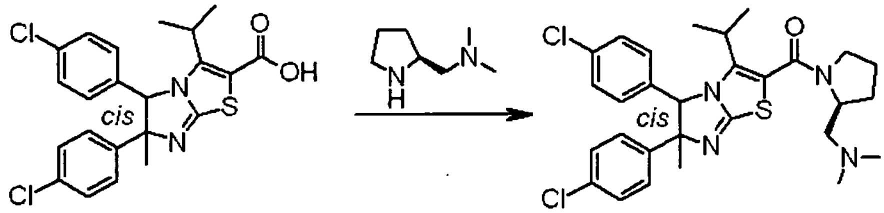

1-((2S)-1-{[(5R*,6S*)-5,6-Bis(4-chlorophenyl)-3-isopropyl-6-methyl-5,6-dihydroimidazo[2,1-b][1,3]thiazol-2-yl]carbonyl}pyrrolidin-2-yl)-N,N-dimethylmethanamine

The compound obtained in Step 3 of Example 16 was reacted in the same way as in Step 4 of Example 1 using N,N-dimethyl-1-[(2S)-pyrrolidin-2-yl]methanamine instead of piperazin-2-one to give the title compound as a mixture of diastereomers.
$^1$H-NMR ($CDCl_3$) δ: 0.90-1.04 (6H, m), 1.83-2.07 (4H, m), 1.94 (3H, m), 2.19-2.27 (1H, m), 2.28 (6H, s), 2.52-2.64 (2H, m), 3.47-3.63 (2H, m), 4.26-4.30 (1H, m), 4.96 (1H, m), 7.09-7.12 (2H, m), 6.70 (2H, d, J=8.1 Hz), 7.01-7.04 (4H, m).
MS (FAB) m/z: 557.

Example 51

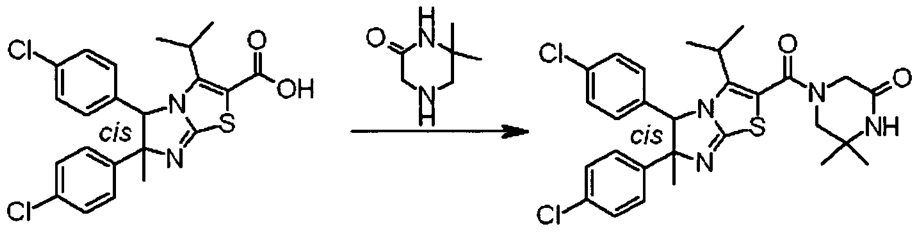

4-{[(5R*6S*)-5,6-Bis(4-chlorophenyl)-3-isopropyl-6-methyl-5,6-dihydroimidazo[2,1-b][1,3]thiazol-2-yl]carbonyl}-6,6-dimethylpiperazin-2-one

The compound obtained in Step 3 of Example 16 was reacted in the same way as in Step 4 of Example 1 using 6,6-dimethylpiperazin-2-one instead of piperazin-2-one to give the title compound as a racemic mixture.
$^1$H-NMR ($CDCl_3$) δ: 0.92 (3H, d, J=7.1 Hz), 1.00 (3H, d, J=7.1 Hz), 1.32 (6H, s), 1.83 (3H, s), 2.48-2.56 (1H, m), 3.58 (1H, d, J=13.2 Hz), 3.69 (1H, d, J=13.2 Hz), 4.20-4.29 (2H, m), 4.97 (1H, s), 6.34 (1H, brs), 6.71 (2H, d, J=7.3 Hz), 7.03 (2H, d, J=8.8 Hz), 7.06 (2H, d, J=9.8 Hz), 7.09 (2H, d, J=8.8 Hz).
MS (ESI) m/z: 557.

Example 52

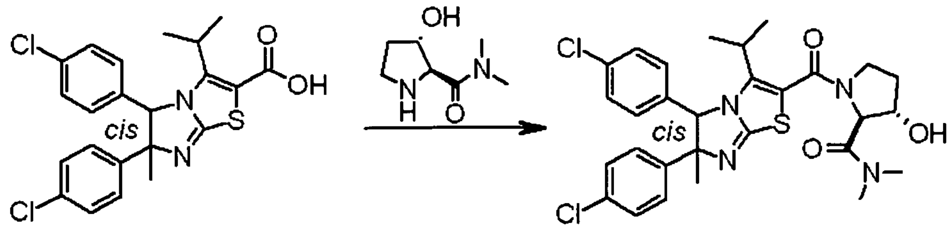

(3S)-1-{[(5R*,6S*)-5,6-Bis(4-chlorophenyl)-3-isopropyl-6-methyl-5,6-dihydroimidazo[2,1-b][1,3]thiazol-2-yl]carbonyl-3-hydroxy-N,N-dimethyl-L-prolinamide

The compound obtained in Step 3 of Example 16 was reacted in the same way as in Step 4 of Example 1 using (3S)-3-hydroxy-N,N-dimethyl-L-prolinamide instead of piperazin-2-one to give the title compound as a mixture of diastereomers.
$^1$H-NMR ($CDCl_3$) δ: 0.85-1.00 (6H, m), 1.81-1.81 (3H, m), 1.98-2.01 (1H, m), 2.27-2.30 (1H, m), 2.59-2.62 (1H, m), 2.93 (3H, s), 3.14-3.16 (3H, m), 3.75-4.04 (2H, m), 4.35 (1H, brs), 4.84 (1H, brs), 4.99 (1H, brs), 6.65-6.69 (2H, m), 7.01-7.09 (6H, m).
MS (FAB) m/z: 587.

Example 53

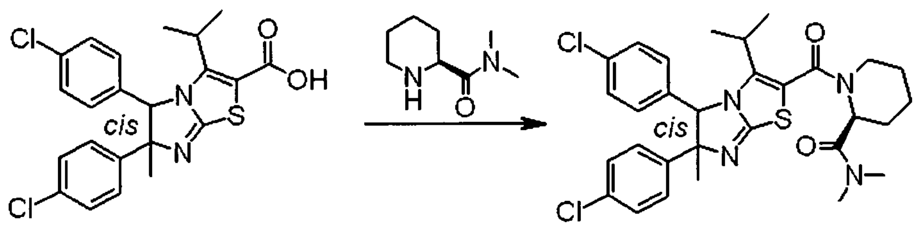

(2S)-1-{[(5R*,6S*)-5,6-Bis(4-chlorophenyl)-3-isopropyl-6-methyl-5,6-dihydroimidazo[2,1-b][1,3]thiazol-2-yl]carbonyl}-N,N-dimethylpiperidine-2-carboxamide

The compound obtained in Step 3 of Example 16 was reacted in the same way as in Step 4 of Example 1 using (2S)-N,N-dimethylpiperidine-2-carboxamide instead of piperazin-2-one to give the title compound as a mixture of diastereomers.
$^1$H-NMR ($CDCl_3$) δ: 0.89-1.00 (6H, m), 1.41-1.51 (1H, m), 1.66-1.97 (8H, m), 2.40-2.48 (1H, m), 2.88-3.07 (6H, m), 3.87-3.79 (1H, m), 4.01 (1H, s), 4.95 (1H, d, J=1.5 Hz), 5.34 (1H, d, J=13.7 Hz), 6.71 (2H, d, J=7.8 Hz), 7.01-7.12 (6H, m).
MS (FAB) m/z: 585.

Example 54

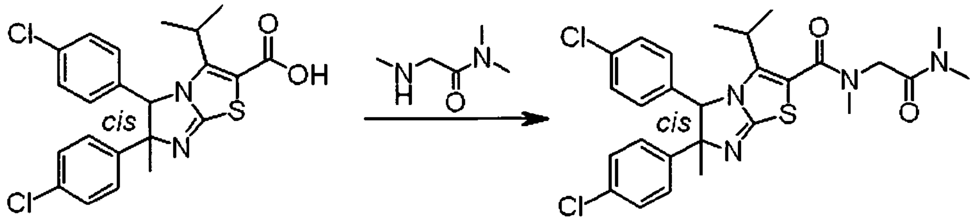

(5R*,6S*)-5,6-Bis(4-chlorophenyl)-N-[2-(dimethylamino)-2-oxoethyl]-3-isopropyl-N,6-dimethyl-5,6-dihydroimidazo [2,1-b][1,3]thiazole-2-carboxamide

The compound obtained in Step 3 of Example 16 was reacted in the same way as in Step 4 of Example 1 using N,N,$N^2$-trimethylglycinamide instead of piperazin-2-one to give the title compound as a racemic mixture.
$^1$H-NMR ($CDCl_3$) δ: 0.97 (3H, d, J=7.1 Hz), 1.00 (3H, d, J=6.8 Hz), 1.82 (3H, s), 2.52-2.57 (1H, m), 2.96 (3H, s), 3.01 (3H, s), 3.18 (3H, s), 4.18 (1H, d, J=17.1 Hz), 4.24 (1H, d, J=16.6 Hz), 4.96 (1H, s), 6.71 (2H, d, J=8.1 Hz), 7.02 (2H, d, J=8.5 Hz), 7.03 (2H, d, J=8.5 Hz), 7.11 (2H, d, J=8.5 Hz).
MS (ESI) m/z: 545.

Example 55

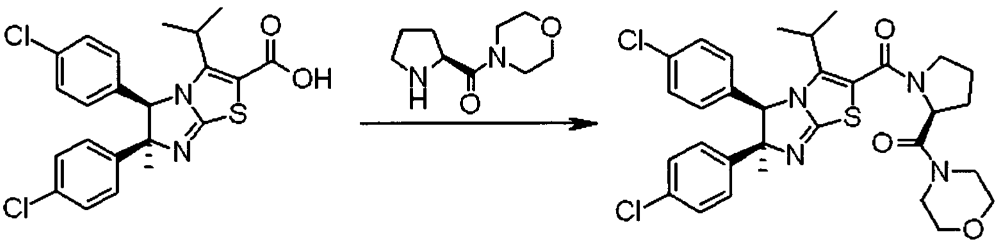

(5R,6S)-5,6-Bis(4-chlorophenyl)-3-isopropyl-6-methyl-2-{[(2S)-2-(morpholin-4-ylcarbonyl)pyrrolidin-1-yl]carbonyl}-5,6-dihydroimidazo[2,1-b][1,3]thiazole

The same reaction as in Step 4 of Example 1 was performed using 4-(L-prolyl)morpholine instead of piperazin-2-one to give the title compound.
$^1$H-NMR ($CDCl_3$) δ: 0.96 (6H, dd, J=7.1, 1.5 Hz), 1.80 (3H, s), 1.91-1.98 (2H, m), 2.15-2.21 (2H, m), 2.62-2.69 (1H, m), 3.53-3.80 (10H, m), 4.89 (1H, dd, J=7.6, 4.6 Hz), 4.94 (1H, s), 6.70 (2H, d, J=8.3 Hz), 7.01 (2H, d, J=8.5 Hz), 7.02 (2H, d, J=8.3 Hz), 7.10 (2H, d, J=8.5 Hz).
MS (ESI) m/z: 613.

Example 56

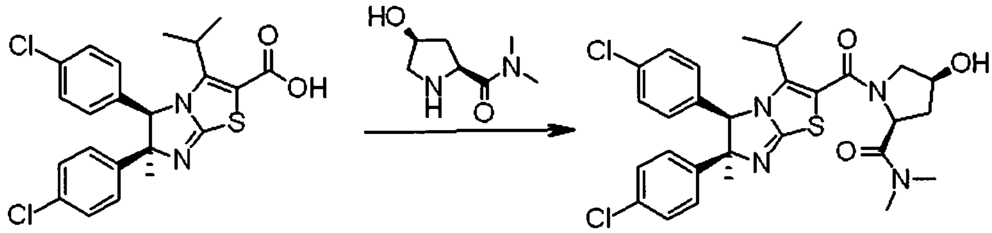

(4S)-1-{[(5R,6S)-5,6-Bis(4-chlorophenyl)-3-isopropyl-6-methyl-5,6-dihydroimidazo[2,1-b][1,3]thiazol-2-yl]carbonyl}-N, N-dimethyl-4-hydroxy-L-prolinamide

The same reaction as in Step 4 of Example 1 was performed using (4S)-N,N-dimethyl-4-hydroxy-L-prolinamide instead of piperazin-2-one to give the title compound.
$^1$H-NMR ($CDCl_3$) δ: 0.88 (3H, d, J=7.1 Hz), 0.96 (3H, t, J=7.1 Hz), 1.82 (3H, s), 2.03 (1H, d, J=13.9 Hz), 2.27-2.34 (1H, m), 2.51-2.58 (1H, m), 3.00 (3H, s), 3.27 (3H, s), 3.76 (1H, dd, J=11.5, 4.1 Hz), 3.91 (1H, d, J=11.5 Hz), 4.39-4.44 (1H, m), 4.94 (1H, d, J=9.5 Hz), 4.97 (1H, s), 6.02 (1H, s), 6.71 (2H, brd, J=8.5 Hz), 7.04 (4H, d, J=8.5 Hz), 7.10 (2H, d, J=8.5 Hz).
MS (FAB) m/z: 587.

Example 57

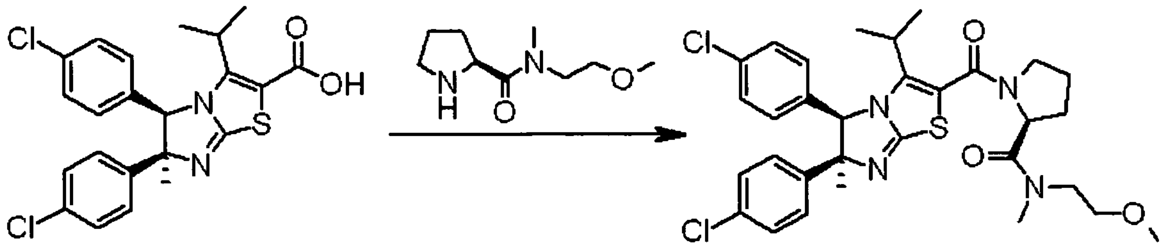

1-{[(5R,6S)-5,6-Bis(4-chlorophenyl)-3-isopropyl-6-methyl-5,6-dihydroimidazo[2,1-b][1,3]thiazol-2-yl]carbonyl}-N-(2-methoxyethyl)-N-methyl-L-prolinamide
The same reaction was performed as in Step 4 of Example 1 using N-(2-methoxyethyl)-N-methyl-L-prolinamide instead of piperazin-2-one to give the title compound.
$^1$H-NMR ($CDCl_3$) δ: 0.92-0.99 (6H, m), 1.81 (3H, s), 1.89-1.94 (1H, m), 2.15-2.22 (1H, m), 2.59-2.65 (1H, m), 2.96 (1H, brs), 3.18 and 3.32 (total 3H, each s), 3.37-3.40 (2H, m), 3.52 and 3.58 (total 2H, each t, J=5.2 and 5.6 Hz), 3.67-3.72 and 3.76-3.80 (total 2H, each m), 4.87-4.90 (1H, m), 4.950 and 4.954 (total 1H, each s), 6.69 (2H, d, J=7.3 Hz), 7.01-7.04 (4H, m), 7.11 (2H, d, J=8.5 Hz).
MS (ESI) m/z: 615.

Example 58

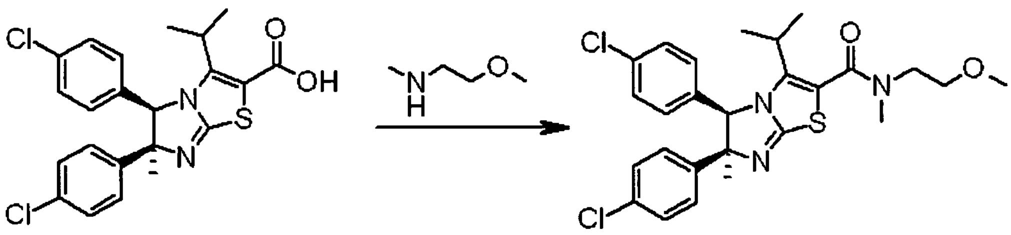

(5R,6S)-5,6-Bis(4-chlorophenyl)-3-isopropyl-N-(2-methoxyethyl)-N,6-dimethyl-5,6-dihydroimidazo[2,1-b][1,3]thiazole-2-carboxamide

The same reaction was performed as in Step 4 of Example 1 using 2-methoxy-N-methylethanamine instead of piperazin-2-one to give the title compound.
$^1$H-NMR ($CDCl_3$) δ: 0.91 (3H, d, J=7.1 Hz), 1.01 (3H, d, J=7.1 Hz), 1.81 (3H, s), 2.40-2.46 (1H, m), 3.13 (3H, s), 3.33 (3H, s), 3.56-3.70 (4H, m), 4.94 (1H, s), 6.71 (2H, d, J=8.1 Hz), 7.02 (2H, d, J=8.3 Hz), 7.03 (2H, d, J=8.8 Hz), 7.11 (2H, d, J=8.5 Hz).
MS (ESI) m/z: 518.

Example 59

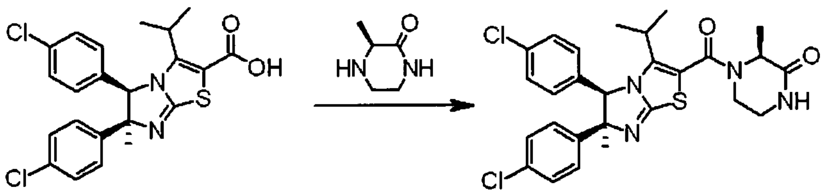

(3S)-4-{[(5R,6S)-5,6-Bis(4-chlorophenyl)-3-isopropyl-6-methyl-5,6-dihydroimidazo[2,1-b][1,3]thiazol-2-yl]carbonyl}-3-methylpiperazin-2-one

The same reaction as in Step 4 of Example 1 was performed using (3S)-3-methylpiperazin-2-one instead of piperazin-2-one to give the title compound.
$^1$H-NMR ($CDCl_3$) δ: 0.88 (3H, d, J=7.1 Hz), 1.11 (3H, d, J=7.1 Hz), 1.58 (3H, s), 1.82 (3H, s), 2.35-2.46 (1H, m), 3.31-3.42 (2H, m), 3.53-3.63 (1H, m), 4.23-4.34 (1H, m), 4.78-4.87 (1H, m), 4.95 (1H, s), 5.83 (1H, brs), 6.67-6.75 (2H, m), 6.99-7.13 (6H, m).
MS (ESI) m/z: 543.

Example 60

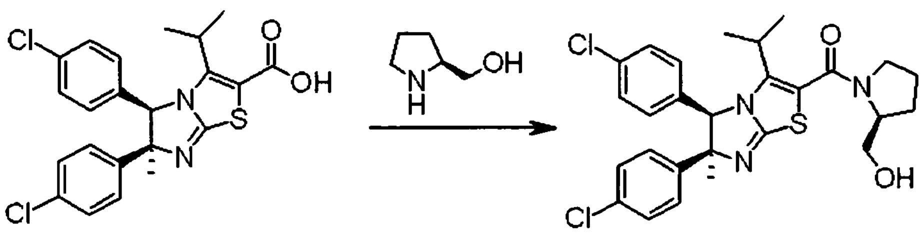

((2S)-1-{[(5R,6S)-5,6-Bis(4-chlorophenyl)-3-isopropyl-6-methyl-5,6-dihydroimidazo[2,1-b][1,3]thiazol-2-yl]carbonyl} pyrrolidin-2-yl)methanol

The same reaction as in Step 4 of Example 1 was performed using (2S)-pyrrolidin-2-ylmethanol instead of piperazin-2-one to give the title compound.
$^1$H-NMR ($CDCl_3$) δ: 0.93 (3H, d, J=7.1 Hz), 1.04 (3H, d, J=7.1 Hz), 1.68-1.86 (2H, m), 1.82 (3H, s), 1.93-2.02 (1H, m), 2.08-2.16 (1H, m), 2.49-2.57 (1H, m), 3.55-3.74 (4H, m), 4.21-4.27 (1H, m), 4.41 (1H, brs), 4.96 (1H, s), 6.70 (2H, brd, J=8.5 Hz), 7.02 (2H, d, J=8.5 Hz), 7.04 (2H, d, J=8.5 Hz), 7.11 (2H, d, J=8.5 Hz).
MS (FAB) m/z: 530.

Example 61

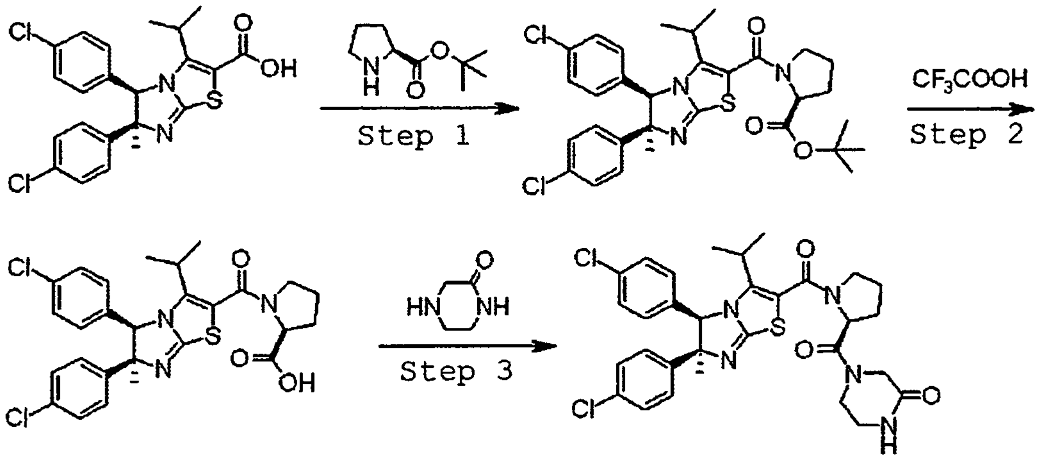


Step 1:tert-Butyl 1-{[(5R,6S)-5,6-Bis(4-chlorophenyl)-3-isopropyl-6-methyl-5,6-dihydroimidazo[2,1-b][1,3]thiazol-2-yl] carbonyl}-L-prolinate

The same reaction as in Step 4 of Example 1 was performed using tert-butyl L-prolinate instead of piperazin-2-one to give the title compound.
$^1$H-NMR ($CDCl_3$) δ: 0.94 (3H, d, J=7.1 Hz), 0.99 (3H, d, J=6.8 Hz), 1.46 (9H, s), 1.81 (3H, s), 1.93-2.04 (3H, m), 2.23-2.28 (1H, m), 2.58-2.63 (1H, m), 3.61-3.66 (1H, m), 3.71-3.76 (1H, m), 4.41-4.45 (1H, m), 4.96 (1H, s), 6.70 (2H, d, J=8.3 Hz), 7.02 (2H, d, J=8.3 Hz), 7.03 (2H, d, J=8.5 Hz), 7.11 (2H, d, J=8.5 Hz).

Step 2: 1-{[(5R,6S)-5,6-Bis(4-chlorophenyl)-3-isopropyl-6-methyl-5,6-dihydroimidazo[2,1-b][1,3]thiazol-2-yl]carbonyl}-L-proline

The compound (1.34 g, 2.23 mmol) obtained in Step 1 above was dissolved in trifluoroacetic acid (10 ml) and the resulting mixture was stirred at room temperature for 1 hour. Then the temperature thereof was warmed to 70°C and the mixture was stirred under heating for 2 hours. The reaction mixture was returned to room temperature and the reaction solvent was evaporated under reduced pressure. A 4 N hydrochloric acid-dioxane solution (10 ml) was added to the residue and after the resulting mixture was stirred at room temperature for 10 minutes, the reaction solvent was evaporated under reduced pressure. Ethanol and diethyl ether were added to the residue and the solidified mixture was dried at 60°C under reduced pressure to give the title compound (1.23 g, 100%) as a colorless solid.
$^1$H-NMR ($CDCl_3$) δ: 0.79 (3H, d, J=7.1 Hz), 1.01 (3H, d, J=6.1 Hz), 1.89-1.95 (3H, m), 2.04 (3H, s), 2.22-2.27 (1H, m), 2.63-2.68 (1H, m), 3.56-3.62 (2H, m), 4.30-4.34 (1H, m), 6.14 (1H, s), 7.20-7.26 (8H, m).
MS (ESI) m/z: 544.

Step 3: 4-(1-{[(5R,6S)-5,6-Bis(4-chlorophenyl)-3-isopropyl-6-methyl-5,6-dihydroimidazo[2,1-b][1,3]thiazol-2-yl]carbonyl}-L-prolyl)piperazin-2-one

The compound obtained in Step 2 above was reacted with piperazin-2-one in the same way as in Step 4 of Example 1 to give the title compound.
$^1$H-NMR ($CDCl_3$) δ: 0. 94 (3H, d, J=7.1 Hz), 0. 96 (3H, d, J=7.3 Hz), 1.81 (3H, s), 1.92-2.00 (2H, m), 2.16-2.25 (2H, m), 2.60-2.66 (1H, m), 3.36-3.44 (2H, m), 3.65-3.96 (4H, m), 4.09-4.17 (1H, m), 4.42 (1H, t, J=18.0 Hz), 4.78-4.89 (1H, m), 4.96 (1H, s), 6.14 (1H, brs), 6.70 (2H, d, J=7.8 Hz), 7.02 (2H, d, J=8.8 Hz), 7.03 (2H, d, J=8.5 Hz), 7.10 (2H, d, J=8.5 Hz).
MS (ESI) m/z: 626.

Example 62

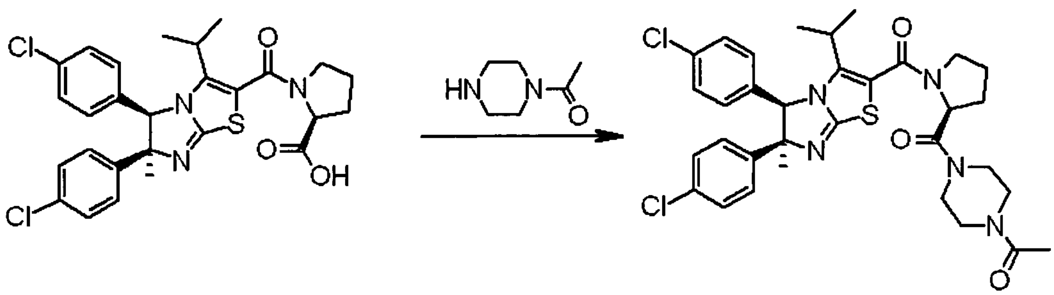

(5R,6S)-2-({(2S)-2-[(4-Acetylpiperazine-1-yl)carbonyl]pyrrolidin-1-yl}carbonyl)-5,6-bis(4-chlorophenyl)-3-isopropyl-6-methyl-5,6-dihydroimidazo[2,1-b][1,3]thiazole

The compound obtained in Step 2 of Example 61 was reacted in the same way as in Step 4 of Example 1 using 1-acetylpiperazine instead of piperazin-2-one to give the title compound.
$^1$H-NMR ($CDCl_3$) δ: 0.96 (6H, t, J=6.6 Hz), 1.81 (3H, s), 1.92-2.01 (2H, m), 2.12 (3H, s), 2.17-2.24 (2H, m), 2.61-2.67 (1H, m), 3.45-3.55 (4H, m), 3.70-3.83 (6H, m), 4.88-4.91 (1H, m), 4.96 (1H, s), 6.70 (2H, d, J=8.3 Hz), 7.02 (2H, d, J=8.8 Hz), 7.03 (2H, d, J=8.8 Hz), 7.10 (2H, d, J=8.8 Hz).
MS (ESI) m/z: 654.

Example 63

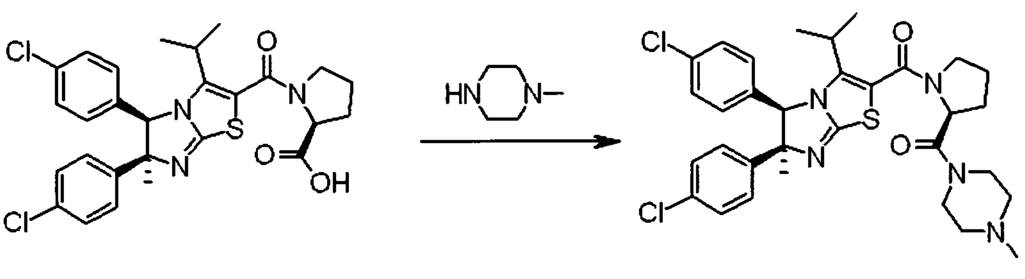

(5R,6S)-5,6-Bis(4-chlorophenyl)-3-isopropyl-6-methyl-2-({(2S)-2-[(4-methylpiperazin-1-yl)carbonyl]pyrrolidin-1-yl}carbonyl)-5,6-dihydroimidazo[2,1-b][1,3]thiazole

The compound obtained in Step 2 of Example 61 was reacted in the same way as in Step 4 of Example 1 using 1-methylpiperazine instead of piperazin-2-one to give the title compound.
$^1$H-NMR ($CDCl_3$) δ: 0.96 (6H, d, J=7.1 Hz), 1.81 (3H, s), 1.91-1.95 (2H, m), 2.16-2.22 (2H, m), 2.31 (3H, s), 2.37-2.42

(2H, m), 2.55-2.65 (1H, m), 3.49-3.77 (8H, m), 4.90-4.93 (1H, m), 4.95 (1H, s), 6.70 (2H, d, J=7.1 Hz), 7.02 (2H, d, J=8.8 Hz), 7.03 (2H, d, J=8.8 Hz), 7.11 (2H, d, J=8.8 Hz).
MS (ESI) m/z: 626.

Example 64

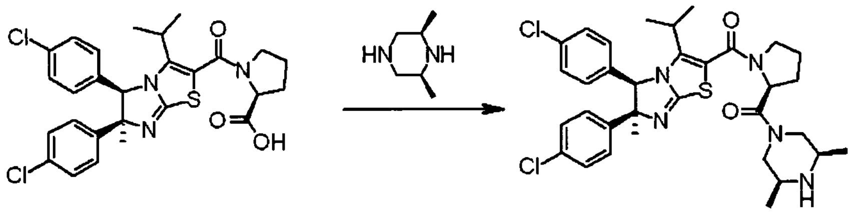

(5R,6S)-5,6-Bis(4-chlorophenyl)-2-[((2S)-2-{[(3R,5S)-3,5-dimethylpiperazin-1-yl]carbonyl}pyrrolidin-1-yl)carbonyl]-3-isopropyl-6-methyl-5,6-dihydroimidazo[2,1-b][1,3]thiazole

The compound obtained in Step 2 of Example 61 was reacted in the same way as in Step 4 of Example 1 using cis-2,6-dimethylpiperazine instead of piperazin-2-one to give the title compound.
$^1$H-NMR ($CDCl_3$) δ: 0.96 (3H, d, J=7.3 Hz), 0.97 (3H, d, J=6.8 Hz), 1.05-1.11 (6H, m), 1.27 (1H, brs), 1.81 (3H, s), 1.90-1.95 (2H, m), 2.11-2.24 (3H, m), 2.66-3.02 (4H, m), 3.70-3.81 (3H, m), 4.45 (1H, d, J=11.7 Hz), 4.87-4.91 (1H, m), 4.95 (1H, s), 6.70 (2H, d, J=7.3 Hz), 7.02 (2H, d, J=8.5 Hz), 7.03 (2H, d, J=7.8 Hz), 7.11 (2H, d, J=8.5 Hz).
MS (ESI) m/z: 640.

Example 65

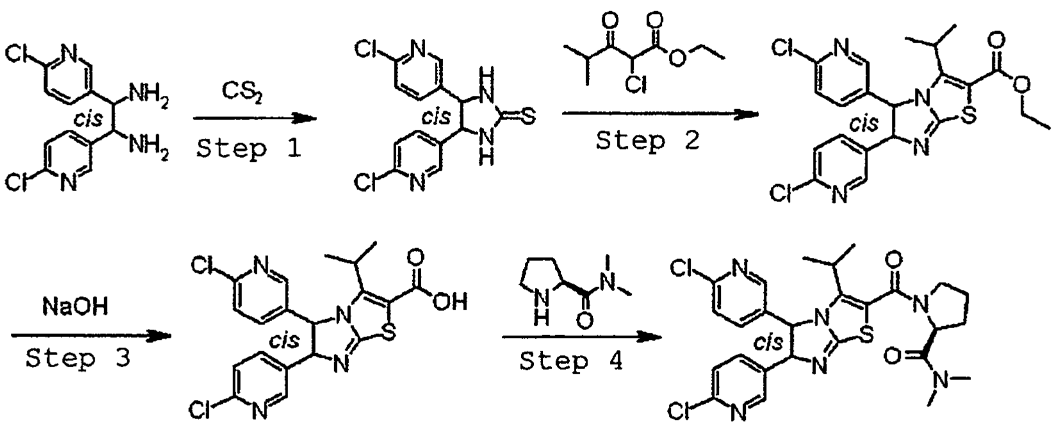


Step 1: 4,5-cis-4,5-Bis(6-chloropyridin-3-yl)imidazolidine-2-thione

meso-1,2-Bis(6-chloropyridin-3-yl)ethane-1,2-diamine was reacted in the same way as in Step 1 of Example 2 to give the title compound.
$^1$H-NMR (DMSO-$d_6$) δ: 5.48 (2H, s), 7.33-7.44 (4H, m), 7.98-7.99 (2H, m), 8.96 (2H, s).
MS (ESI) m/z: 325.

Step 2: Ethyl (5R*,6S*)-5,6-Bis(6-chloropyridin-3-yl)-3-isopropyl-5,6-dihydroimidazo[2,1-b][1,3]thiazole-2-carboxylate

The compound obtained in Step 1 above was reacted with ethyl 2-chloro-4-methyl-3-oxopentanoate in the same way as in Step 1 of Example 4 to give the title compound.

$^1$H-NMR ($CDCl_3$) δ: 0.91 (3H, d, J=7. Hz), 1.08 (3H, d, J=7.1 Hz), 1.34 (3H, t, J=7.1 Hz), 3.39-3.46 (1H, m), 4.27 (2H, q, J=7.1 Hz), 5.62 (1H, d, J=9.3 Hz), 6.01 (1H, d, J=9.3 Hz), 7.02-7.17 (3H, m), 7.33-7.35 (1H, m), 7.86 (1H, d, J=2.4 Hz), 8.18 (1H, d, J=2.4 Hz).
MS (ESI) m/z: 463.

Step 3: (5R*,6S*)-5,6-Bis(6-chloropyridin-3-yl)-3-isopropyl-5,6-dihydroimidazo[2,1-b][1,3]thiazole-2-carboxylic acid

The compound obtained in Step 2 above was reacted in the same way as in Step 3 of Example 1 to give the title compound.
MS (ESI) m/z: 435.

Step 4: 1-{[(5R*,6S*)-5,6-Bis(6-chloropyridin-3-yl)-3-isopropyl-5,6-dihydroimidazo[2,1-b][1,3]thiazol-2-yl]carbonyl}-N,N-dimethyl-L-prolinamide

The compound obtained in Step 3 above was reacted in the same way as in Step 4 of Example 1 using N,N-dimethyl-L-prolinamide instead of piperazin-2-one to give the title compound.
$^1$H-NMR ($CDCl_3$) δ: 0.91-1.10 (6H, m), 1.90-2.00 (2H, m), 2.05-2.29 (2H, m), 2.65-2.85 (1H, m), 2.96 (3H, s), 3.13 (3H, s), 3.69-3.83 (2H, m), 4.88-4.92 (1H, m), 5.46-5.50 (1H, m), 5.97-6.00 (1H, m), 7.01-7.14 (3H, m), 7.31-7.34 (1H, m), 7.80-7.83 (1H, m), 8.17-8.18 (1H, m).
MS (FAB) m/z: 559.

Example 66

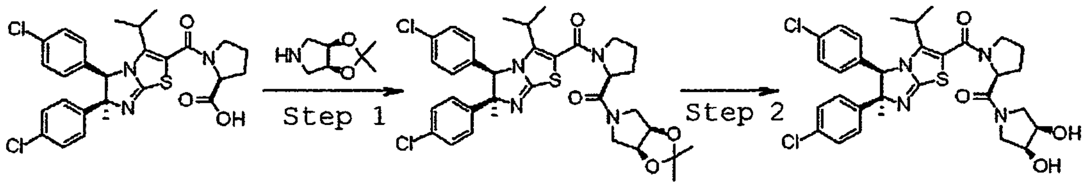


Step 1: (3aR,6aS)-5-(1-{[(5R,6S)-5,6-Bis(4-chlorophenyl)-3-isopropyl-6-methyl-5,6-dihydroimidazo[2,1-b][1,3]thiazol-2-yl]carbonyl}-L-prolyl)-2,2-dimethyltetrahydro-3aH-[1,3]dioxolo[4,5-c]pyrrole

The compound obtained in Step 2 of Example 61 was reacted in the same way as in Step 4 of Example 1 using (3aR,6aS)-2,2-dimethyltetrahydro-3aH-[1,3]dioxolo[4,5-c]pyrrole instead of piperazin-2-one to give the title compound.
$^1$H-NMR ($CDCl_3$) δ: 0.94 (6H, d, J=6.8 Hz), 1.33 (3H, s), 1.45 (3H, s), 1.52 (1H, d, J=6.3 Hz), 1.81 (3H, s), 1.90-1.96 (1H, m), 2.03-2.09 (1H, m), 2.15-2.26 (2H, m), 2.60-2.66 (1H, m), 3.36-3.40 (1H, m), 3.67-3.80 (3H, m), 3.99 (1H, d, J=13.9 Hz), 4.62-4.66 (1H, m), 4.74 (1H, t, J=5.1 Hz), 4.78-4.82 (1H, m), 4.95 (1H, s), 6.69 (2H, d, J=7.6 Hz), 7.02 (2H, d, J=8.8 Hz), 7.02 (2H, d, J=8.8 Hz), 7.10 (2H, d, J=8.5 Hz).
MS (ESI) m/z: 669.

Step 2: (3R,4S)-1-(1-{[(5R,6S)-5,6-Bis(4-chlorophenyl)-3-isopropyl-6-methyl-5,6-dihydroimidazo[2,1-b][1,3]thiazol-2-yl]carbonyl}-L-prolyl)pyrrolidine-3,4-diol)

The compound (200 mg, 0.30 mmol) obtained in Step 1 above was dissolved in a 75% acetic acid aqueous solution (4 ml) and the resulting mixture was heated and stirred at 70°C for 2 days. After the reaction solvent was evaporated under reduced pressure, the operation of adding toluene and evaporating the solvent under reduced pressure was repeated. The obtained residue was purified by silica gel column chromatography (chloroform:methanol = 40:1 → 20:1). Ethyl acetate and hexane were added for solidification to give the title compound (134 mg, 71%) as a colorless solid.
$^1$H-NMR ($CDCl_3$) δ: 0.88 (3H, t, J=6.8 Hz), 0.94 and 0.95 (total 3H, each d, J=7.1 Hz), 1.81 and 1.82 (total 3H, each s), 1.92-2.02 (2H, m), 2.17-2.24 (2H, m), 2.58-2.64 (1H, m), 3.41-3.56 (2H, m), 3.61-3.79 (3H, m), 3.84-3.90 and 4.03-4.07 (total 1H, each m), 4.17-4.22 and 4.24-4.28 (total 1H, each m), 4.26 and 4.34 (total 1H, each dd, J=9.4, 4.3 Hz), 4.57-4.62 (1H, m), 4.95 and 4.96 (1H, each s), 6.69 (2H, d, J=8.1 Hz), 7.01-7.11 (6H, m).
MS (ESI) m/z: 629.

Example 67

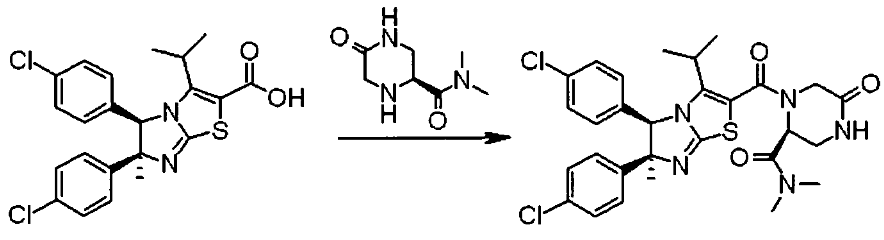

(2S)-1-{[(5R,6S)-5,6-Bis(4-chlorophenyl)-3-isopropyl-6-methyl-5,6-dihydroimidazo[2,1-b][1,3]thiazol-2-yl]carbonyl}-N, N-dimethyl-5-oxopiperazine-2-carboxamide

The same reaction as in Step 4 of Example 1 was performed using (2S)-N,N-dimethyl-5-oxopiperazine-2-carboxamide instead of piperazin-2-one to give the title compound.
$^1$H-NMR ($CDCl_3$) δ: 0.89 (3H, d, J=7.1 Hz), 1.02 (2H, d, J=7.1 Hz), 1.82 (3H, s), 2.44-2.55 (1H, m), 2.97 (3H, s), 3.07 (3H, s), 3.62-3.65 (2H, m), 4.20-4.51 (2H, m), 4.97 (1H, s), 5.35-5.40 (1H, m), 6.71 (2H, d, J=7.1 Hz), 7.02-7.10 (6H, m), 7.57 (1H, d, J=9.3 Hz).
MS (FAB) m/z: 600.

Example 68

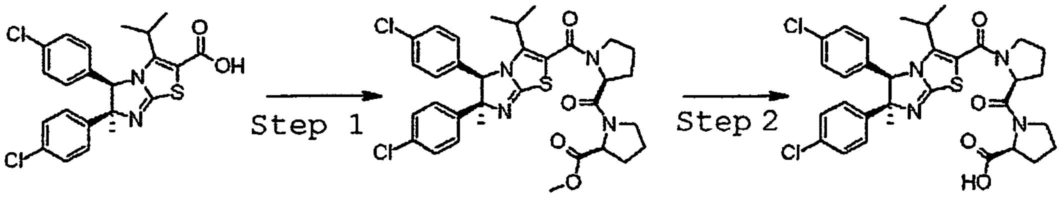


Step 1: Methyl 1-{[(5R,6S)-5,6-bis(4-chlorophenyl)-3-isopropyl-6-methyl-5,6-dihydroimidazo[2,1-b][1,3]thiazol-2-yl]carbonyl}-L-prolyl-L-prolinate

The compound obtained in Step 3 of Example 1 was reacted in the same way as in Step 4 of Example 1 using methyl L-prolinate instead of piperazin-2-one to give the title compound.
$^1$H-NMR ($CDCl_3$) δ: 0.94 (6H, d, J=7.1 Hz), 1.80 (3H, s), 1.88-2.28 (8H, m), 2.62-2.65 (1H, m), 3.60-3.89 (4H, m), 3.71 (3H, s), 4.58 (1H, d, J=4.9 Hz), 4.70-4.73 (1H, m), 4.96 (1H, s), 6.69 (2H, d, J=8.5 Hz), 7.00-7.03 (2H, m), 7.11 (4H, d, J=8.5 Hz).

Step 2: 1-{[(5R,6S)-5,6-Bis(4-chlorophenyl)-3-isopropyl-6-methyl-5,6-dihydroimidazo[2,1-b][1,3]thiazol-2-yl]carbonyl}-L-prolyl-L-proline

The compound obtained in Step 1 above was reacted in the same way as in Example 31 to give the title compound.
$^1$H-NMR ($CDCl_3$) δ: 0.91-1.10 (6H, m), 1.90-2.79 (8H, m), 2.12 (3H, s), 3.55-3.89 (4H, m), 4.34-4.78 (2H, m), 5.49 (1H, s), 7.05-7.09 (6H, m), 7.21-7.23 (2H, m).
MS (FAB) m/z: 641.

Example 69

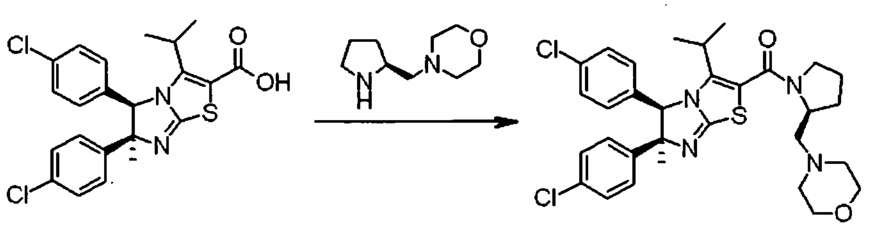

(5R,6S)-5,6-Bis(4-chlorophenyl)-3-isopropyl-6-methyl-2-{[(2S)-2-(morpholin-4-ylmethyl)pyrrolidin-1-yl]carbonyl}-5,6-dihydroimidazo[2,1-b][1,3]thiazole

The same reaction as in Step 4 of Example 1 was performed using (2S)-2-(morpholin-4-ylmethyl)pyrrolidine instead of piperazin-2-one to give the title compound.
$^1$H-NMR ($CDCl_3$) δ: 0.95 (3H, d, J=7.1 Hz), 1.03 (3H, d, J=7.1 Hz), 1.81 (3H, s), 1.81-2.05 (4H, m), 2.29 (1H, dd, J=12.3, 8.9 Hz), 2.44-2.61 (6H, m), 3.52-3.73 (6H, m), 4.29-4.35 (1H, m), 4.95 (1H, s), 6.70 (2H, d, J=8.3 Hz), 7.02-7.12 (6H, m).
MS (FAB) m/z: 599.

Example 70

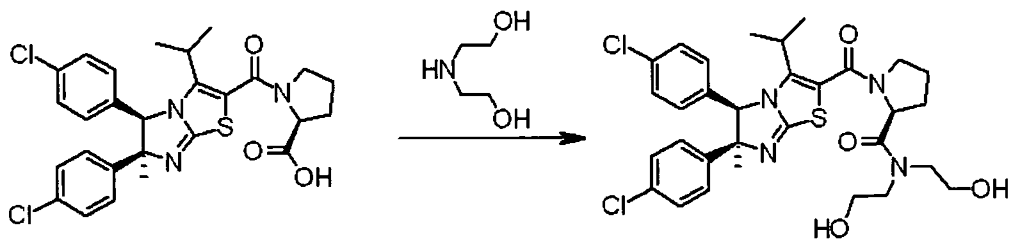

1-{[(5R,6S)-5,6-Bis(4-chlorophenyl)-3-isopropyl-6-methyl-5,6-dihydroimidazo[2,1-b][1,3]thiazol-2-yl]carbonyl}-N,N-bis(2-hydroxyethyl)-L-prolinamide

The compound obtained in Step 2 of Example 61 was reacted in the same way as in Step 4 of Example 1 using bis(2-hydroxyethyl)amine instead of piperazin-2-one to give the title compound.
$^1$H-NMR ($CDCl_3$) δ: 0.91 (3H, d, J=7.1 Hz), 0.97 (3H, d, J=7.1 Hz), 1.81 (3H, s), 1.94-2.03 (2H, m), 2.20-2.27 (2H, m), 2.55-2.62 (1H, m), 3.31-3.37 (1H, m), 3.52-3.57 (2H, m), 3.71-3.76 (2H, m), 3.77-3.85 (3H, m), 3.90-3.97 (2H, m), 4.96 (1H, s), 5.00-5.03 (1H, m), 6.69 (2H, d, J=7.8 Hz), 7.02 (2H, d, J=8.5 Hz), 7.03 (2H, d, J=8.5 Hz), 7.09 (2H, d, J=8.5 Hz).
MS (ESI) m/z: 631.

Example 71

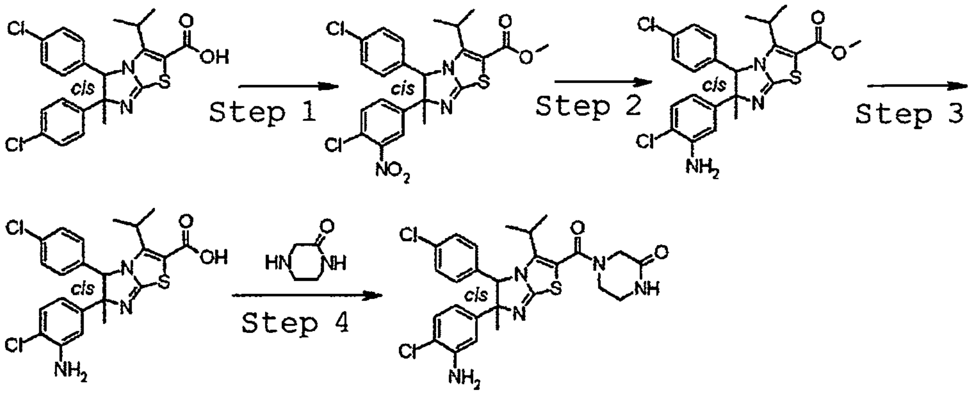


Step 1: Methyl (5R*,6S*)-6-(4-Chloro-3-nitrophenyl)-5-(4-chlorophenyl)-3-isopropyl-6-methyl-5,6-dihydroimidazo[2,1-b][1,3]thiazole-2-carboxylate

Potassium nitrate (226 mg, 2.24 mmol) was slowly added to a concentrated sulfuric acid (10 ml) solution of the compound (0.91 g, 2.03 mmol) obtained in Step 3 of Example 16 under ice cooling. After the resulting mixture was stirred at the same temperature for 30 minutes, the reaction mixture was poured into ice-cold water and stirred. The deposited solid was collected by filtration, washed with water and then dried. The obtained solid was added to benzene-methanol (10:1) (10 ml) and trimethylsilyldiazomethane (2.0 M hexane solution) was added dropwise. After the reactionwas completed, the reaction mixture was concentrated and the residue was purified by silica gel column chromatography to give the title compound (471 mg, 46%) as a racemic mixture.
$^1$H-NMR ($CDCl_3$) δ: 0.87 (3H, d, J=7.1 Hz), 0.99 (3H, d, J=7.1 Hz), 1.82 (3H, s), 3.35 (1H, m), 3.78 (3H, s), 5.11 (1H, s), 6.61-6.86 (2H, brd), 7.09 (2H, d, J=8.5 Hz), 7.21 (1H, t, J=8.5 Hz), 7.34 (1H, dd, J=8.5, 1.7 Hz), 7.72 (1H, d, J=1.7 Hz).
MS (ESI) m/z: 506, 508.

Step 2: Methyl (5R*,6S*)-6-(3-Amino-4-chlorophenyl)-5-(4-chlorophenyl)-3-isopropyl-6-methyl-5,6-dihydroimidazo[2,1-b][1,3]thiazole-2-carboxylate

Acetic acid (6 ml) and iron powder (260 mg, 4.65 mmol) were added to an ethanol (3 ml) solution of the compound (471 mg, 0.93 mmol) obtained in Step 1 above and the resulting mixture was heated to reflux for 1 hour. After the reaction mixture was cooled to room temperature, the resulting mixture was diluted with ethanol and insoluble matter was removed by suction filtration and the filtrate was concentrated under reduced pressure. The residue was diluted with dichloromethane and neutralized with saturated aqueous sodium bicarbonate solution. The organic layer was fractionated, washed with brine and then dried over anhydrous sodium sulfate. The solvent was evaporated under reduced pressure and the residue was purified by silica gel column chromatography to give the title compound (426 mg, 96%) as a racemic light brown solid mixture.
$^1$H-NMR ($CDCl_3$) δ: 0.88 (3H, d, J=7.1 Hz), 0.97 (3H, d, J=7.1 Hz), 1.75 (3H, s), 3.31 (1H, m), 3.77 (3H, s), 3.84 (2H, brs), 5.02 (1H, s), 6.38 (1H, dd, J=8.3, 2.0 Hz), 6.72 (1H, d, J=2.0 Hz), 6.74 (2H, brs), 6.89 (1H, d, J=8.3 Hz), 7.05 (2H, d, J=8.8 Hz).
MS (FAB) m/z: 476, 478.

Step 3: (5R*,6S*)-6-(3-Amino-4-chlorophenyl)-5-(4-chlorophenyl)-3-isopropyl-6-methyl-5,6-dihydroimidazo[2,1-b][1,3]thiazole-2-carboxylic acid

The compound obtained in Step 2 above was reacted in the same way as in Step 3 of Example 1 to give the title compound as a racemic mixture.
$^1$H-NMR ($DMSO-d_6$) δ: 0.83 (3H, d, J=7.1 Hz), 0.92 (3H, d, J=7.1 Hz), 1.65 (3H, s), 3.15 (2H, brs), 3.36 (1H, m), 5.50 (1H, s), 6.38 (1H, dd, J=8.3, 1.5 Hz), 6.80 (2H, d, J=1.5 Hz), 6.91 (2H, brs), 7.15 (2H, d, J=8.3 Hz).
MS (FAB) m/z: 462, 464.

Step 4: 4-{[(5R*,6S*)-6-(3-amino-4-chlorophenyl)-5-(4-chlorophenyl)-3-isopropyl-6-methyl-5,6-dihydroimidazo[2,1-b][1,3]thiazol-2-yl]carbonyl}piperazin-2-one

The compound obtained in Step 3 above was reacted with piperazin-2-one in the same way as in Step 4 of Example 1 to give the title compound as a racemic mixture.
$^1$H-NMR ($CDCl_3$) δ: 0.87 (3H, d, J=7.1 Hz), 0.99 (3H, d, J=7.3 Hz), 1.77 (3H, s), 2.51 (1H, m), 3.45-3.47 (2H, m), 3.79-3.86 (4H, m), 4.26 (2H, brs), 4.92 (1H, s), 6.09 (1H, brs), 6.34 (1H, dd, J=8.4, 2.1 Hz), 6.69 (1H, d, J=2.1 Hz), 6.74 (2H, brd, J=7.6 Hz), 6.89 (1H, d, J=8.4 Hz), 7.06 (2H, d, J=8.8 Hz).
MS (ESI) m/z: 544, 546.

Example 72

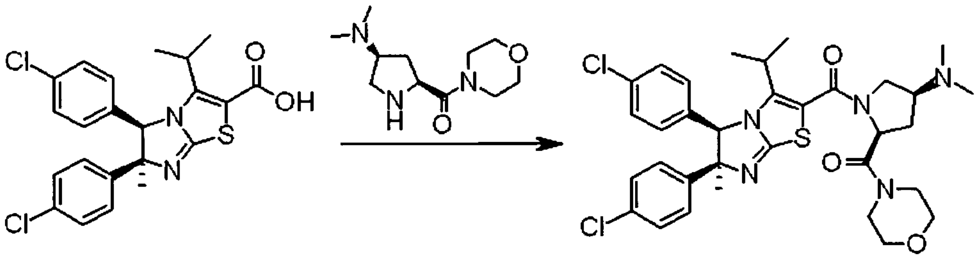

(3S,5S)-1-{[(5R,6S)-5,6-Bis(4-chlorophenyl)-3-isopropyl-6-methyl-5,6-dihydroimidazo[2,1-b][1,3]thiazol-2-yl]carbonyl}-N,N-dimethyl-5-(morpholin-4-ylcarbonyl)pyrrolidin-3-amine

The same reaction as in Step 4 of Example 1 was performed using (3S,5S)-N,N-dimethyl-5(morpholin-4-ylcarbonyl)pyrrolidin-3-amine instead of piperazin-2-one to give the title compound.
$^1$H-N$_M$R ($CDCl_3$) δ: 0.96 (3H, d, J=7.3 Hz), 0.98 (3H, d, J=7.3 Hz), 1.81 (3H, s), 2.28 (6H, s), 2.38-2.45 (1H, m), 2.67-2.79 (2H, m), 3.50-3.90 (11H, m), 4.88 (1H, t, J=8.7 Hz), 4.97 (1H, s), 6.71 (2H, d, J=7.8 Hz), 7.00-7.04 (4H, m), 7.13 (2H, d, J=8.5 Hz).
MS (FAB) m/z: 656.

Example 73

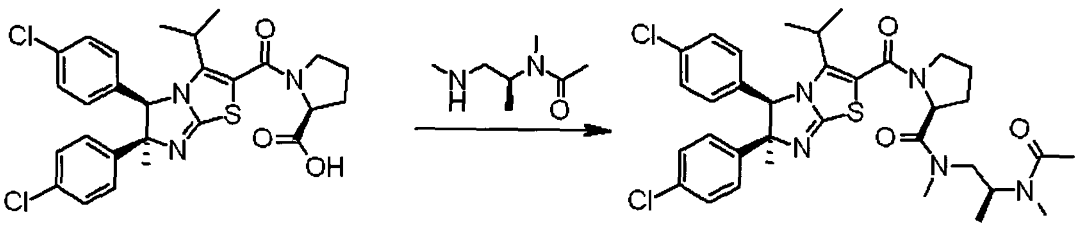

N-{(2S)-2-[Acetyl(methyl)amino]propyl}-1-{[(5R,6S)-5,6-bis(4-chlorophenyl)-3-isopropyl-6-methyl-5,6-dihydroimidazo[2,1-b][1,3]thiazol-2-yl]carbonyl}-N-methyl-L-prolinamide

The compound obtained in Step 2 of Example 61 was reacted in the same way as in Step 4 of Example 1 using N-methyl-N-[(1S)-1-methyl-2-(methylamino)ethyl]acetamide instead of piperazin-2-one to give the title compound.
$^1$H-NMR ($CDCl_3$) δ: 0.92-0.97 (6H, m), 1.10 (3H, d, J=7.1 Hz), 1.80 (3H, s), 1.89-1.96 (1H, m), 2.01-2.02 (1H, m), 2.03 (3H, s), 2.11-2.17 (2H, m), 2.63-2.68 (1H, m), 2.79-2.83 (1H, m), 2.83 (3H, s), 3.13 (3H, s), 3.63-3.69 (1H, m), 3.75-3.82

(1H, m), 4.11-4.16 (1H, m), 4.79-4.84 (1H, m), 4.96 (1H, s), 4.97-5.01 (1H, m), 6.69 (2H, d, J=7.8 Hz), 7.02 (2H, d, J=8.5 Hz), 7.02 (2H, d, J=8.8 Hz), 7.11 (2H, d, J=8.5 Hz).
MS (ESI) m/z: 670.

Example 74

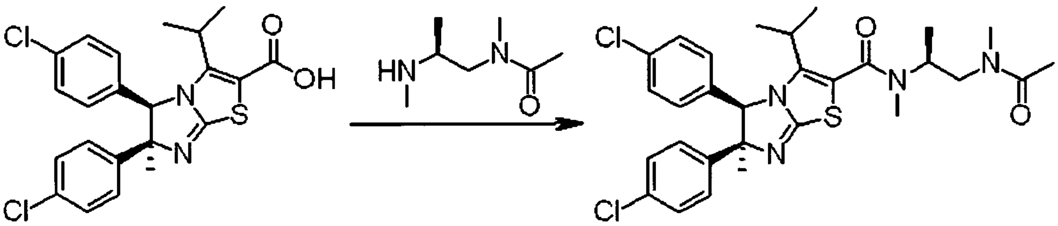

(5R,6S)-N-{(1S)-2-[Acetyl(methyl)amino]-1-methylethyl}-5,6-bis(4-chlorophenyl)-3-isopropyl-N,6-dimethyl-5,6-dihydroimidazo[2,1-b][1,3]thiazole-2-carboxamide

The same reaction was performed as in Step 4 of Example 1 using N-methyl-N-[(2S)-2-(methylamino)propyl]acetamide instead of piperazin-2-one to give the title compound.
$^1$H-NMR ($CDCl_3$) δ: 0.91 (3H, d, J=7.1 Hz), 1.03 (3H, d, J=7.1 Hz), 1.17 (3H, d, J=7.1 Hz), 1.80 (3H, s), 2.07 (3H, s), 2.35-2.40 (1H, m), 2.99 (3H, s), 2.99 (3H, s), 3.00-3.01 (1H, m), 4.02-4.21 (1H, m), 4.92-4.95 (1H, m), 4.92 (1H, s), 6.70 (2H, d, J=8.1 Hz), 7.02 (2H, d, J=8.8 Hz), 7.03 (2H, d, J=8.5 Hz), 7.11 (2H, d, J=8.8 Hz).
MS (ESI) m/z: 573.

Example 75

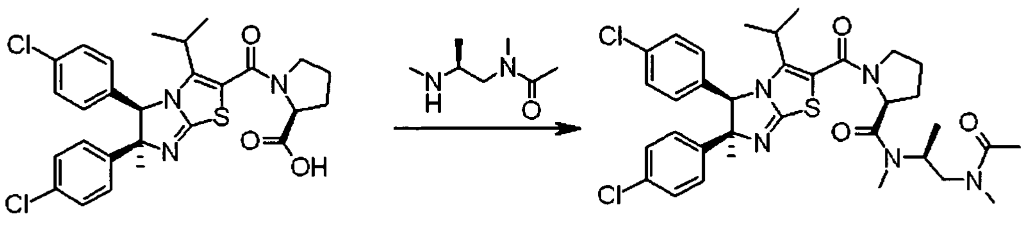

N-{(1S)-2-[Acetyl(methyl)amino]-1-methylethyl}-1-{[(5R,6S)-5,6-bis(4-chlorophenyl)-3-isopropyl-6-methyl-5,6-dihydroimidazo[2,1-b][1,3]thiazol-2-yl]carbonyl}-N-methyl-L-prolinamide

The compound obtained in Step 2 of Example 61 was reacted in the same way as in Step 4 of Example 1 using N-methyl-N-[(2S)-2-(methylamino)propyl]acetamide instead of piperazin-2-one to give the title compound.
$^1$H-NMR ($CDCl_3$) δ: 0.89-1.03 (6H, m), 1.13 and 1.19 (total 3H, each d, J=6.8 Hz), 1.76-1.79 (1H, m), 1.79 and 1.80 (total 3H, each s), 1.90-1.95 (1H, m), 2.02 and 2.16 (total 3H, each s), 2.10-2.23 (2H, m), 2.49-2.54 (1H, m), 2.62-2.67 (1H, m), 2.82 and 2.94 (total 3H, each s), 3.00 and 3.06 (total 3H, each s), 3.59-3.67 (1H, m), 3.74-3.90 (1H, m), 4.18-4.20 and 4.35-4.38 (total 1H, each m), 4.71-4.75 and 4.78-4.81 (total 1H, each m), 4.92 and 4.95 (total 1H, each s), 4.95-4.99 (1H, m), 6.68-6.71 (2H, m), 7.00-7.04 (4H, m), 7.11 (2H, d, J=8.5 Hz).
MS (ESI) m/z: 670.

Example 76

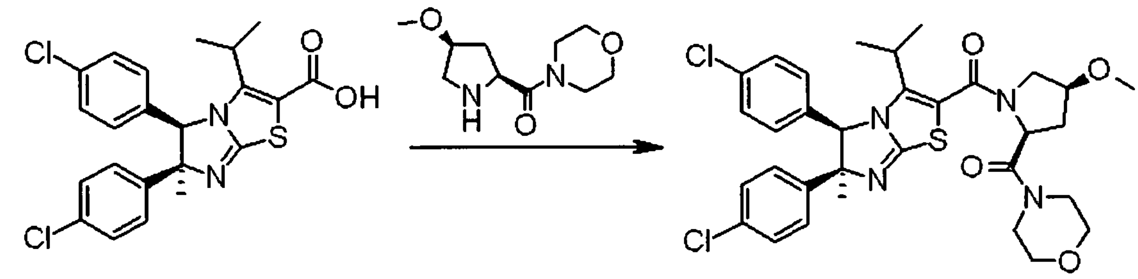

(5R,6S)-5,6-Bis(4-chlorophenyl)-3-isopropyl-2-{[(2S,4S)-4-methoxy-2-(morpholin-4-ylcarbonyl)pyrrolidin-1-yl]carbonyl}-6-methyl-5,6-dihydroimidazo[2,1-b][1,3]thiazole

The same reaction as in Step 4 of Example 1 was performed using 4-[(4S)-4-methoxy-L-prolyl] morpholine instead of piperazin-2-one to give the title compound.
$^1$H-NMR ($CDCl_3$) δ: 0.96 (3H, d, J=7.1 Hz), 0.98 (3H, d, J=7.1 Hz), 1.81 (3H, s), 1.91-1.98 (1H, m), 2.51-2.58 (1H, m), 2.70 (1H, t, J=6.8 Hz), 3.38 (3H, s), 3.58-3.70 (10H, m), 3.95-4.06 (2H, m), 4.88 (1H, t, J=7.9 Hz), 4.96 (1H, s), 6.70 (2H, d, J=8.8 Hz), 7.02 (2H, d, J=8.8 Hz), 7.02 (2H, d, J=8.8 Hz), 7.11 (2H, d, J=8.8 Hz).
MS (FAB) m/z: 643.

Example 77

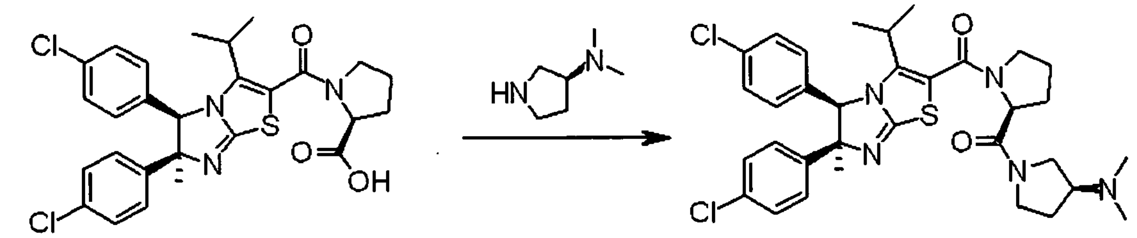

(3S)-1-(1-{[(5R,6S)-5,6-Bis(4-chlorophenyl)-3-isopropyl-6-methyl-5,6-dihydroimidazo[2,1-b][1,3]thiazol-2-yl]carbonyl}-L-prolyl)-N,N-dimethylpyrrolidin-3-amine)

The compound obtained in Step 2 of Example 61 was reacted in the same way as in Step 4 of Example 1 using (3S)-N,N-dimethylpyrrolidin-3-amine instead of piperazin-2-one to give the title compound.
$^1$H-NMR ($CDCl_3$) δ: 0.96 (6H, d, J=6.8 Hz), 1.65-2.02 (3H, m), 1.81 (3H, s), 2.04-2.33 (3H, m), 2.26 (3H, s), 2.28 (3H, s), 2.55-2.88 (2H, m), 3.16-3.25 (1H, m), 3.34-3.48 (1H, m), 3.63-3.83 (3H, m), 3.97-4.13 (1H, m), 4.64-4.73 (1H, m), 4.96 (1H, s), 6.65-6.74 (2H, m), 6.98-7.06 (4H, m), 7.08-7.13 (2H, m).
MS (ESI) m/z: 640.

Example 78

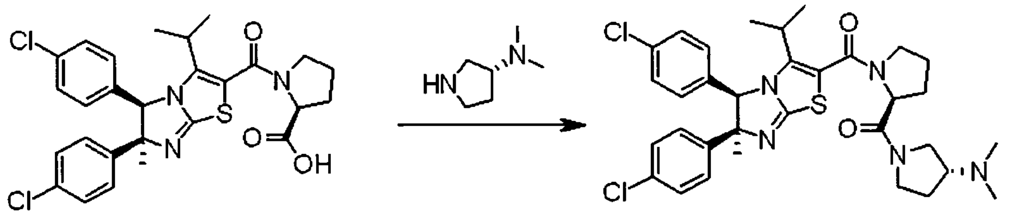

(3R)-1-(1-{[(5R,6S)-5,6-Bis(4-chlorophenyl)-3-isopropyl-6-methyl-5,6-dihydroimidazo[2,1-b][1,3]thiazol-2-yl]carbonyl}-L-prolyl)-N,N-dimethylpyrrolidin-3-amine

The compound obtained in Step 2 of Example 61 was reacted in the same way as in Step 4 of Example 1 using (3R)-N,N-dimethylpyrrolidin-3-amine instead of piperazin-2-one to give the title compound.
$^1$H-NMR ($CDCl_3$) δ: 0.95 (6H, d, J=7.3 Hz), 1.69-2.09 (3H, m), 1.81 (3H, s), 2.11-2.30 (3H, m), 2.26 (3H, s), 2.28 (3H, s), 2.56-2.89 (2H, m), 3.07-3.35 (1H, m), 3.48-3.92 (5H, m), 4.60-4.69 (1H, m), 4.96 (1H, s), 6.64-6.73 (2H, m), 6.98-7.05 (4H, m), 7.07-7.13 (2H, m).
MS (ESI) m/z: 640.

Example 79

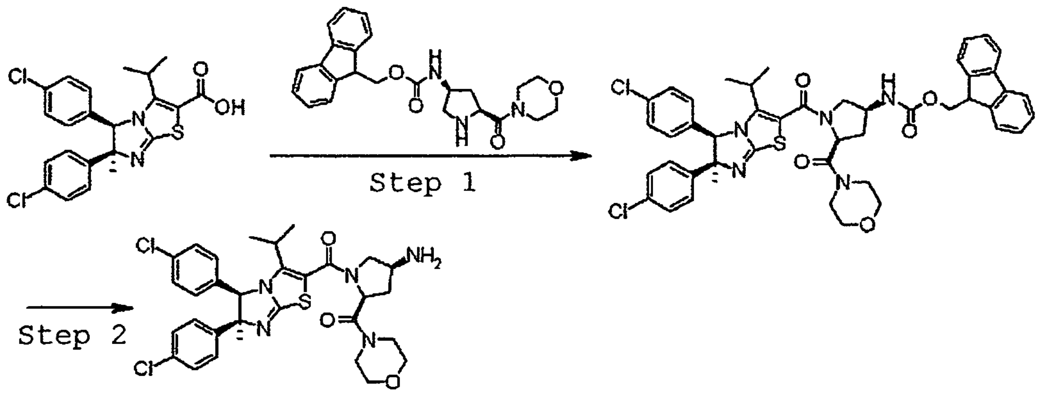


Step 1: 9H-Fluoren-9-ylmethyl [(3S,5S)-1-{[(5R,6S)-5,6-bis(4-chlorophenyl)-3-isopropyl-6-methyl-5,6-dihydroimidazo [2,1-b][1,3]thiazol-2-yl]carbonyl}-5-(morpholin-4-ylcarbonyl)pyrrolidine-3-yl]carbamate

The same reaction as in Step 4 of Example 1 was performed using 9H-fluoren-9-ylmethyl [(3S,5S)-5-(morpholin-4-ylcarbonyl)pyrrolidin-3-yl]carbamate instead of piperazin-2-one to give the title compound.
MS (ESI) m/z: 850.

Step 2: (3S,5S)-1-{[(5R,6S)-5,6-Bis(4-chlorophenyl)-3-isopropyl-6-methyl-5,6-dihydroimidazo[2,1-b][1,3]thiazol-2-yl] carbonyl}-5-(morpholin-4-ylcarbonyl)pyrrolidin-3-amine

Piperidine (1 ml) was added to a dimethylformamide (4 ml) solution of the compound (544 mg, 0.64 mmol) obtained in Step 1 above and stirred at room temperature for 1 hour. Water was added to the reaction mixture and extracted with ethyl acetate and washed with brine. After the organic layer was dried over anhydrous magnesium sulfate, the solvent was evaporated under reduced pressure. Diisopropylether was added to the obtained residue and the solid was collected by filtration to give the title compound (313 mg, 78%) as a colorless solid.
$^1$H-NMR ($CDCl_3$) δ: 0.94-0.98 (6H, m), 1.75-1.81 (2H, m), 1.81 (3H, s), 2.57-2.68 (1H, m), 3.53-3.68 (10H, m), 3.82 (1H, t, J=7.8 Hz), 3.93-3.97 (1H, m), 4.89 (1H, t, J=7.3 Hz), 4.96 (1H, s), 6.69-6.71 (2H, m), 7.01-7.12 (6H, m).
MS (FAB) m/z: 627.

Example 80

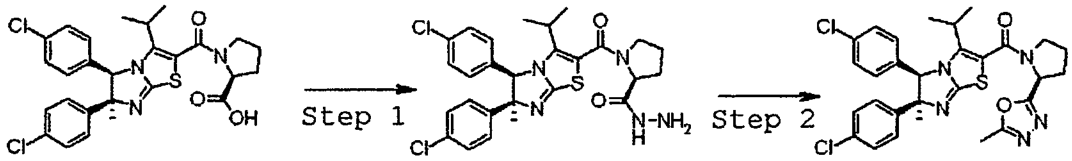


Step 1: 1-{[(5R,6S)-5,6-Bis(4-chlorophenyl)-3-isopropyl-6-methyl-5,6-dihydroimidazo[2,1-b][1,3]thiazol-2-yl]carbonyl}-L-proline hydrazide

The compound obtained in Step 2 of Example 61 was reacted in the same way as in Step 4 of Example 1 using hydrazine monohydrate instead of piperazin-2-one to give the title compound.
$^1$H-NMR ($CDCl_3$) δ: 0.92 (3H, d, J=7.1 Hz), 1.00 (3H, d, J=7.1 Hz), 1.81 (3H, s), 1.90-1.96and2.02-2.08 (1H, each m), 2.12-2.17 and 2.36-2.40 (total 1H, each m), 2.21 (3H, s), 2.34-2.37 (1H, m), 2.57-2.65 (1H, m), 3.20-3.25 (1H, m), 3.64-3.73 (2H, m), 3.86 (2H, brs), 4.49-4.55 (1H, m), 4.96 (1H, s), 6.69 (2H, d, J=8.3 Hz), 7.02 (2H, d, J=8.5 Hz), 7.04 (2H, d, J=8.5 Hz), 7.10 (2H, d, J=8.8 Hz), 7.78 (1H, brs).
MS (ESI) m/z: 558.

Step 2: (5R,6S)-5,6-Bis(4-chlorophenyl)-3-isopropyl-6-methyl-2-{[(2S)-2-(5-methyl-1,3,4-oxadiazol-2-yl)pyrrolidin-1-yl]carbonyl}-5,6-dihydroimidazo[2,1-b][1,3]thiazole

The compound (90 mg, 0.16 mmol) obtained in Step 1 above was dissolved in triethyl orthoacetate (4 ml) and the resulting mixture was heated to reflux at 145°C for 20 hours. The reaction mixture was returned to room temperature and the reaction solvent was evaporated under reduced pressure. The obtained residue was purified by silica gel column chromatography (chloroform:methanol = 40:1) and lyophilized with 1,4-dioxane to give the title compound (5 mg, 5%) as a colorless solid.
$^1$H-NMR ($CDCl_3$) δ: 0.89 (3H, d, J=6.8 Hz), 0.95 (3H, d, J=7.3 Hz), 1.81 (3H, s), 2.05-2.10 (1H, m), 2.21-2.25 (1H, m), 2.35-2.38 (1H, m), 2.48 (3H, s), 2.61-2.65 (1H, m), 3.72-3.76 (1H, m), 3.86-3.89 (1H, m), 4.95 (1H, s), 5.02-5.06 (1H, m), 5.37-5.40 (1H, m), 6.65-6.70 (2H, m), 7.00-7.06 (4H, m), 7.10 (2H, d, J=8.5 Hz).
MS (ESI) m/z: 582.

Example 81

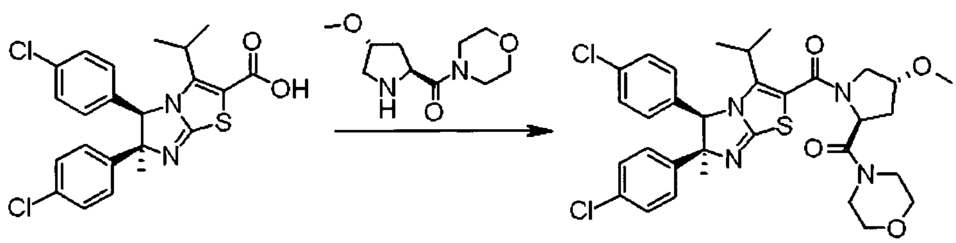

(5R,6S)-5,6-Bis(4-chlorophenyl)-3-isopropyl-2-{[(2S,4R)-4-methoxy-2-(morpholin-4-ylcarbonyl)pyrrolidin-1-yl]carbonyl}-6-methyl-5,6-dihydroimidazo[2,1-b] [1,3]thiazole

The same reaction as in Step 4 of Example 1 was performed using 4-[(4R)-4-methoxy-L-prolyl]morpholine instead of piperazin-2-one to give the title compound.
$^1$H-NMR ($CDCl_3$) δ: 0. 94 (3H, d, J=7.1 Hz), 1.00 (3H, d, J=7.1 Hz), 1.81 (3H, s), 2.11-2.18 (1H, m), 2.25-2.30 (1H, m),

2.56-2.63 (1H, m), 3.28 (3H, s), 3.45-3.57 (2H, m), 3.68-3.87 (9H, m), 4.06-4.10 (1H, m), 4.95 (1H, s), 5.00 (1H, t, J=7.9 Hz), 6.71 (2H, d, J=7.8 Hz), 7.01-7.04 (4H, m), 7.11 (2H, d, J=8.8 Hz).
MS (FAB) m/z: 643.

Example 82

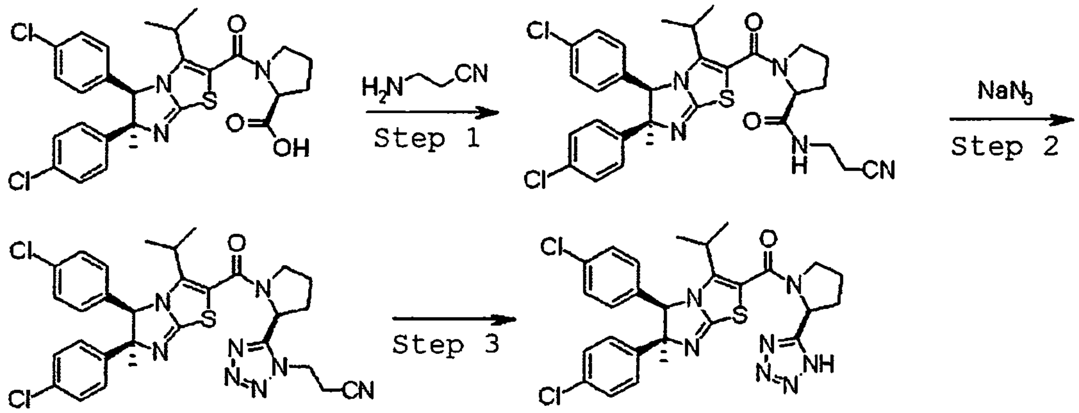


Step 1: 1-{[(5R,6S)-5,6-Bis(4-chlorophenyl)-3-isopropyl-6-methyl-5,6-dihydroimidazo[2,1-b][1,3]thiazol-2-yl]carbonyl}-N-(2-cyanoethyl)-L-prolinamide

The compound obtained in Step 2 of Example 61 was reacted in the same way as in Step 4 of Example 1 using 3-aminopropionitrile instead of piperazin-2-one to give the title compound.
$^1$H-NMR ($CDCl_3$) δ: 0.92 (3H, d, J=7.0 Hz), 1.00 (3H, d, J=7.0 Hz), 1.80-2.20 (3H, m), 1.82 (3H, s), 2.30-2.42 (1H, m), 2.50-2.73 (3H, m), 3.39-3.80 (4H, m), 4.56 (1H, dd, J=7.7, 4.8 Hz), 4.97 (1H, s), 6.65-6.77 (2H, m), 6.98-7.33 (6H, m).

Step 2: 3-[5-((2S)-1-{[(5R,6S)-5,6-Bis(4-chlorophenyl)-3-isopropyl-6-methyl-5,6-dihydroimidazo[2,1-b][1,3]thiazol-2-yl]carbonyl}pyrrolidin-2-yl)-1H-tetrazol-1-yl]propanenitrile

The compound (0.62 g, 1.04 mmol) obtained in Step 1 above was dissolved in acetonitrile (20 ml) followed by the addition of sodium azide (101 mg, 1.56 mmol) and trifluoromethanesulfonic anhydride (262 μl, 1.56 mmol) under argon atmosphere. The resulting mixture was stirring at room temperature for 2 hours followed by the addition of sodium azide (101 mg, 1.56 mmol) and trifluoromethanesulfonic anhydride (262 μl, 1.56 mmol) and stirred at room temperature for 15 hours. Saturated aqueous sodium bicarbonate solution was added to the reaction mixture and extracted with ethyl acetate and the resulting mixture was washed with brine and then dried over anhydrous sodium sulfate, and the solvent was evaporated under reduced pressure. The residue was purified by silica gel thin layer chromatography (chloroform: methanol = 20:1) to give the title compound (192 mg, 30%) as a pale yellow solid.
$^1$H-NMR ($CDCl_3$) δ: 0.79 (3H, d, J=7.1 Hz), 0.89 (3H, d, J=7.1 Hz), 1.76-1.84 (2H, m), 1.88 (3H, s), 2.26-2.36 (2H, m), 2.68 (3H, t, J=6.5 Hz), 3.10 (2H, td, J=6.7, 3.4 Hz), 3.62 (2H, q, J=6.5 Hz), 4.43-4.50 (1H, m), 5.08 (1H, s), 6.72-6.81 (2H, m), 6.98-7.15 (6H, m).

Step 3: (5R,6S)-5,6-Bis(4-chlorophenyl)-3-isopropyl-6-methyl-2-{[(2S)-2-(1H-tetrazol-5-yl)pyrrolidin-1-yl]carbonyl}-5,6-dihydroimidazo[2,1-b][1,3]thiazole

The compound (190 mg, 0.31 mmol) obtained in Step 2 above was dissolved in methylene chloride (5 ml) followed by the dropwise addition of 1,8-diazabicyclo[5.4.0]undec-7-ene (DBU) (229 μl, 1.53 mmol) and the resulting mixture was stirred at room temperature for 15 hours. DBU (229 μl, 1.53 mmol) was added and the resulting mixture was stirred at room temperature for 9 hours. Then DBU (229μ l, 1.53 mmol) was added and the resulting mixture was stirred at room temperature for 24 hours and DBU (229 μl, 1.53 mmol) was further added and the resulting mixture was stirred at room temperature for 24 hours. The reaction mixture was diluted with chloroform, washed with 1 N aqueous hydrochloric acid solution and saturated salt solution and then dried over anhydrous sodium sulfate, and the solvent

was evaporated under reduced pressure. The residue was purified by silica gel thin layer chromatography (chloroform: methanol: water = 8:3:0.5) and then repurified by HPLC (this column was Develosil Combi-PR-5 manufactured by Nomura Chemical Co., Ltd., developing solvent; water: acetonitrile = 69:31 → 40:60 (containing 0.1% formic acid)) and freeze-dried with dioxane to give the title compound (6.1 mg, 4%) as a colorless solid.
$^1$H-NMR ($CDCl_3$) δ: 0.90 (3H, d, J=6.8 Hz), 1.01 (3H, d, J=6.8 Hz), 1.87 (3H, s), 2.02-2.15 (1H, m), 2.26-2.48 (2H, m), 2.56-2.67 (1H, m), 3.72-3.82 (1H, m), 3.81-3.92 (1H, m), 4.71-5.39 (2H, m), 5.50 (1H, s), 6.55-6.85 (2H, m), 6.96-7.14 (7.5H, m), 8.21 (0.5H, s).
MS (ESI) m/z: 568, 570.

Example 83

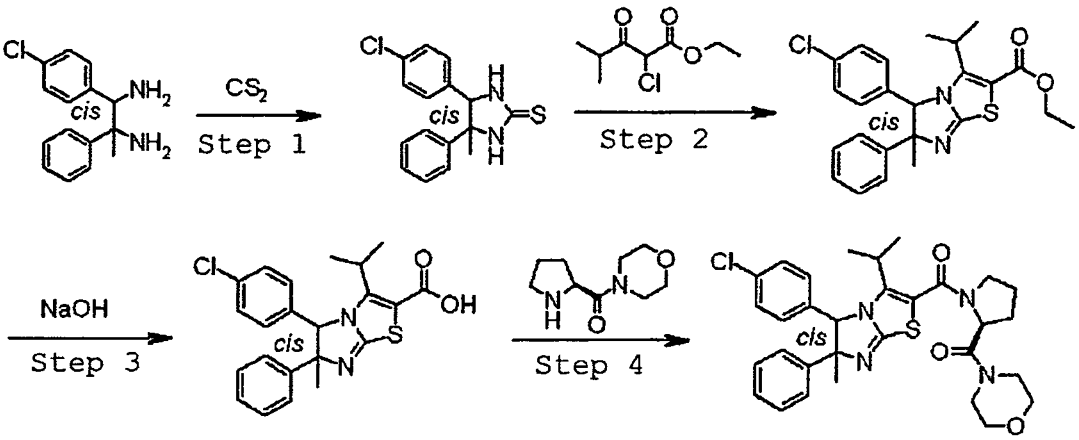


Step 1: (4R*,5S*)-5-(4-chlorophenyl)-4-methyl-4-phenylimidazolidin-2-thione

(1R*,2S*)-1-(4-Chlorophenyl)-2-phenylpropane-1,2-diamine was reacted in the same way as in Step 1 of Example 1 to give the title compound.
$^1$H-NMR ($CDCl_3$) δ: 1.87 (3H, s), 4.92 (1H, s), 6.78 (2H, d, J=8.3 Hz), 6.82-7.29 (9H, m).
MS (ESI) m/z: 261.

Step 2: Ethyl (5R*,6S*)-5-(4-chlorophenyl)-3-isopropyl-6-methyl-6 -phenyl-5,6-dihydroimidazo[2,1-b][1,3]thiazole-2-carboxylate

The compound obtained in Step 1 above was reacted with ethyl 2-chloro-4-methyl-3-oxopentanoate in the same way as in Step 1 of Example 4 to give the title compound.
$^1$H-NMR ($CDCl_3$) δ: 0.89 (3H, d, J=7.1 Hz), 0.99 (3H, d, J=7.1 Hz), 1.32 (3H, t, J=7.1 Hz), 1.84 (3H, s), 3.33 (1H, m), 4.24 (2H, q, J=7.1 Hz), 5.07 (1H, s), 6.68-6.72 (2H, m), 6.96-7.06 (5H, m), 7.16 (2H, d, J=8.3 Hz).
MS (ESI) m/z: 441.

Step 3: (5R*,6S*)-5-(4-chlorophenyl)-3-isopropyl-6-methyl-6- phenyl-5,6-dihydroimidazo[2,1-b][1,3]thiazole-2-carboxylic acid

The compound obtained in Step 2 above was reacted in the same way as in Step 3 of Example 1 to give the title compound.
$^1$H-NMR ($CDCl_3$) δ: 0.92 (3H, d, J=7.1 Hz), 1.06 (3H, d, J=7.1 Hz), 2.11 (3H, s), 3.55-3.62 (1H, m), 5.50 (1H, s), 6.67-6.72 (2H, m), 6.99-7.10 (5H, m), 7.20 (2H, d, J=8.5 Hz).
MS (ESI) m/z: 413.

Step 4: (5R*,6S*)-5-(4-chlorophenyl)-3-isopropyl-6-methyl-2-{[(2S)-2-(morpholin-4-ylcarbonyl)pyrrolidin-1-yl]carbonyl}-6-phenyl-5,6-dihydroimidazo[2,1-b][1,3]thiazole

The compound obtained in Step 3 above was reacted in the same way as in Step 4 of Example 1 using 4-(L-prolyl)morpholine instead of piperazin-2-one to give the title compound.
$^1$H-NMR ($CDCl_3$) δ: 0.90-1.00 (6H, m), 1.84 (3H, s), 1.91-2.21 (4H, m), 2.66-2.78 (1H, m), 3.45-3.81 (10H, m), 4.88-4.91 (1H, m), 4.98 (1H, m), 6.68-6.71 (2H, m), 6.97-7.06 (5H, m), 7.16 (2H, d, J=7.1 Hz).
MS (FAB) m/z: 579.

Example 84

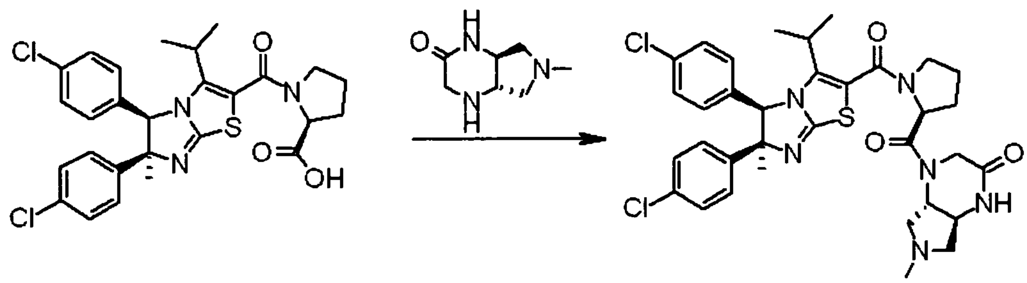

(4aS,7aS)-4-(1-{[(5R,6S)-5,6-Bis(4-chlorophenyl)-3-isopropyl-6-methyl-5,6-dihydroimidazo[2,1-b][1,3]thiazol-2-yl]carbonyl}-L-prolyl)-6-methyloctahydro-2H-pyrrolo[3,4-b]pyrazin-2-one

The compound obtained in Step 2 of Example 61 was reacted in the same way as in Step 4 of Example 1 using (4aS,7aS)-6-methyloctahydro-2H-pyrrolo[3,4-b]pyrazin-2-one instead of piperazin-2-one to give the title compound.
$^1$H-NMR ($CDCl_3$) δ: 0.91 (3H, d, J=7.1 Hz), 0.99 (3H, d, J=7.1 Hz), 1.81 (3H, s), 1.89-1.95 (1H, m), 2.03-2.19 (3H, m), 2.31 (3H, s), 2.32-2.36 (1H, m), 2.49-2.58 (2H, m), 2.73 (1H, dd, J=9.9, 6.5 Hz), 3.07-3.12 (2H, m), 3.53 (2H, d, J=1.5 Hz), 3.61-3.70 (2H, m), 4.01-4.06 (1H, m), 4.51 (1H, dd, J=7.8, 4.4 Hz), 4.95 (1H, s), 6.70 (2H, d, J=7.6 Hz), 6.98 (1H, brs), 7.02 (2H, d, J=8.5 Hz), 7.04 (2H, d, J=8.8 Hz), 7.10 (2H, d, J=8.8 Hz).

Example 85

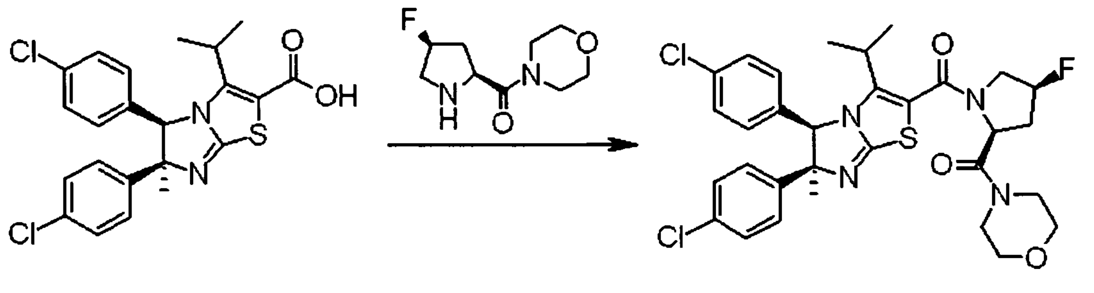

(5R,6S)-5,6-Bis(4-chlorophenyl)-2-{[(2S,4S)-4-fluoro-2-(morpholin-4-ylcarbonyl)pyrrolidin-1-yl]carbonyl}-3-isopropyl-6-methyl-5,6-dihydroimidazo[2,1-b][1,3]thiazole

The same reaction as in Step 4 of Example 1 was performed using 4-[(4S)-4-fluoro-L-prolyl]morpholine instead of piperazin-2-one to give the title compound.
$^1$H-NMR ($CDCl_3$) δ: 0.97 (6H, d, J=5.6 Hz), 1.82 (3H, s), 2.25-2.70 (3H, m), 3.49-3.71 (8H, m), 4.03-4.10 (2H, m), 5.00 (2H, t, J=10.1 Hz), 5.28 (1H, d, J=53.4 Hz), 6.71 (2H, d, J=7.1 Hz), 7.01-7.11 (6H, m).

MS (FAB) m/z: 631.

Example 86

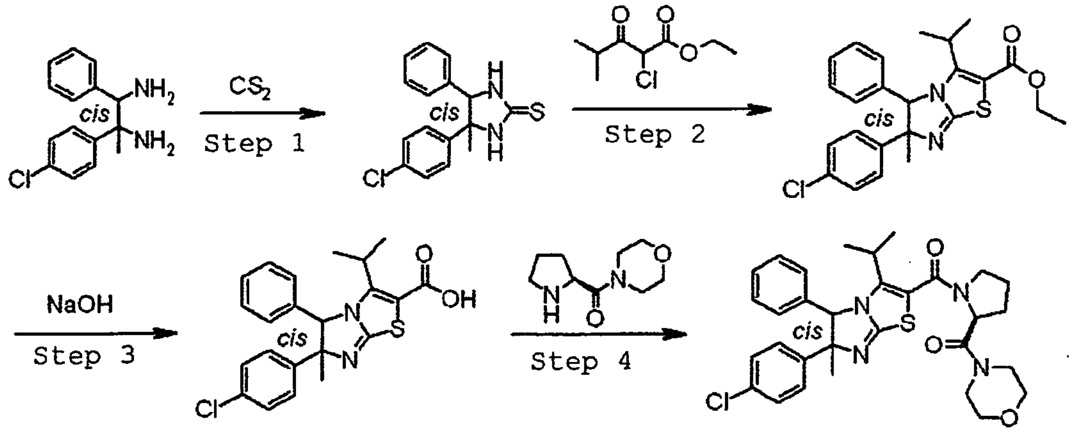


Step 1: (4R*,5S*)-4-(4-Chlorophenyl)-4-methyl-5-phenylimidazolizine-2-thione

(1R*,2S*)-2-(4-Chlorophenyl)-1-phenylpropane-1,2-diamine was reacted in the same way as in Step 1 of Example 1 to give the title compound.
$^1$H-NMR ($CDCl_3$) δ: 1.88 (3H, s), 4.98 (1H, s), 6.49 (1H, brs), 6.84-6.88 (4H, m), 6.98 (1H, brs), 7.03-7.14 (5H, m) .
MS (ESI) m/z: 261.

Step 2: Ethyl (5R*,6S*)-6-(4-Chlorophenyl)-3-isopropyl-6-methyl-5-phenyl-5,6-dihydroimidazo[2,1-b][1,3]thiazole-2-carboxylate

The compound obtained in Step 1 above was reacted with ethyl 2-chloro-4-methyl-3-oxopentanoate in the same way as in Step 1 of Example 4 to give the title compound.
$^1$H-NMR ($CDCl_3$) δ: 0.87 (3H, d, J=6.8 Hz), 0.96 (3H, d, J=6.8 Hz), 1.32 (3H, t, J=7.3 Hz), 1.81 (3H, s), 3.36-3.29 (1H, m), 4.24 (2H, q, J=7.1 Hz), 5.07 (1H, s), 6.75 (2H, brs), 6.97-7.12 (7H, m).
MS (ESI) m/z: 441.

Step 3: (5R*,6S*)-6-(4-Chlorophenyl)-3-isopropyl-6-methyl-5-phenyl-5,6-dihydroimidazo[2,1-b][1,3]thiazole-2-carboxylic acid

The compound obtained in Step 2 above was reacted in the same way as in Step 3 of Example 1 to give the title compound.
$^1$H-NMR ($CDCl_3$) δ: 0.92 (3H, d, J=7.1 Hz), 1.06 (3H, d, J=7.1 Hz), 2.11 (3H, s), 3.55-3.62 (1H, m), 5.50 (1H, s), 6.67-6.72 (2H, m), 6.99-7.10 (5H, m), 7.20 (2H, d, J=8.5 Hz) .
MS (ESI) m/z: 413.

Step 4: (5R*,6S*)-6-(4-hlorophenyl) -3-isopropyl-6-methyl-2-{[(2S)-2-(morpholin-4-ylcarbonyl)pyrrolidin-1-yl]carbonyl}-5-phenyl-5,6-dihydroimidazo[2,1-b][1,3]thiazole

The compound obtained in Step 3 above was reacted in the same way as in Step 4 of Example 1 using 4-(L-prolyl)morpholine instead of piperazin-2-one to give the title compound.
$^1$H-NMR ($CDCl_3$) δ: 0.87-0.97 (6H, m), 1.82 (3H, s), 1.91-1.98 (2H, m), 2.13-2.23 (2H, m), 2.66-2.74 (1H, m), 3.64-3.83 (10H, m), 4.89 (1H, s), 4.98-4.99 (1H, m), 6.74 (2H, brs), 6.96-7.12 (7H, m).
MS (FAB) m/z: 579.

Example 87

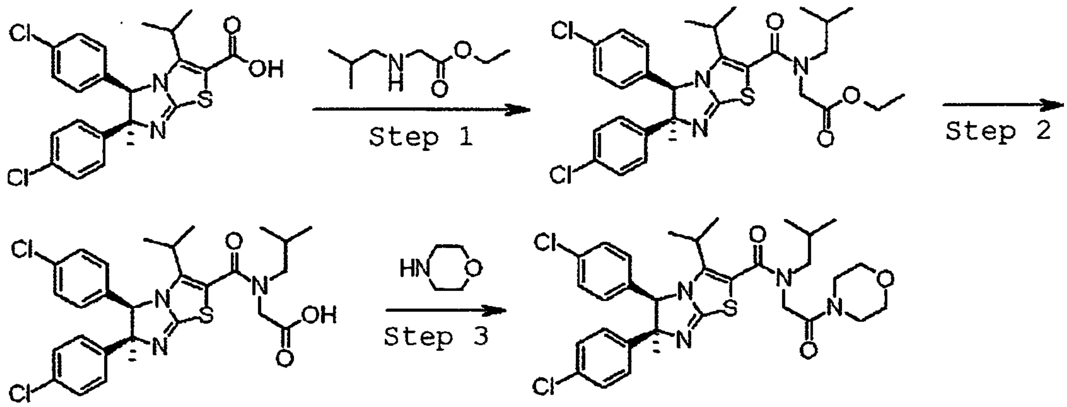


Step 1: Ethyl N-{[(5R,6S)-5,6-Bis(4-chlorophenyl)-3-isopropyl-6-methyl-5,6-dihydroimidazo[2,1-b][1,3]thiazol-2-yl]carbonyl}-N-isobutylglycinate

The same reaction was performed as in Step 4 of Example 1 using ethyl N-isobutylglycinate instead of piperazin-2-one to give the title compound.
$^1$H-NMR ($CDCl_3$) δ: 0.93 (6H, d, J=6.8 Hz), 0.95 (3H, d, J=6.6 Hz), 0.98 (3H, d, J=7.1 Hz), 1.29 (3H, t, J=7.1 Hz), 1.82 (3H, s), 1.93-1.99 (1H, m), 2.56-2.62 (1H, m), 3.33 (2H, d, J=7.3 Hz), 4.14-4.19 (2H, m), 4.21 (2H, q, J=6.8 Hz), 4.96 (1H, s), 6.69 (2H, d, J=8.3 Hz), 7.03 (4H, d, J=8.3 Hz), 7.11 (2H, d, J=8.5 Hz).
MS (ESI) m/z: 588.

Step 2: N-{[(5R,6S)-5,6-Bis(4-chlorophenyl)-3-isopropyl-6-methyl-5,6-dihydroimidazo[2,1-b][1,3]thiazol-2-yl]carbonyl}-N-isobutylglycine

The compound obtained in Step 1 above was reacted in the same way as in Example 31 to give the title compound.
$^1$H-NMR (DMSO-$d_6$) δ: 0.75-0.92 (12H, m), 1.73 (3H, s), 1.86-1.97 (1H, m), 2.56-2.64 (1H, m), 3.16-3.24 (2H, m), 4.05-4.16 (2H, m), 5.56 (1H, s), 6.83-6.91 (2H, m), 7.11 (2H, d, J=8.5 Hz), 7.13 (2H, d, J=8.8 Hz), 7.26 (2H, d, J=8.3 Hz).
MS (ESI) m/z: 560.

Step 3: (5R,6S)-5,6-Bis(4-chlorophenyl)-N-isobutyl-3-isopropyl-6-methyl-N-(2-morpholin-4-yl-2-oxoethyl)-5,6-dihydroimidazo[2,1-b][1,3]thiazole-2-carboxamide

The compound obtained in Step 2 above was reacted in the same way as in Step 4 of Example 1 using morpholine instead of piperazin-2-one to give the title compound.
$^1$H-NMR ($CDCl_3$) δ: 0.89 (3H, d, J=7.1 Hz), 0.92 (3H, d, J=6.6 Hz), 0.95 (3H, d, J=6.6 Hz), 1.02 (3H, d, J=7.1 Hz), 1.82 (3H, s), 1.93-2.00 (1H, m), 2.65-2.71 (1H, m), 3.37-3.40 (2H, m), 3.45-3.48 (2H, m), 3.58-3.62 (2H, m), 3.68-3.72 (4H, m), 4.18 (1H, d, J=16.0 Hz), 4.25 (1H, d, J=16.0 Hz), 4.96 (1H, s), 6.69 (2H, d, J=8.3 Hz), 7.02 (2H, d, J=8.5 Hz), 7.03 (2H, d, J=8.8 Hz), 7.12 (2H, d, J=8.8 Hz).
MS (ESI) m/z: 629.

Example 88

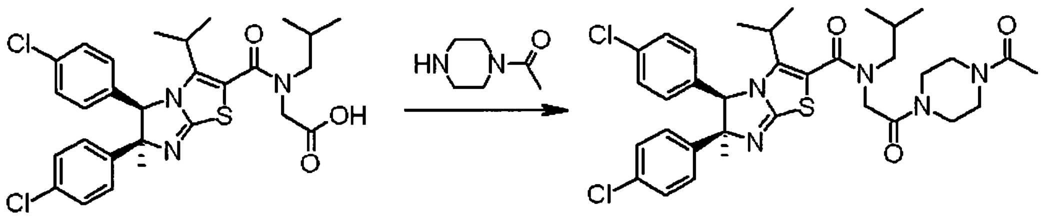

(5R,6S)-N-[2-(4-Acetylpiperazin-1-yl)-2-oxoethyl]-5,6-bis(4-chlorophenyl)-N-isobutyl-3-isopropyl-6-methyl-5,6-dihydroimidazo[2,1-b][1,3]thiazole-2-carboxamide

The compound obtained in Step 2 of Example 87 was reacted in the same way as in Step 4 of Example 1 using 1-acetylpiperazine instead of piperazin-2-one to give the title compound.
$^1$H-NMR ($CDCl_3$) δ: 0.89 (3H, d, J=6.8 Hz), 0.93 (3H, d, J=6.6 Hz), 0.96 (3H, d, J=6.6 Hz), 1.01 (3H, d, J=7.1 Hz), 1.82 (3H, s), 1.93-2.00 (1H, m), 2.13 (3H, s), 2.64-2.71 (1H, m), 3.37-3.41 (2H, m), 3.46-3.50 (2H, m), 3.51-3.56 (2H, m), 3.60-3.70 (4H, m), 4.18-4.27 (2H, m), 4.96 (1H, s), 6.69 (2H, d, J=8.0 Hz), 7.02 (2H, d, J=8.8 Hz), 7.03 (2H, d, J=8.5 Hz), 7.12 (2H, d, J=8.8 Hz).
MS (ESI) m/z: 670.

Example 89

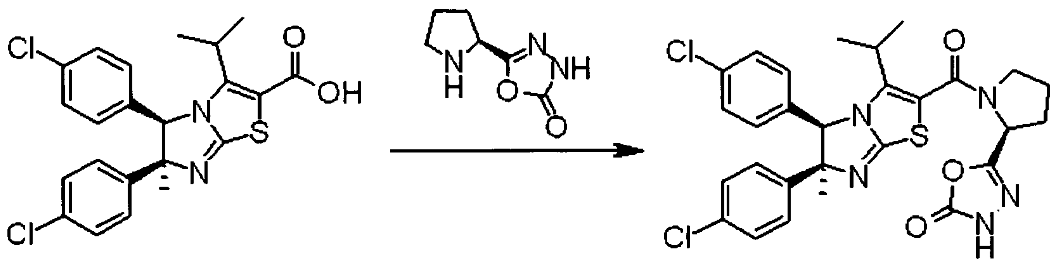

5-((2S)-1-{[(5R,6S)-5,6-Bis(4-chlorophenyl)-3-isopropyl-6-methyl-5,6-dihydroimidazo[2,1-b][1,3]thiazol-2-yl]carbonyl} pyrrolidin-2-yl)-1,3,4-oxadiazol-2(3H)-one

The same reaction as in Step 4 of Example 1 was performed using the hydrochloride of 5-((2S)-pyrrolidin-2-yl)-1,3,4-oxadiazol-2(3H)-one instead of piperazin-2-one to give the title compound.
$^1$H-NMR ($CDCl_3$) δ: 0.92 (3H, d, J=7.1 Hz), 0.97 (3H, d, J=7.1 Hz), 1.83 (3H, s), 2.00-2.05 (1H, m), 2.10-2.17 (2H, m), 2.26-2.33 (1H, m), 2.61-2.67 (1H, m), 3.67-3.72 (1H, m), 3.78-3.82 (1H, m), 4.98 (1H, s), 5.05 (1H, dd, J=7.8, 3.9 Hz), 6.68 (2H, d, J=8.0 Hz), 7.02 (2H, d, J=8.8 Hz), 7.05 (2H, d, J=8.8 Hz), 7.09 (2H, d, J=8.8 Hz).
MS (ESI) m/z: 584.

Example 90

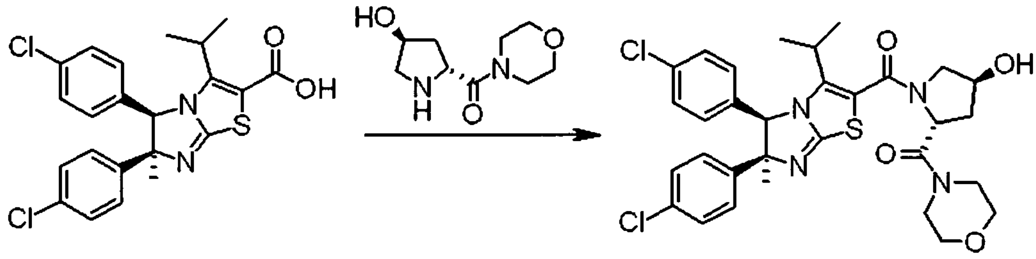

(3S,5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-(morpholin-4-ylcarbonyl)pyrrolidin-3-ol

The same reaction as in Step 4 of Example 1 was performed using (3S,5R)-5-(morpholin-4-ylcarbonyl)pyrrolidin-3-ol instead of piperazin-2-one to give the title compound.
$^1$H-NMR ($CDCl_3$) δ: 0.89 (3H, d, J=6.6 Hz), 0.99 (3H, d, J=6.6 Hz), 1.82 (3H, s), 2.03-2.09 (1H, m), 2.17-2.22 (1H, m), 2.71-2.77 (1H, m), 3.45-3.49 (1H, m), 3.55-3.71 (6H, m), 3.77-3.81 (3H, m), 4.48 (1H, s), 4.99-5.04 (2H, m), 6.67 (2H, d, J=7.3 Hz), 7.01-7.10 (6H, m).
MS (FAB) m/z: 629.

Example 91

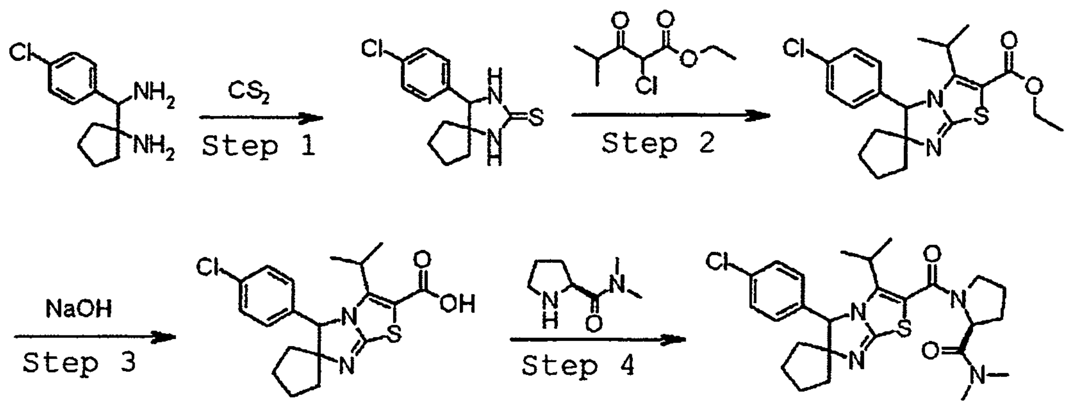


Step 1: 4-(4-Chlorophenyl)-1,3-diazaspiro[4,4]nonane-2-thione

1-[Amino(4-chlorophenyl)methyl]cyclopentanamine was reacted in the same way as in Step 1 of Example 1 to give the title compound.
$^1$H-NMR ($CDCl_3$) δ: 1.17 (1H, m), 1.34-1.59 (3H, m), 1.66-1.75 (2H, m), 1.85-1.92 (1H, m), 2.03 (1H, m), 4.79 (1H, s), 6.05 (1H, brs), 6.17 (1H, brs), 7.21 (2H, d, J=8.3 Hz), 7.36 (2H, d, J=8.3 Hz).
MS (FAB) m/z: 267, 269.

Step 2: Ethyl 5'-(4-Chlorophenyl)-3'-isopropylspiro[cyclopentane-1,6'-imidazo[2,1-b][1,3]thiazole-2'-carboxylate

The compound obtained in Step 1 above was reacted with ethyl 2-chloro-4-methyl-3-oxopentanoate in the same way as in Step 1 of Example 4 to give the title compound.
$^1$H-NMR ($CDCl_3$) δ: 1. 02 (3H, d, J=7.1 Hz), 1. 03 (3H, d, J=7.1 Hz), 1.34 (3H, t, J=7.1 Hz), 1.60 (1H, m), 1.74-1.84 (2H, m), 1.91-2.00 (2H, m), 2.10 (1H, m), 2.32 (1H, m), 3.31 (1H, m), 4.30 (2H, q, J=7.1 Hz), 5.26 (1H, s), 6.77-7.14 (2H, brd), 7.42 (2H, d, J=8.5 Hz).
MS (FAB) m/z: 405, 407.

Step 3: 5'-(4-Chlorophenyl)-3'-isopropylspiro[cyclopentane-1,6'-imidazo[2,1-b][1,3]thiazole]-2'-carboxylic acid

The compound obtained in Step 2 above was reacted in the same way as in Step 3 of Example 1 to give the title compound.
$^1$H-NMR (DMSO-$d_6$) δ: 0.91 (3H, d, J=7.1 Hz), 1.01 (3H, d, J=7.1 Hz), 1.16 (1H, m), 1.39-1.52 (2H, m), 1.66 (1H, m), 1.75-1.82 (2H, m), 2.03-2.17 (2H, m), 3.20 (1H, m), 5.87 (1H, s), 7.30 (2H, brs), 7.50 (2H, d, J=7.6 Hz).
MS (FAB) m/z: 377, 379.

Step 4: 1-{[5'-(4-Chlorophenyl)-3'-isopropylspiro[cyclopentane-1,6'-imidazo[2,1-b][1,3]thiazole]-2'-yl]carbonyl-N,N-dimethyl-L-prolinamide

The compound obtained in Step 3 above was reacted in the same way as in Step 4 of Example 1 using 4-(L-prolyl)morpholine instead of piperazin-2-one to give the title compound as a mixture of diastereomers.
$^1$H-NMR ($CDCl_3$) δ: 0.87-0.99 (6H, m), 1.04-1.13 (1H, m), 1.35-1.42 (1H, m), 1.45-1.52 (1H, m), 1.66-1.81 (3H, m), 1.85-2.02 (4H, m), 2.07-2.23 (2H, m), 2.62-2.74 (1H, m), 2.94 (3H, s), 3.10 and 3.11 (total 3H, each s), 3.66-3.77 (2H, m), 4.78and4.79 (total 1H, each s), 4.86-4.89 (1H, m), 6.98-7.06 (2H, m), 7.30 (2H, d, J=8.5 Hz).
MS (FAB) m/z: 501, 503.

Example 92

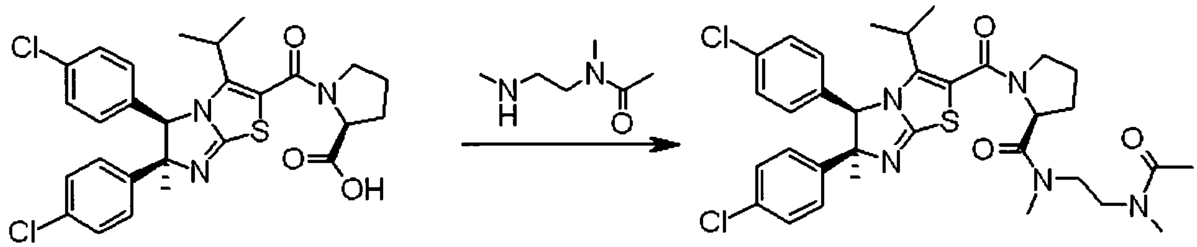

N-{2-[Acetyl(methyl)amino]ethyl}-1-{[(5R,6S)-5,6-bis(4-chlorophenyl)-3-isopropyl-6-methyl-5,6-dihydroimidazo[2,1-b][1,3]thiazol-2-yl]carbonyl}-N-methyl-L-prolinamide

The compound obtained in Step 2 of Example 61 was reacted in the same way as in Step 4 of Example 1 using N-methyl-N-[2-(methylamino)ethyl]acetamide instead of piperazin-2-one to give the title compound.
$^1$H-NMR ($CDCl_3$) δ: 0.88-0.98 (6H, m), 1.81 (3H, s), 1.89-1.96 (2H, m), 2.05 and 2.12 (total 3H, each s), 2.09-2.14 (1H, m), 2.19-2.26 (1H, m), 2.58-2.66 (1H, m), 2.95 and 3.02 (total 3H, each s), 3.10 and 3.17 (total 3H, each s), 3.35-3.43 (2H, m), 3.65-3.72 (2H, m), 3.73-3.82 (2H, m), 4.79-4.83 (1H, m), 4.93 and 4.96 (total 1H, each s), 6.70 (2H, d, J=7.6 Hz), 7.02 (2H, d, J=8.5 Hz), 7.03 (2H, d, J=8.8 Hz), 7.10 (2H, d, J=8.5 Hz).
MS (ESI) m/z: 656.

Example 93

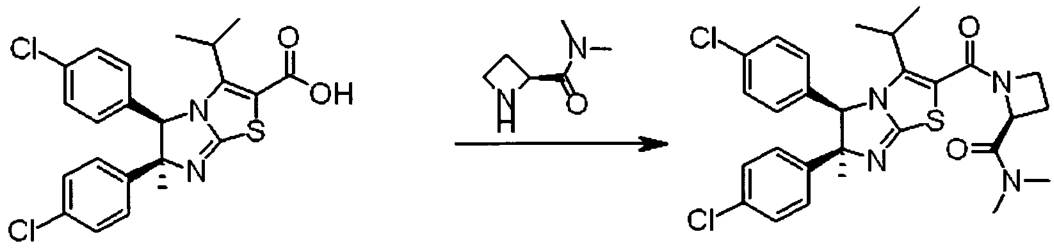

(2S)-1-{[(5R,6S)-5,6-Bis(4-chlorophenyl)-3-isopropyl-6-methyl-5,6-dihydroimidazo[2,1-b][1,3]thiazol-2-yl]carbonyl}-N,N-dimethylazetidine-2-carboxamide

The compound obtained in Step 2 of Example 61 was reacted in the same way as in Step 4 of Example 1 using (2S)-N,N-dimethylazetidine-2-carboxamide instead of piperazin-2-one to give the title compound.
$^1$H-NMR ($CDCl_3$) δ: 0.86 (3H, d, J=7.3 Hz), 1.04 (3H, d, J=7.1 Hz), 1.80 (3H, s), 2.22-2.28 (1H, m), 2.57-2.62 (1H, m), 3.00 (3H, s), 3.03 (3H, s), 3.24-3.31 (1H, m), 4.13-4.17 (1H, m), 4.38-4.43 (1H, m), 5.02 (1H, s), 5.20-5.24 (1H, m), 6.65 (2H, d, J=7.3 Hz), 7.02 (4H, d, J=8.5 Hz), 7.10 (2H, d, J=8.5 Hz).
MS (ESI) m/z: 557.

Example 94

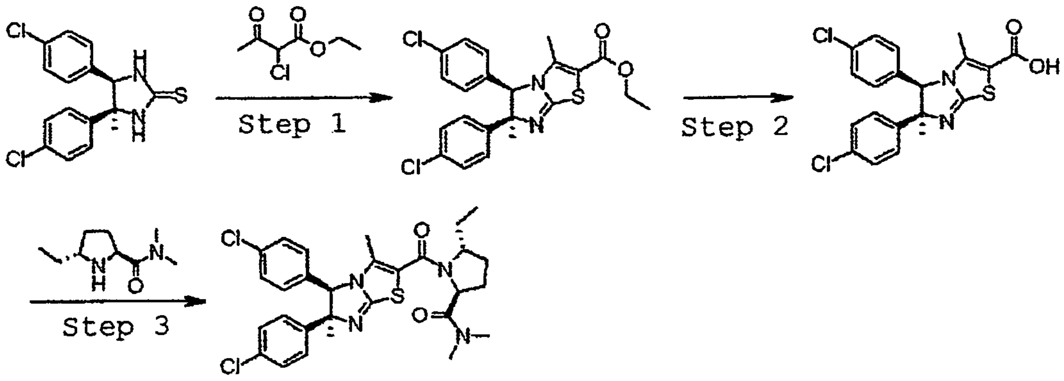


Step 1: Ethyl (5R,6S)-5,6-Bis(4-chlorophenyl)-3,6-dimethyl-5,6-dihydroimidazo[2,1-b][1,3]thiazole-2-carboxylate

The same reaction as in Step 2 of Example 1 was performed using ethyl 2-chloro-3-oxobutanoate instead of ethyl 2-chloro-4-methyl-3-oxopentanoate to give the title compound.
$^1$H-NMR ($CDCl_3$) δ: 1.31 (3H, t, J=7.2 Hz), 1.82 (3H, s), 2.03 (3H, s), 4.24 (2H, q, J=7.1 Hz), 4.97 (1H, s), 6.66-6.80 (2H, m), 7.01-7.07 (6H, m).
MS (ESI) m/z: 447.

Step 2: (5R,6S)-5,6-Bis(4-chlorophenyl)-3,6-dimethyl-5,6-dihydroimidazo[2,1-b][1,3]thiazole-2-carboxylic acid

The compound obtained in Step 1 above was reacted in the same way as in Step 3 of Example 1 to give the title compound.
$^1$H-NMR (DMSO-$d_6$) δ: 1.80 (3H, s), 2.00 (3H, s), 5.63 (1H, s), 6.94-6.99 (2H, m), 7.13 (2H, d, J=8.5 Hz), 7.17 (2H, d, J=7.3 Hz), 7.19 (2H, d, J=8.8 Hz).
MS (ESI) m/z: 419.

Step 3: (5R)-1-{[(5R,6S)-5,6-Bis(4-chlorophenyl)-3,6-dimethyl-5,6-dihydroimidazo[2,1-b][1,3]thiazol-2-yl]carbonyl}-5-ethyl-N,N-dimethyl-L-prolinamide

The compound obtained in Step 2 above was reacted in the same way as in Step 4 of Example 1 using (5R)-5-ethyl-N,N-dimethyl-L-prolinamide instead of piperazin-2-one to give the title compound.
$^1$H-NMR ($CDCl_3$) δ: 0.92 (3H, t, J=7.4 Hz), 1.41-1.44 (1H, m), 1.80 (3H, s), 1.80-1.85 (3H, m), 1.86 (3H, brs), 2.18-2.26 (2H, m), 2.91 (3H, brs), 3.10 (3H, s), 4.17-4.22 (1H, m), 4.91 (1H, s), 4.98 (1H, d, J=8.5 Hz), 6.68-6.72 (2H, m), 7.02 (4H, d, J=8.5 Hz), 7.08 (2H, d, J=8.5 Hz).
MS (ESI) m/z: 571.

Example 95

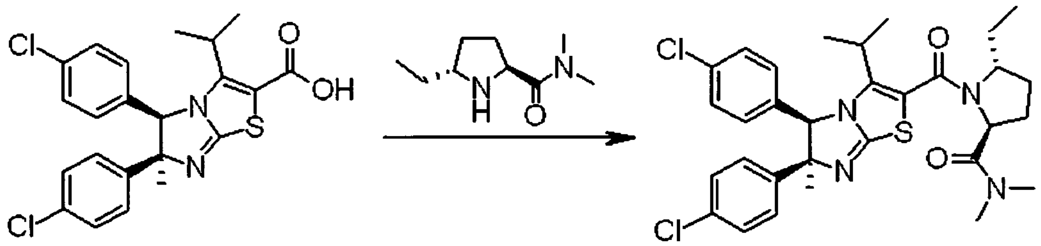

(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-ethyl-N,N-dimethyl-L-prolinamide

The same reaction as in Step 4 of Example 1 was performed using (5R)-5-ethyl-N,N-dimethyl-L-prolinamide instead of piperazin-2-one to give the title compound.
$^1$H-NMR ($CDCl_3$) δ: 0.89-1.04 (9H, m), 1.35-1.43 (1H, m), 1.58-1.64 (1H, m), 1.79 (3H, s), 1.79-1.85 (2H, m), 2.14-2.20 (1H, m), 2.25-2.32 (1H, m), 2.69-2.77 (2H, m), 2.95 (3H, s), 3.11 (3H, s), 4.37-4.40 (1H, m), 4.94 (1H, s), 4.95-4.99 (1H, m), 6.66 (2H, d, J=7.8 Hz), 7.00 (2H, d, J=9.0 Hz), 7.02 (2H, d, J=8.8 Hz), 7.14 (2H, d, J=8.3 Hz).
MS (ESI) m/z: 599.

Example 96

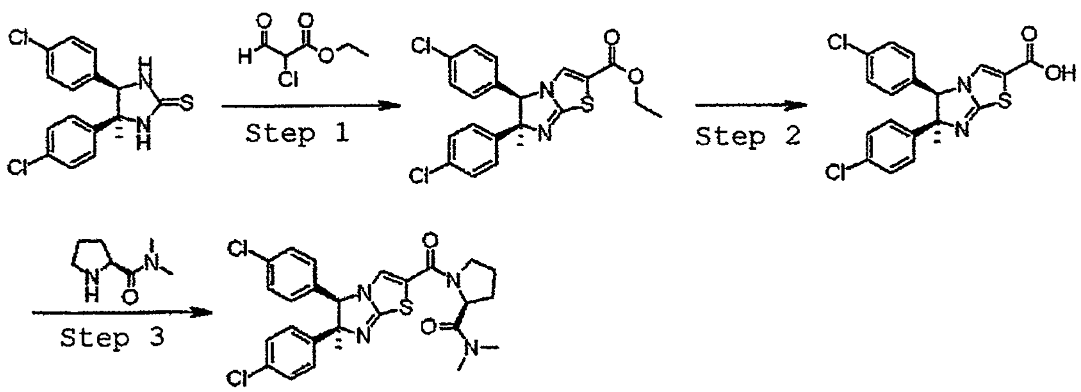


Step 1: Ethyl (5R,6S)-5,6-Bis(4-chlorophenyl)-6-methyl-5,6-dihydroimidazo[2,1-b][1,3]thiazole-2-carboxylate

The same reaction as in Step 2 of Example 1 was performed using ethyl 2-chloro-3-oxopropanoate instead of

ethyl 2-chloro-4-methyl-3-oxopentanoate to give the title compound.
$^1$H-NMR ($CDCl_3$) δ: 1.29 (3H, t, J=7.2 Hz), 1.84 (3H, s), 4.22-4.27 (2H, m), 4.99 (1H, s), 6.71 (2H, d, J=8.5 Hz), 6.97 (2H, d, J=8.8 Hz), 7.04 (2H, d, J=8. 5 Hz), 7.08 (1H, s), 7.09 (2H, d, J=8.0 Hz).
MS (ESI) m/z: 433.

Step 2: (5R,6S)-5,6-Bis(4-chlorophenyl)-6-methyl-5,6-dihydroimidazo[2,1-b][1,3]thiazole-2-carboxylic acid

The compound obtained in Step 1 above was reacted in the same way as in Example 31 to give the title compound.
$^1$H-NMR (DMSO-$d_6$) δ: 1.72 (3H, s), 5.35 (1H, s), 6.84 (2H, d, J=8.3 Hz), 7.09 (2H, d, J=8.5 Hz), 7.15 (2H, d, J=8.5 Hz), 7.17 (2H, d, J=8.8 Hz), 7.56 (1H, s).
MS (ESI) m/z: 405.

Step 3: 1-{[(5R,6S)-5,6-Bis(4-chlorophenyl)-6-methyl-5,6-dihydroimidazo[2,1-b][1,3]thiazol-2-yl]carbonyl}-N,N-dimethyl-L-prolinamide

The compound obtained in Step 2 above was reacted in the same way as in Step 4 of Example 1 using N,N-dimethyl-L-prolinamide instead of piperazin-2-one to give the title compound.
$^1$H-NMR ($CDCl_3$) δ: 1.84 (3H, s), 1.88-1.92 (1H, m), 1.95-2.00 (1H, m), 2.09-2.14 (1H, m), 2.20-2.25 (1H, m), 2.95 (3H, s), 3.14 (3H, s), 3.65-3.71 (2H, m), 4.93-4.98 (1H, m), 4.97 (1H, s), 6.71 (2H, d, J=8.3 Hz), 6.77 (1H, s), 6.96 (2H, d, J=8.8 Hz), 7.04 (2H, d, J=8.8 Hz), 7.09 (2H, d, J=8.8 Hz).

Example 97

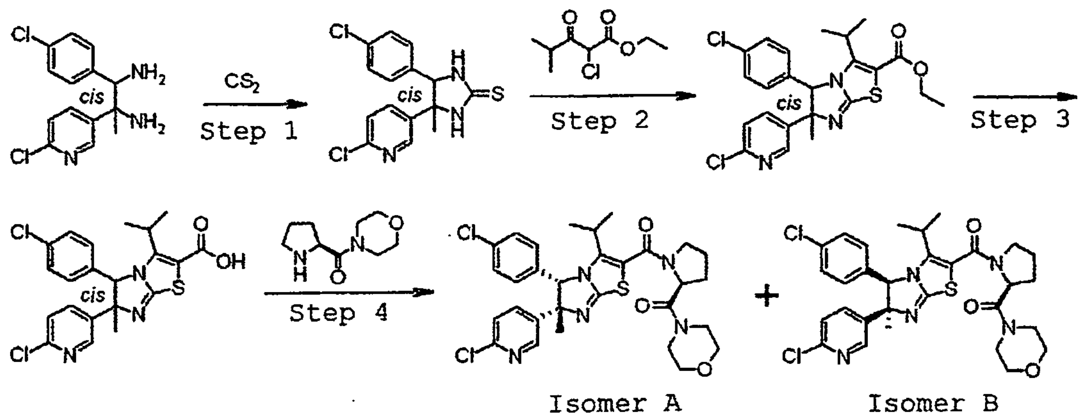


Step 1: (4R*,5S*)-5-(4-Chlorophenyl)-4-(6-chloropyridin-3-yl)-4-methylimidazolidine-2-thione

The same reaction as in Step 1 of Example 1 was performed using (1R*, 2S*)-1-(4-chlorophenyl)-2-(6-chloropyridin-3-yl)propane-1,2-diamine instead of (1R,2S)-1,2-bis(4-chlorophenyl)propane-1,2-diamine to give the title compound as a racemic mixture.
$^1$H-NMR ($CDCl_3$) δ: 1.87 (3H, s), 4.99 (1H, s), 6.83 (2H, d, J=8.3 Hz), 7.05-7.30 (5H, m), 7.95 (1H, s), 7.99 (1H, d, J=2.7 Hz).
MS (ESI) m/z: 338.

Step 2: Ethyl (5R*,6S*)-5-(4-Chlorophenyl)-6-(6-chloropyridin-3-yl)-3-isopropyl-6-methyl-5,6-dihydroimidazo[2,1-b][1,3]thiazole-2-carboxylate

The compound obtained in Step 1 above was reacted in the same way as in Step 2 of Example 1 to give the title compound as a racemic mixture.
$^1$H-NMR ($CDCl_3$) δ: 0.88 (3H, d, J=7.1 Hz), 1.01 (3H, d, J=7.1 Hz), 1.33 (3H, t, J=7.2 Hz), 1.83 (3H, s), 3.36 (1H, t, J=7.2 Hz), 4.25 (2H, q, J=7.2 Hz), 5.13 (1H, s), 6.65-6.81 (2H, m), 7.00 (1H, d, J=8.3 Hz), 7.09 (2H, d, J=8.8 Hz), 7.49 (1H,

dd, J=8.3, 2.4 Hz), 8.21 (1H, d, J=2.4 Hz).
MS (ESI) m/z: 476.

Step 3: (5R*,6S*)-5-(4-Chlorophenyl)-6-(6-chloropyridin-3-yl)-3-isopropyl-6-methyl-5,6-dihydroimidazo[2,1-b][1,3]thiazole-2-carboxylic acid

The compound obtained in Step 1 above was reacted in the same way as in Step 3 of Example 1 to give the title compound as a racemic mixture.
$^1$H-NMR ($CDCl_3$) δ: 0.97 (3H, d, J=7.1 Hz), 1.09 (3H, d, J=7.1 Hz), 2.14 (3H, s), 3.25-3.27 (2H, m), 6.27 (1H, s), 6.68 (1H, s), 7.18-7.30 (4H, m), 7.72 (1H, dd, J=8.5, 2.7 Hz), 8.26 (1H, d, J=2.7 Hz).
MS (ESI) m/z: 448.

Step 4: Isomer A: (5S,6R)-5-(4-Chlorophenyl)-6-(6-chloropyridin-3-yl)-3-isopropyl-6-methyl-2-{[(2S)-2-(morpholin-4-ylcarbonyl)pyrrolidin-1-yl]carbonyl}-5,6-dihydroimidazo[2,1-b][1,3]thiazole and Isomer B: (5R,6S)-5-(4-Chlorophenyl)-6-(6-chloropyridin-3-yl)-3-isopropyl-6-methyl-2-{[(2S)-2-(morpholin-4-ylcarbonyl)pyrrolidin-1-yl]carbonyl-5,6-dihydroimidazo[2,1-b][1,3]thiazole

The compound obtained in Step 3 above was reacted in the same way as in Step 4 of Example 1 using 4-(L-prolyl)morpholine instead of piperazin-2-one to give the diastereoisomer mixture of the title compound. Subsequently, the title compounds were resolved by an optically active column (CHIRALCEL OD-H (Daicel Chemical Industries, ltd.), 2cmϕ × 25cm, eluting solvent; hexane:ethanol = 80:20) to give isomer A (eluted earlier) and isomer B.
$^1$H-NMR ($CDCl_3$) δ: 0.89-1.02 (6H, m), 1.84 (3H, s), 1.91-1.99 (2H, m), 2.14-2.25 (2H, m), 2.63-2.77 (1H, m), 3.45-3.81 (10H, m), 4.87-4.90 (1H, m), 5.02-5.04 (1H, m), 6.71 (2H, brs), 6.99 (1H, d, J=8.1 Hz), 7.08 (2H, d, J=8.3 Hz), 7.49 (1H, dd, J=8.1, 2.4 Hz), 8.20-8.21 (1H, m).
MS (FAB) m/z: 614.

Example 98

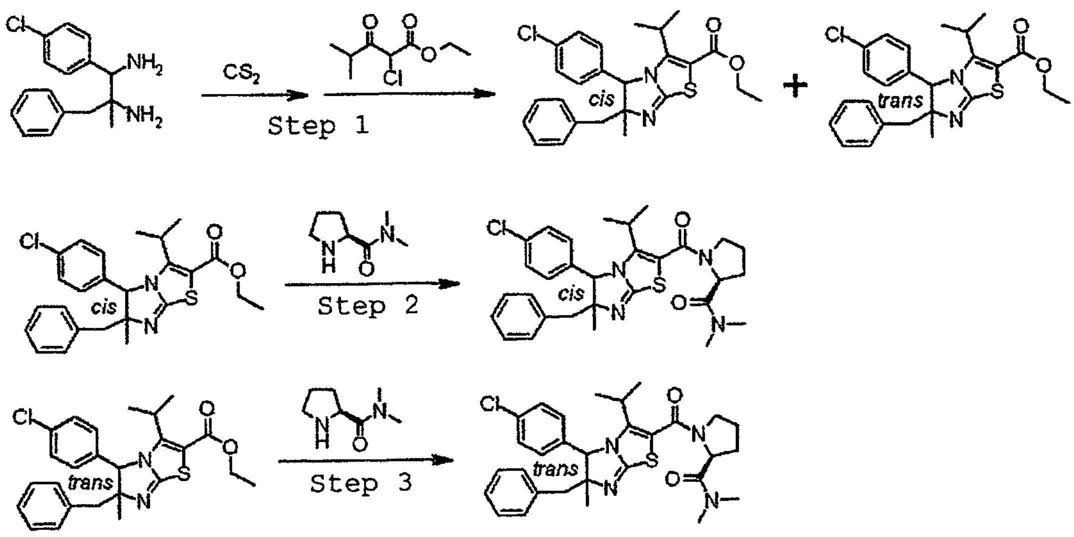


Step 1: Ethyl (5R*,6S*) - and (5R*,6R*)-6-Benzyl-5-(4-chlorophenyl)-3-isopropyl-6-methyl-5,6-dihydroimidazo[2,1-b][1,3]thiazole-2-carboxylate

A stereoisomer mixture of 1-(4-chlorophenyl)-2-methyl-3-phenylpropane-1,2-diamine was reacted in the same way as in Step 1 of Example 1 and then reacted with ethyl 2-chloro-4-methyl-3-oxopentanoate successively in the same way as in Step 2 of Example 1 to give the cis isomer and the trans isomer of the title compound as a racemic compound respectively.
cis isomer

$^1$H-NMR ($CDCl_3$) δ: 0.90 (3H, d, J=7.1 Hz), 1.02 (3H, d, J=7.1 Hz), 1.28 (2H, s), 1.32 (3H, t, J=7.3 Hz), 2.16 (1H, d, J=13.7 Hz), 2.40 (1H, d, J=13.7 Hz), 3.29-3.36 (1H, m), 4.23 (2H, q, J=7.3 Hz), 4.88 (1H, s), 6.99 (1H, dd, J=8.3, 2.2 Hz), 7.13-7.24 (6H, m), 7.31-7.42 (2H, m).
MS (ESI) m/z: 455, 457.
trans isomer
$^1$H-NMR ($CDCl_3$) δ: 0.69 (3H, d, J=7.1 Hz), 0.83 (3H, d, J=7.1 Hz), 0.93 (3H, s), 1.30 (3H, t, J=7.3 Hz), 2.85 (1H, d, J=13.7 Hz), 3.02-3.08 (2H, m), 4.14-4.27 (2H, m), 4.97 (1H, s), 6.83-6.89 (2H, m), 7.21-7.36 (7H, m).
MS (ESI) m/z: 455, 457.

Step 2: 1-{[(5R*,65*)-6-Benzyl-5-(4-chlorophenyl)-3-isopropyl-6-methyl-5,6-dihydroimidazo[2,1-b][1,3]thiazol-2-yl]carbonyl}-N,N-dimethyl-L-prolinamide

The cis isomer compound obtained in Step 1 above was reacted in the same way as in Step 4 of Example 1 using N,N-dimethyl-L-prolinamide instead of piperazin-2-one to give the title compound as a mixture of diastereomers. $^1$H-NMR ($CDCl_3$) δ: 0.81-1.08 (6H, m), 1.17-1.39 (3H, m), 1.84-2.01 (2H, m), 2.05-2.30 (3H, m), 2.39 (1H, d, J=13.2 Hz), 2.58-2.84 (1H, m), 2.95 (3H, s), 3.05-3.18 (3H, m), 3.61-3.88 (2H, m), 4.71-5.01 (2H, m), 6.99-7.56 (9H, m). MS (ESI) m/z: 551, 553.

Step 3: 1-([(5R*,6R*)-6-Benzyl-5-(4-chlorophenyl)-3-isopropyl-6-methyl-5,6-dihydroimidazo[2,1-b][1,3]thiazol-2-yl]carbonyl}-N,N-dimethyl-L-prolinamide

The trans isomer compound obtained in Step 1 above was reacted in the same way as in Step 4 of Example 1 using N,N-dimethyl-L-prolinamide instead of piperazin-2-one to give the title compound as a mixture of diastereomers. $^1$H-NMR ($CDCl_3$) δ: 0.63-0.73 (3H, m), 0.78-1.00 (6H, m), 1.46-2.70 (5H, m), 2.79-3.19 (2H, m), 2.91-2.97 (3H, m), 3.10 (3H, s), 3.32-3.80 (2H, m), 4.75-5.03 (2H, m), 6.66-7.08 (2H, m), 7.16-7.52 (7H, m).
MS (ESI) m/z: 551, 553.

Example 99

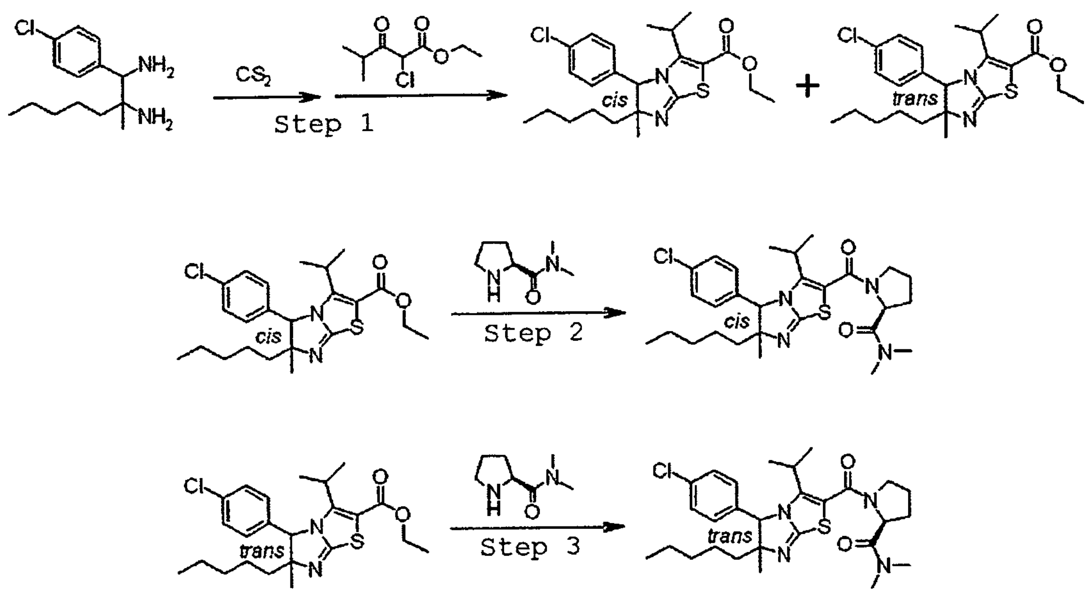


Step 1 : Ethyl (5R*,6S*) - and (5R*,6R*) -5-(4-Chlorophenyl)-3-isopropyl-6-methyl-6-pentyl-5,6-dihydroimidazo[2,1-b][1,3]thiazole-2-carboxylate

A stereoisomer mixture of 1-(4-chlorophenyl)-2-methylheptane-1,2-diamine was reacted in the same way as in Step 1 of Example 1 and then reacted with ethyl 2-chloro-4-methyl-3-oxopentanoate successively in the same way

as in Step 2 of Example 1 to give the cis isomer and the trans isomer of the title compound as a racemic compound respectively.
cis isomer
$^1$H-NMR ($CDCl_3$) δ: 0.84 (3H, s), 0.87-0.93 (6H, m), 1.03 (3H, d, J=7.1 Hz), 1.29-1.45 (6H, m), 1.32 (3H, t, J=7.3 Hz), 1.64-1.72 (2H, m), 3.28-3.37 (1H, m), 4.23 (2H, q, J=7.1 Hz), 4.88 (1H, s), 6.89 (1H, d, J=8.0 Hz), 7.07 (1H, d, J=7.6 Hz), 7.31-7.38 (2H, m).
MS (ESI) m/z: 435.
trans isomer
$^1$H-NMR ($CDCl_3$) δ: 0.78 (3H, t, J=7.2 Hz), 0.88 (3H, d, J=7.1 Hz), 0.91-1.44 (8H, m), 0.99 (3H, d, J=7.6 Hz), 1.31 (3H, t, J=7.2 Hz), 1.40 (3H, s), 3.25-3.38 (1H, m), 4.17-4.26 (2H, m), 4.78 (1H, s), 6.85-6.93 (1H, m), 7.05-7.12 (1H, m), 7.26-7.36 (2H, m).
MS (ESI) m/z: 435.

Step 2: 1-{[(5R*,6S*)-5-(4-chlorophenyl)-3-isopropyl-6-methyl-6-pentyl-5,6-dihydroimidazo[2,1-b][1,3]thiazol-2-yl]carbonyl}-N,N-dimethyl-L-prolinamide

The cis isomer compound obtained in Step 1 above was reacted in the same way as in Step 4 of Example 1 using N,N-dimethyl-L-prolinamide instead of piperazin-2-one to give the title compound as a mixture of diastereomers.
$^1$H-NMR ($CDCl_3$) δ: 0.78 (3H, t, J=7.3 Hz), 0.83-1.29 (11H, m), 1.40 (3H, s), 1.58 (3H, s), 1.86-1.98 (2H, m), 2.06-2.27 (2H, m), 2.61-2.79 (1H, m), 2.94 (3H, s), 3.11 and 3.12 (total 3H, each s), 3.64-3.81 (2H, m), 4.68 and 4.70 (total 1H, each s), 4.84-4.92 (1H, m), 6.87-6.96 (1H, m), 7.02-7.11 (1H, m), 7.25-7.34 (2H, m).
MS (ESI) m/z: 531.

Step 3: 1-{[(5R*,6R*)-(4-chlorophenyl)-3-isopropyl-6-methyl-6-pentyl-5,6-dihydroimidazo[2,1-b][1,3]thiazol-2-yl]carbonyl}-N,N-dimethyl-L-prolinamide

The trans isomer compound obtained in Step 1 above was reacted in the same way as in Step 4 of Example 1 using N,N-dimethyl-L-prolinamide instead of piperazin-2-one to give the title compound as a mixture of diastereomers.
$^1$H-NMR ($CDCl_3$) δ: 0.82 and 0.82 (total 3H, each s), 0.84-0.95 (6H, m), 1.01 (3H, t, J=8.0 Hz), 1.23-1.37 (4H, m), 1.38-1.47 (2H, m), 1.57-1.71 (3H, m), 1.86-1.97 (2H, m), 2.05-2.28 (1H, m), 2.62-2.79 (1H, m), 2.95 and 2.95 (total 3H, each s), 3.12 and 3.12 (total 3H, each s), 3.66-3.81 (2H, m), 4.78 and 4.79 (total 1H, each s), 4.84-4.93 (1H, m), 6.86-6.94 (1H, m), 7.01-7.07 (1H, m), 7.28-7.35 (2H, m).
MS (ESI) m/z: 531.

Example 100

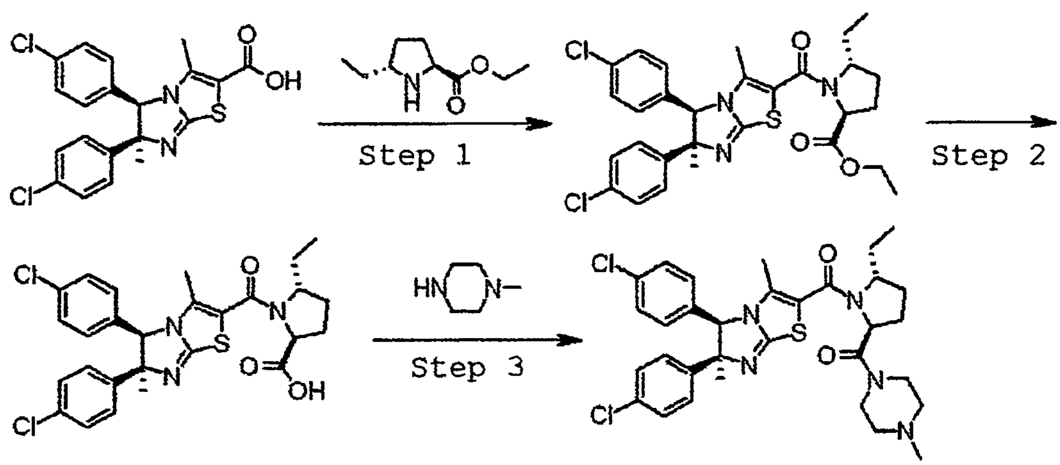

Step 1: Ethyl (5R)-1-{[(5R,6S)-5,6-Bis(4-chlorophenyl)-3,6-dimethyl-5,6-dihydroimidazo[2,1-b][1,3]thiazol-2-yl]carbonyl}-5-ethyl-L-prolinate

The compound obtained in Step 2 of Example 94 was reacted in the same way as in Step 4 of Example 1 using ethyl (5R)-5-ethyl-L-prolinate instead of piperazin-2-one to give the title compound.
$^1$H-NMR ($CDCl_3$) δ: 0.91 (3H, t, J=7.3 Hz), 1.23 (3H, t, J=7.3 Hz), 1.35-1.45 (2H, m), 1.79-1.86 (2H, m), 1.80 (3H, s), 1.82 (3H, s), 2.07-2.21 (2H, m), 4.14-4.26 (3H, m), 4.60-4.66 (1H, m), 4.92 (1H, s), 6.68-6.74 (2H, m), 7.01-7.05 (4H, m), 7.08 (2H, d, J=8.8 Hz).
MS (ESI) m/z: 572.

Step 2: (5R)-1-{[(5R,6S)-5,6-Bis(4-chlorophenyl)-3,6-dimethyl-5,6-dihydroimidazo[2,1-b][1,3]thiazol-2-yl]carbonyl}-5-ethyl-L-proline

The compound obtained in Step 1 above was reacted in the same way as in Step 3 of Example 1 to give the title compound.
$^1$H-NMR (DMSO-$d_6$) δ: 0.80 (3H, t, J=7.6 Hz), 1.31-1.43 (1H, m), 1.68-1.83 (2H, m), 1.73 (3H, s), 1.76 (3H, s), 1.90-2.03 (2H, m), 2.16-2.35 (1H, m), 4.02-4.12 (1H, m), 4.38-4.49 (1H, m), 5.52 (1H, s), 7.10-7.24 (8H, m).
MS (ESI) m/z: 544.

Step 3: (5R,6S)-5,6-Bis(4-chlorophenyl)-2-({(2R,5S)-2-ethyl-5-[(4-methylpiperazin-1-yl)carbonyl]pyrrolidin-1-yl}carbonyl)-3,6-dimethyl-5,6-dihydroimidazo[2,1-b][1,3]thiazole

The compound obtained in Step 2 above was reacted in the same way as in Step 4 of Example 1 using 1-methylpiperazine instead of piperazin-2-one to give the title compound.
$^1$H-NMR ($CDCl_3$) δ: 1.40-1.45 (1H, m), 1.74-1.82 (3H, m), 1.80 (3H, s), 1.86 (3H, s), 2.13-2.24 (2H, m), 2.31 (3H, s), 2.35-2.43 (3H, m), 2.49-2.54 (1H, m), 3.48-3.54 (2H, m), 3.60-3.67 (2H, m), 4.17-4.22 (1H, m), 4.92 (1H, s), 5.00 (1H, d, J=7.3 Hz), 6.67-6.72 (2H, m), 7.01-7.09 (6H, m).
MS (ESI) m/z: 626.

Example 101

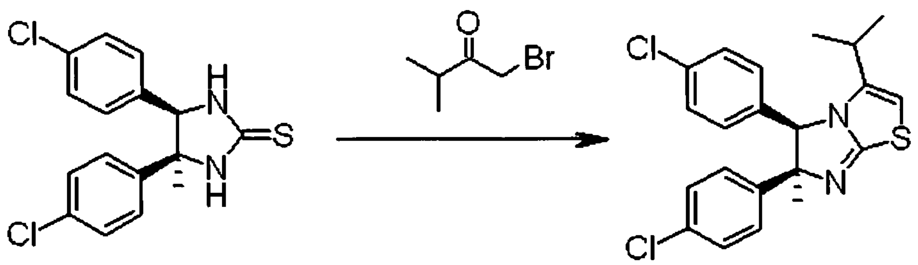

(5R,6S)-5,6-Bis(4-chlorophenyl)-3-isopropyl-6-methyl-5,6-dihydroimidazo[2,1-b][1,3]thiazole

The same reaction as in Step 2 of Example 1 was performed using 1-bromo-3-methylbutan-2one instead of ethyl 2-chloro-4-methyl-3-oxopentanoate to give the title compound.
$^1$H-NMR ($CDCl_3$) δ: 0.90 (3H, d, J=6.8 Hz), 1.11 (3H, d, J=6.8 Hz), 1.82 (3H, s), 1.91-1.97 (1H, m), 4.91 (1H, s), 5.38 (1H, d, J=1.2 Hz), 6.69 (2H, d, J=8.5 Hz), 7.02 (4H, d, J=8.8 Hz), 7.11 (2H, d, J=8.8 Hz).

Example 102

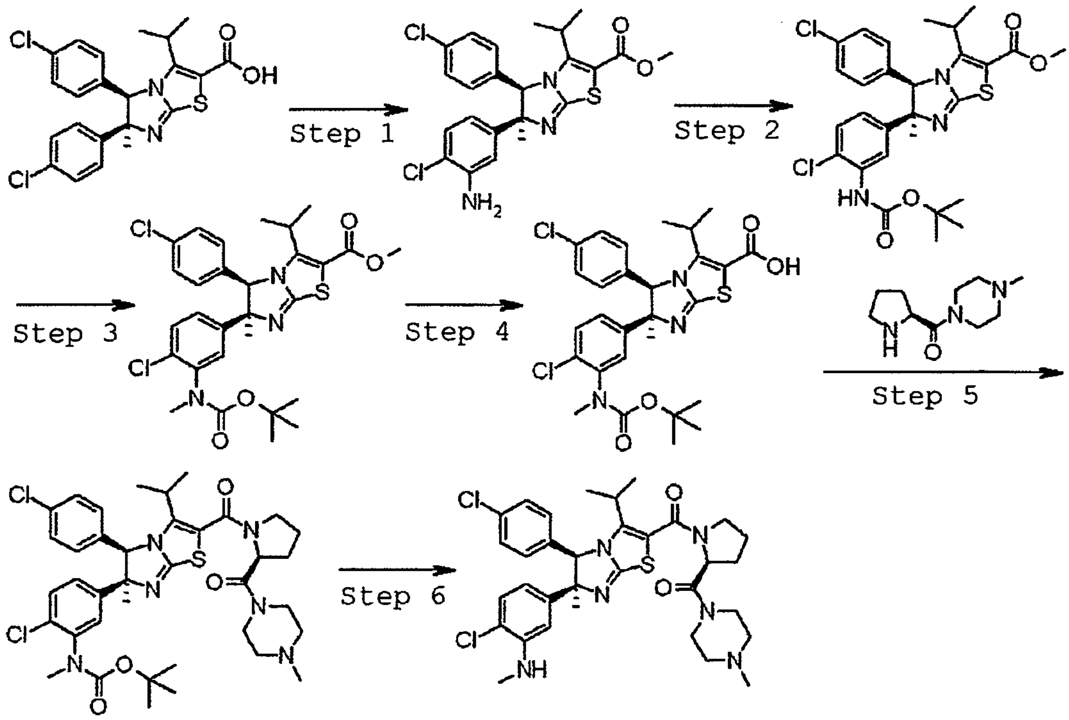


Step 1: Methyl (5R,6S)-6-(3-Amino-4-chlorophenyl)-5-(4-chlorophenyl)-3-isopropyl-6-methyl-5,6-dihydroimidazo[2,1-b][1,3]thiazole-2-carboxylate

The compound obtained in Step 3 of Example 1 was reacted in the same way as in Step 1 and Step 2 of Example 71 to give the title compound.

Step 2: Methyl (5R,6S)-6-{3-[(tert-Butoxycarbonyl)amino]-4-chlorophenyl}-5-(4-chlorophenyl)-3-isopropyl-6-methyl-5,6-dihydroimidazo[2,1-b][1,3]thiazole-2-carboxylate

Di-tert-butyl dicarbonate (1.05 g, 4.82 mmol) and 4-(N,N-dimethylamino) pyridine (0.65 g, 5.31 mmol) were added to an acetonitrile (25 ml) solution of the compound (2.30 g, 4.82 mmol) obtained in Step 1 above and the resulting mixture was stirred at room temperature for 6 hours. After the reaction mixture was concentrated, the residue was purified by silica gel column chromatography to give the title compound (1.46 g, 52%) as a pale yellow solid.

Step 3: Methyl (5R,6S)-6-{3-[(tert-Butoxycarbonyl)(methyl)amino]-4-chlorophenyl}-5-(4-chlorophenyl)-3-isopropyl-6-methyl-5,6-dihydroimidazo[2,1-b][1,3]thiazole-2-carboxylate

A tetrahydrofuran solution (1.0 M; 2.80 ml) of lithium bis(trimethylsilyl)amide was added dropwise to a tetrahydrofuran (20 ml) solution of the compound (1.46 g, 2.53 mmol) obtained Step 2 above under ice cooling and the resulting mixture was stirred at the same temperature for 40 minutes. Then Bromomethane (0.32 ml, 5.06 mmol) was added and the mixture allowed to gradually warm to room temperature. After the reaction mixture was stirred for 1 hour, saturated ammonium chloride aqueous solution was added and the reaction mixture was extracted with ethyl acetate. The organic layer was washed with brine and then dried over anhydrous sodium sulfate. After the solvent was evaporated under reduced pressure, the residue was purified by silica gel column chromatography to give the title compound (440 mg, 30%) as a yellow solid.
$^1$H-NMR ($CDCl_3$) δ: 0.88 (3H, m), 1.00 (3H, m), 1.29 (9H, brs), 1.79-1.83 (3H, m), 2.91-2.99 (3H, m), 3.21-3.47 (1H, m), 3.79 (3H, s), 5.04-5.11 (1H, m), 6.63-6.74 (2H, brs), 7.00-7.12 (5H, m).

Step 4: (5R,6S)-6-{3-[(tert-Butoxycarbonyl)(methyl)amino]-4-chlorophenyl}-5-(4-chlorophenyl)-3-isopropyl-6-methyl-5,6-dihydroimidazo[2,1-b][1,3]thiazole-2-carboxylic acid

The compound obtained in Step 3 above was reacted in the same way as in Step 3 of Example 1 to give the

title compound.
$^1$H-NMR (DMSO-$d_6$) δ: 0.80-0.97 (6H, m), 1.21 (9H, brs), 1.80-1.93 (3H, m), 2.78-2.93 (3H, m), 3.74 (1H, m), 5.90 (1H, brs), 6.58-6.73 (2H, m), 7.11-7.32 (5H, m).

Step 5: tert-Butyl 12-Chloro-5-[(5R,6S)-5-(4-chlorophenyl)-3-isopropyl-6-methyl-2-({(2S)-2-[(4-methylpiperazin-1-yl)carbonyl]pyrrolidin-1-yl}carbonyl)-5,6-dihydroimidazo[2,1-b][1,3]thiazol-6-yl]phenyl} methyl carbamate

The compound obtained in Step 4 above was reacted in the same way as in Step 4 of Example 1 using 1-methyl-4-(L-prolyl)piperazine instead of piperazin-2-one to give the title compound.
$^1$H-NMR ($CDCl_3$) δ: 0.97 (6H, brd, J=7.1 Hz), 1.32 (9H, brs), 1.81 (3H, brs), 1.87-1.94 (2H, m), 2.09-2.25 (2H, m), 2.30 (3H, brs), 2.35-2.76 (5H, m), 2.94 (3H, brs), 3.47-3.86 (6H, m), 4.89-5.00 (2H, m), 6.66-6.79 (2H, m), 7.00-7.11 (5H, m).
MS (ESI) m/z: 755, 757.

Step 6: (5R,6S)-6-(3-Amino-4-chlorophenyl)-5-(4-chlorophenyl)-3-isopropyl-6-methyl-2-({(2S)-2-[(4-methylpiperazin-1-yl)carbonyl]pyrrolidin-1-yl}carbonyl)-5,6-dihydroimidazo[2,1-b][1,3]thiazole

Trifluoroacetic acid (3 ml) was added dropwise to a dichloromethane (6 ml) solution of the compound (420 mg, 0.55 mmol) obtained in Step 5 above under ice cooling, and was stirred at room temperature for 2 hours. After the solvent was evaporated under reduced pressure, 1 N aqueous sodium hydroxide solution was added to the residue to make the solvent basic and then the solution was extracted with dichloromethane. The organic layer was washed with brine and then dried over anhydrous sodium sulfate. After the solvent was evaporated under reduced pressure, the residue was purified by silica gel column chromatography to give the title compound (303 mg, 83%) as a light brown solid.
$^1$H-NMR ($CDCl_3$) δ: 0.97 (3H, d, J=7.1 Hz), 0.98 (3H, d, J=7.1 Hz), 1.80 (3H, s), 1.90-1.95 (2H, m), 2.07-2.22 (2H, m), 2.30 (3H, s), 2.33-2.34 (4H, m), 2.67 (1H, m), 2.77 (3H, d, J=3.7 Hz), 3.55-3.67 (4H, m), 3.71 (1H, m), 3.79 (1H, m), 4.08 (1H, m), 4.93 (1H, s), 4.94 (1H, m), 6.42 (1H, dd, J=8.3, 2.0 Hz), 6.49 (1H, d, J=2.0 Hz), 6.74 (2H, d, J=8.3 Hz), 6.91 (1H, d, J=8.3 Hz), 7.02 (2H, d, J=8.3 Hz).
MS (FAB) m/z: 655, 657.

Example 103

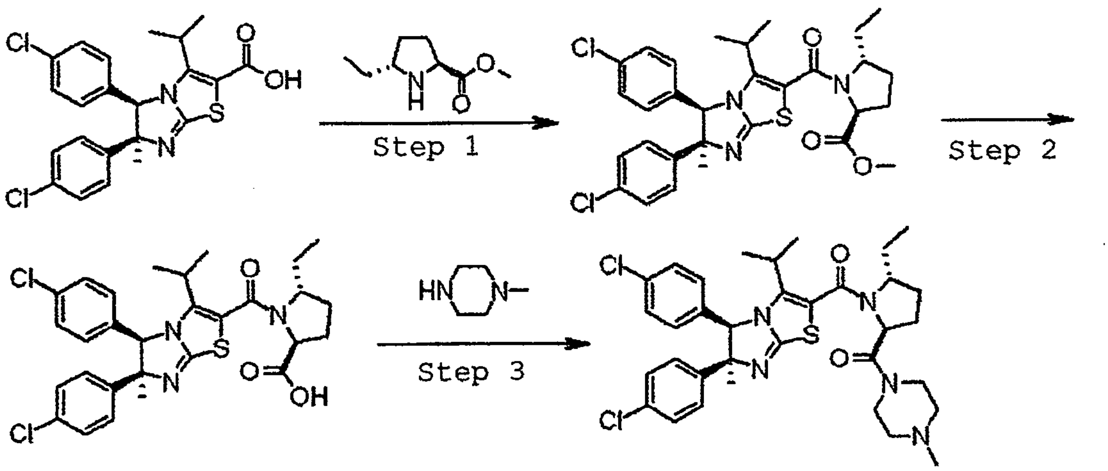


Step 1: Methyl (5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-ethyl-L-prolinate

The same reaction as in Step 4 of Example 1 was performed using methyl (5R)-5-ethyl-L-prolinate instead of piperazin-2-one to give the title compound.
$^1$H-NMR ($CDCl_3$) δ: 0.91-0.96 (6H, m), 0.98-1.04 (3H, m), 1.35-1.43 (1H, m), 1.77-1.83 (2H, m), 1.80 (3H, s), 1.99-2.04 (1H, m), 2.11-2.19 (1H, m), 2.25-2.31 (1H, m), 2.68-2.77 (1H, m), 3.69 (3H, s), 4.29-4.35 (1H, m), 4.95 (1H, s), 6.65 (2H, d, J=8.3 Hz), 7.01 (2H, d, J=8.5 Hz), 7.03 (2H, d, J=8.5 Hz), 7.13 (2H, d, J=8.5 Hz).
MS (ESI) m/z: 586.

Step 2: (5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-ethyl-L-proline

The compound obtained in Step 1 above was reacted in the same way as in Step 3 of Example 1 to give the title compound.
$^1$H-NMR (DMSO-$d_6$) δ: 0.78-0.87 (6H, m), 0.93-0.98 (3H, m), 1.34-1.40 (1H, m), 1.71-2.03 (3H, m), 2.19-2.39 (1H, m), 2.65-2.76 (1H, m), 4.04-4.17 (1H, m), 4.34-4.46 and 4.62-4.73 (total 1H, each m), 5.74-5.84 (1H, m), 7.13-7.18 (4H, m), 7.21-7.29 (4H, m).
MS (ESI) m/z: 572.

Step 3: (5R,6S)-5,6-Bis(4-chlorophenyl)-2-({(2R,5S)-2-ethyl-5-[(4-methylpiperazin-1-yl)carbonyl]pyrrolidin-1-yl}carbonyl)-3-isopropyl-6-methyl-5,6-dihydroimidazo[2,1-b][1,3]thiazole

The compound obtained in Step 2 above was reacted in the same way as in Step 4 of Example 1 using 1-methylpiperazine instead of piperazin-2-one to give the title compound.
$^1$H-NMR ($CDCl_3$) δ: 0.91-0.96 (6H, m), 1.02-1.06 (3H, m), 1.38-1.44 (1H, m), 1.66-1.85 (2H, m), 1.79 (3H, s), 2.22-2.43 (6H, m), 2.31 (3H, s), 2.51-2.59 (1H, m), 2.70-2.77 (1H, m), 3.52-3.66 (4H, m), 4.38 (1H, t, J=8.7 Hz), 4.94 (1H, s), 4.99-5.02 (1H, m), 6.63-6.68 (2H, m), 6.99-7.03 (4H, m), 7.12-7.15 (2H, m).
MS (ESI) m/z: 654.

Example 104

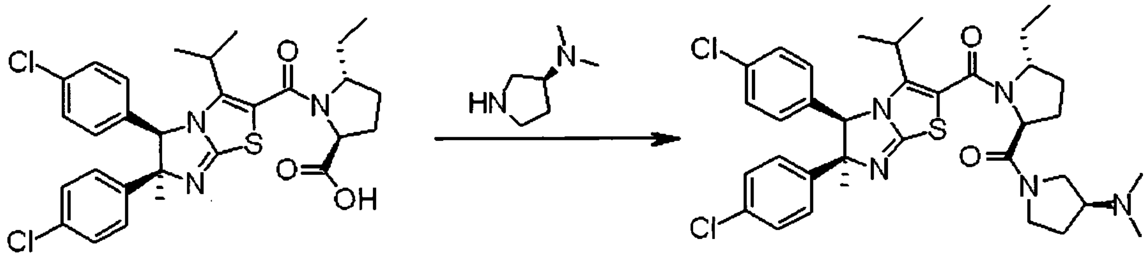

(3S)-1-((5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-ethyl-L-prolyl)-N,N-dimethylpyrrolidin-3-amine

The compound obtained in Step 2 of Example 103 was reacted in the same way as in Step 4 of Example 1 using (3S)-N,N-dimethylpyrrolidin-3-amine instead of piperazin-2-one to give the title compound.
$^1$H-NMR ($CDCl_3$) δ: 0.90-0.98 (6H, m), 1.04 (3H, d, J=6.6 Hz), 1.36-1.45 (1H, m), 1.68-1.88 (4H, m), 1.80 (3H, s), 2.06-2.22 (3H, m), 2.25 (3H, s), 2.28 (3H, s), 2.66-2.83 (2H, m), 3.18-3.24 (1H, m), 3.39-3.46 (1H, m), 3.66-3.77 (1H, m), 3.96-4.04 (1H, m), 4.33-4.41 (1H, m), 4.76-4.82 (1H, m), 4.94 (1H, s), 6.64-6.68 (2H, m), 6.99-7.06 (4H, m), 7.12-7.17 (2H, m).
MS (ESI) m/z: 668.

Example 105

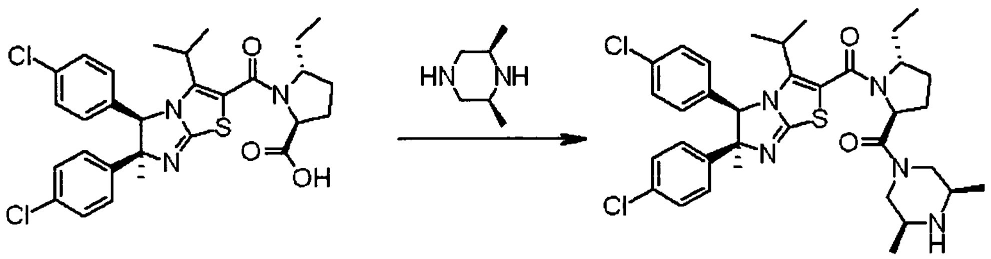

(5R,6S)-5,6-Bis(4-chlorophenyl)-2-[((2S,5R)-2-{[(3R,5S)-3,5-dimethylpiperazin-1-yl]carbonyl}-5-ethylpyrrolidin-1-yl) carbonyl]-3-isopropyl-6-methyl-5,6-dihydroimidazo[2,1-b][1,3]thiazole

The compound obtained in Step 2 of Example 103 was reacted in the same way as in Step 4 of Example 1 using cis-2,6-dimethylpiperazine instead of piperazin-2-one to give the title compound.
$^{1}$H-NMR ($CDCl_3$) δ: 0.91-0.97 (6H, m), 1.02-1.11 (9H, m), 1.37-1.45 (1H, m), 1.62 (1H, s), 1.69-1.83 (3H, m), 2.12-2.29 (3H, m), 2.65-2.88 (4H, m), 3.65-3.72 (1H, m), 4.34-4.41 (1H, m), 4.42-4.49 (1H, m), 4.94 (1H, s), 5.00-5.06 (1H, m), 6.67 (2H, d, J=7.6 Hz), 7.00 (2H, d, J=7.8 Hz), 7.02 (2H, d, J=8.1 Hz), 7.12-7.16 (2H, m).
MS (ESI) m/z: 668.

Example 106

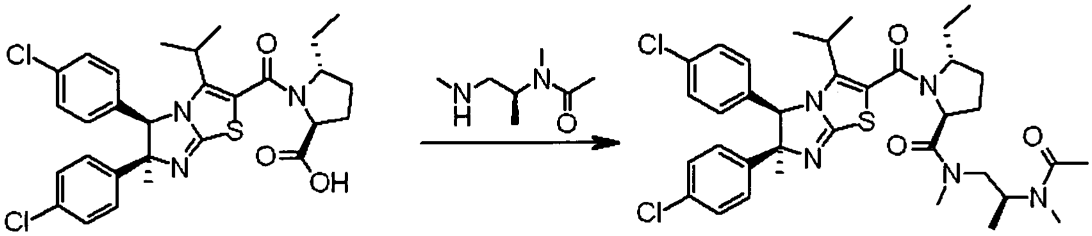

(5R)-N-{(2S)-2-[Acetyl(methyl)amino]propyl}-1-{[(5R,6S)-5,6-bis(4-chlorophenyl)-3-isopropyl-6-methyl-5,6-dihydroimidazo[2,1-b][1,3]thiazol-2-yl]carbonyl}-5-ethyl-N-methyl-L-prolinamide

The compound obtained in Step 2 of Example 103 was reacted in the same way as in Step 4 of Example 1 using N-methyl-N-[(1S)-1-methyl-2-(methylamino)]acetamide instead of piperazin-2-one to give the title compound.
$^{1}$H-NMR ($CDCl_3$) δ: 0.91-0.97 (6H, m), 1.02-1.11 (9H, m), 1.37-1.45 (1H, m), 1.62 (1H, s), 1.69-1.83 (3H, m), 2.12-2.29 (3H, m), 2.65-2.88 (4H, m), 3.65-3.72 (1H, m), 4.34-4.41 (1H, m), 4.42-4.49 (1H, m), 4.94 (1H, s), 5.00-5.06 (1H, m), 6.67 (2H, d, J=7.6 Hz), 7.00 (2H, d, J=7.8 Hz), 7.02 (2H, d, J=8.1 Hz), 7.12-7.16 (2H, m).
MS (ESI) m/z: 698.

Example 107

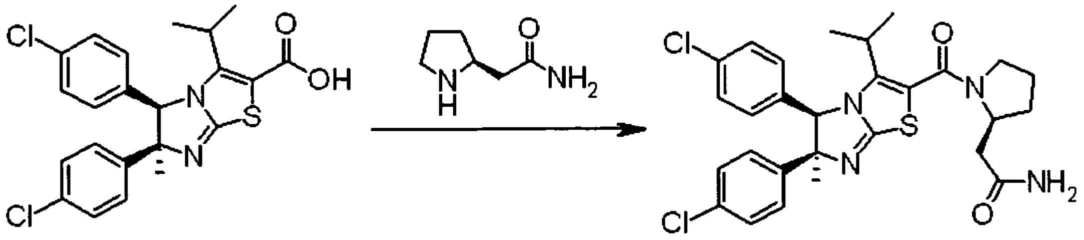

2-((2S)-1-{[(5R,6S)-5,6-Bis(4-chlorophenyl)-3-isopropyl-6-methyl-5,6-dihydroimidazo[2,1-b][1,3]thiazol-2-yl]carbonyl} pyrrolidin-2-yl)acetamide

The compound obtained in Step 3 of Example 1 was reacted in the same way as in Step 4 of Example 1 using ((2S)-pyrrolidin-2-yl)acetamide instead of piperazin-2-one to give the title compound.
$^1$H-NMR ($CDCl_3$) δ: 0.94 (3H, d, J=7.1 Hz), 1.04 (3H, d, J=7.1 Hz), 1.76-1.89 (1H, m), 1.81 (3H, s), 1.93-2.07 (2H, m), 2.12-2.25 (1H, m), 2.46 (1H, dd, J=14.2, 8.3 Hz), 2.51-2.62 (1H, m), 2.79 (1H, dd, J=14.3, 3.3 Hz), 3.53-3.68 (2H, m), 4.29-4.39 (1H, m), 4.95 (1H, s), 5.26 (1H, brs), 6.00 (1H, brs), 6.65-6.73 (2H, m), 7.00-7.06 (4H, m), 7.07-7.13 (2H, m).
MS (ESI) m/z: 557.

Example 108

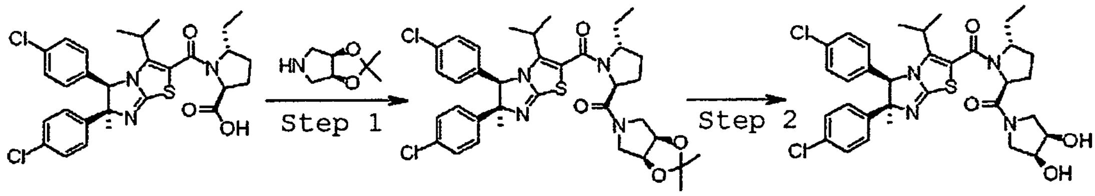


Step 1: (3aR,6aS)-5-((5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-ethyl-L-prolyl)-2,2-dimethyltetrahydro-3aH-[1,3]dioxolo[4,5-c]pyrrole

The compound obtained in Step 2 of Example 103 was reacted in the same way as in Step 4 of Example 1 using (3aR,6aS)-2,2-dimethyltetrahydro-3aH-[1,3]dioxolo[4, 5-c]pyrrole instead of piperazin-2-one to give the title compound.
$^1$H-NMR ($CDCl_3$) δ: 0.93 (6H, t, J=7.6 Hz), 1.04 (3H, d, J=6.8 Hz), 1.32 (3H, s), 1.37-1.41 (1H, m), 1.44 (3H, s), 1.74-1.84 (3H, m), 1.78 (3H, s), 1.96-2.02 (1H, m), 2.20-2.34 (2H, m), 2.73-2.81 (1H, m), 3.71-3.77 (2H, m), 3.97 (1H, d, J=13.9 Hz), 4.33-4.38 (1H, m), 4.70-4.81 (3H, m), 4.93 (1H, s), 6.66 (2H, d, J=8.5 Hz), 6.98-7.04 (4H, m), 7.13 (2H, d, J=8.3 Hz).
MS (ESI) m/z: 697.

Step 2: (3R,4S)-1-((5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-ethyl-L-prolyl)pyrrolidine-3,4-diol

The compound obtained in Step 1 above was reacted in the same way as in Step 2 of Example 66 to give the title compound.
$^1$H-NMR ($CDCl_3$) δ: 0.91-0.95 (6H, m), 1.03 (3H, d, J=7.1 Hz), 1.36-1.44 (1H, m), 1.73-1.81 (2H, m), 1.80 (3H, d, J=2.9 Hz), 1.87-1.94 (1H, m), 2.13-2.21 (1H, m), 2.27-2.34 (1H, m), 2.71-2.77 (1H, m), 3.39-3.46 (1H, m), 3.52-3.59 (1H, m), 3.63 (1H, dd, J=10.5, 5.9 Hz), 3.69-3.76 (1H, m), 4.18-4.37 (3H, m), 4.70 (1H, d, J=7.8 Hz), 4.93 (1H, s), 6.64-6.68 (2H, m), 6.99-7.04 (4H, m), 7.07-7.13 (2H, m).
MS (ESI) m/z: 657.

Example 109

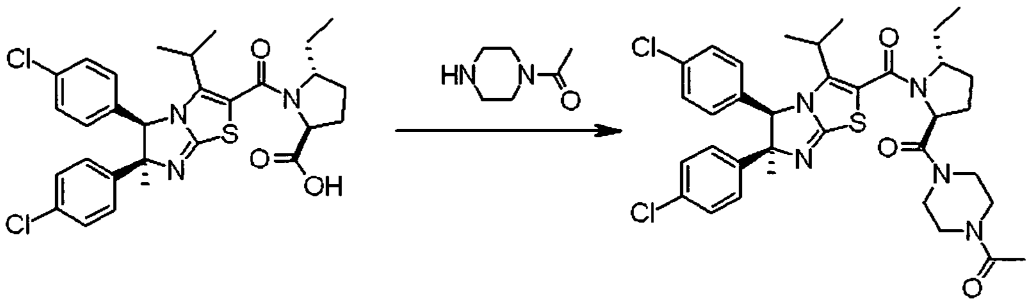

(5R,6S)-2-({(2S,5R)-2-[(4-Acetylpiperazin-1-yl)carbonyl]-5-ethylpyrrolidin-1-yl}carbonyl)-5,6-bis(4-chlorophenyl)-3-isopropyl-6-methyl-5,6-dihydroimidazo[2,1-b][1,3]thiazole

The compound obtained in Step 2 of Example 103 was reacted in the same way as in Step 4 of Example 1 using 1-acetylpiperazine instead of piperazin-2-one to give the title compound.
$^1$H-NMR ($CDCl_3$) δ: 0.94 (6H, t, J=7.3 Hz), 1.03 (3H, d, J=7.1 Hz), 1.36-1.46 (1H, m), 1.77-1.84 (3H, m), 1.78 (3H, s), 2.10 (3H, s), 2.20-2.31 (2H, m), 2.72-2.79 (1H, m), 3.44-3.52 (4H, m), 3.61-3.75 (4H, m), 4.35-4.39 (1H, m), 4.93 (1H, s), 4.95-4.99 (1H, m), 6.67 (2H, d, J=8.3 Hz), 6.98-7.03 (4H, m), 7.13 (2H, d, J=8.5 Hz).
MS (ESI) m/z: 682.

Example 110

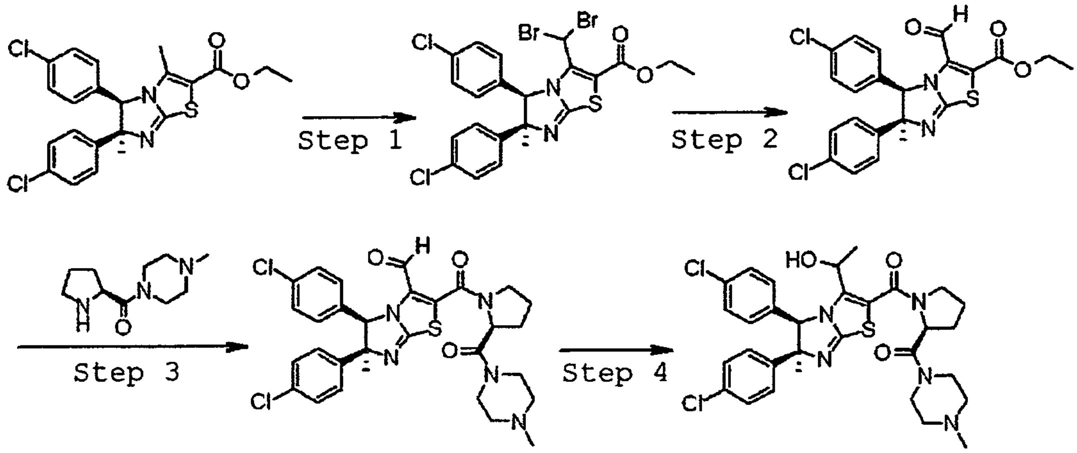


Step 1: Ethyl (5R,6S)-5,6-Bis(4-chlorophenyl)-3-(dibromomethyl)-6-methyl-5,6-dihydroimidazo[2,1-b][1,3]thiazole-2-carboxylate

N-Bromosuccinimide (4.37 g, 24.5 mmol) and 2,2'-azobis (isobutyronitrile) (0.161 g, 0.981 mmol) were added to a carbon tetrachloride (100 ml) solution of the compound (4.39 g, 9.81 mmol) obtained in Step 1 of Example 94 and the resulting mixture was heated to reflux for 20 hours after the atmosphere was substituted with nitrogen. After the reaction mixture was cooled, the insoluble matter was removed by suction filtration and the filtrate was washed with saturated aqueous sodium bicarbonate solution and brine and then dried over anhydrous sodium sulfate. The solvent

was evaporated under reduced pressure and the residue was purified by silica gel column chromatography (hexane: ethyl acetate = 20:1) to give the title compound (2.50 g, 42%) as a yellow solid.
$^1$H-NMR ($CDCl_3$) δ: 1.37 (3H, t, J=7.1 Hz), 1.85 (3H, s), 4.33 (2H, q, J=7.1 Hz), 5.68 (1H, s), 6.73 (1H, brs), 6.92 (1H, brs), 7.04-7.08 (4H, m), 7.15 (2H, d, J=9.1 Hz), 7.79 (1H, s).
MS (FAB) m/z: 602, 604, 606, 608.

Step 2: Ethyl (5R,6S)-5,6-Bis(4-chlorophenyl)-3-formyl-6-methyl-5,6-dihydroimidazo[2,1-b][1,3]thiazole-2-carboxylate

An aqueous solution (10 ml) of silver nitrate (3.51 g) was added to an acetone (40 ml) solution of the compound (2.50 g, 4.13 mmol) obtained in Step 1 above at room temperature. After the resulting mixture was stirred for 10 hours, water and ethyl acetate were added and the resulting mixture was stirred for 10 minutes, and the insoluble matter was removed by suction filtration. The filtrate was extracted with ethyl acetate and then dried over anhydrous sodium sulfate. After the solvent was evaporated under reduced pressure, the residue was purified by silica gel column chromatography (hexane: ethyl acetate = 20:1) to give the title compound (1.27 g, 67%) as a yellow solid.
$^1$H-NMR ($CDCl_3$) δ: 1.38 (3H, t, J=7.1 Hz), 1.80 (3H, s), 4.37 (2H, q, J=7.1 Hz), 5.51 (1H, s), 6.76 (2H, d, J=8.8 Hz), 6.99 (2H, d, J=8.8 Hz), 7.05 (2H, d, J=8.8 Hz), 7.13 (2H, d, J=8.8 Hz), 10.06 (1H, s).
MS (ESI) m/z: 461, 463.

Step 3: (5R,6S)-5,6-Bis(4-chlorophenyl)-6-methyl-2-({(2S)-2-[(4-methylpiperazin-1-yl)carbonyl]pyrrolidin-1-yl}carbonyl)-5,6-dihydroimidazo[2,1-b][1,3]thiazole-3-carbaldehyde

The compound obtained in Step 2 above was reacted in the same way as in Step 3 of Example 1. Subsequently, the obtained compound was reacted in the same way as in Step 4 of Example 1 using 1-methyl-4-(L-prolyl)piperazine instead of piperazin-2-one to give the title compound as a colorless solid.
$^1$H-NMR ($CDCl_3$) δ: 1.79 (3H, s), 1.89-2.04 (2H, m), 2.09-2.54 (9H, m), 3.44-3.79 (6H, m), 4.96 (1H, m), 5.45 (1H, s), 6.66-6.79 (2H, m), 6.96 (2H, d, J=8.8 Hz), 7.02 (2H, d, J=8.8 Hz), 7.12 (2H, d, J=8.8 Hz), 9.56 (1H, s).

Step 4: 1-[(5R,6S)-5,6-Bis(4-chlorophenyl)-6-methyl-2-({(2S)-2-[(4-methylpiperazin-1-yl)carbonyl]pyrrolidin-1-yl}carbonyl)-5,6-dihydroimidazo[2,1-b][1,3]thiazol-3-yl]ethanol

A tetrahydrofuran (5 ml) solution of the compound (350 mg, 0.57 mmol) obtained in Step 3 above was cooled to -78°C and then methylmagnesium bromide (0.87 M tetrahydrofuran solution; 0.79 ml, 0.68 mmol) was added dropwise under nitrogen atmosphere. After the resulting mixture was stirred at the same temperature for 2 hours, saturated ammonium chloride aqueous solution was added to terminate the reaction and the resulting mixture was stirred with water and ethyl acetate. The organic layer was washed with brine and then dried over anhydrous sodium sulfate. After the solvent was evaporated under reduced pressure, the residue was purified by silica gel column chromatography (chloroform: 2-propanol = 5:1) to give the title compound (85 mg, 24%) as a colorless solid.
$^1$H-NMR ($CDCl_3$) δ: 1.37 (3H, d, J=6.6 Hz), 1.84 (3H, s), 1.89-2.01 (3H, m), 2.13-2.23 (2H, m), 2.33 (3H, s), 2.34-2.44 (3H, m), 2.53 (1H, m), 3.49-3.62 (4H, m), 3.83-3.90 (2H, m), 4.26 (1H, s), 4.95 (1H, m), 4.96 (1H, s), 6.73 (2H, d, J=8.3 Hz), 7.00-7.08 (6H, m).
MS (FAB) m/z: 628, 630.

Example 111

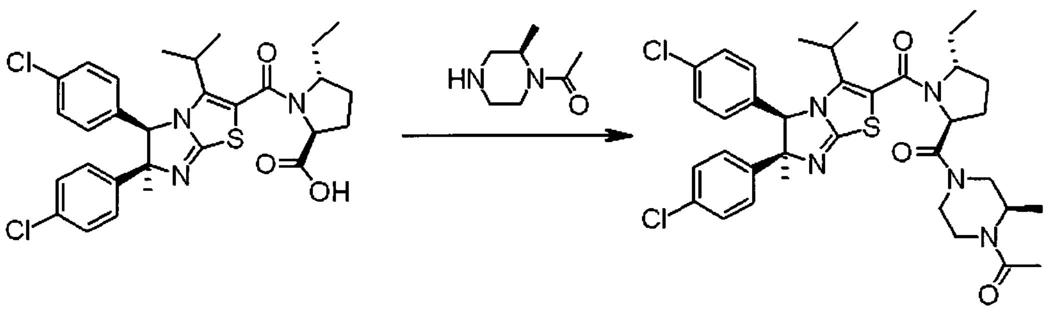

(5R,6S)-2-[((2S,5R)-2-{[(3R)-4-Acetyl-3-methylpiperazin-1-yl]carbonyl}-5-ethylpyrrolidin-1-yl)carbonyl]-5,6-bis(4-chlorophenyl)-3-isopropyl-6-methyl-5,6-dihydroimidazo[2,1-b][1,3]thiazole

The compound obtained in Step 2 of Example 103 was reacted in the same way as in Step 4 of Example 1 using (2R)-1-acetyl-2-methylpiperazine instead of piperazin-2-one to give the title compound.
$^1$H-NMR ($CDCl_3$) δ: 0.92 (3H, t, J=6.8 Hz), 0.94 (3H, t, J=7.2 Hz), 1.04 (3H, d, J=7.1 Hz), 1.21 (3H, brs), 1.39-1.46 (1H, m), 1.78-1.84 (3H, m), 1.79 (3H, s), 2.09 (3H, s), 2.15-2.20 (2H, m), 2.33-2.37 (1H, m), 2.73-2.80 (1H, m), 2.89-2.96 (1H, m), 3.12-3.19 (1H, m), 3.41-3.49 (1H, m), 3.68-3.77 (1H, m), 3.96-4.01 (1H, m), 4.31-4.37 (2H, m), 4.94 (1H, s), 4.95-4.99 (1H, m), 6.68 (2H, d, J=8.3 Hz), 7.00 (2H, d, J=8.5 Hz), 7.01 (2H, d, J=8.8 Hz), 7.12 (2H, d, J=8.5 Hz).
MS (ESI) m/z: 696.

Example 112

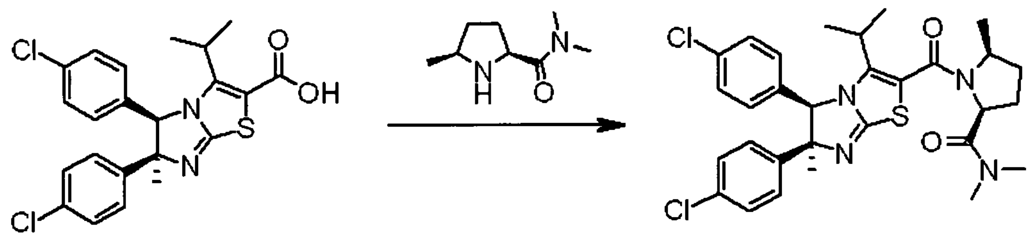

(5S)-1-{[(5R,6S)-5,6-Bis(4-chlorophenyl)-3-isopropyl-6-methyl-5,6-dihydroimidazo[2,1-b][1,3]thiazol-2-yl]carbonyl}-N,N,5-trimethyl-L-prolinamide

The same reaction as in Step 4 of Example 1 was performed using (5S)-N,N,5-trimethyl-L-prolinamide instead of piperazin-2-one to give the title compound.
$^1$H-NMR ($CDCl_3$) δ: 0.89 (3H, d, J=7.3 Hz), 0.95 (3H, d, J=7.1 Hz), 1.45 (3H, d, J=6.3 Hz), 1.80 (3H, s), 1.80-1.85 (1H, m), 1.96-2.01 (1H, m), 2.02-2.10 (1H, m), 2.12-2.18 (1H, m), 2.63-2.71 (1H, m), 2.96 (3H, s), 3.11 (3H, s), 4.22-4.28 (1H, m), 4.84-4.88 (1H, m), 4.96 (1H, s), 6.69 (2H, d, J=8.3 Hz), 7.00-7.04 (4H, m), 7.10 (2H, d, J=8.5 Hz).
MS (ESI) m/z: 585.

Example 113

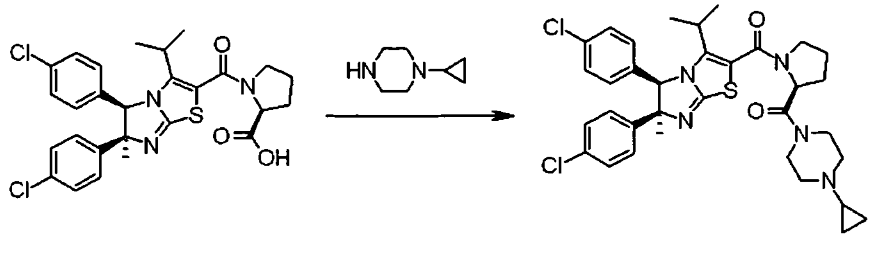

(5R,6S)-5,6-Bis(4-chlorophenyl)-3-isopropyl-6-methyl-2-({(2S)-2-[(4-cyclopropylpiperazin-1-yl)carbonyl]pyrrolidin-1-yl}carbonyl)-5,6-dihydroimidazo[2,1-b][1,3]thiazole

The compound obtained in Step 2 of Example 61 was reacted in the same way as in Step 4 of Example 1 using 1-cyclopropylpiperazine instead of piperazin-2-one to give the title compound.
$^1$H-NMR ($CDCl_3$) δ: 0.39-0.51 (4H, m), 0.88 (1H, t, J=6.8 Hz), 0.97 (6H, d, J=7.3 Hz), 1.59-1.86 (1H, m), 1.81 (3H, s),

1.87-1.99 (2H, m), 2.05-2.29 (2H, m), 2.49-2.79 (4H, m), 3.42-3.52 (1H, m), 3.58 (3H, s), 3.66-3.83 (2H, m), 4.89-4.98 (1H, m), 4.96 (1H, s), 6.65-6.73 (2H, m), 6.99-7.05 (4H, m), 7.11 (2H, d, J=8.5 Hz).
MS (ESI) m/z: 652.

Example 114

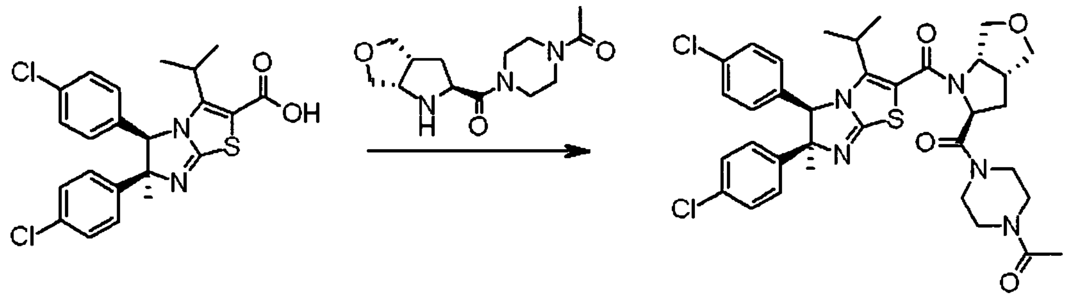

(2S,3aR,6aS)-1-{[(5R,6S)-5,6-Bis(4-chlorophenyl)-3-isopropyl-6-methyl-5,6-dihydroimidazo[2,1-b][1,3]thiazol-2-yl]carbonyl}-N,N-dimethylhexahydro-1H-furo[3,4-b]pyrrole-2-carboxamide

The same reaction as in Step 4 of Example 1 was performed using (2S,3aR,6aS)-2-[(4-acetylpiperazin-1-yl) carbonyl]hexahydro-1H-furo[3,4-b]pyrrole instead of piperazin-2-one to give the title compound.
$^1$H-NMR ($CDCl_3$) δ: 0.95 (3H, d, J=7.1 Hz), 1.01 (3H, d, J=7.1 Hz), 1.78 (3H, s), 2.11-2.14 (2H, m), 2.11 (3H, s), 2.71-2.78 (1H, m), 3.13-3.20 (1H, m), 3.43-3.50 (4H, m), 3.61-3.77 (7H, m), 3.91-3.96 (1H, m), 4.90-4.93 (1H, m), 4.94 (1H, s), 5.15 (1H, t, J=5.5 Hz), 6.66 (2H, d, J=8.3 Hz), 7.01 (4H, d, J=8.8 Hz), 7.10 (2H, d, J=8.5 Hz).
MS (ESI) m/z: 696.

Example 115

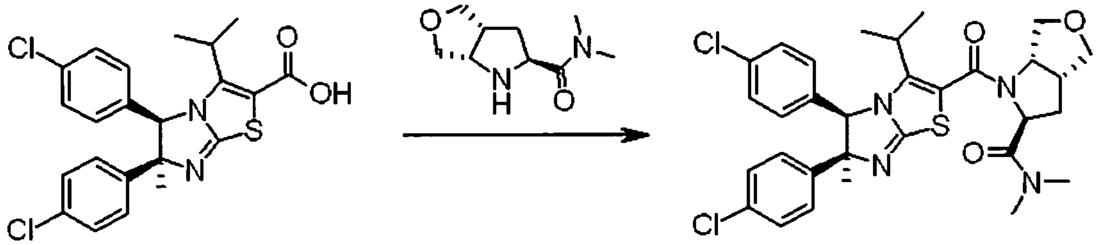

(5R,6S)-2-({(2S,3aR,6aS)-2-[(4-Acetylpiperazin-1-yl)carbonyl]hexahydro-1H-furo[3,4-b]pyrrol-1-yl}carbonyl)-5,6-bis (4-chlorophenyl)-3-isopropyl-6-methyl-5,6-dihydroimidazo[2,1-b][1,3]thiazole

The same reaction as in Step 4 of Example 1 was performed using (2S,3aR,6aS)-N,N-dimethylhexahydro-1H-furo[3,4-b]pyrrole-2-carboxamide instead of piperazin-2-one to give the title compound.
$^1$H-NMR ($CDCl_3$) δ: 0.92-0.94 (3H, m), 1.02 (3H, d, J=6.8 Hz), 1.79 (3H, s), 2.10-2.18 (2H, m), 2.73-2.80 (1H, m), 2.90 (3H, s), 3.08-3.11 (1H, m), 3.09 (3H, s), 3.62-3.66 (1H, m), 3.71-3.77 (2H, m), 3.93-3.98 (1H, m), 4.89-4.93 (1H, m), 4.94 (1H, s), 5.18-5.22 (1H, m), 6.65 (2H, d, J=8.5 Hz), 7.01 (4H, d, J=8.8 Hz), 7.11 (2H, d, J=8.8 Hz). MS (ESI) m/z: 613.

Example 116

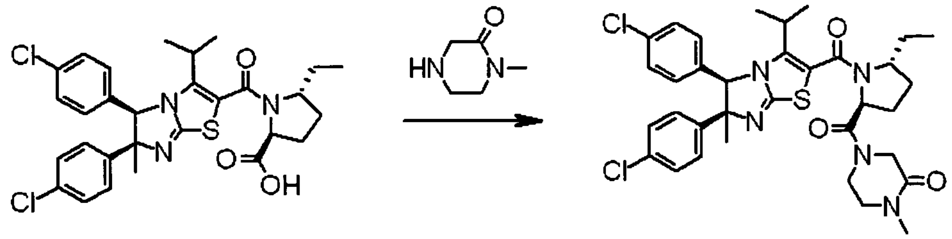

4-((5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-ethyl-L-prolyl)-1-methylpiperazin-2-one

The compound obtained in Step 2 of Example 103 was reacted in the same way as in Step 4 of Example 1 using 1-methylpiperazin-2-one hydrochloride instead of piperazin-2-one to give the title compound.
$^1$H-NMR ($CDCl_3$) δ: 0.93 (3H, t, J=7.3 Hz), 0.94 (3H, d, J=7.3 Hz), 1.02 (3H, d, J=7.1 Hz), 1.37-1.45 (1H, m), 1.76-1.86 (3H, m), 1.79 (3H, s), 2.19-2.33 (2H, m), 2.70-2.76 (1H, m), 3.00 (3H, s), 3.33-3.39 (2H, m), 3.75-3.83 (2H, m), 4.11-4.17 (1H, m), 4.23-4.38 (2H, m), 4.85-4.89 (1H, m), 4.93 (1H, s), 6.67 (2H, d, J=8.3 Hz), 7.00 (2H, d, J=8.5 Hz), 7.02 (2H, d, J=8.3 Hz), 7.13 (2H, d, J=8.5 Hz).
MS (ESI) m/z: 668.

Example 117

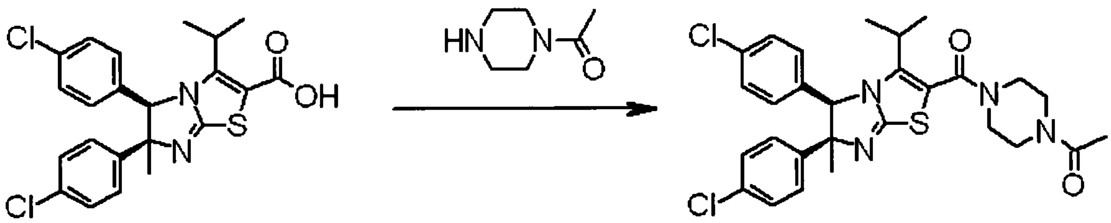

(5R,6S)-2-[(4-Acetylpiperazin-1-yl)carbonyl]-5,6-bis(4-chlorophenyl)-3-isopropyl-6-methyl-5,6-dihydroimidazo[2,1-b][1,3]thiazole

The compound obtained in Step 3 of Example 1 was reacted in the same way as in Step 4 of Example 1 using 1-acetylpiperazine instead of piperazin-2-one to give the title compound.
$^1$H-NMR ($CDCl_3$) δ: 0.90 (3H, d, J=7.1 Hz), 1.01 (3H, d, J=7.1 Hz), 1.82 (3H, s), 2.14 (3H, s), 2.46-2.52 (1H, m), 3.49-3.54 (2H, m), 3.58-3.68 (6H, m), 4.96 (1H, s), 6.72 (2H, d, J=6.1 Hz), 7.03 (2H, d, J=8.8 Hz), 7.05 (2H, d, J=8.8 Hz), 7.09 (2H, d, J=8.5 Hz).
MS (ESI) m/z: 557.

Example 118

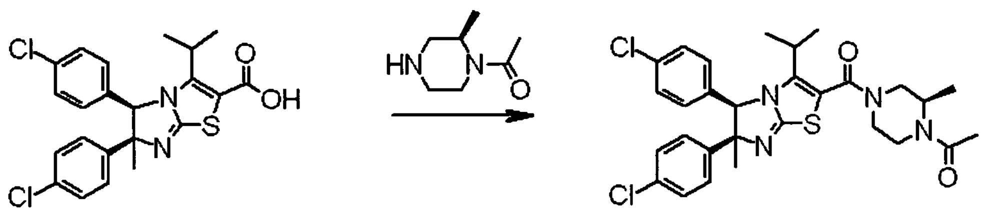

(5R,6S)-2-{[(3R)-4-Acetyl-3-methylpiperazin-1-yl]carbonyl}-5,6-bis(4-chlorophenyl)-3-isopropyl-6-methyl-5,6-dihydroimidazo[2,1-b][1,3]thiazole

The compound obtained in Step 3 of Example 1 was reacted in the same way as in Step 4 of Example 1 using (2R)-1-acetyl-2-methylpiperazine hydrochloride instead of piperazin-2-one to give the title compound.
$^1$H-NMR ($CDCl_3$) δ: 0.93 (3H, d, J=7.1 Hz), 0.97 (3H, d, J=7.1 Hz), 1.22-1.27 (3Hz, m), 1.82 (3H, s), 2.11 (3H, s), 2.50-2.57 (1H, m), 2.97-3.04 (1H, m), 3.12 (1H, dd, J=13.2, 3.9 Hz), 3.53-3.65 (1H, m), 4.03-4.12 (2H, m), 4.16-4.24 (2H, m), 4.96 (1H, s), 6.70 (2H, d, J=8.3 Hz), 7.02 (2H, d, J=8.8 Hz), 7.04 (2H, d, J=9.0 Hz), 7.09 (2H, d, J=8.5 Hz).
MS (ESI) m/z: 571.

Example 119

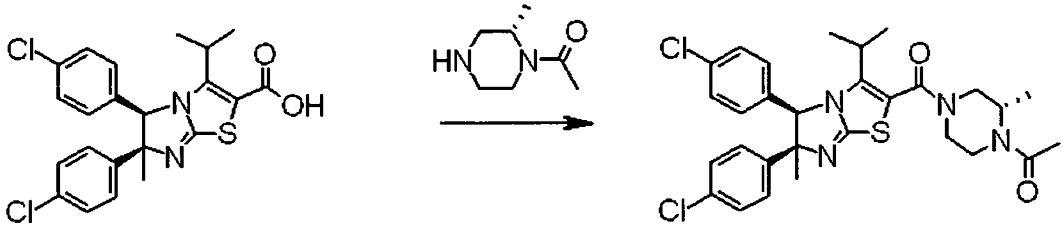

(5R,6S)-2-{[(3S)-4-Acetyl-3-methylpiperazin-1-yl]carbonyl}-5,6-bis(4-chlorophenyl)-3-isopropyl-6-methyl-5,6-dihydroimidazo[2,1-b][1,3]thiazole

The compound obtained in Step 3 of Example 1 was reacted in the same way as in Step 4 of Example 1 using (2S)-1-acetyl-2-methylpiperazine hydrochloride instead of piperazin-2-one to give the title compound.
$^1$H-NMR ($CDCl_3$) δ: 0.92 (3H, d, J=7.1 Hz), 0.99 (3H, d, J=7.1 Hz), 1.18-1.24 (3H, m), 1.82 (3H, s), 2.11 (3H, s), 2.50-2.59 (1H, m), 2.95-3.01 (1H, m), 3.16 (1H, dd, J=13.2, 3.9 Hz), 3.59-3.65 (1H, m), 4.06-4.13 (2H, m), 4.17-4.24 (2H, m), 4.95 (1H, s), 6.70 (2H, d, J=8.5 Hz), 7.02 (2H, d, J=8.5 Hz), 7.04 (2H, d, J=8.5 Hz), 7.09 (2H, d, J=8.5 Hz).
MS (ESI) m/z: 571.

Example 120

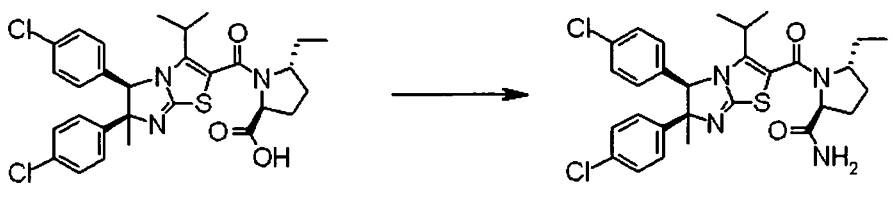

(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-ethyl-L-prolinamide

The compound obtained in Step 2 of Example 103 was reacted in the same way as in Step 4 of Example 1 using ammonium chloride instead of piperazin-2-one to give the title compound.
$^1$H-NMR ($CDCl_3$) δ: 0.94 (3H, t, J=7.7 Hz), 0.97 (3H, d, J=7.3 Hz), 1.03 (3H, d, J=7.1 Hz), 1.36-1.44 (1H, m), 1.76-1.88 (2H, m), 1.81 (3H, s), 2.04-2.11 (1H, m), 2.19-2.30 (2H, m), 2.75-2.83 (1H, m), 4.25-4.30 (1H, m), 4.69-4.72 (1H, m), 4.95 (1H, s), 5.28 (1H, brs), 6.14 (1H, brs), 6.66 (2H, d, J=8.5 Hz), 7.01 (2H, d, J=8.5 Hz), 7.02 (2H, d, J=8.5 Hz), 7.12 (2H, d, J=8.8 Hz).
MS (ESI) m/z: 571.

Example 121

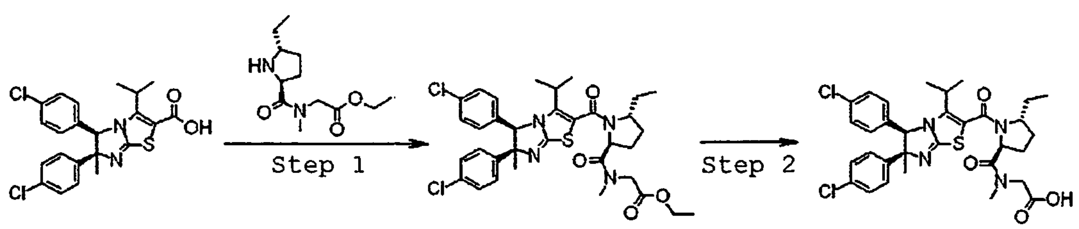


Step 1: Ethyl (5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-ethyl-L-prolyl-N-methyl glycinate

The compound obtained in Step 3 of Example 1 was reacted in the same way as in Example 112 using the compound obtained in Step 2 of Reference Example 26 instead of the compound obtained in Step 2 of Reference Example 18 to give the title compound.
$^1$H-NMR ($CDCl_3$) δ: 0.89-0.95 (6H, m), 1.04 (3H, d, J=7.1 Hz), 1.25 (3H, t, J=7.1 Hz), 1.38-1.44 (1H, m), 1.72-1.78 (2H, m), 1.79 (3H, s), 1.98-2.06 (1H, m), 2.20-2.28 (1H, m), 2.74-2.79 (1H, m), 2.97 (1H, d, J=4.9 Hz), 3.16 (3H, s), 3.46 (1H, d, J=17.1 Hz), 4.14 (2H, dd, J=16.2, 9.1 Hz), 4.36-4.38 (1H, m), 4.66-4.69 (1H, m), 4.93 (1H, s), 5.01-5.07 (1H, m), 6.66 (2H, d, J=8.0 Hz), 7.00 (2H, d, J=8.5 Hz), 7.01 (2H, d, J=8.3 Hz), 7.13 (2H, d, J=8.5 Hz).
MS (ESI) m/z: 671.

Step 2: (5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-ethyl-L-prolyl-N-methylglycine

The compound obtained in Step 1 above was reacted in the same way as in Step 3 of Example 1 to give the title compound.
$^1$H-NMR (DMSO-$d_6$) δ: 0.85 (6H, t, J=7.2 Hz), 0.99 (3H, d, J=7.1 Hz), 1.38-1.43 (1H, m), 1.61-1.67 (1H, m), 1.71-1.88 (3H, m), 1.79 (3H, s), 1.94-2.01 (1H, m), 2.25-2.33 (1H, m), 2.73-2.82 (3H, m), 3.67 (1H, d, J=16.8 Hz), 4.13-4.19 (2H, m), 5.00-5.04 (1H, m), 5.60 (1H, s), 6.87-6.89 (2H, m), 7.11 (2H, d, J=9.8 Hz), 7.13 (2H, d, J=8.8 Hz), 7.26 (2H, d, J=8.5 Hz).
MS (ESI) m/z: 643.

Example 122

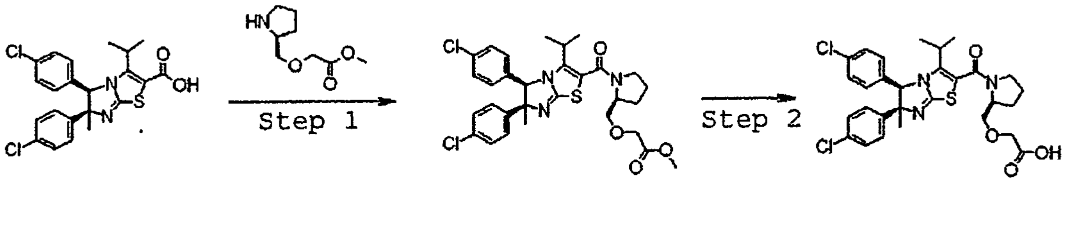

Step 1: Methyl [((2S)-1-{[(5R,6S)-5,6-Bis(4-chlorophenyl)-3-isopropyl-6-methyl-5,6-dihydroimidazo[2,1-b][1,3]thiazol-2-yl]carbonyl}pyrrolidin-2-yl)methoxy]acetate

The compound obtained in Step 3 of Example 1 was reacted in the same way as in Example 112 using the compound obtained in Step 3 of Reference Example 27 instead of the compound obtained in Step 2 of Reference Example 18 to give the title compound.
$^1$H-NMR ($CDCl_3$) δ: 0.94 (3H, d, J=7.1 Hz), 1.02 (3H, d, J=7.1 Hz), 1.80-1.85 (1H, m), 1.80 (3H, s), 2.01-2.10 (2H, m), 2.12-2.17 (1H, m), 2.51-2.60 (1H, m), 3.55-3.62 (2H, m), 3.69 (2H, d, J=4.4 Hz), 3.74 (3H, s), 4.04-4.07 (2H, m), 4.28-4.33 (1H, m), 4.93 (1H, s), 6.69 (2H, d, J=8.5 Hz), 7.01 (2H, d, J=8.8 Hz), 7.03 (2H, d, J=8.5 Hz), 7.10 (2H, d, J=8.8 Hz).
MS (ESI) m/z: 602.

Step 2: [((2S)-1-{[(5R,6S)-5,6-Bis(4-chlorophenyl)-3-isopropyl-6-methyl-5,6-dihydroimidazo[2,1-b][1,3]thiazol-2-yl]carbonyl}pyrrolidin-2-yl)methoxy]acetic acid

The compound obtained in Step 1 above was reacted in the same way as in Step 3 of Example 1 to give the title compound.
$^1$H-NMR (DMSO-$d_6$) δ: 0.85 (3H, d, J=6.8 Hz), 0.90 (3H, d, J=7.1 Hz), 1.69-1.77 (4H, m), 1.94 (3H, brs), 2.50-2.53 (1H, m), 3.41-3.50 (3H, m), 3.53-3.58 (1H, m), 3.97 (2H, brs), 4.11-4.18 (1H, m), 5.48 (1H, s), 6.82-6.92 (2H, m), 7.09 (2H, d, J=8.3 Hz), 7.15 (2H, d, JJ=8.1 Hz), 7.26 (2H, d, J=8.5 Hz).
MS (ESI) m/z: 588.

Example 123

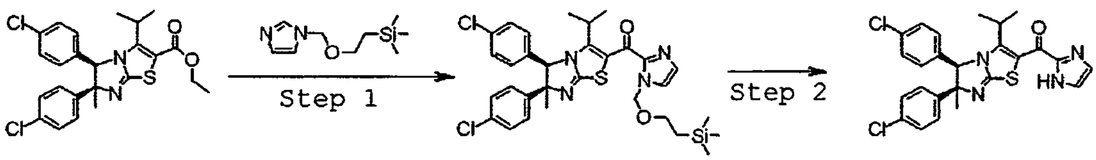


Step 1: [(5R,6S)-5,6-Bis(4-chlorophenyl)-3-isopropyl-6-methyl-5,6-dihydroimidazo[2,1-b][1,3]thiazol-2-yl](1-{[2-(trimethylsilyl)ethoxy]methyl}-1H-imidazol-2-yl)methanone

1-{[2-(Trimethylsilyl)ethoxy]methyl)-1H-imidazole (238 mg, 1.20 mmol) was dissolved in tetrahydrofuran (5 ml) followed by the dropwise addition of 1.6 M n-butyllithium/hexane solution (0.83 ml, 1.32 mmol) at - 78°C. After the resulting mixture was stirred at the same temperature for 25 minutes, a tetrahydrofuran (4 ml) solution of the compound (475 mg, 1.00 mmol) obtained in Step 2 of Example 1 was added dropwise and the resulting mixture was stirred for 2.5 hours while warming to the room temperature slowly. Saturated ammonium chloride aqueous solution was added to the reaction mixture and the resulting mixture was extracted with ethyl acetate. The organic layer was washed with brine and then dried over anhydrous magnesium sulfate. The solvent was evaporated under reduced pressure, and the obtained residue was purified by silica gel column chromatography (hexane: ethyl acetate = 3:1 → 1:1) to give the title compound (272 mg, 43%) as a yellow oil.
$^1$H-NMR ($CDCl_3$) δ: -0.01 (9H, s), 0.91 (3H, d, J=7.1 Hz), 0.95 (2H, t, J=8.4 Hz), 1.08 (3H, d, J=7.1 Hz), 1.84 (3H, s), 3.60 (2H, t, J=8.4 Hz), 3.61-3.66 (1H, m), 5.16 (1H, s), 5.82 (2H, d, J=2.7 Hz), 6.76 (2H, brs), 7.04-7.06 (4H, m), 7.15 (2H, d, J=8.3 Hz), 7.25 (1H, s), 7.31 (1H, s).
MS (ESI) m/z: 627.

Step 2: [(5R,6S)-5,6-Bis(4-chlorophenyl)-3-isopropyl-6-methyl-5,6-dihydroimidazo[2,1-b][1,3]thiazol-2-yl](1H-imidazol-2-yl)methanone

The compound (270 mg, 0.43 mmol) obtained in Step 1 above was dissolved in ethanol (10 ml) followed by the addition of 3 N hydrochloric acid (20 ml) and the resulting mixture was heated to reflux for 1.5 hours. The reaction mixture was concentrated under reduced pressure and followed by the addition of saturated aqueous sodium bicarbonate solution and the residue was extracted with chloroform. After the organic layer was dried over anhydrous magnesium sulfate, the solvent was evaporated under reduced pressure to give the title compound (230 mg, 100%) as a yellow solid.

$^1$H-NMR ($CDCl_3$) δ: 0.97 (3H, d, J=7.1 Hz), 1.06 (3H, d, J=7.1 Hz), 1.84 (3H, s), 3.55-3.62 (1H, m), 5.17 (1H, s), 6.75 (2H, brs), 7.02-7.05 (4H, m), 7.14 (2H, d, J=8.2 Hz), 7.17 (1H, brs), 7.31 (1H, brs), 1.1.71 (1H, brs).
MS (ESI) m/z: 497.

Example 124

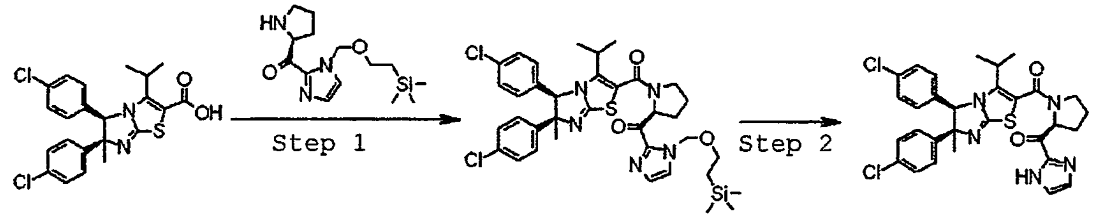


Step 1: [(2S)-1-{[(5R,6S)-5,6-Bis(4-chlorophenyl)-3-isopropyl-6-methyl-5,6-dihydroimidazo[2,1-b][1,3]thiazol-2-yl]carbonyl}pyrrolidin-2-yl](1-{[2-(trimethylsilyl)ethoxy]methyl}-1H-imidazol-2-yl)methanone

The compound obtained in Step 3 of Example 1 was reacted in the same way as in Step 4 of Example 1 using the compound obtained in Step 2 of Reference Example 28 instead of piperazin-2-one to give the title compound.
MS (ESI) m/z: 724.

Step 2: [(2S)-1-{[(5R,6S)-5,6-Bis(4-chlorophenyl)-3-isopropyl-6-methyl-5,6-dihydroimidazo[2,1-b][1,3]thiazol-2-yl]carbonyl}pyrrolidin-2-yl](1H-imidazol-2-yl)methanone

The compound obtained in Step 1 above was reacted in the same way as in Step 2 of Example 123 to give the title compound.
$^1$H-NMR ($CDCl_3$) δ: 0.95-1.03 (6H, m), 1.82 (3H, s), 1.99-2.12 (3H, m), 2.43-2.59 (2H, m), 3.71-3.97 (2H, m), 4.96 (1H, s), 5.69 (1H, s), 6.69 (2H, brs), 7.00-7.06 (5H, m), 7.12-7.17 (3H, m), 13.14 (1H, brs).
MS (ESI) m/z: 594.

Example 125

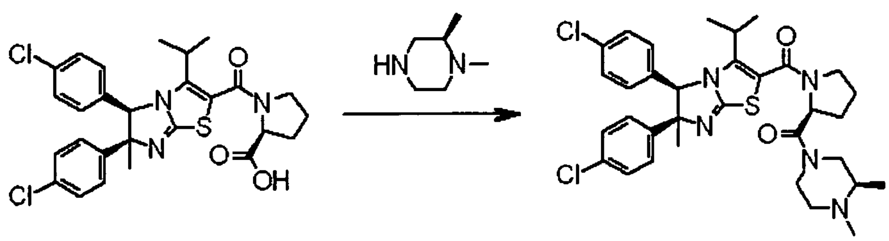

(5R,6S)-5,6-Bis(4-chlorophenyl)-2-[((2S)-2-{[(3R)-3,4-dimethylpiperazin-1-yl]carbonyl}pyrrolidin-1-yl)carbonyl]-3-isopropyl-6-methyl-5,6-dihydroimidazo[2,1-b][1,3]thiazole

The compound obtained in Step 2 of Example 61 was reacted using (2R)-1,2-dimethylpiperazine instead of piperazin-2-one in the same way as in Step 4 of Example 1 to give the title compound.
$^1$H-NMR ($CDCl_3$) δ: 0.96 (6H, d, J=7.1 Hz), 1.04-1.09 (3H, m), 1.80 (3H, s), 1.90-1.95 (2H, m), 2.12-2.23 (2H, m), 2.28 (3H, s), 2.58-2.65 (2H, m), 2.70-2.78 (1H, m), 3.43-3.49 (1H, m), 2.82-2.91 (1H, m), 3.69-3.81 (3H, m), 4.17-4.18 (1H, m), 4.36-4.37 (1H, m), 4.90-4.93 (1H, m), 4.94 (1H, s), 6.69 (2H, d, J=8.5 Hz), 7.01 (2H, d, J=8.5 Hz), 7.02 (2H, d, J=8.8 Hz), 7.10 (2H, d, J=8.5 Hz).
MS (ESI) m/z: 640.

Example 126

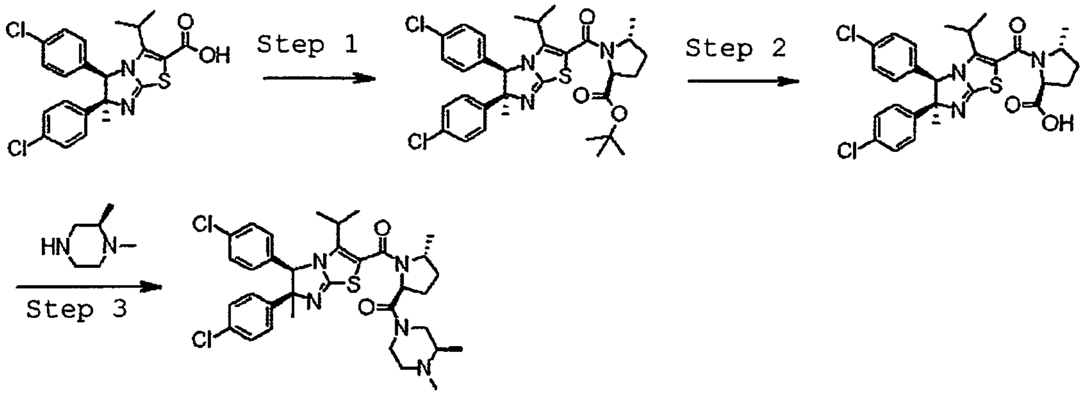


Step 1: tert-Butyl (5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolinate

The compound obtained in Step 3 of Example 1 was reacted in the same way as in Example 112 using the compound obtained in Reference Example 30 instead of the compound obtained in Step 2 of Reference Example 18 to give the title compound.
$^1$H-NMR ($CDCl_3$) δ: 1.00-1.03 (6H, m), 1.20 (3H, d, J=6.1 Hz), 1.45 (9H, s), 1.66-1.68 (1H, m), 1.80 (3H, s), 1.96-2.00 (1H, m), 2.23-2.29 (2H, m), 2.62-2.68 (1H, m), 4.47-4.59 (2H, m), 4.95 (1H, s), 6.69 (2H, d, J=8.1 Hz), 7.01 (2H, d, J=8.3 Hz), 7.02 (2H, d, J=8.5 Hz), 7.13 (2H, d, J=8.3 Hz).

Step 2: (5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-proline

The compound obtained in Step 1 above was reacted in the same way as in Step 2 of Example 61 to give the title compound.
$^1$H-NMR (DMSO-$d_6$) δ: 0.92 (3H, d, J=7.3 Hz), 0.95 (3H, d, J=6.8 Hz), 1.16 (3H, d, J=6.1 Hz), 1.63-1.67 (1H, m), 1.94 (3H, s), 1.96-2.01 (1H, m), 2.04-2.10 (1H, m), 2.34-2.40 (1H, m), 2.67-2.75 (1H, m), 4.26-4.35 (1H, m), 4.52-4.59 (1H, m), 5.89 (1H, s), 6.84-6.92 (2H, m), 7.17 (4H, d, J=8.3 Hz), 7.23 (2H, d, J=8.5 Hz).
MS (ESI) m/z: 558.

Step 3: (5R,6S)-5,6-Bis(4-chlorophenyl)-2-[((2S,5R)-2-{[(3R)-3,4-dimethylpiperazin-1-yl]carbonyl}-5-methylpyrrolidin-1-yl)carbonyl]-3-isopropyl-6-methyl-5,6-dihydroimidazo[2,1-b][1,3]thiazole

The compound obtained in Step 2 above was reacted in the same way as in Step 4 of Example 1 using (2R)-1,2-dimethylpiperazine instead of piperazin-2-one to give the title compound.
$^1$H-NMR ($CDCl_3$) δ: 0.94 (3H, d, J=6.8 Hz), 1.04 (3H, d, J=7.1 Hz), 1.05-1.07 (3H, m), 1.23 (3H, d, J=6.3 Hz), 1.51-1.54 (1H, m), 1.61-1.66 (1H, m), 1.79 (3H, s), 1.83-1.87 (1H, m), 1.97-2.01 (1H, m), 2.17-2.26 (3H, m), 2.29 (3H, s), 2.71-2.79 (2H, m), 2.83-2.88 and 3.40-3.45 (total 1H, each m), 3.64-3.74 (1H, m), 4.16-4.22 and 4.32-4.37 (total 1H, each m), 4.52-4.57 (1H, m), 4.93 (1H, s), 4.99-5.04 (1H, m), 6.68 (2H, d, J=7.6 Hz), 7.01 (4H, d, J=8.3 Hz), 7.12 (2H, d, J=8.5 Hz).
MS (ESI) m/z: 654.

Example 127

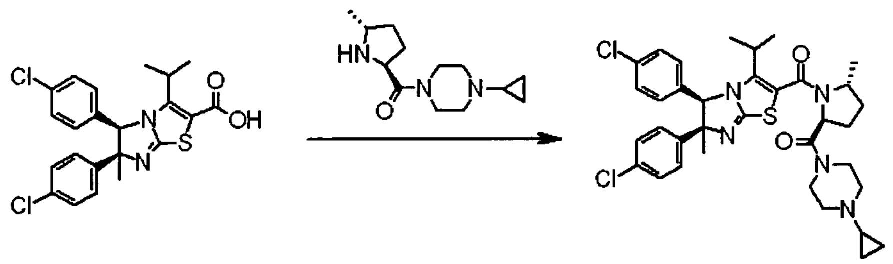

(5R,6S)-5,6-Bis(4-chlorophenyl)-2-({(2S,5R)-2-[(4-cyclopropylpiperazin-1-yl)carbonyl]-5-methylpyrrolidin-1-yl}carbonyl)-3-isopropyl-6-methyl-5,6-dihydroimidazo[2,1-b][1,3]thiazole

The compound obtained in Step 3 of Example 1 was reacted in the same way as in Example 112 using the compound obtained in Step 2 of Reference Example 32 instead of the compound obtained in Step 2 of Reference Example 18 to give the title compound.
$^1$H-NMR ($CDCl_3$) δ: 0.40-0.48 (4H, m), 0.94 (3H, d, J=6.8 Hz), 1.04 (3H, d, J=7.1 Hz), 1.23 (3H, d, J=6.3 Hz), 1.61-1.66 (2H, m), 1.79 (3H, s), 1.82-1.87 (1H, m), 2.25-2.32 (2H, m), 2.53-2.64 (3H, m), 2.70-2.77 (2H, m), 3.43-3.55 (4H, m), 4.52-4.57 (1H, m), 4.93 (1H, s), 5.02 (1H, d, J=6.3 Hz), 6.68 (2H, d, J=8.3 Hz), 7.01 (4H, d, J=7.6 Hz), 7.12 (2H, d, J=8.5 Hz).
MS (ESI) m/z: 666.

Example 128

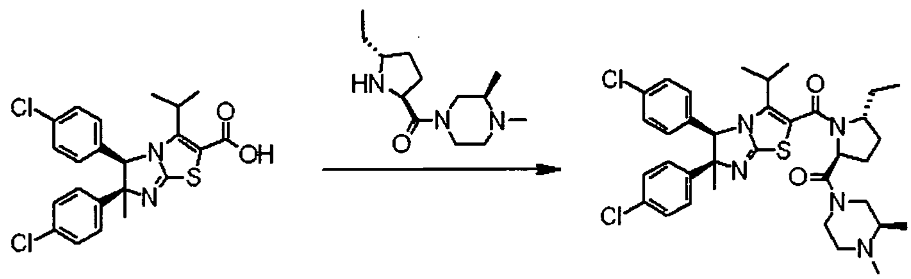

(5R,6S)-5,6-Bis(4-chlorophenyl)-2-[((2S,5R)-2-{[(3R)-3,4-dimethylpiperazin-1-yl]carbonyl}-5-ethylpyrrolidin-1-yl)carbonyl]-3-isopropyl-6-methyl-5,6-dihydroimidazo[2,1-b][1,3]thiazole

The compound obtained in Step 3 of Example 1 was reacted in the same way as in Example 112 using the compound obtained in Step 2 of Reference Example 33 instead of the compound obtained in Step 2 of Reference Example 18 to give the title compound.
$^1$H-NMR ($CDCl_3$) δ: 0.93 (6H, t, J=7.4 Hz), 1.05 (3H, d, J=7.1 Hz), 1.05-1.08 (3H, m), 1.37-1.44 (1H, m), 1.79 (3H, s), 1.79-1.83 (3H, m), 2.17-2.26 (3H, m), 2.29 (3H, s), 2.76-2.84 (3H, m), 3.40-3.46 (1H, m), 3.66-3.74 (1H, m), 4.17-4.21 (1H, m), 4.35-4.39 (2H, m), 4.93 (1H, s), 4.99-5.02 (1H, m), 6.66 (2H, d, J=8.1 Hz), 7.01 (4H, d, J=8.5 Hz), 7.12 (2H, d, J=8.5 Hz).
MS (ESI) m/z: 668.

Example 129

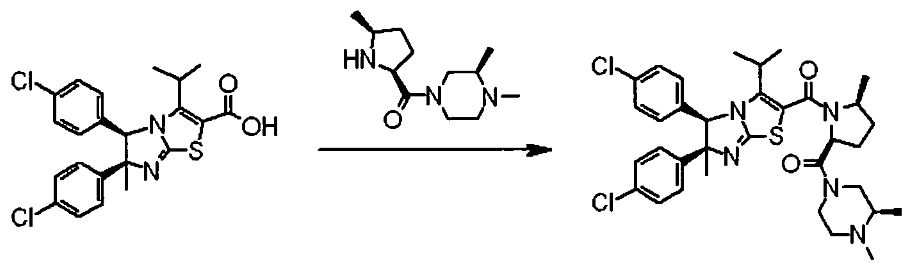

(5R,6S)-5,6-Bis(4-chlorophenyl)-2-[((2S,5S)-2-{[(3R)-3,4-dimethylpiperazin-1-yl]carbonyl}-5-methylpyrrolidin-1-yl)carbonyl]-3-isopropyl-6-methyl-5,6-dihydroimidazo[2,1-b][1,3]thiazole

The compound obtained in Step 3 of Example 1 was reacted in the same way as in Example 112 using the compound obtained in Step 2 of Reference Example 34 instead of the compound obtained in Step 2 of Reference Example 18 to give the title compound.
$^1$H-NMR ($CDCl_3$) δ: 0.90-0.93 (3H, m), 0.94 (3H, d, J=7.1 Hz), 1.07 (3H, d, J=6.1 Hz), 1.44 (3H, d, J=6.3 Hz), 1.80 (3H, s), 1.96-2.15 (4H, m), 2.29 (3H, s), 2.59-2.65 (2H, m), 2.78-2.92 (2H, m), 3.41-3.50 (1H, m), 3.69-3.75 (1H, m), 4.18-4.29 (2H, m), 4.36-4.43 (1H, m), 4.84-4.89 (1H, m), 4.95 (1H, s), 6.69 (2H, d, J=8.5 Hz), 7.01 (2H, d, J=8.8 Hz), 7.02 (2H, d, J=8.5 Hz), 7.09 (2H, d, J=8.8 Hz).
MS (ESI) m/z: 654.

Example 130

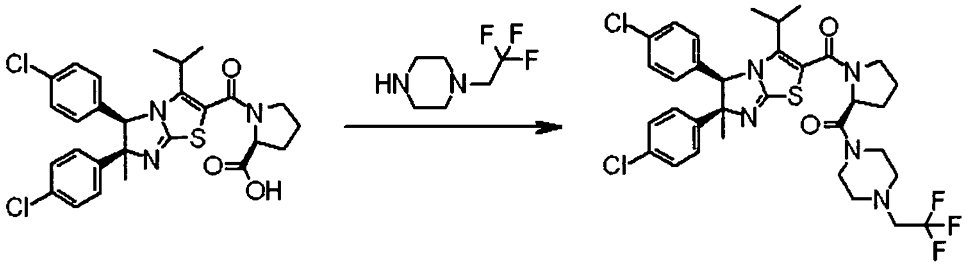

(5R,6S)-5,6-Bis(4-chlorophenyl)-3-isopropyl-6-methyl-2-[((2S)-2-{[4-(2,2,2-trifluoroethyl)piperazin-1-yl]carbonyl}pyrrolidin-1-yl)carbonyl]-5,6-dihydroimidazo[2,1-b][1,3]thiazole

The compound obtained in Step 2 of Example 61 was reacted in the same way as in Step 4 of Example 1 using 1-(2,2,2-trifluoroethyl)piperazine instead of piperazin-2-one to give the title compound.
$^1$H-NMR ($CDCl_3$) δ: 0.96 (3H, d, J=7.1 Hz), 0.96 (3H, d, J=7.1 Hz), 1.80 (3H, s), 1.91-1.96 (2H, m), 2.14-2.19 (2H, m), 2.62-2.69 (4H, m), 2.78-2.81 (1H, m), 2.99 (2H, q, J=9.4 Hz), 3.53-3.57 (2H, m), 3.67-3.81 (4H, m), 4.90 (1H, dd, J=7.9, 4.5 Hz), 4.94 (1H, s), 6.70 (2H, d, J=8.5 Hz), 7.01 (2H, d, J=8.5 Hz), 7.02 (2H, d, J=8.3 Hz), 7.10 (2H, d, J=8.5 Hz).
MS (ESI) m/z: 694.

Example 131

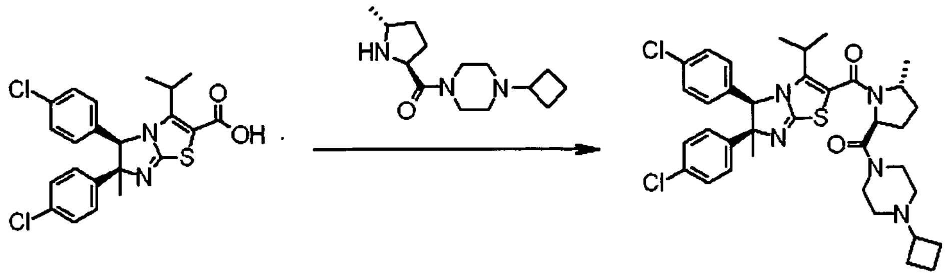

(5R,6S)-5,6-Bis(4-chlorophenyl)-2-({(2S,5R)-2-[(4-cyclobutylpiperazin-1-yl)carbonyl]-5-methylpyrrolidin-1-yl}carbonyl)-3-isopropyl-6-methyl-5,6-dihydroimidazo[2,1-b][1,3]thiazole

The compound obtained in Step 3 of Example 1 was reacted in the same way as in Example 112 using the compound obtained in Step 2 of Reference Example 35 instead of the compound obtained in Step 2 of Reference Example 18 to give the title compound.
$^1$H-NMR ($CDCl_3$) δ: 0.94 (3H, d, J=6.8 Hz), 1.03 (3H, d, J=7.1 Hz), 1.23 (3H, d, J=6.3 Hz), 1.62-1.75 (2H, m), 1.79 (3H, s), 1.81-1.89 (4H, m), 1.97-2.07 (2H, m), 2.21-2.47 (6H, m), 2.71-2.78 (2H, m), 3.48-3.61 (4H, m), 4.53-4.57 (1H, m), 4.93 (1H, s), 5.00-5.03 (1H, m), 6.68 (2H, d, J=8.3 Hz), 7.00-7.03 (4H, m), 7.12 (2H, d, J=8.5 Hz).
MS (ESI) m/z: 680.

Example 132

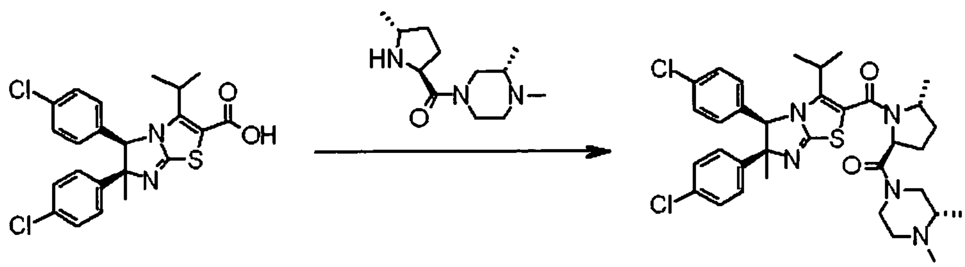

(5R,6S)-5,6-Bis(4-chlorophenyl)-2-[((2S,-5R)-2-{[(3S)-3,4-dimethylpiperazin-1-yl]carbonyl}-5-methylpyrrolidin-1-yl)carbonyl]-3-isopropyl-6-methyl-5,6-dihydroimidazo[2,1-b][1,3]thiazole

The compound obtained in Step 3 of Example 1 was reacted in the same way as Example 112 using the compound obtained in Step 2 of Reference Example 36 instead of the compound obtained in Step 2 of Reference Example 18 to give the title compound.
$^1$H-NMR ($CDCl_3$) δ : 0.95 (3H, d, J=6.8 Hz), 1.03 (3H, d, J=7.1 Hz), 1.09-1.11 (3H, m), 1.23 (3H, d, J=6.3 Hz), 1.61-1.64 (1H, m), 1.79 (3H, s), 1.82-1.86 (1H, m), 2.07-2.12 (2H, m), 2.22-2.27 (1H, m), 2.29 (3H, s), 2.71-2.80 (2H, m), 2.98-3.05 (1H, m), 3.27-3.33 (1H, m), 3.59-3.64 (1H, m), 3.76-3.82 (1H, m), 4.23-4.27 (1H, m), 4.52-4.58 (1H, m), 4.93 (1H, s), 5.00-5.03 (1H, m), 6.68 (2H, d, J=8.1 Hz), 7.01 (4H, d, J=8.5 Hz), 7.12 (2H, d, J=8.5 Hz).
MS (ESI) m/z: 654.

Example 133

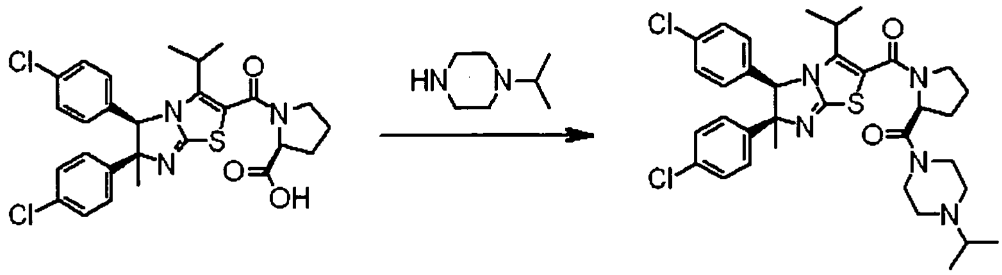

(5R,6S)-5,6-Bis(4-chlorophenyl)-3-isopropyl-2-({(2S)-2-[(4-isopropylpiperazin-1-yl)carbonyl]pyrrolidin-1-yl}carbonyl)-6-methyl-5,6-dihydroimidazo[2,1-b][1,3]thiazole

The compound obtained in Step 2 of Example 61 was reacted in the same way as in Step 4 of Example 1 using 1-isopropylpiperazine instead of piperazin-2-one to give the title compound.
$^1$H-NMR ($CDCl_3$) δ: 0.96 (3H, d, J=7.1 Hz), 0.97 (3H, d, J=6.8 Hz), 1.04 (6H, d, J=6.6 Hz), 1.80 (3H, s), 1.89-1.97 (2H, m), 2.09-2.22 (2H, m), 2.46-2.52 (3H, m), 2.62-2.74 (3H, m), 3.48-3.82 (6H, m), 4.91-4.93 (1H, m), 4.94 (1H, s), 6.69 (2H, d, J=8.3 Hz), 7.01 (2H, d, J=8.5 Hz), 7.03 (2H, d, J=7.3 Hz), 7.10 (2H, d, J=8.5 Hz).
MS (ESI) m/z: 654.

Example 134

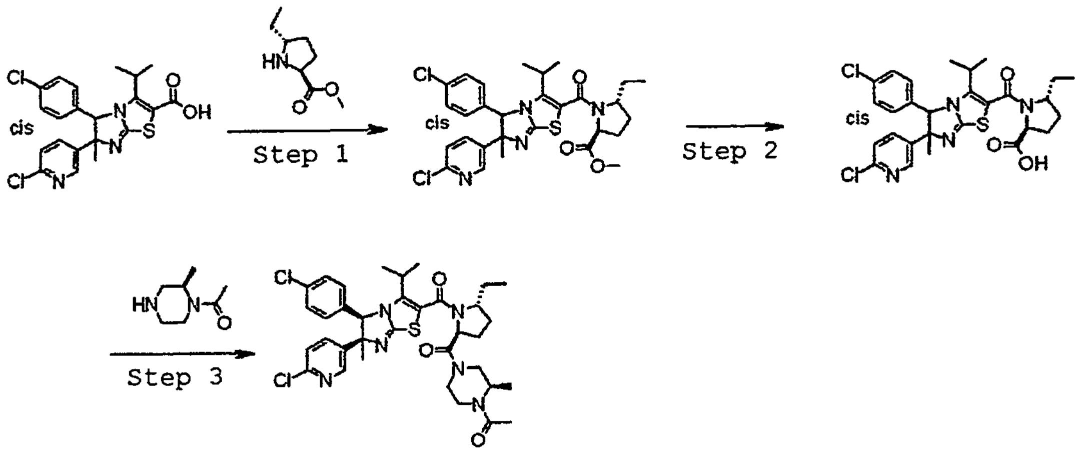


Step 1: Methyl (5R)-1-{[(5R*,6S*)-5-(4-chlorophenyl)-6-(6-chloropyridin-1-yl)-3-isopropyl-6-methyl-5,6-dihydroimidazo [2,1-b][1,3]thiazol-2-yl]carbonyl}-5-ethyl-L-prolinate

The compound obtained in Step 3 of Example 97 was reacted in the same way as in Example 112 using methyl (5R)-5-ethyl-L-prolinate instead of the compound obtained in Step 2 of Reference Example 18 to give the title compound.
MS (ESI) m/z: 587.

Step 2: (5R)-1-{[(5R*,6S*)-5-(4-Chlorophenyl)-6-(6-chloropyridin-3-yl)-3-isopropyl-6-methyl-5,6-dihydroimidazo[2,1-b] [1,3]thiazol-2-yl]carbonyl}-5-ethyl-L-proline

The compound obtained in Step 1 above was reacted in the same way as in Step 3 of Example 1 to give the title compound.
$^1$H-NMR ($CDCl_3$) δ: 0.89-1.02 (9H, m), 1.21-1.37 (1H, m), 1.75-2.35 (9H, m), 2.56-2.64 (1H, m), 4.19-4.26 (1H, m), 5.13-5.55 (1H, m), 6.54-6.82 (1H, m), 6.97-7.14 (4H, m), 7.47-7.56 (1H, m), 8.19-8.22 (1H, m).

MS (ESI) m/z: 573.

Step 3: (5R,6S)-2-{[(2S,5R)-2-{[(3R)-4-Acetyl-3-methylpiperazin-1-yl]carbonyl}-5-ethylpyrrolidin-1-yl]carbonyl}-5-(4-chlorophenyl)-6-(6-chloropyridin-3-yl)-3-isopropyl-6-methyl-5,6-dihydroimidazo[2,1-b][1,3]thiazole

The compound obtained in Step 2 above was reacted in the same way as in Step 4 of Example 97 using (2R)-1-acetyl-2-methylpiperazine instead of 4-(L-prolyl)morpholine to give the title compound.
$^1$H-NMR ($CDCl_3$) δ: 0.90-0.97 (6H, m), 1.06 (3H, d, J=7.1 Hz), 1.20-1.26 (3H, m), 1.38-1.45 (1H, m), 1.65-1.86 (5H, m), 1.82 (3H, s), 2.12 (3H, s), 2.90-3.75 (9H, m), 4.77-4.85 (1H, m), 4.91 (1H, brs), 5.01 (1H, s), 6.69 (2H, brs), 6.99 (1H, d, J=8.0 Hz), 7.06 (2H, d, J=8.3 Hz), 7.51 (1H, dd, J=8.0, 1.8 Hz), 8.23 (1H, s).
MS (ESI) m/z: 697.

Example 135

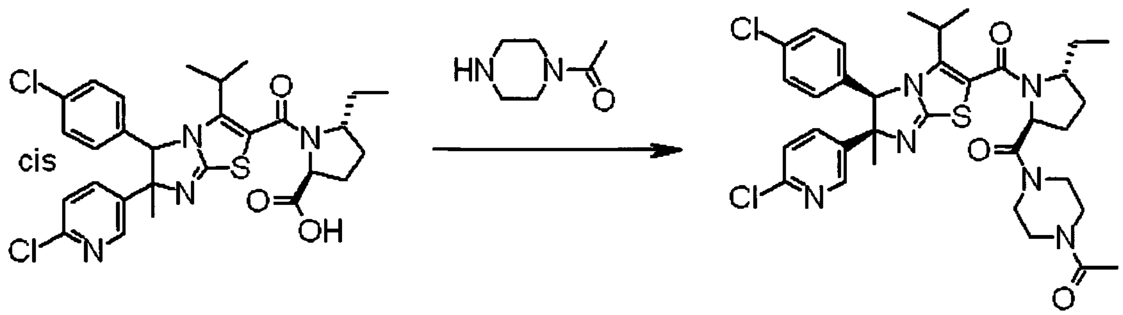

The compound obtained in Step 2 of Example 134 was reacted in the same way as in Step 4 of Example 97 using 1-acetylpiperazine instead of 4-(L-prolyl)morpholine to give the title compound.
$^1$H-NMR (CDCl3) δ: 0.93-0.97 (6H, m), 1.04 (3H, d, J=6.8 Hz), 1.36-1.47 (1H, m), 1.71-1.86 (3H, m), 1.82 (3H, s), 2.12 (3H, s), 2.18-2.32 (2H, m), 2.69-2.76 (1H, m), 3.43-3.79 (8H, m), 4.37 (1H, t, J=7.8 Hz), 4.98 (1H, brs), 5.00 (1H, s), 6.67-6.69 (2H, m), 7.00 (1H, d, J=8.5 Hz), 7.06 (2H, d, J=8.3 Hz), 7.51 (1H, dd, J=8.3, 2.2 Hz), 8.23 (1H, brs).
MS (ESI) m/z: 683.

Example 136

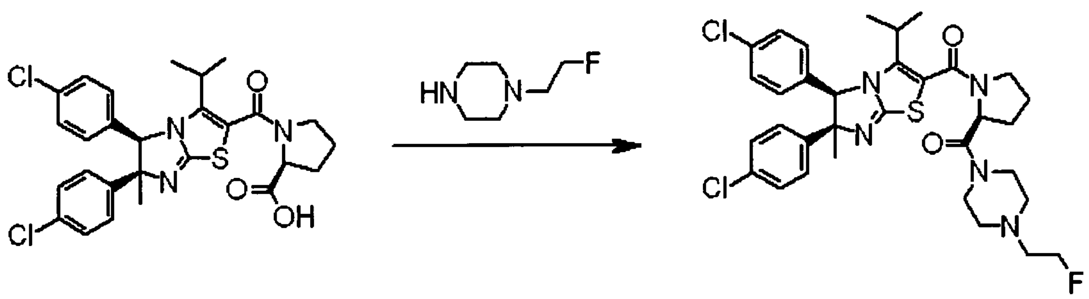

(5R,6S)-5,6-Bis(4-chlorophenyl)-2-{[(2S)-2-{[4-(2-fluoroethyl)piperazin-1-yl]carbonyl}pyrrolidin-1-yl]carbonyl}-3-isopropyl-6-methyl-5,6-dihydroimidazo[2,1-b][1,3]thiazole

The compound obtained in Step 2 of Example 61 was reacted in the same way as in Step 4 of Example 1 using 1-(2-fluoroethyl) piperazine instead of piperazin-2-one to give the title compound.

$^1$H-NMR ($CDCl_3$) δ: 0.96 (6H, d, J=6.8 Hz), 1.81 (3H, s), 1.87-1.99 (2H, m), 2.07-2.29 (2H, m), 2.46-2.59 (3H, m), 2.59-2.81 (4H, m), 3.50-3.83 (6H, m), 4.51 (1H, t, J=4.8 Hz), 4.63 (1H, t, J=4.8 Hz), 4.88-4.95 (1H, m), 4.96 (1H, s), 6.70 (2H, d, J=7.6 Hz), 7.00-7.05 (4H, m), 7.11 (2H, d, J=8.5 Hz).
MS (FAB) m/z: 658, 660.

Example 137

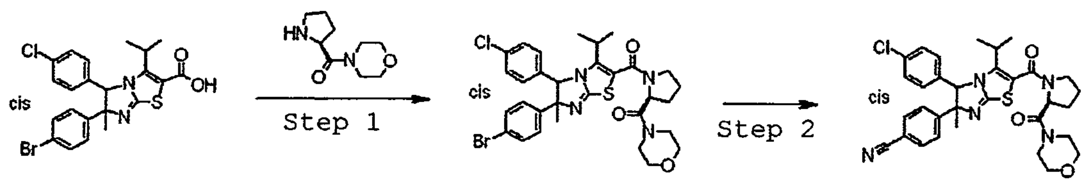


Step 1: (5R*,6S*) -6- (4-Bromophenyl) -5- (4-chlorophenyl) -3-isopropyl-6-methyl-2-{[(2S)-2-(morpholin-4-ylcarbonyl) pyrrolidin-1-yl]carbonyl}-5,6-dihydroimidazo[2,1-b][1,3]thiazole

The compound obtained in Step 10 of Reference Example 37 was reacted in the same way as in Step 4 of Example 1 using 4-L-prolylmorpholine instead of piperazin-2-one to give the title compound.
$^1$H-NMR ($CDCl_3$) δ: 0.87-1.00 (6H, m), 1.81 (3H, s), 1.94-1.97 (2H, m), 2.12-2.25 (2H, m), 2.65 (1H, m), 3.49-3.83 (10H, m), 4.89 (1H, m), 4.97 (1H, m), 6.68-6.71 (2H, m), 7.02-7.04 (4H, m), 7.18 (2H, d, J=8.5 Hz).

Step 2: 4-[(5R*,6S*)-5-(4-Chlorophenyl)-3-isopropyl-6-methyl-2-{[(2S)-2-(morpholin-4-ylcarbonyl)pyrrolidin-1-yl]carbonyl}-5,6-dihydroimidazo[2,1-b][1,3]thiazol-6-yl]benzonitrile

Copper cyanide (47 mg, 0.526 mmol) was added to an N,N-dimethylformamide (3 ml) solution of the compound (300 mg, 0.437 mmol) obtained in Step 1 above, and the resulting mixture was heated under nitrogen atmosphere at 140°C for 20 hours. After the reaction mixture was cooled, 1 N aqueous sodium hydroxide solution was added to the reaction mixture and stirred, and then the reaction mixture was extracted with ethyl acetate. The organic layer was washed with brine and then dried over anhydrous sodium sulfate. The solvent was evaporated under reduced pressure and the residue was purified by silica gel column chromatography (chloroform:methanol = 10:1) to give the title compound (134 mg, 51%) as a colorless solid.
$^1$H-NMR ($CDCl_3$) δ: 0.94-0.99 (6H, m), 1.83 (3H, s), 1.91-1.97 (2H, m), 2.12-2.25 (2H, m), 2.68 (1H, m), 3.44-3.84 (10H, m), 4.88 (1H, m), 5.01 (1H, m), 6.69 (2H, brs), 7.02 (2H, d, J=8.5 Hz), 7.29-7.36 (4H, m).
MS (FAB) m/z: 604, 606.

Example 138

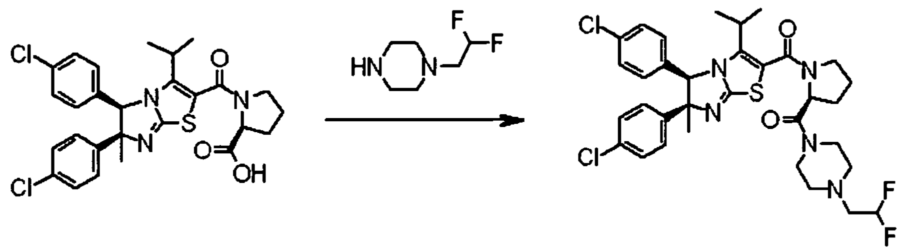

(5R,6S)-5,6-Bis(4-chlorophenyl)-2-{[(2S)-2-{[4-(2,2-difluoroethyl)piperazin-1-yl]carbonyl}pyrrolidin-1-yl]carbonyl}-3-isopropyl-6-methyl-5,6-dihydroimidazo[2,1-b][1,3]thiazole

The compound obtained in Step 2 of Example 61 was reacted in the same way as in Step 4 of Example 1 using 1-(2,2-difluoroethyl)piperazine instead of piperazin-2-one to give the title compound.
$^1$H-NMR ($CDCl_3$) δ: 0.96 (6H, d, J=7.1 Hz), 1.81 (3H, s), 1.86-2.00 (2H, m), 2.07-2.28 (2H, m), 2.50-2.75 (5H, m), 2.76 (2H, td, J=14.9, 4.2 Hz), 3.47-3.83 (6H, m), 4.87-4.93 (1H, m), 4.96 (1H, s), 5.88 (1H, tt, J=56.2, 4.2 Hz), 6.70 (2H, d, J=8.1 Hz), 7.00-7.05 (4H, m), 7.11 (2H, d, J=8.5 Hz).
MS (FAB) m/z: 676, 678.

Example 139

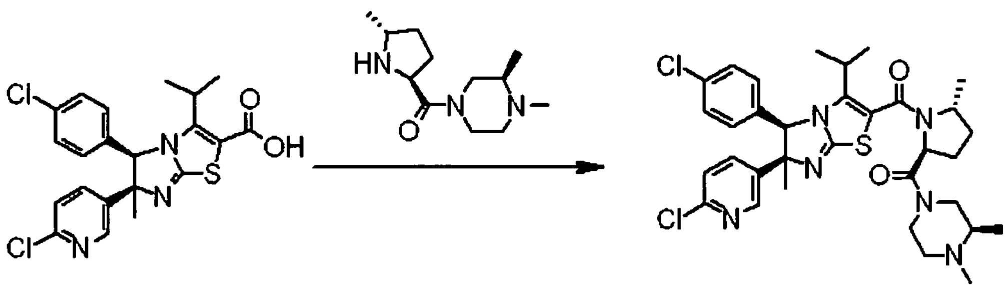

(5R,6S)-5-(4-Chlorophenyl)-6-(6-chloropyridin-3-yl)-2-{[(2S,5R)-2-{[(3R)-3,4-dimethylpiperazin-1-yl]carbonyl}-5-methylpyrrolidin-1-yl]carbonyl}-3-isopropyl-6-methyl-5,6-dihydroimidazo[2,1-b][1,3]thiazole

The compound obtained in Step 9 of Reference Example 38 was reacted in the same way as in Example 112 using the compound obtained in Step 3 of Reference Example 31 instead of the compound obtained in Step 2 of Reference Example 18 to give the title compound.
$^1$H-NMR ($CDCl_3$) δ: 0.95-1.11 (9H, m), 1.22-1.25 (3H, m), 1.65-1.71 (1H, m), 1.82-1.84 (1H, m), 1.83 (3H, s), 2.13-2.91 (8H, m), 3.41-3.75 (1H, m), 4.23-4.43 (1H, m), 4.55 (1H, t, J=6.6 Hz), 5.00 (1H, s), 5.01-5.05 (1H, m), 6.69-6.72 (2H, m), 6.99 (1H, d, J=8.0 Hz), 7.06 (2H, d, J=8.5 Hz), 7.51 (1H, d, J=8.0 Hz), 8.22 (1H, d, J=2.0 Hz).
MS (ESI) m/z: 655.

Example 140

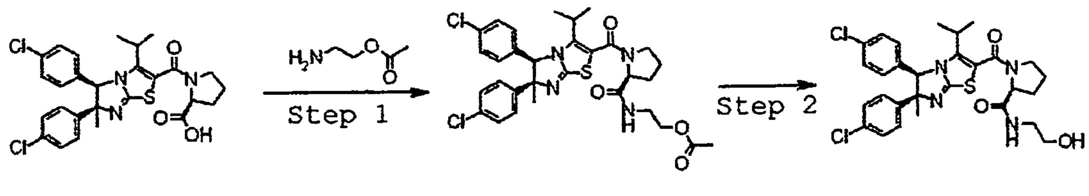


Step 1: 2-[(1-{[(5R,6S)-5,6-Bis(4-chlorophenyl)-3-isopropyl-6-methyl-5,6-dihydroimidazo[2,1-b][1,3]thiazol-2-yl]carbonyl}-L-prolyl)amino]ethyl acetate

The compound obtained in Step 2 of Example 61 was reacted in the same way as in Step 4 of Example 1 using 2-aminoethyl acetate instead of piperazin-2-one to give the title compound.
$^1$H-NMR ($CDCl_3$) δ : 0.92 (3H, d, J=7.1 Hz), 1.01 (3H, d, J=7.1 Hz), 1.81 (3H, s), 1.88-2.14 (3H, m), 2.06 (3H, s), 2.37-2.43

(1H, m), 2.54-2.61 (1H, m), 3.47-3.51 (2H, m), 3.61-3.70 (2H, m), 4.14 (2H, t, J=5.4 Hz), 4.58 (1H, dd, J=7.8, 4.2 Hz), 4.94 (1H, s), 6.70 (2H, d, J=8.3 Hz), 6.88 (1H, brs), 7.02 (2H, d, J=8.8 Hz), 7.04 (2H, d, J=7.8 Hz), 7.09 (2H, d, J=8.8 Hz).
MS (ESI) m/z: 629.

Step 2: 1-{[(5R,6S)-5,6-Bis(4-chlorophenyl)-3-isopropyl-6-methyl-5,6-dihydroimidazo[2,1-b][1,3]thiazol-2-yl]carbonyl}-N-(2-hydroxyethyl)-L-prolinamide

The compound (70 mg, 0.11 mmol) obtained in Step 1 above was dissolved in methanol (4.00 ml) followed by the addition of 28% sodium methoxide/methanol solution (2 μl, 0.01 mmol) and the resulting mixture was stirred at room temperature for 1 hour. Ion-exchange resin was added to the reaction mixture and the pH of the reaction mixture was adjusted to 7. After filtration, the filtrate was evaporated under reduced pressure and ethyl acetate and hexane were added to solidify to the obtained residue. The resulting mixture was dried under reduced pressure at 60°C to give the title compound (65 mg, 100%) as a colorless solid.
$^1$H-NMR ($CDCl_3$) δ: 0.92 (3H, d, J=7.1 Hz), 0.99 (3H, d, J=7.3 Hz), 1.81 (3H, s), 1.88-1.94 (1H, m), 2.03-2.09 (1H, m), 2.11-2.18 (1H, m), 2.33-2.39 (1H, m), 2.55-2.62 (1H, m), 3.32-3.37 (1H, m), 3.41-3.48 (1H, m), 3.64-3.74 (4H, m), 4.51 (1H, dd, J=7.9, 4.8 Hz), 4.95 (1H, s), 6.70 (2H, d, J=8.3 Hz), 6.78 (1H, brs), 7.01 (2H, d, J=8.8 Hz), 7.04 (2H, d, J=8.3 Hz), 7.09 (2H, d, J=8.5 Hz).
MS (ESI) m/z: 587.

Example 141

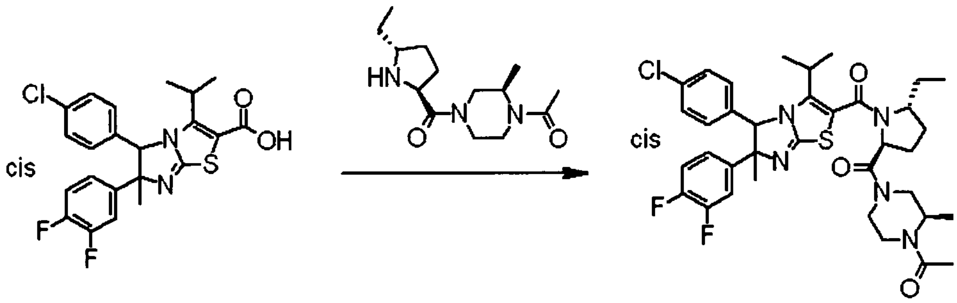

(5R*,6S*)-2-{[(25,5R)-2-{[(3R)-4-Acetyl-3-methylpiperazin-1-yl]carbonyl}-5-ethylpyrrolidin-1-yl]carbonyl}-5-(4-chlorophenyl)-6-(3,4-difluorophenyl)-3-isopropyl-6-methyl-5,6-dihydroimidazo[2,1-b][1,3]thiazole

The compound obtained in Step 8 of Reference Example 39 was reacted in the same way as in Example 112 using the compound obtained in Reference Example 97 instead of the compound obtained in Step 2 of Reference Example 18 to give the title compound.
$^1$H-NMR ($CDCl_3$) δ: 0.69-1.96 (18H, m), 1.79 (3H, s), 2.12 (3H, s), 2.66-3.74 (6H, m), 3.94-4.05 (1H, m), 4.31-4.45 (2H, m), 4.79-5.01 (2H, m), 6.68 (2H, d, J=8.0 Hz), 6.80-6.91 (2H, m), 7.08-7.01 (3H, m).
MS (ESI) m/z: 698.

Example 142

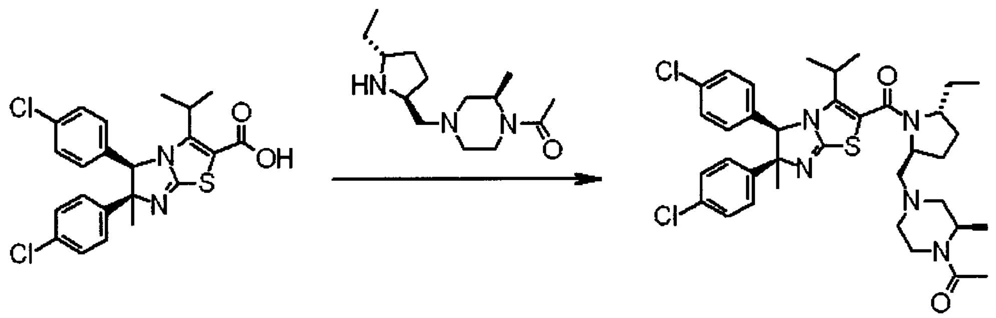

(5R,6S)-2-[((2S,5R)-2-{[(3R)-4-Acetyl-3-methylpiperazin-1-yl]methyl}-5-ethylpyrrolidin-1-yl)carbonyl]-5,6-bis(4-chlorophenyl)-3-isopropyl-6-methyl-5,6-dihydroimidazo[2,1-b][1,3]thiazole

The compound obtained in Step 3 of Example 1 was reacted in the same way as in Example 112 using the compound obtained in Step 4 of Reference Example 40 instead of the compound obtained in Step 2 of Reference Example 18 to give the title compound.
$^1$H-NMR ($CDCl_3$) δ: 0.91 (3H, t, J=7.4 Hz), 0. 97 (3H, d, J=7.3 Hz), 1.05 (3H, d, J=7.1 Hz), 1.27-1.29 (3H, m), 1.34-1.41 (1H, m), 1.69-1.73 (1H, m), 1.80 (3H, s), 2.06 (3H, s), 2.09-2.23 (4H, m), 2.42-2.46 (1H, m), 2.66-2.72 (1H, m), 2.74-2.88 (3H, m), 3.34-3.49 (2H, m), 3.91-4.00 (1H, m), 4.16-4.19 (1H, m), 4.27-4.35 (1H, m), 4.30-4.33 (1H, m), 4.71-4.76 (1H, m), 4.95 (1H, s), 6.66 (2H, d, J=8.5 Hz), 7.00 (2H, d, J=8.5 Hz), 7.02 (2H, d, J=8.5 Hz), 7.12 (2H, d, J=8.8 Hz).
MS (ESI) m/z: 682.

Example 143

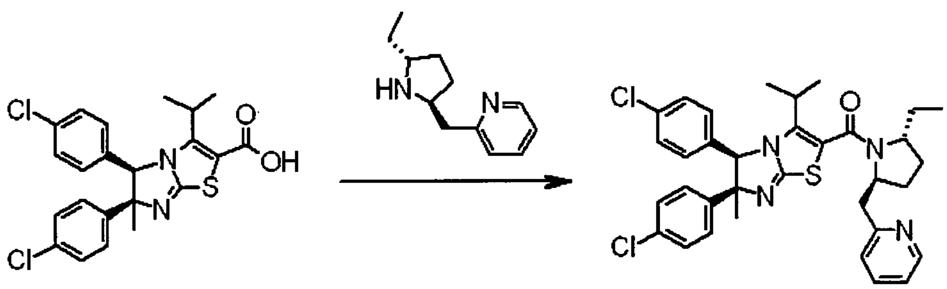

(5R,6S)-5,6-Bis(4-chlorophenyl)-2-{[(2R,5S)-2-ethyl-5-(pyridin-2-ylmethyl)pyrrolidin-1-yl]carbonyl}-3-isopropyl-6-methyl-5,6-dihydroimidazo[2,1-b][1,3]thiazole

The compound obtained in Step 3 of Example 1 was reacted in the same way as in Example 112 using the compound obtained in Step 2 of Reference Example 41 instead of the compound obtained in Step 2 of Reference Example 18 to give the title compound.
$^1$H-NMR ($CDCl_3$) δ: 0.88 (3H, t, J=7.3 Hz), 1.02 (3H, d, J=7.1 Hz), 1.07 (3H, d, J=7.1 Hz), 1.33-1.40 (1H, m), 1.67-1.95 (5H, m), 1.81 (3H, d, J=4.6 Hz), 2.74-2.82 (1H, m), 2.86-2.92 (1H, m), 3.19 (1H, dd, J=13.2, 3.2 Hz), 4.11-4.15 (1H, m), 4.64-4.68 (1H, m), 4.95 (1H, s), 6.67 (2H, d, J=8.5 Hz), 6.86-6.90 (2H, m), 7.02 (2H, d, J=8.5 Hz), 7.12-7.20 (4H, m), 7.61 (1H, td, J=7.6, 1.9 Hz), 8.56 (1H, d, J=4.2 Hz).
MS (ESI) m/z: 619.

Example 144

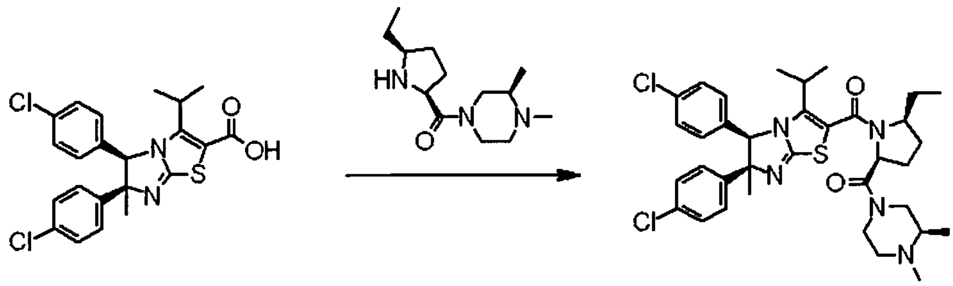

(5R,6S)-5,6-Bis(4-chlorophenyl)-2-[((2S,5S)-2-{[(3R)-3,4-dimethylpiperazin-1-yl]carbonyl}-5-ethylpyrrolidin-1-yl)carbonyl]-3-isopropyl-6-methyl-5,6-dihydroimidazo[2,1-b][1,3]thiazole

The compound obtained in Step 3 of Example 1 was reacted in the same way as in Example 112 using the compound obtained in Step 5 of Reference Example 42 instead of the compound obtained in Step 2 of Reference Example 18 to give the title compound.

$^1$H-NMR ($CDCl_3$) δ: 0.89-0.96 (9H, m), 1.06-1.09 (3H, m), 1.69-1.74 (1H, m), 1.80 (3H, s), 1.84-1.89 (1H, m), 1.94-2.14 (4H, m), 2.29 (3H, s), 2.60-2.66 (2H, m), 2.74-2.78 (1H, m), 2.95-3.03 (1H, m), 3.29-3.34 (1H, m), 3.59-3.65 (1H, m), 3.81-3.86 (1H, m), 3.98-4.02 (1H, m), 4.25-4.31 (1H, m), 4.83-4.88 (1H, m), 4.95 (1H, s), 6.68 (2H, d, J=8.3 Hz), 7.01 (2H, d, J=8.5 Hz), 7.02 (2H, d, J=8.8 Hz), 7.10 (2H, d, J=8.8 Hz).

MS (ESI) m/z: 668.

Example 145

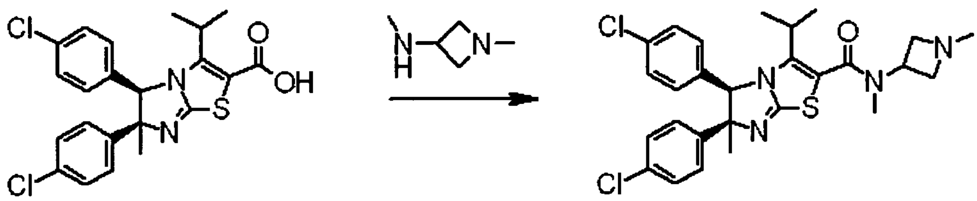

(5R,6S)-5,6-Bis(4-chlorophenyl)-3-isopropyl-N,6-dimethyl-N-(1 methylazetidin-3-yl)-5,6-dihydroimidazo[2,1-b][1,3]thiazole-2-carboxamide

The compound obtained in Step 3 of Example 1 was reacted in the same way as in Step 4 of Example 1 using N,1-dimethylazetidin-3-amine instead of piperazin-2-one to give the title compound.

$^1$H-NMR ($CDCl_3$) δ: 0.86 (3H, d, J=7.1 Hz), 0.97 (3H, d, J=7.1 Hz), 1.81 (3H, s), 2.37 (3H, s), 2.42-2.49 (1H, m), 3.11 (3H, s), 3.13-3.19 (2H, m), 3.58-3.66 (2H, m), 4.67-4.73 (1H, m), 4.94 (1H, s), 6.70-6.72 (2H, m), 6.99-7.11 (6H, m).

MS (FAB) m/z: 529, 531.

Example 146

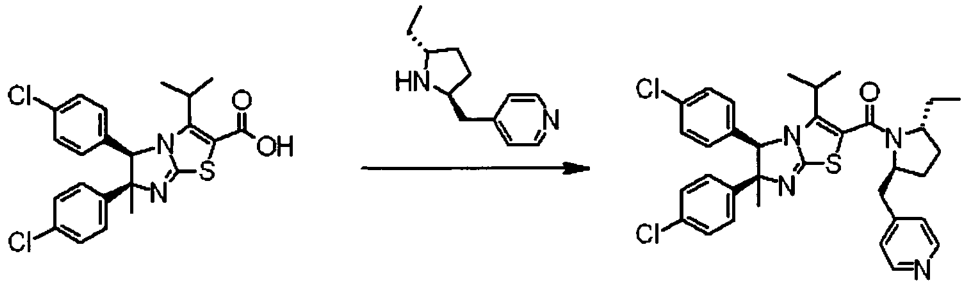

(5R,6S)-5,6-Bis(4-chlorophenyl)-2-{[(2R,5S)-2-ethyl-5-(pyridin-4-ylmethyl)pyrrolidin-1-yl]carbonyl}-3-isopropyl-6-methyl-5,6-dihydroimidazo[2,1-b][1,3]thiazole

The compound obtained in Step 3 of Example 1 was reacted in the same way as in Example 112 using the compound obtained in Reference Example 43 instead of the compound obtained in Step 2 of Reference Example 18 to give the title compound.
$^1$H-NMR ($CDCl_3$) δ: 0.90 (3H, t, J=7.4 Hz), 1.02 (3H, d, J=7.3 Hz), 1.06 (3H, d, J=6.8 Hz), 1.33-1.40 (1H, m), 1.64-1.76 (3H, m), 1.82 (3H, s), 1.86-1.94 (2H, m), 2.60 (1H, dd, J=13.1, 9.6 Hz), 2.77-2.84 (1H, m), 3.12 (1H, dd, J=13.3, 3.3 Hz), 4.15-4.19 (1H, m), 4.48-4.51 (1H, m), 4.97 (1H, s), 6.66 (2H, d, J=8.5 Hz), 6.93 (2H, d, J=6.8 Hz), 7.03 (2H, d, J=8.5 Hz), 7.13-7.16 (4H, m), 8.54 (2H, d, J=5.9 Hz).
MS (ESI) m/z: 619.

Example 147

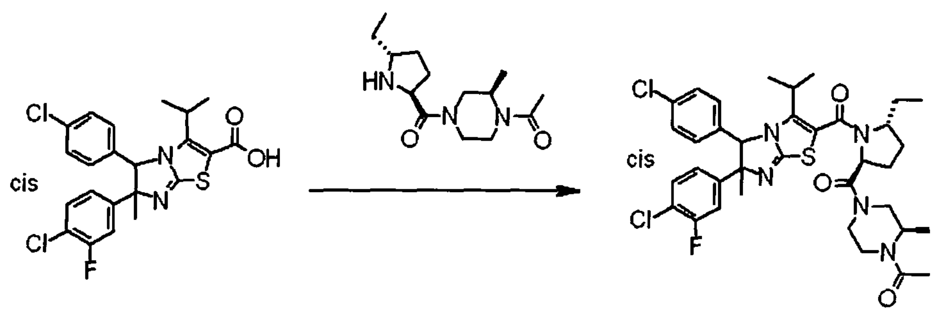

(5R*,6S*)-2-{[(2S,5R)-2-{[(3R)-4-Acetyl-3-methylpiperazin-1-yl]carbonyl}-5-ethylpyrrolidin-1-yl]carbonyl}-6-(4-chloro-3-fluorophenyl)-5-(4-chlorophenyl)-3-isopropyl-6-methyl-5,6-dihydroimidazo[2,1-b][1,3]thiazole

The compound obtained in Step 10 of Reference Example 44 was reacted in the same way as in Example 112 using the compound obtained in Reference Example 97 instead of the compound obtained in Step 2 of Reference Example 18 to give the title compound.
$^1$H-NMR ($CDCl_3$) δ: 0.71 (1H, d, J=6.8 Hz), 0.87-0.99 (5H, m), 0.99-1.33 (6H, m), 1.34-1.55 (1H, m), 1.57-1.98 (4H, m), 1.78 (3H, s), 2.02-2.43 (4H, m), 2.67-3.84 (5H, m), 3.90-4.20 (1H, m), 4.24-4.50 (2H, m), 4.75-5.03 (3H, m), 6.70 (2H, d, J=6.3 Hz), 6.84-6.95 (1H, m), 7.01-7.10 (4H, m).
MS (FAB) m/z: 714.

Example 148

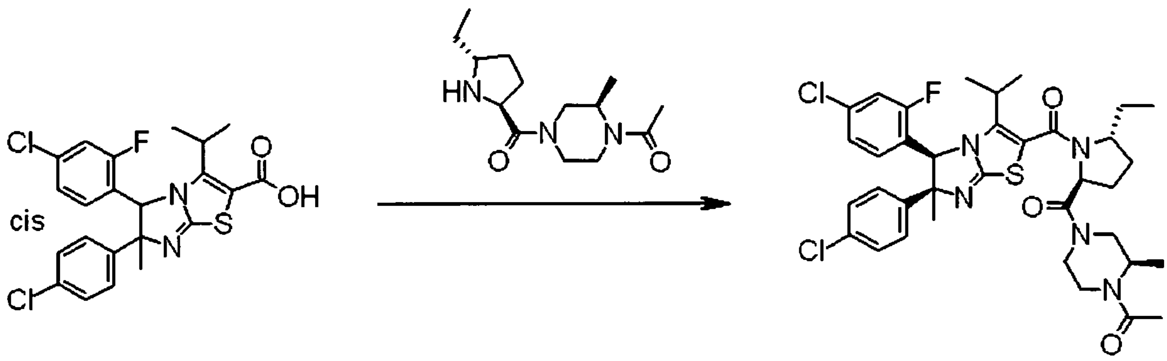

(5R,6S)-2-{[(2S,5R)-2-{[(3R)-4-Acetyl-3-methylpiperazin-1-yl]carbonyl}-5-ethylpyrrolidin-1-yl]carbonyl}-5-(4-chloro-2-fluorophenyl)-6-(4-chlorophenyl)-3-isopropyl-6-methyl-5,6-dihydroimidazo[2,1-b][1,3]thiazole

The compound obtained in Step 8 of Reference Example 45 was reacted in the same way as in Step 4 of Example 97 using the compound obtained in Reference Example 97 instead of 4-(L-prolyl)morpholine to give the title compound.
$^1$H-NMR ($CDCl_3$) δ: 0.89-0.96 (6H, m), 1.10 (3H, d, J=6.8 Hz), 1.17-2.24 (6H, m), 1.83 (3H, s), 2.12 (3H, s), 2.33-4.51 (8H, m), 4.92-5.06 (2H, m), 5.42 (1H, s), 6.59-6.61 (1H, m), 6.79-6.85 (2H, m), 7.06 (2H, d, J=8.5 Hz), 7.21 (2H, d, J=8.5 Hz).
MS (ESI) m/z: 714.

Example 149

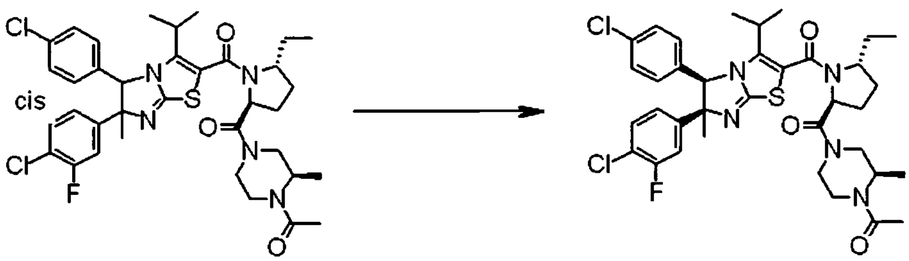

(5R,6S)-2-{[(2S,5R)-2-{[(3R)-4-Acetyl-3-methylpiperazin-1-yl]carbonyl}-5-ethylpyrrolidin-1-yl]carbony}-6-(4-chloro-3-fluorophenyl)-5-(4-chlorophenyl)-3-isopropyl-6-methyl-5,6-dihydroimidazo[2,1-b][1,3]thiazole

The compound (60 mg) obtained in Example 147 was resolved with an optically active column to give the title compound (27.2 mg, 45%) as a colorless solid.
$^1$H-NMR ($CDCl_3$) δ: 0.87-0.98 (5H, m), 1.04 (3H, d, J=6.8 Hz), 1.07-1.34 (3H, m), 1.35-1.48 (1H, m), 1.58-1.97 (6H, m), 2.03-2.46 (5H, m), 2.66-2.99 (2H, m), 3.09-3.25 (1H, m), 3.27-3.81 (2H, m), 3.88-4.11 (1H, m), 4.27-4.49 (3H, m), 4.74-5.04 (3H, m), 6.66-6.73 (2H, m), 6.90 (1H, d, J=8.3 Hz), 7.01-7.10 (4H, m).
MS (FAB) m/z: 714, 716.

Example 150

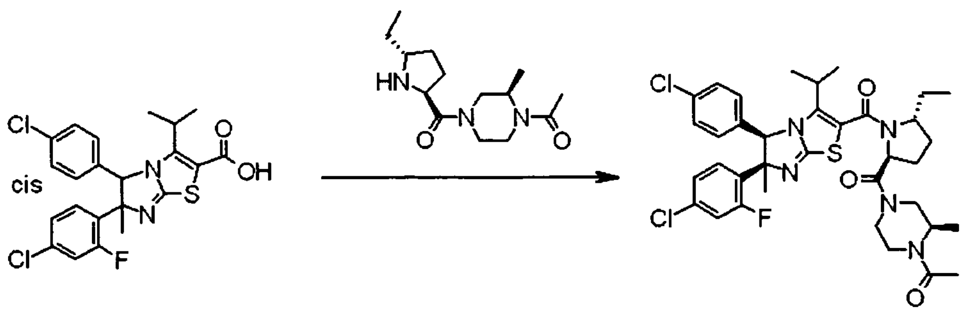

(5R,6S)-2-{[(2S,5R)-2-{[(3R)-4-Acetyl-3-methylpiperazin-1-yl]carbonyl}-5-ethylpyrrolidin-1-yl]carbony}-6-(4-chloro-2-fluorophenyl)-5-(4-chlorophenyl)-3-isopropyl-6-methyl-5,6-dihydroimidazo[2,1-b][1,3]thiazole

The compound obtained in Step 7 of Example 46 was reacted in the same way as in Step 4 of Example 97 using the compound obtained in Reference Example 97 instead of 4-(L-prolyl)morpholine to give the title compound.
$^1$H-NMR ($CDCl_3$) δ: 0.78-0.88 (6H, m), 1.03 (3H, d, J=6.8 Hz), 1.07-1.95 (9H, m), 1.74 (3H, s), 2.11 (3H, s), 2.16-2.40 (1H, m), 2.75-2.93 (2H, m), 3.15-3.74 (2H, m), 3.91-4.04 (1H, m), 4.28-4.44 (2H, m), 4.79-5.05 (2H, m), 5.14 (1H, s), 6.76 (1H, d, J=11.2 Hz), 6.90-6.93 (3H, m), 7.03 (2H, d, J=8.5 Hz), 7.66-7.72 (1H, m).
MS (ESI) m/z: 714.

Example 151

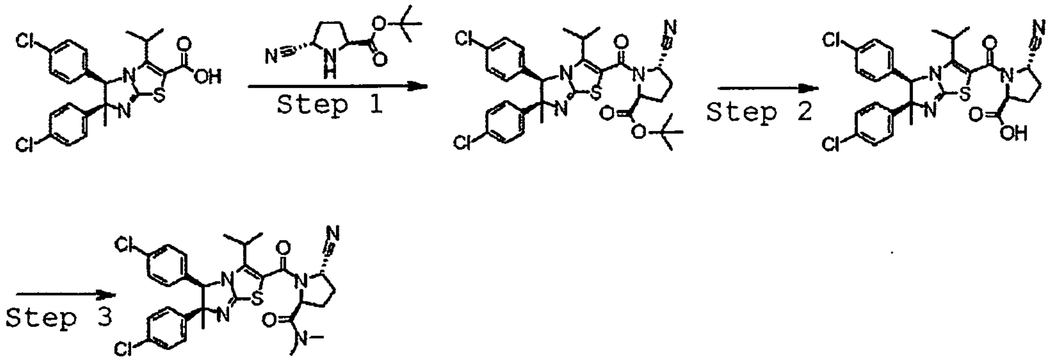


Step 1: tert-Butyl (5S)-1-{[(5R,6S)-5,6-Bis(4-chlorophenyl)-3-isopropyl-6-methyl-5,6-dihydroimidazo[2,1-b][1,3]thiazol-2-yl]carbonyl}-5-cyano-L-prolinate

The compound obtained in Step 3 of Example 1 was reacted in the same way as in Example 112 using the compound (Isomer A) obtained in Reference Example 47 instead of the compound obtained in Step 2 of Reference Example 18 to give the title compound.
$^1$H-NMR ($CDCl_3$) δ: 1.02 (3H, d, J=7.1 Hz), 1.04 (3H, d, J=7.1 Hz), 1.46 (9H, s), 1.82 (3H, s), 2.20-2.25 (1H, m), 2.35-2.53 (3H, m), 2.64-2.69 (1H, m), 4.70 (1H, d, J=7.8 Hz), 4.98 (1H, s), 5.11-5.14 (1H, m), 6.67 (2H, d, J=8.1 Hz), 7.02 (2H, d, J=8.5 Hz), 7.03 (2H, d, J=8.5 Hz), 7.10 (2H, d, J=8.5 Hz).
MS (ESI) m/z: 625.

Step 2: (5S)-1-{[(5R,6S)-5,6-Bis(4-chlorophenyl)-3-isopropyl-6-methyl-5,6-dihydroimidazo[2,1-b][1,3]thiazol-2-yl]carbonyl}-5-cyano-L-proline

The compound obtained in Step 1 above was reacted in the same way as in Step 2 of Example 61 to give the

title compound.
$^1$H-NMR (DMSO-$d_6$) δ: 0.89 (3H, d, J=7.1 Hz), 0.94 (3H, d, J=7.1 Hz), 1.75 (3H, s), 2.13-2.18 (1H, m), 2.24-2.34 (2H, m), 2.42-2.47 (1H, m), 2.64-2.71 (1H, m), 4.72-4.74 (1H, m), 5.08-5.11 (1H, m), 5.51 (1H, s), 6.83 (2H, d, J=7.3 Hz), 7.08 (2H, d, J=8.8 Hz), 7.09 (2H, d, J=8.8 Hz), 7.24 (2H, d, J=8.5 Hz).
MS (ESI) m/z: 569.

Step 3: (5S)-1-{[(5R,6S)-5,6-Bis(4-chlorophenyl)-3-isopropyl-6-methyl-5,6-dihydroimidazo[2,1-b][1,3]thiazol-2-yl]carbonyl}-5-cyano-N,N-dimethyl-L-prolinamide

The compound obtained in Step 2 above was reacted in the same way as in Step 4 of Example 1 using dimethylamine instead of piperazin-2-one to give the title compound.
$^1$H-NMR ($CDCl_3$) δ: 1.00 (3H, d, J=7.3 Hz), 1.03 (3H, d, J=7.1 Hz), 1.81 (3H, s), 2.01-2.08 (1H, m), 2.29-2.36 (1H, m), 2.45-2.52 (1H, m), 2.58-2.63 (1H, m), 2.69-2.76 (1H, m), 2.93 (3H, s), 3.13 (3H, s), 4.96 (1H, s), 5.11-5.14 (1H, m), 5.19 (1H, d, J=7.1 Hz), 6.66 (2H, d, J=8.5 Hz), 7.01 (2H, d, J=8.5 Hz), 7.02 (2H, d, J=8.5 Hz), 7.10 (2H, d, J=8.5 Hz).
MS (ESI) m/z: 596.

Example 152

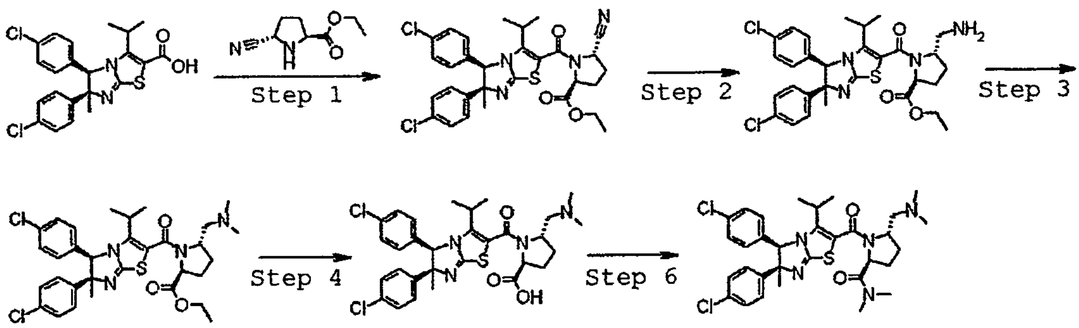


Step 1: Ethyl (5S)1-{[(5R,6S)-5,6-Bis(4-chlorophenyl)-3-isopropyl-6-methyl-5,6-dihydroimidazo[2,1-b][1,3]thiazol-2-yl] carbonyl}-5-cyano-L-prolinate

The compound obtained in Step 3 of Example 1 was reacted in the same way as in Example 112 using ethyl (5S)-5-cyano-L-prolinate instead of the compound obtained in Step 2 of Reference Example 18 to give the title compound.
$^1$H-NMR ($CDCl_3$) δ: 1.00 (3H, d, J=7.1 Hz), 1.03 (3H, d, J=7.1 Hz), 1.29 (3H, t, J=7.1 Hz), 1.83 (3H, s), 2.13-2.18 (1H, m), 2.22-2.27 (1H, m), 2.31-2.40 (1H, m), 2.46-2.53 (1H, m), 2.64-2.71 (1H, m), 4.15-4.22 (2H, m), 4.80-4.83 (1H, m), 4.98 (1H, s), 5.12-5.15 (1H, m), 6.66 (2H, d, J=8.0 Hz), 7.02 (2H, d, J=8.8 Hz), 7.03 (2H, d, J=8.8 Hz), 7.10 (2H, d, J=8.5 Hz).
MS (ESI) m/z: 597.

Step 2: Ethyl (5S)-5-(Aminomethyl)-1-{[(5R,6S)-5,6-bis(4-chlorophenyl)-3-isopropyl-6-methyl-5,6-dihydroimidazo[2,1-b][1,3]thiazol-2-yl]carbonyl}-L-prolinate

The compound (100 mg, 0.17 mmol) obtained in Step 1 above was dissolved in methanol (4 ml) followed by the addition of cobalt (II) chloride (22 mg, 0.17 mmol) and sodium borohydride (34 mg, 0.90 mmol) and the resulting mixture was stirred at room temperature for 1 hour. After the reaction mixture was filtrated, the filtrate was evaporated under reduced pressure and the obtained residue was purified by silica gel column chromatography (chloroform → chloroform: methanol = 50:1) to give the title compound (99 mg, 94%) as a light brown oil.
$^1$H-NMR ($CDCl_3$) δ: 0.92-0.96 (3H, m), 1.02-1.05 (3H, m), 1.25-1.29 (3H, m), 1.80 (3H, s), 1.93-2.02 (2H, m), 2.12-2.20 (1H, m), 2.33-2.37 (1H, m), 2.68 (1H, dd, J=12.8, 7.9 Hz), 2.69-2.77 (1H, m), 2.90-2.95 (1H, m), 3.67-3.75 (1H, m), 4.12 (2H, q, J=7.2 Hz), 4.35-4.40 (1H, m), 4.95 (1H, s), 6.65 (2H, d, J=8.1 Hz), 7.02 (7H, d, J=8.5 Hz), 7.12 (7H, d, J=8.8 Hz).

Step 3: Ethyl (5S) -1-{[(5R,6S)-5,6-Bis(4-chlorophenyl)-3-isopropyl-6-methyl-5,6-dihydroimidazo[2,1-b][1,3]thiazol-2-yl]carbonyl}-5-[(dimethylamino)methyl]-L-prolinate

The compound (99 mg, 0.16 mmol) obtained in Step 2 above was dissolved in 1,4-dioxane (4.00 ml) followed by the addition of 35% aqueous formaldehyde solution (124 μl, 1.6 mmol) and sodium triacetoxyborohydride (84 mg, 0.4 mmol) and the resulting mixture was stirred at room temperature for 1 hour. The reaction mixture was diluted with ethyl acetate and the organic layer was washed with saturated aqueous sodium bicarbonate solution and brine. The organic layer was dried over anhydrous magnesium sulfate and, after the reaction mixture was filtrated, the filtrate was evaporated under reduced pressure. The obtained residue was purified by silica gel column chromatography (chloroform: methanol = 15:1) to give the title compound (45 mg, 45%) as a colorless solid.
$^1$H-NMR ($CDCl_3$) δ: 0.88-0.95 (3H, m), 1.08 (3H, d, J=7.1 Hz), 1.26 (3H, t, J=7.1 Hz), 1.79 (3H, s), 2.03-2.13 (3H, m), 2.24-2.34 (3H, m), 2.28 (6H, s), 2.75-2.82 (1H, m), 3.65-3.72 (1H, m), 4.07-4.18 (2H, m), 4.45-4.50 (1H, m), 4.95 (1H, s), 6.67-6.69 (2H, m), 7.01 (2H, d, J=8.8 Hz), 7.02 (2H, d, J=8.8 Hz), 7.11 (2H, d, J=8.5 Hz).

Step 4: (5S)-1-{[(5R,6S)-5,6-Bis(4-chlorophenyl)-3-isopropyl-6-methyl-5,6-dihydroimidazo[2,1-b][1,3]thiazol-2-yl]carbonyl}-5-[(dimethylamino)methyl]-L-proline

The compound obtained in Step 3 above was reacted in the same way as in Step 3 of Example 1 to give the title compound.
$^1$H-NMR (DMSO-$d_6$) δ: 0.79 (3H, d, J=7.1 Hz), 1.02 (3H, d, J=7.1 Hz), 1.71 (3H, s), 1.89-1.97 (4H, m), 2.17 (6H, s), 2.22-2.31 (2H, m), 2.76-2.81 (1H, m), 4.26-4.29 (1H, m), 4.48-4.52 (1H, m), 5.45 (1H, s), 6.84-6.88 (2H, m), 7.07 (2H, d, J=8.5 Hz), 7.09 (2H, d, J=10.0 Hz), 7.25 (2H, d, J=8.5 Hz).
MS (ESI) m/z: 601.

Step 5: (5S)-1-{[(5R,6S)-5,6-Bis(4-chlorophenyl)-3-isopropyl-6-methyl-5,6-dihydroimidazo[2,1-b][1,3]thiazol-2-yl]carbonyl}-5-[(dimethylamino)methyl]-N,N-dimethyl-L-prolinamide

The compound obtained in Step 4 above was reacted in the same way as in Step 4 of Example 1 using dimethylamine instead of piperazin-2-one to give the title compound.
$^1$H-NMR ($CDCl_3$) δ: 0.83-0.88 (3H, m), 1.10 (3H, d, J=7.1 Hz), 1.47-1.63 (2H, m), 1.78 (3H, s), 1.86-1.91 (1H, m), 2.13-2.30 (3H, m), 2.34 (6H, brs), 2.88 (3H, brs), 2.89-2.93 (1H, m), 3.11 (3H, brs), 4.52-4.58 (1H, m), 4.95 (1H, s), 5.00-5.05 (1H, m), 6.67 (2H, d, J=6.8 Hz), 7.00 (2H, d, J=8.8 Hz), 7.01 (2H, d, J=8.8 Hz), 7.11 (2H, d, J=8.5 Hz).
MS (ESI) m/z: 628.

Example 153

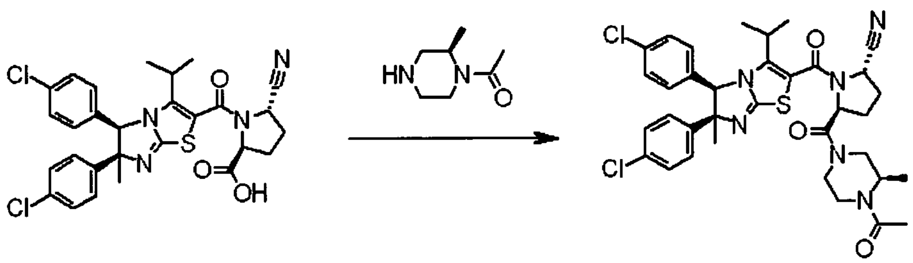

(2S,5S)-5-{[(3R)-4-Acetyl-3-methylpiperazin-1-yl]carbonyl}-1-{[(5R,6S)-5,6-bis(4-chlorophenyl)-3-isopropyl-6-methyl-5,6-dihydroimidazo[2,1-b][1,3]thiazol-2-yl]carbonyl}pyrrolidine-2-carbonitrile

The compound obtained in Step 2 of Example 151 was reacted in the same way as in Step 4 of Example 1 using (2R)-1-acetyl-2-methylpiperazine hydrochloride instead of piperazin-2-one to give the title compound.
$^1$H-NMR ($CDCl_3$) δ: 1.00-1.05 (6H, m), 1.14-1.20 (3H, m), 1.81 (3H, s), 2.06-2.09 (2H, m), 2.10 (3H, s), 2.34-2.45 (2H, m), 2.64-2.73 (2H, m), 2.90-2.95 (1H, m), 3.17-3.22 (1H, m), 3.45-3.49 (1H, m), 3.70-3.74 (1H, m), 3.95-4.00 (1H, m), 4.27-4.32 (1H, m), 4.96 (1H, s), 5.10-5.13 (1H, m), 5.21 (1H, d, J=7.8 Hz), 6.68 (2H, d, J=8.5 Hz), 7.02 (4H, d, J=8.5

Hz), 7.09 (2H, d, J=8.8 Hz).
MS (ESI) m/z: 693.

Example 154

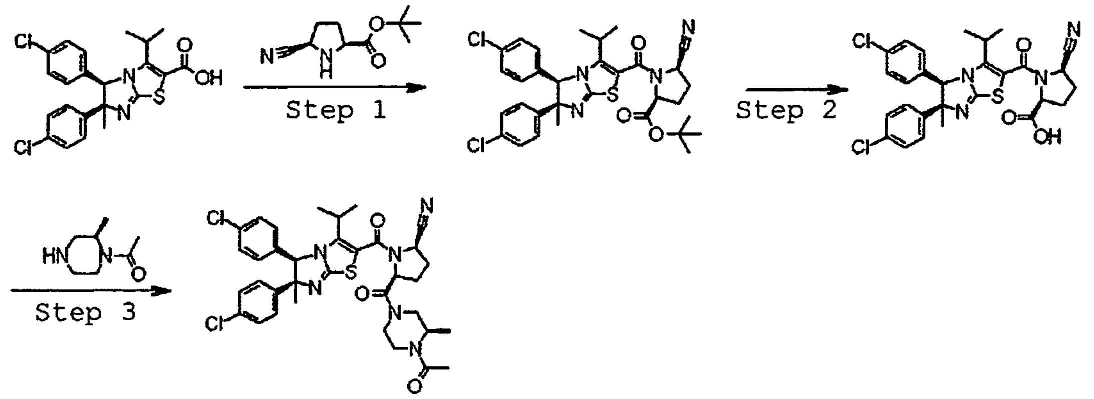


Step 1: tert-Butyl (5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-cyano-L-prolinate

The compound obtained in Step 3 of Example 1 was reacted in the same way as in Example 112 using the compound (Isomer B) obtained in Reference Example 47 instead of the compound obtained in Step 2 of Reference Example 18 to give the title compound.
$^1$H-NMR ($CDCl_3$) δ: 0. 93 (3H, d, J=7.1 Hz), 0. 98 (3H, d, J=6.8 Hz), 1.51 (9H, s), 1.82 (3H, s), 2.27-2.41 (4H, m), 2.73-2.81 (1H, m), 4.59-4.62 (1H, m), 4.87 (1H, t, J=6.6 Hz), 5.01 (1H, s), 6.70 (2H, d, J=7.6 Hz), 7.02 (2H, d, J=8.5 Hz), 7.04 (2H, d, J=8.8 Hz), 7.08 (2H, d, J=8.5 Hz).
MS (ESI) m/z: 625.

Step 2: (5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-cyano-L-proline

The compound obtained in Step 1 above was reacted in the same way as in Step 2 of Example 61 to give the title compound.
$^1$H-NMR (DMSO-$d_6$) δ: 0.89 (3H, d, J=7.1 Hz), 0.92 (3H, d, J=7.1 Hz), 1.84 (3H, s), 2.10-2.23 (2H, m), 2.35-2.44 (2H, m), 2.66-2.73 (1H, m), 4.63-4.66 (1H, m), 4.92-4.96 (1H, m), 5.70 (1H, s), 6.86-6.88 (2H, m), 7.12 (2H, d, J=8.8 Hz), 7.15 (2H, d, J=8.8 Hz), 7.23 (2H, d, J=8.5 Hz).
MS (ESI) m/z: 569.

Step 3: (2R,5S)-5-{[(3R)-4-Acetyl-3-methylpiperazin-1-yl]carbonyl}-1-{[(5R,6S)-5,6-bis(4-chlorophenyl)-3-isopropyl-6-methyl-5,6-dihydroimidazo[2,1-b][1,3]thiazol-2-yl]carbonyl}pyrrolidine-2-carbonitrile

The compound obtained in Step 2 above was reacted in the same way as in Step 4 of Example 1 using (2R)-1-acetyl-2-methylpiperazine instead of piperazin-2-one to give the title compound.
$^1$H-NMR ($CDCl_3$) δ: 0.86-0.93 (6H, m), 1.19-1.22 (3H, m), 1.82 (3H, s), 2.09 (3H, s), 2.12-2.17 (1H, m), 2.22-2.28 (2H, m), 2.40-2.46 (1H, m), 2.58-2.68 (2H, m), 2.90-2.98 (1H, m), 3.17-3.22 (1H, m), 3.44-3.51 (1H, m), 3.59-3.66 (1H, m), 3.94-3.98 (1H, m), 4.38-4.41 (1H, m), 4.93-4.98 (2H, m), 5.02 (1H, s), 6.72 (2H, d, J=7.1 Hz), 7.01 (2H, d, J=8.8 Hz), 7.03 (2H, d, J=8.5 Hz), 7.07 (2H, d, J=8.8 Hz).
MS (ESI) m/z: 693.

Example 155

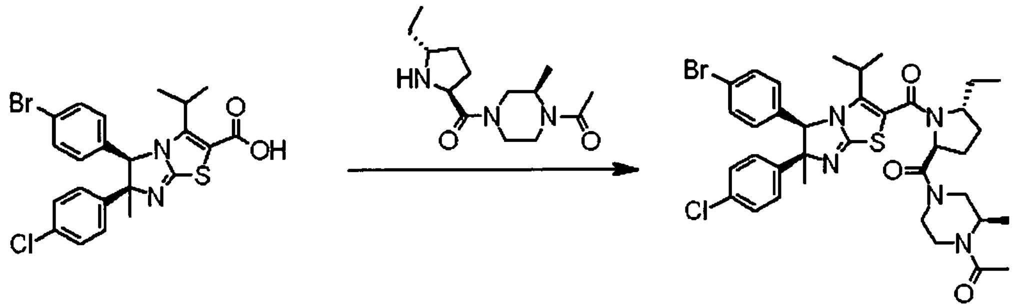

(5R,6S)-2-{[(2S,5R)-2-{[(3R)-4-Acetyl-3-methylpiperazin-1-yl]carbonyl}-5-ethylpyrrolidin-1-yl]carbonyl}-5-(4-bromophenyl)-6-(4-chlorophenyl)-3-isopropyl-6-methyl-5,6-dihydroimidazo[2,1-b][1,3]thiazole

The compound obtained in Step 10 of Reference Example 48 was reacted in the same way as in Example 112 using the compound obtained in Reference Example 97 instead of the compound obtained in Step 2 of Reference Example 18 to give the title compound.
$^1$H-NMR ($CDCl_3$) δ: 0.90-2.40 (18H, m), 1.79 (3H, s), 2.12 (3H, s), 2.70-2.96 (2H, m), 3.12-4.44 (7H, m), 4.78-5.02 (2H, m), 6.60-6.63 (2H, m), 7.03 (2H, d, J=8.3 Hz), 7.13-7.17 (4H, m).

Example 156

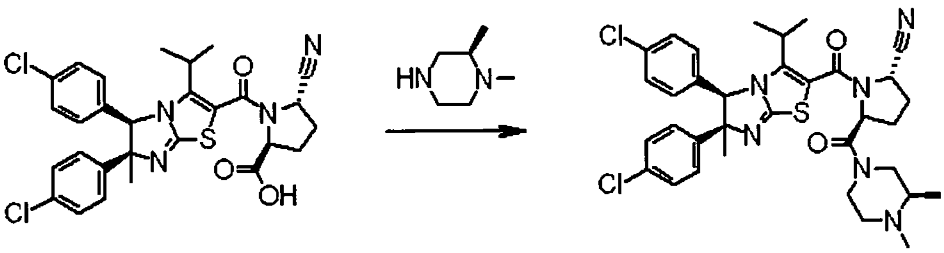

(2S,5S)-1-{[(5R,6S)-5,6-Bis(4-chlorophenyl)-3-isopropyl-6-methyl-5,6-dihydroimidazo[2,1-b][1,3]thiazol-2-yl]carbonyl}-5-{[(3R)-3,4-dimethylpiperazin-1-yl]carbonyl}pyrrolidine-2-carbonitrile

The compound obtained in Step 2 of Example 151 was reacted in the same way as in Step 4 of Example 1 using (2R)-1,2-dimethylpiperazine instead of piperazin-2-one to give the title compound.
$^1$H-NMR ($CDCl_3$) δ: 1.00-1.12 (9H, m), 1.81 (3H, s), 2.15-2.26 (2H, m), 2.30 (3H, s), 2.45-2.56 (2H, m), 2.69-2.93 (3H, m), 3.42-3.48 (1H, m), 3.63-3.72 (2H, m), 4.14 (1H, d, J=13.7 Hz), 4.33 (1H, d, J=12.2 Hz), 4.96 (1H, s), 5.13 (1H, d, J=7.3 Hz), 5.19 (1H, d, J=8.1 Hz), 6.66 (2H, d, J=7.6 Hz), 7.01-7.05 (6H, m), 7.09 (2H, d, J=8.5 Hz).
MS (ESI) m/z: 665.

Example 157

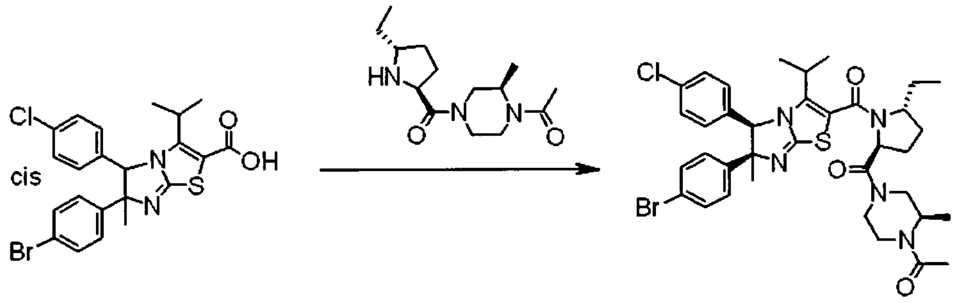

(5R,6S)-2-{[(2S,5R)-2-{[(3R)-4-Acetyl-3-methylpiperazin-1-yl]carbonyl}-5-ethylpyrrolidin-1-yl]carbony}-6-(4-bromophenyl)-5-(4-chlorophenyl)-3-isopropyl-6-methyl-5,6-dihydroimidazo[2,1-b][1,3]thiazole

The compound obtained in Step 10 of Reference Example 37 was reacted in the same way as in Step 4 of Example 97 using the compound obtained in Reference Example 97 instead of 4-(L-prolyl)morpholine to give the title compound.
$^1$H-NMR (CDCl3) δ: 0.91-0.96 (6H, m), 1.04 (3H, d, J=6.8 Hz), 1.12-2.33 (10H, m), 1.80 (3H, s), 2.12 (3H, s), 2.74-3.73 (4H, m), 3.94-4.43 (2H, m), 4.80-5.02 (1H, m), 4.95 (1H, s), 6.68 (2H, d, J=8.5 Hz), 7.01 (2H, d, J=8.5 Hz), 7.08 (2H, d, J=8.5 Hz), 7.18 (2H, d, J=8.5 Hz).
MS (ESI) m/z: 740.

Example 158

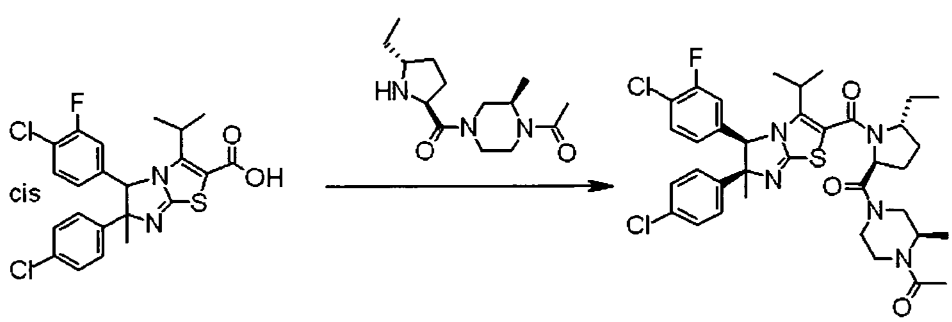

(5R,6S)-2-{[(2S,5R)-2-{[(3R)-4-Acetyl-3-methylpiperazin-1-yl]carbonyl}-5-ethylpyrrolidin-1-yl]carbonyl}-5-(4-chloro-3-fluorophenyl)-6-(4-chlorophenyl)-3-isopropyl-6-methyl-5,6-dihydroimidazo[2,1-b][1,3]thiazole

The compound obtained in Step 6 of Reference Example 50 was reacted in the same way as in Step 4 of Example 97 using the compound obtained in Reference Example 97 instead of 4-(L-prolyl)morpholine to give the title compound.
$^1$H-NMR (CDCl3) δ: 0.93-0.96 (6H, m), 1.06 (3H, d, J=7.1 Hz), 1.14-1.91 (5H, m), 1.53 (3H, brs), 1.80 (3H, brs), 2.05-2.36 (2H, m), 2.12 (3H, brs), 2.74-3.73 (4H, m), 4.00-4.38 (2H, m), 4.81-5.03 (2H, m), 4.93 (1H, s), 6.48-6.54 (2H, m), 7.17-7.04 (5H, m).
MS (ESI) m/z: 714.

Example 159

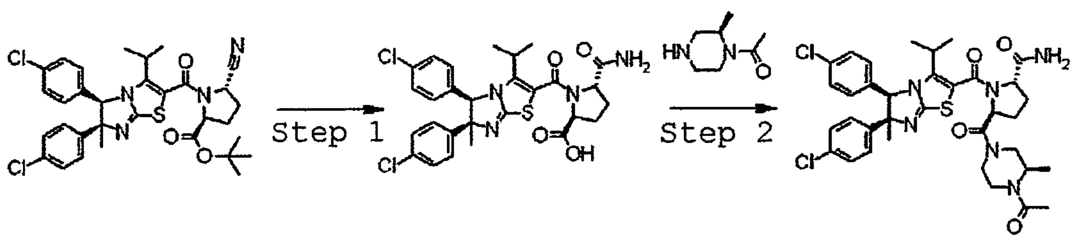


Step 1: (5S)-5-(Aminocarbonyl)-1-{[(5R,6S)-5,6-bis(4-chlorophenyl)-3-isopropyl-6-methyl-5,6-dihydroimidazo[2,1-b][1,3]thiazol-2-yl]carbonyl}-L-proline

The compound (300 mg, 0.48 mmol) obtained in Step 1 of Example 151 was suspended in ethanol (10 ml) followed by the addition of 1 N aqueous sodium hydroxide solution (2 ml), and the resulting mixture was heated and stirred at 70°C for 16 hours. The reaction mixture was returned to room temperature and the reaction solvent was evaporated under reduced pressure. Ice-cold water (10 ml) was added to the obtained residue and the residue was dissolved. 1 N aqueous hydrochloric acid solution (2 ml) was added and the deposited solid was collected by filtration. The resulting substance was dried under reduced pressure at 60°C to give a colorless solid including the title compound.
MS (ESI) m/z: 587.

Step 2: (2S,5S)-5-{[(3R)-4-Acetyl-3-methylpiperazin-1-yl]carbonyl}-1-{[(5R,6S)-5,6-bis(4-chlorophenyl)-3-isopropyl-6-methyl-5,6-dihydroimidazo[2,1-b][1,3]thiazol-2-yl]carbonyl}pyrrolidine-2-carboxamide

The compound obtained in Step 1 above was reacted in the same way as in Step 4 of Example 1 using (2R)-1-acetyl-2-methylpiperazine hydrochloride instead of piperazin-2-one to give the title compound.
$^1$H-NMR ($CDCl_3$) δ: 0.91 (3H, d, J=6.8 Hz), 0.95 (3H, d, J=7.1 Hz), 1.22-1.27 (3H, m), 1.78 (3H, s), 2.02-2.06 (1H, m), 2.11 (3H, s), 2.16-2.26 (2H, m), 2.34-2.39 (1H, m), 2.56-2.61 (1H, m), 2.91-3.00 (1H, m), 3.19-3.28 (1H, m), 3.55-3.72 (1H, m), 4.02-4.06 (1H, m), 4.35-4.58 (3H, m), 4.84-4.94 (2H, m), 5.52 (1H, s), 6.68 (2H, d, J=6.8 Hz), 7.00 (2H, d, J=8.5 Hz), 7.06 (2H, d, J=9.5 Hz), 7.08 (2H, d, J=8.8 Hz), 9.34-9.47 (2H, m).
MS (ESI) m/z: 711.

Example 160

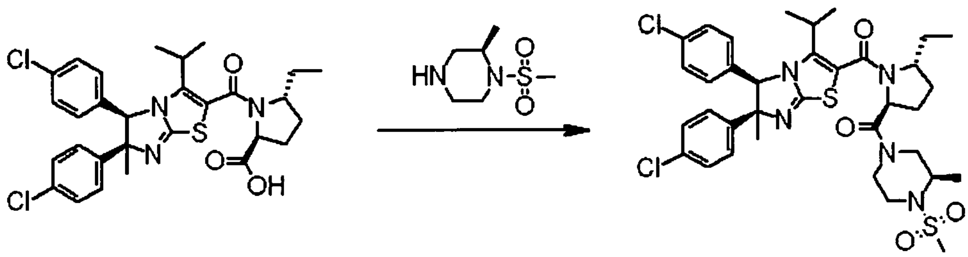

(5R,6S)-5,6-Bis(4-chlorophenyl)-2-[((2R,5S)-2-ethyl-5-{[(3R)-3-methyl-4-(methylsulfonyl)piperazin-1-yl]carbonyl}pyrrolidin-1-yl)carbonyl]-3-isopropyl-6-methyl-5,6-dihydroimidazo[2,1-b][1,3]thiazole

The compound obtained in Step 2 of Example 103 was reacted in the same way as in Step 4 of Example 1 using the compound obtained in Step 2 of Reference Example 49 instead of piperazin-2-one to give the title compound.
$^1$H-NMR ($CDCl_3$) δ: 0.89 (3H, d, J=6.6 Hz), 0.93 (3H, t, J=7.3 Hz), 1.03 (3H, d, J=6.8 Hz), 1.21-1.24 (3H, m), 1.38-1.45 (1H, m), 1.73-1.76 (1H, m), 1.79 (3H, s), 1.81-1.86 (2H, m), 2.14-2.18 (1H, m), 2.34-2.38 (1H, m), 2.73-2.80 (1H, m), 2.85 (3H, s), 2.94-2.99 (1H, m), 3.27-3.30 (1H, m), 3.43-3.47 (1H, m), 3.58-3.62 (1H, m), 4.01-4.03 (1H, m), 413-4.15 (1H, m), 4.28-4.33 (2H, m), 4.94 (1H, s), 4.94-4.98 (1H, m), 6.68 (2H, d, J=8.1 Hz), 7.01 (2H, d, J=8.5 Hz), 7.01 (2H, d,

J=8.8 Hz), 7.12 (2H, d, J=8.5 Hz).
MS (ESI) m/z: 732.

Example 161

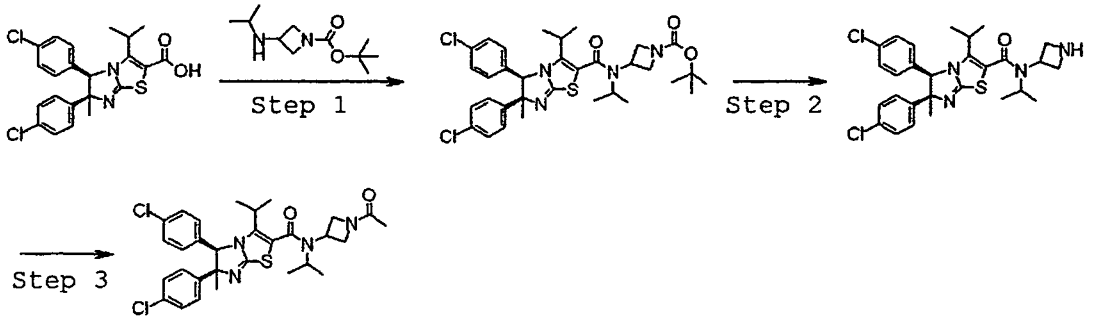


Step 1: tert-Butyl 3-[{[(5R,6S)-5,6-Bis(4-chlorophenyl)-3-isopropyl-6-methyl-5,6-dihydroimidazo[2,1-b][1,3]thiazol-2-yl]carbonyl}(isopropyl)amino]azetidine-1-carboxylate

The compound obtained in Step 3 of Example 1 was reacted in the same way as in Example 112 using the compound obtained in Step 3 of Reference Example 51 instead of the compound obtained in Step 2 of Reference Example 18 to give the title compound.
$^1$H-NMR ($CDCl_3$: 60°C) δ: 0.88-0.91 (3H, m), 1.01-1.05 (3H, m), 1.18-1.32 (6H, m), 1.45 (9H, s), 1.81 (3H, s), 2.45 (1H, m), 3.45 (1H, m), 4.02-4.19 (3H, m), 4.30-4.36 (2H, m), 4.92 (1H, s), 6.71 (2H, d, J=7.8 Hz), 7.00-7.11 (6H, m) .

Step 2: (5R,6S)-N-Azetidin-3-yl-5,6-bis(4-chlorophenyl)-N,3-diisopropyl-6-methyl-5,6-dihydroimidazo[2,1-b][1,3]thiazole-2-carboxamide

The compound obtained in Step 1 above was reacted in the same way as in Step 6 of Example 102 to give the title compound.
$^1$H-NMR ($CDCl_3$) δ: 0.88 (3H, d, J=7.1 Hz), 1.04 (3H, d, J=7.1 Hz), 1.17-1.30 (6H, m), 1.80 (3H, s), 2.40 (1H, m), 3.38-3.51 (2H, m), 3.63 (1H, m), 4.16-4.24 (2H, m), 4.92 (1H, s), 6.69-6.71 (2H, m), 6.98-7.11 (6H, m).

Step 3: (5R,6S)-N-(1-Acetylazetidin-3-yl)-5,6-bis(4-chlorophenyl)-N,3-diisopropyl-6-methyl-5,6-dihydroimidazo[2,1-b][1,3]thiazole-2-carboxamide

Acetic anhydride (58 μl, 0.62 mmol) was added to a dichloromethane (3 ml) solution of the compound (305 mg, 0.56 mmol) obtained in Step 2 above and triethylamine (0.12 ml, 0.84 mmol). The resulting mixture was stirred at room temperature for 3 hours, diluted with dichloromethane, washed with 10% aqueous citric acid solution and brine and dried over anhydrous sodium sulfate. The solvent was evaporated under reduced pressure, and the thus obtained residue was purified by silica gel column chromatography (chloroform:methanol = 20:1) to give the title compound (229 mg, 70%) as a colorless solid.
$^1$H-NMR ($CDCl_3$) δ: 0.88 and 0.91 (total 3H, each d, J=7.1 Hz), 1.05 and 1.09 (tota 13H, each d, J=7.1 Hz), 1.22 and 1.25 (total 3H, each d, J=6.8 Hz), 1.29 and 1.31 (total 3H, each d, J=6.8 Hz), 1.81 and 1.83 (total 3H, each s), 1.90 and 1.91 (total 3H, each s), 2.41-2.50 (1H, m), 410-4.32 (4H, m), 4.44-4.50 (1H, m), 4.63-4.70 (1H, m), 4.96 and 4.97 (total 1H, each s), 6.69-6.76 (2H, m), 7.03-7.12 (6H, m).
MS (ESI) m/z: 585, 587.

Example 162

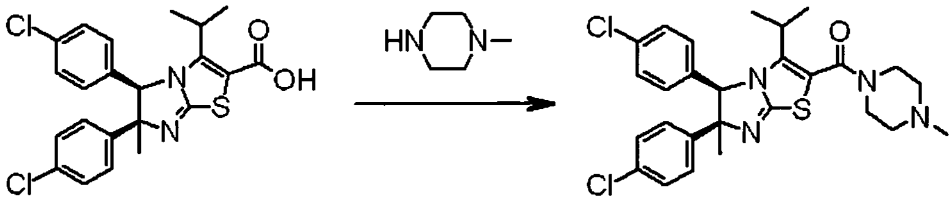

(5R,6S)-5,6-Bis(4-chlorophenyl)-3-isopropyl-6-methyl-2-[(4-methylpiperazin-1-yl)carbonyl]-5,6-dihydroimidazo[2,1-b][1,3]thiazole

The compound obtained in Step 3 of Example 1 was reacted in the same way as in Step 4 of Example 1 using 1-methylpiperazine instead of piperazin-2-one to give the title compound.
$^1$H-NMR ($CDCl_3$) δ: 0.91 (3H, d, J=7.1 Hz), 1.01 (3H, d, J=7.1 Hz), 1.82 (3H, s), 2.32 (3H, s), 2.37-2.53 (5H, m), 3.63 (4H, t, J=4.5 Hz), 4.94 (1H, s), 6.71 (2H, d, J=7.8 Hz), 6.99-7.13 (6H, m).
MS (ESI) m/z: 529, 531.

Example 163

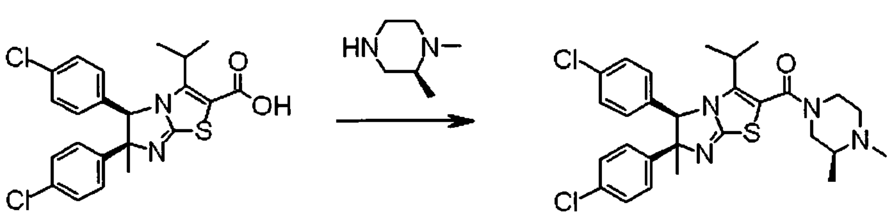

(5R,6S)-5,6-Bis(4-chlorophenyl)-2-{[(3S)-3,4-dimethylpiperazin-1-yl]carbonyl}-3-isopropyl-6-methyl-5,6-dihydroimidazo[2,1-b][1,3]thiazole

The compound obtained in Step 3 of Example 1 was reacted in the same way as in Step 4 of Example 1 using (2S)-1,2-dimethylpiperazine instead of piperazin-2-one to give the title compound.
$^1$H-NMR ($CDCl_3$) δ: 0.91 (3H, d, J=6.8 Hz), 1.01 (3H, d, J=6.8 Hz), 1.11 (3H, d, J=6.1 Hz), 1.82 (3H, s), 2.01-2.11 (1H, m), 2.17 (1H, td, J=11.6, 3.2 Hz), 2.31 (3H, s), 2.41-2.51 (1H, m), 2.73 (1H, dd, J=12.8, 10.4 Hz), 2.82 (1H, dt, J=11.6, 3.2 Hz), 3.18 (1H, t, J=11.6 Hz), 3.97-4.18 (2H, m), 4.94 (1H, s), 6.71 (2H, d, J=7.8 Hz), 7.01-7.11 (6H, m).
MS (ESI) m/z: 543, 545.

Example 164

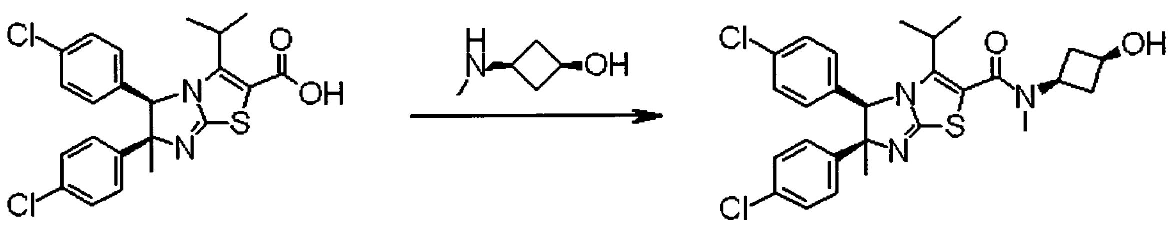

(5R,6S)-5,6-Bis(4-chlorophenyl)-N-(cis-3-hydroxycyclobutyl)-3-isopropyl-N,6-dimethyl-5,6-dihydroimidazo[2,1-b][1,3]thiazole-2-carboxamide

The compound obtained in Step 3 of Example 1 was reacted in the same way as in Step 4 of Example 1 using 3-cis-(methylamino)cyclobutanol instead of piperazin-2-one to give the title compound.
$^1$H-NMR ($CDCl_3$) δ: 0. 87 (3H, d, J=7.0 Hz), 0. 99 (3H, d, J=7.0 Hz), 1.82 (3H, s), 2.10-2.18 (2H, m), 2.41-2.48 (1H, m), 2.60-2.69 (2H, m), 3.02 (3H, s), 4.09-4.13 (1H, m), 4.16-4.23 (1H, m), 4.94 (1H, s), 6.72 (2H, d, J=8.0 Hz), 7.01-7.11 (6H, m).
MS (ESI) m/z: 530, 532.

Example 165

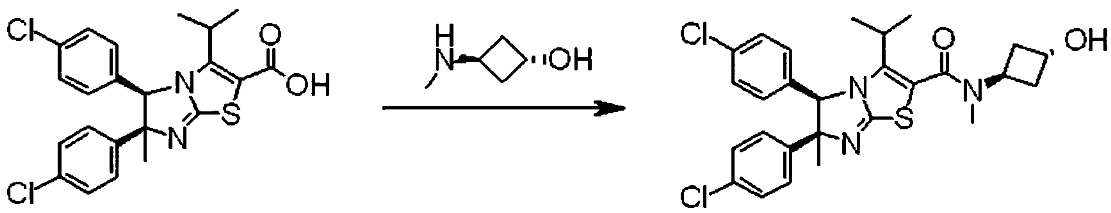

(5R,6S)-5,6-Bis(4-chlorophenyl)-N-(trans-3-hydroxycyclobutyl)-3-isopropyl-N,6-dimethyl-5,6-dihydroimidazo[2,1-b][1,3]thiazole-2-carboxamide

The compound obtained in Step 3 of Example 1 was reacted in the same way as in Step 4 of Example 1 using 3-trans-(methylamino)cyclobutanol instead of piperazin-2-one to give the title compound.
$^1$H-NMR ($CDCl_3$) δ: 0.85 (3H, d, J=7.1 Hz), 1.00 (3H, d, J=7.1 Hz), 1.82 (3H, s), 2.20-2.26 (1H, m), 2.29-2.39 (1H, m), 2.41-2.55 (3H, m), 2.98 (3H, s), 4.47 (1H, t, J=6.2 Hz), 4.95 (1H, s), 5.04-5.10 (1H, m), 6.72 (2H, d, J=8.0 Hz), 7.00-7.12 (6H, m).
MS (ESI) m/z: 530, 532.

Example 166

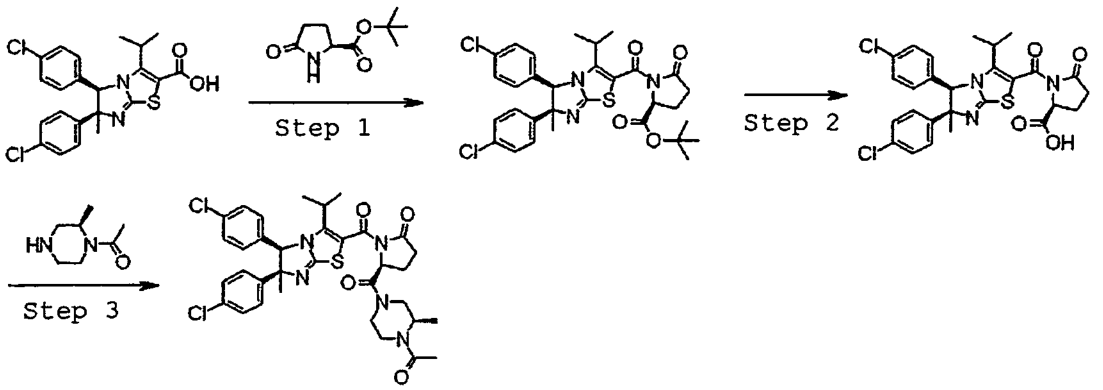


Step 1: tert-Butyl 1-{[(5R,6S)-5,6-Bis(4-chlorophenyl)-3-isopropyl-6-methyl-5,6-dihydroimidazo[2,1-b][1,3]thiazol-2-yl]carbonyl}-5-oxo-L-prolinate

The compound (3.0 g, 6.7 mmol) obtained in Step 3 of Example 1 was suspended in toluene (10 ml), and thionyl chloride (1.0 ml) was added, and the resulting mixture was heated and stirred at 70°C for 1 hour. The reaction

mixture was returned to room temperature and after the reaction solvent was evaporated under reduced pressure, the obtained residue was subjected to azeotropic operation with toluene. Tetrahydrofuran and hexane were added for solidification and the resulting mixture was dried under reduced pressure at 60°C to give an acid chloride (3.5 g) as a pale orange solid.
Separately, tert-butyl 5-oxo-L-prolinate (1.0 g, 5.4 mmol) was suspended in toluene (3 ml) followed by the addition of triethylamine (903 μl, 6.5 mmol). After the resulting mixture was heated to reflux at 100°C for 1 hour, a toluene (1 ml) solution of chlorotrimethylsilane (818 μl, 6.5 mmol) was added dropwise at the same temperature, and the resulting mixture was heated to reflux for 5 hours. The reaction mixture was returned to room temperature and after the deposited matter was filtered off, the filtrate was evaporated under reduced pressure to give N-silyl compound (1.2 g) as a pale orange oil.
The acid chloride (1.17 g, 2.33 mmol) mentioned above was dissolved in tetrahydrofuran (10 ml) and the N-silyl compound prepared separately and triethylamine (974 μl, 6.99 mmol) were added and the resulting mixture was heated to reflux at 70°C for 2 days. The reaction mixture was returned to room temperature and the resulting mixture was diluted with ethyl acetate. The organic layer was washed with brine and dried over anhydrous sodium sulfate. The solvent was evaporated under reduced pressure, and the obtained residue was purified by silica gel column chromatography (hexane: ethyl acetate = 4:1). Ethyl acetate and hexane were added for solidification and the resulting mixture was dried under reduced pressure at 60°C to give the title compound (586 mg, 41%) as a colorless solid.
$^1$H-NMR ($CDCl_3$) δ: 0.93 (3H, d, J=6.9 Hz), 0.97 (3H, d, J=6.9 Hz), 1.48 (9H, s), 1.83 (3H, s), 2.03-2.11 (1H, m), 2.36-2.46 (1H, m), 2.53-2.62 (1H, m), 2.66-2.74 (1H, m), 2.83-2.91 (1H, m), 4.65 (1H, dd, J=8.7, 5.5 Hz), 5.01 (1H, s), 6.73 (2H, d, J=8.7 Hz), 7.02 (2H, d, J=8.3 Hz), 7.05 (2H, d, J=8.7 Hz), 7.10 (2H, d, J=8.3 Hz).

Step 2: 1-{[(5R,6S)-5,6-Bis(4-chlorophenyl)-3-isopropyl-6-methyl-5,6-dihydroimidazo[2,1-b][1,3]thiazol-2-yl]carbonyl}-5-oxo-L-proline

The compound obtained in Step 1 above was reacted in the same way as in Step 2 of Example 61 to give the title compound.
$^1$H-NMR (DMSO-$d_6$) δ: 0.81 (3H, d, J=7.1 Hz), 0.89 (3H, d, J=7.1 Hz), 1.89 (3H, s), 1.95-2.03 (1H, m), 2.34-2.44 (1H, m), 2.60 (2H, t, J=8.2 Hz), 2.79-2.86 (1H, m), 4.66 (1H, dd, J=9.0, 4.4 Hz), 5.99 (1H, s), 7.17-7.25 (8H, m).
MS (ESI) m/z: 558.

Step 3: (5S)-5-{[(3R)-4-Acetyl-3-methylpiperazin-1-yl]carbonyl}-1-{[(5R,6S)-5,6-bis(4-chlorophenyl)-3-isopropyl-6-methyl-5,6-dihydroimidazo[2,1-b][1,3]thiazol-2-yl]carbonyl}pyrrolidin-2-one

The compound obtained in Step 2 above was reacted in the same way as in Step 4 of Example 1 using (2R)-2-methyl-1-acetylpiperazine hydrochloride instead of piperazin-2-one to give the title compound.
$^1$H-NMR ($CDCl_3$) δ: 0.89-0.95 (6H, m), 1.19-1.23 (3H, m), 1.83 (3H, s), 2.00-2.05 (1H, m), 2.10 (3H, s), 2.28-2.34 (1H, m), 2.53-2.61 (1H, m), 2.76-2.87 (2H, m), 2.91-2.97 (1H, m), 3.19-3.26 (1H, m), 3.44-3.50 (1H, m), 3.61-3.66 (1H, m), 3.90-3.95 (1H, m), 4.36-4.45 (2H, m), 5.01 (1H, s), 5.12-5.16 (1H, m), 6.72 (2H, d, J=8.5 Hz), 7.01 (2H, d, J=8.5 Hz), 7.05 (2H, d, J=8.5 Hz), 7.10 (2H, d, J=8.5 Hz).
MS (ESI) m/z: 682.

Example 167

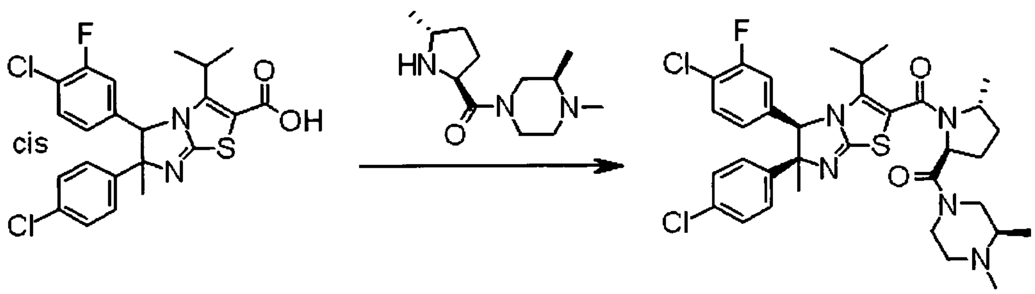

(5R,6S)-5-(4-Chloro-3-fluorophenyl)-6-(4-chlorophenyl)-2-{[(2S,5R)-2-{[(3R)-3,4-dimethylpiperazin-1-yl]carbonyl}-5-methylpyrrolidin-1-yl]carbonyl}-3-isopropyl-6-methyl-5,6-dihydroimidazo[2,1-b][1,3]thiazole

The compound obtained in Step 6 of Reference Example 50 was reacted in the same way as in Step 4 of Example 97 using the compound obtained in Step 3 of Reference Example 31 instead of 4-(L-prolyl)morpholine to give the title compound.
$^1$H-NMR ($CDCl_3$) δ: 0.92-1.12 (6H, m), 1.17-1.27 (6H, m), 1.58-2.02 (5H, m), 2.09-2.42 (5H, m), 2.48-2.94 (3H, m), 3.37-3.77 (4H, m), 4.19-4.44 (1H, m), 4.50-4.61 (1H, m), 4.87-5.06 (2H, m), 6.43-6.57 (2H, m), 7.00-7.18 (5H, m). MS (FAB) m/z: 672.

Example 168

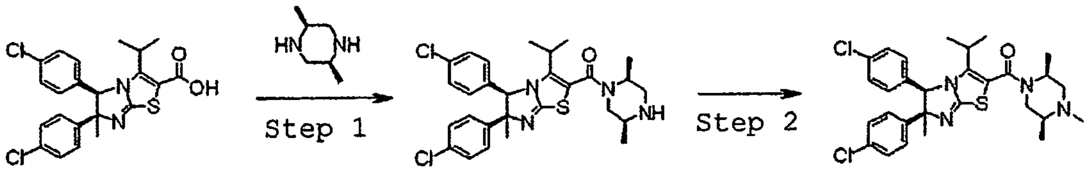


Step 1: (5R,6S)-5,6-Bis(4-chlorophenyl)-2-{[(2S, 5S)-2,5-dimethylpiperazin-1-yl]carbonyl}-3-isopropyl-6-methyl-5,6-dihydroimidazo[2,1-b][1,3]thiazole

The compound obtained in Step 3 of Example 1 was reacted in the same way as in Step 4 of Example 1 using (2S,5S)-2,5-dimethylpiperazine instead of piperazin-2-one to give the title compound.
$^1$H-NMR ($CDCl_3$) δ: 0.91 (3H, d, J=6.8 Hz), 1.06 (3H, d, J=6.8 Hz), 1.13 (3H, d, J=6.1 Hz), 1.34 (3H, d, J=6.1 Hz), 1.82 (3H, s), 2.33-2.48 (1H, m), 2.63-2.79 (2H, m), 2.86 (1H, dd, J=12.2, 1.3 Hz), 2.95 (1H, dd, J=12.2, 4.2 Hz), 3.82-4.07 (1H, m), 4.33-4.53 (1H, m), 4.94 (1H, s), 6.71 (2H, d, J=7.8 Hz), 7.00-7.12 (6H, m).

Step 2: (5R,6S)-5,6-Bis(4-chlorophenyl)-3-isopropyl-6-methyl-2-{[(2S,5S)-2,4,5-trimethylpiperazin-1-yl]carbonyl}-5,6-dihydroimidazo[2,1-b][1,3]thiazole

1 N hydrochloric acid/ethanol solution (0.5 ml, 0.5 mmol), 1 M sodium cyanoborohydride/tetrahydrofuran solution (0.5 ml, 0.5 mmol) and 35% aqueous formaldehyde solution (4.2 μl, 0.55 mmol) were sequentially added to an ethanol (4 ml) solution of the compound (150 mg, 0.28 mmol) obtained in Step 1 above under ice cooling and the resulting mixture was stirred for 18 hours while warming to the room temperature slowly. The reaction mixture was diluted with ethyl acetate and washed with saturated aqueous sodium bicarbonate solution and brine and the organic layer was dried over anhydrous magnesium sulfate. The solvent was evaporated under reduced pressure, the obtained residue was separated and purified by thin layer silica gel column chromatography (chloroform:methanol = 97:3) and the desired fraction was concentrated. Hexane was added to the obtained residue and the resulting precipitate was collected by filtration to give the title compound (77 mg, 50%) as a pale yellow solid.
$^1$H-NMR ($CDCl_3$) δ: 0.89 (3H, d, J=7.1 Hz), 1.03 (3H, d, J=7.1 Hz), 1.10 (3H, d, J=6.6 Hz), 1.35 (3H, d, J=6.6 Hz), 1.79 (3H, s), 1.86-1.99 (1H, m), 2.13-2.31 (1H, m), 2.22 (3H, s), 2.34-2.41 (1H, m), 2.65 (1H, d, J=11.7 Hz), 2.74-2.93 (1H, m), 3.68-4.03 (1H, m), 4.32-4.54 (1H, m), 4.91 (1H, s), 6.68 (2H, d, J=8.1 Hz), 6.98-7.05 (4H, m), 7.09 (2H, d, J=8.1 Hz). MS (ESI) m/z: 557, 559.

Example 169

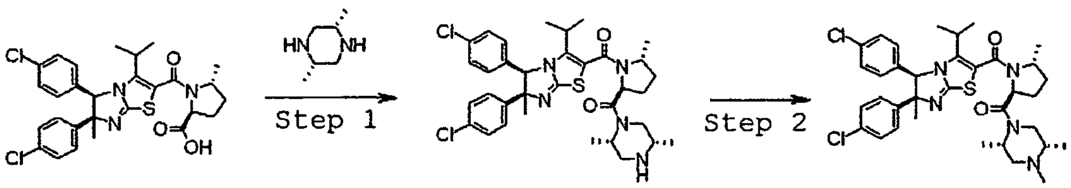


Step 1: (5R,6S)-5,6-Bis(4-chlorophenyl)-2-{[(2S,5R)-2,5-dimethylpiperazin-1-yl]-carbonyl}-5-methylpyrrolidin-1-yl]carbonyl}-3-isopropyl-6-methyl-5,6-dihydroimidazo[2,1-b][1,3]thiazole

The compound obtained in Step 2 of Example 126 was reacted in the same way as in Step 4 of Example 1 using (2S,5S)-2,5-dimethylpiperazine instead of piperazin-2-one to give the title compound.
MS (ESI) m/z: 654, 656.

Step 2: (5R,6S)-5,6-Bis(4-chlorophenyl)-3-isopropyl-6-methyl-5-{[(2R,5S)-2-methyl-2-{[(2S,5S)-2,4,5-trimethylpiperazin-1-yl]carbonyl}pyrrolidin-1-yl]carbonyl}-5,6-dihydroimidazo[2,1-b][1,3]thiazole

The compound obtained in Step 1 above was reacted in the same way as in Step 2 of Example 168 to give the title compound.
$^1$H-NMR (CDCl3, 70°C) δ: 0.72-2.86 (8H, m), 0.94 (3H, d, J=7.1 Hz), 1.03 (3H, d, J=7.1 Hz), 1.06-1: 14 (3H, m), 1.23 (3H, d, J=6.3 Hz), 1.25-1.34 (3H, m), 1.79 (3H, s), 2.23 (3H, s), 2.99-3.59 (1H, m), 4.15-4.69 (2H, m), 4.30 (1H, s), 4.82-5.08 (1H, m), 4.92 (1H, s), 6.68 (2H, d, J=8.3 Hz), 7.00 (4H, d, J=8.3 Hz), 7.10 (2H, d, J=8.3 Hz).
MS (ESI) m/z: 668, 670.

Example 170

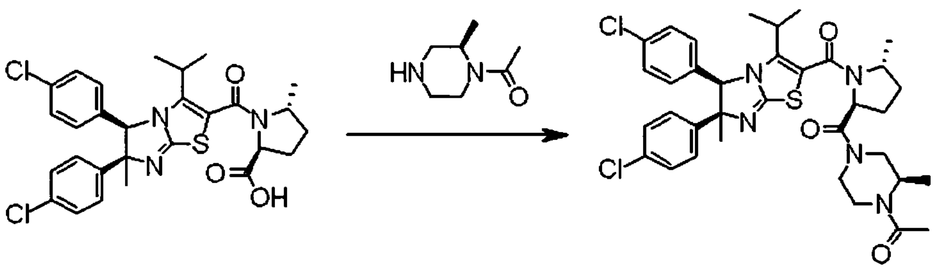

(5R,6S)-2-{[(2S,5R)-2-{[(3R)-4-Acetyl-3-methylpiperazin-1-yl]carbonyl}-5-methylpyrrolidin-1-yl]carbonyl}-5,6-bis (4-chlorophenyl)-3-isopropyl-6-methyl-5,6-dihydroimidazo[2,1-b][1,3]thiazole

The compound obtained in Step 2 of Example 126 was reacted in the same way as in Step 4 of Example 1 using (2R)-2-methyl-1-acetylpiperazine hydrochloride instead of piperazin-2-one to give the title compound.
$^1$H-NMR ($CDCl_3$) δ: 0.94 (3H, d, J-7.1 Hz), 1. 02 (3H, d, J=7.1 Hz), 1.19-1.21 (3H, m), 1.24 (3H, d, J=6.3 Hz), 1.65-1.71 (1H, m), 1.81 (3H, s), 1.83-1.87 (1H, m), 2.09 (3H, s), 2.20-2.25 (1H, m), 2.41-2.47 (1H, m), 2.69-2.76 (1H, m), 2.89-2.94 (1H, m), 3.14-3.20 (1H, m), 3.44-3.50 (1H, m), 3.69-3.75 (1H, m), 3.97-4.03 (1H, m), 4.31-4.37 (1H, m), 4.49-4.55 (1H, m), 4.84-4.88 (1H, m), 4.97 (1H, s), 5.00-5.02 (1H, m), 6.69 (2H, d, J=8.3 Hz), 7.01 (2H, d, J=8.5 Hz), 7.02 (2H, d, J=8.5 Hz), 7.12 (2H, d, J=8.5 Hz).
MS (ESI) m/z: 682.

Example 171

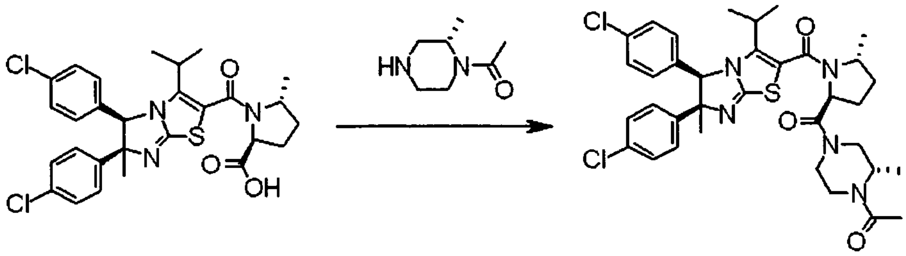

(5R,6S)-2-{[(2S,5R)-2-{[(3S)-4-Acetyl-3-methylpiperazin-1-yl]carbonyl}-5-methylpyrrolidin-1-yl]carbonyl}-5,6-bis (4-chlorophenyl)-3-isopropyl-6-methyl-5,6-dihydroimidazo[2,1-b][1,3]thiazole

The compound obtained in Step 2 of Example 126 was reacted in the same way as in Step 4 of Example 1 using (2S)-2-methyl-1-acetylpiperazine hydrochloride instead of piperazin-2-one to give the title compound.
$^1$H-NMR ($CDCl_3$) δ: 0.96 (3H, d, J=7.1 Hz), 1.02 (3H, d, J=7.1 Hz), 1.17-1.20 (3H, m), 1.24 (3H, d, J=6.3 Hz), 1.67-1.71 (1H, m), 1.80 (3H, s), 1.84-1.89 (1H, m), 2.10 (3H, s), 2.13-2.16 (1H, m), 2.28-2.32 (1H, m), 2.70-2.75 (1H, m), 2.90-2.93 (1H, m), 3.30-3.34 (1H, m), 3.65-3.69 (1H, m), 3.85-3.89 (1H, m), 4.05-4.09 (1H, m), 4.35-4.40 (1H, m), 4.53-4.57 (1H, m), 4.84-4.87 (1H, m), 4.95 (1H, s), 4.98-5.01 (1H, m), 6.69 (2H, d, J=8.3 Hz), 7.01 (2H, d, J=8.5 Hz), 7.02 (2H, d, J=8.5 Hz), 7.12 (2H, d, J=8.3 Hz).
MS (ESI) m/z: 682.

Example 172

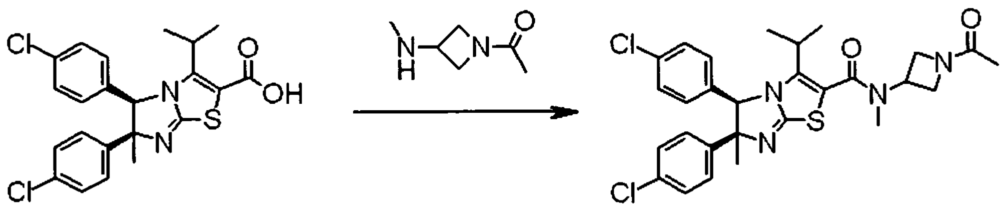

(5R,6S)-N-(1-Acetylazetidin-3-yl)-5,6-bis(4-chlorophenyl)-3-isopropyl-N,6-dimethyl-5,6-dihydroimidazo[2,1-b][1,3]thiazole-2-carboxamide

The compound obtained in Step 3 of Example 1 was reacted in the same way as in Step 4 of Example 1 using 1-acetyl-N-methylazetidin-3-amine instead of piperazin-2-one to give the title compound.
$^1$H-NMR ($CDCl_3$) δ: 0.86 (3H, d, J=6.8 Hz), 0.96 (3H, d, J=6.8 Hz), 1.81 (3H, s), 1.90 (3H, s), 2.50 (1H, m), 3.11 (3H, s), 4.06 (1H, dd, J=10.1, 5.9 Hz), 4.15 (1H, m), 4.24 (1H, m), 4.39 (1H, dt, J=16.4, 7.3 Hz), 4.95 (1H, s), 4.97 (1H, m), 6.69-6.71 (2H, m), 7.00-7.09 (6H, m).
MS (ESI) m/z: 557, 559.

Example 173

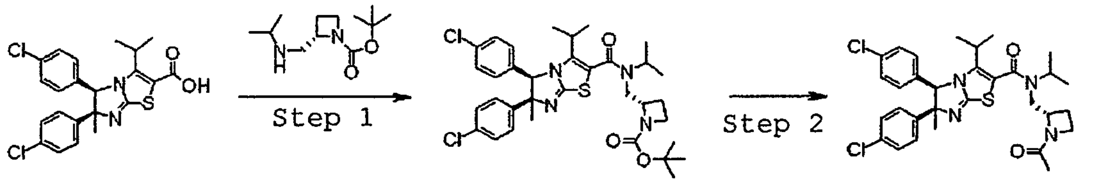


Step 1: tert-Butyl (2S)-2-{[{[(5R,6S)-5,6-Bis(4-chlorophenyl)-3-isopropyl-6-methyl-5,6-dihydroimidazo[2,1-b][1,3]thiazol-2-yl]carbonyl} (isopropyl)amino]methyl}azetidine-1-carboxylate

The compound obtained in Step 3 of Example 1 was reacted in the same way as in Example 112 using the compound obtained in Reference Example 52 instead of the compound obtained in Step 2 of Reference Example 18 to give the title compound.
$^{1}$H-NMR ($CDCl_3$) δ: 0.88 (3H, d, J=7.1 Hz), 0.97 (3H, d, J=7.1 Hz), 1.24 (3H, d, J=7.1 Hz), 1.26 (3H, d, J=7.1 Hz), 1.45 (9H, s), 1.81 (3H, s), 2.21-2.23 (2H, m), 2.42 (1H, m), 3.50 (1H, m), 3.72-3.78 (3H, m), 4.33-4.36 (2H, m), 4.93 (1H, s), 6.69-6.71 (2H, m), 6.98-7.03 (4H, m), 7.07-7.10 (2H, m).

Step 2: (5R,6S)-N-{[(2S)-1-Acetylazetidin-2-yl]methyl}-5,6-bis(4-chlorophenyl)-N,3-diisopropyl-6-methyl-5,6-dihydroimidazo[2,1-b][1,3]thiazole-2-carboxamide

Trifluoroacetic acid (4 ml) was added dropwise to a dichloromethane (8 ml) solution of the compound (487 mg, 0.74 mmol) obtained in Step 1 above under ice cooling, and the resulting mixture was stirred at room temperature for 1 hour. The reaction mixture was concentrated under reduced pressure and the residue was neutralized with 1 N aqueous sodium hydroxide solution and then extracted with chloroform. After the organic layer was dried over anhydrous sodium sulfate, the solvent was evaporated under reduced pressure. The obtained residue was dissolved in dichloromethane (5 ml) and acetic anhydride (48 μl, 0.51 mmol) was added dropwise at room temperature. After the resulting mixture was stirred for 2 hours, the reaction mixture was diluted with dichloromethane, washed with 10% aqueous citric acid solution and brine and dried over anhydrous sodium sulfate. The reaction solvent was evaporated under reduced pressure and the thus obtained residue was purified by silica gel thin layer chromatography (chloroform:methanol = 10:1) to give the title compound (113 mg, 26%) as a colorless solid.
$^{1}$H-NMR ($CDCl_3$) δ: 0.88 (3H, d, J=7.1 Hz), 0.98 (3H, d, J=7.1 Hz), 1.24 (3H, d, J=6.8 Hz), 1.28 (3H, d, J=6.8 Hz), 1.81 (3H, s), 1.83 (3H, s), 2.36-2.39 (2H, m), 3.69-3.72 (2H, m), 3.95 (1H, m), 4.01 (1H, m), 4.33-4.39 (2H, m), 4.93 (1H, s), 6.68-6.70 (2H, m), 7.00-7.04 (4H, m), 7.06-7.09 (2H, m).
MS (ESI) m/z: 599, 560.

Example 174

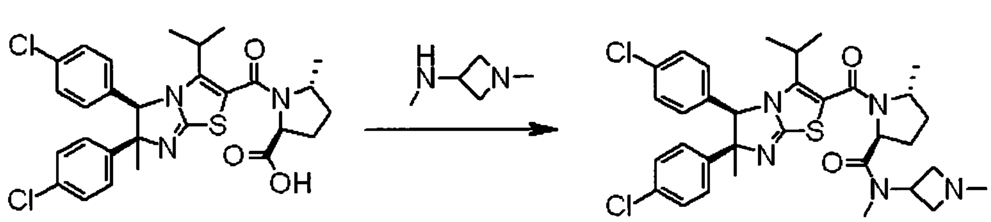

(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-N,5-dimethyl-N-(1-methylazetidin-3-yl)-L-prolinamide

The compound obtained in Step 2 of Example 126 was reacted in the same way as in Step 4 of Example 1 using N,1-dimethylazetidin-3-amine instead of piperazin-2-one to give the title compound.
$^{1}$H-NMR (CDCl3, 60°C) δ: 0.94 (3H, d, J=7.1 Hz), 1.02 (3H, d, J=7.1 Hz), 1.23 (3H, d, J=6.3 Hz), 1.79 (3H, s), 2.25 (1H, m), 2.34 (3H, s), 2.39 (3H, s), 2.72 (1H, m), 3.01-3.35 (5H, m), 3.57-3.71 (2H, m), 4.53 (1H, m), 4.67 (1H, m), 4.91 (1H, m), 4.92 (1H, s), 6.68 (2H, d, J=8.0 Hz), 7.00 (5H, dd, J=8.5, 8.0 Hz), 7.11 (2H, d, J=8.5 Hz).

MS (ESI) m/z: 640, 642.

Example 175

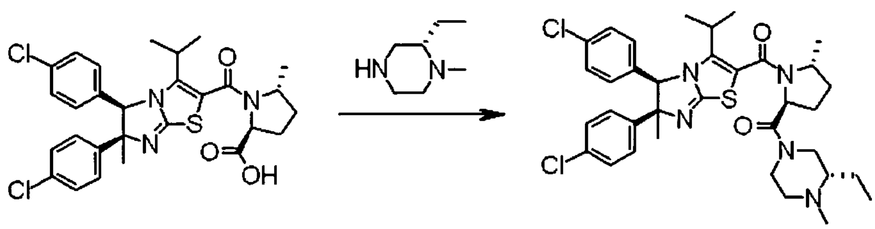

(5R,6S)-5,6-Bis(4-chlorophenyl)-2-{[(2S,5R)-2-{[(3S)-3-ethyl-4-methylpiperazin-1-yl]carbonyl}-5-methylpyrrolidin-1-yl] carbonyl]-3-isopropyl-6-methyl-5,6-dihydroimidazo[2,1-b][1,3]thiazole

The compound obtained in Step 2 of Example 126 was reacted in the same way as in Step 4 of Example 1 using the compound obtained in Step 2 of Reference Example 53 instead of piperazin-2-one to give the title compound. $^1$H-NMR ($CDCl_3$) δ: 0.92-0.98 (6H, m), 1.03 (3H, d, J=7.1 Hz), 1.23 (3H, d, J=6.3 Hz), 1.60-1.71 (2H, m), 1.79 (3H, s), 1.81-1.86 (1H, m), 2.09-2.16 (1H, m), 2.30 (3H, s), 2.31-2.38 (2H, m), 2.69-2.85 (3H, m), 3.04-3.13 (1H, m), 3.36-3.44 (1H, m), 3.68-3.81 (2H, m), 4.25-4.32 (1H, m), 4.52-4.57 (1H, m), 4.93 (1H, s), 4.99-5.03 (1H, m), 6.69 (2H, d, J=6.8 Hz), 7.01 (4H, d, J=8.5 Hz), 7.12 (2H, d, J=8.5 Hz).

Example 176

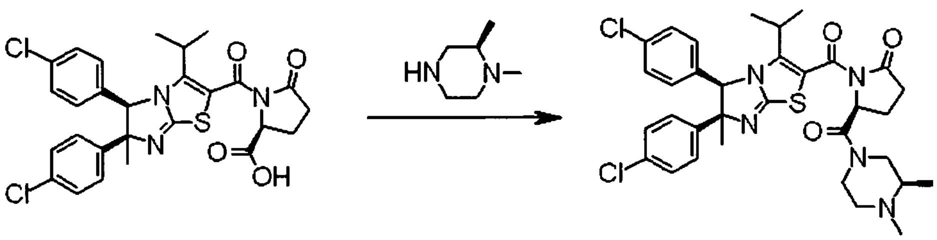

(5S)-1-{[(5R,6S)-5,6-Bis(4-chlorophenyl)-3-isopropyl-6-methyl-5,6-dihydroimidazo[2,1-b][1,3]thiazol-2-yl]carbonyl}-5-{[ (3R)-3,4-dimethylpiperazin-1-yl]carbonyl}pyrrolidin-2-one

The compound obtained in Step 2 of Example 166 was reacted in the same way as in Step 4 of Example 1 using (2R)-1,2-dimethylpiperazine instead of piperazin-2-one to give the title compound.
$^1$H-NMR ($CDCl_3$) δ: 0.94 (6H, d, J=7.3 Hz), 1.05-1.11 (3H, m), 1.48-1.55 (1H, m), 1.81 (3H, s), 1.97-2.05 (1H, m), 2.16-2.23 (1H, m), 2.30 (3H, s), 2.31-2.36 (1H, m), 2.51-2.59 (1H, m), 2.72-2.79 (1H, m), 2.83-2.89 (1H, m), 2.93-2.98 (1H, m), 3.43-3.49 (1H, m), 3.60-3.66 (1H, m), 4.20-4.25 (1H, m), 4.36-4.42 (1H, m), 4.98 (1H, s), 5.11-5.17 (1H, m), 6.73 (2H, d, J=8.5 Hz), 7.00 (2H, d, J=8.5 Hz), 7.04 (2H, d, J=8.3 Hz), 7.10 (2H, d, J=8.5 Hz).

Example 177

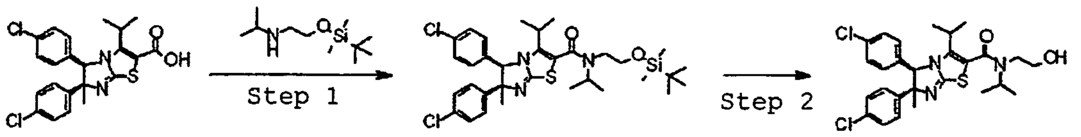


Step 1: (5R,6S)}-N-(2-{[tert-Butyl(dimethyl)silyl]oxy}ethyl)-5,6-bis(4-chlorophenyl)-N,3-diisopropyl-6-methyl-5,6-dihydroimidazo[2,1-b][1,3]thiazole-2-carboxamide

The compound obtained in Step 3 of Example 1 was reacted in the same way as in Example 112 using N-(2-{[tert-butyl(dimethyl)silyl]oxy}ethyl)propan-2-amine instead of the compound obtained in Step 2 of Reference Example 18 to give the title compound.
$^1$H-NMR ($CDCl_3$) δ: 0.08 (6H, s), 0.91 (9H, s), 0.92 (3H, d, J=7.1 Hz), 1.03 (3H, d, J=7.1 Hz), 1.21 (3H, d, J=6.6 Hz), 1.27 (3H, d, J=6.6 Hz), 1.83 (3H, s), 2.41 (1H, m), 3.36-3.42 (2H, m), 3.76-3.79 (2H, m), 4.36 (1H, m), 4.95 (1H, s), 6.72 (2H, d, J=8.1 Hz), 7.02-7.06 (4H, m), 7.11 (2H, d, J=8.5 Hz).

Step 2: (5R,6S)-5,6-Bis(4-chlorophenyl)-N-(2-hydroxyethyl)-N,3-diisopropyl-6-methyl-5,6-dihydroimidazo[2,1-b][1,3]thiazole-2-carboxamide

1 M tetrabutylammonium fluoride/tetrahydrofuran solution (0.29 ml, 0.290 mmol) was added dropwise to a tetrahydrofuran (3 ml) solution of the compound (170 mg, 0.262 mmol) obtained in Step 1 above. After the resulting mixture was stirred at room temperature for 1 hour, water was added and the resulting mixture was extracted with ethyl acetate, washed with 10% aqueous citric acid solution and brine and dried over anhydrous sodium sulfate. After the solvent was evaporated under reduced pressure, the residue was purified by silica gel column chromatography (chloroform: methanol = 10:1) to give the title compound (120 mg, 86%) as a colorless solid.
$^1$H-NMR ($CDCl_3$) δ: 0.91 (3H, d, J=7.1 Hz), 1.05 (3H, d, J=7.1 Hz), 1.21 (3H, d, J=6.6 Hz), 1.28 (3H, d, J=6.6 Hz), 1.83 (3H, s), 2.41 (1H, m), 3.49-3.50 (2H, m), 3.70-3.81 (3H, m), 4.41 (1H, m), 4.95 (1H, s), 6.72 (2H, d, J=8.5 Hz), 7.03-7.05 (4H, m), 7.10 (2H, d, J=8.8 Hz).

Example 178

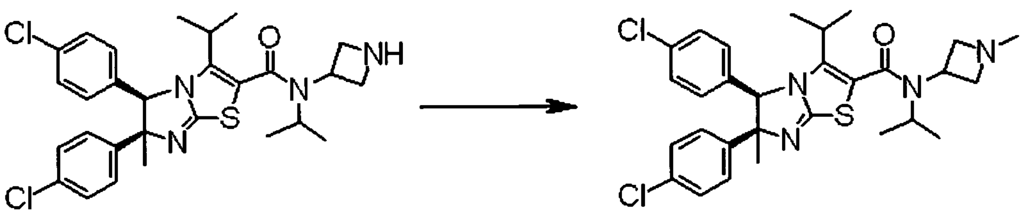

(5R,6S)-5,6-Bis(4-chlorophenyl)-N,3-diisopropyl-6-methyl-N-(1-methylazetidin-3-yl)-5,6-dihydroimidazo[2,1-b]thiazole-2-carboxamide

The compound obtained in Step 2 of Example 161 was reacted in the same way as in Step 3 of Example 152 to give the title compound.
$^1$H-NMR ($CDCl_3$) δ: 0.90 (3H, d, J=7.1 Hz), 1.03 (3H, d, J=7.1 Hz), 1.31 (3H, d, J=6.6 Hz), 1.35 (3H, d, J=6.6 Hz), 1.83 (3H, s), 2.42 (3H, s), 2.47 (1H, m), 3.36-3.39 (2H, m), 3.66-3.70 (2H, m), 4.17 (1H, m), 4.30 (1H, m), 4.95 (1H, s), 6.73 (2H, d, J=8.1 Hz), 7.04-7.06 (4H, m), 7.11 (2H, d, J=8.5 Hz).
MS (ESI) m/z: 557, 559.

Example 179

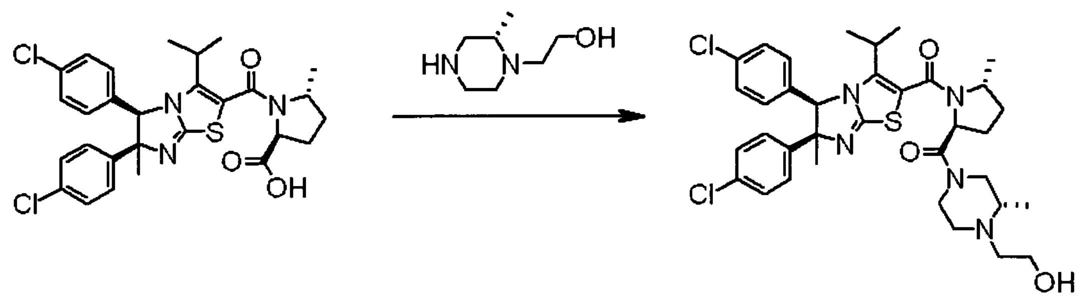

2-{(2S)-4-[(5R)-1-{[(5R,6S)-5,6-Bis(4-chlorophenyl)-3-isopropyl-6-methyl-5,6-dihydroimidazo[2,1-b][1,3]thiazol-2-yl] carbonyl}-5-methyl-L-prolyl]-2-methylpiperazin-1-yl}ethanol

The compound obtained in Step 2 of Example 126 was reacted in the same way as in Step 4 of Example 1 using the compound obtained in Step 2 of Reference Example 54 instead of piperazin-2-one to give the title compound.
$^1$H-NMR ($CDCl_3$) δ: 0.80-4.11 (20H, m), 0.94 (3H, d, J=7.3 Hz), 1.03 (3H, d, J=7.1 Hz), 1.23 (3H, d, J=6.3 Hz), 1.79 (3H, s), 4.48-4.59 (1H, m), 4.92 (1H, s), 5.00 (1H, d, J=8.3 Hz), 6.68 (2H, d, J=8.5 Hz), 7.00 (4H, d, J=8.5 Hz), 7.11 (2H, d, J=8.5 Hz).
MS (ESI) m/z: 684.686.

Example 180

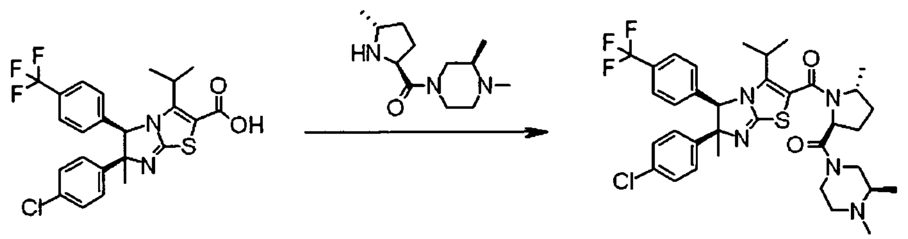

(5R,6S)-6-(4-Chlorophenyl)-2-{[(2S,5R)-2-{[(3R)-3,4-dimethylpiperazin-1-yl]carbonyl}-5-methylpyrrolidin-1-yl]carbonyl}-3-isopropyl-6-methyl-5-[4-(trifluoromethyl)phenyl]-5,6-dihydroimidazo[2,1-b][1,3]thiazole

The compound obtained in Step 10 of Reference Example 55 was reacted in the same way as in Example 112 using the compound obtained in Step 3 of Reference Example 31 instead of the compound obtained in Step 2 of Reference Example 18 to give the title compound.
$^1$H-NMR ($CDCl_3$) δ: 0.85-1: 13 (9H, m), 1.22-1.29 (3H, m), 1.62-2.04 (6H, m), 2.08-2.44 (5H, m), 2.52-2.97 (3H, m), 3.39-3.49 (1H, m), 3.62-3.81 (1H, m), 4.20-4.33 (1H, m), 4.34-4.47 (1H, m), 4.52-4.61 (1H, m), 4.96-5.07 (1H, m), 5.01 (1H, s), 6.82-6.89 (2H, m), 6.99 (2H, d, J=8.5 Hz), 7.06-7.14 (2H, m), 7.26-7.33 (2H, m).
MS (FAB) m/z: 688.

Example 181

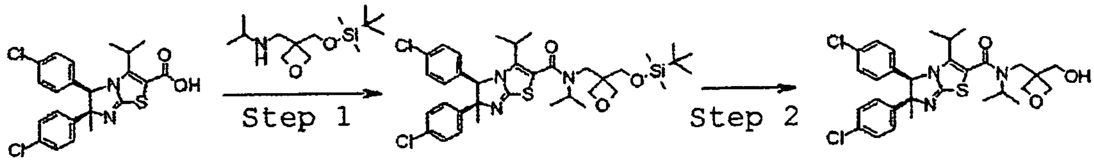


Step 1: (5R,6S)-N-{[3-({[tert-Butyl(dimethyl)silyl]oxy}methyl)oxetan-3-yl]methyl}-5,6-bis(4-chlorophenyl)-N,3-diisopropyl-6-methyl-5,6-dihydroimidazo[2,1-b][1,3]thiazole-2-carboxamide

The compound obtained in Step 3 of Example 1 was reacted in the same way as in Example 112 using the compound obtained in Step 2 of Reference Example 56 instead of the compound obtained in Step 2 of Reference Example 18 to give the title compound.
$^1$H-NMR ($CDCl_3$) δ: 0.11 (6H, s), 0.92 (9H, s), 0.93 (3H, d, J=6.4 Hz), 0.96 (3H, d, J=6.4 Hz), 1.18 (3H, d, J=6.6 Hz), 1.25 (3H, d, J=6.6 Hz), 1.82 (3H, s), 2.50-2.57 (1H, m), 3.26 (1H, d, J=14.0 Hz), 3.38 (1H, d, J=14.0 Hz), 3.82 (2H, AB type d, J=10.0 Hz), 4.27 (2H, dd, J=8.5, 6.4 Hz), 4.35-4.42 (1H, m), 4.58 (1H, d, J=6.4 Hz), 4.66 (1H, d, J=6.4 Hz), 4.96 (1H, s), 6.70 (2H, d, J=7.9 Hz), 7.01-7.11 (6H, m).

Step 2: (5R,6S)-5,6-Bis(4-chlorophenyl)-N-{[3-(hydroxymethyl)oxetan-3-yl]methyl}-N,3-diisopropyl-6-methyl-5,6-dihydroimidazo[2,1-b][1,3]thiazole-2-carboxamide

The compound obtained in Step 1 above was reacted in the same way as in Step 2 of Example 177 to give the title compound.
$^1$H-NMR ($CDCl_3$) δ: 0.91 (3H, d, J=6.9 Hz), 0.99 (3H, d, J=6.9 Hz), 1.19 (3H, d, J=6.6 Hz), 1.28 (3H, d, J=6.6 Hz), 1.82 (3H, s), 2.46-2.53 (1H, m), 3.58 (2H, AB type d, J=14.4 Hz), 3.77-3.82 (3H, m), 4.37 (2H, d, J=6.3 Hz), 4.51 (2H, t, J=6.1 Hz), 4.96 (1H, s), 6.70 (2H, d, J=7.8 Hz), 7.01-7.10 (6H, m).
MS (ESI) m/z: 588, 590.

Example 182

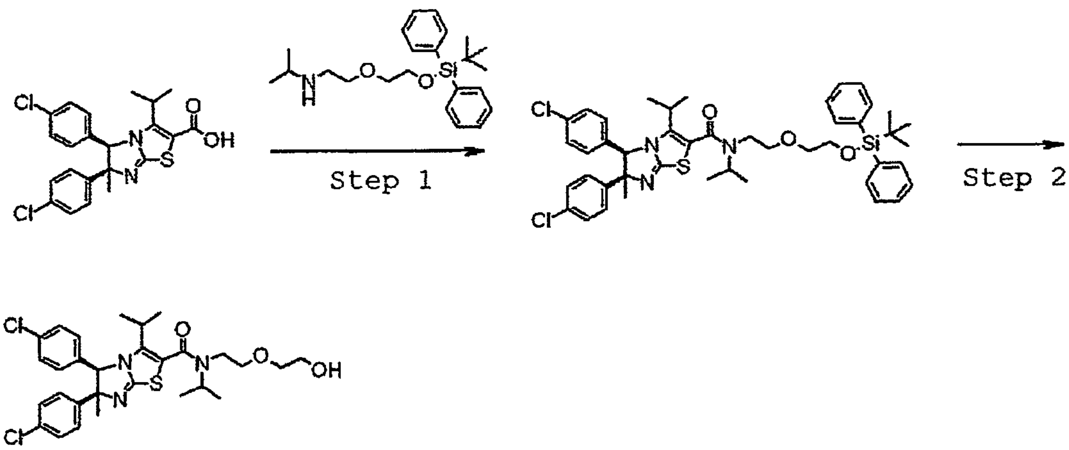


Step 1: (5R,6S)-N-[2-(2-{[tert-Butyl(diphenyl)silyl]oxy}ethoxy)ethyl]-5,6-bis(4-chlorophenyl)-N,3-diisopropyl-6-methyl-5,6-dihydroimidazo[2,1-b][1,3]thiazole-2-carboxamide

The compound obtained in Step 3 of Example 1 was reacted in the same way as in Example 112 using the compound obtained in Step 3 of Reference Example 57 instead of the compound obtained in Step 2 of Reference Example 18 to give the title compound.

$^1$H-NMR ($CDCl_3$) δ: 0.88 (3H, d, J=7.0 Hz), 1.01 (3H, d, J=7.0 Hz), 1.05 (9H, s), 1.18 (3H, d, J=6.6 Hz), 1.24 (3H, d, J=6.6 Hz), 1.82 (3H, s), 2.32-2.43 (1H, m), 3.39-3.47 (2H, m), 3.54-3.58 (2H, m), 3.61-3.65 (2H, m), 3.78 (2H, t, J=5.1 Hz), 4.31-4.37 (1H, m), 4.93 (1H, s), 6.72 (2H, d, J=7.5 Hz), 7.01-7.69 (16H, m).

Step 2: (5R,6S)-5,6-Bis(4-chlorophenyl)-N-[2-(2-hydroxyethoxy)ethyl]-N,3-diisopropyl-6-methyl-5,6-dihydroimidazo [2,1-b][1,3]thiazole-2-carboxamide

The compound obtained in Step 1 above was reacted in the same way as in Step 2 of Example 177 to give the title compound.
$^1$H-NMR ($CDCl_3$) δ: 0.90 (3H, d, J=6.8 Hz), 1.03 (3H, d, J=6.8 Hz), 1.20 (3H, d, J=6.6 Hz), 1.27 (3H, d, J=6.6 Hz), 1.82 (3H, s), 1.99 (1H, br), 2.36-2.43 (1H, m), 3.43-3.51 (2H, m), 3.57 (2H, t, J=4.5 Hz), 3.64-3.67 (2H, m), 3.71-3.73 (2H, m), 4.35-4.41 (1H, m), 4.94 (1H, s), 6.71 (2H, d, J=7.3 Hz), 7.01-7.11 (6H, m).
MS (ESI) m/z: 576, 578.

Example 183

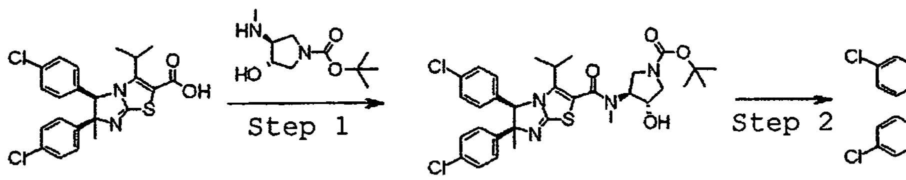


Step 1: tert-Butyl (3S,4S)-3-[{[(5R,6S)-5,6-Bis(4-chlorophenyl)-3-isopropyl-6-methyl-5,6-dihydroimidazo[2,1-b][1,3]thiazol-2-yl]carbonyl}(methyl)amino]-4-hydroxypyrrolidine-1-carboxylate

The compound obtained in Step 3 of Example 1 was reacted in the same way as in Step 4 of Example 1 using tert-butyl (3S,4S)-3-hydroxy-4-(methylamino)pyrrolidine-1-carboxylate instead of piperazin-2-one to give the title compound.
$^1$H-NMR ($CDCl_3$) δ: 0.90 (3H, d, J=6.6 Hz), 0.99 (3H, d, J=6.6 Hz), 1.26 (1H, t, J=7.0 Hz), 1.48 (9H, s), 1.83 (3H, s), 2.46-2.53 (1H, m), 3.02 (3H, s), 3.22 (1H, dd, J=11.2, 6.6 Hz), 3.25 (1H, br), 3.72-3.77 (2H, m), 4.37 (1H, q, J=6.6 Hz), 4.63 (1H, br), 4.97 (1H, s), 6.70 (2H, d, J=7.6 Hz), 7.02-7.11 (6H, m).
MS (ESI) m/z: 645, 647.

Step 2: (5R,6S)-5,6-Bis(4-chlorophenyl)-N-[(3S,4S)-4-hydroxy-1-methylpyrrolidin-3-yl]-3-isopropyl-N,6-dimethyl-5,6-dihydroimidazo[2,1-b][1,3]thiazole-2-carboxamide

Trifluoroacetic acid (1 ml) was added to a dichloromethane (5 ml) solution of the compound (200 mg, 0.31 mmol) obtained in Step 1 above and the resulting mixture was stirred at room temperature for 40 minutes. After the mixture was concentrated, the obtained residue was dissolved in dichloromethane (5 ml) followed by the addition of triethylamine (0.045 ml, 0.32 mmol) and stirred at room temperature for 10 minutes. Then, 35% aqueous formaldehyde solution (0.055 ml, 0.64 mmol), acetic acid (0.027 ml, 0.47 mmol) and sodium triacetoxyborohydride (105 mg, 0.50 mmol) were added, and the resulting mixture was stirred for 3 hours at room temperature. The reaction mixture was concentrated and diluted with ethyl acetate and after the solution was washed with saturated aqueous sodium bicarbonate solution and brine and dried over anhydrous sodium sulfate. The drying agent was filtered off and the solvent was concentrated under reduced pressure. The obtained residue was purified by silica gel thin layer chromatography (chloroform: methanol = 10:1). Ether/n-hexane was added and the deposited solid was collected by filtration and dried to give the title compound (109 mg, 63%) as a colorless solid.
$^1$H-NMR ($CDCl_3$) δ: 0.90 (3H, d, J=7.1 Hz), 0.99 (3H, d, J=7.1 Hz), 1.82 (3H, s), 2.35 (3H, s), 2.41-2.53 (2H, m), 2.70-2.74 (1H, m), 2.82-2.87 (1H, m), 2.96-3.00 (1H, m), 3.08 (3H, s), 4.18-4.21 (1H, m), 4.42-4.45 (1H, m), 4.96 (1H, s), 6.70 (2H, d, J=6.8 Hz), 7.00-7.10 (6H, m).
MS (ESI) m/z: 559, 561.

Example 184

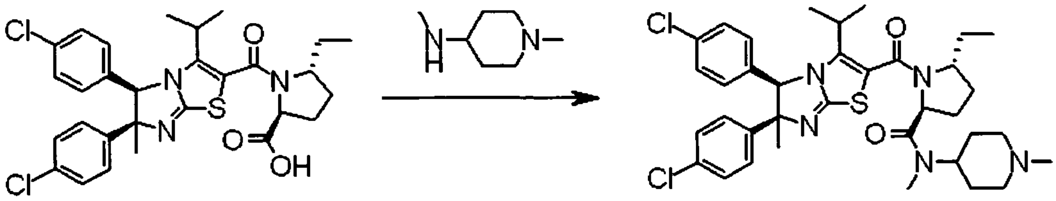

(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-ethyl-N-methyl-N-(1-methylpiperidin-4-yl)-L-prolinamide

The compound obtained in Step 2 of Example 103 was reacted in the same way as in Step 4 of Example 1 using N,1-dimethylpiperidin-4-amine instead of piperazin-2-one to give the title compound.
$^1$H-NMR ($CDCl_3$) δ: 0.90-1.07 (10H, m), 1.41-1.43 (2H, m), 1.61-1.63 (4H, m), 1.73-1.79 (5H, m), 1.92-2.07 (3H, m), 2.24-2.28 (4H, m), 2.69-2.96 (5H, m), 4.39-4.43 (2H, m), 4.93-4.94 (2H, m), 6.67 (2H, d, J=8.3 Hz), 7.01 (4H, t, J=8.2 Hz), 7.14 (2H, d, J=7.6 Hz).
MS (FAB) m/z: 682.

Example 185

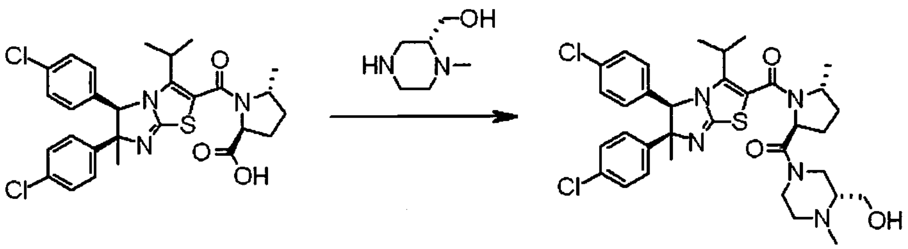

{(2R)-4-[(5R)-1-{[(5R,6S)-5,6-Bis(4-chlorophenyl)-3-isopropyl-6-methyl-5,6-dihydroimidazo[2,1-b][1,3] thiazol-2-yl]carbonyl}-5-methyl-L-prolyl]-1-methylpiperazin-2-yl}methanol

The compound obtained in Step 2 of Example 126 was reacted in the same way as in Step 4 of Example 1 using [(2R)-1-methylpiperazin-2-yl]methanol dihydrochloride instead of piperazin-2-one to give the title compound.
$^1$H-NMR ($CDCl_3$, 70°C) δ: 0.96 (3H, d, J=7.1 Hz), 1.02 (3H, d, J=7.1 Hz), 1.23 (3H, d, J=6.3 Hz), 1.59-1.69 (1H, m), 1.75-1.87 (1H, m), 1.79 (3H, s), 2.09-4.26 (13H, m), 2.39 (3H, s), 4.48-4.59 (1H, m), 4.92 (1H, s), 5.00-5.14 (1H, m), 6.68 (2H, d, J=8.5 Hz), 6.97-7.03 (4H, m), 7.12 (2H, d, J=8.5 Hz).
MS (ESI) m/z: 670, 672.

Example 186

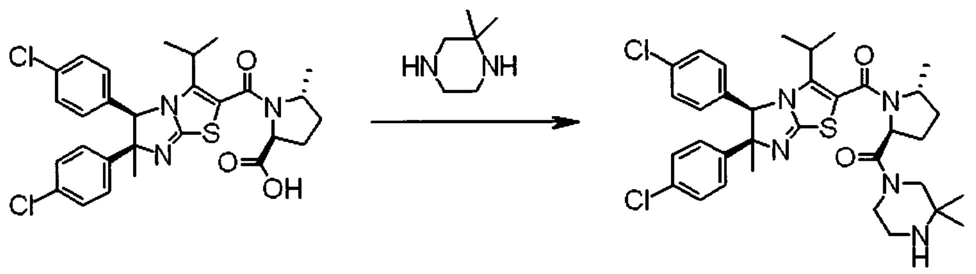

(5R,6S)-5,6-Bis(4-chlorophenyl)-2-({(2S,5R)-2-[(3,3-dimethylpiperazin-1-yl)carbonyl]-5-methylpyrrolidin-1-yl}carbonyl)-3-isopropyl-6-methyl-5,6-dihydroimidazo[2,1-b][1,3]thiazole

The compound obtained in Step 2 of Example 126 was reacted in the same way as in Step 4 of Example 1 using 2,2-dimethylpiperazine dihydrochloride instead of piperazin-2-one to give the title compound.
$^1$H-NMR ($CDCl_3$) δ: 0.93 (3H, d, J=7.1 Hz), 1.03 (3H, d, J=7.1 Hz), 1.07-1: 15 (6H, m), 1.23 (3H, d, J=6.3 Hz), 1.62-1.69 (1H, m), 1.79 (3H, s), 1.83-1.88 (1H, m), 2.22-2.39 (2H, m), 2.72-2.78 (1H, m), 2.87-2.96 (2H, m), 3.20-3.27 (1H, m), 3.38-3.55 (3H, m), 4.52-4.56 (1H, m), 4.93 (1H, s), 5.01-5.04 (1H, m), 6.69 (2H, d, J=8.5 Hz), 7.01 (5H, d, J=8.8 Hz), 7.12 (2H, d, J=8.5 Hz).
MS (ESI) m/z: 654.

Example 187

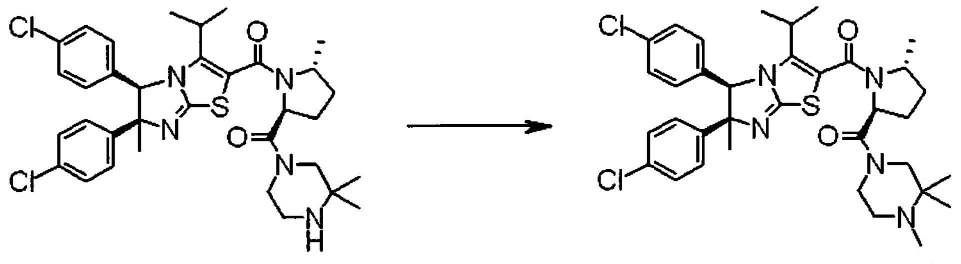

(5R,6S)-5,6-Bis(4-chlorophenyl)-3-isopropyl-6-methyl-2-({(2R,5S)-2-methyl-5-[(3,3,4-trimethylpiperazin-1-yl)carbonyl] pyrrolidin-1-yl}carbonyl)-5,6-dihydroimidazo[2,1-b][1,3]thiazole

The compound obtained in Example 186 was reacted in the same way as in Step 3 of Example 152 to give the title compound.
$^1$H-NMR ($CDCl_3$) δ: 0.92 (3H, d, J=7.1 Hz), 0.95-1.02 (6H, m), 1.03 (3H, d, J=7.1 Hz), 1.23 (3H, d, J=6.6 Hz), 1.61-1.67 (1H, m), 1.79 (3H, s), 1.82-1.86 (1H, m), 2.24 (3H, s), 2.26-2.36 (1H, m), 2.51-2.54 (1H, m), 2.73-2.76 (1H, m), 3.18-3.34 (2H, m), 3.47-3.65 (4H, m), 4.51-4.56 (1H, m), 4.93 (1H, s), 5.02-5.05 (1H, m), 6.68 (2H, d, J=7.8 Hz), 7.01 (4H, d, J=8. 5 Hz), 7.12 (2H, d, J=8.3 Hz).
MS (ESI) m/z: 668.

Example 188

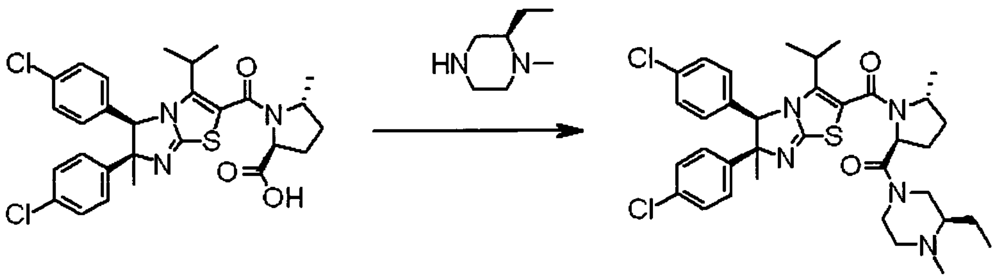

(5R,6S)-5,6-Bis(4-chlorophenyl)-2-{[(2S,5R)-2-{[(3R)-3-ethyl-4-methylpiperazin-1-yl]carbonyl}-5-methylpyrrolidin-1-yl] carbonyl}-3-isopropyl-6-methyl-5,6-dihydroimidazo[2,1-b][1,3]thiazole

The compound obtained in Step 2 of Example 126 was reacted in the same way as in Step 4 of Example 1 using (2R)-2-ethyl-1-methylpiperazine instead of piperazin-2-one to give the title compound.
$^1$H-NMR ($CDCl_3$) δ: 0.89-0.97 (6H, m), 1.03 (3H, d, J=7.1 Hz), 1.24 (3H, d, J=6.3 Hz), 1.63-1.67 (2H, m), 1.79 (3H, s), 1.84-1.87 (1H, m), 2.18-2.26 (3H, m), 2.29 (3H, s), 2.72-3.02 (3H, m), 3.39-3.46 (1H, m), 3.67-3.72 (2H, m), 4.24-4.30 (2H, m), 4.50-4.57 (1H, m), 4.93 (1H, s), 4.98-5.02 (1H, m), 6.68 (2H, d, J=7.6 Hz), 7.01 (4H, d, J=8.3 Hz), 7.12 (2H, d, J=8.5 Hz).
MS (ESI) m/z: 668.

Example 189

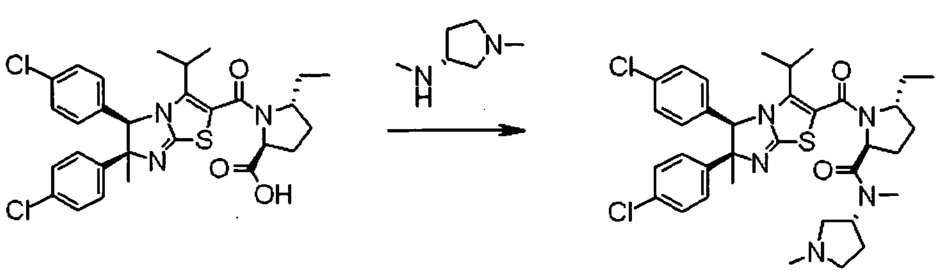

(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-ethyl-N-methyl-N-[(3R)-1-methylpyrrolidin-3-yl]-L-prolinamide

The compound obtained in Step 2 of Example 103 was reacted in the same way as in Step 4 of Example 1 using the compound obtained in Reference Example 58 instead of piperazin-2-one to give the title compound.
$^1$H-NMR ($CDCl_3$) δ: 0.92-0.94 (8H, m), 1.04 (3H, dd, J=6.7, 3.1 Hz), 1.25-1.40 (3H, m), 1.78-1.80 (6H, m), 2.22-2.31 (6H, m), 2.72-2.89 (4H, m), 3.09 (2H, s), 4.36-4.39 (1H, m), 4.92-4.95 (2H, m), 6.66 (2H, d, J=8.5 Hz), 7.02 (4H, dd, J=8.5, 5.9 Hz), 7.14 (2H, d, J=6.3 Hz).
MS (FAB) m/z: 668.

Example 190

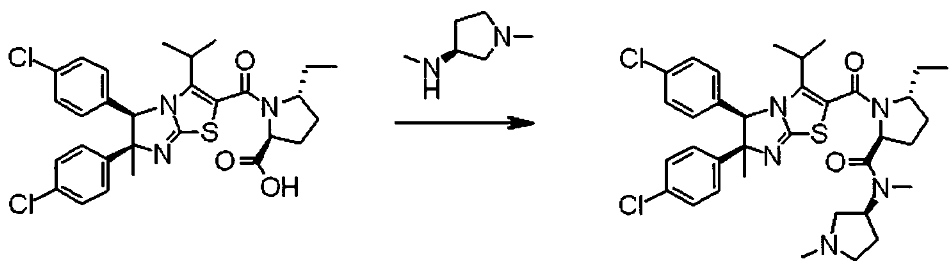

(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-ethyl-N-methyl-N-[(3S)-1-methylpyrrolidin-3-yl]-L-prolinamide

The compound obtained in Step 2 of Example 103 was reacted in the same way as in Step 4 of Example 1 using the compound obtained in Reference Example 59 instead of piperazin-2-one to give the title compound.
$^1$H-NMR ($CDCl_3$) δ: 0.88-1.04 (10H, m), 1.31-1.41 (3H, m), 1.58-1.60 (2H, m), 1.78-1.80 (5H, m), 2.26-2.89 (9H, m), 3.10-3.13 (2H, m), 4.35-4.38 (2H, m), 4.92-4.95 (2H, m), 6.67 (2H, d, J=8.3 Hz), 7.02 (4H, dd, J=8.2, 6.5 Hz), 7.13-7.14 (2H, m).
MS (FAB) m/z: 668.

Example 191

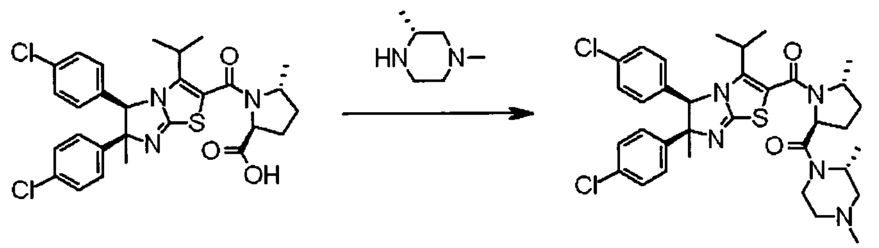

(5R,6S)-5,6-Bis(4-chlorophenyl)-2-{[(2S,5R)-2-{[(2R)-2,4-dimethylpiperazin-1-yl]carbonyl}-5-methylpyrrolidin-1-yl]carbonyl}-3-isopropyl-6-methyl-5,6-dihydroimidazo[2,1-b][1,3]thiazole

The compound obtained in Step 2 of Example 126 was reacted in the same way as in Step 4 of Example 1 using the compound obtained in Step 3 of Reference Example 60 instead of piperazin-2-one to give the title compound.
$^1$H-NMR ($CDCl_3$) δ: 0.85-1.05 (7H, m), 1.25-1.28 (6H, m), 1.51-1.56 (5H, m), 1.82-1.85 (4H, m), 2.23-2.40 (5H, m), 2.69-2.72 (2H, m), 4.00-4.28 (1H, m), 4.55 (1H, m), 4.94-5.00 (2H, m), 6.68-6.69 (2H, m), 7.00-7.03 (4H, m), 7.13-7.15 (2H, m).
MS (FAB) m/z: 654.

Example 192

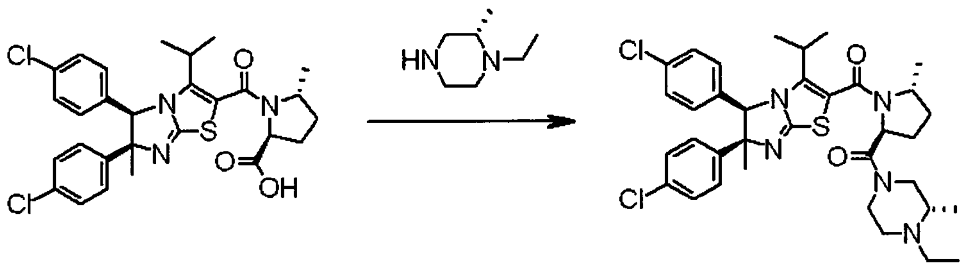

(5R,6S)-5,6-Bis(4-chlorophenyl)-2-{[(2S,5R)-2-{[(3S)-4-ethyl-3-methylpiperazin-1-yl]carbonyl}-5-methylpyrrolidin-1-yl] carbonyl}-3-isopropyl-6-methyl-5,6-dihydroimidazo[2,1-b][1,3]thiazole

The compound obtained in Step 2 of Example 126 was reacted in the same way as in Step 4 of Example 1 using the compound obtained in Reference Example 61 instead of piperazin-2-one to give the title compound.
$^1$H-NMR ($CDCl_3$, 70°C) δ: 0.77-4.29 (14H, m), 0.89 (3H, t, J=6.5 Hz), 0.94 (3H, d, J=7.1 Hz), 1.03 (3H, d, J=7.1 Hz), 1.06 (3H, d, J=7.1 Hz), 1.23 (3H, d, J=6.3 Hz), 1.79 (3H, s), 4.48-4.59 (1H, m), 4.92 (1H, s), 5.01 (1H, d, J=7.1 Hz), 6.68 (2H, d, J=8.3 Hz), 6.96-7.03 (4H, m), 7.11 (2H, d, J=8.3 Hz).
MS (ESI) m/z: 668.

Example 193

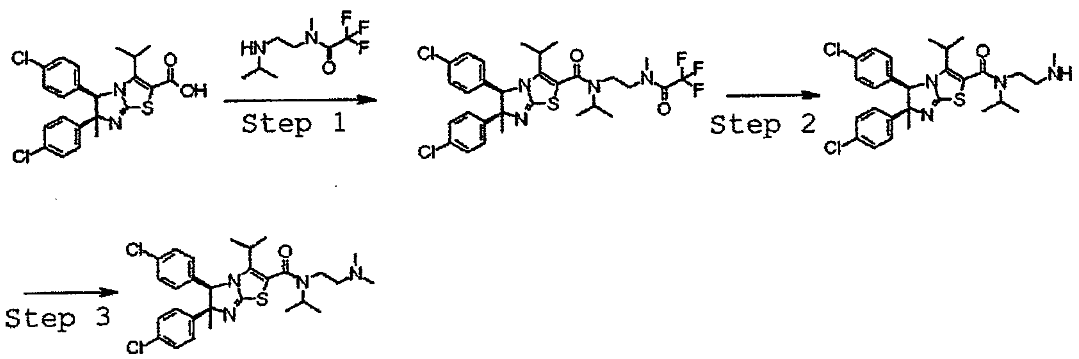


Step 1: (5R,6S)-5,6-Bis(4-chlorophenyl)-N,3-diisopropyl-6-methyl-N-{2-[methyl(trifluoroacetyl)amino]ethyl}-5,6-dihydroimidazo[2,1-b][1,3]thiazole-2-carboxamide

The compound obtained in Step 3 of Example 1 was reacted in the same way as in Example 112 using the compound obtained in Step 3 of Reference Example 62 instead of the compound obtained in Step 2 of Reference Example 18 to give the title compound.
$^1$H-NMR ($CDCl_3$) δ: 0.90 (3H, d, J=7.1 Hz), 1. 02 (3H, d, J=7.1 Hz), 1.18-1.24 (3H, m), 1.26-1.29 (3H, m), 1.83 (3H, s), 2.39-2.46 (1H, m), 3.23 (3H, s), 3.42-3.45 (2H, m), 3.55-3.62 (2H, m), 4.35-4.41 (1H, m), 4.95 (1H, s), 6.71 (2H, d, J=7.8 Hz), 7.00-7.11 (6H, m).
MS (ESI) m/z: 641, 643.

Step 2: (5R,6S)-5,6-Bis(4-chlorophenyl)-N,3-diisopropyl-6-methyl-N-[2-(methylamino)ethyl]-5,6-dihydroimidazo[2,1-b] [1,3]thiazole-2-carboxamide

Potassium carbonate (140 mg, 1.0 mmol) was added to a methanol (15 ml)/water (1.5 ml) solution of the compound (620 mg, 0.966 mmol) obtained in Step 1 above and the resulting mixture was stirred at room temperature for 14 hours. After concentrated, the mixture was diluted with ethyl acetate, washed with saturated aqueous sodium

bicarbonate solution and brine and dried over anhydrous sodium sulfate. Then drying agent was filtered off and the solvent was concentrated under reduced pressure. The obtained residue was purified by silica gel column chromatography (chloroform:methanol 10:1) to give the title compound (505 mg, 96%) as a colorless solid.
$^1$H-NMR ($CDCl_3$) δ: 0.90 (3H, d, J=7.1 Hz), 1.03 (3H, d, J=7.1 Hz), 1.20 (3H, d, J=6.8 Hz), 1.27 (3H, d, J=6.8 Hz), 1.82 (3H, s), 2.38-2.44 (1H, m), 2.47 (3H, s), 3.35-3.38 (2H, m), 4.36-4.39 (1H, m), 4.94 (1H, s), 6.71 (2H, d, J=8.0 Hz), 7.01-7.11 (6H, m).
MS (ESI) m/z: 544, 546.

Step 3: (5R,6S)-5,6-Bis(4-chlorophenyl)-N-[2-(dimethylamino)ethyl]-N,3-diisopropyl-6-methyl-5,6-dihydroimidazo [2,1-b][1,3]thiazole-2-carboxamide

The compound obtained in Step 2 above was reacted in the same way as in Step 3 of Example 152 to give the title compound.
$^1$H-NMR ($CDCl_3$) δ: 0.91 (3H, d, J=6.9 Hz), 1.02 (3H, d, J=6.9 Hz), 1.20 (3H, d, J=6.6 Hz), 1.26 (3H, d, J=6.6 Hz), 1.82 (3H, s), 2.29 (6H, s), 2.37-2.44 (1H, m), 2.46-2.50 (2H, m), 3.32-3.43 (2H, m), 4.33-4.40 (1H, m), 4.94 (1H, s), 6.71 (2H, d, J=7.8 Hz), 7.01-7.11 (6H, m).
MS (ESI) m/z: 559, 561.

Example 194

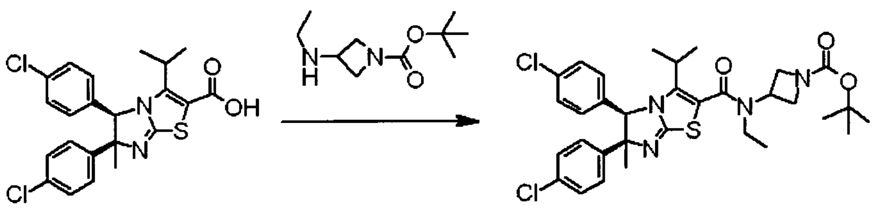

tert-Butyl 3-[{[(5R,6S)-5,6-Bis(4-chlorophenyl)-3-isopropyl-6-methyl-5,6-dihydroimidazo[2,1-b][1,3]thiazol-2-yl]carbonyl}(ethyl)amino]azetidine-1carboxylate

The compound obtained in Step 3 of Example 1 was reacted in the same way as in Step 4 of Example 1 using tert-butyl 3-(ethylamino)azetidine-1-carboxylate instead of piperazin-2-one to give the title compound.
$^1$H-NMR ($CDCl_3$) δ: 0.88 (3H, d, J=7.1 Hz), 1.01 (3H, d, J=7.1 Hz), 1.24 (3H, t, J=7.0 Hz), 1.47 (9H, s), 1.84 (3H, s), 2.47 (1H, m), 3.59-3.63 (2H, m), 3.99-4.01 (2H, m), 4.18-4.26 (2H, m), 4.77 (1H, m), 4.97 (1H, s), 6.73 (2H, d, J=8.1 Hz), 7.01-7.12 (6H, m).

Example 195

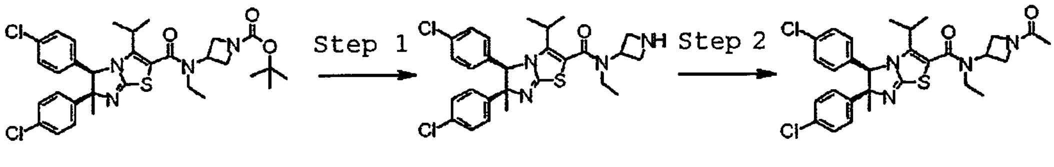


Step 1: (5R,6S)-N-Azetidin-3-yl-5,6-bis(4-chlorophenyl)-N-ethyl-3-isopropyl-6-methyl-5,6-dihydroimidazo[2,1-b][1,3]thiazole-2-carboxamide

The compound obtained in Example 194 was reacted in the same way as in Step 6 of Example 102 to give the title compound.
$^1$H-NMR ($CDCl_3$) δ: 0.88 (3H, d, J=7.1 Hz), 1.01 (3H, d, J=7.1 Hz), 1.19 (3H, t, J=7.1 Hz), 1.83 (3H, s), 2.44 (1H, m),

3.57-3.66 (2H, m), 3.72-3.85 (4H, m), 4.88 (1H, m), 4.95 (1H, s), 6.72 (2H, d, J=8.1 Hz), 7.02-7.12 (6H, m). Step 2: N-(1-Acetylazetidin-3-yl)-5,6-Bis(4-chlorophenyl)-N-ethyl-3-isopropyl-6-methyl-5,6-dihydroimidazo[2,1-b][1,3]thiazole-2-carboxamide

The compound obtained in Step 1 above was reacted in the same way as in Step 3 of Example 161 to give the title compound.

$^1$H-NMR ($CDCl_3$) δ: 0.88 (3H, d, J=7.1 Hz), 1.00 (3H, d, J=7.1 Hz), 1.21 (3H, td, J=7.0, 2.1 Hz), 1.82 (3H, s), 1.90 and 1.91 (total 3H, each s), 2.49 (1H, m), 3.54 (1H, m), 3.66 (1H, m), 4.09-4.29 (3H, m), 4.41 (1H, m), 4.71 (1H, m), 4.96 (1H, s), 6.68-6.75 (2H, m), 7.01-7.11 (6H, m) .

MS (ESI) m/z: 571, 573.

Example 196

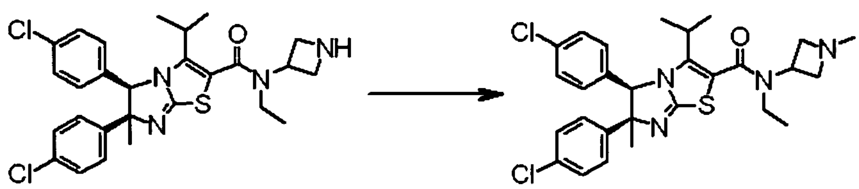

5,6-Bis(4-chlorophenyl)-N-ethyl-3-isopropyl-6-methyl-N-(1-methylazetidin-3-yl)-5,6-dihydroimidazo[2,1-b][1,3]thiazole-2-carboxamide

The compound obtained in Step 2 of Example 195 was reacted in the same way as in Step 3 of Example 152 to give the title compound.

$^1$H-NMR ($CDCl_3$) δ: 0.87 (3H, d, J=7.1 Hz), 1.00 (3H, d, J=7.1 Hz), 1.17 (3H, t, J=7.1 Hz), 1.82 (3H, s), 2.37 (3H, s), 2.43 (1H, m), 3.01-3.06 (2H, m), 3.54-3.63 (2H, m), 3.66-3.74 (2H, m), 4.57 (1H, m), 4.94 (1H, s), 6.68-6.76 (2H, d, J=8.1 Hz), 7.01-7.11 (6H, m).

MS (ESI) m/z: 543.

Example 197

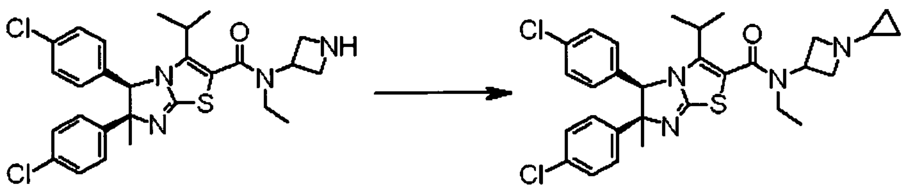

5,6-Bis(4-chlorophenyl)-N-(1-cyclopropylazetidin-3-yl)-N-ethyl-3-isopropyl-6-methyl-5,6-dihydroimidazo[2,1-b][1,3]thiazole-2-carboxamide

The compound obtained in Step 2 of Example 195 was reacted in the same way as in Step 2 of Example 168 using 1-ethoxycyclopropyltrimethylsilane instead of 35% aqueous formaldehyde solution to give the title compound.

$^1$H-NMR ($CDCl_3$) δ: 0.76-0.84 (2H, m), 0.92-1.06 (7H, m), 1.13 (3H, td, J=7.1, 2.2 Hz), 1.82 (3H, s), 2.63-2.74 (2H, m), 2.76-2.95 (2H, m), 3.31-3.47 (2H, m), 3.60-3.76 (2H, m), 4.98 (1H, s), 6.70 (2H, d, J=8.3 Hz), 7.00-7.14 (6H, m) .

MS (ESI) m/z: 569.

Example 198

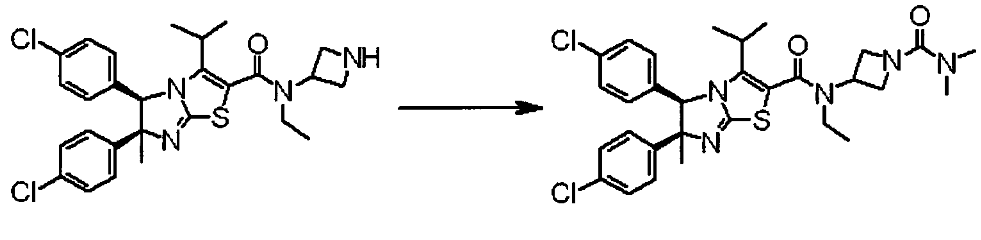

5,6-Bis(4-chlorophenyl)-N-[1-(dimethylcarbamoyl)azetidin-3-yl]-N-ethyl-3-isopropyl-6-methyl-5,6-dihydroimidazo [2,1-b][1,3]thiazole-2-carboxamide

Triethylamine (80 μl, 0.57 mmol) and N,N-dimethylcarbamoyl chloride (38 μl, 0.42 mmol) were added to a dichloromethane (3 ml) solution of the compound (200 mg, 0.337 mmol) obtained in Step 2 of Example 195 under ice cooling. After the resulting mixture was stirred for 1 hour, the mixture was extracted with chloroform and washed with 10% aqueous citric acid solution and brine and dried over anhydrous sodium sulfate. After the solvent was evaporated under reduced pressure, the residue was solidified with diethyl ether to give the title compound (205 mg, 91%) as a colorless solid.
$^1$H-NMR ($CDCl_3$) δ: 0.88 (3H, d, J=7.1 Hz), 1.00 (3H, d, J=7.1 Hz), 1.21 (3H, t, J=7.1 Hz), 1.84 (3H, s), 2.48 (1H, m), 2.88 (6H, s), 3.58-3.64 (2H, m), 3.96-4.02 (2H, m), 4.17-4.26 (2H, m), 4.73 (1H, m), 4.97 (1H, s), 6.69-6.72 (2H, m), 7.00-7.11 (6H, m).
MS (ESI) m/z: 600, 602.

Example 199

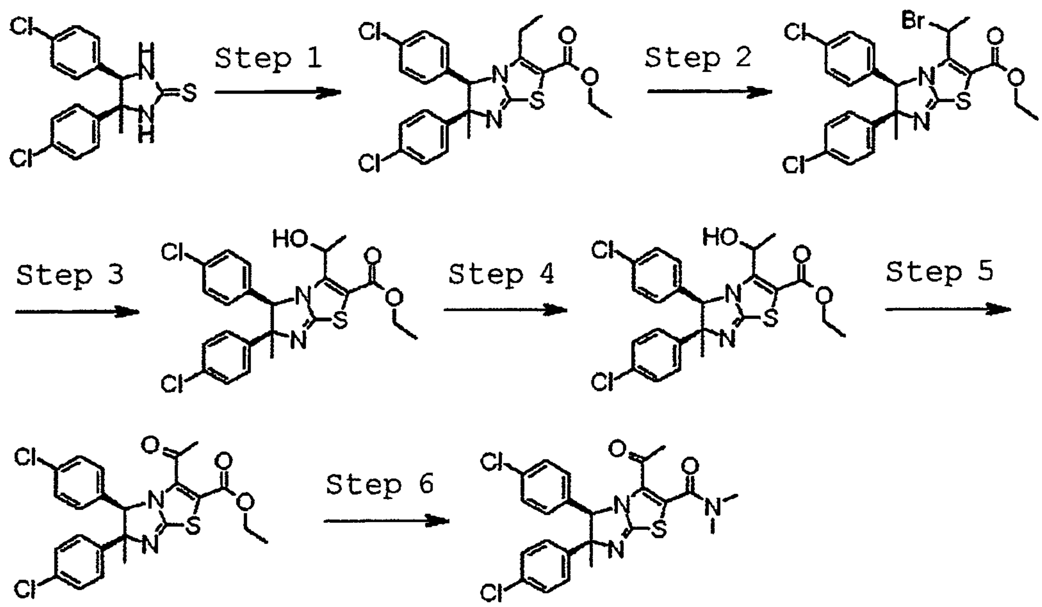


Step 1: Ethyl (5R,6S)-5,6-Bis(4-chlorophenyl)-3-ethyl-6-methyl-5,6-dihydroimidazo[2,1-b][1,3]thiazole-2-carboxylate

(4S,5R)-4,5-bis(4-chlorophenyl)-4-methylimidazolidine-2-thione (2.00 g, 5.93 mmol) was added to an ethanol

(50 ml) solution of ethyl 2-chloro-3-oxopentanoate (1.48 g, 8.30 mmol) and the resulting mixture was heated to reflux for 2 days. After the reaction mixture was concentrated under reduced pressure, the obtained residue was diluted with ethyl acetate and washed with saturated aqueous sodium bicarbonate solution and brine, and then the organic layer was dried over anhydrous magnesium sulfate. The solvent was evaporated under reduced pressure and the obtained residue was separated and purified by silica gel column chromatography (hexane → hexane:ethyl acetate = 1:1) to give the title compound (2.90 g, 100%) as a pale yellow solid.

Step 2: Ethyl (5R,6S)-3-(1-Bromoethyl)-5,6-bis(4-chlorophenyl)-6-methyl-5,6-dihydroimidazo[2,1-b][1,3]thiazole-2-carboxylate

N-Bromosuccinimide (1.23 g, 6.91 mmol) and 2,2'-azobis (isobutyronitrile) (50 mg) were added to a carbon tetrachloride (50 ml) solution of the compound (2.90 g, 6.29 mmol) obtained in Step 1 above and the resulting mixture was heated to reflux for 18 hours. After the reaction mixture was diluted with chloroform and washed with saturated aqueous sodium bicarbonate solution and brine, the organic layer was dried over anhydrous magnesium sulfate. The solvent was evaporated under reduced pressure and the obtained residue was separated and purified by silica gel column chromatography (hexane → hexane:ethyl acetate = 4:1) to give the title compound (1.87 g, 55%) as a yellow solid.
$^1$H-NMR ($CDCl_3$) δ: 1.08 (3H, d, J=7.6 Hz), 1.35 (3H, t, J=7.3 Hz), 1.83 (3H, s), 4.28 (2H, q, J=7.3 Hz), 5.59 (1H, s), 6.27 (1H, q, J=7.6 Hz), 6.64 (1H, brs), 6.84 (1H, brs), 7.01-7.07 (4H, m), 7.11-7.16 (2H, m).

Step 3: Ethyl (5R,6S)-5,6-Bis(4-chlorophenyl)-3-[1-hydroxyethyl]-6-methyl-5,6-dihydroimidazo[2,1-b][1,3]thiazole-2-carboxylate

A water (10 ml) solution of silver nitrate (1.18 g, 6.92 mmol) was added to an acetone (40 ml) solution of the compound (1.87 g, 3.46 mmol) obtained in Step 2 above and the resulting mixture was stirred at room temperature for 18 hours. The reaction mixture was diluted with ethyl acetate and the insoluble matter was filtered off through a celite pad. Then the filtrate was washed with brine and the organic layer was dried over anhydrous magnesium sulfate. The solvent was evaporated under reduced pressure and the obtained residue was separated and purified by silica gel column chromatography (hexane → hexane:ethyl acetate = 1:1) to give the title compound (592 mg, 36%) as a pale yellow solid.
$^1$H-NMR ($CDCl_3$) δ: 0.77 (3H, d, J=6.6 Hz), 1.35 (3H, t, J=7.1 Hz), 1.80 (3H, s), 4.24-4.40 (3H, m), 5.06 (1H, s), 5.35 (1H, d, J=12.0 Hz), 6.63-7.09 (8H, m).

Step 4: Ethyl (5R,6S)-3-Acetyl-5,6-bis(4-chlorophenyl)-6-methyl-5,6-dihydroimidazo[2,1-b][1,3]thiazole-2-carboxylate

Manganese dioxide (1.84 g, 18.6 mmol) was added to a chloroform (20 ml) solution of the compound (592 mg, 1.24 mmol) obtained in Step 3 above, and the resulting mixture was heated and stirred at 60°C for 10 days. The insoluble matter was filtered off through a celite pad and the filtrate was concentrated under reduced pressure. The obtained residue was separated and purified by silica gel column chromatography (hexane → hexane:ethyl acetate = 1:1) to give the title compound (250 mg, 42%) as a pale yellow solid.
$^1$H-NMR ($CDCl_3$) δ: 1.32 (3H, t, J=7.2 Hz), 1.81 (3H, s), 1.88 (3H, s), 4.23-4.31 (2H, m), 5.07 (1H, s), 6.57-6.81 (2H, m), 7.01-7.12 (6H, m).

Step 5: (5R,6S)-3-Acetyl-5,6-bis(4-chlorophenyl)-N,N,6-trimethyl-5,6-dihydroimidazo[2,1-b][1,3]thiazole-2-carboxamide

1 N aqueous sodium hydroxide solution (2.0 ml) was added to an ethanol (10 ml) solution of the compound (225 mg, 0.473 mmol) obtained in Step 4 above and the resulting mixture was heated to reflux for 20 minutes. 1 N aqueous hydrochloric acid solution (2.0 ml) and water (50 ml) were added to the reaction mixture under ice cooling and after the solution was stirred, the deposited insoluble matter was collected by filtration. The obtained solid (174 mg) was dissolved in N,N-dimethylformamide (2.0 ml) and dimethylamine hydrochloride (63.4 mg, 0.778 mmol), 1-(3-dimethylaminopropyl)-3-ethylcarbodiimide hydrochloride (149 mg, 0.778 mmol), 1-hydroxybenzotriazole (52.6 mg, 0.389 mmol) and triethylamine (54 μl, 0.39 mmol) were added and the resulting mixture was stirred at room temperature for 2 hours. The reaction mixture was poured into saturated aqueous sodium bicarbonate solution and, after the resulting mixture was stirred, the deposited insoluble matter was collected by filtration. The obtained solid was separated and purified by thin layer silica gel column chromatography (hexane:ethyl acetate = 1:2) to give the title compound (30 mg, 16%) as a pale yellow solid.
$^1$H-NMR ($CDCl_3$) δ: 1.81 (3H, s), 2.04 (3H, s), 3.11 (6H, s), 5.46 (1H, s), 6.74 (2H, d, J=7.8 Hz), 6.95-7.06 (4H, m), 7.13 (2H, d, J=7.8 Hz).

MS (ESI) m/z: 474, 476.

Example 200

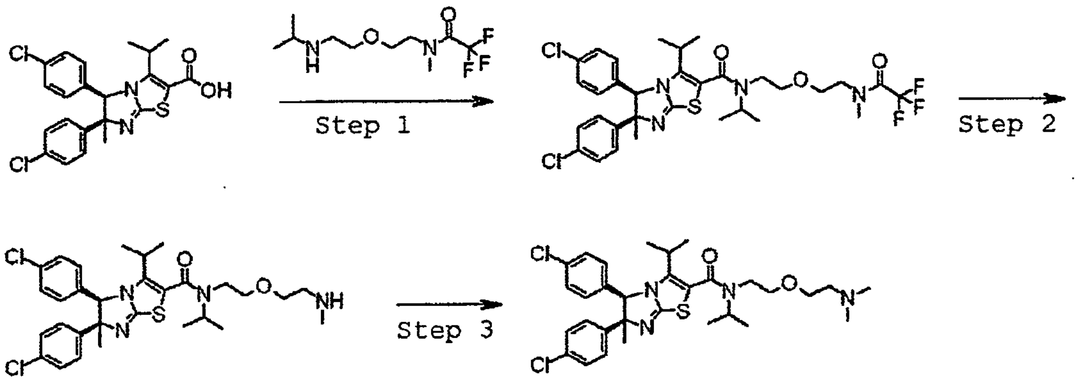


Step 1: (5R,6S)-5,6-Bis(4-chlorophenyl)-N,3-diisopropyl-6-methyl-N-(2-{2-[methyl(trifluoroacetyl)amino]ethoxy}ethyl)-5,6-dihydroimidazo[2,1-b][1,3]thiazole-2-carboxamide

The compound obtained in Step 3 of Example 1 was reacted in the same way as in Example 112 using the compound obtained in Step 5 of Reference Example 63 instead of the compound obtained in Step 2 of Reference Example 18 to give the title compound.
$^1$H-NMR ($CDCl_3$) δ: 0.89 (3H, d, J=7.1 Hz), 1.03 (3H, d, J=7.1 Hz), 1.18 (3H, d, J=6.6 Hz), 1.25 (3H, d, J=6.6 Hz), 1.82 (3H, s), 2.36-2.43 (1H, m), 3.20-3.21 (2H, m), 3.40-3.44 (2H, m), 3.61-3.65 (4H, m), 4.09-4.15 (1H, m), 4.94 (1H, s), 6.71 (2H, d, J=7.5 Hz), 7.01-7.11 (6H, m).
MS (ESI) m/z: 685, 687.

Step 2: (5R,6S)-5,6-Bis(4-chlorophenyl)-N,3-diisopropyl-6-methyl-N-{2-[2-(methylamino)ethoxy]ethyl}-5,6-dihydroimidazo[2,1-b][1,3]thiazole-2-carboxamide

The compound obtained in Step 1 above was reacted in the same way as in Step 2 of Example 193 to give the title compound.
$^1$H-NMR ($CDCl_3$) δ: 0.89 (3H, d, J=7.0 Hz), 1.02 (3H, d, J=7.0 Hz), 1.20 (3H, d, J=6.6 Hz), 1.26 (3H, d, J=6.6 Hz), 1.83 (3H, s), 2.36-2.41 (1H, m), 2.50 (3H, s), 2.80 (2H, t, J=5.6 Hz), 3.42-3.53 (2H, m), 3.59-3.64 (4H, m), 4.34-4.41 (1H, m), 4.94 (1H, s), 6.71 (2H, d, J=8.1 Hz), 7.01-7.11 (6H, m).
MS (ESI) m/z: 589, 561.

Step 3: (5R,6S)-5,6-Bis(4-chlorophenyl)-N-{2-[2-(dimethylamino)ethoxy]ethyl}-N,3-diisopropyl-6-methyl-5,6-dihydroimidazo[2,1-b][1,3]thiazole-2-carboxamide

The compound obtained in Step 2 above was reacted in the same way as in Step 3 of Example 152 to give the title compound.
$^1$H-NMR ($CDCl_3$) δ: 0.91 (3H, d, J=7.0 Hz), 1. 02 (3H, d, J=7.0 Hz), 1.19 (3H, d, J=6.6 Hz), 1.25 (3H, d, J=6.6 Hz), 1.82 (3H, s), 2.26 (6H, s), 2.36-2.43 (1H, m), 2.49 (2H, t, J=5.8 Hz), 3.42-3.47 (2H, m), 3.54 (2H, t, J=5.8 Hz), 3.59-3.64 (2H, m), 4.32-4.39 (1H, m), 4.93 (1H, s), 6.70 (2H, d, J=7.8 Hz), 7.01-7.11 (6H, m).
MS (ESI) m/z: 603, 605.

Example 201

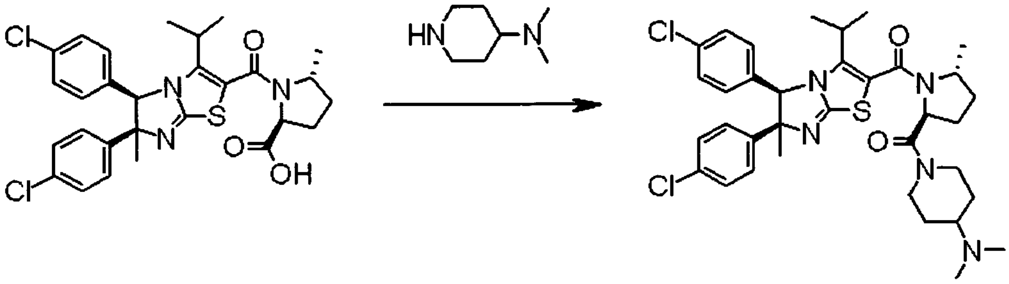

1-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-N,N-dimethylpiperidine-4-amine

The compound obtained in Step 2 of Example 126 was reacted in the same way as in Step 4 of Example 1 using 4-dimethylaminopiperidine instead of piperazin-2-one to give the title compound.
$^1$H-NMR (DMSO-$d_6$, 100°C) δ: 0.84 (1H, m), 0.85 (3H, d, J=7.1 Hz), 0.93 (1H, m), 0.95 (3H, d, J=7.1 Hz), 1.17 (3H, d, J=6.3 Hz), 1.25-1.42 (3H, m), 1.58 (1H, m), 1.72 (3H, s), 1.73-1.82 (4H, m), 2.04-2.16 (2H, m), 2.20 (6H, s), 2.27-2.36 (2H, m), 2.71 (1H, m), 3.98-4.12 (2H, m), 4.32 (1H, m), 4.97 (1H, m), 5.38 (1H, s), 6.88 (2H, d, J=7.6 Hz), 7.06 (2H, d, J=7.6 Hz), 7.11 (2H, d, J=7.6 Hz), 7.25 (2H, d, J=7.6 Hz) .
MS (ESI) m/z: 668, 670.

Example 202

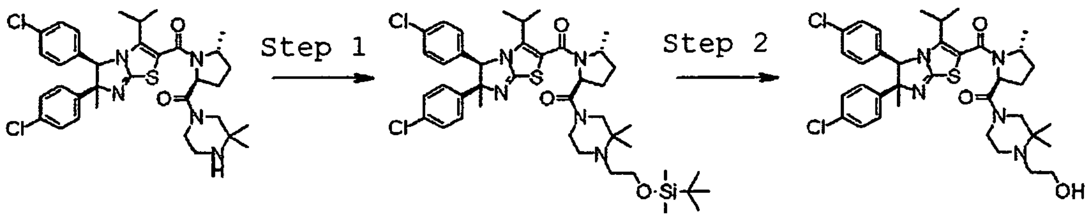


Step 1: (5R,6S)-2-{[(2S,5R)-2-{[4-(2-{[tert-Butyl(dimethyl)silyl]oxy}ethyl)-3,3-dimethylpiperazin-1-yl]carbonyl}-5-methylpyrrolidin-1-yl]carbonyl}-5,6-bis(4-chlorophenyl)-3-isopropyl-6-methyl-5,6-dihydroimidazo[2,1-b][1,3]thiazole

The compound obtained in Example 186 was reacted in the same way as in Step 3 of Example 152 using (tert-butyldimethylsilyloxy)acetaldehyde instead of 35% aqueous formaldehyde solution to give the title compound.
$^1$H-NMR ($CDCl_3$) δ: 0.00 (6H, s), 0.83-0.94 (18H, m), 0.98 (3H, d, J=7.1 Hz), 1.17 (3H, d, J=6.3 Hz), 1.48-1.51 (1H, m), 1.55-1.58 (1H, m), 1.73 (3H, s), 1.78-1.81 (1H, m), 2.22-2.26 (1H, m), 2.39-2.45 (2H, m), 2.53-2.59 (2H, m), 2.67-2.73 (2H, m), 3.12-3.17 (1H, m), 3.44-3.49 (2H, m), 3.54-3.59 (2H, m), 4.44-4.49 (1H, m), 4.87 (1H, s), 4.94-4.99 (1H, m), 6.63 (2H, d, J=8.3 Hz), 6.93-6.97 (4H, m), 7.06 (2H, d, J=8.5 Hz).
MS (ESI) m/z: 813.

Step 2: 2-{4-[(5R)-1-{[(5R,6S)-5,6-Bis(4-chlorophenyl)-3-isopropyl-6-methyl-5,6-dihydroimidazo[2,1-b][1,3]thiazol-2-yl] carbonyl)-5-methyl-L-prolyl]-2,2-dimethylpiperazin-1-yl}ethanol

The compound obtained in Step 1 above was reacted in the same way as in Step 2 of Example 177 to give the title compound.
$^1$H-NMR ($CDCl_3$) δ: 0.93 (3H, d, J=7.1 Hz), 0.99-1: 10 (9H, m), 1.24 (3H, d, J=6.3 Hz), 1.56-1.67 (2H, m), 1.79 (3H, s), 1.84-1.87 (1H, m), 2.23-2.40 (2H, m), 2.47-2.64 (3H, m), 2.70-2.78 (1H, m), 3.20-3.71 (6H, m), 4.50-4.55 (1H, m), 4.93 (1H, s), 5.01-5.04 (1H, m), 6.69 (2H, d, J=8.3 Hz), 7.01 (2H, d, J=8.3 Hz), 7.01 (2H, d, J=8.5 Hz), 7.12 (2H, d, J=8.5 Hz).
MS (ESI) m/z: 698.

Example 203

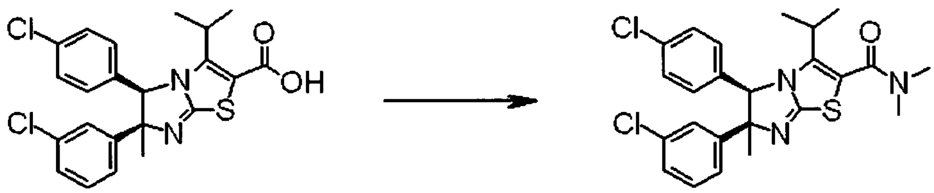

(5R,6S)-6-(3-Chlorophenyl)-5-(4-chlorophenyl)-3-isopropyl-N,N,6-trimethyl-5,6-dihydroimidazo[2,1-b][1,3]thiazole-2-carboxamide

The compound obtained in Step 12 of Reference Example 64 was reacted in the same way as in Step 4 of Example 1 using 2 N dimethylamine/tetrahydrofuran solutions instead of piperazin-2-one to give the title compound.
$^1$H-NMR ($CDCl_3$) δ: 0.91 (3H, d, J=7.1 Hz), 1.01 (3H, d, J=7.1 Hz), 1.83 (3H, s), 2.41-2.49 (1H, m), 3.07 (6H, s), 4.94 (1H, s), 6.72 (2H, d, J=7.6 Hz), 6.95-7.06 (5H, m), 7.19 (1H, s).
MS (ESI) m/z: 474.

Example 204

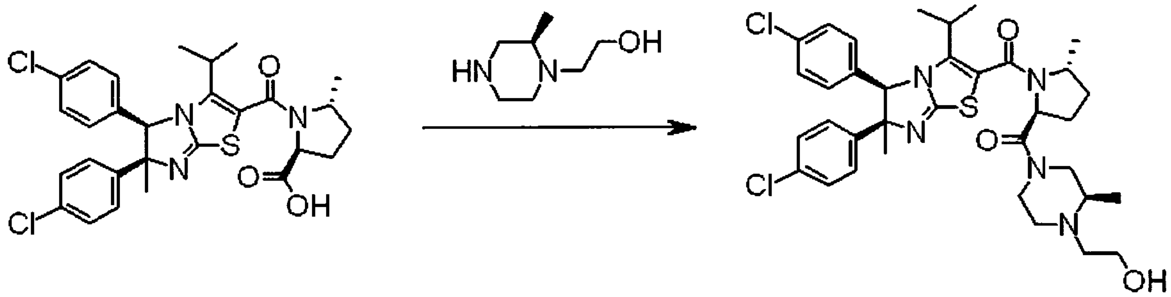

2-{(2R)-4-[(5R)-1-{[(5R,6S)-5,6-Bis(4-chlorophenyl)-3-isopropyl-6-methyl-5,6-dihydroimidazo[2,1-b][1,3]thiazol-2-yl] carbonyl}-5-methyl-L-prolyl]-2-methylpiperazin-1-yl}ethanol

The compound obtained in Step 2 of Example 126 was reacted in the same way as in Step 4 of Example 1 using the compound obtained in Step 2 of Reference Example 65 instead of piperazin-2-one to give the title compound.
$^1$H-NMR ($CDCl_3$) δ: 0.88 (2H, t, J=7.0 Hz), 0.96 (3H, d, J=6.8 Hz), 1.02 (3H, d, J=7.1 Hz), 1.06 (1H, d, J=6.1 Hz), 1.12-1.15 (1H, m), 1.23-1.30 (4H, m), 1.48-1.80 (8H, m), 2.06-2.71 (5H, m), 2.99-3.11 (2H, m), 3.61-3.67 (3H, m), 4.54-4.56 (1H, m), 4.94 (1H, s), 4.98-5.00 (1H, m), 6.69 (2H, d, J=8.0 Hz), 7.00-7.03 (4H, m), 7.13 (2H, d, J=8.3 Hz).
MS (FAB) m/z: 684.

Example 205

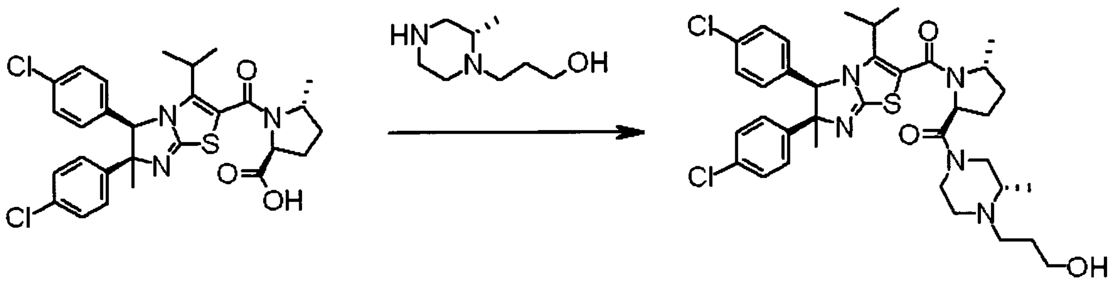

3-{(2S)-4-[(5R)-1-{[(5R,6S)-5,6-Bis(4-chlorophenyl)-3-isopropyl-6-methyl-5,6-dihydroimidazo[2,1-b][1,3]thiazol-2-yl] carbonyl}-5-methyl-L-prolyl]-2-methylpiperazin-1-yl}propan-1-ol

The compound obtained in Step 2 of Example 126 was reacted in the same way as in Step 4 of Example 1 using the compound obtained in Step 3 of Reference Example 66 instead of piperazin-2-one to give the title compound. $^1$HNMR (DMSO-$d_6$, 100°C) δ: 0.86 (3H, d, J=7.1 Hz), 0.96 (3H, d, J=7.1 Hz), 1.01 (3H, d, J=5.3 Hz), 1.16 (3H, d, J=6.3 Hz), 1.55-1.62 (3H, m), 1.69-1.78 (1H, m), 1.73 (3H, s), 2.03-2.38 (3H, m), 2.40-2.54 (6H, m), 2.63-2.81 (2H, m), 3.47 (2H, t, J=6.2 Hz), 3.66 (2H, dd, J=12.9, 2.2 Hz), 4.27-4.39 (1H, m), 4.96 (1H, dd, J=8.5, 2.2 Hz), 5.38 (1H, s), 6.88 (2H, d, J=8.3 Hz), 7.03-7.13 (4H, m), 7.23-7.28 (2H, m).
MS (ESI) m/z: 698, 700.

Example 206

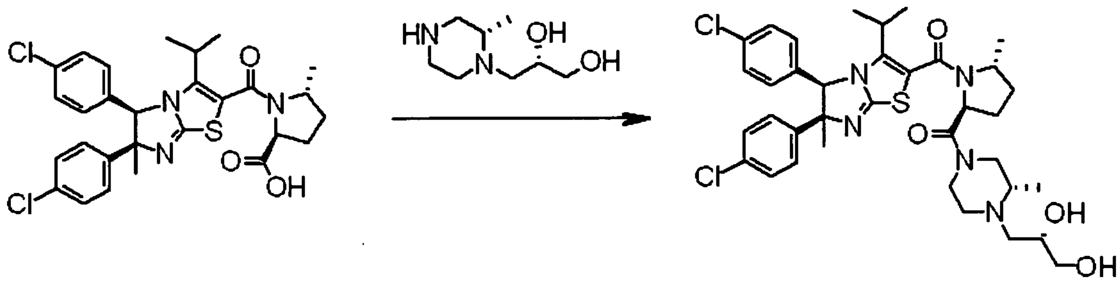

(2S)-3-{(2S)-4-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-2-methylpiperazin-1-yl}propane-1,2-diol

The compound obtained in Step 2 of Example 126 was reacted in the same way as in Step 4 of Example 1 using the compound obtained in Step 2 of Reference Example 67 instead of piperazin-2-one to give the title compound. $^1$HNMR (DMSO-$d_6$, 100°C) δ: 0.86 (3H, d, J=7.1 Hz), 0.96 (3H, d, J=7.1 Hz), 1.01 (3H, d, J=4.9 Hz), 1.16 (3H, d, J=6.3 Hz), 1.51-1.62 (1H, m), 1.70-1.79 (1H, m), 1.73 (3H, s), 2.03-2.13 (1H, m), 2.22-2.40 (2H, m), 2.46-2.58 (1H, m), 2.62-2.75 (1H, m), 2.83-3.01 (5H, m), 3.18-3.44 (3H, m), 3.60-3.66 (3H, m), 4.03 (1H, brs), 4.27-4.37 (1H, m), 4.96 (1H, dd, J=8.7, 2.1 Hz), 5.38 (1H, s), 6.88 (2H, d, J=8.3 Hz), 7.03-7.12 (4H, m), 7.25 (2H, d, J=8.3 Hz).
MS (ESI) m/z: 714, 716.

Example 207

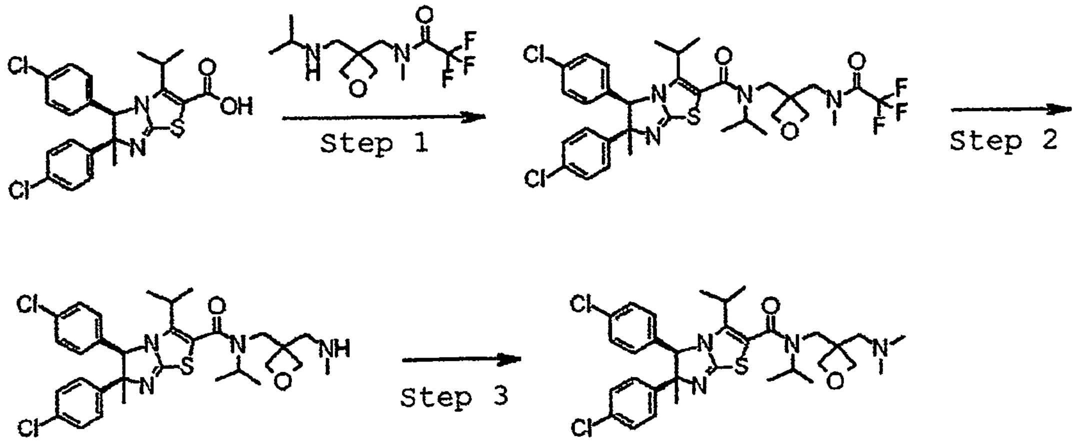


Step 1: (5R,6S)-5,6-Bis(4-chlorophenyl)-N,3-diisopropyl-6-methyl-N-[(3-{[methyl(trifluoroacetyl)amino]methyl}oxetan-3-yl)methyl]-5,6-dihydroimidazo[2,1-b][1,3]thiazole-2-carboxamide

The compound obtained in Step 3 of Example 1 was reacted in the same way as in Example 112 using the compound obtained in Step 5 of Reference Example 68 instead of the compound obtained in Step 2 of Reference Example 18 to give the title compound.
$^1$H-NMR ($CDCl_3$) δ: 0.91 (3H, d, J=7.1 Hz), 0.97 (3H, d, J=7.1 Hz), 1.22 (3H, d, J=6.6 Hz), 1.30 (3H, d, J=6.6 Hz), 1.82 (3H, s), 2.49-2.56 (1H, m), 3.29 (1H, d, J=13.9 Hz), 3.37 (3H, s), 3.64 (1H, d, J=13.9 Hz), 3.75 (1H, d, J=9.0 Hz), 4.38-4.49 (3H, m), 4.59 (1H, d, J=7.1 Hz), 4.65 (1H, d, J=7.1 Hz), 4.96 (1H, s), 6.71 (2H, d, J=7.3 Hz), 7.01-7.12 (6H, m).
MS (ESI) m/z: 697, 699.

Step 2: (5R,6S)-5,6-Bis(4-chlorophenyl)-N,3-diisopropyl-6-methyl-N-({3-[(methylamino)methyl]oxetan-3-yl}methyl)-5,6-dihydroimidazo[2,1-b][1,3]thiazole-2-carboxamide

The compound obtained in Step 1 above was reacted in the same way as in Step 2 of Example 193 to give the title compound.
$^1$H-NMR ($CDCl_3$) δ: 0.92 (3H, d, J=7.1 Hz), 0.94 (3H, d, J=7.1 Hz), 1.20 (3H, d, J=6.6 Hz), 1.27 (3H, d, J=6.6 Hz), 1.82 (3H, s), 2.49 (3H, s), 2.52-2.57 (1H, m), 2.86 (2H, s), 3.33 (1H, d, J=14.2 Hz), 3.45 (1H, d, J=14.2 Hz), 4.34-4.43 (3H, m), 4.57 (1H, d, J=6.3 Hz), 4.62 (1H, d, J=6.3 Hz), 4.96 (1H, s), 6.71 (2H, d, J=7.8 Hz), 7.00-7.11 (6H, m).
MS (ESI) m/z: 601, 603.

Step 3: (5R,6S)-5,6-Bis(4-chlorophenyl)-N-({3-[(dimethylamino)methyl]oxetan-3-yl}methyl)-N,3-diisopropyl-6-methyl-5,6-dihydroimidazo[2,1-b][1,3]thiazole-2-carboxamide

The compound obtained in Step 2 above was reacted in the same way as in Step 3 of Example 152 to give the title compound.
$^1$H-NMR ($CDCl_3$) δ: 0.94 (3H, d, J=7.0 Hz), 0.95 (3H, d, J=7.0 Hz), 1.22 (3H, d, J=6.6 Hz), 1.30 (3H, d, J=6.6 Hz), 1.82 (3H, s), 2.22 (6H, s), 2.55-2.59 (1H, m), 2.70 (2H, s), 3.38 (1H, d, J=6.3 Hz), 3.48 (1H, d, J=6.3 Hz), 4.38 (2H, d, J=6.3 Hz), 4.42-4.45 (1H, m), 4.59 (1H, d, J=6.3 Hz), 4.65 (1H, d, J=6.3 Hz), 4.97 (1H, s), 6.70 (2H, d, J=7.3 Hz), 7.01-7.11 (6H, m).
MS (ESI) m/z: 615, 617.

Example 208

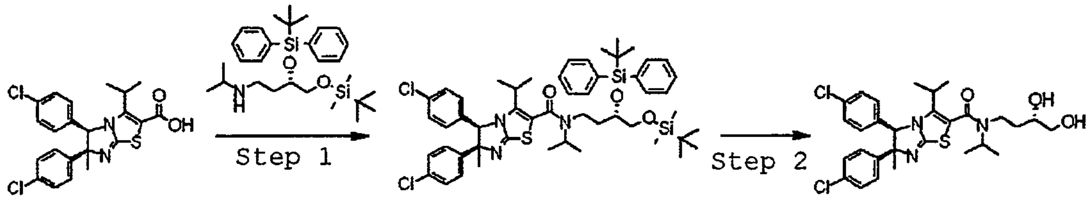


Step 1: (5R,6S)-N-[(3S)-4-{[tert-Butyl(dimethyl)silyl]oxy}-3-{[tert-butyl(diphenyl)silyl]oxy}butyl]-5,6-bis(4-chlorophenyl)-N,3-diisopropyl-6-methyl-5,6-dihydroimidazo[2,1-b][1,3]thiazole-2-carboxamide

The compound obtained in Step 3 of Example 1 was reacted in the same way as in Example 112 using the compound obtained in Reference Example 69 instead of the compound obtained in Step 2 of Reference Example 18 to give the title compound.
$^1$H-NMR ($CDCl_3$) δ: -0.15 (3H, s), -0.11 (3H, s), 0.80 (9H, s), 0.86 (3H, d, J=7.1 Hz), 1.01 (3H, d, J=7.1 Hz), 1.07 (9H, s), 1.12 (3H, d, J=6.8 Hz), 1.15 (3H, d, J=6.8 Hz), 1.72-1.79 (1H, m), 1.81 (3H, s), 1.94-2.01 (1H, m), 2.29-2.36 (1H, m), 3.23-3.29 (2H, m), 3.39-3.48 (2H, m), 3.76-3.81 (1H, m), 4.25-4.31 (1H, m), 4.91 (1H, s), 6.69 (2H, d, J=7.3 Hz), 7.01-7.11 (6H, m), 7.30-7.44 (6H, m), 7.68-7.71 (4H, m).
MS (ESI) m/z: 928, 930.

Step 2: (5R,6S)-5,6-Bis(4-chlorophenyl)-N-[(3S)-3,4-dihydroxybutyl]-N,3-diisopropyl-6-methyl-5,6-dihydroimidazo [2,1-b][1,3]thiazole-2-carboxamide

The compound obtained in Step 1 above was reacted in the same way as in Step 2 of Example 177 to give the title compound.
$^1$H-NMR ($CDCl_3$) δ: 0.91 (3H, d, J=6.9 Hz), 0.99 (3H, d, J=6.9 Hz), 1.19 (3H, d, J=6.6 Hz), 1.28 (3H, d, J=6.6 Hz), 1.82 (3H, s), 2.46-2.53 (1H, m), 3.58 (2H, AB type d, J=14.4 Hz), 3.77-3.82 (3H, m), 4.37 (2H, d, J=6.3 Hz), 4.51 (2H, t, J=6.1 Hz), 4.96 (1H, s), 6.70 (2H, d, J=7.8 Hz), 7.01-7.10 (6H, m).
MS (ESI) m/z: 576, 578.

Example 209

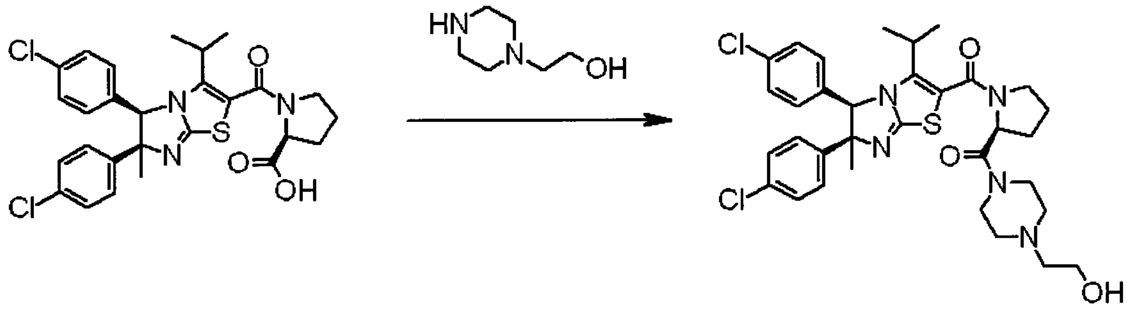

2-[4-(1-{[(5R,6S)-5,6-Bis(4-chlorophenyl)-3-isopropyl-6-methyl-5,6-dihydroimidazo[2,1-b][1,3]thiazol-2-yl]carbonyl}-L-prolyl)piperazin-1-yl]ethanol

The compound obtained in Step 2 of Example 61 was reacted in the same way as in Step 4 of Example 1 using 1-(2-hydroxyethyl)piperazine instead of piperazin-2-one to give the title compound.
$^1$HNMR (DMSO-$d_6$, 100°C) δ: 0.89 (6H, d, J=7.1 Hz), 1.62-2.04 (3H, m), 1.73 (3H, s), 2.18-2.32 (1H, m), 2.39-2.53 (6H, m), 2.58-2.69 (1H, m), 3.41-3.63 (8H, m), 4.00 (1H, t, J=5.4 Hz), 4.87 (1H, dd, J=8.4, 4.5 Hz), 5.38 (1H, s), 6.88 (2H, d, J=8.3 Hz), 7.03-7.15 (4H, m), 7.24 (2H, dt, J=8.3, 2.1 Hz).
MS (ESI) m/z: 656, 658.

Example 210

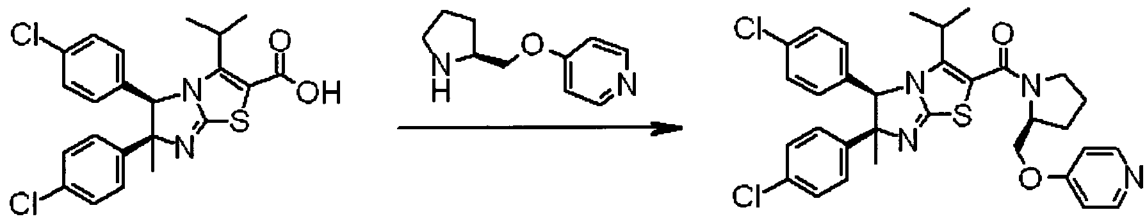

(5R,6S)-5,6-Bis(4-chlorophenyl)-3-isopropyl-6-methyl-2-({(2S)-2-[(pyridin-4-yloxy)methyl]pyrrolidin-1-yl}carbonyl)-5,6-dihydroimidazo[2,1-b][1,3]thiazole

The compound obtained in Step 3 of Example 1 was reacted in the same way as in Step 4 of Example 1 using the compound obtained in Reference Example 70 instead of piperazin-2-one to give the title compound.
$^1$H-NMR ($CDCl_3$) δ: 0.90 (3H, d, J=7.1 Hz), 0.99 (3H, d, J=7.1 Hz), 1.79 (3H, s), 1.89 (1H, m), 2.03-2.20 (3H, m), 2.53 (1H, m), 3.63-3.66 (2H, m), 4.19-4.20 (2H, m), 4.47 (1H, m), 4.93 (1H, s), 6.67-6.69 (2H, m), 6.81 (2H, dd, J=4.6, 1.5 Hz), 7.01 (4H, d, J=8.5 Hz), 7.09 (2H, d, J=8.5 Hz), 8.41 (2H, dd, J=4.6, 1.5 Hz).

Example 211

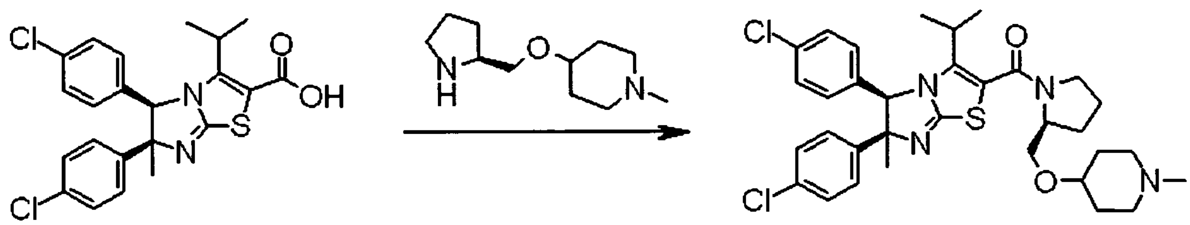

(5R,6S)-5,6-Bis(4-chlorophenyl)-3-isopropyl-6-methyl-2-{[(2S)-2-{[(1-methylpiperidin-4-yl)oxy]methyl}pyrrolidin-1-yl] carbonyl}-5,6-dihydroimidazo[2,1-b][1,3]thiazole

The compound obtained in Step 3 of Example 1 was reacted in the same way as in Step 4 of Example 1 using the compound obtained in Step 2 of Reference Example 71 instead of piperazin-2-one to give the title compound.
$^1$H-NMR ($CDCl_3$, 60°C) δ: 0.96 (3H, d, J=7.1 Hz), 1.04 (3H, d, J=7.1 Hz), 1.56-1.66 (2H, m), 1.76-1.87 [6H, m (include 3H, s) ], 1.99-2.08 (3H, m), 2.12-2.17 (2H, m), 2.25 (3H, s), 2.52-2.64 (3H, m), 3.28 (1H, m), 3.53-3.67 (4H, m), 4.28 (1H, m), 4.93 (1H, s), 6.71 (2H, d, J=8.5 Hz), 7.02 (2H, d, J=8.5), 7.03 (2H, d, J=8.5 Hz), 7.11 (2H, d, J=8.5 Hz).
MS (ESI) m/z: 627.

Example 212

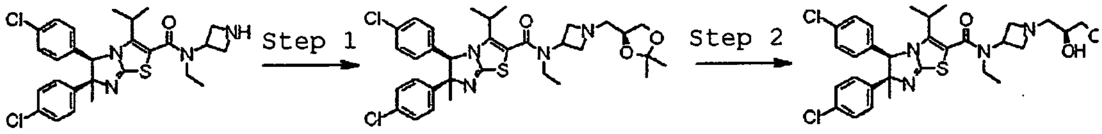


Step 1: (5R,6S)-5,6-Bis(4-chlorophenyl)-N-(1-{[(4S)-2,2-dimethyl-1,3-dioxolan-4-yl]methyl}azetidin-3-yl)-N-ethyl-3-isopropyl-6-methyl-5,6-dihydroimidazo[2,1-b][1,3]thiazole-2-carboxamide

The compound obtained in Step 2 of Example 195 was reacted in the same way as in Step 3 of Example 152 using 4R)-2,2-dimethyl-1,3-dioxolane-4-carbaldehyde instead of 35% aqueous formaldehyde solution to give the title compound.
$^1$H-NMR ($CDCl_3$) δ: 0.87 (3H, d, J=7.1 Hz), 1.00 (3H, d, J=7.1 Hz), 1.16 (3H, t, J=7.0 Hz), 1.36 (3H, s), 1.43 (3H, s), 1.82 (3H, s), 2.43 (1H, m), 2.59-2.68 (2H, m), 3.06-3.13 (2H, m), 3.52-3.64 (3H, m), 3.77-3.82 (3H, m), 4.24 (1H, m), 4.64 (1H, m), 4.94 (1H, s), 6.72 (2H, d, J=8.1 Hz), 7.00-7.10 (6H, m).

Step 2: (5R,6S)-5,6-Bis(4-chlorophenyl)-N-{1-[(2S)-2,3-dihydroxypropyl]azetidin-3-yl}-N-ethyl-3-isopropyl-6-methyl-5,6-dihydroimidazo[2,1-b][1,3]thiazole-2-carboxamide

1 N aqueous hydrochloric acid solution (5 ml) was added to a methanol (1 ml) solution of the compound (322 mg, 0.50 mmol) obtained in Step 1 above under ice cooling and then the temperature of the resulting mixture was warmed to room temperature. After the reaction was completed, the reaction mixture was added into an ice cooled 1 N aqueous solution of sodium hydroxide and extracted with chloroform. The organic layer was washed with brine and then dried over anhydrous sodium sulfate. The solvent was evaporated under reduced pressure and diethyl ether and hexane were added to the residue, and the resulting solid was collected by filtration and dried to give the title compound (233 mg, 77%) as a colorless solid.
$^1$H-NMR ($CDCl_3$) δ: 0.86 (3H, d, J=7.1 Hz), 0.99 (3H, d, J=7.1 Hz), 1.16 (3H, t, J=7.1 Hz), 1.81 (3H, s), 2.44 (1H, m), 2.57 (1H, dd, J=12.1, 3.8 Hz), 2.72 (1H, dd, J=12.1, 7.2 ,Hz) , 3.13 (1H, t, J=7.4 Hz), 3.18 (1H, t, J=7.4 Hz), 3.50-3.61 (3H, m), 3.63-3.72 (3H, m), 3.80 (1H, m), 4.57 (1H, m), 4.94 (1H, s), 6.67-6.73 (2H, m), 7.00-7.05 (4H, m), 7.07-7.10 (2H, m).
MS (ESI) m/z: 603, 605.

Example 213

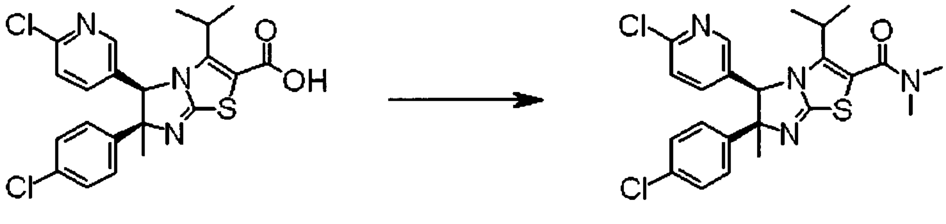

(5R,6S)-6-(4-Chlorophenyl)-5-(6-chloropyridin-3-yl)-3-isopropyl-N,N,6-trimethyl-5,6-dihydroimidazo[2,1-b]thiazole-2-carboxamide

The compound obtained in Step 14 of Reference Example 72 was reacted in the same way as in Step 4 of Example 1 using dimethylamine instead of piperazin-2-one to give the title compound.
$^1$H-NMR ($CDCl_3$) δ: 0.94 (3H, d, J=7.3 Hz), 1.03 (3H, d, J=7.1 Hz), 1.84 (3H, s), 2.39-2.48 (1H, m), 3.07 (6H, s), 4.99 (1H, s), 6.99-7.02 (2H, m), 7.08 (2H, d, J=8.5 Hz), 7.12 (2H, d, J=8.8 Hz), 7.90 (1H, s).
MS (EI) m/z: 475, 497.

Example 214

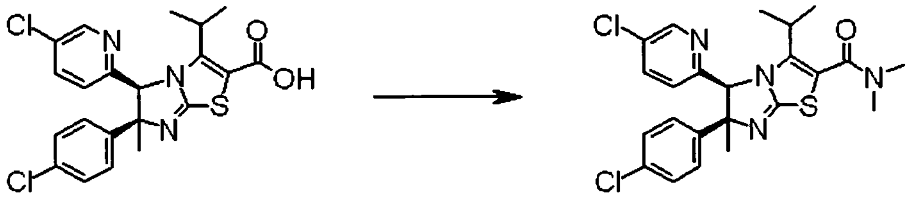

(5S,6S)-5-(5-Bromopyridin-2-yl)-6-(4-chlorophenyl)-3-isopropyl-N,N,6-trimethyl-5,6-dihydroimidazo[2,1-b][1,3]thiazole-2-carboxamide

The compound obtained in Step 13 of Reference Example 73 was reacted in the same way as in Step 4 of Example 1 using a 2 N dimethylamine/tetrahydrofuran solutions instead of piperazin-2-one to give the title compound. $^1$H-NMR ($CDCl_3$) δ: 0.93 (3H, d, J=7.3 Hz), 0.95 (3H, d, J=7.3 Hz), 1.84 (3H, s), 2.42-2.54 (1H, m), 3.07 (6H, s), 5.18 (1H, s), 6.62 (1H, d, J=8.3 Hz), 7.01-7.06 (2H, m), 7.14-7.20 (2H, m), 7.48 (1H, dd, J=8.3, 2.2 Hz), 8.37 (1H, d, J=2.2 Hz). MS (ESI) m/z: 519.

Example 215

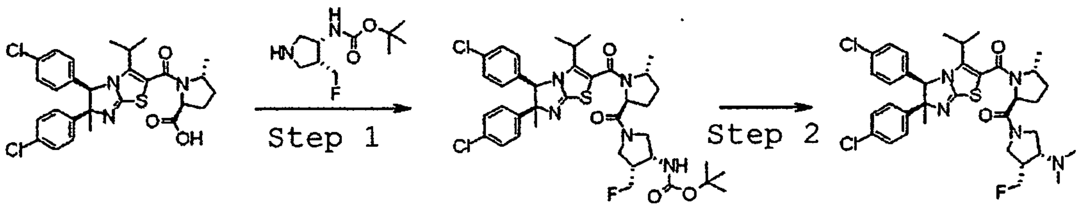


Step 1: tert-Butyl [(3R,4R)-1-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-4-(fluoromethyl)pyrrolidin-3-yl]carbamate

The compound obtained in Step 2 of Example 126 was reacted in the same way as in Step 4 of Example 1 using tert-butyl [(3R,4R)-4-(fluoromethyl)pyrrolidin-3-yl]carbamate instead of piperazin-2-one to give the title compound.

Step 2: (3R,4R)-1-[(5R)-1-{[5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-4-(fluoromethyl)-N,N-dimethylpyrrolidin-3-amine

The compound (1.14 g, 1.46 mmol) obtained in Step 1 above was dissolved in 1,4-dioxane (5 ml) followed by the addition of a anisole (179 μL, 1.65 mmol) and 4 N hydrochloric acid/1,4-dioxane solution (10 ml), and the resulting mixture was stirred at room temperature for 40 minutes. After the solvent was evaporated, the residue was dissolved in methanol (20 ml) followed by the addition of a 37% formaldehyde aqueous solution (440 μL, 5.84 mmol) and acetic acid (334 μL, 5.84 mmol) and the resulting mixture was stirred at 0°C for 10 minutes. Subsequently, sodium cyanoborohydride (367 mg, 5.84 mmol) was added and the temperature of the resulting mixture was warmed to room temperature and the mixture was stirred for 26 hours. The solvent was evaporated and ethyl acetate and saturated aqueous sodium bicarbonate solution were added to the residue and the mixture was allowed to separate. The organic layer was washed with brine and after the mixture was dried over anhydrous sodium sulfate, the solvent was evaporated. After purified by silica gel column chromatography (chloroform/methanol =100:0 → 20:1), diethyl ether and hexane were added. The deposited solid was collected by filtration to give the title compound (775 mg, 74%) as a white solid.
$^1$H-NMR ($CDCl_3$) δ: 0.87-1.03 (7H, m), 1.23-1.25 (3H, m), 1.56-1.58 (3H, m), 1.67 (1H, brs), 1.80 (3H, s), 1.89 (1H, brs), 2.22-2.24 (6H, m), 2.69-2.70 (3H, m), 3.75-3.78 (2H, m), 4.26-4.75 (4H, m), 4.94 (1H, s), 6.69 (2H, d, J=7.8 Hz), 7.00-7.07

(4H, m), 7.10-7.13 (2H, m).
MS (FAB) m/z: 684.

Example 216

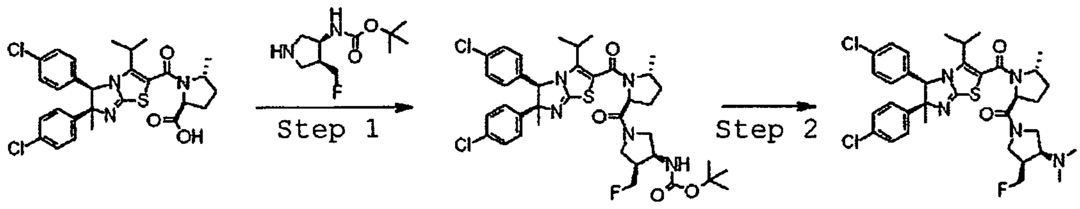


Step 1: tert-Butyl[(3S, 4S)-1-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-4-(fluoromethyl)pyrrolidin-3-yl]carbamate

The compound obtained in Step 2 of Example 126 was reacted in the same way as in Step 4 of Example 1 using tert-butyl [(3S,4S)-4-(fluoromethyl)pyrrolidin-3-yl]carbamate instead of piperazin-2-one to give the title compound.

Step 2: (3S,4S)-1-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-4-(fluoromethyl)-N,N-dimethylpyrrolidin-3-amine

The compound obtained in Step 1 above was reacted in the same way as in Step 2 of Example 216 to give the title compound.
$^1$H-NMR ($CDCl_3$) δ: 0.90-0.99 (6H, m), 1.21-1.26 (3H, m), 1.57-1.60 (4H, m), 1.81-1.85 (4H, m), 2.24 (6H, d, J=12.0 Hz), 2.68-2.69 (1H, m), 3.22-3.99 (5H, m), 4.44-4.69 (4H, m), 4.94 (1H, s), 6.69 (2H, d, J=7.8 Hz), 7.03-7.11 (6H, m) .
MS (FAB) m/z: 684.

Example 217

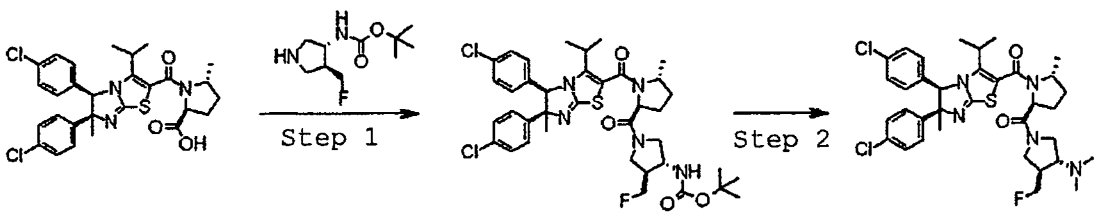


Step 1: tert-Butyl [(3R,4S)-1-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-4-(fluoromethyl)pyrrolidin-3-yl]carbamate

The compound obtained in Step 2 of Example 126 was reacted in the same way as in Step 4 of Example 1 using the compound ((3R,4S)-form) obtained in Step 2 of Reference Example 74 instead of piperazin-2-one to give the title compound.

Step 2: (3R,4S)-1-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-4-(fluoromethyl)-N,N-dimethylpyrrolidin-3-amine

The compound obtained in Step 1 above was reacted in the same way as in Step 2 of Example 216 to give the title compound.
$^1$H-NMR ($CDCl_3$) δ: 0.97 (3H, d, J=7.1 Hz), 1.02 (3H, d, J=7.1 Hz), 1.23 (3H, d, J=6.1 Hz), 1.61-1.64 (3H, m), 1.80 (3H, s), 1.86-1.89 (1H, m), 2.31 (6H, d, J=9.8 Hz), 2.57-2.64 (2H, m), 3.09-3.24 (1H, m), 3.49-3.61 (3H, m), 3.96-4.07 (1H,

m), 4.36-4.55 (3H, m), 4.77-4.80 (1H, m), 4.94 (1H, s), 6.69 (2H, d, J=8.0 Hz), 7.01-7.03 (4H, m), 7.13 (2H, d, J=8.0 Hz). MS (FAB) m/z: 684.

Example 218

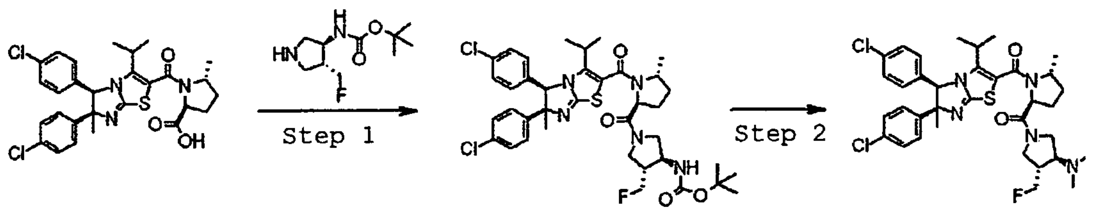


Step 1: tert-Butyl [(3S,4R)-1-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-4-(fluoromethyl)pyrrolidin-3-yl]carbamate

The compound obtained in Step 2 of Example 126 was reacted in the same way as in Step 4 of Example 1 using the compound ((3S,4R)-compound) obtained in Step 2 of Reference Example 74 instead of piperazin-2-one to give the title compound.

Step 2: (3S,4R)-1-[(SR)-1-{[(SR,6S)-S,6-Bis(4-chlorophenyl)-3-isopropyl-6-methyl-5,6-dihydroimidazo[2,1-b][1,3]thiazol-2-yl]carbonyl}-5-methyl-L-prolyl]-4-(fluoromethyl)-N,N-dimethylpyrrolidin-3-amine

The compound obtained in Step 1 above was reacted in the same way as in Step 2 of Example 216 to give the title compound.
$^1$H-NMR ($CDCl_3$) δ: 0.96 (3H, d, J=6.8 Hz), 1.02 (3H, d, J=7.1 Hz), 1.23 (3H, d, J=6.3 Hz), 1.61-1.63 (3H, m), 1.80 (3H, s), 1.86-1.89 (1H, m), 2.28 (3H, s), 2.35 (2H, s), 2.57-2.82 (2H, m), 3.29-3.31 (1H, m), 3.41-3.59 (1H, m), 3.71-3.76 (1H, m), 4.40-4.41 (1H, m), 4.49-4.60 (1H, m), 4.75-4.78 (1H, m), 4.94 (1H, s), 6.69 (2H, d, J=7.3 Hz), 7.02 (4H, dd, J=8.7, 3.3 Hz), 7.10-7.12 (2H, m).
MS (FAB) m/z: 684.

Example 219

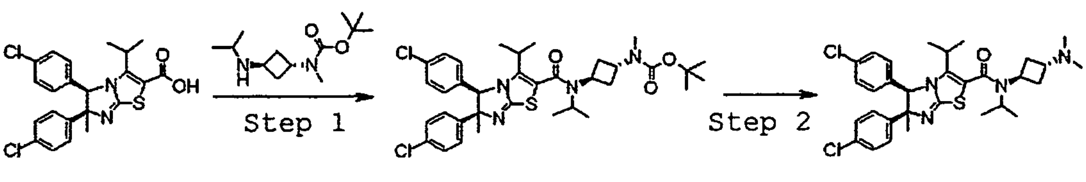


Step 1: tert-Butyl {trans-3-[{[(5R,6S)-5,6-Bis(4-chlorophenyl)-3-isopropyl-6-methyl-5,6-dihydroimidazo[2,1-b][1,3]thiazol-2-yl]carbonyl}(isopropyl)amino]cyclobutyl}methylcarbamate

The compound obtained in Step 3 of Example 1 was reacted in the same way as in Example 112 using the compound obtained in Step 5 of Reference Example 75 instead of the compound obtained in Step 2 of Reference Example 18 to give the title compound.
$^1$H-NMR ($CDCl_3$) δ: 0.90 (3H, d, J=7.0 Hz), 1.07 (3H, d, J=7.0 Hz), 1.22 (3H, d, J=6.6 Hz), 1.28 (3H, d, J=6.6 Hz), 1.48 (9H, s), 1.82 (3H, s), 2.35-2.43 (1H, m), 2.50-2.55 (2H, m), 2.88-2.93 (2H, m), 2.92 (3H, s), 4.10-4.15 (2H, m), 4.67-4.72 (1H, m), 4.93 (1H, s), 6.71 (2H, d, J=7.3 Hz), 7.00-7.12 (6H, m).

MS (ESI) m/z: 671, 673.

Step 2: (5R,6S)-5,6-Bis(4-chlorophenyl)-N-[trans-3-(dimethylamino)cyclobutyl]-N,3-diisopropyl-6-methyl-5,6-dihydroimidazo[2,1-b][1,3]thiazole-2-carboxamide

The compound obtained by the process of Example 1 was reacted in the same way as in Step 2 of Example 183 to give the title compound.
$^1$H-NMR ($CDCl_3$) δ: 0.89 (3H, d, J=7.1 Hz), 1.06 (3H, d, J=7.1 Hz), 1.23 (3H, d, J=6.6 Hz), 1.29 (3H, d, J=6.6 Hz), 1.82 (3H, s), 2.18 (6H, s), 2.16-2.22 (2H, m), 2.33-2.39 (1H, m), 2.72-2.76 (2H, m), 2.85-2.88 (1H, m), 4.05-4.08 (1H, m), 4.20-4.23 (1H, m), 4.92 (1H, s), 6.70 (2H, d, J=7.1 Hz), 7.01-7.11 (6H, m).
MS (ESI) m/z: 585, 587.

Example 220

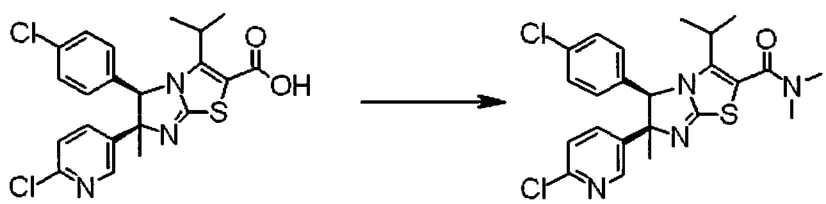

(5R,6S)-5-(4-Chlorophenyl)-6-(6-chloropyridin-3-yl)-3-isopropyl-N,N,6-trimethyl-5,6-dihydroimidazo[2,1-b][1,3]thiazole-2-carboxamide

The compound obtained in Step 9 of Reference Example 38 was reacted in the same way as in Step 4 of Example 1 using dimethylamine instead of piperazin-2-one to give the title compound.
$^1$H-NMR ($CDCl_3$) δ: 0.92 (3H, d, J=7.1 Hz), 1.00 (3H, d, J=7.1 Hz), 1.84 (3H, s), 2.42-2.51 (1H, m), 3.07 (6H, s), 5.00 (1H, s), 6.70-6.75 (2H, m), 7.00 (1H, d, J=8.3 Hz), 7.09 (2H, d, J=8.8 Hz), 7.49 (1H, dd, J=8.4, 2.6 Hz), 8.19 (1H, d, J=2.4 Hz).
MS (ESI) m/z: 475.

Example 221

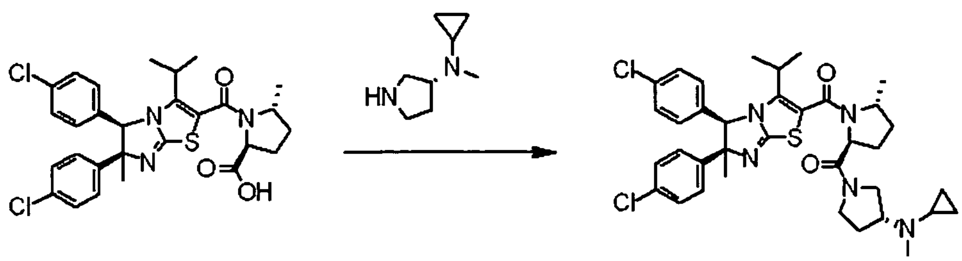

(3R)-1-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-N-cyclopropyl-N-methylpyrrolidin-3-amine

The compound obtained in Step 2 of Example 126 was reacted in the same way as in Step 4 of Example 1 using the compound obtained in Step 2 of Reference Example 76 instead of piperazin-2-one to give the title compound.
$^1$H-NMR ($CDCl_3$) δ: 0.43-0.52 (4H, m), 0.96 (3H, d, J=7.3 Hz), 1.03 (3H, d, J=7.1 Hz), 1.23 (3H, d, J=6.3 Hz), 1.58-1.65

(7H, m), 1.80 (3H, s), 1.88 (1H, brs), 2.29-2.35 (4H, m), 2.69-2.71 (1H, m), 3.20-3.22 (1H, m), 3.56-3.58 (2H, m), 4.55 (1H, t, J=5.9 Hz), 4.77 (1H, brs), 4.94 (1H, s), 6.66-6.69 (2H, m), 7.00-7.03 (4H, m), 7.11-7.14 (2H, m).
MS (FAB) m/z: 680.

Example 222

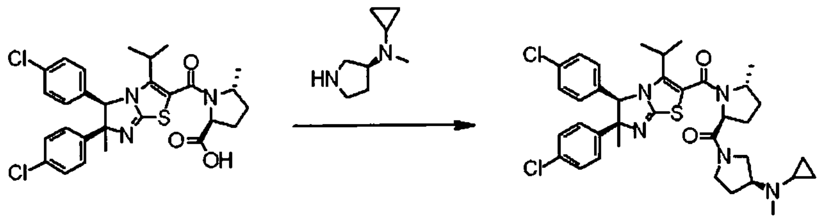

(3S)-1-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-N-cyclopropyl-N-methylpyrrolidin-3-amine

The compound obtained in Step 2 of Example 126 was reacted in the same way as in Step 4 of Example 1 using (3R)-N-cyclopropyl-N-methylpyrrolidin-3-amine instead of piperazin-2-one to give the title compound.
$^1$H-NMR ($CDCl_3$) δ: 0.48-0.54 (4H, m), 0.97 (3H, d, J=6.6 Hz), 1.03 (3H, d, J=6.8 Hz), 1.23 (3H, d, J=6.3 Hz), 1.59-1.62 (6H, m), 1.80 (3H, s), 1.88 (1H, brs), 2.14-2.16 (1H, m), 2.36 (3H, d, J=9.3 Hz), 2.67-2.70 (1H, m), 3.18-3.49 (2H, m), 3.62-3.82 (1H, m), 3.95-4.01 (1H, m), 4.53-4.55 (1H, m), 4.78-4.81 (1H, m), 4.94 (1H, s), 6.69 (2H, d, J=8.1 Hz), 7.01 (4H, dt, J=8.5, 3.2 Hz), 7.10-7.13 (2H, m).
MS (FAB) m/z: 680.

Example 223

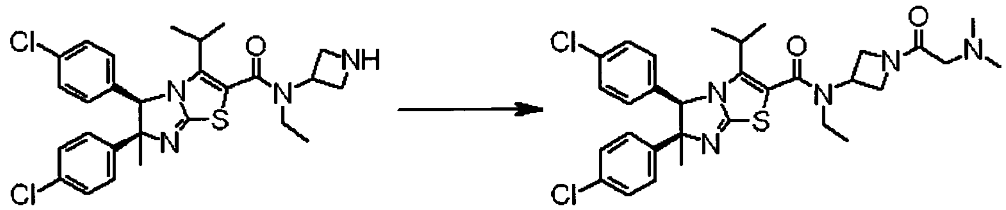

(5R,6S)-5,6-Bis(4-chlorophenyl)-N-[1-(N,N-dimethylglycyl) azetidin-3-yl]-N-ethyl-3-isopropyl-6-methyl-5,6-dihydroimidazo[2,1-b][1,3]thiazole-2-carboxamide

The compound obtained in Step 2 of Example 195 was reacted in the same way as in Step 4 of Example 1 using N,N-dimethylglycine instead of piperazin-2-one to give the title compound.
$^1$H-NMR ($CDCl_3$) δ: 0.87 (3H, d, J=7.1 Hz), 0.99 (3H, d, J=7.1 Hz), 1.21 (3H, t, J=7.0 Hz), 1.81 (3H, s), 2.27 (6H, s), 2.47 (1H, m), 2.98 (2H, s), 3.52 (1H, m), 3.65 (1H, m), 4.12 (1H, m), 4.24-4.30 (2H, m), 4.51 (1H, m), 4.73 (1H, m), 4.95 (1H, s), 6.69-6.71 (2H, m), 7.02-7.08 (6H, m) .
MS (ESI) m/z: 614.

Example 224

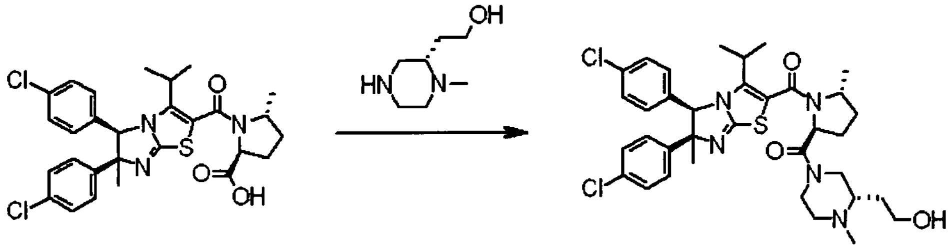

2-{(2S)-4-[(5R)-1-{[(5R,6S)-5,6-Bis(4-chlorophenyl)-3-isopropyl-6-methyl-5,6-dihydroimidazo[2,1-b][1,3]thiazol-2-yl] carbonyl}-5-methyl-L-prolyl]-1-methylpiperazin-2-yl}ethanol

The compound obtained in Step 2 of Example 126 was reacted in the same way as in Step 4 of Example 1 using the compound obtained in Step 2 of Reference Example 77 instead of piperazin-2-one to give the title compound.
$^1$H-NMR (DMSO-$d_6$, 100°C) δ: 0.86 (3H, d, J=7.1 Hz), 0.96 (3H, d, J=7.1 Hz), 1.16 (3H, d, J=6.3 Hz), 1.40-1.64 (2H, m), 1.67-1.82 (2H, m), 1.73 (3H, s), 2.01-2.39 (4H, m), 2.24 (3H, s), 2.62-2.77 (2H, m), 2.88-3.07 (2H, m), 3.52 (2H, brs), 3.68-3.89 (2H, m), 4.12 (1H, brs), 4.25-4.37 (1H, m), 4.95 (1H, dd, J=8.5, 2.2 Hz), 5.38 (1H, s), 6.88 (2H, d, J=8.3 Hz), 7.05-7.11 (4H, m), 7.26 (2H, d, J=8.3 Hz).
MS (ESI) m/z: 684, 686.

Example 225

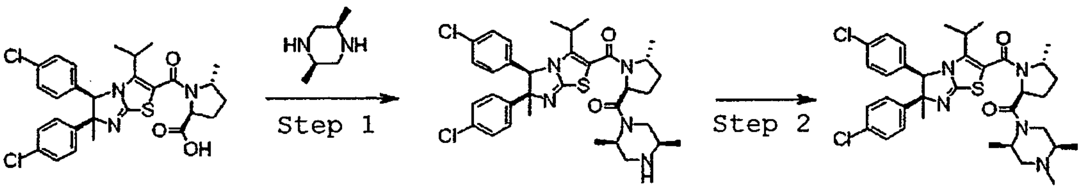


Step 1: (5R,6S)-5,6-Bis(4-chlorophenyl)-2-{[(2S,5R)-2-{[(2R,5R)-2,5-dimethylpiperazin-1-yl]-carbonyl}-5-methylpyrrolidin-1-yl]carbonyl}-3-isopropyl-6-methyl-5,6-dihydroimidazo[2,1-b][1,3]thiazole

The compound obtained in Step 2 of Example 126 was reacted in the same way as in Step 4 of Example 1 using (2R,5R)-2,5-dimethylpiperazine dihydrobromide instead of piperazin-2-one to give the title compound.
$^1$H-NMR ($CDCl_3$) δ: 0.96-1.16 (13H, m), 1.42-1.47 (2H, m), 1.79-1.81 (7H, m), 2.04-2.51 (2H, m), 2.81-2.88 (4H, m), 3.57-3.92 (1H, m), 4.25-4.71 (2H, m), 4.96-5.02 (2H, m), 6.69 (2H, d, J=7.8 Hz), 7.02 (4H, dd, J=8.3, 5.1 Hz), 7.11-7.13 (2H, m).
MS (FAB) m/z: 654.

Step 2: (5R,6R)-5,6-Bis(4-chlorophenyl)-3-isopropyl-6-methyl-2-{[(2R,5S)-2-methyl-5-{[(2R,5R)-2,4,5-trimethylpiperazin-1-yl]carbonyl}pyrrolidin-1-yl]carbonyl}-5,6-dihydroimidazo[2,1-b][1,3]thiazole

The compound obtained in Step 1 above was reacted in the same way as in Step 2 of Example 168 to give the title compound.
$^1$H-NMR ($CDCl_3$) δ: 0.99-1.16 (13H, m), 1.50-1.70 (10H, m), 2.12-2.23 (6H, m), 2.69-2.72 (2H, m), 3.96-4.17 (1H, m), 4.56 (1H, d, J=6.1 Hz), 4.96-5.01 (2H, m), 6.69 (2H, d, J=6.1 Hz), 7.01 (4H, dd, J=8.3, 3.9 Hz), 7.11-7.14 (2H, m).
MS (FAB) m/z: 668.

Example 226

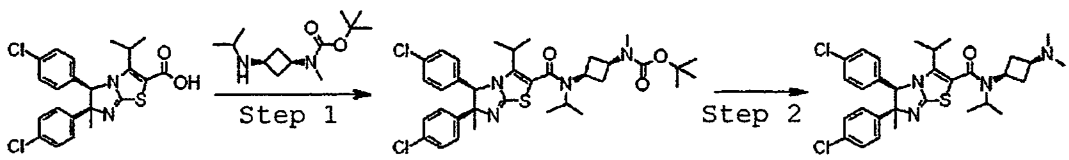


Step 1: tert-Butyl {cis-3-[{[(5R,6S)-5,6-Bis(4-chlorophenyl)-3-isopropyl-6-methyl-5,6-dihydroimidazo[2,1-b][1,3]thiazol-2-yl]carbonyl}(isopropyl)amino]cyclobutyl}methylcarbamate

The compound obtained in Step 3 of Example 1 was reacted in the same way as in Example 112 using the compound obtained in Step 6 of Reference Example 78 instead of the compound obtained in Step 2 of Reference Example 18 to give the title compound.
$^1$H-NMR ($CDCl_3$) δ: 0.89 (3H, d, J=7.0 Hz), 1.06 (3H, d, J=7.0 Hz), 1.25 (3H, d, J=7.0 Hz), 1.30 (3H, d, J=7.0 Hz), 1.46 (9H, s), 1.82 (3H, s), 2.26-2.34 (2H, m), 2.36-2.42 (1H, m), 2.92 (3H, s), 2.95-3.00 (2H, m), 3.58-3.64 (1H, m), 4.07-4.15 (2H, m), 4.93 (1H, s), 6.71 (2H, d, J=7.6 Hz), 7.00-7.11 (6H, m).
MS (ESI) m/z: 671, 673.

Step 2: (5R,6S)-5,6-Bis(4-chlorophenyl)-N-[cis-3-(dimethylamino)cyclobutyl]-N,3-diisopropyl-6-methyl-5,6-dihydroimidazo[2,1-b][1,3]thiazole-2-carboxamide

The compound obtained in the process of Example 1 was reacted in the same way as in Step 2 of Example 183 to give the title compound.
$^1$H-NMR ($CDCl_3$) δ: 0.86 (3H, d, J=7.0 Hz), 1.05 (3H, d, J=7.0 Hz), 1.36 (3H, d, J=6.8 Hz), 1.38 (3H, d, J=6.8 Hz), 1.82 (3H, s), 2.16 (6H, s), 2.17-2.23 (2H, m), 2.33-2.46 (4H, m), 3.85-3.89 (1H, m), 4.04-4.11 (1H, m), 4.93 (1H, s), 6.72 (2H, d, J=7.6 Hz), 7.00-7.12 (6H, m).
MS (ESI) m/z: 585, 587.

Example 227

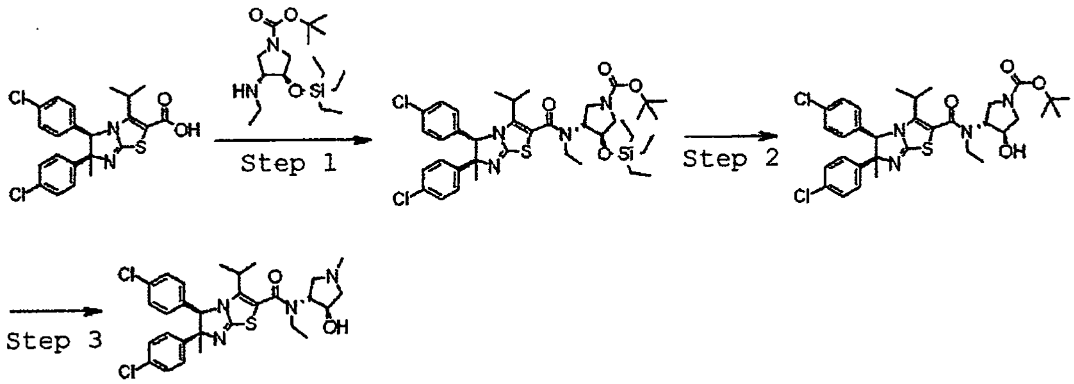


Step 1: tert-Butyl (3R,4R)-3-[{[(5R,6S)-5,6-Bis(4-chlorophenyl)-3-isopropyl-6-methyl-5,6-dihydroimidazo[2,1-b][1,3]thiazol-2-yl]carbonyl}(ethyl)amino]-4-[(triethylsilyl)oxy]pyrrolidine-1-carboxylate

The compound obtained in Step 3 of Example 1 was reacted in the same way as in Example 112 using the compound obtained in Step 2 of Reference Example 79 instead of the compound obtained in Step 2 of Reference Example 18 to give the title compound.
$^1$H-NMR ($CDCl_3$) δ: 0.62 (6H, t, J=7.6 Hz), 0.88-0.97 (15H, m), 1.25 (3H, t, J=7.2 Hz), 1.46 (9H, s), 1.83 (3H, s), 2.30-2.55 (2H, m), 3.07 (2H, q, J=7.2 Hz), 3.37-4.04 (5H, m), 4.94 (1H, s), 6.70 (2H, d, J=6.9 Hz), 7.00-7.11 (6H, m) .
MS (ESI) m/z: 773, 775.

Step 2: tert-Butyl (3R,4R)-3-[{[(5R,6S)-5,6-Bis(4-chlorophenyl)-3-isopropyl-6-methyl-5,6-dihydroimidazo[2,1-b][1,3]thiazol-2-yl]carbonyl}(ethyl)amino]-4-hydroxypyrrolidine-1-carboxylate

The compound obtained in Step 1 above was reacted in the same way as in Step 2 of Example 177 to give the title compound.
$^1$H-NMR ($CDCl_3$) δ: 0.91 (3H, d, J=7.0 Hz), 1.03 (3H, d, J=7.0 Hz), 1.26 (3H, d, J=7.2 Hz), 1.47 (9H, s), 1.83 (3H, s), 2.43 (1H, br), 3.21 (1H, dd, J=10.2, 7.0 Hz), 3.46-3.51 (4H, m), 3.70 (1H, br), 3.80 (1H, br), 4.29 (1H, br), 4.54 (1H, q, J=7.0 Hz), 4.96 (1H, s), 6.71 (2H, d, J=6.9 Hz), 7.00-7.10 (6H, m).
MS (ESI) m/z: 659, 661.

Step 3: (5R,6S)-5,6-Bis(4-chlorophenyl)-N-ethyl-N-[(3R,4R)-4-hydroxy-1-methylpyrrolidin-3-yl]-3-isopropyl-6-methyl-5,6-dihydroimidazo[2,1-b][1,3]thiazole-2-carboxamide

The compound obtained in Step 1 above was reacted in the same way as in Step 2 of Example 183 to give the title compound.
$^1$H-NMR ($CDCl_3$) δ: 0.91 (3H, d, J=7.1 Hz), 1.03 (3H, d, J=7.1 Hz), 1.26 (3H, d, J=7.0 Hz), 1.83 (3H, s), 2.35 (3H, s), 2.40-2.46 (1H, m), 2.59-2.66 (2H, m), 2.88-2.94 (2H, m), 3.53 (2H, q, J=7.0 Hz), 4.20 (1H, br), 4.29 (1H, br), 4.95 (1H, s), 6.70 (2H, d, J=7.8 Hz), 7.00-7.11 (6H, m).
MS (ESI) m/z: 573, 575.

Example 228

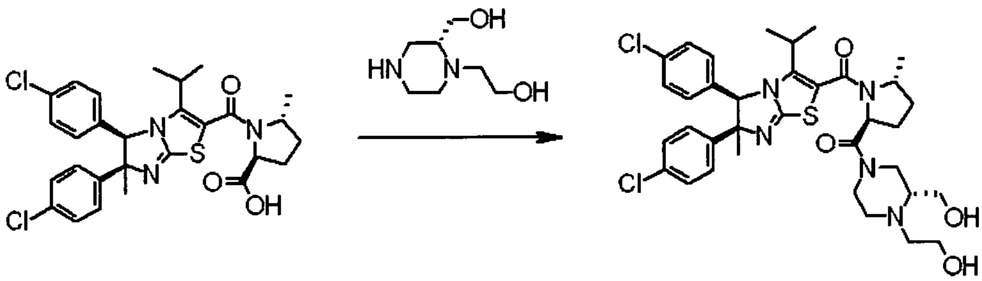

2-[(2R)-4-[(5R)-1-{[(5R,6S)-5,6-Bis(4-chlorophenyl)-3-isopropyl-6-methyl-5,6-dihydroimidazo[2,1-b][1,3]thiazol-2-yl] carbonyl}-5-methyl-L-prolyl]-2-(hydroxymethyl)piperazin-1-yl]ethanol

The compound obtained in Step 2 of Example 126 was reacted in the same way as in Step 4 of Example 1 using the compound obtained in Step 3 of Reference Example 80 instead of piperazin-2-one to give the title compound.
$^1$H-NMR (DMSO-$d_6$, 100°C) δ: 0.87 (3H, d, J=6.8 Hz), 0.96 (3H, d, J=7.3 Hz), 1.17 (3H, d, J=6.1 Hz), 1. 52-1. 63 (1H, m), 1.70-1.81 (1H, m), 1.73 (3H, s), 2.01-2.16 (1H, m), 2.27-2.55 (5H, m), 2.62-2.74 (1H, m), 2.75-2.89 (1H, m), 3.05-3.81 (8H, m), 3.97-4.40 (3H, m), 4.96 (1H, dd, J=8.5, 2.2 Hz), 5.38 (1H, s), 6.88 (2H, d, J=8.3 Hz), 7.03-7.14 (4H, m), 7.26 (2H, d, J=8.3 Hz).
MS (ESI) m/z: 700, 702.

Example 229

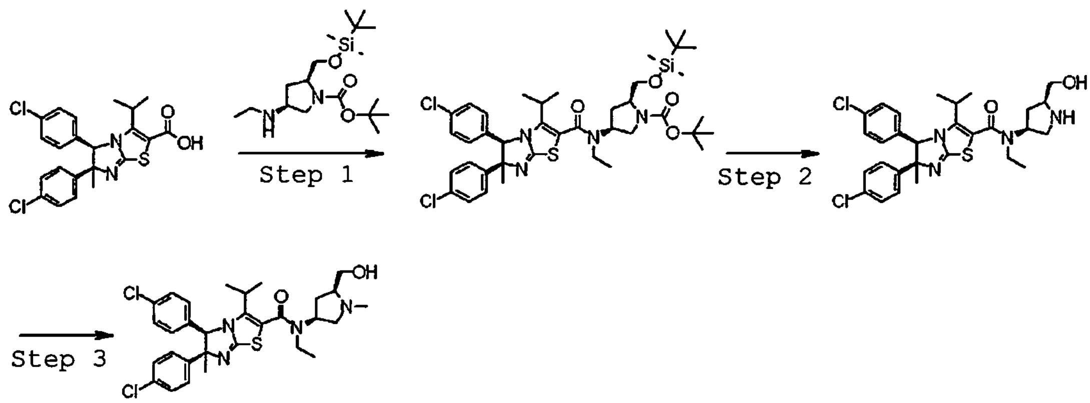


Step 1: tert-Butyl (2S,4S)-4-[{[(5R,6S)-5,6-Bis(4-chlorophenyl)-3-isopropyl-6-methyl-5,6-dihydroimidazo[2,1-b][1,3]thiazol-2-yl]carbonyl}(ethyl)amino]-2-({[tert-butyl(dimethyl)silyl]oxy}methyl)pyrrolidine-1-carboxylate

The compound obtained in Step 3 of Example 1 was reacted in the same way as in Example 112 using the compound obtained in Reference Example 81 instead of the compound obtained in Step 2 of Reference Example 18 to give the title compound.
$^1$H-NMR ($CDCl_3$, 60°C) δ: 0.06 (6H, s), 0.89 (3H, d, J=7.1 Hz), 0.91 (9H, s), 1.03 (3H, d, J=7.1 Hz), 1.20 (3H, t, J=7.0 Hz), 1.47 (9H, s), 1.82 (3H, s), 2.23-2.30 (3H, m), 2.41 (1H, m), 3.15 (1H, m), 3.43-3.44 (2H, m), 3.63 (1H, m), 3.75 (1H, m), 3.91 (1H, m), 4.58 (1H, m), 4.93 (1H, s), 6.69 (2H, d, J=8.3 Hz), 7.00-7.04 (4H, m), 7.09 (3H, d, J=8.5 Hz).
MS (ESI) m/z: 787.

Step 2: (5R,6S)-5,6-Bis(4-chlorophenyl)-N-ethyl-N-[(3S,5S)-5-(hydroxymethyl)pyrrolidin-3-yl]-3-isopropyl-6-methyl-5,6-dihydroimidazo[2,1-b][1,3]thiazole-2-carboxamide

Concentrated hydrochloric acid (3 ml) was added to a compound (256 mg, 0.324 mmol) obtained in Step 1 above. The resulting mixture was added to an ice cooled 1 N aqueous sodium hydroxide solution 30 minutes later, and the deposited solid was collected by filtration, washed with water and then dried to give the title compound (125 mg, 67%) as a colorless solid.
$^1$H-NMR ($CDCl_3$) δ: 0.87 (3H, d, J=7.1 Hz), 1.05 (3H, d, J=7.1 Hz), 1.23 (3H, t, J=7.1 Hz), 1.71-1.77 (2H, m), 1.82 (3H, s), 2.14 (1H, m), 2.39 (1H, m), 2.92 (1H, dd, J=11.2, 7.1 Hz), 3.13 (1H, dd, J=11.2, 8.1 Hz), 3.35 (1H, m), 3.40-3.49 (2H, m), 3.53 (1H, dd, J=11.0, 5.7 Hz), 3.68 (1H, dd, J=11.0, 3.9 Hz), 4.51 (1H, m), 4.94 (1H, s), 6.69-6.74 (2H, m), 7.01-7.05 (4H, m), 7.08-7.10 (2H, m). Step 3: (5R,6S)-5,6-Bis(4-chlorophenyl)-N-ethyl-N-[(3S,5S)-5-(hydroxymethyl)-1-methylpyrrolidin-3-yl]-3-isopropyl-6-methyl-5,6-dihydroimidazo[2,1-b][1,3]thiazole-2-carboxamide

The compound obtained in Step 2 above was reacted in the same way as in Step 3 of Example 152 to give the title compound.
$^1$H-NMR ($CDCl_3$) δ: 0.85 (3H, d, J=7.1 Hz), 1.03 (3H, d, J=7.1 Hz), 1.24 (3H, t, J=7.1 Hz), 1.80 (3H, s), 2.05 (1H, m), 2.24 (1H, m), 2.28 (3H, s), 2.32-2.41 (2H, m), 2.56 (1H, t, J=10.1 Hz), 3.03 (1H, dd, J=10.7, 2.9 Hz), 3.42-3.60 (3H, m), 3.77 (1H, dd, J=11.3, 3.3 Hz), 4.71 (1H, m), 4.92 (1H, s), 6.96-6.71 (2H, m), 7.00-7.03 (4H, m), 7.08 (2H, d, J=8.8 Hz).
MS (ESI) m/z: 587, 589.

Example 230

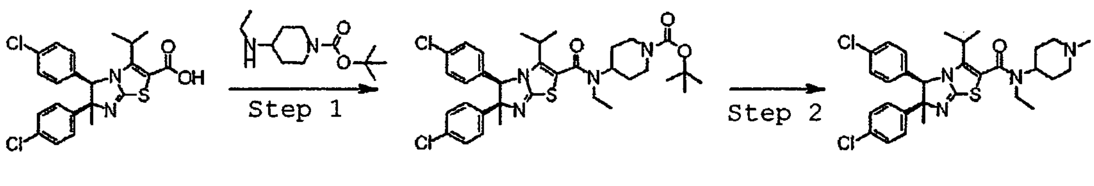

Step 1: tert-Butyl 4-[{[(5R,6S)-5,6-Bis(4-chlorophenyl)-3-isopropyl-6-methyl-5,6-dihydroimidazo[2,1-b][1,3]thiazol-2-yl] carbonyl}(ethyl)amino]piperidine-1-carboxylate

The compound obtained in Step 3 of Example 1 was reacted in the same way as in Example 112 using tert-butyl 4-(ethylamino)piperidine-1-carboxylate instead of the compound obtained in Step 2 of Reference Example 18 to give the title compound.
$^1$H-NMR ($CDCl_3$) δ: 0.88 (3H, d, J=7. 1 Hz), 1.04 (3H, d, J=7.1 Hz), 1.19 (3H, t, J=7.1 Hz), 1.46 (9H, s), 1.61-1.76 (4H, m), 1.79 (3H, s), 2.38 (1H, m), 2.57-2.85 (2H, m), 3.30-3.40 (2H, m), 410 (1H, m), 4.16-4.32 (2H, m), 4.93 (1H, s), 6.67-6.71 (2H, m), 7.00-7.04 (4H, m), 7.10 (2H, d, J=8.5 Hz).
MS (ESI) m/z: 657, 659.

Step 2: (5R,6S)-5,6-Bis(4-chlorophenyl)-N-ethyl-3-isopropyl-6-methyl-N-(1-methylpiperidin-4-yl)-5,6-dihydroimidazo [2,1-b][1,3]thiazole-2-carboxamide

Concentrated hydrochloric acid (3 ml) was added to the compound (265 mg, 0.402 mmol) obtained in Step 1 above. The resulting mixture was added to an ice cooled 1 N aqueous sodium hydroxide solution 30 minutes later, and the deposited solid was collected by filtration, washed with water and then dried. The obtained solid (222 mg) was dissolved in dichloromethane (3 ml) followed by the addition of 35% formalin (0.450 ml). After the resulting mixture was stirred for 1 hour, the reaction mixture was ice cooled and sodium triacetoxyborohydride (101 mg, 0.477 mmol) was added. After the resulting mixture was stirred at room temperature for 2 hours, 1 N aqueous sodium hydroxide solution was added, then the mixture was extracted with chloroform and dried over anhydrous sodium sulfate. The solvent was evaporated under reduced pressure and the residue was purified by silica gel thin layer chromatography (chloroform: methanol = 20:1) to give the title compound (193 mg, 58%) as a colorless solid.
$^1$H-NMR ($CDCl_3$) δ: 0.89 (3H, d, J=7.1 Hz), 1.04 (3H, d, J=7.1 Hz), 1.18 (3H, t, J=7.0 Hz), 1.63 (3H, s), 1.74 (1H, m), 1.81 (3H, s), 1.84-1.95 (3H, m), 2.06 (1H, m), 2.30 (3H, s), 2.36 (1H, m), 2.91-2.98 (2H, m), 3.32-3.42 (2H, m), 3.96 (1H, m), 4.92 (1H, s), 6.96-6.71 (2H, m), 7.01-7.04 (4H, m), 7. 11 (2H, d, J=8.3 Hz).
MS (ESI) m/z: 571, 573.

Example 231

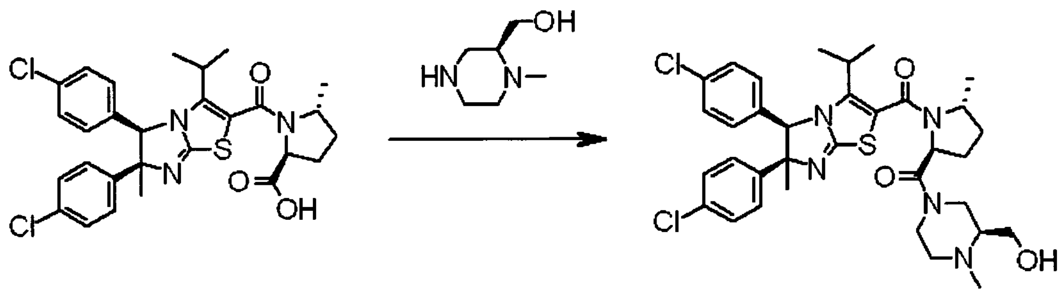

{(2S)-4-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-1-methylpiperazin-2-yl}methanol

The compound obtained in Step 2 of Example 126 was reacted in the same way as in Step 4 of Example 1 using [(2S)-1-methylpiperazin-2-yl]methanol instead of piperazin-2-one to give the title compound.
$^1$H-NMR ($CDCl_3$) δ: 0.97 (3H, d, J=7.1 Hz), 1.02 (3H, d, J=6.8 Hz), 1.24 (3H, t, J=5.4 Hz), 1.68 (1H, brs), 1.79-1.82 (6H, m), 2.24 (1H, brs), 2.38-2.40 (6H, m), 2.68-2.69 (1H, m), 2.87-2.94 (2H, m), 3.34-3.36 (1H, m), 3.55 (1H, brs), 3.73-3.84 (2H, m), 4.54-4.56 (1H, m), 4.95 (1H, s), 5.00-5.03 (1H, m), 6.69 (2H, d, J=6.6 Hz), 7.00-7.03 (4H, m), 7.12-7.14 (2H, m).
MS (FAB) m/z: 670.

Example 232

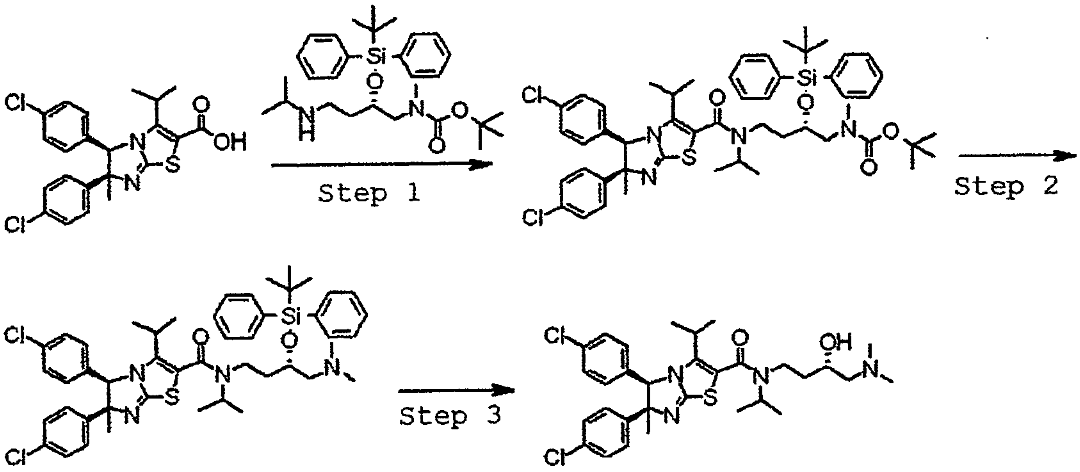


Step 1: tert-Butyl [(2S)-4-[{[(5R,6S)-5,6-Bis(4-chlorophenyl)-3-isopropyl-6-methyl-5,6-dihydroimidazo[2,1-b][1,3]thiazol-2-yl]carbonyl}(isopropyl)amino]-2-{[tert-butyl(diphenyl)silyl]oxy}butyl]methylcarbamate

The compound obtained in Step 3 of Example 1 was reacted in the same way as in Example 112 using the compound obtained in Step 6 of Reference Example 82 instead of the compound obtained in Step 2 of Reference Example 18 to give the title compound.
$^1$H-NMR ($CDCl_3$) δ: -0.15 (3H, s), 0.83 (3H, d, J=6.8 Hz), 1.00 (3H, d, J=6.8 Hz), 1.04 (3H, d, J=7.2 Hz), 1.06 (3H, d, J=7.2 Hz), 1.08 (9H, s), 1.37, 1.41 (total 9H, each s), 1.80 (3H, s), 1.82-1.85 (1H, m), 2.30 (1H, br), 2.56, 2.66 (total 3H, each s), 2.75-3.60 (5H, m), 3.95-3.98 (1H, m), 419 (1H, br), 4.91 (1H, s), 6.69 (2H, d, J=7.3 Hz), 7.00-7.10 (6H, m), 7.38-7.72 (10H, m).

Step 2: (5R,6S)-N-[(3S)-3-{[tert-butyl(diphenyl)silyl]oxy}-4-(dimethylamino)butyl-5,6-bis(4-chlorophenyl)-N,3-diisopropyl-6-methyl-5,6-dihydroimidazo[2,1-b][1,3]thiazole-2-carboxamide

The compound obtained in Step 1 above was reacted in the same way as in Step 2 of Example 183 to give the title compound.
$^1$H-NMR ($CDCl_3$) δ: 0. 88 (3H, d, J=7.1 Hz), 1. 00 (3H, d, J=7.1 Hz), 1.06 (9H, s), 1.15 (3H, d, J=4.4 Hz), 1.17 (3H, d, J=4.4 Hz), 1.72 (1H, br), 1.81 (3H, s), 1.88 (6H, s), 1.96 (1H, br), 2.17-2.39 (3H, m), 3.31-3.35 (2H, m), 3.79-3.83 (1H, m), 4.25-4.31 (1H, m), 4.92 (1H, s), 6.70 (2H, d, J=7.3 Hz), 7.00-7.11 (6H, m), 7.34-7.42 (6H, m), 7.69-7.73 (4H, m).

Step 3: (5R,6S)-5,6-Bis(4-chlorophenyl)-N-[(3S)-4-(dimethylamino)-3-hydroxybutyl]-N,3-diisopropyl-6-methyl-5,6-dihydroimidazo[2,1-b][1,3]thiazole-2-carboxamide

The compound obtained in Step 2 above was reacted in the same way as in Step 2 of Example 177 to give the title compound.
$^1$H-NMR ($CDCl_3$) δ: 0.90 (3H, d, J=7.1 Hz), 1.03 (3H, d, J=7.1 Hz), 1.22 (3H, d, J=6.9 Hz), 1.28 (3H, d, J=6.9 Hz), 1.46-1.52 (4H, m), 1.82 (3H, s), 2.20-2.41 (3H, m), 2.29 (6H, s), 3.43-3.51 (1H, m), 3.67-3.72 (1H, m), 4.94 (1H, s), 6.71 (2H, d, J=7.6 Hz), 7.00-7.11 (6H, m).
MS (ESI) m/z: 603, 605.

Example 233

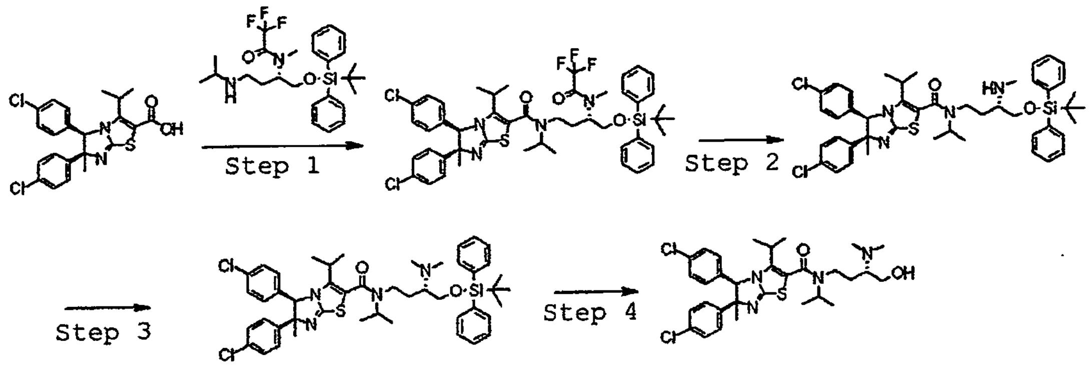


Step 1: (5R,6S)-N-{(3S)-4-{[tert-Butyl(diphenyl)silyl]oxy}-3-[methyl(trifluoroacetyl)amino]butyl-5,6-bis(4-chlorophenyl)-N,3-diisopropyl-6-methyl-5,6-dihydroimidazo[2,1-b][1,3]thiazole-2-carboxamide

The compound obtained in Step 3 of Example 1 was reacted in the same way as in Example 112 using the compound obtained in Step 8 of Reference Example 83 instead of the compound obtained in Step 2 of Reference Example 18 to give the title compound.
$^1$H-NMR ($CDCl_3$) δ: 0.86 (3H, d, J=7.2 Hz), 1.01 (3H, d, J=7.2 Hz), 1.02 (9H, s), 1.14 (3H, d, J=6.9 Hz), 1.21 (3H, d, J=6.9 Hz), 1.81 (3H, s), 1.80-1.90 (2H, m), 2.34-2.37 (1H, m), 3.06 (3H, s), 3.13-3.20 (2H, m), 3.65 (2H, d, J=6.6 Hz), 4.30-4.37 (1H, m), 4.70 (1H, br), 4.92 (1H, s), 6.69 (2H, d, J=7.2 Hz), 7.01-7.12 (6H, m), 7.38-7.63 (10H, m).

Step 2: (5R,6S)-N-[(3S)-4-{[tert-Butyl(diphenyl)silyl]oxy}-3-(methylamino)butyl]-5,6-bis(4-chlorophenyl)-N,3-diisopropyl-6-methyl-5,6-dihydroimidazo[2,1-b][1,3]thiazole-2-carboxamide

The compound obtained in Step 1 above was reacted in the same way as in Step 2 of Example 193 to give the title compound.
$^1$H-NMR ($CDCl_3$) δ: 0.89 (3H, d, J=7.1 Hz), 1.02 (3H, d, J=7.1 Hz), 1.06 (9H, s), 1.16 (3H, d, J=6.6 Hz), 1.23 (3H, d, J=6.6 Hz), 1.69-1.74 (2H, m), 1.81 (3H, s), 2.34 (6H, s), 2.33-2.40 (1H, m), 2.54-2.57 (1H, m), 3.23-3.33 (1H, m), 3.62 (2H, dq, J=10.2, 5.6 Hz), 4.31-4.34 (1H, m), 4.92 (1H, s), 6.70 (2H, d, J=8.0 Hz), 7.00-7.11 (6H, m), 7.36-7.44 (6H, m), 7.64-7.66 (4H, m).

Step 3: (5R,6S)-N-[(3S)-4-{[tert-Butyl(diphenyl)silyl]oxy}-3-(dimethylamino)butyl]-5,6-bis(4-chlorophenyl)-N,3-diisopropyl-6-methyl-5,6-dihydroimidazo[2,1-b][1,3]thiazole-2-carboxamide

The compound obtained in Step 2 above was reacted in the same way as in Step 3 of Example 152 to give the title compound.
$^1$H-NMR ($CDCl_3$) δ: 0.90 (3H, d, J=7.1 Hz), 1.02 (3H, d, J=7.1 Hz), 1.05 (9H, s), 1.17 (3H, d, J=6.6 Hz), 1.24 (3H, d, J=6.6 Hz), 1.74-1.77 (2H, m), 1.81 (3H, s), 2.32 (6H, s), 2.33-2.36 (1H, m), 2.61 (1H, br), 3.17 (1H, br), 3.37 (1, br), 3.60-3.63 (1H, m), 3.73-3.77 (1H, m), 4.92 (1H, s), 6.70 (2H, d, J=7.8 Hz), 7.01-7.11 (6H, m), 7.36-7.45 (6H, m), 7.65-7.67 (4H, m).

Step 4: (5R,6S)-5,6-Bis(4-chlorophenyl)-N-[(3S)-3-(dimethylamino)-4-hydroxybutyl]-N,3-diisopropyl-6-methyl-5,6-dihydroimidazo[2,1-b][1,3]thiazole-2-carboxamide

The compound obtained in Step 3 above was reacted in the same way as in Step 2 of Example 177 to give the title compound.
$^1$H-NMR ($CDCl_3$) δ: 0.90 (3H, d, J=7.1 Hz), 1.02 (3H, d, J=7.1 Hz), 1.20 (3H, d, J=6.6 Hz), 1.26 (3H, d, J=6.6 Hz), 1.43-1.47 (1H, m), 1.66-1.81 (3H, m), 1.82 (3H, s), 2.31 (6H, s), 2.36-2.44 (1H, m), 2.58-2.63 (1H, m), 3.18-3.27 (1H, m), 3.35-3.46 (1H, m), 3.58-3.62 (1H, m), 4.34-4.39 (1H, m), 4.94 (1H, s), 6.71 (2H, d, J=7.6 Hz), 7.00-7.11 (6H, m).
MS (ESI) m/z: 603, 605.

Example 234

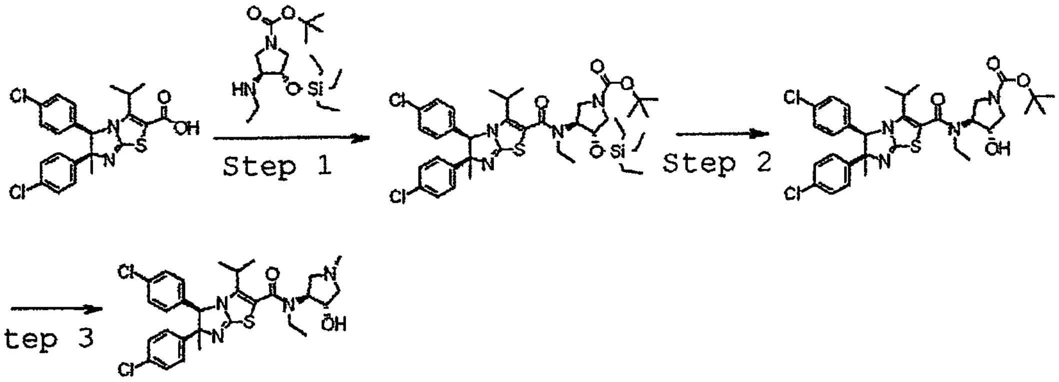


Step 1: tert-Butyl (3S,4S)-3-[{[(5R,6S)-5,6-Bis(4-chlorophenyl)-3-isopropyl-6-methyl-5,6-dihydroimidazo[2,1-b][1,3]thiazol-2-yl]carbonyl}(ethyl)amino]-4-[(triethylsilyl)oxy]pyrrolidine-1-carboxylate

The compound obtained in Step 3 of Example 1 was reacted in the same way as in Example 112 using the compound obtained in Step 2 of Reference Example 84 instead of the compound obtained in Step 2 of Reference Example 18 to give the title compound.
$^1$H-NMR ($CDCl_3$) δ: 0.50-0.63 (6H, m), 0.86-1.01 (15H, m), 1.25 (3H, t, J=7.1 Hz), 1.48 (9H, s), 1.82 (3H, s), 2.46 (2H, br), 3.02-3.07 (2H, m), 3.36-4.13 (5H, m), 4.95 (1H, s), 6.70 (2H, d, J=6.8 Hz), 7.00-7.11 (6H, m).
MS (ESI) m/z: 773, 775.

Step 2: tert-Butyl (3S,4S)-3-[{[(5R,6S)-5,6-Bis(4-chlorophenyl)-3-isopropyl-6-methyl-5,6-dihydroimidazo[2,1-b][1,3]thiazol-2-yl]carbonyl}(ethyl)amino]-4-hydroxypyrrolidine-1-carboxylate

The compound obtained in Step 1 above was reacted in the same way as in Step 2 of Example 177 to give the title compound.
$^1$H-NMR ($CDCl_3$) δ: 0.90 (3H, d, J=7.1 Hz), 1.02 (3H, d, J=7.1 Hz), 1.26 (3H, d, J=7.2 Hz), 1.48 (9H, s), 1.83 (3H, s), 2.41 (1H, q, J=7.1 Hz), 3.19 (1H, dd, J=11.3, 6.7 Hz), 3.40-3.48 (3H, m), 3.75-3.80 (2H, m), 4.31-4.37 (1H, m), 4.51 (1H, br), 4.95 (1H, s), 6.71 (2H, d, J=6.8 Hz), 7.00-7.11 (6H, m).
MS (ESI) m/z: 659, 661.

Step 3: (5R,6S)-5,6-Bis(4-chlorophenyl)-N-ethyl-N-[(3S,4S)-4-hydroxy-1-methylpyrrolidin-3-yl]-3-isopropyl-6-methyl-5,6-dihydroimidazo[2,1-b][1,3]thiazole-2-carboxamide

The compound obtained in Step 2 above was reacted in the same way as in Step 2 of Example 183 to give the title compound.
$^1$H-NMR ($CDCl_3$) δ: 0.93 (3H, d, J=7.1 Hz), 1.03 (3H, d, J=7.1 Hz), 1.25 (3H, d, J=7.1 Hz), 1.82 (3H, s), 2.36 (3H, s), 2.37-2.45 (1H, m), 2.59-2.63 (2H, m), 2.87 (1H, dd, J=9.8, 6.0 Hz), 2.99 (1H, t, J=8.8 Hz), 3.42-3.49 (1H, m), 3.54-3.59 (1H, m), 4.26-4.29 (2H, m), 4.94 (1H, s), 6.69 (2H, d, J=8.0 Hz), 7.00-7.11 (6H, m).
MS (ESI) m/z: 573, 575.

Example 235

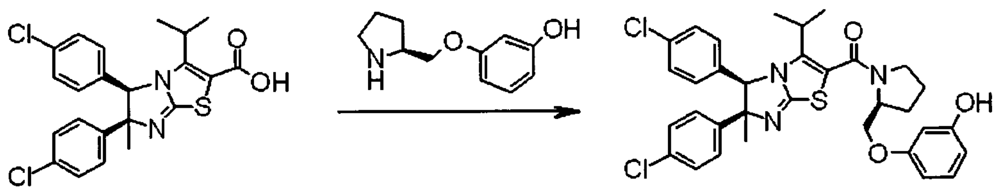

3-{[(2S)-1-{[(5R,6S)-5,6-Bis(4-chlorophenyl)-3-isopropyl-6-methyl-5,6-dihydroimidazo[2,1-b][1,3]thiazol-2-yl]carbonyl} pyrrolidin-2-yl]methoxy}phenol

The compound obtained in Step 3 of Example 1 was reacted in the same way as in Step 4 of Example 1 using the compound obtained in Step 2 of Reference Example 85 instead of piperazin-2-one to give the title compound.
$^1$HNMR (DMSO-$d_6$, 100°C) δ: 0.87 (3H, d, J=6.8 Hz), 0.92 (3H, d, J=7.3 Hz), 1.73 (3H, s), 1.77-1.90 (1H, m), 1.96-2.12 (3H, m), 2.46-2.57 (1H, m), 3.52-3.59 (2H, m), 4.00-4.05 (2H, m), 4.31-4.41 (1H, m), 5.38 (1H, s), 6.30-6.40 (3H, m), 6.87 (2H, d, J=8.1 Hz), 6.98-7.12 (5H, m), 7.24 (2H, d, J=8.1 Hz), 8.98 (1H, brs).
MS (ESI) m/z: 622, 624.

Example 236

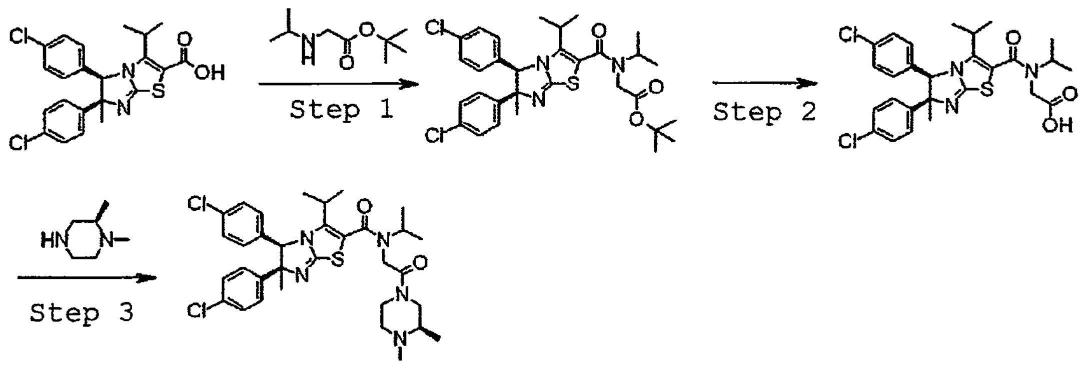


Step 1: tert-Butyl N-{[(5R,6S)-5,6-Bis(4-chlorophenyl)-3-isopropyl-6-methyl-5,6-dihydroimidazo[2,1-b][1,3]thiazol-2-yl] carbonyl}-N-isopropylglycinate

The compound obtained in Step 3 of Example 1 was reacted in the same way as in Example 112 using tert-butyl N-isopropylglycinate instead of the compound obtained in Step 2 of Reference Example 18 to give the title compound.
$^1$H-NMR ($CDCl_3$) δ: 0.92 (3H, d, J=7.1 Hz), 1.01 (3H, d, J=7.1 Hz), 1.18 (3H, d, J=6.6 Hz), 1.23 (3H, d, J=6.6 Hz), 1.48 (9H, s), 1.82 (3H, s), 2.40-2.49 (1H, m), 3.88 (2H, s), 4.40-4.49 (1H, m), 4.95 (1H, s), 6.71 (2H, d, J=7.8 Hz), 7.01-7.06 (4H, m), 7.08-7.12 (2H, m).

Step 2: N-{[(5R,6S)-5,6-Bis(4-chlorophenyl)-3-isopropyl-6-methyl-5,6-dihydroimidazo[2,1-b][1,3]thiazol-2-yl]carbonyl}-N-isopropylglycine

The compound obtained in Step 1 above was reacted in the same way as in Step 2 of Example 126 to give the title compound.
$^1$H-NMR ($CDCl_3$) δ: 0.84 (3H, d, J=6.1 Hz), 1.10 (3H, d, J=6.8 Hz), 1.15-1.23 (6H, m), 2.09 (3H, s), 2.47-2.57 (1H, m), 4.00-4.18 (2H, m), 4.61-4.73 (1H, m), 5.50 (1H, s), 6.65-6.89 (2H, m), 7.02-7.16 (6H, m).

Step 3: (5R,6S)-5,6-Bis(4-chlorophenyl)-N-{2-[(3R)-3,4-dimethylpiperazin-1-yl]-2-oxoethyl}-N,3-diisopropyl-6-methyl-5,6-dihydroimidazo[2,1-b][1,3]thiazole-2-carboxamide

The compound obtained in Step 2 above was reacted in the same way as in Step 4 of Example 1 using (2R)-1,2-dimethylpiperazine instead of piperazin-2-one to give the title compound.
$^1$H-NMR ($CDCl_3$) δ: 0.96 (3H, d, J=7.1 Hz), 1.00 (3H, d, J=7.1 Hz), 1.06, 1.11 (total 3H, each d, J=6.1 Hz), 1.18 (3H, d, J=6.8 Hz), 1.24 (3H, d, J=6.3 Hz), 1.82 (3H, s), 2.11-4.36 (9H, m), 2.30 (3H, s), 2.46-2.57 (1H, m), 4.41-4.52 (1H, m), 4.96 (1H, s), 6.69-6.74 (2H, m), 7.00-7.05 (4H, m), 7.11 (2H, d, J=8.8 Hz).
MS (ESI) m/z: 642.

Example 237

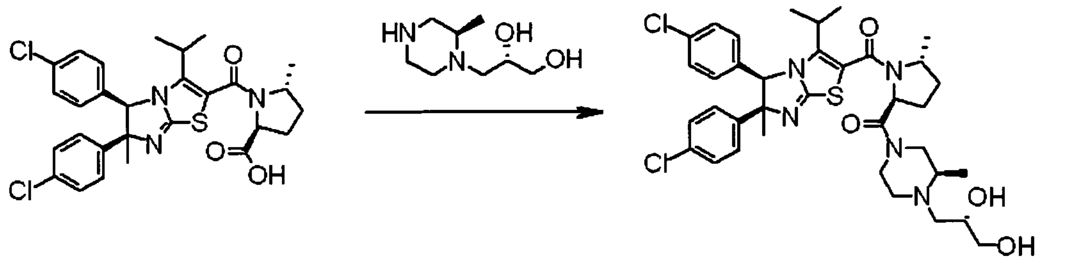

(2S)-3-{(2R)-4-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-2-methylpiperazin-1-yl}propane-1,2-diol

The compound obtained in Step 2 of Example 126 was reacted in the same way as in Step 4 of Example 1 using the compound obtained in Step 2 of Reference Example 86 instead of piperazin-2-one to give the title compound.
$^1$H-NMR ($CDCl_3$) δ: 0.97-1.01 (6H, m), 1.07-1.09 (3H, m), 1.23 (3H, d, J=6.1 Hz), 1.68 (4H, brs), 1.79-1.81 (4H, m), 2.24-2.27 (3H, m), 2.37-2.51 (2H, m), 2.68-2.70 (1H, m), 2.95-2.97 (3H, m), 3.49-3.52 (1H, m), 3.78 (3H, d, J=10.7 Hz), 4.06-4.28 (1H, m), 4.54-4.57 (1H, m), 4.94 (1H, s), 4.99 (1H, brs), 6.69 (2H, d, J=7.8 Hz), 7.02 (4H, dd, J=8.4, 3.3 Hz), 7.13 (2H, d, J=8.5 Hz).
MS (FAB) m/z: 714.

Example 238

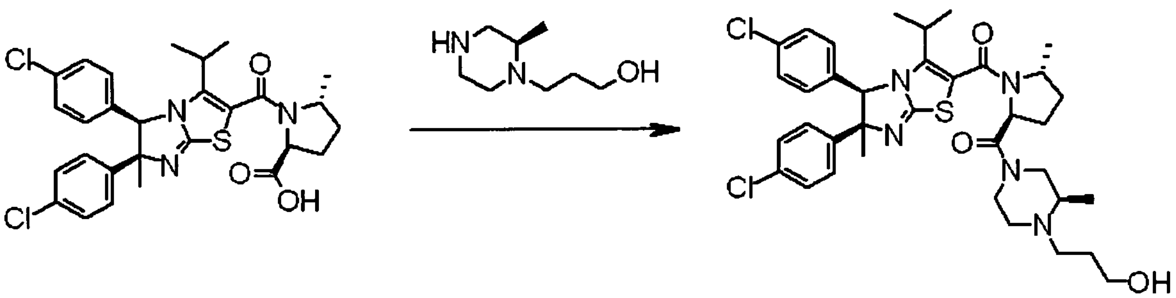

3-{(2R)-4-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-2-methylpiperazin-1-yl}propane-1-ol

The compound obtained in Step 2 of Example 126 was reacted in the same way as in Step 4 of Example 1 using the compound obtained in Step 3 of Reference Example 87 instead of piperazin-2-one to give the title compound.
$^1$H-NMR ($CDCl_3$) δ: 0.90-0.92 (1H, m), 0.96 (3H, d, J=7.1 Hz), 1.02 (3H, d, J=7.1 Hz), 1.08 (2H, d, J=6.1 Hz), 1.15 (1H, d, J=5.1 Hz), 1.23 (3H, d, J=6.3 Hz), 1.65-1.66 (5H, m), 1.79-1.84 (5H, m), 2.21-2.24 (2H, m), 2.41-2.44 (2H, m), 2.66-2.73 (1H, m), 3.01-3.04 (3H, m), 3.58 (1H, brs), 3.79-3.82 (2H, m), 4.55 (1H, t, J=6.2 Hz), 4.94 (1H, s), 4.99 (1H, t, J=9.6 Hz), 6.69 (2H, d, J=7.8 Hz), 7.02 (4H, dd, J=8.7, 2.8 Hz), 7.13 (2H, d, J=8.3 Hz).
MS (FAB) m/z: 698.

Example 239

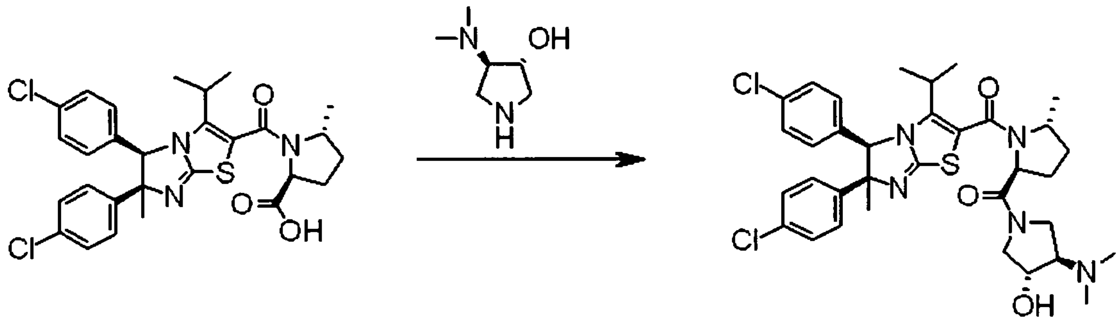

(3R,4R)-1-[(5R)-1-{[(5R,6S)-5,6-Bis(4-chlorophenyl)-3-isopropyl-6-methyl-5,6-dihydroimidazo[2,1-b][1,3]thiazol-2-yl] carbonyl}-5-methyl-L-prolyl]-4-(dimethylamino)pyrrolidin-3-ol

The compound obtained in Step 2 of Example 126 was reacted in the same way as in Step 4 of Example 1 using the compound obtained in Step 2 of Reference Example 88 instead of piperazin-2-one to give the title compound. $^1$H-NMR ($CDCl_3$) δ: 0.85 (3H, d, J=7.1 Hz), 0.95 (3H, d, J=7.1 Hz), 1.15 (3H, d, J=6.3 Hz), 1.55-1.60 (1H, m), 1.71 (3H, s), 1.70-1.73 (1H, m), 2.23 (6H, s), 2.13-2.30 (2H, m), 2.66-2.73 (2H, m), 3.01-3.66 (4H, m), 4.17 (1H, br), 4.32 (1H, t, J=5.1 Hz), 4.74 (1H, br), 4.88 (1H, br), 5.37 (1H, s), 6.86 (2H, d, J=8.6 Hz), 7.04-7.15 (6H, m).
MS (ESI) m/z: 670, 672.

Example 240

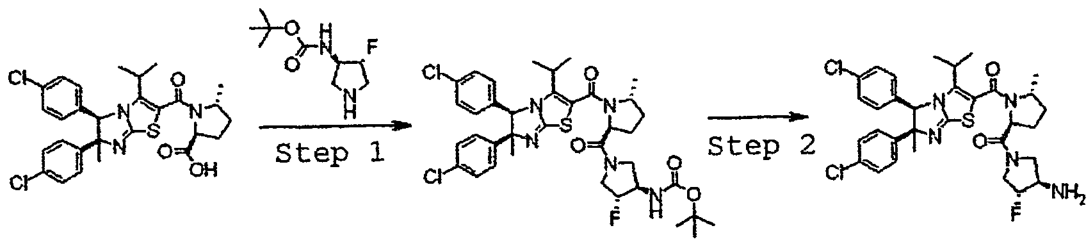


Step 1: tert-Butyl {(3R,4R)-1-[(5R)-1-{[(5R,6S)-5,6-Bis(4-chlorophenyl)-3-isopropyl-6-methyl-5,6-dihydroimidazo[2,1-b] [1,3]thiazol-2-yl]carbonyl}-5-methyl-L-prolyl]-4-fluoropyrrolidin-3-yl}carbamate

The compound obtained in Step 2 of Example 126 was reacted in the same way as in Step 4 of Example 1 using tert-butyl [(3R,4R)-4-fluoropyrrolidin-3-yl]carbamate instead of piperazin-2-one to give the title compound. $^1$H-NMR ($CDCl_3$) δ: 0. 95-1.02 (6H, m), 1.25 (3H, d, J=6.1 Hz), 1.41 (9H, s), 1.65-1.76 (1H, m), 1.80 (3H, s), 1.92-2.05 (2H, m), 2.15-2.30 (1H, m), 2.40-2.67 (2H, m), 3.50-3.90 (3H, m), 4.02-4.38 (2H, m), 4.47-4.65 (2H, m), 4.94 (1H, s), 5.01-5.23 (1H, m), 6.70 (2H, d, J=7.8 Hz), 6.98-7.05 (4H, m), 7.13 (2H, d, J=8.3 Hz).

Step 2: (3R,4R)-1-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-4-fluoropyrrolidin-3-amine

The compound (210 mg, 0.28 mmol) obtained in Step 1 above was dissolved in methanol (5 ml) and 4 N hydrochloric acid/1,4-dioxane solution (10 ml) was added, and the resulting mixture was stirred at room temperature for

4 hours. The solvent was evaporated and saturated aqueous sodium bicarbonate solution was added to the residue. The resulting mixture was extracted with chloroform, washed with brine and dried over anhydrous sodium sulfate. Then the solvent was evaporated and the residue was lyophilized with 1,4-dioxane to give the title compound (169 mg, 93%) as a colorless solid.
$^1$H-NMR ($CDCl_3$) δ: 0.96 (3H, d, J=7.1 Hz), 0.99 (3H, d, J=7.1 Hz), 1.24 (3H, d, J=6.3 Hz), 1.42-1.73 (3H, m), 1.79 (3H, s), 1.84-2.58 (3H, m), 2.59-2.72 (1H, m), 3.28-3.90 (4H, m), 3.98-4.36 (1H, m), 4.48-4.82 (2H, m), 4.82-5.01 (1H, m), 4.94 (1H, s), 6.69 (2H, d, J=8.0 Hz), 6.98-7.05 (4H, m), 7.13 (2H, d, J=8.3 Hz).
MS (FAB) m/z: 644.

Example 241

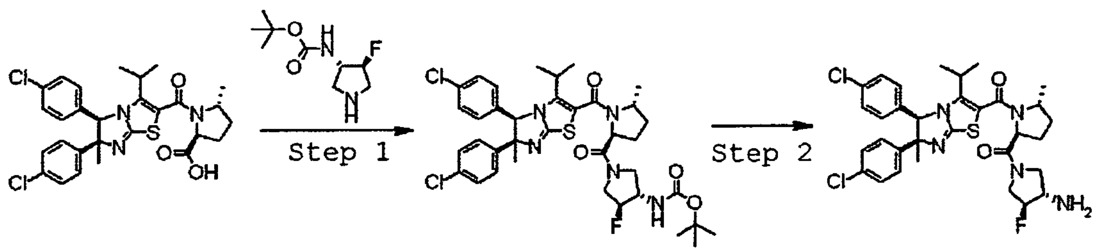


Step 1: tert-Butyl {(3S,4S)-1-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-4-fluoropyrrolidin-3-yl}carbamate

The compound obtained in Step 2 of Example 126 was reacted in the same way as in Step 4 of Example 1 using tert-butyl [(3S,4S)-4-fluoropyrrolidin-3-yl]carbamate instead of piperazin-2-one to give the title compound. $^1$H-NMR ($CDCl_3$) δ: 0.95 (3H, d, J=6.8 Hz), 1.05 (3H, d, J=7.1 Hz), 1.25 (3H, d, J=6.3 Hz), 1.55 (9H, s), 1.64-1.84 (1H, m), 1.80 (3H, s), 1.97-2.22 (2H, m), 2.48-2.63 (2H, m), 3.50 (1H, dd, J=27.0, 14.8 Hz), 3.70-3.97 (2H, m), 4.11-4.26 (2H, m), 4.46-4.54 (1H, m), 4.57-4.63 (1H, m), 4.93 (1H, s), 5.00-5.18 (1H, m), 5.64-5.73 (1H, m), 6.69 (2H, d, J=8.3 Hz), 6.98-7.05 (4H, m), 7.13 (2H, d, J=8.5 Hz).

Step 2: (3S,4S)-1-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-4-fluoropyrrolidin-3-amine

The compound obtained in Step 1 above was reacted in the same way as in Step 2 of Example 214 to give the title compound.
$^1$H-NMR ($CDCl_3$) δ: 0.96 (3H, d, J=7.1 Hz), 1.00 (3H, d, J=6.6 Hz), 1.24 (3H, d, J=5.9 Hz), 1.37-1.74 (4H, m), 1.79 (3H, s), 1.87-2.02 (1H, m), 2.13-2.77 (3H, m), 3.38-4.11 (6H, m), 4.49-4.61 (1H, m), 4.69-5.00 (2H, m), 4.94 (1H, s), 6.70 (2H, d, J=8.0 Hz), 7.01 (4H, d, J=8.8 Hz), 7.06-7.16 (2H, m).
MS (FAB) m/z: 644.

Example 242

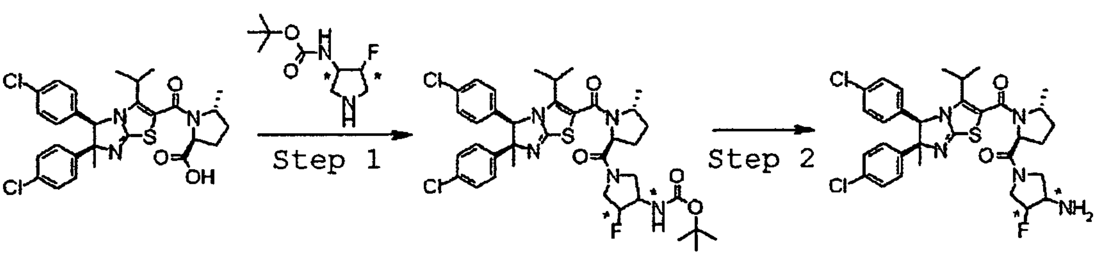

Step 1: tert-Butyl {3,4-cis-1-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-4-fluoropyrrolidin-3-yl}carbamate (Isomer A)

The compound obtained in Step 2 of Example 126 was reacted in the same way as in Step 4 of Example 1 using a compound (Isomer A) obtained in Step 2 of Reference Example 89 instead of piperazin-2-one to give the title compound.
$^1$H-NMR ($CDCl_3$) δ: 3.02-3.15 (1H, m), 0.92-1.05 (6H, m), 1.20-1.27 (3H, m), 1.42-1.96 (3H, m), 1.46 (9H, s), 1.80 (3H, s), 2.16-2.74 (2H, m), 3.47-4.18 (3H, m), 4.22-4.47 (1H, m), 4.50-4.85 (2H, m), 4.90-5.24 (2H, m), 4.94 (1H, s), 6.69 (2H, d, J=6.6 Hz), 6.99-7.05 (4H, m), 7.13 (2H, d, J=8.5 Hz).

Step 2: 3,4-cis-1-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-4-fluoropyrrolidin-3-amine(Isomer A)

The compound obtained in Step 1 above was reacted in the same way as in Step 2 of Example 214 to give the title compound.
$^1$H-NMR ($CDCl_3$) δ: 0.86-1.07 (6H, m), 1.15-1.29 (3H, m), 1.42-1.96 (4H, m), 1.80 (3H, s), 2.96-3.08 (1H, m), 3.37-4.12 (8H, m), 4.48-4.69 (1H, m), 4.71-5.08 (1H, m), 4.94 (1H, s), 6.59-6.73 (2H, m), 6.94-7.05 (4H, m), 7.05-7.16 (2H, m).
MS (FAB) m/z: 644.

Example 243

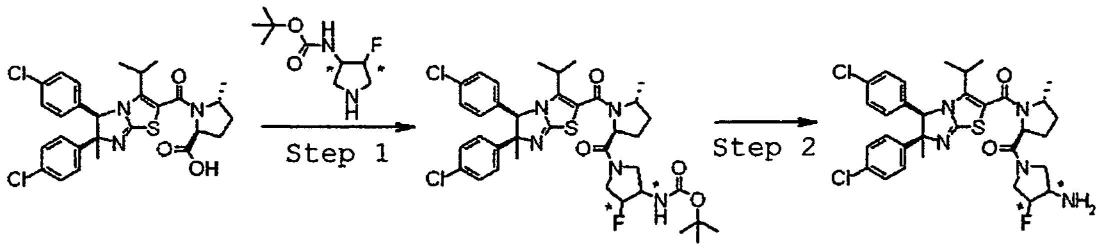


Step 1: tert-Butyl {3,4-cis-1-[(SR)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-4-fluoropyrrolidin-3-yl}carbamate] (Isomer B)

The compound obtained in Step 2 of Example 126 was reacted in the same way as in Step 4 of Example 1 using a compound (isomer B) obtained in Step 2 of Reference Example 89 instead of piperazin-2-one to give the title compound.
$^1$H-NMR ($CDCl_3$) δ: 0.97-1.02 (6H, m), 1.20-1.27 (3H, m), 1.44-1.48 (9H, m), 1.59-1.73 (3H, m), 1.77-1.93 (3H, m), 1.84 (1H, d, J=35.4 Hz), 2.14-2.52 (2H, m), 2.60-2.72 (1H, m), 3.22 (1H, t, J=9.8 Hz), 3.56-4.01 (2H, m), 419-4.60 (2H, m), 4.65-5.16 (3H, m), 6.69 (2H, d, J=8.3 Hz), 6.98-7.05 (4H, m), 7.14 (2H, d, J=8.5 Hz).

Step 2: 3,4-cis-1-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-4-fluoropyrrolidin-3-amine (Isomer B)

The compound obtained in Step 1 above was reacted in the same way as in Step 2 of Example 214 to give the title compound.
$^1$H-NMR ($CDCl_3$) δ: 0.91-1.05 (6H, m), 1.19-1.25 (3H, m), 1.40-1.75 (3H, m), 1.80 (3H, s), 1.83-1.97 (1H, m), 2.14-2.31 (1H, m), 2.35-2.52 (1H, m), 2.58-2.72 (1H, m), 3.11-3.22 (1H, m), 3.53-3.92 (4H, m), 4.09-4.24 (1H, m), 4.48-4.58 (1H, m), 4.72-4.98 (1H, m), 4.94 (1H, s), 6.69 (2H, d, J=7.6 Hz), 6.96-7.05 (4H, m), 7.07-7.17 (2H, m).
MS (FAB) m/z: 644.

Example 244

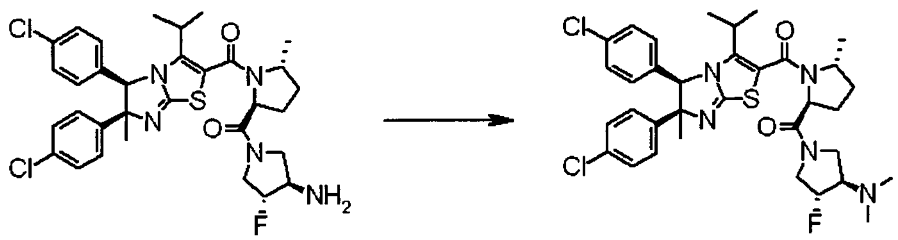

(3R,4R)-1-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-4-fluoro-N,N-dimethylpyrrolidin-3-amine

The compound obtained in Step 2 of Example 240 was reacted in the same way as in Step 2 of Example 168 to give the title compound.
$^1$H-NMR ($CDCl_3$) δ: 0.85-0.91 (3H, m), 0.92-1.05 (4H, m), 1.18-1.32 (4H, m), 1.61-2.01 (1H, m), 1.80 (3H, s), 2.17-2.76 (3H, m), 2.29 (6H, s), 2.85-3.20 (1H, m), 3.35-3.94 (2H, m), 3.99-4.33 (1H, m), 4.49-4.60 (1H, m), 4.64-4.86 (1H, m), 4.88-5.26 (1H, m), 4.94 (1H, s), 6.61-6.74 (2H, m), 6.98-7.05 (4H, m), 7.13 (2H, d, J=8.0 Hz).
MS (FAB) m/z: 672.

Example 245

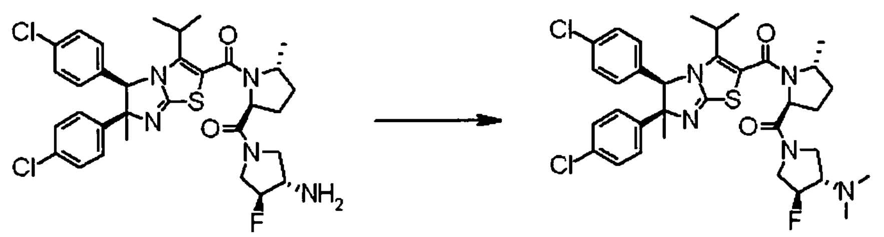

(3S,4S)-1-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-4-fluoro-N,N-dimethylpyrrolidin-3-amine

The compound obtained in Step 2 of Example 241 was reacted in the same way as in Step 2 of Example 168 to give the title compound.
$^1$H-NMR ($CDCl_3$) δ: 0.83-1.08 (7H, m), 1.18-1.32 (4H, m), 1.59-1.97 (3H, m), 2.18-2.52 (2H, m), 2.29 (6H, s), 2.56-2.95 (1H, m), 2.99-3.25 (1H, m), 3.34-4.01 (4H, m), 4.49-4.60 (1H, m), 4.63-4.84 (1H, m), 4.88-5.28 (1H, m), 4.94 (1H, s), 6.61-6.74 (2H, m), 6.98-7.04 (4H, m), 7.06-7.16 (2H, m).
MS (FAB) m/z: 672.

Example 246

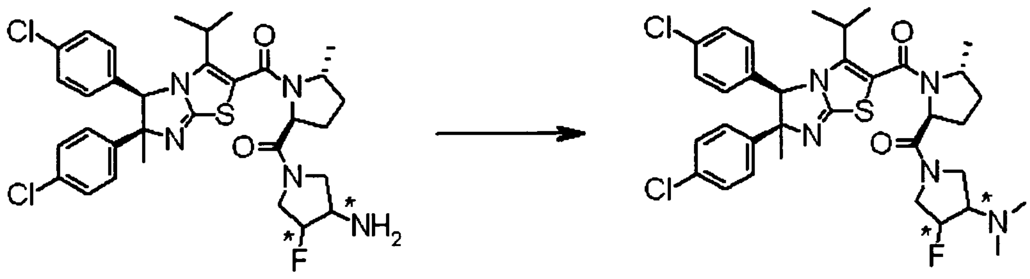

3,4-cis-1-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-4-fluoro-N,N-dimethylpyrrolidin-3-amine (Isomer A)

The compound obtained in Step 2 of Example 242 was reacted in the same way as in Step 2 of Example 168 to give the title compound.
$^1$H-NMR ($CDCl_3$) δ: 0.96 (3H, d, J=7.1 Hz), 1.01 (3H, d, J=7.1 Hz), 1.20-1.27 (4H, m), 1.60-2.00 (1H, m), 1.80 (3H, s), 2.17-2.76 (4H, m), 2.35 (6H, s), 3.27-3.36 (1H, m), 3.47-3.88 (2H, m), 3.98-4.18 (1H, m), 4.50-4.72 (2H, m), 4.94 (1H, s), 5.03-5.28 (1H, m), 6.69 (2H, d, J=7.6 Hz), 6.99-7.05 (4H, m), 7.09-7.16 (2H, m).
MS (FAB) m/z: 672.

Example 247

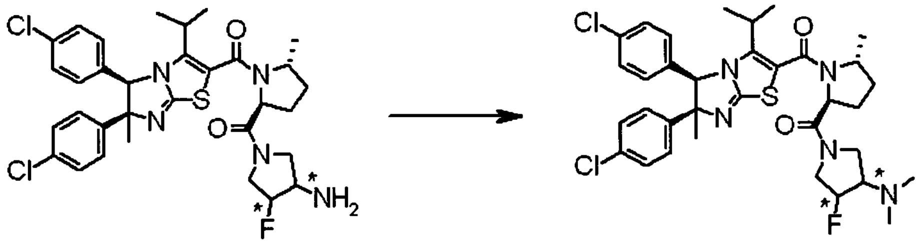

3,4-cis-1-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-4-fluoro-N,N-dimethylpyrrolidin-3-amine (Isomer B)
The compound obtained in Step 2 of Example 243 was reacted in the same way as in Step 2 of Example 168 to give the title compound.
$^1$H-NMR ($CDCl_3$) δ: 0.98 (3H, d, J=7.2 Hz), 1.01 (3H, d, J=7.2 Hz), 1.21-1.28 (4H, m), 1.60-1.97 (2H, m), 1.81 (3H, s), 2.18-2.56 (2H, m), 2.38 (6H, s), 2.59-2.80 (1H, m), 3.45-3.53 (1H, m), 3.54-3.78 (1H, m), 3.89 (1H, dd, J=24.9, 14.6 Hz), 4.11-4.26 (1H, m), 4.50-4.60 (1H, m), 4.78-4.84 (1H, m), 4.94 (1H, s), 5.02-5.29 (1H, m), 6.69 (2H, d, J=8.0 Hz), 6.99-7.05 (4H, m), 7.13 (2H, d, J=8.5 Hz).
MS (FAB) m/z: 672.

Example 248

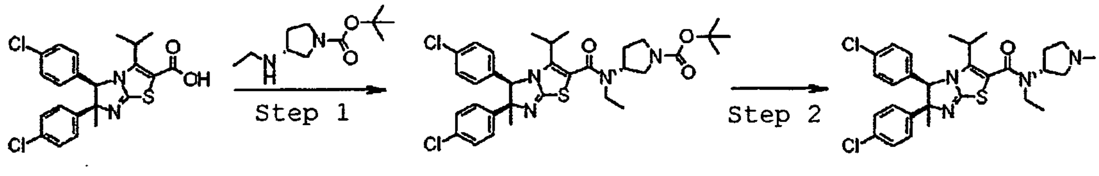


Step 1 : tert-Butyl (3R)-3-[{[(5R,6S)-5,6-Bis(4-chlorophenyl)-3-isopropyl-6-methyl-5,6-dihydroimidazo[2,1-b][1,3]thiazol-2-yl]carbonyl}(ethyl)amino]pyrrolidine-1-carboxylate

The compound obtained in Step 3 of Example 1 was reacted in the same way as in Example 112 using tert-butyl (3R)-3-(ethylamino)pyrrolidine-1-carboxylate instead of the compound obtained in Step 2 of Reference Example 18 to give the title compound.
$^1$H-NMR ($CDCl_3$, 60°C) δ: 0.89 (3H, d, J=6.8 Hz), 1.03 (3H, d, J=6.8 Hz), 1.23 (3H, t, J=7.1 Hz), 1.48 (9H, s), 1.82 (3H, s), 2.05 (2H, m), 2.42 (1H, m), 3.26-3.31 (2H, m), 3.38-3.42 (2H, m), 3.60-3.66 (2H, m), 4.65 (1H, m), 4.93 (1H, s), 6.71 (2H, d, J=8.3 Hz), 7.01-7.03 (4H, m), 7.10 (2H, d, J=8.5 Hz) .

Step 2: (5R,6S)-5,6-Bis(4-chlorophenyl)-N-ethyl-3-isopropyl-6-methyl-N-[(3R)-methylpyrrolidin-3-yl]-5,6-dihydroimidazo[2,1-b][1,3]thiazole-2-carboxamide

The compound obtained in Step 1 above was reacted in the same way as in Step 2 of Example 231 to give the title compound.
$^1$H-NMR ($CDCl_3$) δ: 0.85 (3H, d, J=7.1 Hz), 1.02 (3H, d, J=7.1 Hz), 1.23 (3H, t, J=7.1 Hz), 1.81 (3H, s), 1.84 (1H, m), 2.14 (1H, m), 2.34 (1H, m), 2.35 (3H, s), 2.47 (1H, m), 2.60 (1H, m), 2.67-2.76 (2H, m), 3.36-3.53 (2H, m), 4.71 (1H, m), 4.92 (1H, s), 6.66-6.73 (2H, m), 6.99-7.09 (6H, m).
MS (ESI) m/z: 557, 559.

Example 249

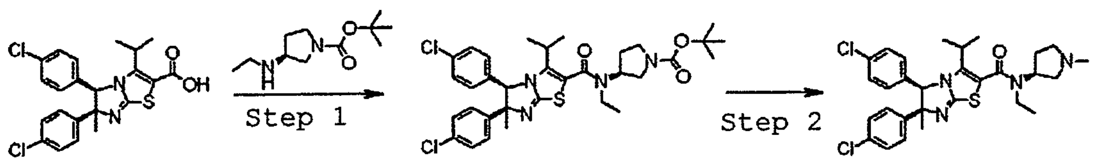


Step 1: tert-Butyl (3S)-3-[{[(5R,6S)-5,6-Bis(4-chlorophenyl)-3-isopropyl-6-methyl-5,6-dihydroimidazo[2,1-b][1,3]thiazol-2-yl]carbonyl}(ethyl)amino]pyrrolidine-1-carboxylate

The compound obtained in Step 3 of Example 1 was reacted in the same way as in Example 112 using tert-butyl (3S)-3-(ethylamino)pyrrolidine-1-carboxylate instead of the compound obtained in Step 2 of Reference Example 18 to give the title compound.
$^1$H-NMR ($CDCl_3$, 60°C) δ : 0.90 (3H, d, J=7.1 Hz), 1.03 (3H, d, J=7.1 Hz), 1.22 (3H, t, J=7.0 Hz), 1.47 (9H, s), 1.82 (3H, s), 2.06-2.16 (2H, m), 2.42 (1H, m), 3.24-3.50 (4H, m), 3.57-3.59 (2H, m), 4.60-4.68 (1H, m), 4.93 (1H, s), 6.70 (2H, d, J=8.3 Hz), 7.00-7.04 (4H, m), 7.09 (2H, d, J=8.5 Hz).

Step 2: (5R,6S)-5,6-Bis(4-chlorophenyl)-N-ethyl-3-isopropyl-6-methyl-N-[(3S)-methylpyrrolidin-3-yl]-5,6-dihydroimidazo[2,1-b][1,3]thiazole-2-carboxamide

The compound obtained in Step 1 above was reacted in the same way as in Step 2 of Example 231 to give the title compound.
$^1$H-NMR ($CDCl_3$) δ: 0.85 (3H, d, J=7.1 Hz), 1.03 (3H, d, J=7.1 Hz), 1.23 (3H, t, J=7.1 Hz), 1.81 (3H, s), 1.88 (1H, m), 2.21-2.32 (3H, m), 2.33 (3H, s), 2.45 (1H, m), 2.54-2.60 (2H, m), 2.74 (1H, m), 3.46 (2H, q, J=7.1 Hz), 4.70 (1H, m), 4.91 (1H, s), 6.67-6.73 (2H, m), 6.98-7.09 (6H, m).

MS (ESI) m/z: 557, 559.

Example 250

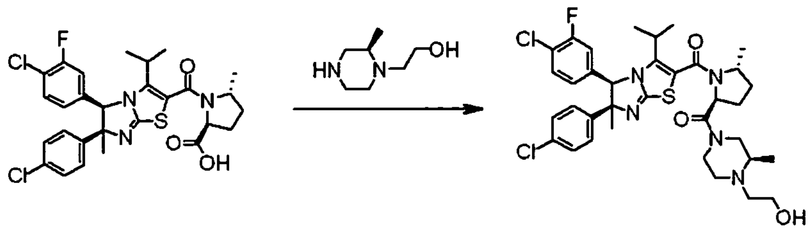

2-{(2R)-4-[(5R)-1-{[(5R,6S)-5-(4-chloro-3-fluorophenyl)-6-(4-chlorophenyl)-3-isopropyl-6-methyl-5,6-dihydroimidazo [2,1-b][1,3]thiazol-2-yl]carbonyl}-5-methyl-L-prolyl]-2-methylpiperazin-1-yl}ethanol

The compound obtained in Step 8 of Reference Example 50 was reacted in the same way as in Step 4 of Example 1 using the compound obtained in Step 2 of Reference Example 65 instead of piperazin-2-one to give the title compound.
$^1$H-NMR ($CDCl_3$) δ: 0.87-1.09 (8H, m), 1.24-1.25 (6H, m), 1.78-1.81 (5H, m), 2.34 (4H, brs), 2.55-2.66 (2H, m), 2.93 (2H, brs), 3.08 (1H, brs), 3.57-3.64 (3H, m), 3.94-4.07 (1H, m), 4.55-4.58 (1H, m), 4.92 (1H, s), 5.00 (1H, s), 6.49-6.52 (2H, m), 7.06-7.12 (5H, m).
MS (FAB) m/z: 702.

Example 251

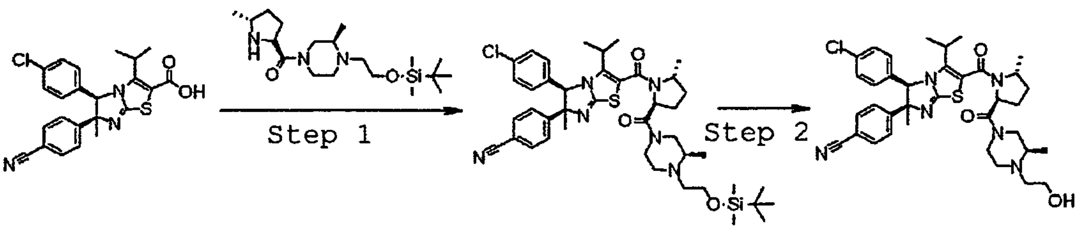


Step 1: 4-[(5R,6S)-2-{[(2S,5R)-2-{[(3R)-4-(2-{[tert-butyl(dimethyl)silyl]oxy}ethyl)-3-methylpiperazin-1-yl]carbonyl}-5-methylpyrrolidin-1-yl]carbonyl}-5-(4-chlorophenyl)-3-isopropyl-6-methyl-5,6-dihydroimidazo[2,1-b][1,3]thiazol-6-yl] benzonitrile

The compound obtained in Step 5 of Reference Example 90 was reacted in the same way as in Example 112 using the compound obtained in Step 3 of Reference Example 91 instead of the compound obtained in Step 2 of Reference Example 18 to give the title compound.
$^1$H-NMR ($CDCl_3$, 65°C) δ: 0.06 (6H, s), 0.91 (9H, s), 0.93-0.96 (3H, d, J=6.3 Hz), 1.05 (6H, d, J=7.1 Hz), 1.24 (3H, d, J=6.3 Hz), 1.44-1.51 (2H, m), 1.64 (1H, m), 1.82 (3H, s), 1.83-1.88 (2H, m), 2.20-2.54 (4H, m), 2.78-2.98 (4H, m), 3.41 (1H, m), 3.70-3.74 (3H, m), 3.99 (1H, m), 4.53 (1H, m), 4.98 (1H, s), 5.01 (1H, d, J=9.8 Hz), 6.69 (2H, d, J=8.3 Hz), 7.03 (2H, d, J=8.3 Hz), 7.32-7.33 (4H, m).
MS (ESI) m/z: 789, 791.

Step 2: 4-[(5R,6S)-5-(4-Chlorophenyl)-2-{[(2S,5R)-2-{[(3R)-4-(2-hydroxyethyl)-3-methylpiperazin-1-yl]carbonyl}-5-methylpyrrolidin-1-yl]carbonyl}-3-isopropyl-6-methyl-5,6-dihydroimidazo[2,1-b][1,3]thiazol-6-yl]benzonitrile

The compound obtained in Step 1 above was reacted in the same way as in Step 2 of Example 177 to give the title compound.
$^1$H-NMR (DMSO-$d_6$, 100°C) δ: 0.85 (3H, d, J=7.1 Hz), 0.93-0.98 (6H, m), 1.16 (3H, d, J=6.3 Hz), 1.32-1.38 (2H, m), 1.53-1.66 (2H, m), 1.73 (1H, m), 1.75 (3H, s), 2.09 (1H, m), 2.32-2.43 (3H, m), 2.67-2.78 (2H, m), 2.91 (1H, m), 3.18 (1H, m), 3.48-3.54 (2H, m), 3.66-3.79 (2H, m), 4.31 (1H, m), 4.95 (1H, d, J=7.8 Hz), 5.45 (1H, s), 6.88 (2H, d, J=7.8 Hz), 7.09 (2H, d, J=7.8 Hz), 7.44-7.46 (4H, m).
MS (ESI) m/z: 675.

Example 252

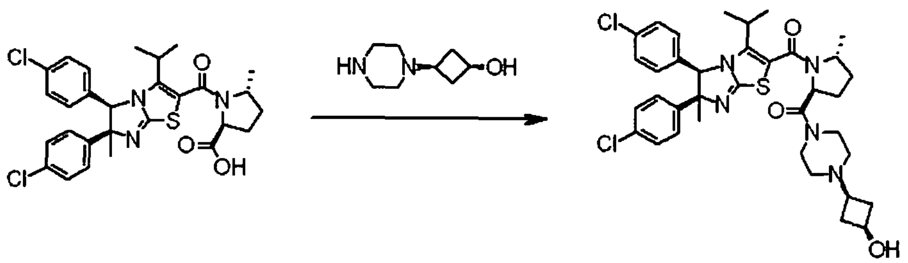

cis-3-{4-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]piperazin-1-yl}cyclobutanol

The compound obtained in Step 2 of Example 126 was reacted in the same way as in Step 4 of Example 1 using the compound obtained in Step 3 of Reference Example 92 instead of piperazin-2-one to give the title compound.
$^1$HNMR (DMSO-$d_6$, 100°C) δ: 0.86 (3H, d, J=6.8 Hz), 0.96 (3H, d, J=6.8 Hz), 1.16 (3H, d, J=6.3 Hz), 1.52-1.79 (5H, m), 1.73 (3H, s), 1.99-2.41 (8H, m), 2.63-2.78 (1H, m), 3.34-3.56 (4H, m), 3.72-3.90 (1H, m), 4.23-4.40 (1H, m), 4.58 (1H, d, J=6.1 Hz), 4.95 (1H, d, J=6.6 Hz), 5.38 (1H, s), 6.88 (2H, d, J=8.5 Hz), 7.00-7.14 (4H, m), 7.26 (2H, d, J=8.5 Hz).
MS (ESI) m/z: 696, 698.

Example 253

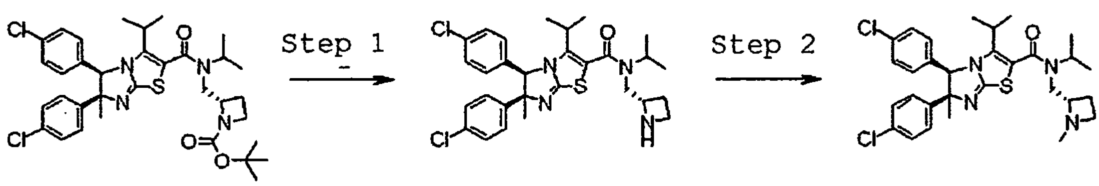


Step 1: (5R,6S)-N-[(2S)-Azetidin-2-ylmethyl]-5,6-bis(4-chlorophenyl)-N,3-diisopropyl-6-methyl-5,6-dihydroimidazo [2,1-b][1,3]thiazole-2-carboxamide

The compound obtained in Step 2 of Example 173 was reacted in the same way as in Step 6 of Example 102 to give the title compound.
$^1$H-NMR ($CDCl_3$) δ: 0.88 (3H, d, J=7. Hz), 1.00 (3H, d, J=7.1 Hz), 1.18 (3H, d, J=6.6 Hz), 1.23 (3H, t, J=6.6 Hz), 1.81 (3H, s), 2.11 (1H, m), 2.29 (1H, m), 2.39 (1H, m), 3.26-3.34 (2H, m), 3.45 (1H, dd, J=14.0, 6.0 Hz), 3.55 (1H, q, J=8.0 Hz), 410 (1H, m), 4.31 (1H, m), 4.92 (1H, s), 6.66-6.72 (2H, m), 7.00-7.03 (4H, m), 7.06-7.10 (2H, m).

Step 2: (5R,6S)-5,6-Bis(4-chlorophenyl)-N,3-diisopropyl-6-methyl-N-{[(2S)-1-methylazetidin-2-yl]methyl}-5,6-dihydroimidazo[2,1-b][1,3]thiazole-2-carboxamide

The compound obtained in Step 1 above was reacted in the same way as in Step 3 of Example 152 to give the title compound.
$^1$H-NMR ($CDCl_3$) δ: 0.90 (3H, d, J=7.1 Hz), 0.99 (3H, d, J=7.1 Hz), 1.19 (3H, d, J=6.6 Hz), 1.21 (3H, d, J=6.6 Hz), 1.80 (3H, s), 1.95 (1H, m), 2.06 (1H, m), 2.33 (3H, s), 2.42 (1H, m), 2.70 (1H, m), 3.13-3.25 (2H, m), 3.35 (1H, m), 3.43 (1H, dd, J=13.4, 3.4 Hz), 4.31 (1H, m), 4.93 (1H, s), 6.66-6.73 (2H, m), 7.00-7.03 (4H, m), 7.08 (2H, d, J=8.5 Hz).
MS (ESI) m/z: 571.

Example 254

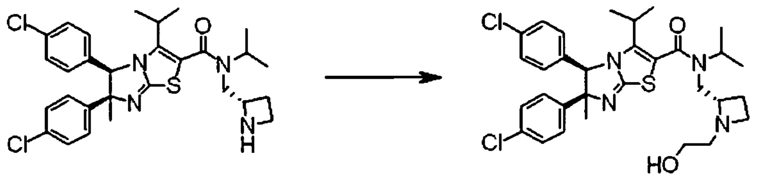

(5R,6S)-5,6-Bis(4-chlorophenyl)-N-{[(2S)-1-(2-hydroxyethyl)azetidin-2-yl]methyl}-N,3-diisopropyl-6-methyl-5,6-dihydroimidazo[2,1-b][1,3]thiazole-2-carboxamide

The compound obtained in Step 1 of Example 253 was reacted in the same way as in Step 2 of Example 168 using tert-butyldimethylsilyloxy)acetaldehyde instead of 35% aqueous formaldehyde solution and then reacted in the same way as in Step 2 of Example 177 to give the title compound.
$^1$H-NMR ($CDCl_3$) δ: 0.89 (3H, d, J=7.1 Hz), 0.99 (3H, d, J=7.1 Hz), 1.18 (3H, d, J=6.8 Hz), 1.22 (3H, d, J=6.8 Hz), 1.80 (3H, s), 2.00 (1H, m), 2.12 (1H, m), 2.41 (1H, m), 2.52 (1H, td, J=8.2, 4.1 Hz), 2.76-2.89 (2H, m), 3.23 (1H, dd, J=13.4, 7.1 Hz), 3.42-3.62 (5H, m), 4.32 (1H, m), 4.93 (1H, s), 6.68-6.70 (2H, m), 7.00-7.02 (4H, m), 7.07-7.09 (2H, m).
MS (ESI) m/z: 601.

Example 255

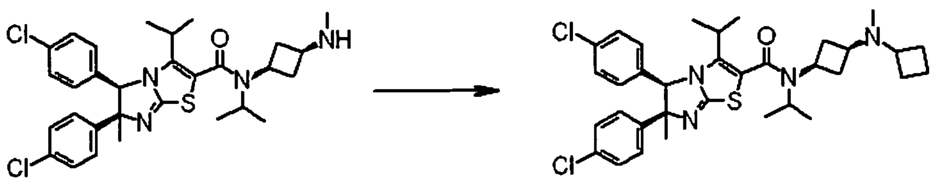

(5R,6S)-5,6-Bis(4-chlorophenyl)-N-{cis-3-[cyclobutyl(methyl)amino]cyclobutyl}-N,3-diisopropyl-6-methyl-5,6-dihydroimidazo[2,1-b][1,3]thiazole-2-carboxamide

The compound obtained in Step 1 of Example 226 was reacted in the same way as in Step 2 of Example 168 using cyclobutanone instead of 35% aqueous formaldehyde solution to give the title compound.
$^1$H-NMR ($CDCl_3$) δ: 0.86 (3H, d, J=6.8 Hz), 1.05 (3H, d, J=7.1 Hz), 1.34 (3H, d, J=6.8 Hz), 1.36 (3H, d, J=6.8 Hz), 1.59-1.72 (2H, m), 1.82 (3H, s), 1.92-2.41 (9H, m), 2.05 (3H, s), 2.43-2.53 (1H, m), 2.77-2.87 (1H, m), 3.86-4.02 (2H, m), 4.93 (1H, s), 6.69-6.75 (2H, m), 7.01-7.06 (4H, m), 7.08-7.12 (2H, m).
MS (ESI) m/z: 625.

Example 256

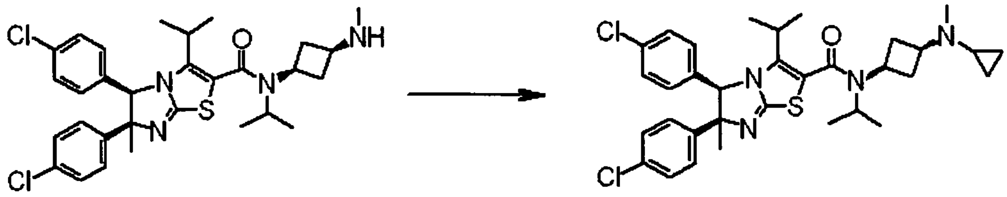

(5R,6S)-5,6-Bis(4-chlorophenyl)-N-{cis-3-[cyclopropyl(methyl)amino]cyclobutyl}-N,3-diisopropyl-6-methyl-5,6-dihydroimidazo[2,1-b][1,3]thiazole-2-carboxamide

The compound obtained in Step 1 of Example 226 was reacted in the same way as in Example 197 to give the title compound.
$^1$H-NMR ($CDCl_3$) δ: 0.42-0.50 (4H, m), 0.90 (3H, d, J=7.1 Hz), 1.08 (3H, d, J=7.1 Hz), 1.37 (3H, d, J=6.8 Hz), 1.39 (3H, d, J=6.8 Hz), 1.51-1.57 (10H, m), 1.84 (3H, s), 2.29 (3H, s), 2.31-2.49 (5H, m), 2.71-2.80 (1H, m), 3.85-3.93 (1H, m), 3.98-4.08 (1H, m), 4.95 (1H, s), 6.73-6.77 (2H, m), 7.03-7.08 (4H, m), 7.11-7.15 (2H, m).
MS (ESI) m/z: 611.

Example 257

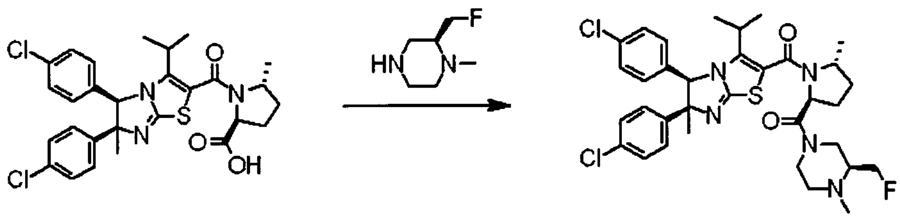

(5R,6S)-5,6-Bis(4-chlorophenyl)-2-{[(2S,5R)-2-{[(3S)-3-(fluoromethyl)-4-methylpiperazin-1-yl]carbonyl}-5-methylpyrrolidin-1-ylcarbonyl}-3-isopropyl-6-methyl-5,6-dihydroimidazo[2,1-b][1,3]thiazole

The compound obtained in Step 2 of Example 126 was reacted in the same way as in Step 4 of Example 1 using the compound obtained in Step 3 of Reference Example 93 instead of piperazin-2-one to give the title compound.
$^1$H-NMR ($CDCl_3$) δ: 0.96-1.03 (7H, m), 1.24 (3H, d, J=6.3 Hz), 1.59 (8H, brs), 1.81 (3H, s), 2.26-2.44 (5H, m), 2.68-2.77 (1H, m), 2.97-3.07 (1H, m), 3.59-3.63 (1H, m), 4.54-4.56 (1H, m), 4.94-5.01 (2H, m), 6.69 (2H, d, J=7.3 Hz), 7.02-7.03 (4H, m), 7.12-7.14 (2H, m).
MS (FAB) m/z: 672.

Example 258

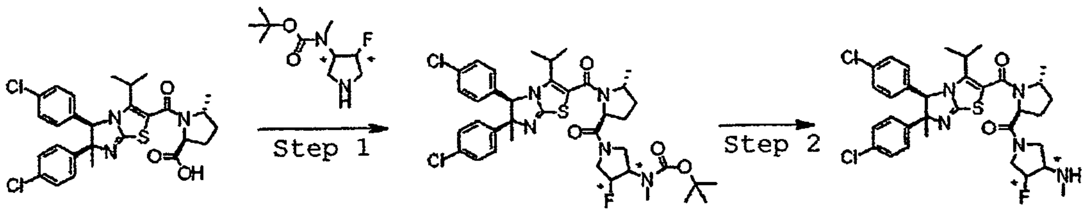


Step 1: 3,4-cis-tert-Butyl {1-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-4-fluoropyrrolidin-3-yl}methylcarbamate (Isomer A)

The compound obtained in Step 2 of Example 126 was reacted in the same way as in Step 4 of Example 1 using the compound (Isomer A) obtained in Step 2 of Reference Example 94 instead of piperazin-2-one to give the title compound.
$^1$H-NMR ($CDCl_3$) δ: 0.92-1.05 (6H, m), 1.23 (3H, d, J=6.3 Hz), 1.48 (9H, s), 1.48-1.51 (1H, m), 1.59-1.78 (1H, m), 1.80 (3H, s), 1.84-1.97 (1H, m), 2.18-2.55 (1H, m), 2.60-3.04 (2H, m), 2.91 (3H, s), 3.45-3.81 (2H, m), 3.81-4.07 (2H, m), 4.51-4.88 (2H, m), 4.94 (1H, s), 5.04-5.36 (1H, m), 6.69 (2H, d, J=6.3 Hz), 6.98-7.05 (4H, m), 7.14 (2H, d, J=8.3 Hz).

Step 2: 3,4-cis-1-[(5R)-1-{[(5R,6S)-5,6-Bis(4-chlorophenyl)-3-isopropyl-6-methyl-5,6-dihydroimidazo[2,1-b][1,3]thiazol-2-yl]carbonyl}-5-methyl-L-prolyl]-4-fluoro-N-methylpyrrolidin-3-amine (Isomer A)

The compound obtained in Step 1 above was reacted in the same way as in Step 2 of Example 214 to give the title compound.
$^1$H-NMR ($CDCl_3$) δ: 0.85-1.07 (9H, m), 1.19-1.31 (2H, m), 1.45-1.73 (1H, m), 1.80 (3H, s), 1.83-1.97 (1H, m), 2.16-2.47 (1H, m), 2.49-2.77 (1H, m), 2.50 (3H, s), 3.05 (1H, t, J=10.6 Hz), 3.14-3.66 (2H, m), 3.73-4.18 (2H, m), 4.50-4.83 (2H, m), 4.94 (1H, s), 5.04-5.31 (1H, m), 6.69 (2H, d, J=7.3 Hz), 6.98-7.05 (4H, m), 7.09-7.16 (2H, m).
MS (FAB) m/z: 658.

Example 259

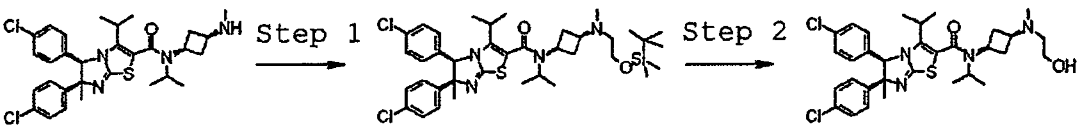


Step 1: (5R,6S)-N-{cis-3-[(2-{[tert-butyl(dimethyl)silyl]oxy}ethyl)(methyl)amino]cyclobutyl}-5,6-bis(4-chlorophenyl)-N,3-diisopropyl-6-methyl-5,6-dihydroimidazo[2,1-b][1,3]thiazole-2-carboxamide

The compound obtained in Step 1 of Example 226 was reacted in the same way as in Step 2 of Example 168 using tert-butyldimethylsilyloxy)acetaldehyde instead of 35% aqueous formaldehyde solution to give the title compound.
$^1$H-NMR ($CDCl_3$) δ: 0.08 (6H, d, J=5.1 Hz), 0.86 (3H, d, J=7.1 Hz), 0.91 (9H, d, J=1.7 Hz), 1.05 (3H, d, J=7.1 Hz), 1.36 (3H, d, J=6.8 Hz), 1.38 (3H, d, J=6.6 Hz), 1.82 (3H, s), 2.12-2.20 (1H, m), 2.20 (3H, s), 2.27-2.49 (5H, m), 2.55-2.63 (1H, m), 2.93 (1H, s), 3.62-3.66 (1H, m), 3.69-3.75 (3H, m), 3.87 (1H, t, J=6.8 Hz), 4.03 (1H, s), 4.93 (1H, s), 6.72 (2H, d, J=7.3 Hz), 7.03 (6H, dd, J=8.5, 5.1 Hz), 7.10 (6H, t, J=5.7 Hz).

Step 2: (5R,6S)-5,6-Bis(4-chlorophenyl)-N-{cis-3-[(2-hydroxyethyl)(methyl)amino]cyclobutyl}-N,3-diisopropyl-6-methyl-5,6-dihydroimidazo[2,1-b][1,3]thiazole-2-carboxamide

The compound obtained in Step 1 above was reacted in the same way as in Step 2 of Example 177 to give the title compound.
$^1$H-NMR ($CDCl_3$) δ: 0.87 (3H, d, J=7.1 Hz), 1.05 (3H, d, J=7.1 Hz), 1.33 (3H, d, J=6.8 Hz), 1.36 (3H, d, J=6.8 Hz), 1.82

(3H, s), 2.17 (3H, s), 2.29-2.48 (8H, m), 2.59-2.67 (1H, m), 3.61 (2H, t, J=5.2 Hz), 3.89-3.99 (2H, m), 4.93 (1H, s), 6.72 (2H, d, J=7.6 Hz), 7.01-7.05 (4H, m), 7.08-7.12 (2H, m).
MS (ESI) m/z: 615.

Example 260

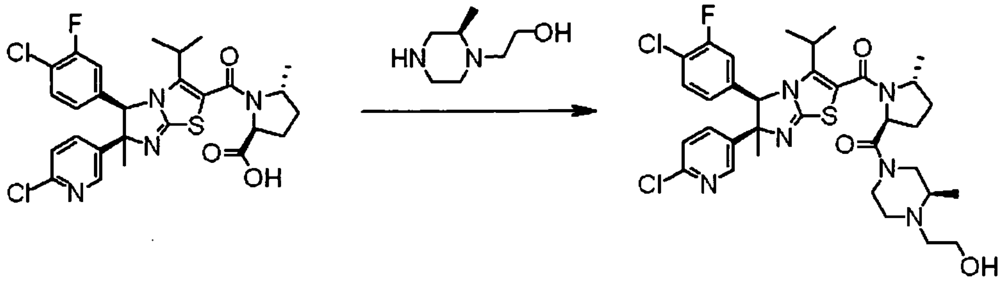

2-{(2R)-4-[(5R)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-methyl-L-prolyl]-2-methylpiperazin-1-yl}ethanol

The compound obtained in Step 9 of Reference Example 95 was reacted in the same way as in Step 4 of Example 1 using the compound obtained in Step 2 of Reference Example 65 instead of piperazin-2-one to give the title compound.
$^1$H-NMR ($CDCl_3$) δ: 0.83-0.90 (3H, m), 0.95-1.02 (6H, m), 1.15 (3H, d, J=6.3 Hz), 1.56-1.60 (1H, m), 1.74-1.78 (1H, m), 1.75 (3H, s), 2.05-2.10 (1H, m), 2.29-2.41 (3H, m), 2.68-2.75 (2H, m), 2.82-2.97 (2H, m), 3.41-3.51 (3H, m), 3.67-3.86 (2H, m), 4.09-4.16 (1H, m), 4.29-4.35 (1H, m), 4.96 (1H, d, J=7.3 Hz), 5.54 (1H, s), 6.70-6.74 (1H, m), 6.90-6.93 (1H, m), 7.19 (1H, d, J=8.3 Hz), 7.35 (1H, t, J=8.1 Hz), 7.67 (1H, dd, J=8.3, 2.4 Hz), 8.28 (1H, d, J=2.4 Hz).
MS (ESI) m/z: 703.

Reference Example 1

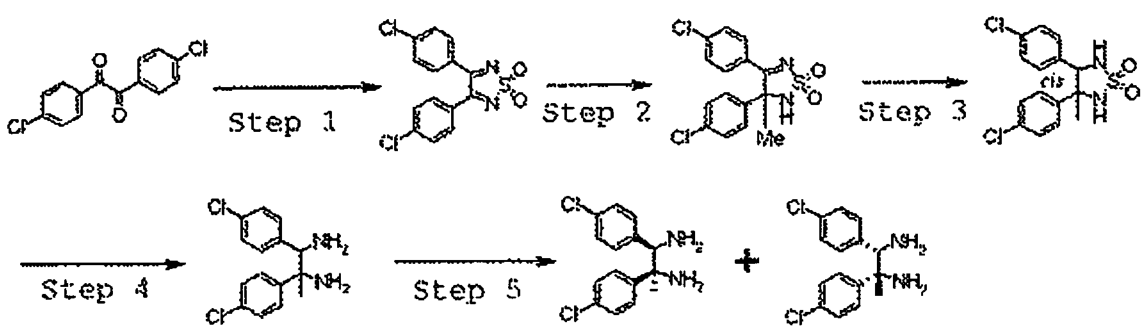


Step 1: 3,4-Bis(4-chlorophenyl)-1,2,5-thiadiazole 1,1-dioxide

1,2-Bis(4-chlorophenyl)ethane-1,2-dione (80.0 g, 0.29 mol) was suspended in ethanol (1.5 1), triethylamine (15 ml) and sulfamide (55.1 g, 0.57 mol) were added, and the resulting mixture was heated to reflux for 19 hours. After cooling, toluene was added and the solvent was evaporated under reduced pressure. Ethyl acetate was added to the residue and insoluble matter was removed by filtration. The filtrate was concentrated and the deposited matter was collected by filtration, almost dissolved in ethyl acetate, washed with water and brine, and then dried over anhydrous sodium sulfate. The solvent was evaporated under reduced pressure. Hexane and isopropyl ether were added to the

residue and insoluble matter was collected by filtration to give the title compound (59.5 g, 61%) as a pale yellow solid. $^1$H-NMR ($CDCl_3$) $\delta$: 7.47 (4H, d, J=8.8 Hz), 7.53 (4H, d, J=8.8 Hz).

Step 2: 3,4-Bis(4-chlorophenyl)-3-methyl-2,3-dihydro-1,2,5-thiadiazole 1,1-dioxide

The compound (10.0 g, 29.5 mmol) obtained in Step 1 above was suspended in toluene (200 ml) and a 0.89 M solution of methylmagnesium bromide in tetrahydrofuran (43.1 ml, 38.3 mmol) was added dropwise at 0°C over 10 minutes in an argon atmosphere. The resulting mixture was stirred at room temperature for 1 hour and then 1 N aqueous hydrochloric acid solution was added. After extraction with ethyl acetate, the extract was washed with saturated aqueous sodium bicarbonate solution and brine and then dried over anhydrous sodium sulfate. The solvent was evaporated under reduced pressure to give the title compound (11.1 g) as a colorless substance.
$^1$H-NMR ($CDCl_3$) $\delta$: 2.06 (3H, s), 4.70 (1H, s), 7.30-7.48 (6H, m), 7.63 (2H, d, J=9.0 Hz).

Step 3: (3R*,4S*)-3,4-Bis(4-chlorophenyl)-3-methyl-1,2,5-thiadiazolidine 1,1-dioxide

The compound (11.1 g) obtained in Step 2 above was dissolved in ethanol (300 ml), sodium borohydride (4.5 g, 0.12 mol) was added in small moieties under ice cooling, and the resulting mixture was stirred at room temperature for 1 hour. The solvent was evaporated under reduced pressure and then 1 N aqueous hydrochloric acid solution was added to the residue. After extraction with ethyl acetate, the extract was washed with saturated aqueous sodium bicarbonate solution and brine and then dried over anhydrous sodium sulfate and the solvent was evaporated under reduced pressure. Chloroform and ethyl acetate were added to the residue and insoluble matter was collected by filtration to give the title compound (4.9 g, 47%) as a colorless solid.
$^1$H-NMR ($CDCl_3$) $\delta$: 1.85 (3H, s), 4.63 (1H, d, J=6.8 Hz), 4.72 (1H, s), 4.93 (1H, d, J=6.8 Hz), 6.77 (2H, d, J=8.5 Hz), 7.01 (2H, d, J=8.5 Hz), 7.17 (2H, d, J=8.5 Hz), 7.18 (2H, d, J=8.5 Hz).

Step 4: (1R*,2S*)-1,2-Bis(4-chlorophenyl)propane-1,2-diamine

Pyridine (55 ml) and water (5.5 ml) were added to the compound (14.2 g, 39.8 mmol) obtained in Step 3 above and the resulting mixture was heated to reflux for 34 hours. After cooling, the solvent was evaporated under reduced pressure, toluene was added, and the solvent was evaporated under reduced pressure again. 1 N aqueous sodium hydroxide solution was added to the residue.
After extraction with ethyl acetate, the extract was concentrated and subjected to extraction with 1 N aqueous hydrochloric acid solution. The extract was made alkaline with sodium hydroxide under ice cooling, followed by extraction with ethyl acetate. The extract was washed with brine and then dried over anhydrous sodium sulfate and the solvent was evaporated under reduced pressure. Hexane and diethyl ether were added to the residue and insoluble matter was collected by filtration to give the title compound (8.2 g, 70%) as a colorless solid.
$^1$H-NMR ($CDCl_3$) $\delta$: 1.49 (3H, s), 4.08 (1H, s), 6.98 (2H, d, J=8.5 Hz), 7.17 (2H, d, J=8.3 Hz), 7.25-7.28 (4H, m).

Step 5: (1R,2S)-1,2-Bis(4-chlorophenyl)propane-1,2-diamine and (1S,2R)-1,2-bis(4-chlorophenyl)propane-1,2-diamine

L-Tartaric acid (5.05 g, 33.9 mmol) was added to an ethanol (100 ml) solution of the compound (10.00 g, 33.9 mmol) obtained in Step 4 above and the resulting mixture was heated to reflux until insoluble matter was dissolved. The reaction mixture was concentrated and then recrystallized from a mixed solvent of ethanol and diethyl ether and the deposited solid was collected by filtration. The obtained solid was made into an alkaline solution by the addition of 1 N aqueous sodium hydroxide solution, followed by extraction with diethyl ether. The organic layer was dried over potassium carbonate and then the solvent was evaporated under reduced pressure to give one of the title compounds, (1R,2S)-1,2-bis(4-chlorophenyl)propane-1,2-diamine, (3.05 g, 31%), as a colorless solid.
The filtrate obtained above was concentrated and made into an alkaline solution by the addition of 1 N aqueous sodium hydroxide solution, followed by extraction with diethyl ether. The organic layer was dried over potassium carbonate and then the solvent was evaporated under reduced pressure. D-Tartaric acid (3.56 g, 23.7 mmol) was added to an ethanol (100 ml) solution of the residue (7.00 g, 23.7 mmol) and the resulting mixture was heated to reflux until insoluble matter was dissolved. The reaction mixture was concentrated and then recrystallized from a mixed solvent of ethanol and water and the deposited solid was collected by filtration. The obtained solid was made into an alkaline solution by the addition of 1 N aqueous sodium hydroxide solution, followed by extraction with diethyl ether. The organic layer was dried over potassium carbonate and then the solvent was evaporated under reduced pressure to give the other title compound (1S,2R)-1,2-bis(4-chlorophenyl) propane-1,2-diamine (3.85 g, 39%) as a colorless solid.
Both the title compounds were confirmed to be single compounds of different isomers from results of HPLC analysis using instrumental data and a chiral column.

HPLC conditions
Column: DAICEL CHEMICAL INDUSTRIES, LTD., CHIRALPAK AS-H
(0.46ϕ × 25 cm)
Eluent: hexane/isopropanol (4/1)
Flow rate: 1.0 ml/min.
Detection: UV 254 nm
Retention time: 8.73 min. (for the former); 7.52 min.
(for the latter)
Optical rotation
The former compound: $[\alpha]^{23}_D$=+69.2°(c.1.05, methanol)

Reference Example 2

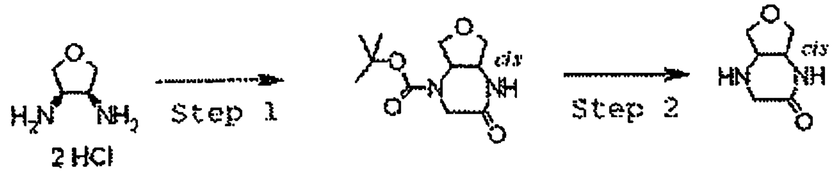


Step 1: tert-Butyl (4aR*,7aS*)-3-Oxohexahydrofuro[3,4-b]pyrazine-1(2H)-carboxylate

A suspension of (3R,4S)-tetrahydrofuran-3,4-diamine dihydrochloride (1.41 g, 8.05 mmol) and triethylamine (5.64 ml, 40.2 mmol) in acetonitrile (50 ml) was added to an acetonitrile (20 ml) solution of phenyl bromoacetate (1.94 g, 8.86 mmol) and the resulting mixture was stirred at room temperature for 18 hours. The reaction mixture was concentrated, then diluted with chloroform, and dried over potassium carbonate. The solvent was evaporated under reduced pressure and the residue was dissolved in chloroform (20 ml). Di-tert-butyl dicarbonate (3.74 ml, 16.1 mmol) was added and the resulting mixture was stirred at room temperature for 1 hour. The reaction mixture was diluted with chloroform, washed with 1 N aqueous sodium hydroxide solution and brine, and then dried over anhydrous sodium sulfate. The solvent was evaporated under reduced pressure. The residue was purified by silica gel column chromatography (hexane: ethyl acetate = 3:1 → ethyl acetate) to give the title compound (0.56 g, 29%) as a pale yellow oil.
$^1$H-NMR ($CDCl_3$) δ: 1.48 (9H, s), 3.74-3.92 (4H, m), 4.00 (1H, t, J=8.7 Hz), 4.06-4.12 (1H, m), 4.34 (1H, d, J=18.1 Hz), 5.00 (1H, brs), 6.66 (1H, brs).
MS (ESI) m/z: 187 $(M-55)^+$.

Step 2: (4aR*,7aS*)-Hexahydrofuro[3,4-b]pyrazin-2(1H)-one

A 4 N solution of hydrochloric acid in 1,4-dioxane (10 ml) was added to a 1,4-dioxane (20 ml) solution of the compound (0.56 g, 2.31 mmol) obtained in Step 1 above and the resulting mixture was stirred at room temperature for 18 hours. The reaction mixture was concentrated, diluted with a mixed solvent of chloroform and methanol (9:1), and dried over potassium carbonate. The solvent was evaporated under reduced pressure to give the title compound (0.35 g) as a pale orange oil.
$^1$H-NMR ($CDCl_3$) δ: 3.39 (1H, d, J=17.6 Hz), 3.51 (1H, d, J=17.6 Hz), 3.68-3.79 (2H, m), 3.82 (1H, dd, J=9.9, 2.8 Hz), 3.88-3.97 (2H, m), 4.01 (1H, dd, J=9.9, 6.1 Hz), 6.57 (1H, brs).
MS (ESI) m/z: 143.

Reference Example 3

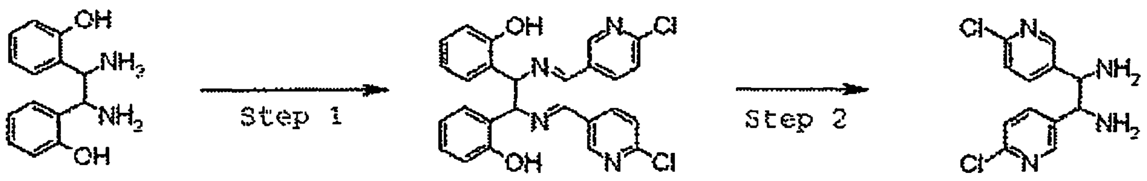


meso-1,2-Bis(6-chloropyridin-3-yl)ethane-1,2-diamine

1,2-Bis(2-hydroxyphenyl)ethane-1,2-diamine (2.44 g, 10.0 mmol) and 6-chloronicotinaldehyde (2.83 g, 20.0 mmol) were dissolved in acetonitrile (50 ml) and the resulting solution was heated to reflux for 14 hours. The reaction mixture was left standing to cool to room temperature and then cooled on ice. The deposited diimine was collected by filtration (Step 1). This deposited matter was suspended in ethanol (18 ml), 4 N sulfuric acid (18 ml) was added, and the resulting mixture was heated at 65°C for 10 minutes and further stirred overnight at room temperature. The reaction mixture was made basic by the addition of 1 N aqueous sodium hydroxide solution, followed by extraction with chloroform twice. The organic layers were combined and dried over anhydrous magnesium sulfate and then the solvent was evaporated under reduced pressure to give a yellow solid. This solid was washed with diethyl ether and collected by filtration to give the title compound (1.82 g, 64%) as a pale yellow solid (Step 2).
$^1$H-NMR (DMSO-$d_6$) δ: 1.94 (4H, brs), 4.01 (2H, brs), 7.39 (2H, d, J=8.3 Hz), 7.63 (2H, dd, J=8.3, 2.7 Hz), 8.12 (2H, d, J=2.7 Hz) .
MS (ESI) m/z: 283.

Reference Example 4

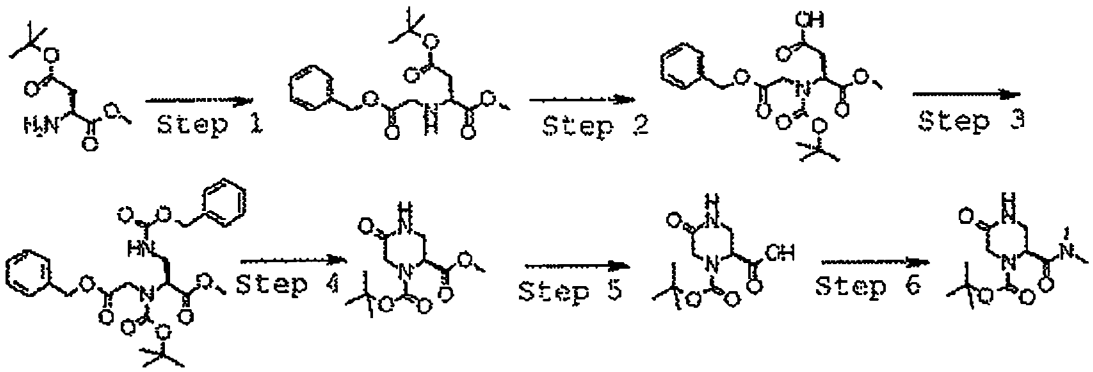


Step 1: 4-tert-Butyl 1-Methyl N-[2-(benzyloxy)-2-oxoethyl]-L-aspartate

4-tert-Butyl 1-methyl L-aspartate hydrochloride (19.6 g, 81.8 mmol) was suspended in acetonitrile (150 ml), potassium carbonate (27.6 g, 200 mmol) and benzyl bromoacetate (24.3 g, 106 mmol) were added with stirring at room temperature, and the resulting mixture was stirred overnight at 45°C. The mixture was left standing to cool to room temperature and then insoluble matter was removed by filtration. The filtrate was dried over anhydrous magnesium sulfate and the solvent was evaporated under reduced pressure. The residue was purified by silica gel column chromatography (ethyl acetate:hexane = 4:1 → 2:1) to give the title compound (25.4 g, 89%) as a yellow oil.
$^1$H-NMR ($CDCl_3$) δ: 1.43 (9H, s), 2.33 (1H, brs), 2.60-2.72 (2H, m), 3.49 (1H, d, J=17.3 Hz), 3.57 (1H, d, J=17.6 Hz), 3.63 (1H, t, J=7.0 Hz), 3.71 (3H, d, J=3.4 Hz), 5.15 (2H, s), 7.32-7.36 (5H, m).

Step 2: 1-Methyl N-[2-(Benzyloxy)-2-oxoethyl]-N-(tert-butoxycarbonyl)-L-aspartate

The compound (25.4 g, 72.3 mmol) obtained in Step 1 above was cooled in an ice-methanol bath (-10°C), a

95% aqueous solution of trifluoroacetic acid (50 ml) was added, and the resulting mixture was brought to room temperature and stirred for 3 hours. The reaction mixture was concentrated under reduced pressure and a 5 N aqueous solution of sodium hydroxide was added with stirring under ice cooling to adjust its pH to approximately 3. The mixture was extracted with chloroform. The organic layer was dried over anhydrous magnesium sulfate and then concentrated under reduced pressure. The residue was dissolved in saturated aqueous sodium bicarbonate solution and washed with diethyl ether. Then, concentrated hydrochloric acid was added dropwise with stirring under ice cooling to adjust its pH to approximately 3, followed by extraction with chloroform again. The organic layer was dried over anhydrous magnesium sulfate and then concentrated under reduced pressure to give a colorless oil. This substance was dissolved in dichloromethane (100 ml), triethylamine (7.32 ml, 52 mmol) and di-tert-butyl dicarbonate (11.4 g, 52.3 mmol) were added with stirring under ice cooling, and the resulting mixture was stirred overnight at room temperature. The reaction mixture was washed with 1 N aqueous hydrochloric acid solution and brine and then dried over anhydrous magnesium sulfate and the solvent was evaporated under reduced pressure to give the title compound (16.6 g, 65%) as a yellow oil.
MS (ESI) m/z: 396.

Step 3: Methyl 3-{[(Benzyloxy)carbonyl]amino}-N-[2-(benzyloxy)-2-oxoethyl]-N-(tert-butoxycarbonyl)-L-alaninate

The compound (16.5 g, 41.7 mmol) obtained in Step 2 above was dissolved in benzene (100 ml) and diphenylphosphoryl azide (11.0 g, 40.0 mmol) and subsequently triethylamine (5.6 ml, 40.0 mmol) were added with stirring at room temperature. The resulting mixture was stirred at room temperature for 45 minutes and then heated to reflux for 45 minutes. Benzyl alcohol (10.3 ml, 100 mmol) was added and the resulting mixture was further heated to reflux overnight. The reaction mixture was left standing to cool and then concentrated under reduced pressure. The residue was dissolved in ethyl acetate and washed with 1 N aqueous hydrochloric acid solution, saturated aqueous sodium bicarbonate solution, and brine. After drying over anhydrous magnesium sulfate, the solvent was evaporated under reduced pressure. The residue was purified by silica gel column chromatography (hexane:ethyl acetate = 4:1 → 2:1) to give the title compound (4.95 g, 25%) as a yellow oil.
$^1$H-NMR ($CDCl_3$) δ: 1.33 (9H, s), 1.40 (9H, s), 3.11-3.22 (2H, m), 3.71 (3H, s), 3.72 (3H, s), 3.80-4.20 (4H, m), 4.68 (1H, dd, J=10.0, 4.4 Hz), 4.89 (1H, dd, J=10.0, 4.4 Hz), 5.06-5.23 (8H, m), 5.98-6.13 (2H, m), 7.28-7.38 (20H, m).
MS (ESI) m/z: 501.

Step 4: 1-tert-Butyl 2-Methyl (2S)-5-oxopiperazine-1,2-dicarboxylate

The compound (4.95 g, 9.89 mmol) obtained in Step 3 above was dissolved in methanol (50 ml), 5% palladium-carbon (2.00 g) was added, and the resulting mixture was stirred at room temperature for 1.5 hours in a hydrogen atmosphere. The catalyst was removed by filtration and the solvent was evaporated under reduced pressure. Toluene was added to the residue and the solvent was evaporated under reduced pressure again. The obtained oil was dissolved in dimethylformamide (100 ml), 1-hydroxybenzotriazole (1.49 g, 11.0 mmol) and subsequently 1-(3-dimethylaminopropyl)-3-ethylcarbodiimide hydrochloride (2.49 g, 13.0 mmol) were added with stirring under ice cooling, and the resulting mixture was stirred overnight while gradually heated to room temperature. The reaction mixture was concentrated under reduced pressure and saturated aqueous sodium bicarbonate solution was added to the residue, followed by extraction with chloroform. The organic layer was dried over anhydrous magnesium sulfate and then the solvent was evaporated under reduced pressure. The residue was purified by silica gel column chromatography (chloroform:methanol = 50:1) to give the title compound (3.13 g) as a pale yellow oil.
$^1$H-NMR ($CDCl_3$) δ: 1.46-1.49 (9H, m), 3.64-3.68 (1H, m), 3.73-3.82 (1H, m), 3.78 (3H, s), 3.96-4.05 (1H, m), 4.19-4.24 (1H, m), 4.76-4.99 (1H, m), 7.02-7.18 (1H, m).
MS (ESI) m/z: 259.

Step 5: (2S)-1-(tert-Butoxycarbonyl)-5-oxopiperazine-2-carboxylic acid

The compound (3.13 g, 9.45 mmol) obtained in Step 4 above was dissolved in methanol (35 ml), 1 N aqueous sodium hydroxide solution (15 ml) was added, and the resulting mixture was stirred at room temperature for 1.5 hours. 1 N aqueous hydrochloric acid solution was added dropwise with stirring under ice cooling to adjust its pH to 7. Then, methanol was evaporated under reduced pressure. 1 N aqueous sodium hydroxide solution was added to the obtained aqueous solution to adjust its pH to approximately 12. After washing with diethyl ether, 1 N aqueous hydrochloric acid solution was added with stirring under ice cooling to adjust its pH to approximately 2. After extraction with ethyl acetate, the organic layer was dried over anhydrous magnesium sulfate and then the solvent was evaporated under reduced pressure. The residue was dissolved in ethyl acetate and solidified by the addition of hexane to give the title compound (1.79 g, 78%) as a white solid.
$^1$H-NMR (DMSO-$d_6$) δ: 1.37-1.41 (9H, m), 3.40-3.54 (2H, m), 3.61-4.04 (2H, m), 4.52-4.64 (1H, m), 8.10 (1H, d, J=4.4

Hz), 13.13 (1H, brs).
MS (ESI) m/z: 245.

Step 6: (2S)-1-(tert-Butoxycarbonyl)-N,N-dimethyl-5-oxopiperazine-2-carboxamide

The compound (244 mg, 1.00 mmol) obtained in Step 5 above and 1-hydroxybenzotriazole (203 mg, 1.50 mmol) were dissolved in dimethylformamide (2 ml), a 50% aqueous solution of dimethylamine (0.16 ml, 1.5 mmol) and subsequently 1-(3-dimethylaminopropyl)-3-ethylcarbodiimide hydrochloride (288 mg, 1.50 mmol) were added with stirring under ice cooling, and the resulting mixture was stirred overnight while gradually heated to room temperature. The reaction mixture was concentrated under reduced pressure. Saturated aqueous sodium bicarbonate solution was added to the residue and then common salt was added until saturation, followed by extraction with chloroform. The organic layer was dried over anhydrous magnesium sulfate and then the solvent was evaporated under reduced pressure. The residue was purified by silica gel column chromatography (chloroform:methanol = 40:1 → 10:1) to give the title compound (270 mg, 99%) as a colorless oil.
$^1$H-NMR ($CDCl_3$) δ: 1.47 (9H, s), 2.97 (3H, s), 3.08 (3H, s), 3.54-3.69 (2H, m), 4.08-4.31 (2H, m), 5.17-5.19 (1H, m), 6.75 (1H, brs).
MS (ESI) m/z: 272.
The title compound was led to an amine compound by the removal of the tert-butoxycarbonyl group through the same reaction as in Step 6 of Reference Example 9, which was in turn used directly in the reaction shown in Examples.

Reference Example 5

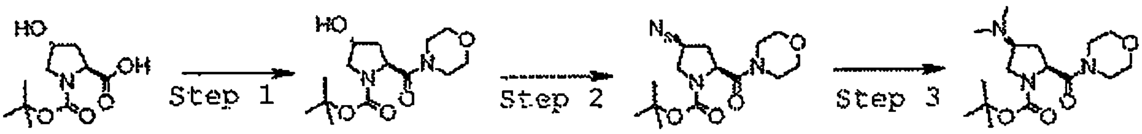


Step 1: tert-Butyl (2S,4R)-4-Hydroxy-2-(morpholin-4-ylcarbonyl)pyrrolidine-1-carboxylate

(4R)-1-(tert-Butoxycarbonyl)-4-hydroxy-L-proline (11.6 g, 50.0 mmol) and 1-hydroxybenzotriazole (8.11 g, 60.0 mmol) were dissolved in dimethylformamide (100 ml), morpholine (5.25 ml, 60 mmol) and subsequently 1-(3-dimethylaminopropyl)-3-ethylcarbodiimide hydrochloride (12.5 g, 65.0 mmol) were added with stirring under ice cooling, and the resulting mixture was stirred overnight while gradually heated to room temperature. The reaction mixture was concentrated under reduced pressure. Saturated aqueous sodium bicarbonate solution was added to the residue, followed by extraction with ethyl acetate. The organic layer was dried over anhydrous magnesium sulfate and then the solvent was evaporated under reduced pressure. The residue was purified by silica gel column chromatography (chloroform:methanol = 50:1 → 10:1) to give the title compound (10.4 g, 69%) as a colorless oil.
$^1$H-NMR ($CDCl_3$) δ: 1.41-1.45 (9H, m), 1.95-2.24 (2H, m), 2.67-2.91 (1H, m), 3.46-3.82 (9H, m), 4.48-4.54 (1H, m), 4.69-4.81 (1H, m).
MS (ESI) m/z: 301.

Step 2: tert-Butyl (2S,4S)-4-Azido-2-(morpholin-4-ylcarbonyl)pyrrolidine-1-carboxylate

The compound (2.40 g, 7.99 mmol) obtained in Step 1 above was dissolved in dry tetrahydrofuran (50 ml), triphenylphosphine (2.62 g, 9.99 mmol), diisopropyl azodicarboxylate (2.16 ml, 10.4 mmol), and subsequently diphenylphosphoryl azide (2.15 ml, 9.99 mmol) were added with stirring under ice cooling, and the resulting mixture was stirred at this temperature for 10 minutes and then overnight at room temperature. The reaction mixture was concentrated under reduced pressure. The residue was dissolved in ethyl acetate, washed with 1 N aqueous hydrochloric acid solution, saturated aqueous sodium bicarbonate solution, and brine, and then dried over anhydrous magnesium sulfate. The solvent was evaporated under reduced pressure. The residue was purified by silica gel column chromatography (chloroform:methanol = 100:1) to give the title compound (2.03 g, 78%) as a colorless oil.

Step 3: (3S,5S)-1-(tert-Butoxycarbonyl)-N,N-dimethyl-5-(morpholin-4-ylcarbonyl)pyrrolidin-3-amine

The compound (1.00 g, 3.07 mmol) obtained in Step 2 above was dissolved in methanol (15 ml), 5% palladium-carbon (0.5 g) was added, and the resulting mixture was stirred at room temperature for 100 minutes in a hydrogen atmosphere. The catalyst was removed by filtration and then the solvent was evaporated under reduced pressure. 1,2-Dichloroethane was added to the residue and the resulting mixture was concentrated under reduced pressure. The residue was dissolved in 1,2-dichloroethane (15 ml), a 37% formalin solution (0.572 ml, 7.68 mmol) and subsequently sodium triacethoxyborohydride (1.82 g, 8.60 mmol) were added with stirring under ice cooling, and the resulting mixture was stirred overnight while gradually heated to room temperature. Chloroform was added to the reaction mixture and the organic layer was washed with saturated aqueous sodium bicarbonate solution and then dried over anhydrous magnesium sulfate. The solvent was evaporated under reduced pressure. The residue was purified by silica gel column chromatography (chloroform:methanol = 40:1 $\rightarrow$ 10:1) to give the title compound (703 mg, 70%) as a white solid.
$^1$H-NMR ($CDCl_3$) δ: 1.41-1.46 (9H, m), 1.65-1.80 (2H, m), 2.23-2.24 (6H, m), 2.32-2.41 (1H, m), 2.61-2.74 (1H, m), 3.21-3.27 (1H, m), 3.48-3.91 (8H, m), 4.49-4.63 (1H, m).
MS (ESI) m/z: 328.
The title compound was led to an amine compound by the removal of the tert-butoxycarbonyl group through the same reaction as in Step 6 of Reference Example 9, which was in turn used directly in the reaction shown in Examples.

Reference Example 6

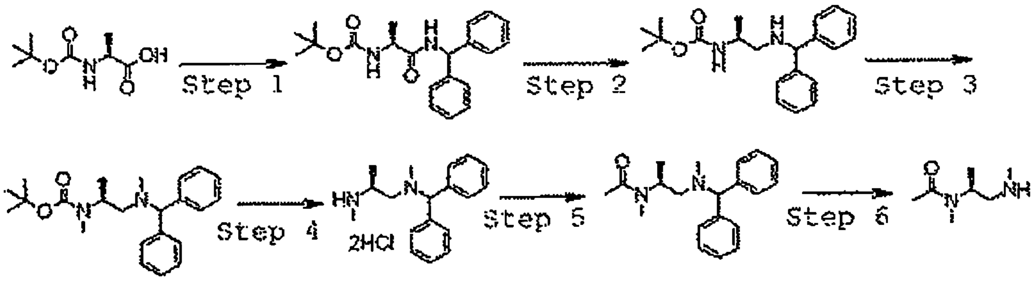


Step 1: tert-Butyl {(1S)-2-[(Diphenylmethyl)amino]-1-methyl-2-oxoethyl}carbamate

N-(tert-Butoxycarbonyl)-L-alanine (3.8 g, 0.02 mol) was dissolved in N,N-dimethylformamide (40 ml), 1-hydroxybenzotriazole (270 mg, 2.0 mmol) and 1-(3-dimethylaminopropyl)-3-ethylcarbodiimide hydrochloride (4.6 g, 0.024 mol) were added, and the resulting mixture was stirred at room temperature for 10 minutes. Benzhydrylamine (4.4 g, 0.024 mol) was added and the resulting mixture was heated and stirred at 55°C for 5 hours. The reaction mixture was brought to room temperature, ice water (100 ml) and saturated aqueous sodium bicarbonate solution (50 ml) were added, and the deposited solid was filtered. The obtained solid was dissolved in ethyl acetate. The organic layer was washed with a 10% aqueous solution of citric acid, saturated aqueous sodium bicarbonate solution, and brine. The resulting organic layer was dried over anhydrous magnesium sulfate. After filtration, the filtrate was evaporated under reduced pressure. The residue was recrystallized with diethyl ether and hexane and dried under reduced pressure at 60°C to give the title compound (4.78 g, 67%) as a colorless solid.
$^1$H-NMR ($CDCl_3$) δ: 1.38 (3H, d, J=7.1 Hz), 1.43 (9H, s), 4.21 (1H, brs), 4.92 (1H, brs), 6.21 (1H, d, J=8.1 Hz), 6.97 (1H, brs), 7.21-7.34 (10H, m).
MS (ESI) m/z: 377.

Step 2: tert-Butyl {(1S)-2-[(Diphenylmethyl)amino]-1-methylethyl}carbamate

The compound (4.1 g, 0.012 mol) obtained in Step 1 above was dissolved in tetrahydrofuran (20 ml) in a nitrogen atmosphere, 1 M tetrahydrofuran solution (51 ml, 0.051 mol) of a borane-tetrahydrofuran complex was added dropwise under ice cooling, and then the resulting mixture was stirred at room temperature for 20 hours. The reaction mixture was cooled on ice again, methanol (20 ml) was added dropwise, and the resulting mixture was stirred for 1 hour. The reaction solvent was evaporated under reduced pressure. The residue was purified by silica gel column chromatography (hexane:ethyl acetate = 7:1) to give the title compound (1.21 g, 30%) as a colorless solid.

$^1H$-NMR ($CDCl_3$) δ: 1.13 (3H, d, J=6.8 Hz), 1.44 (9H, s), 2.53 (1H, dd, J=12.0, 6.6 Hz), 2.61 (1H, dd, J=12.0, 4.9 Hz), 3.74-3.87 (1H, m), 4.54-4.66 (1H, m), 4.81 (1H, s), 7.18-7.23 (2H, m), 7.27-7.33 (4H, m), 7.37-7.40 (4H, m).
MS (ESI) m/z: 341.

Step 3: tert-Butyl {(1S)-2-[(Diphenylmethyl)(methyl)amino]-1-methylethyl}methylcarbamate

The compound (1.85 g, 5.43 mmol) obtained in Step 2 above was dissolved in tetrahydrofuran (20 ml), sodium hydride (60% oil, 650 mg, 16.3 mmol) and methyl iodide (2.0 ml, 32.6 mmol) were added, and then the resulting mixture was heated to reflux at 70°C for 2 days. The reaction mixture was cooled on ice once, sodium hydride (60% oil, 440 mg, 10.9 mmol) and methyl iodide (2.0 ml, 32.6 mmol) were added again, and then the resulting mixture was heated to reflux at 70°C for 3 hours. The reaction mixture was brought to room temperature and diluted with ethyl acetate. The organic layer was washed with water and brine and dried over anhydrous magnesium sulfate. After filtration, the filtrate was evaporated under reduced pressure. The residue was purified by silica gel column chromatography (hexane:ethyl acetate = 20:1) to give the title compound (695 mg, 35%) as a colorless oil.
$^1H$-NMR ($CDCl_3$) δ: 1.02 (3H, d, J=6.8 Hz), 1.48 and 1.50 (total 9H, each s), 2.08-2.12 (1H, m), 2.19 (3H, s), 2.38-2.42 (1H, m), 2.52 and 2.56 (total 3H, each s), 4.36-4.44 (1H, m), 4.53-4.61 (1H, m), 7.16-7.20 (2H, m), 7.24-7.28 (4H, m), 7.36-7.39 (4H, m).
MS (ESI) m/z: 369.

Step 4: (2S)-N,$N^2$-Dimethyl-N-(diphenylmethyl)propane-1,2-diamine dihydrochloride

The compound (350 mg, 0.95 mmol) obtained in Step 3 above was dissolved in dioxane (6 ml), a 4 N solution of hydrochloric acid in dioxane (2 ml) was added, and the resulting mixture was heated and stirred at 40°C for 4 hours. The reaction mixture was brought to room temperature and the reaction solvent was evaporated under reduced pressure to give the title compound (325 mg, 100%) as a colorless solid.
$^1H$-NMR (DMSO-$d_6$) δ: 1.21 (3H, brs), 2.74-2.88 (2H, m), 3.40 (3H, brs), 3.43 (3H, brs), 4.03-4.05 (1H, m), 5.66-5.68 (1H, m), 7.22-7.59 (6H, m), 7.66-8.00 (4H, m), 9.17 (1H, brs), 9.53 (1H, brs).
MS (ESI) m/z: 269.

Step 5: N-{(1S)-2-[(Diphenylmethyl)(methyl)amino]-1-methylethyl}-N-methylacetamide

The compound (325 mg, 0.95 mmol) obtained in Step 4 above was dissolved in tetrahydrofuran (10 ml), triethylamine (530 μl, 3.8 mmol) and acetyl chloride (101μ l, 1.43 mmol) were added under ice cooling, and then the resulting mixture was stirred at room temperature for 16 hours. The mixture was diluted with ethyl acetate. The organic layer was washed with saturated aqueous sodium bicarbonate solution and brine and dried over anhydrous magnesium sulfate. After filtration, the filtrate was evaporated under reduced pressure. The residue was purified by silica gel column chromatography (hexane:ethyl acetate = 4:1) to give the title compound (329 mg, 100%) as a colorless oil.
$^1H$-NMR ($CDCl_3$) δ: 1.01 and 1.11 (total 3H, each d, J=6.8 Hz), 2.12 and 2.19 (total 3H, each s), 2.13-2.18 (2H, m), 2.20 (3H, s), 2.40 and 2.54 (total 1H, each dd, J=12.4, 9.3 Hz), 2.53 and 2.66 (total 3H, each s), 3.99-4.04 and 5.02-5.09 (total 1H, each m), 4.35 and 4.41 (total 1H, each s), 7.17-7.20 (2H, m), 7.24-7.30 (4H, m), 7.33-7.38 (4H, m).

Step 6: N-Methyl-N-[(1S)-1-methyl-2-(methylamino)ethyl]acetamide

The compound (320 mg, 1.03 mmol) obtained in Step 5 above was dissolved in ethanol (4 ml), 20% palladium hydroxide-carbon (50 mg) was added, and the resulting mixture was stirred at room temperature for 2 hours in a hydrogen atmosphere under atmospheric pressure. The reaction mixture was filtered through celite and the filtrate was evaporated under reduced pressure. The residue was purified by silica gel column chromatography (chloroform → chloroform: methanol = 9:1) to give the title compound (130 mg, 88%) as a colorless oil.
$^1H$-NMR ($CDCl_3$) δ: 1.08 and 1.16 (total 3H, each d, J=6.8 Hz), 2.11 and 2.15 (total 3H, each s), 2.41 and 2.44 (total 3H, each s), 2.49-2.58 (1H, m), 2.63-2.73 (1H, m), 2.76 and 2.83 (total 3H, each s), 3.97-4.05 and 4.83-4.91 (total 1H, each m), 4.35 and 4.41 (total 1H, each s), 7.17-7.20 (2H, m), 7.24-7.30 (4H, m), 7.33-7.38 (4H, m).
MS (ESI) m/z: 145.

Reference Example 7

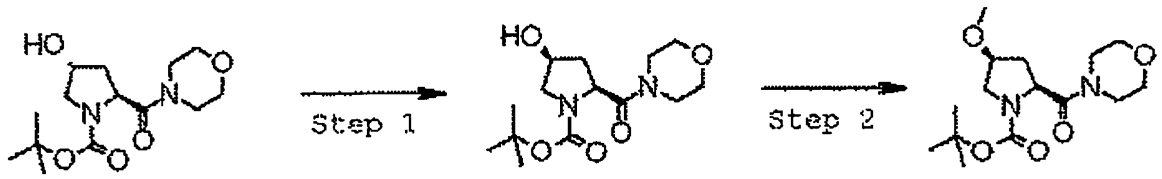


Step 1: tert-Butyl (2S,4S)-4-Hydroxy-2-(morpholin-4-ylcarbonyl)pyrrolidine-1-carboxylate

The compound (4.51 g, 15.0 mmol) obtained in Step 1 of Reference Example 5, triphenylphosphine (4.72 g, 18.0 mmol), and 4-nitrobenzoic acid (3.04 g, 18.2 mmol) were dissolved in dry tetrahydrofuran (100 ml), and diisopropyl azodicarboxylate (3.73 ml, 18.0 mmol) was added with stirring under ice cooling, and the resulting mixture was stirred at this temperature for 5 minutes and then overnight at room temperature. The reaction mixture was concentrated under reduced pressure. The residue was purified by silica gel column chromatography (hexane:ethyl acetate = 3:7) to give 4-nitrobenzoic acid ester (9.38 g). This ester was dissolved in tetrahydrofuran (70 ml), an aqueous solution (20 ml) of lithium hydroxide (479 mg, 20 mmol) was added, and the resulting mixture was stirred for 2.5 hours. 1 N aqueous hydrochloric acid solution was added to the reaction mixture to adjust its pH to approximately 7. Then, the solvent was evaporated under reduced pressure. Saturated aqueous sodium bicarbonate solution was added to the residue, followed by extraction with chloroform. The organic layer was dried over anhydrous magnesium sulfate and then concentrated under reduced pressure. The residue was purified by silica gel column chromatography (chloroform:methanol = 100: 1→20:1) to give the title compound (3.84 g, 85%) as a white solid.
$^1$H-NMR ($CDCl_3$) δ: 1.24-1.61 (9H, m), 1.93-1.99 (1H, m), 2.22-2.34 (1H, m), 3.46-3.98 (10H, m), 4.31-4.37 (1H, m), 4.65-4.72 (1H, m), 4.79 (1H, d, J=9.5 Hz), 5.70 (1H, d, J=11.7 Hz).
MS (ESI) m/z: 301.

Step 2: 4-[(4S)-1-tert-Butoxycarbonyl-4-methoxy-L-prolyl]morpholine

The compound (1.05 g, 3.50 mmol) obtained in Step 1 above was dissolved in dry dimethylformamide (5 ml) and sodium hydride (50% oil, 235 mg, 4.90 mmol) was added with stirring under ice cooling. The resulting mixture was stirred at room temperature for 1 hour and then methyl iodide (1.09 ml, 17.5 mmol) was added with stirring under ice cooling. The resulting mixture was stirred at room temperature for 45 minutes and then ice water was added, followed by extraction with ethyl acetate. The organic layer was dried over anhydrous magnesium sulfate and then concentrated under reduced pressure. The residue was purified by silica gel column chromatography (chloroform:methanol = 100:1) to give the title compound (773 mg, 70%) as a white solid.
$^1$H-NMR ($CDCl_3$) δ: 1.41-1.46 (9H, m), 1.84-1.92 (1H, m), 2.45-2.56 (1H, m), 3.31 (3H, s), 3.49-3.36 (3H, m), 3.49-4.00 (8H, m), 4.49-4.63 (1H, m).
MS (ESI) m/z: 315.

The title compound was led to an amine compound by the removal of the tert-butoxycarbonyl group through the same reaction as in Step 6 of Reference Example 9, which was in turn used directly in the reaction shown in Examples.

Reference Example 8

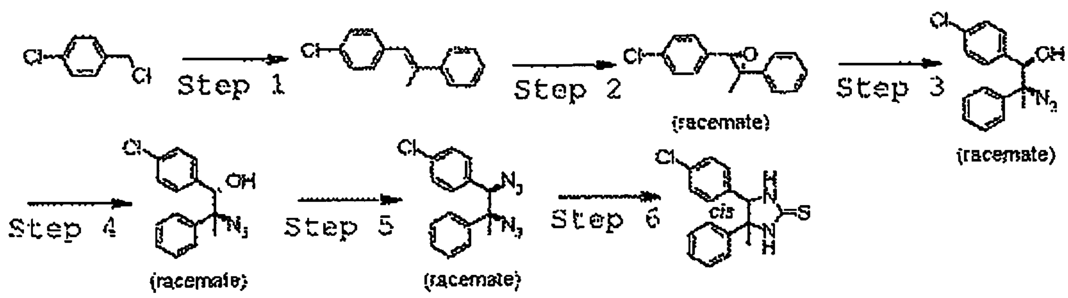

Step 1: 1-Chloro-4-[(1E)-2-phenylprop-1-en-1-yl]benzene

Magnesium powder (3.65 g, 150 mmol) and diethyl ether (80 ml) were placed in a 300-ml three-neck flask equipped with a reflux condenser and a diethyl ether (15 ml) solution of 4-chlorobenzyl chloride (25.0 g, 155 mmol) was added dropwise in 17 minutes with vigorous stirring at room temperature (the reaction was controlled by occasional ice cooling) while moderate reflux was maintained. After the completion of dropwise addition, the resulting mixture was stirred at room temperature for 20 minutes and a diethyl ether (80 ml) solution of acetophenone (21.6 g, 140 mmol) was added dropwise in 40 minutes. After the completion of dropwise addition, the resulting mixture was heated to reflux for 4 hours. 1 N aqueous hydrochloric acid solution (170 ml) was added to the obtained white slurry with stirring under ice cooling (slurry was gradually dissolved). The organic layer was separated, washed with brine, and then dried over anhydrous magnesium sulfate. The solvent was evaporated under reduced pressure to give a yellow oil. This substance was dissolved in benzene (200 ml), p-toluenesulfonic acid monohydrate (2.0 g) was added, and the resulting mixture was heated to reflux overnight while generated water was removed from the system using a Dean-Stark water separator. The reaction mixture was left standing to cool to room temperature, then washed with 1 N aqueous sodium hydroxide solution and brine, and dried over anhydrous magnesium sulfate. The solvent was evaporated under reduced pressure. Hexane was added to the residue and the resulting mixture was crystallized under ice cooling to give the title compound (16.2 g, 51%) as a light brown solid.
$^1$H-NMR ($CDCl_3$) δ: 2.25 (3H, brs), 6.76 (1H, s), 7.25-7.39 (7H, m), 7.52 (2H, d, J=8.1 Hz).

Step 2: (2R*,3R*)-3-(4-Chlorophenyl)-2-methyl-2-phenyloxirane

The compound (8.00 g, 35.0 mmol) obtained in Step 1 above was dissolved in acetonitrile (210 ml) and a 0.4 M aqueous solution of sodium ethylenediaminetetraacetate (140 ml) was added with stirring at room temperature. Tetrahydro-4H-thiopyran-4-one 1,1-dioxide (317 mg, 2.5 mmol) was added to the obtained suspension and then a mixture of oxone™ (34.4 g, 56.0 mmol) and sodium bicarbonate (14.1 g, 168 mmol) was gradually added over 5 hours with stirring at room temperature. Then, the resulting mixture was further stirred at room temperature for 30 minutes. Then, insoluble matter was removed by filtration and insoluble matter was washed with diethyl ether. The filtrates were combined, followed by extraction with diethyl ether twice. The organic layer was washed with brine. After drying over anhydrous magnesium sulfate, the solvent was evaporated under reduced pressure to give the title compound (8.50 g) as a colorless oil.

Step 3: (1R*,2S*)-2-Azido-1-(4-chlorophenyl)-2-phenylpropan-1-ol

The compound (8.44 g, 34.5 mmol) obtained in Step 2 above was dissolved in dimethylformamide (100 ml), sodium azide (6.83 g, 105 mmol) and ammonium chloride (3.74 g, 70.0 mmol) were added, and the resulting mixture was stirred at 105°C for 14 hours. The mixture was left standing to cool to room temperature and then the solvent was evaporated under reduced pressure. Water was added to the residue, followed by extraction with diethyl ether. The organic layer was washed with brine and then dried over anhydrous magnesium sulfate. The solvent was evaporated under reduced pressure. The residue was purified by silica gel column chromatography (hexane:ethyl acetate = 9:1 → 4:1) to give the title compound (9.78 g) as a colorless oil. This compound contained approximately 12% structural isomers as impurities.
$^1$H-NMR ($CDCl_3$) δ: 1.73 (3H, s), 2.34 (1H, d, J=3.7 Hz), 4.68 (1H, d, J=3.7 Hz), 6.99 (2H, d, J=8.3 Hz), 7.17 (2H, d, J=8.3 Hz), 7.23-7.32 (5H, m).

Step 4: (1R*,2R*)-2-Azido-1-(4-chlorophenyl)-2-phenylpropan-1-ol

The compound obtained in Step 3 above was reacted in the same way as in Step 1 of Reference Example 7 to give the title compound. This compound contained structural isomers as impurities.
$^1$H-NMR ($CDCl_3$) δ: 1.61 (3H, s), 4.76 (1H, d, J=2.4 Hz), 6.81 (2H, d, J=8.5 Hz), 7.11-7.34 (7H, m).

Step 5: 1-Chloro-4-[(1R*,2S*)-1,2-diazido-2-phenylpropyl]benzene

The compound obtained in Step 4 above was reacted in the same way as in Step 2 of Reference Example 5 to give the title compound.
$^1$H-NMR ($CDCl_3$) δ: 1.76 (3H, s), 4.61 (1H, s), 6.99 (2H, d, J=8.5 Hz), 7.20 (2H, d, J=8.5 Hz), 7.25-7.31 (5H, m).

Step 6: (4R*,5S*)-5-(4-Chlorophenyl)-4-methyl-4-phenylimidazolidine-2-thione

The compound (675 mg, 2.16 mmol) obtained in Step 5 above was dissolved in anhydrous tetrahydrofuran (40 ml) and lithium aluminium hydride powder (328 mg, 8.63 mmol) was added under ice cooling. The resulting mixture was stirred at this temperature for 15 minutes and then at room temperature for 1.5 hours. Water (330 μl), a 5 N aqueous solution of sodium hydroxide (330 μl), and water (990 μl) were added to the reaction mixture with stirring under ice cooling. Insoluble matter was removed by filtration and then the residue was dried over anhydrous magnesium sulfate. The solvent was evaporated under reduced pressure to obtain a diamine compound. This compound was dissolved in ethanol (15 ml), carbon disulfide (1 ml, 16 mmol) was added, and the resulting mixture was heated to reflux for 2 hours. The reaction mixture was left standing to cool, then concentrated under reduced pressure, and purified by silica gel column chromatography (hexane:ethyl acetate = 2:1) to give the title compound (326 mg, 50%) as a colorless oil.

This compound is the compound obtained in Step 1 of Example 83.

Reference Example 9

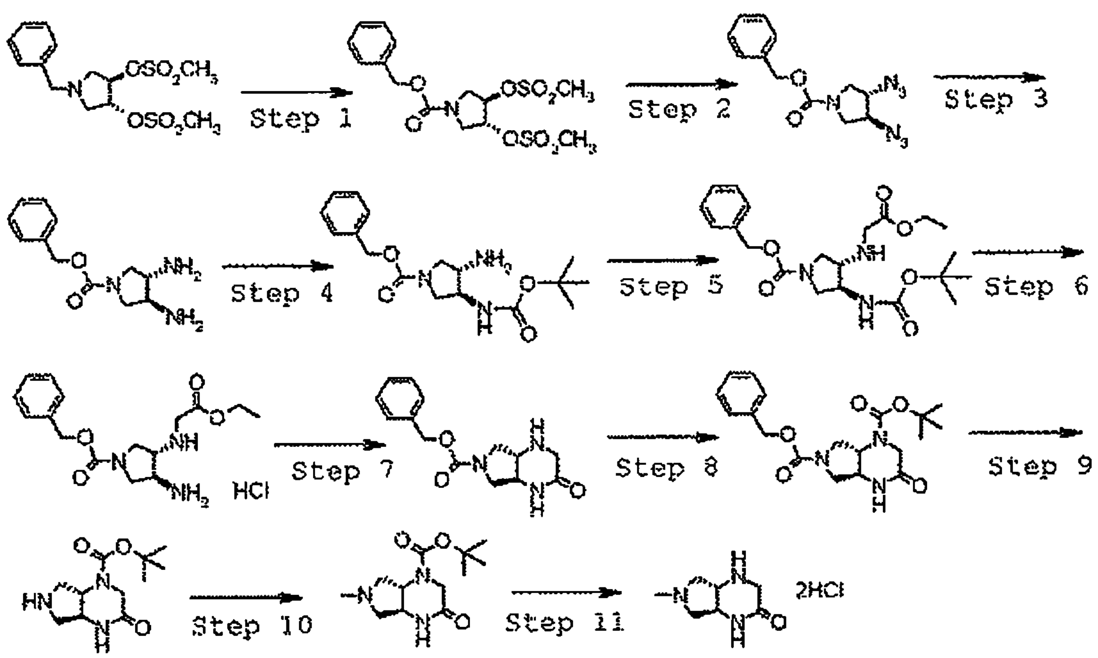


Step 1: Benzyl (3R,4R)-3,4-Bis[(methylsulfonyl)oxy]pyrrolidine-1-carboxylate

(3R,4R)-1-Benzylpyrrolidine-3,4-diyl dimethanesulfonate (7.7 g, 0.022 mol) was dissolved in dichloromethane (100 ml), benzyloxycarbonyl chloride (4.7 ml, 0.033 mol) was added, and the resulting mixture was stirred at room temperature for 20 hours. The reaction solvent was evaporated under reduced pressure. The residue was purified by silica gel column chromatography (hexane:ethyl acetate = 1:1 → 1:2) to give the title compound (7.77 g, 90%) as a colorless solid.

$^1H$-NMR ($CDCl_3$) δ: 3.10 (6H, s), 3.79-3.90 (4H, m), 5.16 (2H, s), 5.20-5.23 (2H, brm), 7.35-7.39 (5H, m).

Step 2: Benzyl (3S,4S)-3,4-Diazidopyrrolidine-1-carboxylate

The compound (7.77 g, 0.02 mol) obtained in Step 1 above was dissolved in a mixed solvent of N,N-dimethylformamide (64 ml) and water (16 ml), sodium azide (10.45 g, 0.16 mol) was added, and the resulting mixture was heated and stirred at 100°C for 24 hours. The reaction mixture was brought to room temperature and diluted with ethyl acetate. The organic layer was washed with water and brine and dried over anhydrous magnesium sulfate. After filtration, the filtrate was evaporated under reduced pressure. The residue was purified by silica gel column chromatography (hexane:ethyl acetate = 6:1) to give the title compound (3.65 g, 64%) as a colorless oil.

$^1$H-NMR ($CDCl_3$) δ: 3.51 (2H, m), 3.74 (2H, m), 3.98-4.01 (2H, m), 5.15 (2H, s), 7.31-7.38 (5H, m).

Step 3: Benzyl (3S,4S)-3,4-Diaminopyrrolidine-1-carboxylate

The compound (3.65 g, 0.013 mol) obtained in Step 2 above was dissolved in ethyl acetate (40 ml), a Lindlar catalyst (3.5 g) was added, and the resulting mixture was stirred at room temperature for 16 hours in a hydrogen atmosphere under atmospheric pressure. The reaction mixture was filtered through celite and the filtrate was evaporated under reduced pressure to give the title compound (2.87 g, 94%) as a pale yellow oil.
$^1$H-NMR ($CDCl_3$) δ: 1.35 (4H, brs), 3.07-3.10 (2H, m), 3.09-3.13 (2H, m), 3.75-3.81 (2H, m), 5.13 (2H, s), 7.29-7.38 (5H, m).

Step 4: Benzyl (3S,4S)-3-Amino-4-[(tert-butoxycarbonyl)amino]pyrrolidine-1-carboxylate

The compound (1.69 g, 5.88 mmol) obtained in Step 3 above was dissolved in tetrahydrofuran (80 ml), triethylamine (983 μl, 7.06 mmol) was added, and then a tetrahydrofuran (10 ml) dilution of di-tert-butyl dicarbonate (1.35 ml, 5.88 mmol) was added dropwise at 0°C. The resulting mixture was stirred for 1 hour under ice cooling. The mixture was diluted with ethyl acetate and the organic layer was washed with water and brine and dried over anhydrous magnesium sulfate. After filtration, the filtrate was evaporated under reduced pressure. The residue was purified by silica gel column chromatography (chloroform:methanol = 40:1 → 20:1) to give the title compound (875 mg, 48%) as a colorless solid.
$^1$H-NMR ($CDCl_3$) δ: 1.36 (2H, brs), 1.44 (9H, s), 3.13-3.18 (1H, m), 3.18-3.25 (1H, m), 3.30-3.38 (1H, m), 3.67-3.72 (1H, m), 3.77-3.82 (1H, m), 3.85-3.92 (1H, m), 4.61 (1H, brs), 5.12 (2H, s), 7.31-7.37 (5H, m).

Step 5: Benzyl (3S,4S)-3-[(tert-Butoxycarbonyl)amino]-4-[(2-ethoxy-2-oxoethyl)amino]pyrrolidine-1-carboxylate

The compound (870 mg, 2.6 mmol) obtained in Step 4 above was dissolved in acetonitrile (10 ml), potassium carbonate (540 mg, 3.9 mmol) and ethyl bromoacetate (346 μl, 3.12 mmol) were added, and the resulting mixture was heated and stirred at 55°C for 16 hours. The reaction mixture was brought to room temperature and the reaction solvent was evaporated under reduced pressure. The residue was dissolved in ethyl acetate, washed with water and brine, and dried over anhydrous magnesium sulfate. After filtration, the filtrate was evaporated under reduced pressure. The residue was purified by silica gel column chromatography (chloroform:methanol = 70:1 → 50:1) to give the title compound (757 mg, 69%) as a pale yellow oil.
$^1$H-NMR ($CDCl_3$) δ: 1.27 (3H, t, J=7.1 Hz), 1.44 (9H, s), 1.78 (1H, brs), 3.16-3.27 (3H, m), 3.41 (2H, d, J=17.6 Hz), 3.49 (2H, d, J=17.6 Hz), 3.66-3.72 (1H, m), 3.84-3.93 (2H, m), 4.19 (2H, q, J=7.1 Hz), 4.69 (1H, m), 5.12 (2H, s), 7.30-7.36 (5H, m).

Step 6: Benzyl (3S,4S)-3-Amino-4-[(2-ethoxy-2-oxoethyl)amino]pyrrolidine-1-carboxylate hydrochloride

The compound (750 mg, 1.72 mmol) obtained in Step 5 above was dissolved in 1,4-dioxane (10 ml), a 4 N solution of hydrochloric acid in dioxane (4 ml) was added, and the resulting mixture was stirred at room temperature for 16 hours. The reaction solvent was evaporated under reduced pressure and a crude product containing the title compound was directly subjected to next reaction.

Step 7: Benzyl (4aS,7aS)-2-Oxooctahydro-6H-pyrrolo[3,4-b]pyrazine-6-carboxylate

The compound obtained in Step 6 above was dissolved in ethanol (8 ml), triethylamine (2 ml) was added, and the resulting mixture was heated to reflux at 85°C for 3 days. The reaction mixture was brought to room temperature and the reaction solvent was evaporated under reduced pressure. Then, ethyl acetate was added to the residue and the resulting slurry was filtered. The filtrate was evaporated under reduced pressure to give a crude product as a light brown solid containing the title compound, which was in turn subjected directly to next reaction.

Step 8: 6-Benzyl 1-tert-Butyl (4aS,7aS)-3-oxooctahydro-1H-pyrrolo[3,4-b]pyrazine-1,6(2H)-dicarboxylate

The compound obtained in Step 7 above was dissolved in tetrahydrofuran (10 ml), triethylamine (288 μl, 2.06 mmol) and di-tert-butyl dicarbonate (474 μl, 2.06 mmol) were added, and the resulting mixture was heated and stirred at 60°C for 1 hour. The reaction mixture was brought to room temperature and diluted with ethyl acetate. The organic layer was washed with brine and dried over anhydrous magnesium sulfate. After filtration, the filtrate was evaporated under reduced pressure. The residue was purified by silica gel column chromatography (chloroform:methanol = 50:1) to give the title compound (600 mg, 93%, 3 steps) as a pale pink solid.
$^1$H-NMR ($CDCl_3$) δ: 1.46 and 1.48 (total 9H, each s), 3.17-3.24 (1H, m), 3.43-3.49 (2H, m), 3.70-3.76 (1H, m), 3.87-3.94

(1H, m), 4.09-4.14 (2H, m), 4.31-4.35 (1H, m), 5.12 (1H, d, J=12.5 Hz), 5.17 (1H, d, J=12.2 Hz), 6.64 and 6.81 (total 1H, each brs), 7.32-7.37 (5H, m).

Step 9: tert-Butyl (4aS,7aS)-3-Oxooctahydro-1H-pyrrolo[3,4-b]pyrazine-1-carboxylate

The compound (590 mg, 1.57 mmol) obtained in Step 8 above was dissolved in ethanol (10 ml), a 10% palladium-carbon catalyst (100 mg) was added, and the resulting mixture was stirred at room temperature for 24 hours in a hydrogen atmosphere under atmospheric pressure. The reaction mixture was filtered through celite and the filtrate was evaporated under reduced pressure to give the title compound (351 mg, 93%) as a colorless solid.
$^1$H-NMR ($CDCl_3$) δ: 1.45and1.46 (total 9H, each s), 3.34-3.54 (2H, m), 3.65-3.76 (1H, m), 3.90-4.24 (5H, m), 6.33 (1H, brs).

Step 10: tert-Butyl (4aS,7aS)-6-Methyl-3-oxooctahydro-1H-pyrrolo[3,4-b]pyrazine-1-carboxylate

The compound (350 mg, 1.45 mmol) obtained in Step 9 above was dissolved in a mixed solvent of 1,4-dioxane (10 ml) and methanol (2 ml), sodium triacetoxyborohydride (614 mg, 2.90 mmol) was added at 0°C, and the resulting mixture was stirred at room temperature for 1 hour. The reaction solvent was evaporated under reduced pressure. Chloroform was added to the residue and the resulting slurry was filtered. The filtrate was evaporated under reduced pressure. The residue was purified by silica gel column chromatography (chloroform:methanol = 40:1 → 9:1) to give the title compound (100 mg, 27%) as a colorless solid.
$^1$H-NMR ($CDCl_3$) δ: 1.46 (9H, s), 2.50 (3H, s), 2.68 (1H, dd, J=10.1, 8.7 Hz), 3.00-3.07 (2H, m), 3.38-3.50 (2H, m), 3.72-3.81 (1H, m), 4.09 (2H, d, J=1.2 Hz), 6.35 (1H, brs). Step 11: (4aS,7aS)-6-Methyloctahydro-2H-pyrrolo[3,4-b]pyrazin-2-one dihydrochloride

The compound (100 mg, 0.39 mmol) obtained in Step 10 above was dissolved in 1,4-dioxane (4 ml), a 4 N solution of hydrochloric acid in dioxane (2 ml) was added, and the resulting mixture was stirred at room temperature for 3 days. The reaction solvent was evaporated under reduced pressure. Ethanol was added to the residue and the resulting slurry was collected by filtration and dried under reduced pressure at 60°C to give the title compound (83 mg, 94%) as a colorless solid.
$^1$H-NMR (DMSO-$d_6$) δ: 2.92 (3H, s), 3.53-3.57 (2H, m), 3.60-3.63 (1H, m), 3.87-3.95 (2H, m), 3.96-4.04 (2H, m), 4.17-4.23 (1H, m).

Reference Example 10

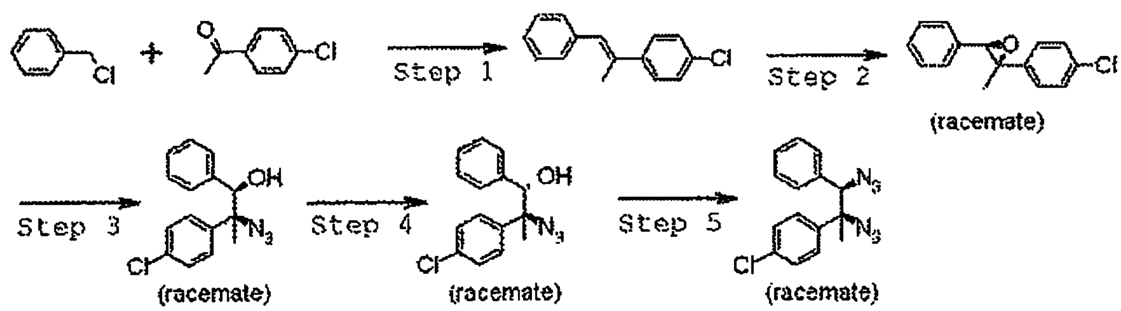


Step 1: 1-Chloro-4-[(E)-1-methyl-2-phenylvinyl]benzene

Benzyl chloride and 4'-chloroacetophenone were reacted in the same way as in Step 1 of Reference Example 8 to give the title compound as a white solid.
$^1$H-NMR ($CDCl_3$) δ: 2.25 (3H, d, J=1.5 Hz), 6.81 (1H, brs), 7.21-7.27 (1H, m), 7.30-7.39 (6H, m), 7.45 (2H, d, J=8.8 Hz).

Step 2: (2R*,3R*)-2-(4-Chlorophenyl)-2-methyl-3-phenyloxirane

The compound obtained in Step 1 above was reacted in the same way as in Step 2 of Reference Example 8 to give the title compound as a colorless oil.

Step 3: (1R*,2S*)-2-Azido-2-(4-chlorophenyl)-1-phenylpropan-1-ol

The compound obtained in Step 2 above was reacted in the same way as in Step 3 of Reference Example 8 to give the title compound as a colorless oil. This compound contained structural isomers as impurities.
$^1$H-NMR ($CDCl_3$) δ: 1.74 (3H, s), 2.31 (1H, d, J=3.4 Hz), 4.71 (1H, d, J=3.4 Hz), 7.02-7.27 (9H, m).

Step 4: (1R*,2R*)-2-Azido-2-(4-chlorophenyl)-1-phenylpropan-1-ol

The compound obtained in Step 3 above was reacted in the same way as in Step 1 of Reference Example 7 to give the title compound as a colorless oil. This compound contained structural isomers as impurities.
$^1$H-NMR ($CDCl_3$) δ: 1.64 (3H, s), 2.60 (1H, d, J=2.9 Hz), 4.76 (1H, d, J=2.7 Hz), 6.92 (2H, d, J=7.6 Hz), 7.16-7.30 (7H, m).

Step 5: 1-Chloro-4-[(1R*,2S*)-1,2-diazido-2-phenyl-1-methylethyl]benzene

The compound obtained in Step 4 above was reacted in the same way as in Step 2 of Reference Example 5 to give the title compound.
$^1$H-NMR ($CDCl_3$) δ: 1.75 (3H, brs), 4.62 (1H, s), 7.06 (2H, d, J=8.3 Hz), 7.20-7.30 (7H, m).
This compound was reacted in the same way as in Step 6 of Reference Example 8 to give the compound shown in Step 1 of Example 86.

Reference Example 11

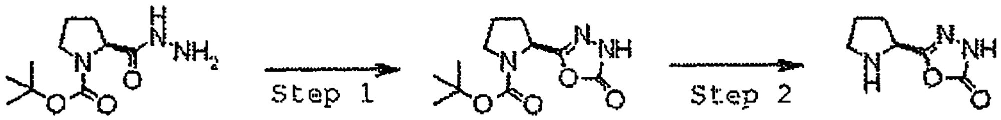


Step 1: tert-Butyl (2S)-2-(5-Oxo-4,5-dihydro-1,3,4-oxadiazol-2-yl)pyrrolidine-1-carboxylate

tert-Butyl (2S)-2-hydrazinocarbonylpyrrolidine-1-carboxylate (600 mg, 2.62 mmol) was dissolved in tetrahydrofuran (10 ml), 1,1'-carbonylbis-1H-imidazole (422 mg, 2.62 mmol) was added under ice cooling, and then the resulting mixture was stirred at room temperature for 3 hours. The mixture was diluted with ethyl acetate, washed with brine, and dried over anhydrous magnesium sulfate. The solvent was evaporated under reduced pressure. The residue was purified by silica gel column chromatography (chloroform:methanol = 50:1) to give the title compound (567 mg, 85%) as a colorless solid.
$^1$H-NMR ($CDCl_3$) δ: 1.40 and 1.46 (1H, each s), 1.92-1.99 (1H, m), 2.02-2.12 (2H, m), 2.18-2.27 (1H, m), 3.42-3.58 (2H, m), 4.63-4.67 and 4.74-4.79 (total 1H, each m). Step 2: 5-((2S)-Pyrrolidin-2-yl)-1,3,4-oxadiazol-2(3H)-one hydrochloride The compound obtained in Step 1 above was reacted in the same way as in Step 11 of Reference Example 9 to give the title compound as a colorless solid.
$^1$H-NMR (DMSO-$d_6$) δ: 1.92-2.05 (2H, m), 2.11-2.19 (1H, m), 2.21-2.28 (1H, m), 3.22-3.26 (2H, m), 4.64 (1H, t, J=7.7 Hz), 10.12 (1H, s), 12.69 (1H, s).
MS (ESI) m/z: 156.

Reference Example 12

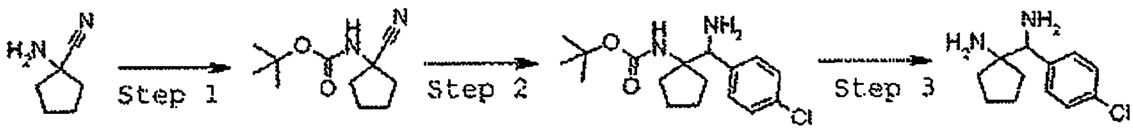

Step 1: tert-Butyl (1-Cyanocyclopentyl)carbamate

1 N aqueous sodium hydroxide solution (100 ml) and di-tert-butyl dicarbonate (14.4 g, 65.9 mmol) were added to a dioxane (250 ml) suspension of 1-aminocyclopentane carbonitrile (8.05 g, 54.9 mmol) and the resulting mixture was stirred at room temperature for 19 hours.
The mixture was diluted with water, followed by extraction with ethyl acetate. The extract was washed with brine and then dried over anhydrous sodium sulfate. The solvent was evaporated under reduced pressure. Then, the residue was purified by silica gel column chromatography (hexane:ethyl acetate = 3:1) to give the title compound (7.61 g, 66%) as a colorless solid.
$^1$H-NMR ($CDCl_3$) δ: 1.49 (9H, s), 1.82-1.88 (4H, m), 2.00-2.08 (2H, m), 2.31-2.34 (2H, m), 4.76 (1H, brs).
MS (EI) m/z: 210.

Step 2: tert-Butyl {1-[Amino(4-chlorophenyl)methyl]cyclopentyl}carbamate

4-Chlorophenylmagnesium bromide (1.0 M diethyl ether solution; 42 ml, 42 mmol) was added dropwise under ice cooling to a toluene (120 ml) solution of the compound (3.86 g, 18.3 mmol) obtained in Step 1 above. The resulting mixture was brought to room temperature, stirred for 3 hours, and then cooled on ice again and a methanol (10 ml) suspension of sodium borohydride (1.38 g, 36.4 mmol) was added dropwise. The resulting mixture was stirred for 3 hours and then the reaction mixture was diluted with water, followed by extraction with ethyl acetate. The organic layer was washed with brine and dried over anhydrous sodium sulfate. The solvent was evaporated under reduced pressure. Then, the residue was purified by silica gel column chromatography (chloroform:methanol = 10:1) to give the title compound (4.69 g, 79%) as a pale yellow oil.
$^1$H-NMR ($CDCl_3$) δ: 1.44 (9H, s), 1.53-1.87 (8H, m), 4.32 (1H, s), 4.47 (1H, s), 7.25-7.28 (4H, m).

Step 3: 1-[Amino(4-chlorophenyl)methyl]cyclopentanamine

Trifluoroacetic acid (60 ml) was added dropwise under ice cooling to a dichloromethane (60 ml) solution of the compound (4.69 g, 14.4 mmol) obtained in Step 2 above and the resulting mixture was stirred at room temperature for 3 hours. The reaction mixture was evaporated under reduced pressure. Then, the residue was diluted with dichloromethane and made weakly basic with 1 N aqueous sodium hydroxide solution, followed by extraction with dichloromethane. The organic layer was washed with brine and then dried over anhydrous sodium sulfate. The solvent was evaporated under reduced pressure to give the title compound (2.56 g, 79%) as a brown oil.
$^1$H-NMR ($CDCl_3$) δ: 0.97-1.02 (1H, m), 1.34-1.94 (7H, m), 3.86 (1H, s), 7.27-7.34 (4H, m).

Reference Example 13

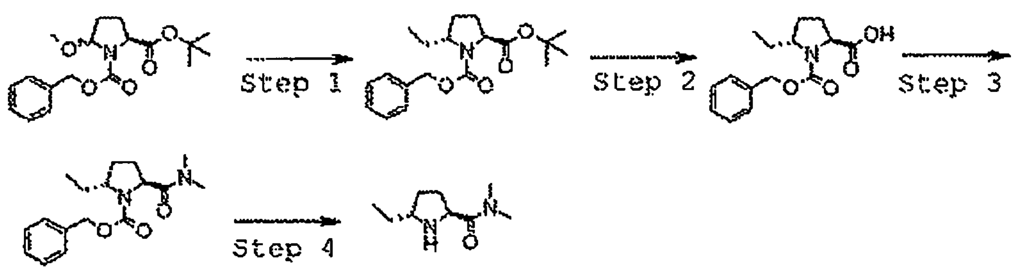


Step 1: 1-Benzyl 2-tert-Butyl (2S,5R)-5-ethylpyrrolidine-1,2-dicarboxylate

Diethyl ether (50 ml) and bromo(dimethyl sulfide)copper (I) (2.45 g, 11.9 mmol) were added to a container subjected in advance to nitrogen substitution. A 0.5 M solution of ethyllithium in benzene/cyclohexane (9:1) (22.4 ml, 11.9 mmol) was added dropwise at -78°C and the resulting mixture was stirred for 30 minutes. Then, boron trifluoride-diethyl ether (1.51 ml, 11.9 mmol) was added at -78°C and the resulting mixture was stirred for 5 minutes. Subsequently, a tetrahydrofuran (10 ml) solution of 1-benzyl 2-tert-butyl (2S)-5-methoxypyrrolidine-1,2-dicarboxylate (2.0 g, 5.96 mmol) was added dropwise at -78°C and then the resulting mixture was heated to room temperature and stirred for 3 hours. An aqueous solution of ammonium chloride (100 ml) was added to the reaction mixture and the deposited matter was

filtered. The filtrate was diluted with ethyl acetate and the organic layer was washed with brine. The resulting organic layer was dried over anhydrous magnesium sulfate. After filtration, the filtrate was evaporated under reduced pressure. The residue was purified by silica gel column chromatography (hexane:ethyl acetate = 8:1) to give the title compound (1.25 g, 63%) as a colorless oil.
$^1$H-NMR ($CDCl_3$) δ: 0.83 and 0.89 (total 3H, each t, J=7.4 Hz), 1.33 and 1.44 (total 9H, each s), 1.66-1.73 (2H, m), 1.83-1.94 (2H, m), 1.99-2.07 (1H, m), 2.15-2.23 (1H, m), 3.92-3.99 (1H, m), 4.24 (1H, t, J=7.8 Hz), 5.06-5.20 (2H, m), 7.27-7.36 (5H, m).
MS (ESI) m/z: 356 (M+23).

Step 2: (5R)-1-[(Benzyloxy)carbonyl]-5-ethyl-L-proline

The compound (1.1 g, 3.3 mmol) obtained in Step 1 above was dissolved in chloroform (10 ml) and trifluoroacetic acid (4 ml) was added. The resulting mixture was stirred at room temperature for 1 hour, then heated to 60°C, and further heated and stirred for 1 hour. The reaction mixture was brought to room temperature and the reaction solvent was evaporated under reduced pressure. Ethyl acetate was added to the residue and the organic layer was washed with brine. The resulting organic layer was dried over anhydrous magnesium sulfate. After filtration, the filtrate was evaporated under reduced pressure to give the title compound (1.0 g) as a light brown oil.
$^1$H-NMR ($CDCl_3$) δ: 0.85 and 0.89 (total 3H, each t, J=7.6and7.9 Hz), 1.31-1.48 (2H, m), 1.73-1.79 and 1.82-1.89 (total 1H, each m), 2.02-2.32 (3H, m), 3.89-3.94 and 3.96-4.02 (total 1H, each m), 4.41 (1H, dd, J=13.5, 8.9 Hz), 5.06-5.23 (2H, m), 7.20-7.38 (5H, m).
MS (ESI) m/z: 278 (M+23).

Step 3: Benzyl (2S,5R)-2-[(Dimethylamino)carbonyl]-5-ethylpyrrolidine-1-carboxylate

The compound (437 mg, 1.58 mmol) obtained in Step 2 above was dissolved in dimethylformamide (6 ml), 1-hydroxybenzotriazole (21 mg, 0.158 mmol) and 1-(3-dimethylaminopropyl)-3-ethylcarbodiimide hydrochloride (454 mg, 2.37 mmol) were added, and the resulting mixture was stirred at room temperature for 10 minutes. Subsequently, dimethylamine hydrochloride (258 mg, 3.16 mmol) and diisopropylethylamine (550 μl, 3.16 mmol) were added and the resulting mixture was stirred at room temperature for 2 days. The mixture was diluted with ethyl acetate and washed with saturated aqueous sodium bicarbonate solution and brine. The organic layer was dried over anhydrous magnesium sulfate. After filtration, the filtrate was evaporated under reduced pressure. The residue was purified by silica gel column chromatography (chloroform:methanol = 80:1) to give the title compound (377 mg, 78%) as a colorless oil.
$^1$H-NMR ($CDCl_3$) δ: 0.84 and 0.90 (total 3H, each t, J=7.4 Hz), 1.33-1.41 (1H, m), 1.66-1.93 (3H, m), 2.15-2.25 (2H, m), 2.83 and 2.88 (total 3H, each s), 2.97 and 3.08 (total 3H, each s), 4.00-4.04 and 4.06-4.10 (total 1H, each m), 4.63 and 4.71 (total 1H, each d, J=8.3 Hz), 4.96 and 5.07 (total 1H, each d, J=12.2 Hz), 5.12 and 5.23 (total 1H, each d, J=12.2 Hz), 7.26-7.37 (5H, m).
MS (ESI) m/z: 305.

Step 4: (5R)-5-Ethyl-N,N-dimethyl-L-prolinamide

The compound (370 mg, 1.22 mmol) obtained in Step 3 above was dissolved in methanol (10 ml), 10% palladium-carbon (100 mg) was added, and the resulting mixture was stirred at room temperature for 5 hours in a hydrogen atmosphere under atmospheric pressure. The reaction mixture was filtered through celite and the filtrate was evaporated under reduced pressure. The residue was subjected to silica gel column chromatography (chloroform:methanol = 9:1) to give the title compound (204 mg, 98%) as a colorless oil.
$^1$H-NMR ($CDCl_3$) δ: 0.93 (3H, t, J=7.3 Hz), 1.37-1.49 (3H, m), 1.60-1.70 (1H, m), 1.89-1.97 (1H, m), 2.17-2.27 (1H, m), 2.97 (3H, s), 3.00 (3H, s), 3.16-3.23 (1H, m), 3.98 (1H, t, J=7.7 Hz).
MS (ESI) m/z: 171.

Reference Example 14

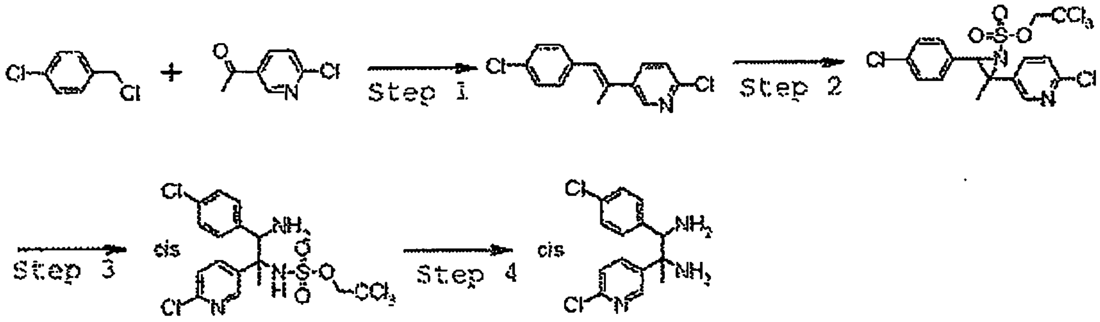


Step 1: 2-Chloro-5-[(E)-2-(4-chlorophenyl)-1-methylvinyl]pyridine

4-Chlorobenzyl chloride and 1-(6-chloropyridin-3-yl)ethanone were reacted in the same way as in Step 1 of Reference Example 8 to give the title compound as a pale yellow solid.
$^1$H-NMR ($CDCl_3$) δ: 2.24 (3H, d, J=1.2 Hz), 6.78 (1H, s), 7.26-7.37 (5H, m), 7.75 (1H, dd, J=8.4, 2.7 Hz), 8.52 (1H, dd, J=2.7, 0.7 Hz).

Step 2: 2,2,2-Trichloroethyl (2R*,3R*)-3-(4-Chlorophenyl)-2-(6-chloropyridin-3-yl)-2-methylaziridine-1-sulfonate

The compound (2.64 g, 10.0 mmol) obtained in Step 1 above, 2,2,2-trichloroethoxysulfonamide (2.50 g, 11.0 mmol), rhodiumbis(perfluorobutyrylamido) dimer (238 mg, 0.226 mmol), magnesium oxide (0.93 g, 23.0 mmol), and iodobenzene diacetate (4.18 g, 13.0 mmol) were suspended in benzene (20 ml) and the resulting solution was stirred overnight at room temperature in a nitrogen atmosphere. Ethyl acetate was added to the reaction mixture. Insoluble matter was removed by filtration and the filtrate was washed with saturated aqueous sodium bicarbonate solution and brine. After drying over anhydrous magnesium sulfate, the solvent was evaporated under reduced pressure. The residue was purified by silica gel column chromatography (hexane:ethyl acetate = 9:1) to give the title compound (1.68 g, 34%) as a pale yellow solid.
$^1$H-NMR ($CDCl_3$) δ: 1.44 (3H, s), 4.64 (1H, s), 4.80 (2H, s), 7.38-7.45 (5H, m), 7.94 (1H, dd, J=8.3, 2.7 Hz), 8.62 (1H, d, J=2.7 Hz).

Step 3: 2,2,2-Trichloroethyl [(1R*,2S*)-2-Amino-2-(4-chlorophenyl)-1-(6-chloropyridin-3-yl)-1-methylethyl]sulfamate

The compound (923 mg, 1.88 mmol) obtained in Step 2 above was suspended in a 7 M solution of ammonia in methanol (30 ml) and the resulting solution was stirred at room temperature for 20 hours. The solution was further stirred at 50°C for 1 hour and then the solvent was evaporated under reduced pressure to give the title compound (1.06 g, quantitative) as a light brown oil. $^1$H-NMR ($CDCl_3$) δ: 1.72 (3H, s), 3.49 (2H, d, J=1.0 Hz), 3.75 (1H, s), 4.36 (2H, s), 4.64 (1H, s), 6.83 (2H, d, J=8.3 Hz), 7.16 (2H, d, J=8.3 Hz), 7.23 (1H, d, J=8.3 Hz), 7.41 (1H, dd, J=8.3, 2.2 Hz), 8.19 (1H, d, J=2.2 Hz).

Step 4: (1R*,2S*)-1-(4-Chlorophenyl)-2-(6-chloropyridin-3-yl)propane-1,2-diamine

The compound (1.06 g) obtained in Step 3 above was dissolved in a 0.5 N solution of hydrochloric acid in methanol and the resulting solution was stirred at 60°C for 20 hours in a sealed tube. The reaction mixture was concentrated under reduced pressure, 1 N aqueous sodium hydroxide solution was added to the residue, and then common salt was added until saturation, followed by extraction with chloroform. The organic layer was dried over anhydrous magnesium sulfate and then the solvent was evaporated under reduced pressure. The residue was purified by silica gel column chromatography (chloroform:methanol = 49:1 → 9:1) to give the title compound (386 mg, 69%) as a colorless oil.
$^1$H-NMR ($CDCl_3$) δ: 1.52 (3H, s), 4.07 (1H, s), 6.96 (2H, d, J=8.3 Hz), 7.19 (2H, d, J=8.3 Hz), 7.21 (1H, d, J=8.5 Hz), 7.57 (1H, dd, J=8.5, 2.7 Hz), 8.33 (1H, d, J=2.7 Hz).

Reference Example 15

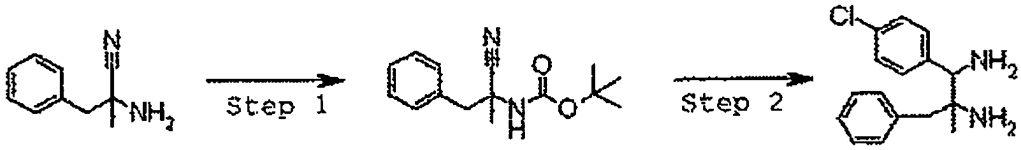


Step 1: tert-Butyl (1-Cyano-1-methyl-2-phenylethyl)carbamate

2-Amino-2-methyl-3-phenylpropanenitrile was reacted in the same way as in Step 1 of Reference Example 12 to give the title compound.
$^1$H-NMR ($CDCl_3$) δ: 1.50 (9H, s), 1.59 (3H, s), 3.17 (1H, d, J=13.4 Hz), 3.31 (1H, d, J=13.4 Hz), 4.58 (1H, brs), 7.25-7.38 (5H, m).

Step 2: 1-(4-Chlorophenyl)-2-methyl-3-phenylpropane-1,2-diamine

The compound obtained in Step 1 above was reacted in the same way as in Step 2 of Reference Example 12 and subsequently subjected to the same reaction as in Step 3 of Reference Example 12 to give the title compound as a diastereoisomer mixture.
$^1$H-NMR ($CDCl_3$) δ: 0.79-1.04 (3H, m), 2.55-2.85 (2H, m), 3.74-3.91 (1H, m), 7.14-7.40 (9H, m).

Reference Example 16

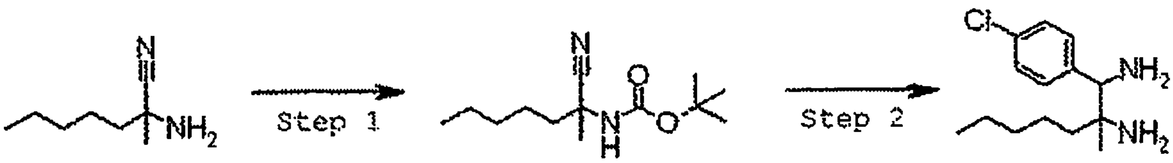


Step 1: tert-Butyl (1-Cyano-1-methylhexyl)carbamate

2-Amino-2-methylheptanenitrile was reacted in the same way as in Step 1 of Reference Example 12 to give the title compound as a pale yellow oil.
$^1$H-NMR ($CDCl_3$) δ: 0.91 (3H, t, J=7.0 Hz), 1.30-1.37 (4H, m), 1.43-1.54 (11H, m), 1.59 and 1.63 (total 3H, esch s), 1.74-1.94 (2H, m), 4.42 and 4.69 (total 1H, each brs). Step 2: 1-(4-Chlorophenyl)-2-methylheptane-1,2-diamine
The compound obtained in Step 1 above was reacted in the same way as in Step 2 of Reference Example 12 and subsequently subjected to the same reaction as in Step 3 of Reference Example 12 to give the title compound as a diastereoisomer mixture.

Reference Example 17

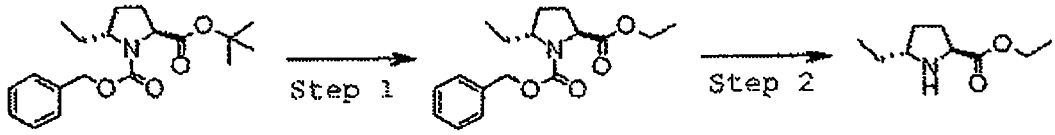

Step 1: 1-Benzyl 2-Ethyl (2S,5R)-5-ethylpyrrolidine-1,2-dicarboxylate

The compound (400 mg, 1.2 mmol) obtained in Step 1 of Reference Example 13 was reacted in the same way as in Step 2 of Reference Example 13. The obtained oil was dissolved in ethanol (10 ml), p-toluenesulfonic acid monohydrate (23 mg, 0.12 mmol) was added, and the resulting mixture was heated to reflux at 85°C for 16 hours. The reaction mixture was brought to room temperature and the reaction solvent was evaporated under reduced pressure. The residue was purified by silica gel column chromatography (hexane:ethyl acetate = 4:1) to give the title compound (275 mg, 75%) as a colorless oil. $^1$H-NMR ($CDCl_3$) δ: 1.19 (3H, t, J=7.1 Hz), 2.04-2.11 (1H, m), 2.30-2.40 (1H, m), 2.47-2.55 (1H, m), 2.61-2.70 (1H, m), 4.12-4.19 (2H, m), 4.67 (1H, dd, J=9.5, 2.7 Hz), 5.23 (1H, d, J=12.4 Hz), 5.32 (1H, d, J=12.4 Hz), 7.32-7.41 (5H, m).
MS (ESI) m/z: 306.

Step 2: Ethyl (5R)-5-Ethyl-L-prolinate

The compound obtained in Step 1 above was reacted in the same way as in Step 4 of Reference Example 13 to give the title compound as a colorless oil.
$^1$H-NMR ($CDCl_3$) δ: 0.96 (3H, t, J=7.4 Hz), 1.29 (3H, t, J=7.1 Hz), 1.47-1.57 (2H, m), 1.66-1.74 (1H, m), 1.87-1.93 (1H, m), 1.95-2.02 (1H, m), 2.27-2.34 (1H, m), 3.24-3.31 (1H, m), 4.03 (1H, dd, J=8.4, 6.2 Hz), 4.21 (2H, q, J=7.2 Hz).
MS (ESI) m/z: 172.

Reference Example 18

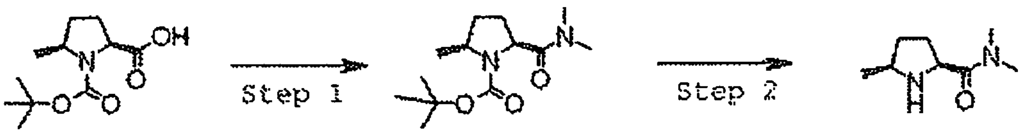


Step 1: tert-Butyl (2S,5S)-2-[(Dimethylamino)carbonyl]-5-methylpyrrolidine-1-carboxylate

(5S)-1-(tert-Butoxycarbonyl)-5-methyl-L-proline was reacted in the same way as in Step 3 of Reference Example 13 to give the title compound as a colorless oil.
$^1$H-NMR ($CDCl_3$) δ: 1.35 (3H, d, J=6.1 Hz), 1.40 and 1.46 (total 9H, each s), 1.66-1.75 (1H, m), 1.87-1.93 (1H, m), 2.01-2.12 (2H, m), 2.97 (3H, s), 3.07 and 3.11 (total 3H, each s), 3.91-3.95 and 4.02-4.07 (total 1H, each m), 4.53-4.58 and 4.68-4.72 (total 1H, each m).
MS (ESI) m/z: 157 (M-99).

Step 2: (5S)-N,N,5-Trimethyl-L-prolinamide hydrochloride

The compound obtained in Step 1 above was reacted in the same way as in Step 11 of Reference Example 9 to give the title compound as a colorless solid.
$^1$H-NMR (DMSO-$d_6$) δ: 1.32 (3H, d, J=6.6 Hz), 1.49-1.58 (1H, m), 1.81-1.90 (1H, m), 2.03-2.11 (1H, m), 2.32-2.42 (1H, m), 2.89 (3H, s), 2.98 (3H, s), 3.57-3.61 (1H, m), 4.55-4.60 (1H, m).
MS (ESI) m/z: 157.

Reference Example 19

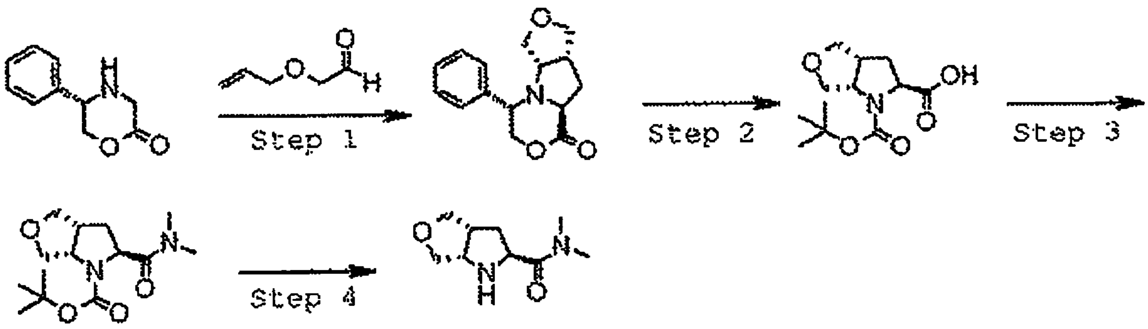


Step 1: (3aS,5S,8aS,9aR)-5-Phenyloctahydro-8H-furo[3',4':4,5]pyrrolo[2,1-c][1,4]oxazin-8-one

(Allyloxy)acetaldehyde (1.77 g, 0.018 mol) was dissolved in benzene (20 ml), a benzene (20 ml) solution of (5S)-5-phenylmorpholin-2-one (2.9 g, 0.016 mol) was added, and the resulting mixture was stirred at room temperature for 1 hour. Then, a dropping funnel filled with molecular sieves 3A was attached, and the mixture was heated to reflux for 16 hours while generated water was removed. The reaction mixture was brought to room temperature and the reaction solvent was evaporated under reduced pressure. The residue was purified by silica gel column chromatography (hexane: ethyl acetate = 4:1). The purified product was solidified using diethyl ether and hexane and dried under reduced pressure at 60°C to give the title compound (1.08 g, 23%) as a colorless solid.
$^1$H-NMR ($CDCl_3$) δ: 2.04-2.11 (1H, m), 2.61-2.68 (1H, m), 2.86-2.93 (1H, m), 3.34 (1H, dd, J=9.3, 5.1 Hz), 3.48-3.55 (2H, m), 3.64 (1H, dd, J=9.3, 6.6 Hz), 3.71 (1H, dd, J=9.1, 3.0 Hz), 3.92 (1H, dd, J=10.6, 4.8 Hz), 4.16-4.27 (3H, m), 7.31-7.44 (5H, m).
MS (ESI) m/z: 260.

Step 2: (2S,3aR,6aS)-1-(tert-Butoxycarbonyl)hexahydro-1H-furo[3,4-b]pyrrole-2-carboxylic acid

The compound (1.08 g, 4.17 mmol) obtained in Step 1 above was dissolved in methanol (100 ml), 20% palladium hydroxide-carbon (500 mg) and trifluoroacetic acid (2 ml) were added, and the resulting mixture was stirred at room temperature for 2 days in a hydrogen atmosphere under atmospheric pressure. The reaction mixture was filtered through celite and the filtrate was evaporated under reduced pressure. The residue was dissolved in 1,4-dioxane (30 ml), sodium bicarbonate (1.74 g, 20.9 mmol), water (20 ml), and di-tert-butyl dicarbonate (1.09 g, 5.0 mmol) were added at 0°C, and the resulting mixture was heated to room temperature and stirred for 2 days. A 10% aqueous solution of citric acid was added to the reaction mixture to adjust its pH to 3 to 4, followed by dilution with ethyl acetate. The organic layer was washed with brine and dried over anhydrous sodium sulfate. After filtration, the filtrate was evaporated under reduced pressure. The residue was purified by silica gel column chromatography (chloroform:methanol = 9:1) to give the title compound (811 mg, 76%) as a pale pink solid.
$^1$H-NMR (DMSO-$d_6$) δ: 1.33 and 1.37 (total 9H, each s), 1.91-2.13 (2H, m), 2.81-2.89 (1H, m), 3.42-3.56 (2H, m), 3.59 (1H, d, J=8.0 Hz), 3.75 (1H, d, J=9.8 Hz), 4.18-4.28 (2H, m), 12.63 (1H, brs).
MS (ESI) m/z: 280 (M+23).

Step 3: tert-Butyl (2S,3aR,6aS)-2-[(Dimethylamino)carbonyl]hexahydro-1H-furo[3,4-b]pyrrole-1-carboxylate

The compound obtained in Step 2 above was reacted in the same way as in Step 3 of Reference Example 13 to give the title compound as a colorless oil.
$^1$H-NMR ($CDCl_3$) δ: 1.39 and 1.45 (total 9H, each s), 2.00-2.09 (2H, m), 2.95 and 2.97 (total 3H, each s), 2.99-3.04 (1H, m), 3.03 and 3.06 (total 3H, each s), 3.53-3.57 (1H, m), 3.60-3.64 (1H, m), 3.68-3.73 (1H, m), 3.96 and 4.07 (total 1H, each d, J=9.8 Hz), 4.51 and 4.60 (total 1H, each t, J=6.1 Hz), 4.72 and 4.84 (total 1H, each t, J=5.4 Hz).

Step 4: (2S,3aR,6aS)-N,N-Dimethylhexahydro-1H-furo[3,4-b]pyrrole-2-carboxamide hydrochloride

The compound obtained in Step 3 above was reacted in the same way as in Step 11 of Reference Example 9 to give the title compound as a light brown solid.
$^1$H-NMR (DMSO-$d_6$) δ: 1.86-1.94 (1H, m), 2.28-2.34 (1H, m), 2.88 (3H, s), 3.00-3.05 (1H, m), 3.01 (3H, s), 3.57-3.64 (2H, m), 3.84 (1H, dd, J=9.1, 2.8 Hz), 4.23 (1H, d, J=10.7 Hz), 4.33-4.39 (1H, m), 4.41-4.47 (1H, m).
MS (ESI) m/z: 185.

Reference Example 20

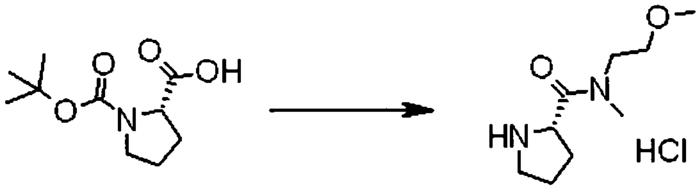

N-(2-Methoxyethyl)-N-methyl-L-prolinamide hydrochloride

1-(tert-Butoxycarbonyl)-1-proline and (2-methoxyethyl)methylamine were reacted in the same way as in Step 1 of Reference Example 5 to give an amide compound. Subsequently, this compound was reacted in the same way as in Step 11 of Reference Example 9 to give the title compound as a colorless oil.
$^1$H-NMR (DMSO-$d_6$) δ: 1.68-1.77 (1H, m), 1.83-1.94 (2H, m), 2.34-2.43 (1H, m), 2.89 and 3.01 (total 3H, each s), 3.10-3.17 (1H, m), 3.23 and 3.26 (total 3H, each s), 3.45-3.58 (5H, m), 4.48-4.56 (1H, m), 8.40 (1H, brs), 10.22 (1H, brs).
MS (EI) m/z: 187.

Reference Example 21

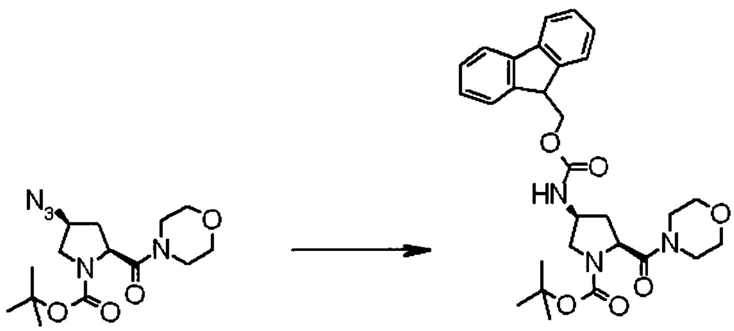

tert-Butyl (2S,4S)-4-{[(9H-Fluoren-9-ylmethoxy)carbonyl]amino}-2-(morpholin-4-ylcarbonyl)pyrrolidine-1-carboxylate

The compound (0.80 g, 2.46 mmol) obtained in Step 2 of Reference Example 5 was dissolved in methanol (15 ml), 5% palladium-carbon (0.5 g) was added, and the resulting mixture was stirred at room temperature for 3 hours in a hydrogen atmosphere. The catalyst was removed by filtration and then the solvent was evaporated under reduced pressure. The residue was dissolved in dichloromethane (20 ml), N-[(9H-fluoren-9-ylmethoxy)carbonyloxy]succinimide (843 mg, 2.50 mmol) and subsequently triethylamine (350 μl, 2.50 mmol) were added with stirring under ice cooling, and then the resulting mixture was stirred overnight at room temperature. The reaction mixture was washed with 1 N aqueous hydrochloric acid solution and saturated aqueous sodium bicarbonate solution and then dried over anhydrous magnesium sulfate. The solvent was evaporated under reduced pressure. The residue was purified by silica gel column chromatography (chloroform:methanol=100:1) to give the title compound (1.25 g, 98%) as a colorless oil.
$^1$H-NMR ($CDCl_3$) δ: 1.45-1.47 (9H, m), 1.86-1.90 (1H, m), 2.37-2.44 (1H, m), 3.52-3.84 (10H, m), 4.22-4.77 (5H, m), 6.39-7.77 (8H, m).
The title compound was led to an amine compound by the removal of the tert-butoxycarbonyl group through the same reaction as in Step 6 of Reference Example 9, which was in turn used directly in the reaction shown in Examples.

Reference Example 22

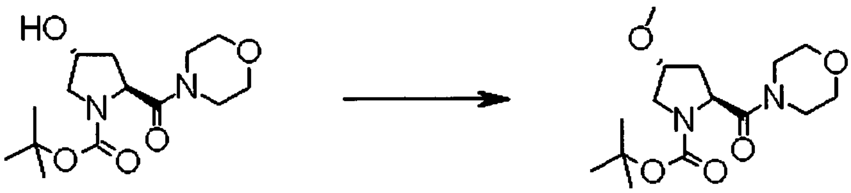

tert-Butyl (2S,4R)-4-Methoxy-2-(morpholin-4-ylcarbonyl)pyrrolidine-1-carboxylate
The compound obtained in Step 1 of Reference Example 5 was reacted in the same way as in Step 2 of Reference Example 7 to give the title compound as a colorless oil. $^1$H-NMR ($CDCl_3$) δ: 1.41-1.45 (9H, m), 1.97-2.28 (2H, m), 3.32 (3H, s), 3.51-3.81 (10H, m), 3.98-4.10 (1H, m), 4.63-4.77 (1H, m).
The title compound was led to an amine compound by the removal of the tert-butoxycarbonyl group through the same reaction as in Step 6 of Reference Example 9, which was in turn used directly in the reaction shown in Examples.

Reference Example 23

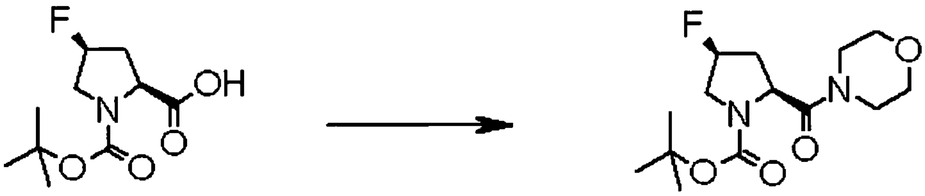

tert-Butyl (2S,4S)-4-Fluoro-2-(morpholin-4-ylcarbonyl)pyrrolidine-1-carboxylate

(4S)-1-tert-Butoxycarbonyl-4-fluoro-L-proline was reacted in the same way with morpholine as in Step 1 of Reference Example 5 to give the title compound as a pale yellow solid.
$^1$H-NMR ($CDCl_3$) δ: 1.43-1.47 (10H, m), 2.16-2.52 (2H, m), 3.23-3.95 (10H, m), 4.60-4.73 (1H, m), 5.15-5.29 (1H, m).
The title compound was led to an amine compound by the removal of the tert-butoxycarbonyl group through the same reaction as in Step 6 of Reference Example 9, which was in turn used directly in the reaction shown in Examples.

Reference Example 24

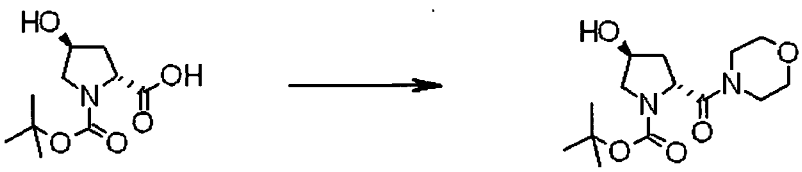

tert-Butyl (2R,4S)-4-Hydroxy-2-(morpholin-4-ylcarbonyl)pyrrolidine-1-carboxylate

(4S)-1-(tert-Butoxycarbonyl)-4-hydroxy-D-proline was reacted in the same way with morpholine as in Step 1 of Reference Example 5 to give the title compound as a colorless oil.

$^1$H-NMR ($CDCl_3$) δ: 1.40-1.45 (9H, m), 1.93-2.06 (1H, m), 2.13-2.23 (1H, m), 3.37-3.69 (11H, m), 4.46-4.51 (1H, m), 4.69-4.79 (1H, m).
The title compound was led to an amine compound by the removal of the tert-butoxycarbonyl group through the same reaction as in Step 6 of Reference Example 9, which was in turn used directly in the reaction shown in Examples.

Reference Example 25

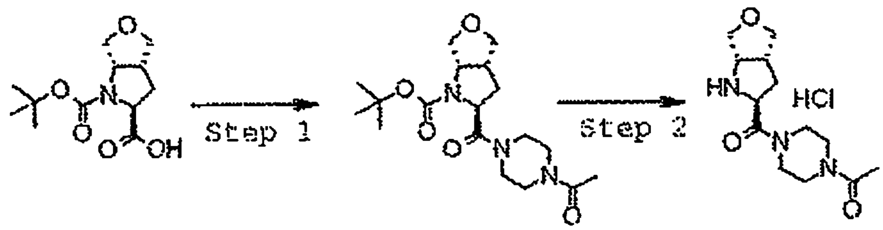


Step 1: tert-Butyl (2S,3aR,6aS)-2-[(4-Acetylpiperazin-1-yl)carbonyl]hexahydro-1H-furo[3,4-b]pyrrole-1-carboxylate

The compound obtained in Step 2 of Reference Example 19 was reacted in the same way as in Step 1 of Reference Example 5 using 1-acetylpiperazine instead of morpholine to give the title compound as a colorless solid.
$^1$H-NMR ($CDCl_3$) δ: 1.39 and 1.45 (total 9H, each s), 2.01-2.06 (1H, m), 2.12 and 2.14 (total 3H, each s), 2.94-2.99 and 3.05-3.10 (total 1H, each m), 3.41-3.80 (12H, m), 3.97 and 4.08 (total 1H, each d, J=10.3 Hz), 4.48-4.52 and 4.57-4.61 (total 1H, each m), 4.70-4.74 and 4.81-4.85 (total 1H, each m).
MS (ESI) m/z: 368.

Step 2: (2S,3aR,6aS)-2-[(4-Acetylpiperazin-1-yl)carbonyl]hexahydro-1H-furo[3,4-b]pyrrole hydrochloride

The compound obtained in Step 1 above was reacted in the same way as in Step 11 of Reference Example 9 to give the title compound as a colorless solid.
$^1$H-NMR (DMSO-$d_6$) δ: 2.00-2.05 (1H, m), 2.03 (3H, s), 2.27-2.32 (1H, m), 3.03-3.09 (1H, m), 3.47-3.53 (8H, m), 3.64-3.69 (2H, m), 3.85 (1H, dd, J=9.2, 3.1 Hz), 4.20 (1H, d, J=10.7 Hz), 4.39-4.44 (1H, m), 4.52-4.59 (1H, m).
MS (ESI) m/z: 268.

Reference Example 26

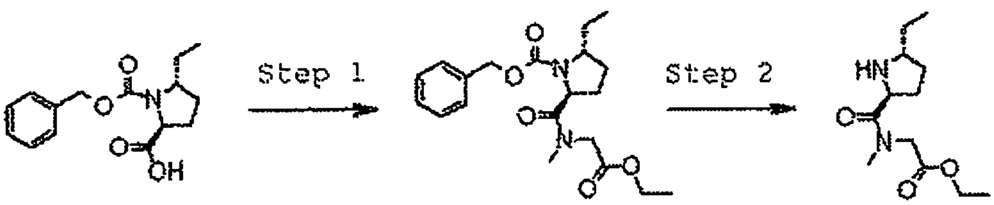


Step 1: Ethyl (5R)-1-[(Benzyloxy)carbonyl]-5-ethyl-L-prolyl-N-methylglycinate

The compound obtained in Step 2 of Reference Example 13 used instead of (5R,6S)-5,6-bis(4-chlorophenyl)-3-isopropyl-6-methyl-5,6-dihydroimidazo[2,1-b][1,3]thiazole-2-carboxylic acid and sarcosine ethyl hydrochloride used instead of piperazin-2-one were reacted in the same way as in Step 4 of Example 1 to give the title compound.
$^1$H-NMR ($CDCl_3$) δ: 0.85 and 0.90 (total 3H, each t, J=7.4 Hz), 1.25-1.29 (3H, m), 1.33-1.40 (1H, m), 1.68-1.74 (1H, m), 1.86-1.97 (1H, m), 2.14-2.23 (2H, m), 2.96 and 3.14 (total 3H, each s), 3.49 (1H, dd, J=17.4, 5.5 Hz), 3.98-4.07 (1H, m), 4.13-4.21 (2H, m), 4.45-4.81 (2H, m), 5.04-5.23 (2H, m), 7.28-7.35 (5H, m).

MS (ESI) m/z: 377.

Step 2: Ethyl (5R)-5-Ethyl-L-prolyl-N-methylglycinate

The compound obtained in Step 1 above was reacted in the same way as in Step 4 of Reference Example 13 to give the title compound as a pale yellow oil.
$^1$H-NMR (DMSO-$d_6$) δ: 0.89-0.94 (3H, m), 1.14-1.24 (3H, m), 1.57-1.70 (2H, m), 1.72-1.84 (2H, m), 2.04-2.13 (1H, m), 2.50-2.57 (1H, m), 3.06 (3H, s), 3.42-3.50 (1H, m), 4.01-4.29 (4H, m), 4.64-4.73 (1H, m).
MS (ESI) m/z: 243.

Reference Example 27

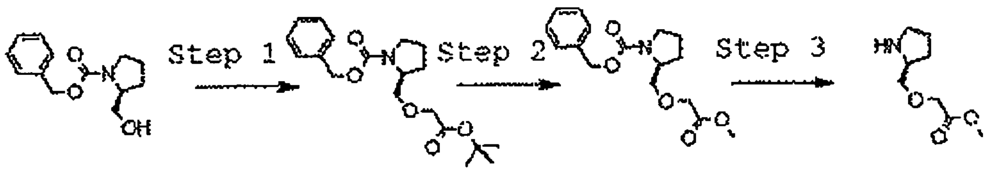


Step 1: Benzyl (2S)-2-[(2-tert-Butoxy-2-oxoethoxy)methyl]pyrrolidine-1-carboxylate

Benzyl (2S)-2-(hydroxymethyl)pyrrolidine-1-carboxylate (1.0 g, 4.25 mmol) was dissolved in benzene (20 ml), a 40% aqueous solution of sodium hydroxide (10 ml), tert-butyl bromoacetate (1.57 ml, 10.6 mmol), and tetra-n-butyl ammonium bisulfate (361 mg, 1.06 mmol) were added under ice cooling, and the resulting mixture was stirred at 5°C for 24 hours. 1 N aqueous hydrochloric acid solution (60 ml) was added to the reaction mixture and the resulting mixture was stirred for 10 minutes. The reaction mixture was diluted with ethyl acetate and the organic layer was washed with brine. After drying over anhydrous magnesium sulfate, the solvent was evaporated under reduced pressure. The obtained residue was purified by silica gel column chromatography (hexane:ethyl acetate = 4:1) to give the title compound (1.84 g, quantitative) as a colorless oil.
$^1$H-NMR ($CDCl_3$) δ: 1.47 (9H, s), 1.82-1.86 (1H, m), 1.92-1.99 (2H, m), 2.07-2.11 (1H, m), 3.38-3.46 (2H, m), 3.56-3.70 (2H, m), 3.90-4.10 (3H, m), 5.10-5.16 (2H, m), 7.30-7.38 (5H, m).
MS (ESI) m/z: 372 $(M+23)^+$.

Step 2: Benzyl (2S)-2-[(2-Methoxy-2-oxoethoxy)methyl]pyrrolidine-1-carboxylate

The compound (1.50 g, 4.25 mmol) obtained in Step 1 above was dissolved in chloroform (20 ml), trifluoroacetic acid (5 ml) was added, and the resulting mixture was heated and stirred at 60°C for 1 hour. The solvent was evaporated under reduced pressure. The obtained residue was dissolved in methanol (30 ml), then p-toluenesulfonic acid monohydrate (120 mg, 0.64 mmol) was added, and the resulting mixture was heated to reflux at 70°C for 16 hours. The solvent was evaporated under reduced pressure. The obtained residue was purified by silica gel column chromatography (hexane:ethyl acetate = 2:1) to give the title compound (1.23 g, 94%) as a colorless oil.
$^1$H-NMR ($CDCl_3$) δ: 1.82-1.87 (1H, m), 1.92-1.99 (2H, m), 2.04-2.10 (1H, m), 3.39-3.45 (2H, m), 3.57-3.63 (1H, m), 3.69-3.75 (4H, m), 4.02 and 4.11 (total 3H, each s), 5.08-5.19 (2H, m), 7.29-7.38 (5H, m).
MS (ESI) m/z: 308.

Step 3: Methyl [(2S)-Pyrrolidin-2-ylmethoxy]acetate

The compound obtained in Step 2 above was reacted in the same way as in Step 4 of Reference Example 13 to give the title compound as a light brown oil.
$^1$H-NMR (DMSO-$d_6$) δ: 1.57-1.64 (1H, m), 1.79-1.91 (2H, m), 1.95-2.02 (1H, m), 3.10-3.16 (2H, m), 3.63-3.67 (2H, m), 3.66 (3H, s), 3.70-3.74 (1H, m), 4.17-4.23 (2H, m).

Reference Example 28

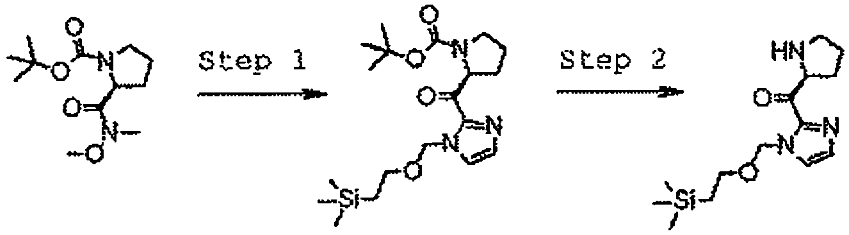


Step 1: tert-Butyl (2S)-2-[(1-{[2-(Trimethylsilyl)ethoxy]methyl}-1H-imidazol-2-yl)carbonyl]pyrrolidine-1-carboxylate

1-{[2-(Trimethylsilyl)ethoxy]methyl}-1H-imidazole was reacted in the same way as in Step 1 of Example 123 to give the title compound.
$^1$H-NMR ($CDCl_3$) δ: -0.01 (9H, s), 0.91-1.02 (2H, m), 1.28 and 1.47 (total 9H, each s), 1.88-2.02 (3H, m), 2.41 (1H, m), 3.46-3.68 (4H, m), 5.54-5.95 (3H, m), 7.22-7.38 (2H, m).

Step 2: tert-Butyl (2S)-2-[Methoxy(methyl)carbamoyl]pyrrolidine-1-carboxylate

The compound (680 mg, 1.72 mmol) obtained in Step 1 above was dissolved in 4 N hydrochloric acid/dioxane (8 ml) and the resulting solution was stirred at room temperature for 20 minutes. The reaction mixture was concentrated under reduced pressure. The residue was dissolved in chloroform and washed with saturated aqueous sodium bicarbonate solution. The organic layer was dried over anhydrous magnesium sulfate and then concentrated under reduced pressure to give the title compound. This compound was used in next reaction without being purified.

Reference Example 29

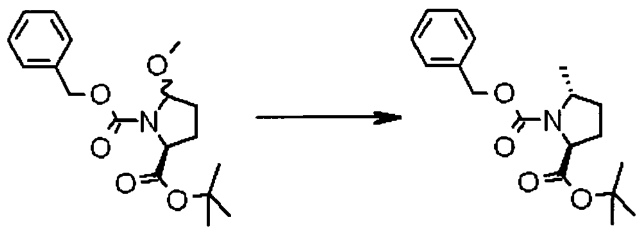

1-Benzyl 2-tert-Butyl (2S,5R)-5-methylpyrrolidine-1,2-dicarboxylate

1-Benzyl 2-tert-butyl(2S)-5-methoxypyrrolidine-1,2-dicarboxylate was reacted in the same way as in Step 1 of Reference Example 13 to give the title compound as a colorless oil.
$^1$H-NMR ($CDCl_3$) δ: 1.15 and 1.23 (total 3H, each d, J=6.3 Hz), 1.33 and 1.44 (total 9H, each s), 1.51-1.57 (1H, m), 1.89-1.95 (1H, m), 2.09-2.29 (2H, m), 4.13-4.28 (2H, m), 5.02-5.22 (2H, m), 7.22-7.38 (5H, m).
MS (ESI) m/z: 342 $(M+23)^+$.

Reference Example 30

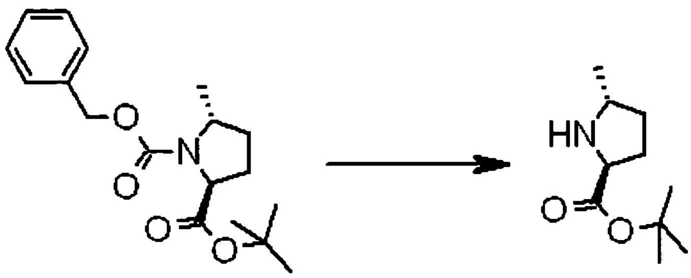

tert-Butyl (5R)-5-Methyl-L-prolinate

1-Benzyl 2-tert-butyl (2S,5R)-5-methylpyrrolidine-1,2-dicarboxylate was reacted in the same way as in Step 4 of Reference Example 13 to give the title compound as a colorless solid.
$^1$H-NMR ($CDCl_3$) δ: 1.22 (3H, d, J=6.1 Hz), 1.38-1.43 (1H, m), 1.47 (9H, s), 1.80-1.88 (1H, m), 1.88-1.95 (1H, m), 2.23-2.31 (1H, m), 3.38-3.46 (1H, m), 3.87 (1H, dd, J=8.7, 6.2 Hz).
MS (ESI) m/z: 186.

Reference Example 31

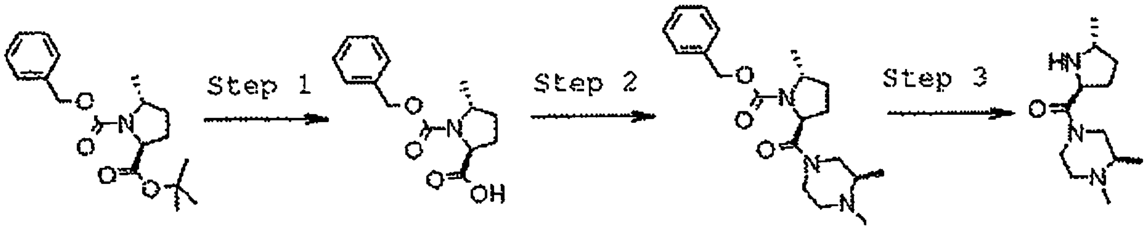


Step 1: (5R)-1-[(Benzyloxy)carbonyl]-5-methyl-L-proline

1-Benzyl 2-tert-butyl (2S,5R)-5-methylpyrrolidine-1,2-dicarboxylate was reacted in the same way as in Step 2 of Example 61 to give the title compound as a colorless oil.
$^1$H-NMR ($CDCl_3$) δ: 1.15-1.28 (3H, m), 1.53-1.63 (1H, m), 2.05-2.34 (3H, m), 4.12-4.25 (1H, m), 4.38-4.47 (1H, m), 5.06-5.23 (2H, m), 7.25-7.37 (5H, m).
MS (ESI) m/z: 264.

Step 2: Benzyl (2S,5R)-2-{[(3R)-3,4-Dimethylpiperazin-1-yl]carbonyl}-5-methylpyrrolidine-1-carboxylate

The compound obtained in Step 2 of Reference Example 13 used instead of (5R,6S)-5,6-bis(4-chlorophenyl)-3-isopropyl-6-methyl-5,6-dihydroimidazo[2,1-b][1,3]thiazole-2-carboxylic acid and (2R)-1,2-dimethylpiperazine used instead of piperazin-2-one were reacted in the same way as in Step 4 of Example 1 to give the title compound as a colorless oil.

Step 3: (2R)-1,2-Dimethyl-4-[(5R)-5-methyl-L-prolyl]piperazine

The compound obtained in Step 2 above was reacted in the same way as in Step 4 of Reference Example 13 to give the title compound.

Reference Example 32

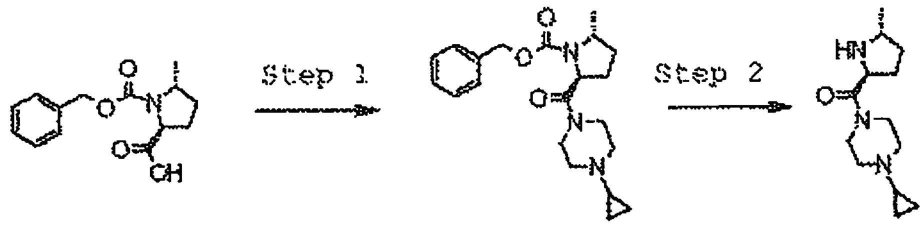


Step 1: Benzyl (2S,5R)-2-[(4-Cyclopropylpiperazin-1-yl)carbonyl]-5-methylpyrrolidine-1-carboxylate

The compound obtained in Step 2 of Reference Example 13 used instead of (5R,6S)-5,6-bis(4-chlorophenyl)-3-isopropyl-6-methyl-5,6-dihydroimidazo[2,1-b][1,3]thiazole-2-carboxylic acid and 1-cyclopropylpiperazine used instead of piperazin-2-one were reacted in the same way as in Step 4 of Example 1 to give the title compound as a colorless oil.
$^1$H-NMR ($CDCl_3$) δ: 0.35-0.49 (4H, m), 1.18 and 1.26 (total 3H, each d, J=6.3 Hz), 1.49-1.54 (1H, m), 1.76-1.81 (1H, m), 2.21-2.28 (1H, m), 2.30-2.37 (1H, m), 2.52-2.73 (4H, m), 3.30-3.43 (2H, m), 3.55-3.64 (3H, m), 4.23-4.31 (1H, m), 4.66 and 4.73 (total 1H, each d, J=7.8 Hz), 5.00 and 5.07 (total 1H, each d, J=12.4 Hz), 5.11 and 5.25 (total 1H, each d, J=12.4 Hz), 7.27-7.35 (5H, m).
MS (ESI) m/z: 372.

Step 2: 1-Cyclopropyl-4-[(5R)-5-methyl-L-prolyl]piperazine

The compound obtained in Step 1 above was reacted in the same way as in Step 4 of Reference Example 13 to give the title compound as a colorless solid.
$^1$H-NMR (DMSO-$d_6$) δ: 0.31-0.35 (2H, m), 0.41-0.45 (2H, m), 1.32 (3H, d, J=6.6 Hz), 1.55-1.66 (2H, m), 1.70-1.76 (1H, m), 2.01-2.08 (1H, m), 2.55-2.60 (1H, m), 3.34-3.49 (8H, m), 3.59-3.65 (1H, m), 4.61 (1H, t, J=8.4 Hz).
MS (ESI) m/z: 238.

Reference Example 33

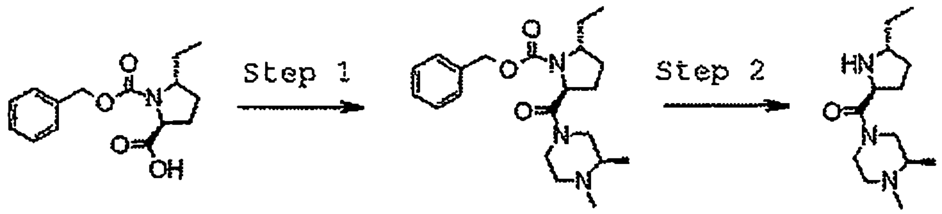


Step 1: Benzyl (2S,5R)-2-{[(3R)-3,4-Dimethylpiperazin-1-yl]carbonyl}-5-ethylpyrrolidine-1-carboxylate

The compound obtained in Step 2 of Reference Example 13 used instead of (5R,6S)-5,6-bis(4-chlorophenyl)-3-isopropyl-6-methyl-5,6-dihydroimidazo[2,1-b][1,3]thiazole-2-carboxylic acid and (2R)-1,2-dimethylpiperazine used instead of piperazin-2-one were reacted in the same way as in Step 4 of Example 1 to give the title compound as a colorless oil.
MS (ESI) m/z: 374.

Step 2: (2R)-4-[(5R)-5-Ethyl-L-prolyl]-1,2-dimethylpiperazine

The compound obtained in Step 1 above was reacted in the same way as in Step 4 of Reference Example 13 to give the title compound as a pale yellow solid.
$^1$H-NMR (DMSO-$d_6$) δ: 0.95 (3H, t, J=7.4 Hz), 1.09-1.11 (3H, m), 1.59-1.69 (2H, m), 1.78-1.88 (2H, m), 2.08-2.14 (1H, m), 2.34 (3H, brs), 2.42-2.47 (1H, m), 2.88-3.11 (5H, m), 3.46-3.55 (1H, m), 3.74-3.82 (1H, m), 4.03-4.20 (1H, m), 4.58-4.66 (1H, m).

MS (ESI) m/z: 240.

Reference Example 34

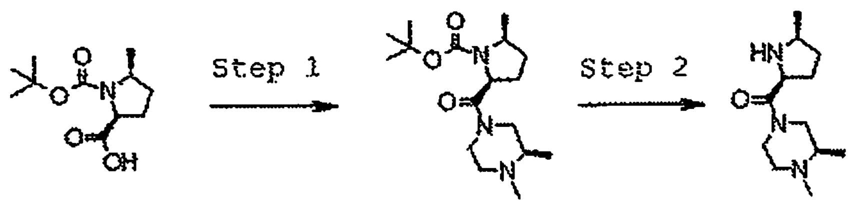


Step 1: tert-Butyl (2S,5S)-2-{[(3R)-3,4-Dimethylpiperazin-1-yl]carbonyl}-5-methylpyrrolidine-1-carboxylate

The compound obtained in Step 1 of Reference Example 18 used instead of (5R,6S)-5,6-bis(4-chlorophenyl)-3-isopropyl-6-methyl-5,6-dihydroimidazo[2,1-b][1,3]thiazole-2-carboxylic acid and (2R)-1,2-dimethylpiperazine used instead of piperazin-2-one were reacted in the same way as in Step 4 of Example 1 to give the title compound as a colorless solid.
MS (ESI) m/z: 326.

Step 2: (2R)-1,2-Dimethyl-4-[(5S)-5-methyl-L-prolyl]piperazine

The compound obtained in Step 1 above was reacted in the same way as in Step 2 of Example 214 to give the title compound as a colorless solid.
$^1$H-NMR (DMSO-$d_6$) δ: 1.15-1.22 (1H, m), 1.30-1.37 (6H, m), 1.50-1.58 (1H, m), 1.92-1.99 (1H, m), 2.02-2.15 (1H, m), 2.29-2.39 (1H, m), 2.68-2.73 (1H, m), 2.73-2.76 (3H, m), 2.98-3.09 (1H, m), 3.22-3.32 (1H, m), 3.42-3.51 (1H, m), 3.57-3.69 (1H, m), 4.00 and 4.10 (total 1H, each d, J=13.9 Hz), 4.37 and 4.43 (total 1H, each d, J=14.2 Hz), 4.56-4.67 and 4.74-4.82 (total 1H, each m).
MS (ESI) m/z: 226.

Reference Example 35

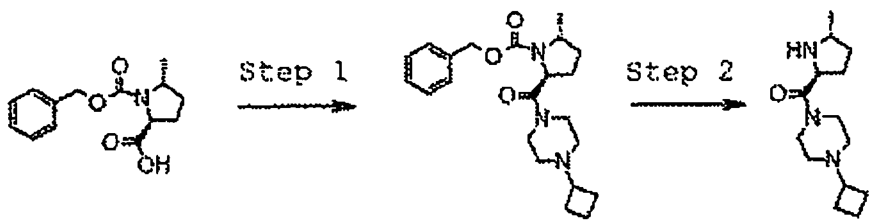


Step 1: Benzyl (2S,5R)-2-[(4-Cyclobutylpiperazin-1-yl)carbonyl]-5-methylpyrrolidine-1-carboxylate

The compound obtained in Step 1 of Reference Example 31 used instead of (5R,6S)-5,6-bis(4-chlorophenyl)-3-isopropyl-6-methyl-5,6-dihydroimidazo[2,1-b][1,3]thiazole-2-carboxylic acid and 1-cyclobutylpiperazine used instead of piperazin-2-one were reacted in the same way as in Step 4 of Example 1 to give the title compound as a colorless oil.
$^1$H-NMR ($CDCl_3$) δ: 1.17 and 1.25 (total 3H, each d, J=6.3 Hz), 1.64-1.90 (6H, m), 1.99-2.08 (4H, m), 2.24-2.37 (4H, m), 2.70-2.77 (1H, m), 3.34-3.49 (2H, m), 3.55-3.70 (2H, m), 4.22-4.31 (1H, m), 4.65 and 4.72 (total 1H, each d, J=7.8 Hz), 4.98-5.27 (2H, m), 7.28-7.37 (5H, m).
MS (ESI) m/z: 386.

Step 2: 1-Cyclobutyl-4-[(5R)-5-methyl-L-prolyl]piperazine

The compound obtained in Step 1 above was reacted in the same way as in Step 4 of Reference Example 13 to give the title compound as a pale orange solid.
$^1$H-NMR (DMSO-$d_6$) δ: 1.05 (3H, d, J=6.3 Hz), 1.26-1.32 (1H, m), 1.58-1.66 (3H, m), 1.76-1.84 (3H, m), 1.92-1.99 (2H, m), 2.12-2.17 (1H, m), 2.18-2.24 (4H, m), 2.68-2.76 (1H, m), 3.28-3.34 (1H, m), 3.42-3.46 (4H, m), 4.01 (1H, t, J=7.6 Hz).
MS (ESI) m/z: 252.

Reference Example 36

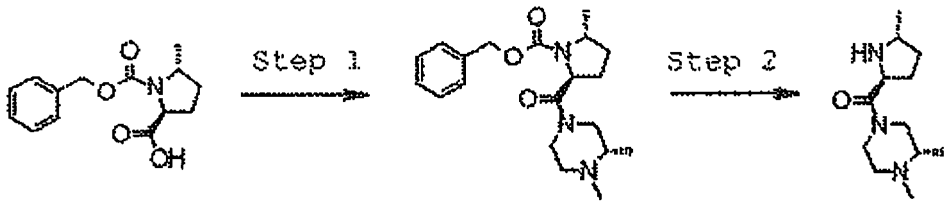


Step 1: Benzyl (2S,5R)-2-{[(3S)-3,4-Dimethylpiperazin-1-yl]carbonyl}-5-methylpyrrolidine-1-carboxylate

The compound obtained in Step 1 of Reference Example 31 used instead of (5R,6S)-5,6-bis(4-chlorophenyl)-3-isopropyl-6-methyl-5,6-dihydroimidazo[2,1-b][1,3]thiazole-2-carboxylic acid and (2S)-1,2-dimethylpiperazine used instead of piperazin-2-one were reacted in the same way as in Step 4 of Example 1 to give the title compound as a colorless oil.
$^1$H-NMR ($CDCl_3$) δ: 0.99 and 1.09 (total 3H, each dd, J=13.0, 6.2 Hz), 1.18 and 1.25 (total 3H, each d, J=6.3 Hz), 1.51-1.58 (1H, m), 1.75-1.88 (1H, m), 2.04-2.31 (5H, m), 2.37-2.54 (1H, m), 2.61-2.81 (2H, m), 2.96-3.07 (1H, m), 3.17-3.32 (1H, m), 3.46-3.81 (1H, m), 4.19-4.36 (2H, m), 4.61-4.75 (1H, m), 4.95-5.26 (2H, m), 7.28-7.36 (5H, m).
MS (ESI) m/z: 360.

Step 2: (2S)-1,2-Dimethyl-4-[(5R)-5-methyl-L-prolyl]piperazine

The compound obtained in Step 1 above was reacted in the same way as in Step 4 of Reference Example 13 to give the title compound as a pale yellow solid.
$^1$H-NMR (DMSO-$d_6$) δ: 0.96-1.02 (3H, m), 1.17 (3H, d, J=6.6 Hz), 1.38-1.49 (1H, m), 1.61-1.73 (1H, m), 1.89-1.99 (2H, m), 2.18 (3H, s), 2.24-2.34 (1H, m), 2.68-2.74 (1H, m), 2.80-2.88 (1H, m), 3.28-3.53 (3H, m), 3.59-3.76 (1H, m), 3.96-4.12 (1H, m), 4.22-4.31 (1H, m).
MS (ESI) m/z: 226.

Reference Example 37

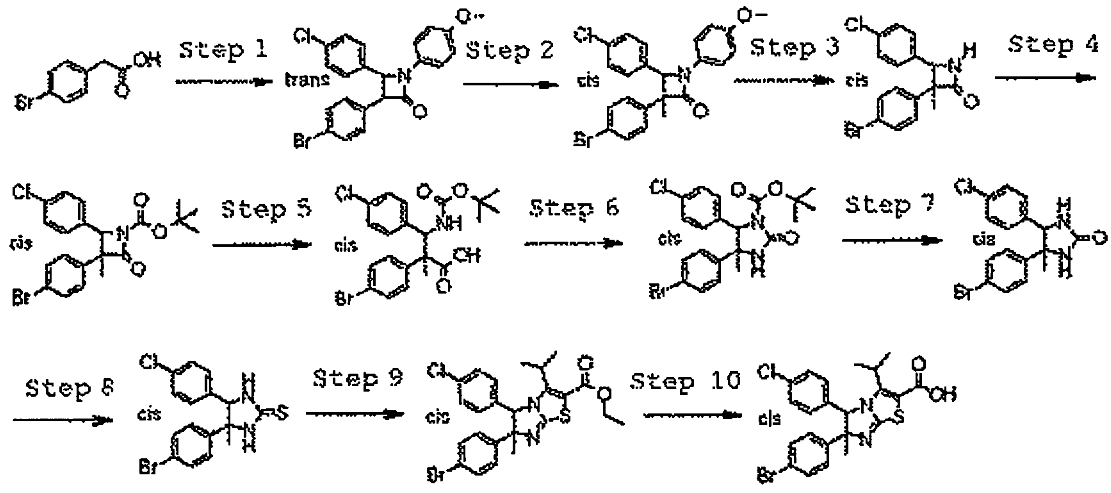


Step 1: (3R*,4S*)-3-(4-Bromophenyl)-4-(4-chlorophenyl)-1-(4-methoxyphenyl)azetidin-2-one

Oxalyl chloride (12.0 ml, 140 mmol) and N,N-dimethylformamide (0.1 ml) were added to a dichloromethane (150 ml) suspension of (4-bromophenyl)acetic acid (27.4 g, 127 mmol) and the resulting mixture was stirred at room temperature for 4 hours. The solvent was removed by concentration under reduced pressure. A toluene solution (100 ml) of the obtained (4-bromophenyl)acetyl chloride was added dropwise to a toluene solution (400 ml) of N-(4-chlorobenzylidene)-4-methoxyaniline (20.0 g, 80.5 mmol) and n-butylamine (29 ml, 127 mmol) at 80°C and the resulting mixture was heated to reflux for 13 hours. After cooling, the reaction mixture was added into 1 N aqueous hydrochloric acid solution (250 ml) and the resulting mixture was stirred, followed by extraction with ethyl acetate. The organic layer was washed with brine and then dried over anhydrous sodium sulfate and the solvent was evaporated under reduced pressure. Diethyl ether was added to the residue and the deposited solid was collected by filtration and dried to give the title compound (21.3 g, 60%) as a colorless solid.
$^1$H-NMR ($CDCl_3$) δ: 3.76 (3H, s), 4.18 (1H, d, J=2.4 Hz), 4.84 (1H, d, J=2.4 Hz), 6.82 (2H, d, J=8.5 Hz), 7.21 (2H, d, J=8.5 Hz), 7.25 (2H, d, J=8.5 Hz), 7.31 (2H, d, J=8.5 Hz), 7.39 (2H, d, J=8.5 Hz), 7.51 (2H, d, J=8.5 Hz).

Step 2: (3S*,4R*)-3-(4-Bromophenyl)-4-(4-chlorophenyl)-1-(4-methoxyphenyl)-3-methylazetidin-2-one

1 M solution of lithium bis(trimethylsilyl)amide in tetrahydrofuran (100 ml, 100 mmol) was added dropwise at -78°C in a nitrogen atmosphere to a tetrahydrofuran (400 ml) solution of the compound (40.0 g, 90.3 mmol) obtained in Step 1 above. The resulting mixture was stirred for 1 hour and then methyl iodide (8.5 ml, 135 mmol) was added dropwise. The resulting mixture was stirred for 30 minutes and then an aqueous solution of saturated ammonium chloride was added. The resulting mixture was stirred at room temperature for 30 minutes, followed by extraction with ethyl acetate. The extract was washed with brine and then dried over anhydrous sodium sulfate. The solvent was evaporated under reduced pressure. The residue was purified by silica gel column chromatography (hexane:ethyl acetate = 4:1) to give the title compound (34.8 g, 84%) as a colorless solid.
$^1$H-NMR ($CDCl_3$) δ: 1.86 (3H, s), 3.74 (3H, s), 4.97 (1H, s), 6.78 (2H, d, J=8.5 Hz), 6.92 (2H, d, J=8.5 Hz), 6.96 (2H, d, J=8.5 Hz), 7.10 (2H, d, J=8.5 Hz), 7.22 (2H, d, J=8.5 Hz), 7.24-7.26 (2H, m).

Step 3: (3S*,4R*)-3-(4-Bromophenyl)-4-(4-chlorophenyl)-3-methylazetidin-2-one

An aqueous solution (100 ml) of ceric ammonium nitrate (116 g, 211 mmol) was added dropwise under ice cooling to a mixture of the compound (32.2 g, 70.4 mmol) obtained in Step 2 above in tetrahydrofuran (400 ml), acetonitrile (900 ml), and water (100 ml). After the completion of reaction, potassium carbonate (30.0 g, 217 mmol) and water (100 ml) were added and the resulting mixture was stirred. Then, the reaction mixture was diluted with ethyl acetate and insoluble matter was removed using celite. The filtrate was washed with water and brine and then dried over anhydrous sodium sulfate. The solvent was evaporated under reduced pressure. Diethyl ether was added to the residue and the deposited solid was collected by filtration and dried to give the title compound (11.1 g, 45%) as a light brown solid. $^1$H-NMR ($CDCl_3$) δ: 1.84 (3H, s), 4.74 (1H, s), 6.07 (1H, brs), 6.92 (2H, d, J=8.5 Hz), 6.97 (2H, d, J=8.5 Hz), 7.15 (2H, d, J=8.5 Hz), 7.24 (2H, d, J=8.5 Hz).

Step 4: tert-Butyl (2R*,3S*)-3-(4-Bromophenyl)-2-(4-chlorophenyl)-3-methyl-4-oxoazetidine-1-carboxylate

Di-tert-butyl dicarbonate (8.80 g, 40.3 mmol), triethylamine (7.10 ml, 50.9 mmol), and dimethylaminopyridine (0.41 g, 3.35 mmol) were added to an acetonitrile (120 ml) suspension of the compound (11.1 g, 31.6 mmol) obtained in Step 3 above and the resulting mixture was stirred for 15 hours. The reaction mixture was concentrated under reduced pressure. Then, the residue was diluted with ethyl acetate, washed with a 10% aqueous solution of citric acid and brine, and dried over anhydrous sodium sulfate. The solvent was evaporated under reduced pressure. The residue was purified by silica gel column chromatography (hexane:ethyl acetate = 4:1) to give the title compound (14.1 g, 99%) as a colorless solid.
$^1$H-NMR ($CDCl_3$) δ: 1.41 (9H, s), 1.84 (3H, s), 4.94 (1H, s), 6.89 (2H, d, J=8.5 Hz), 6.91 (2H, t, J=8.5 Hz), 7.15 (2H, d, J=8.5 Hz), 7.25 (2H, d, J=8.5 Hz).

Step 5: (2S*, 3R*)-2-(4-Bromophenyl)-3-[(tert-butoxycarbonyl)amino]-3-(4-chlorophenyl)-2-methylpropionic acid

1 N aqueous sodium hydroxide solution (62 ml) and water (130 ml) were added to a dioxane (200 ml) solution of the compound (14.1 g, 31.2 mmol) obtained in Step 4 above and the resulting mixture was heated to reflux for 17 hours. After cooling, the mixture was concentrated under reduced pressure. The residue was diluted with water and then made acidic by the addition of 1 N hydrochloric acid, followed by extraction with ethyl acetate. The organic layer was washed with brine and then dried over anhydrous sodium sulfate. The solvent was evaporated under reduced pressure to give the title compound as a light brown solid. This compound was used in next reaction without being purified.

Step 6: tert-Butyl (4S*,5R*)-4-(4-Bromophenyl)-5-(4-chlorophenyl)-4-methyl-2-oxoimidazopyridine-1-carboxylate

Diphenylphosphoryl azide (8.10 ml, 8.05 mmol) was added dropwise to a tert-butanol (150 ml) solution of the compound obtained in Step 5 above and triethylamine (10.9 ml, 78.0 mmol). The resulting mixture was stirred at room temperature for 1 hour and then heated to reflux for 4 hours. After cooling, the mixture was concentrated. The residue was diluted with ethyl acetate, washed with a 10% aqueous solution of citric acid and brine, and then dried over anhydrous sodium sulfate. The solvent was evaporated under reduced pressure. The residue was purified by silica gel column chromatography (hexane:ethyl acetate = 2:1) to give the title compound (8.65 g, 60%) as a pale yellow solid.
$^1$H-NMR ($CDCl_3$) δ: 1.25 (9H, s), 1.88 (3H, s), 4.99 (1H, s), 5.52 (1H, s), 6.83 (2H, d, J=8.5 Hz), 6.92 (2H, d, J=8.5 Hz), 7.06 (2H, d, J=8.5 Hz), 7.23 (2H, d, J=8.5 Hz).

Step 7: (4S*,5R*)-4-(4-Bromophenyl)-5-(4-chlorophenyl)-4-methylimidazolidin-2-one

The compound obtained in Step 6 above was reacted in the same way as in Step 6 of Example 102 to give the title compound as a colorless solid.
$^1$H-NMR ($CDCl_3$) δ: 1.84 (3H, s), 4.67 (1H, brs), 4.78 (1H, brs), 4.82 (1H, brs), 6.85 (2H, d, J=8.5 Hz), 6.86 (2H, d, J=8.5 Hz), 7.09 (2H, d, J=8.8 Hz), 7.25 (2H, d, J=8.5 Hz).

Step 8: (4S*,5R*)-4-(4-Bromophenyl)-5-(4-chlorophenyl)-4-methylimidazopyridine-2-thione

Diphosphorus pentasulfide (677 mg, 3.05 mmol) was added to a dioxane (20 ml) solution of the compound (928 mg, 2.53 mmol) obtained in Step 7 above and the resulting mixture was heated to reflux for 4 hours. After cooling, the mixture was neutralized with saturated aqueous sodium bicarbonate solution, followed by extraction with ethyl acetate. The organic layer was washed with brine and then dried over anhydrous sodium sulfate. The solvent was evaporated under reduced pressure. The residue was purified by silica gel column chromatography (chloroform:methanol = 10:1) to give the title compound (931 mg, 96%) as a colorless solid.
$^1$H-NMR ($CDCl_3$) δ: 1.87 (3H, s), 4.96 (1H, s), 6.11 (1H, brs), 6.33 (1H, brs), 6.78-6.83 (4H, m), 7.10 (2H, d, J=8.9 Hz), 7.25-7.28 (2H, m).

Step 9: Ethyl (5R*,6S*)-6-(4-Bromophenyl)-5-(4-chlorophenyl)-3-isopropyl-6-methyl-5,6-dihydroimidazo[2,1-b][1,3]thiazole-2-carboxylate

The compound obtained in Step 8 above was reacted in the same way as in Step 2 of Example 1 to give the title compound as a pale yellow solid.
$^1$H-NMR ($CDCl_3$) δ: 1. 02 (3H, d, J=7.1 Hz), 1. 04 (3H, d, J=7.1 Hz), 1.37 (3H, t, J=7.2 Hz), 2.10 (3H, s), 3.37 (1H, m), 4.34 (2H, q, J=7.2 Hz), 5.50 (1H, brs), 6.50-6.85 (2H, m), 7.07 (2H, d, J=8.5 Hz), 7.14 (2H, d, J=8.5 Hz), 7.29 (3H, d, J=8.5 Hz).

Step 10: (5R*,6S*)-6-(4-Bromophenyl)-5-(4-chlorophenyl)-3-isopropyl-6-methyl-5,6-dihydroimidazo[2,1-b][1,3]thiazole-2-carboxylic acid

The compound obtained in Step 10 above was reacted in the same way as in Step 3 of Example 1 to give the title compound as a colorless solid.
$^1$H-NMR (DMSO-$d_6$) δ: 0.93 (3H, d, J=7.1 Hz), 0.96 (3H, d, J=7.1 Hz), 2.04 (3H, s), 3.27 (1H, m), 6.29 (1H, s), 6.65-6.68 (2H, m), 7.17 (2H, d, J=8.5 Hz), 7.23 (2H, brs), 7.38 (2H, d, J=8.5 Hz).

Reference Example 38

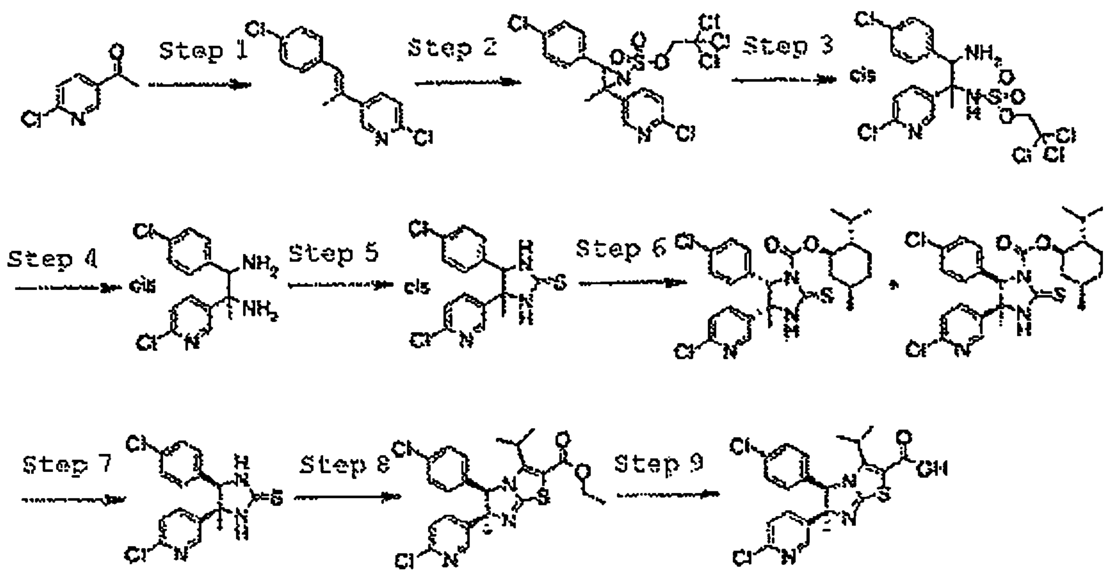


Step 1: 2-Chloro-5-[(E)-2-(4-chlorophenyl)-1-methylvinyl]-pyridine

A diethyl ether (60 ml) solution of 4-chlorobenzyl chloride (75.0 g, 466 mmol) was added dropwise to a mixture of magnesium powder (11.4 g, 470 mmol) in diethyl ether (270 ml) with vigorous stirring. After the completion of dropwise addition, the mixture was stirred at room temperature for 25 minutes and then a tetrahydrofuran (300 ml) solution of 1-(6-chloropyridin-3-yl)-ethanone (66.0 g, 424 mmol) was added dropwise.
The resulting mixture was stirred for 2 hours and then an aqueous solution of saturated ammonium chloride was added, followed by extraction with ethyl acetate. The organic layer was separated and dried over anhydrous magnesium sulfate and then the solvent was evaporated under reduced pressure. The obtained residue was dissolved in benzene (700 ml), p-toluenesulfonic acid monohydrate (89.4 g, 470 mmol) was added, and the resulting mixture was heated to reflux for 3 days using a dehydration tube. A 5 N aqueous solution of sodium hydroxide (100 ml) was added to the reaction mixture under ice cooling, followed by extraction with diethyl ether. The organic layer was washed with brine and dried over anhydrous magnesium sulfate. The solvent was evaporated under reduced pressure. The residue was purified by silica gel column chromatography (hexane:ethyl acetate = 19:1) and then recrystallized from hexane to give the title compound (20.0 g, 17%) as a pale yellow solid.
$^1$H-NMR ($CDCl_3$) δ: 2.24 (3H, d, J=1.2 Hz), 6.78 (1H, s), 7.26-7.37 (5H, m), 7.75 (1H, dd, J=8.4, 2.7 Hz), 8.52 (1H, dd, J=2.7, 0.7 Hz).

Step 2: 2,2,2-Trichloroethyl (2R*,3R*)-3-(4-Chlorophenyl)-2-(6-chloropyridin-3-yl)-2-methylaziridine-1-sulfamate

The compound (2.64 g, 10.0 mmol) obtained in Step 1 above, 2,2,2-trichloroethoxysulfonamide (2.50 g, 11.0 mmol), rhodiumbis(perfluorobutyrylamido) dimer (238 mg, 0.226 mmol), magnesium oxide (0.93 g, 23.0 mmol), and iodobenzene diacetate (4.18 g, 13.0 mmol) were suspended in benzene (20 ml) and the resulting solution was stirred overnight at room temperature in a nitrogen atmosphere. Ethyl acetate was added to the reaction mixture. Insoluble

matter was removed by filtration and the filtrate was washed with saturated aqueous sodium bicarbonate solution and brine. After drying over anhydrous magnesium sulfate, the solvent was evaporated under reduced pressure. The residue was purified by silica gel column chromatography (hexane:ethyl acetate = 9:1) to give the title compound (1.68 g, 34%) as a pale yellow solid.
$^1$H-NMR ($CDCl_3$) δ: 1.44 (3H, s), 4.64 (1H, s), 4.80 (2H, s), 7.38-7.45 (5H, m), 7.94 (1H, dd, J=8.3, 2.7 Hz), 8.62 (1H, d, J=2.7 Hz).

Step 3: 2,2,2-Trichloroethyl [(1S*,2R*)-2-Amino-2-(4-chlorophenyl)-1-(6-chloropyridin-3-yl)-1-methylethyl]sulfamate

The compound (923 mg, 1.88 mmol) obtained in Step 2 above was suspended in a 7 M solution of ammonia in methanol (30 ml) and the resulting solution was stirred at room temperature for 20 hours. The solution was further stirred at 50°C for 1 hour. Then, the solvent was evaporated under reduced pressure to give the title compound (1.06 g, quantitative) as a light brown oil. $^1$H-NMR ($CDCl_3$) δ: 1.72 (3H, s), 3.49 (2H, d, J=1.0 Hz), 3.75 (1H, s), 4.36 (2H, s), 4.64 (1H, s), 6.83 (2H, d, J=8.3 Hz), 7.16 (2H, d, J=8.3 Hz), 7.23 (1H, d, J=8.3 Hz), 7.41 (1H, dd, J=8.3, 2.2 Hz), 8.19 (1H, d, J=2.2 Hz).

Step 4: (1R*,2S*)-1-(4-Chlorophenyl)-2-(6-chloropyridin-3-yl)-propane-1,2-diamine

The compound obtained in Step 3 above was dissolved in a 0.5 N solution of hydrochloric acid in methanol and the resulting solution was stirred at 60°C for 20 hours in a sealed tube. The reaction mixture was concentrated under reduced pressure and 1 N sodium hydroxide was added, followed by extraction with chloroform. The organic layer was dried over anhydrous magnesium sulfate and then the solvent was evaporated under reduced pressure. The obtained residue was purified by silica gel column chromatography (chloroform:methanol = 49:1 → 9:1) to give the title compound (386 mg, 69%) as a colorless oil. $^1$H-NMR ($CDCl_3$) δ: 1.52 (3H, s), 4.07 (1H, s), 6.96 (2H, d, J=8.3 Hz), 7.19 (2H, d, J=8.3 Hz), 7.21 (1H, d, J=8.5 Hz), 7.57 (1H, dd, J=8.5, 2.7 Hz), 8.33 (1H, d, J=2.7 Hz).

Step 5: (4S*,5R*)-5-(4-Chlorophenyl)-4-(6-chloropyridin-3-yl)-4-methyl-imidazopyridine-2-thione

The compound obtained in Step 4 above was reacted in the same way as in Step 1 of Example 1 to give the title compound as a pale yellow solid.
$^1$H-NMR ($CDCl_3$) δ: 1.92 (3H, s), 5.01 (1H, s), 6.42 (1H, s), 6.85 (2H, d, J=8.3 Hz), 7.11 (1H, dd, J=8.1, 0.7 Hz), 7.15 (2H, d, J=8.3 Hz), 7.25-7.27 (2H, m), 7.97 (1H, d, J=2.0 Hz).
MS (ESI) m/z: 519.

Step 6: (1R,2S,5R)-2-Isopropyl-5-methylcyclohexyl (4S,5R)-5-(4-chlorophenyl)-4-(6-chloropyridin-3-yl)-4-methyl-2-thioxoimidazopyridine-1-carboxylate and (1R,2S,5R)-2-isopropyl-5-methyl-cyclohexyl (4R,5S)-5-(4-chlorophenyl)-4-(6-chloropyridin-3-yl)-4-methyl-2-thioxoimidazopyridine-1-carboxylate

Triethylamine (0.735 ml, 5.25 mmol) and 4-dimethylaminopyridine (104 mg, 0.85 mmol) were added to a dichloromethane (40 ml) suspension of the compound (1.48 g, 4.38 mmol) obtained in Step 5 above and then (-)-menthyl chloroformate (1.13 ml, 5.25 mmol) was added dropwise under ice cooling. The resulting mixture was stirred overnight at room temperature and then the reaction mixture was concentrated under reduced pressure. Ethyl acetate was added and the organic layer was washed with 1 N hydrochloric acid, saturated aqueous sodium bicarbonate solution, and brine. After drying over anhydrous magnesium sulfate, the solvent was evaporated under reduced pressure. The residue was purified by silica gel column chromatography (hexane:ethyl acetate = 9:1 → 2:1) to respectively give the title compounds (Isomer A: 1.07 g, 46%; and Isomer B: 1.08 g, 47%) as a colorless solid.
Isomer A: $^1$H-NMR ($CDCl_3$) δ: 0.37 (1H, dd, J=11.8, 5.9 Hz), 0.71 (3H, d, J=6.6 Hz), 0.74-0.78 (2H, m), 0.75 (3H, d, J=6.6 Hz), 0.89 (3H, d, J=10.0 Hz), 0.92-1.02 (1H, m), 1.21-1.36 (3H, m), 1.59 (3H, s), 1.60-1.64 (1H, m), 1.84-1.92 (1H, m), 1.96 (3H, s), 4.57 (1H, td, J=10.9, 4.4 Hz), 5.30 (1H, s), 6.80 (2H, d, J=8.5 Hz), 7.08 (1H, d, J=8.5 Hz), 7.12 (2H, d, J=8.5 Hz), 7.33 (1H, dd, J=8.5, 2.7 Hz), 7.56 (1H, brs), 8.15 (1H, d, J=2.7 Hz).
Isomer B: $^1$H-NMR ($CDCl_3$) δ: 0.39 (3H, d, J=6.6 Hz), 0.48 (3H, d, J=6.6 Hz), 0.51-0.57 (1H, m), 0.71-1.07 (5H, m), 0.89 (3H, d, J=6.8 Hz), 1.37-1.55 (2H, m), 1.60 (3H, s), 1.97 (3H, s), 2.09-2.12 (1H, m), 4.56 (1H, td, J=10.7, 4.4 Hz), 5.31 (1H, s), 6.80 (2H, d, J=8.5 Hz), 7.06 (1H, d, J=8.5 Hz), 7.11 (2H, d, J=8.5 Hz), 7.33 (1H, dd, J=8.5, 2.7 Hz), 7.73 (1H, s), 8.17 (1H, d, J=2.7 Hz).

Step 7: (4S,5R)-5-(4-Chlorophenyl)-4-(6-chloropyridin-3-yl)-4-methyl-imidazopyridine-2-thione

1 N sodium hydroxide (15 ml) was added to a methanol (45 ml) solution of the Isomer A (1.05 g, 2.02 mmol)

obtained in Step 6 above and the resulting mixture was heated to reflux for 48 hours. After cooling, the reaction mixture was concentrated under reduced pressure. Water was added to the obtained residue, followed by extraction with chloroform. The organic layer was dried over anhydrous magnesium sulfate and then the solvent was evaporated under reduced pressure. The residue was purified by silica gel column chromatography (chloroform:methanol = 100:1 → 50:1) to give the title compound (466 mg, 68%) as a colorless solid.
$^1$H-NMR ($CDCl_3$) δ: 1.92 (3H, s), 5.01 (1H, s), 6.62 (1H, s), 6.85 (2H, d, J=8.3 Hz), 7.10 (1H, d, J=8.5 Hz), 7.15 (2H, d, J=8.3 Hz), 7.26 (2H, dd, J=8.5, 2.7 Hz), 7.98 (1H, d, J=2.7 Hz).

Step 8: Ethyl (5R,6S)-5-(4-Chlorophenyl)-6-(6-chloropyridin-3-yl)-3-isopropyl-6-methyl-5,6-dihydroimidazo[2,1-b]thiazole-2-carboxylate

The compound obtained in Step 7 above was reacted in the same way as in Step 2 of Example 1 to give the title compound as a colorless solid.
$^1$H-NMR ($CDCl_3$) δ: 0.88 (3H, d, J=7.1 Hz), 1.01 (3H, t, J=7.1 Hz), 1.83 (3H, s), 1.86 (3H, m), 3.40-3.33 (1H, m), 4.26 (2H, q, J=7.1 Hz), 5.12 (1H, s), 6.81-6.64 (2H, m), 7.00 (1H, d, J=8.3 Hz), 7.09 (2H, d, J=8.8 Hz), 7.50 (1H, dd, J=8.3, 2.4 Hz), 8.20 (1H, d, J=2.4 Hz).

Step 9: (5R,6S)-5-(4-Chlorophenyl)-6-(6-chloropyridin-3-yl)-3-isopropyl-6-methyl-5,6-dihydroimidazo[2,1-b]thiazole-2-carboxylic acid

The compound obtained in Step 8 above was reacted in the same way as in Step 3 of Example 1 to give the title compound as a colorless solid.
$^1$H-NMR ($CDCl_3$) δ: 0.91 (3H, d, J=6.8 Hz), 1.03 (3H, d, J=6.8 Hz), 2.10 (3H, s), 3.51-3.43 (1H, m), 5.65 (1H, s), 6.62-6.51 (2H, m), 7.06 (1H, d, J=8.3 Hz), 7.13 (2H, d, J=8.5 Hz), 7.63 (1H, dd, J=8.3, 2.0 Hz), 8.26 (1H, d, J=2.0 Hz).

Reference Example 39

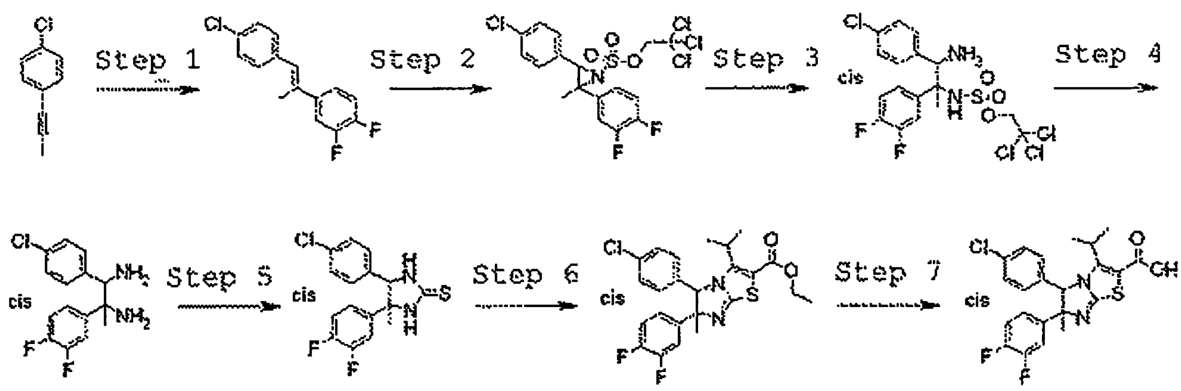


Step 1: 4-[(E)-2-(4-Chlorophenyl)-1-methylvinyl]-1,2-difluorobenzene

(3,4-Difluorophenyl)borane acid (3.97 g, 25.0 mmol), hydroxy(1,5-cyclooctadiene)rhodium(I) dimer (91 mg, 0.20 mmol), and 3,3',3''-phosphanotriylbenzenecarboxylic acid trilithium copper iodide (171 mg, 0.90 mmol) were added to a pyrrolidine (100 ml) solution of 1-chloro-4-prop-1-yn-1-ylbenzene (1.51 g, 10.0 mmol) in an argon atmosphere, propyne was bubbled thereinto at -78°C, and then the resulting mixture was stirred overnight while gradually heated to room temperature. Insoluble matter was removed by filtration through celite and the filtrate was concentrated under reduced pressure. Diethyl ether was added to the residue and the organic layer was washed with saturated ammonium chloride, 1 N hydrochloric acid, and brine. The organic layer was dried over anhydrous magnesium sulfate and then the solvent was evaporated under reduced pressure. The residue was purified by silica gel column chromatography (hexane: ethyl acetate = 9:1) to give the title compound (1.66 g, 62%) as a white solid.
$^1$H-NMR ($CDCl_3$) δ: 2.21 (3H, d, J=1.2 Hz), 6.72 (1H, s), 7.11-7.36 (7H, m).

Step 2: 2,2,2-Trichloroethyl (2R*,3R*)-3-(4-Chlorophenyl)-2-(3,4-difluorophenyl)-2-methylaziridine-1-sulfonate

The compound obtained in Step 1 above was reacted in the same way as in Step 2 of Reference Example 38 to give the title compound as a white solid.
$^1$H-NMR ($CDCl_3$) δ: 1. 42 (3H, s), 4.59 (1H, s), 4.78 (2H, d, J=2.0 Hz), 7.23 (1H, d, J=8.3 Hz), 7.34-7.46 (6H, m).

Step 3: 2,2,2-Trichloroethyl [(1S*,2R*)-2-Amino-2-(4-chlorophenyl)-1-(3,4-difluorophenyl)-1-methylethyl]sulfamate

The compound obtained in Step 2 above was reacted in the same way as in Step 3 of Reference Example 38 to give the title compound as a colorless oil. This compound was used in next reaction without being purified.

Step 4: (1R*,2S*)-1-(4-Chlorophenyl)-2-(3,4-difluorophenyl)propane-1,2-diamine

The compound obtained in Step 3 above was reacted in the same way as in Step 4 of Reference Example 38 to give the title compound as a colorless oil.
$^1$H-NMR ($CDCl_3$) δ: 1.48 (3H, s), 1.61 (4H, brs), 4.06 (1H, brs), 6.98-7.07 (4H, m), 7.16-7.22 (3H, m).

Step 5: (4S*,5R*)-5-(4-Chlorophenyl)-4-(3,4-difluorophenyl)-4-methylimidazopyridine-2-thione

The compound obtained in Step 4 above was reacted in the same way as in Step 1 of Example 1 to give the title compound as a white solid. This compound was used in next reaction without being purified.
MS (ESI) m/z: 339.

Step 6: Ethyl (5R*,6S*)-5-(4-Chlorophenyl)-6-(3,4-difluorophenyl)-3-isopropyl-6-methyl-5,6-dihydroimidazo[2,1-b][1,3] thiazole-2-carboxylate

The compound obtained in Step 5 above was reacted in the same way as in Step 2 of Example 1 to give the title compound as a colorless oil.
$^1$H-NMR ($CDCl_3$) δ: 0.89 (3H, d, J=7.1 Hz), 0.99 (3H, d, J=7.1 Hz), 1.33 (3H, t, J=7.2 Hz), 1.81 (3H, s), 3.29-3.37 (1H, m), 4.25 (2H, q, J=7.1 Hz), 5.06 (1H, s), 6.71 (1H, brs), 6.80-6.90 (2H, m), 7.08-7.02 (3H, m).

Step 7: (5R*,6S*)-5-(4-Chlorophenyl)-6-(3,4-difluorophenyl)-3-isopropyl-6-methyl-5,6-dihydroimidazo[2,1-b][1,3]thiazole-2-carboxylic acid

The compound obtained in Step 6 above was reacted in the same way as in Step 3 of Example 1 to give the title compound as a colorless solid.
$^1$H-NMR ($CDCl_3$) δ: 0.93 (4H, d, J=6.8 Hz), 1.03 (4H, d, J=6.8 Hz), 2.08 (3H, brs), 3.44-3.51 (1H, m), 5.54 (1H, s), 6.86-7.15 (6H, m), 7.36 (1H, s).

Reference Example 40

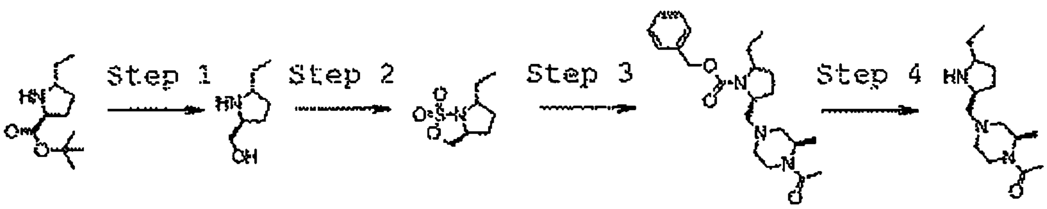


Step 1: [(2S,5R)-5-Ethylpyrrolidin-2-yl]methanol

tert-Butyl (5R)-5-ethyl-L-prolinate was reacted in the same way as in Example 9 to give the title compound as a pale yellow oil. This compound was used in next reaction without being purified.

Step 2: (3aS,6R)-6-Ethyltetrahydro-3H-pyrrolo[1,2-c][1,2,3]oxathiazole 1,1-dioxide

Triethylamine (11.7 ml, 84.0 mmol) was added to a dichloromethane (400 ml) solution of the compound obtained in Step 1 above and the resulting mixture was cooled to -78°C. A dichloromethane (100 ml) solution of sulfuryl chloride (3.37 ml, 42.0 mmol) was added dropwise and the resulting mixture was stirred at room temperature for 16 hours. The mixture was diluted with chloroform and the organic layer was washed with 1 N aqueous hydrochloric acid solution and brine. After drying over anhydrous magnesium sulfate, the solvent was evaporated under reduced pressure. The obtained residue was purified by silica gel column chromatography (hexane:ethyl acetate = 3:2) to give the title compound (2.75 g, 29%) as a pale yellow oil.
$^1$H-NMR ($CDCl_3$) δ: 0.98 (3H, t, J=7. Hz), 1.50-1.57 (1H, m), 1.63-1.82 (3H, m), 2.12-2.26 (2H, m), 3.81-3.87 (1H, m), 4.11 (1H, dd, J=8.7, 5.2 Hz), 4.23-4.29 (1H, m), 4.53 (1H, dd, J=8.8, 6.6 Hz).
MS (ESI) m/z: 192.

Step 3: Benzyl (2S,5R)-2-{[(3R)-4-Acetyl-3-methylpiperazin-1-yl]methyl}-5-ethylpyrrolidine-1-carboxylate

(3aS,6R)-6-Ethyltetrahydro-3H-pyrrolo[1,2-c][1,2,3]oxathiazole 1,1-dioxide (200 mg, 1.04 mmol) and trifluoroacetic acid (1 drop) were added to a chloroform (15 ml) solution of (2R)-1-acetyl-2-methylpiperazine (440 mg, 3.12 mmol) and the resulting mixture was heated to reflux at 65°C for 24 hours. Subsequently, benzyloxycarbonyl chloride (742 μl, 5.2 mmol) was added under ice cooling and triethylamine (870 μl, 6.24 mmol) was added dropwise. The resulting mixture was stirred for 30 minutes and then diluted with chloroform. The organic layer was washed with saturated aqueous sodium bicarbonate solution and brine and dried over anhydrous sodium sulfate. The solvent was evaporated under reduced pressure. The obtained residue was purified by silica gel column chromatography (hexane:ethyl acetate = 5:1) to give the title compound (220 mg, 55%) as a colorless oil.
$^1$H-NMR ($CDCl_3$) δ: 0.83-0.86 (3H, m), 1.17-1.23 (2H, m), 1.25-1.33 (1H, m), 1.66-1.71 (2H, m), 1.88-1.93 (1H, m), 1.94 (3H, s), 2.03 (3H, s), 2.12-2.19 (2H, m), 2.53-2.69 (2H, m), 2.81-2.92 (1H, m), 3.03-3.11 (1H, m), 3.31-3.43 (1H, m), 3.72-3.92 (3H, m), 4.09-4.32 (1H, m), 5.04-5.16 (2H, m), 7.28-7.36 (5H, m).
MS (ESI) m/z: 388.

Step 4: (2R)-1-Acetyl-4-{[(2S,5R)-5-ethylpyrrolidin-2-yl]methyl}-2-methylpiperazine

The compound obtained in Step 3 above was reacted in the same way as in Step 4 of Reference Example 13 to give the title compound as a colorless oil.
$^1$H-NMR (DMSO-$d_6$) δ: 0.95 (3H, t, J=7.4 Hz), 1.20-1.23 (3H, m), 1.56-1.66 (3H, m), 1.77-1.91 (2H, m), 1.97 (3H, s), 2.07-2.17 (3H, m), 2.41 (1H, dd, J=13.1, 5.5 Hz), 2.62 (1H, dd, J=13.1, 8.9 Hz), 2.72 (1H, d, J=11.2 Hz), 2.91-2.95 (1H, m), 3.08-3.18 (3H, m), 3.41-3.49 (1H, m), 3.71-3.78 (1H, m).
MS (ESI) m/z: 254.

Reference Example 41

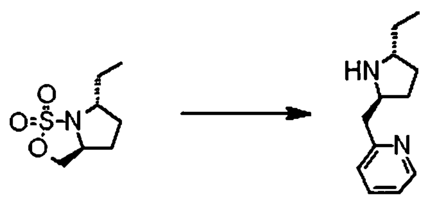

2-{[(2S,5R)-5-Ethylpyrrolidin-2-yl]methyl}pyridine

A 1.6 M solution of n-butyllithium in hexane (1.18 ml, 1.88 mmol) was added dropwise to a tetrahydrofuran (8 ml) solution of 2-bromopyridine (180 μl, 1.88 mmol) at - 78°C in a nitrogen atmosphere and the resulting mixture was stirred for 1 hour. Then, a tetrahydrofuran (1 ml) solution of (3aS,6R)-6-ethyltetrahydro-3H-pyrrolo[1,2-c][1,2,3]oxathiazole 1,1-dioxide (300 mg, 1.57 mmol) was added dropwise. The resulting mixture was stirred at the same temperature for 2 hours and then further at room temperature for 16 hours. The solvent was evaporated under reduced pressure and then the obtained residue was dissolved in ethanol (4 ml). A 2 N aqueous solution of hydrochloric acid (6 ml) was added

and the resulting mixture was heated to reflux at 100°C for 20 hours. A 5 N aqueous solution of sodium hydroxide (8 ml) was added to the reaction mixture under ice cooling, followed by extraction with dichloromethane. The organic layer was washed with brine. After drying over anhydrous sodium sulfate, the solvent was evaporated under reduced pressure. The obtained residue was purified by silica gel column chromatography (chloroform:methanol = 90:1) to give the title compound (247 mg, 83%) as a light brown oil.
$^1$H-NMR ($CDCl_3$) δ: 0.89 (3H, t, J=7.3 Hz), 1.30-1.38 (2H, m), 1.44-1.53 (2H, m), 1.87 (1H, brs), 1.90-2.01 (2H, m), 2.84-2.93 (2H, m), 3.08-3.15 (1H, m), 3.60-3.66 (1H, m), 7.11 (1H, dd, J=7.6, 4.9 Hz), 7.17 (1H, d, J=7.8 Hz), 7.59 (1H, td, J=7.6, 1.8 Hz), 8.53 (1H, d, J=4.9 Hz).
MS (ESI) m/z: 191.

Reference Example 42

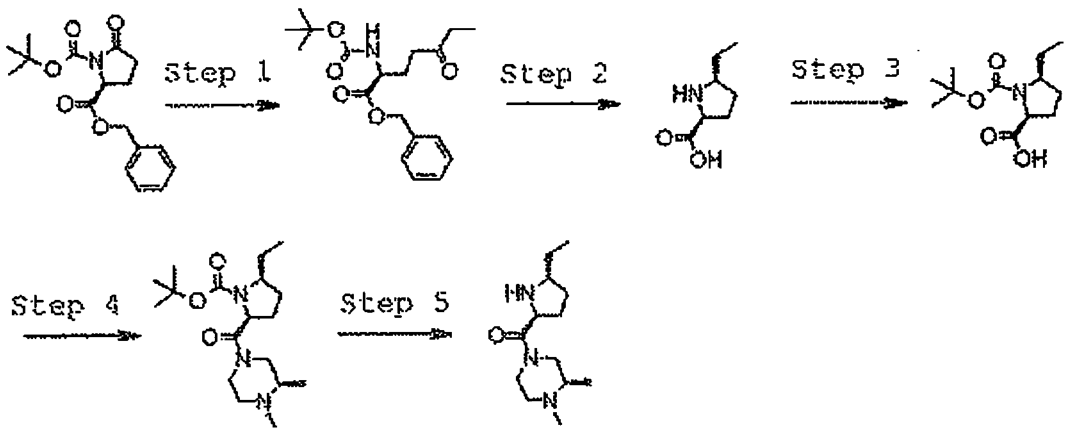


Step 1: Benzyl (2S)-2-[(tert-Butoxycarbonyl)amino]-5-oxoheptanecarboxylate

A 0.5 M solution of ethyllithium in benzene:cyclohexane (9:1) (41.0 ml, 20.0 mmol) was added dropwise to a tetrahydrofuran (80 ml) solution of 2-benzyl 1-tert-butyl(2S)-5-oxopyrrolidine-1,2-dicarboxylate (6.5 g, 20.0 mmol) at -78°C in a nitrogen atmosphere and then the resulting mixture was stirred at room temperature for 2 hours. An aqueous solution of saturated ammonium chloride (100 ml) was added, followed by extraction with ethyl acetate. The organic layer was washed with brine and dried over anhydrous magnesium sulfate and the solvent was evaporated under reduced pressure. The obtained residue was purified by silica gel column chromatography (hexane:ethyl acetate = 3:1) to give the title compound (3.45 g, 49%) as a colorless oil.
$^1$H-NMR ($CDCl_3$) δ: 1.02 (3H, t, J=7.3 Hz), 1.43 (9H, s), 1.88-1.96 (1H, m), 2.09-2.15 (1H, m), 2.37 (2H, q, J=7.3 Hz), 2.38-2.52 (2H, m), 4.32 (1H, brs), 5.11 (1H, brs), 5.13 (1H, d, J=12.2 Hz), 5.19 (1H, d, J=12.2 Hz), 7.34-7.38 (5H, m).
MS (ESI) m/z: 372.

Step 2: (5S)-5-Ethyl-L-proline

The compound obtained in Step 1 above was reacted in the same way as in Step 6 of Example 102 to give the title compound as a colorless oil. This compound was used in next reaction without being purified.

Step 3: (5S)-1-(tert-Butoxycarbonyl)-5-ethyl-L-proline

The compound obtained in Step 2 above was reacted in the same way as in Step 4 of Reference Example 9 to give the title compound as a light brown solid.
$^1$H-NMR ($CDCl_3$) δ: 0.87 (3H, t, J=7.1 Hz), 1.39-1.45 (1H, m), 1.48 (9H, s), 1.71-1.77 (2H, m), 1.93-2.00 (1H, m), 2.09-2.11 (1H, brm), 2.33-2.35 (1H, m), 3.79-3.82 (1H, m), 4.32-4.34 (1H, m).
MS (ESI) m/z: 266.

Step 4: tert-Butyl (2S,5S)-2-{[(3R)-3,4-Dimethylpiperazin-1-yl]carbonyl}-5-ethylpyrrolidine-1-carboxylate

The compound obtained in Step 3 above used instead of (5R,6S)-5,6-bis(4-chlorophenyl)-3-isopropyl-6-methyl-5,6-dihydroimidazo[2,1-b][1,3]thiazole-2-carboxylic acid and (2R)-1,2-dimethylpiperazine used instead of piperazin-2-one were reacted in the same way as in Step 4 of Example 1 to give the title compound.
$^1$H-NMR ($CDCl_3$) δ: 0.92 (3H, t, J=7.4 Hz), 1.05-1.11 (3H, m), 1.46 (9H, s), 1.50-1.53 (1H, m), 1.71-1.77 (2H, m), 1.89-1.99 (3H, m), 2.07-2.13 (2H, m), 2.29 (3H, s), 2.49-2.55 and 3.23-3.30 (total 1H, each m), 2.76-2.81 (1H, m), 2.87-2.94 (1H, m), 3.67-3.89 (2H, m), 4.31 and 4.44 (total 1H, each d, J=13.2 Hz), 4.54-4.71 (1H, m).
MS (ESI) m/z: 340.

Step 5: (2R)-4-[(5S)-5-Ethyl-L-prolyl]-1,2-dimethylpiperazine

The compound obtained in Step 4 above was reacted in the same way as in Step 2 of Example 214 to give the title compound as a colorless solid.
$^1$H-NMR (DMSO-$d_6$) δ: 0.96 (3H, t, J=7.4 Hz), 1.35-1.37 (3H, m), 1.55-1.71 (2H, m), 1.82-1.90 (1H, m), 1.93-2.01 (1H, m), 2.09-2.16 (1H, m), 2.32-2.40 (1H, m), 2.75 (3H, s), 3.06-3.22 (3H, m), 3.36-3.48 (2H, m), 3.99-4.15 (1H, m), 4.30-4.43 (1H, m), 4.60-4.72 (2H, m), 7.93 (1H, brs).
MS (ESI) m/z: 240.

Reference Example 43

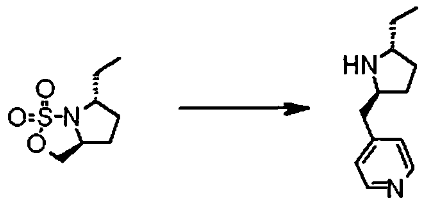

4-{[(2S,5R)-5-Ethylpyrrolidin-2-yl]methyl}pyridine

(3aS,6R)-6-Ethyltetrahydro-3H-pyrrolo[1,2-c][1,2,3]oxathiazole 1,1-dioxide was reacted in the same way as in Reference Example 41 to give the title compound as a pale orange oil.
$^1$H-NMR ($CDCl_3$) δ: 0.89 (3H, t, J=7.3 Hz), 1.35-1.48 (3H, m), 1.66-1.72 (1H, m), 1.90-1.99 (2H, m), 2.61-2.69 (1H, m), 2.73-2.79 (1H, m), 3.07-3.14 (1H, m), 3.40-3.48 (1H, m), 7.12-7.14 (2H, m), 8.50-8.51 (2H, m).
MS (ESI) m/z: 191.

Reference Example 44

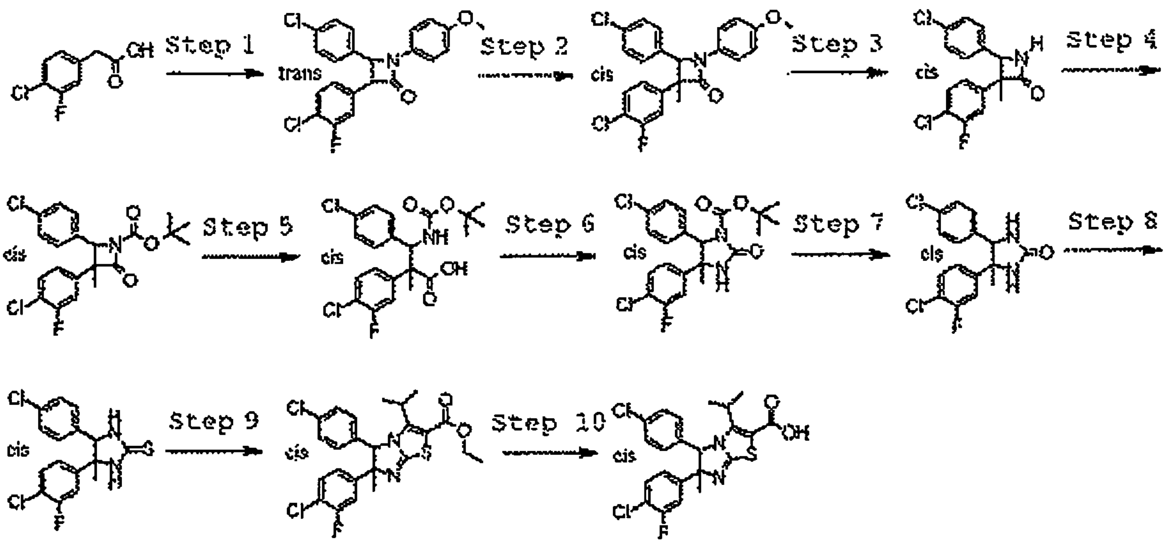


Step 1: (3R*,4S*)-3-(4-Chloro-3-fluorophenyl)-4-(4-chlorophenyl)-1-(4-methoxyphenyl)azetidin-2-one

4-Chloro-3-fluorophenylacetic acid was reacted in the same way as in Step 1 of Reference Example 37 to give the title compound as a red-orange oil.
$^1$H-NMR ($CDCl_3$) δ: 3.76 (3H, s), 4.19 (1H, d, J=2.4 Hz), 4.84 (1H, d, J=2.4 Hz), 6.82 (2H, d, J=9.0 Hz), 7.07 (1H, d, J=8.3 Hz), 7.14 (1H, dd, J=9.5, 2.0 Hz), 7.21-7.28 (2H, m), 7.32 (2H, d, J=8.5 Hz), 7.36-7.44 (3H, m).

Step 2: (3S*,4R*)-3-(4-Chloro-3-fluorophenyl)-4-(4-chlorophenyl)-1-(4-methoxyphenyl)-3-methylazetidin-2-one

The compound obtained in Step 1 above was reacted in the same way as in Step 2 of Reference Example 37 to give the title compound as a pale yellow solid.
$^1$H-NMR ($CDCl_3$) δ: 1.87 (3H, s), 3.75 (3H, s), 4.98 (1H, s), 6.80 (3H, d, J=9.0 Hz), 7.09-7.16 (3H, m), 7.10-7.15 (3H, m), 7.25 (2H, d, J=9.0 Hz).

Step 3: (3S*,4R*)-3-(4-Chloro-3-fluorophenyl)-4-(4-chlorophenyl)-3-methylazetidin-2-one

The compound obtained in Step 2 above was reacted in the same way as in Step 3 of Reference Example 37 to give the title compound as a red-orange oil.

Step 4: tert-Butyl (2R*,3S*)-3-(4-Chloro-3-fluorophenyl)-2-(4-chlorophenyl)-3-methyl-4-oxoazetidine-1-carboxylate

The compound obtained in Step 3 above was reacted in the same way as in Step 4 of Reference Example 37 to give the title compound as a red-orange solid.
$^1$H-NMR ($CDCl_3$) δ: 1.40 (9H, s), 1.83 (3H, s), 4.92 (1H, s), 6.71 (1H, dd, J=8.3, 2.1 Hz), 6.86 (1H, dd, J=10.1, 2.1 Hz), 6.92 (2H, d, J=8.5 Hz), 7.09-7.19 (3H, m).

Step 5: (2S*,3R*)-3-[(tert-Butoxycarbonyl)amino]-2-(4-chloro-3-fluorophenyl)-3-(4-chlorophenyl)-2-methylpropionic acid

The compound obtained in Step 4 above was reacted in the same way as in Step 5 of Reference Example 37 to give the title compound as a pale orange solid.
MS (ESI) m/z: 464 $(M+23)^+$.

Step 6: tert-Butyl (4S*,5R*)-4-(4-Chloro-3-fluorophenyl)-5-(4-chlorophenyl)-4-methyl-2-oxoimidazopyridine-1-carboxylate

The compound obtained in Step 5 above was reacted in the same way as in Step 6 of Reference Example 37 to give the title compound as a colorless solid.

$^1$H-NMR ($CDCl_3$) δ: 1.23 (9H, s), 1.89 (3H, s), 4.99 (1H, s), 6.48 (1H, brs), 6.80 (1H, dd, J=8.5, 1.7 Hz), 6.83-6.91 (3H, m), 7.08 (2H, d, J=8.5 Hz), 7.13 (1H, t, J=8.0 Hz).
MS (ESI) m/z: 461 $(M+23)^+$.

Step 7: (4S*,5R*)-4-(4-Chloro-3-fluorophenyl)-5-(4-chlorophenyl)-4-methylimidazolidin-2-one

The compound obtained in Step 6 above was reacted in the same way as in Step 6 of Example 102 to give the title compound as a colorless solid.
$^1$H-NMR ($CDCl_3$) δ: 1.84 (3H, s), 4.75 (1H, brs), 4.78 (1H, s), 4.91 (1H, brs), 6.71 (1H, dd, J=8.5, 1.7 Hz), 6.81 (1H, dd, J=10.7, 1.7 Hz), 6.88 (2H, d, J=8.5 Hz), 7.11 (2H, d, J=8.5 Hz), 7.14 (1H, t, J=8.0 Hz).

Step 8: (4S*,5R*)-4-(4-Chloro-3-fluorophenyl)-5-(4-chlorophenyl)-4-methylimidazopyridine-2-thione

The compound obtained in Step 7 above was reacted in the same way as in Step 8 of Reference Example 37 to give the title compound as a colorless solid.
$^1$H-NMR ($CDCl_3$) δ: 1.88 (3H, s), 4.96 (1H, s), 6.24 (1H, brs), 6.53 (1H, brs), 6.68 (1H, dd, J=8.3, 1.5 Hz), 6.75 (1H, dd, J=10.2, 1.5 Hz), 6.85 (2H, d, J=8.5 Hz), 7.10-7.19 (3H, m).

Step 9: Ethyl (5R*,6S*)-6-(4-Chloro-3-fluorophenyl)-5-(4-chlorophenyl)-3-isopropyl-6-methyl-5,6-dihydroimidazo[2,1-b][1,3]thiazole-2-carboxylate

The compound obtained in Step 8 above was reacted in the same way as in Step 2 of Example 1 to give the title compound as a colorless solid.
$^1$H-NMR ($CDCl_3$) δ: 0.88 (3H, d, J=7.2 Hz), 0.99 (3H, d, J=7.2 Hz), 1.33 (3H, t, J=7.1 Hz), 1.79 (3H, s), 3.26-3.39 (1H, m), 4.25 (2H, q, J=7.2 Hz), 5.06 (1H, s), 6.62-6.82 (2H, m), 6.88 (2H, dd, J=8.4, 2.1 Hz), 7.02-7.11 (4H, m).

Step 10: (5R*,6S*)-6-(4-Chloro-3-fluorophenyl)-5-(4-chlorophenyl)-3-isopropyl-6-methyl-5,6-dihydroimidazo[2,1-b][1,3]thiazole-2-carboxylic acid

The compound obtained in Step 9 above was reacted in the same way as in Step 3 of Example 1 to give the title compound as a colorless solid.
$^1$H-NMR ($CDCl_3$) δ : 0.89 (3H, d, J=7.1 Hz), 1.02 (3H, d, J=7.1 Hz), 1.96 (3H, s), 3.37-3.50 (1H, m), 5.29 (1H, s), 6.62-6.84 (1H, m), 6.93 (1H, dd, J=8.5, 1.8 Hz), 7.06 (1H, dd, J=10.2, 1.8 Hz), 7.07-7.14 (4H, m).

Reference Example 45

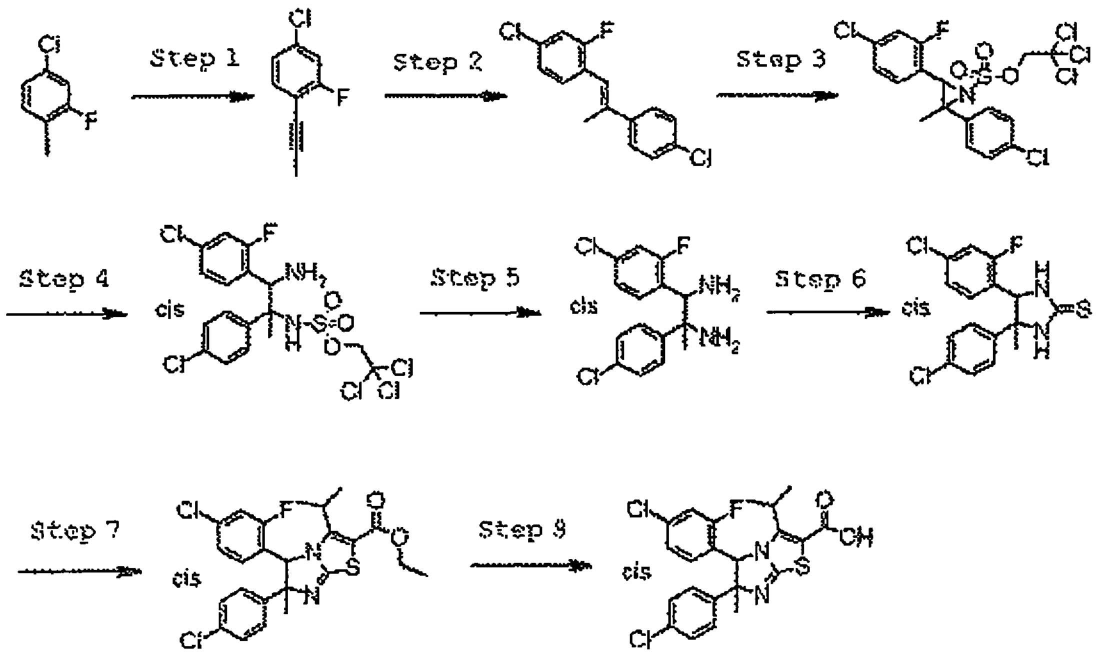


Step 1: 4-Chloro-2-fluoro-1-propyn-1-ylbenzene

A mixture of 4-chloro-2-fluoro-1-iodobenzene (5.00 g, 19.5 mmol), dichlorobis(triphenylphosphine)palladium (II) (421 mg, 0.60 mmol), triphenylphosphine (315 mg, 1.2 mmol), and copper iodide (190 mg, 1.00 mmol) in diisopropylethylamine (50 ml) was cooled to -78°C, propyne was bubbled thereinto, and then the resulting mixture was stirred at room temperature for 30 minutes and further overnight at 85°C. The mixture was left standing to cool. Then, insoluble matter was removed by filtration through celite and the filtrate was concentrated under reduced pressure. Diethyl ether was added to the obtained residue and the organic layer was washed with 1 N aqueous hydrochloric acid solution and brine. The organic layer was dried over anhydrous magnesium sulfate and then the solvent was evaporated under reduced pressure. The residue was purified by silica gel column chromatography (hexane) to give the title compound (3.21 g, 98%) as a pale yellow solid.
$^1$H-NMR ($CDCl_3$) δ: 2.09 (3H, s), 7.04-7.09 (2H, m), 7.30 (1H, t, J=8.1 Hz).

Step 2: 4-Chloro-1-[(1E)-2-(4-chlorophenyl)propen-1-yl]-2-fluorobenzene

The compound obtained in Step 1 above was reacted in the same way as in Step 1 of Reference Example 39 to give the title compound as a colorless solid.
$^1$H-NMR ($CDCl_3$) δ: 2.16 (3H, t, J=1.2 Hz), 6.70 (1H, s), 7.10-7.16 (2H, m), 7.27 (1H, t, J=7.6 Hz), 7.33 (2H, d, J=8.5 Hz), 7.45 (2H, d, J=8.5 Hz).

Step 3: 2,2,2-Trichloroethyl (2R*,3R*)-3-(4-Chloro-2-fluorophenyl)-2-(4-chlorophenyl)-2-methylaziridine-1-sulfamate

The compound obtained in Step 2 above was reacted in the same way as in Step 2 of Reference Example 14 to give the title compound as a colorless oil.
$^1$H-NMR ($CDCl_3$) δ: 1.41 (3H, s), 4.65 (1H, d, J=10.7 Hz), 4.65 (1H, s), 4.75 (1H, d, J=10.7 Hz), 7.20-7.24 (2H, m), 7.38-7.41 (1H, m), 7.44 (2H, d, J=8.5 Hz), 7.58 (2H, d, J=8.5 Hz).

Step 4: 2,2,2-Trichloroethyl [(1S*,2R*)-2-Amino-2-(4-chloro-2-fluorophenyl)-1-(4-chlorophenyl)-1-methylethyl]sulfamate

The compound obtained in Step 3 above was reacted in the same way as in Step 3 of Reference Example 14 to give the title compound.
This compound was used in next reaction without being purified.

Step 5: (1R*,2S*)-1-(4-Chloro-2-fluorophenyl)-2-(4-chlorophenyl)propane-1,2-diamine

The compound obtained in Step 4 above was reacted in the same way as in Step 4 of Reference Example 14 to give the title compound.
This compound was used in next reaction without being purified.

Step 6: (4S*,5R*)-5-(4-Chloro-2-fluorophenyl)-4-(4-chlorophenyl)-4-methylimidazopyridine-2-thione

The compound obtained in Step 5 above was reacted in the same way as in Step 1 of Example 1 to give the title compound as a white solid.
$^1$H-NMR ($CDCl_3$) δ: 1.95 (3H, s), 5.31 (1H, s), 6.49 (1H, s), 6.76 (1H, s), 6.85-6.90 (3H, m), 7.01 (2H, d, J=8.8 Hz), 7.11 (2H, d, J=8.8 Hz).

Step 7: Ethyl (5R*,6S*)-5-(4-Chloro-2-fluorophenyl)-6-(4-chlorophenyl)-3-isopropyl-6-methyl-5,6-dihydroimidazo[2,1-b][1,3]thiazole-2-carboxylate

The compound obtained in Step 6 above was reacted in the same way as in Step 2 of Example 1 to give the title compound as a colorless oil.
$^1$H-NMR ($CDCl_3$) δ: 0.87 (3H, d, J=7.3 Hz), 1.06 (3H, d, J=7.3 Hz), 1.33 (3H, t, J=7.1 Hz), 1.81 (3H, s), 3.39-3.46 (1H, m), 4.25 (2H, q, J=7.1 Hz), 5.50 (1H, s), 6.60 (1H, t, J=8.0 Hz), 6.88-6.81 (2H, m), 7.05 (2H, d, J=8.3 Hz), 7.20 (2H, d, J=8.3 Hz).

Step 8: (5R*,6S*)-5-(4-Chloro-2-fluorophenyl)-6-(4-chlorophenyl)-3-isopropyl-6-methyl-5,6-dihydroimidazo[2,1-b][1,3]thiazole-2-carboxylic acid

The compound obtained in Step 7 above was reacted in the same way as in Step 3 of Example 1 to give the title compound as a colorless solid.
$^1$H-NMR ($CDCl_3$) δ: 0.85 (3H, d, J=7.1 Hz), 1.10 (3H, d, J=7.1 Hz), 2.02 (3H, s), 3.62-3.69 (1H, m), 5.70 (1H, s), 6.54 (1H, t, J=8.0 Hz), 6.85 (1H, dd, J=9.5, 2.0 Hz), 6.93 (1H, dd, J=8.0, 2.0 Hz), 7.09 (2H, d, J=8.5 Hz), 7.23 (2H, d, J=8.5 Hz), 8.64 (1H, s).

Reference Example 46

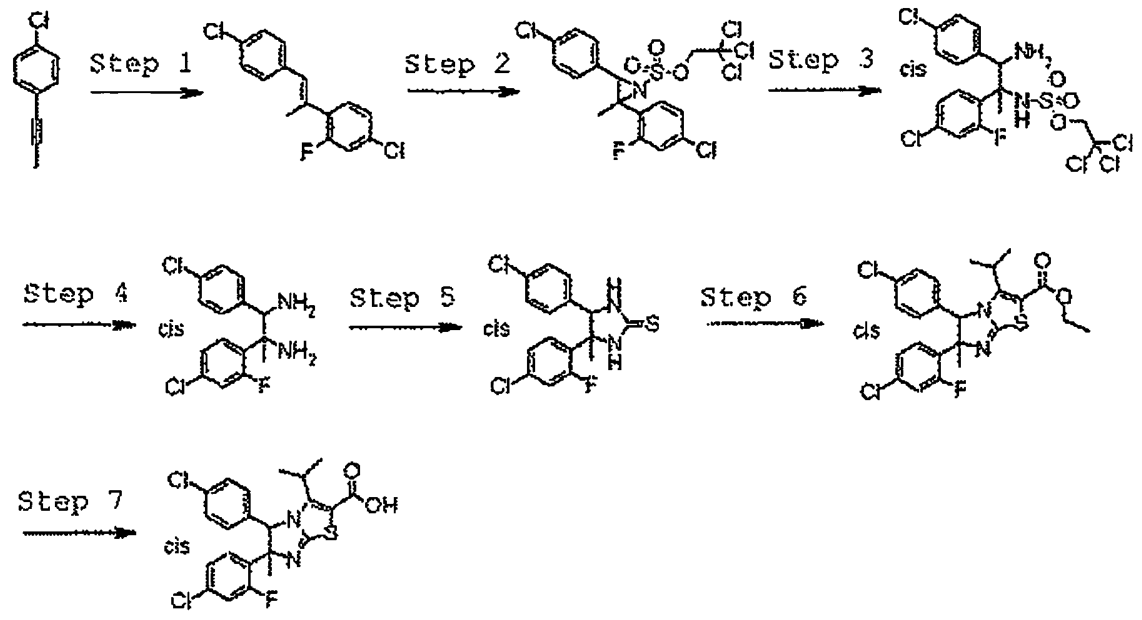

Step 1: 4-Chloro-1-[(E)-2-(4-chlorophenyl)-1-methylvinyl]-2-fluorobenzene

(4-Chloro-2-fluorophenyl)borane acid used instead of (3,4-difluorophenyl)borane acid was reacted in the same way as in Step 1 of Reference Example 39 to give the title compound as a colorless oil.
$^1$H-NMR ($CDCl_3$) δ: 2.03 (1H, s), 2.21 (3H, t, J=1.6 Hz), 6.57 (1H, s), 7.09-7.14 (2H, m), 7.23-7.35 (7H, m).

Step 2: 2,2,2-Trichloroethyl (2R*,3R*)-2-(4-Chloro-2-fluorophenyl)-3-(4-chlorophenyl)-2-methylaziridine-1-sulfamate

The compound obtained in Step 1 above was reacted in the same way as in Step 2 of Reference Example 14 to give the title compound as a white solid.
$^1$H-NMR ($CDCl_3$) δ: 1.38 (3H, s), 4.53 (1H, d, J=2.2 Hz), 4.80 (2H, s), 7.18-7.26 (2H, m), 7.42-7.41 (4H, m), 7.64 (1H, t, J=8.0 Hz).

Step 3: 2,2,2-Trichloroethyl (1S*,2R*)-2-Amino-1-(4-chloro-2-fluorophenyl)-2-(4-chlorophenyl)-1-methylethyl]sulfamate

The compound obtained in Step 2 above was reacted in the same way as in Step 3 of Reference Example 14 to give the title compound.
This compound was used in next reaction without being purified.

Step 4: (1R*,2S*)-2-(4-Chloro-2-fluorophenyl)-1-(4-chlorophenyl)propane-1,2-diamine

The compound obtained in Step 3 above was reacted in the same way as in Step 4 of Reference Example 14 to give the title compound.
This compound was used in next reaction without being purified.

Step 5: (4S*,5R*)-4-(4-Chloro-2-fluorophenyl)-5-(4-chlorophenyl)-4-methylimidazopyridine-2-thione

The compound obtained in Step 4 above was reacted in the same way as in Step 1 of Example 1 to give the title compound as a white solid.
$^1$H-NMR ($CDCl_3$) δ: 1.89 (3H, d, J=1.5 Hz), 4.96 (1H, d, J=2.7 Hz), 6.62 (1H, brs), 6.79 (1H, dd, J=11.2, 2.0 Hz), 6.98-7.10 (6H, m), 7.21-7.26 (1H, m).

Step 6: Ethyl (5R*,6S*)-6-(4-Chloro-2-fluorophenyl)-5-(4-chlorophenyl)-3-isopropyl-6-methyl-5,6-dihydroimidazo[2,1-b][1,3]thiazole-2-carboxylate

The compound obtained in Step 5 above was reacted in the same way as in Step 2 of Example 1 to give the title compound as a colorless oil.
$^1$H-NMR ($CDCl_3$) δ: 0.89 (3H, d, J=7.1 Hz), 0.96 (3H, d, J=7.1 Hz), 1.32 (3H, t, J=7.1 Hz), 1.75 (3H, s), 3.31-3.38 (1H, m), 4.24 (2H, q, J=7. 1 Hz), 5.24 (1H, d, J=3.4 Hz), 6.74 (1H, dd, J=11.0, 2.0 Hz), 6.93 (1H, dd, J=8.4, 2.0 Hz), 7.04-7.08 (4H, m), 7.63 (1H, t, J=8.5 Hz).
MS (ESI) m/z: 493.

Step 7: (5R*,6S*)-6-(4-Chloro-2-fluorophenyl)-5-(4-chlorophenyl)-3-isopropyl-6-methyl-5,6-dihydroimidazo[2,1-b][1,3]thiazole-2-carboxylic acid

The compound obtained in Step 6 above was reacted in the same way as in Step 3 of Example 1 to give the title compound as a colorless solid.
$^1$H-NMR ($CDCl_3$) δ: 0.93 (3H, d, J=7.1 Hz), 1.01 (3H, d, J=7.1 Hz), 1.96 (3H, s), 3.50-3.57 (1H, m), 5.51 (1H, d, J=3.4 Hz), 6.79 (1H, dd, J=11.0, 2.0 Hz), 6.97 (1H, dd, J=8.3, 2.0 Hz), 7.08-7.12 (4H, m), 7.60 (1H, t, J=8.5 Hz), 8.62 (1H, brs).

Reference Example 47

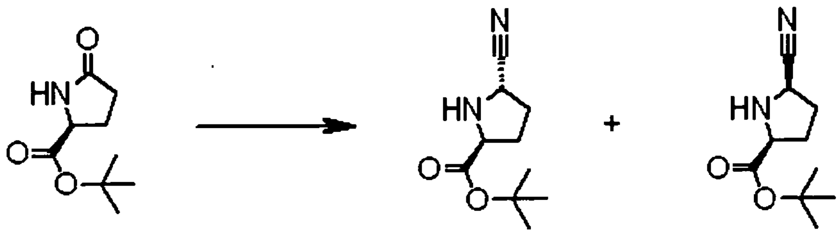

tert-Butyl (5S)-5-Cyano-L-prolinate and tert-butyl (5R)-5-Cyano-L-prolinate

A tetrahydrofuran (40 ml) suspension of bis(cyclopentadienyl)zirconium chloride hydride (9.05 g, 0.035 mol) was added dropwise to a tetrahydrofuran (60 ml) solution of tert-butyl 5-oxo-L-prolinate (5.0 g, 0.027 mol) at -20°C in a nitrogen atmosphere. The resulting mixture was stirred at room temperature for 3 hours and then trimethylsilyl cyanide (3.96 ml, 0.030 mol) was added dropwise. The resulting mixture was stirred for 1 hour, then potassium carbonate (5.0 g) was added, and the resulting mixture was stirred. Insoluble matter was removed by filtration and the filtrate was concentrated under reduced pressure. The obtained residue was purified by silica gel column chromatography (hexane: ethyl acetate = 2:1 → 2:3) to respectively give the title compounds (Isomer A: 3.06 g, 58%; and Isomer B: 275 mg, 5%) as a colorless solid.

tert-Butyl (5S)-5-cyano-L-prolinate (Isomer A):

$^1$H-NMR ($CDCl_3$) δ: 1.46 (9H, s), 1.93-2.00 (1H, m), 2.11-2.16 (2H, m), 2.28-2.36 (1H, m), 2.65 (1H, brs), 3.82-3.86 (1H, m), 4.19 (1H, q, J=6.2 Hz).

MS (ESI) m/z: 196.

tert-Butyl (5R)-5-cyano-L-prolinate (Isomer B):

$^1$H-NMR ($CDCl_3$) δ: 1.50 (9H, s), 2.07-2.18 (3H, m), 2.22-2.28 (1H, m), 2.65 (1H, s), 3.75-3.79 (1H, m), 4.00-4.05 (1H, m).

MS (ESI) m/z: 196.

Reference Example 48

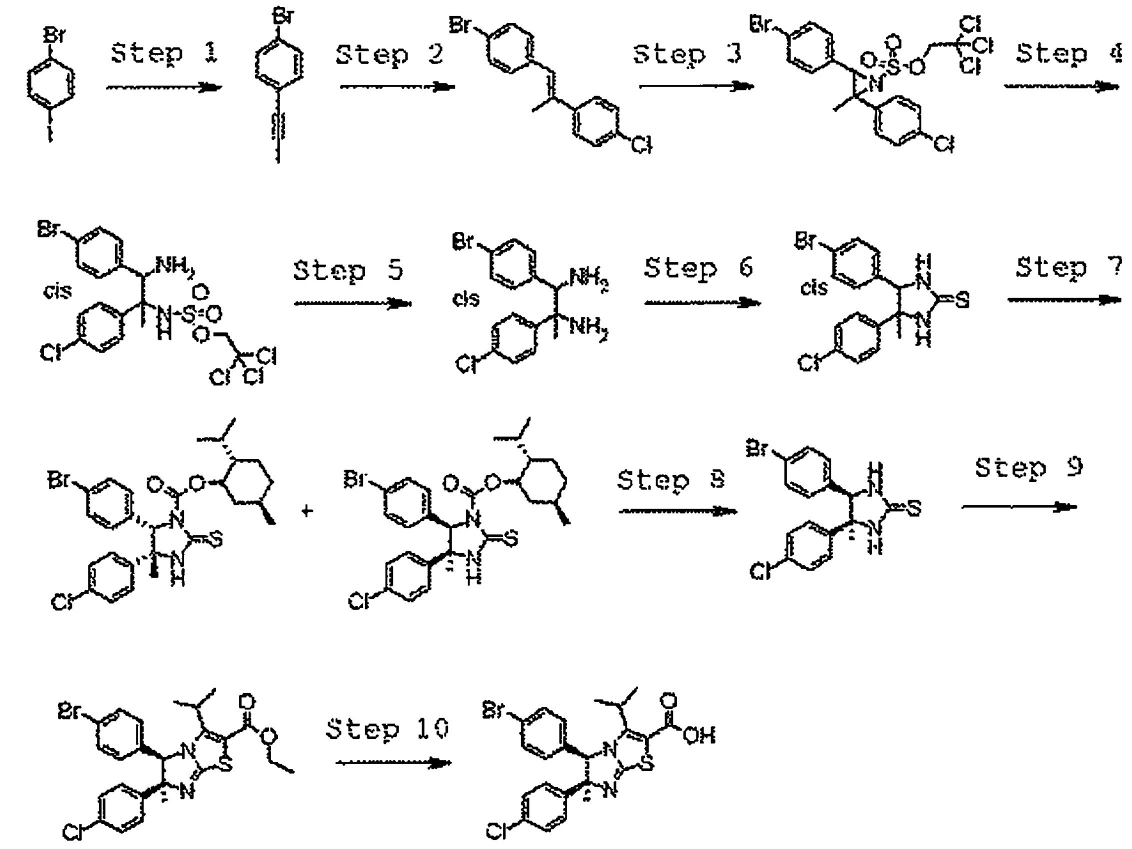


Step 1: 1-Bromo-4-propyn-1-ylbenzene

1-Bromo-4-iodobenzene used instead of 4-chloro-2-fluoro-1-iodobenzene was reacted in the same way as in Step 1 of Reference Example 45 to give the title compound as a pale yellow oil.
$^1$H-NMR ($CDCl_3$) $\delta$: 2.03 (3H, s), 7.24 (2H, d, J=8.5 Hz), 7.40 (2H, d, J=8.5 Hz).

Step 2: 1-Bromo-4-[(1E)-2-(4-chlorophenyl)propen-1-yl]benzene

The compound obtained in Step 1 above was reacted in the same way as in Step 1 of Reference Example 39 to give the title compound as a colorless solid.
$^1$H-NMR ($CDCl_3$) $\delta$: 2.22 (3H, d, J=1.2 Hz), 6.72 (1H, brs), 7.21 (2H, d, J=8.3 Hz), 7.33 (2H, d, J=8.5 Hz), 7.43 (2H, d, J=8.5 Hz), 7.50 (2H, d, J=8.3 Hz).

Step 3: 2,2,2-Trichloroethyl (2R*,3R*)-3-(4-Bromophenyl)-2-(4-chlorophenyl)-2-methylaziridine-1-sulfonate

The compound obtained in Step 2 above was reacted in the same way as in Step 2 of Reference Example 14 to give the title compound as a colorless solid.
$^1$H-NMR ($CDCl_3$ ) $\delta$: 1.41 (3H, s), 4.59 (1H, s), 4.69 (1H, d, J=11.0 Hz), 4.76 (1H, d, J=11.0 Hz), 7.33 (2H, d, J=8.5 Hz), 7.41 (2H, d, J=8.5 Hz), 7.54 (2H, d, J=8.5 Hz), 7.57 (2H, d, J=8.5 Hz).

Step 4: 2,2,2-Trichloroethyl {(1S*,2R*)-2-Amino-1-(4-bromophenyl)-1-methyl-2-(4-chlorophenyl)ethyl}sulfamate

The compound obtained in Step 3 above was reacted in the same way as in Step 3 of Reference Example 14 to give the title compound. This compound was used in next reaction without being purified.

Step 5: (1R*,2S*)-1-(4-Bromophenyl)-2-(4-chlorophenyl)propane-1,2-diamine

The compound obtained in Step 4 above was reacted in the same way as in Step 4 of Reference Example 14

to give the title compound. This compound was used in next reaction without being purified.

Step 6: (4S*,5R*)-5-(4-Bromophenyl)-4-(4-chlorophenyl)-4-methylimidazopyridine-2-thione

The compound obtained in Step 5 above was reacted in the same way as in Step 1 of Example 1 to give the title compound as a colorless solid.
$^1$H-NMR ($CDCl_3$) δ: 1.86 (3H, t, J=7.4 Hz), 4.94 (1H, s), 6.29 (1H, s), 6.57 (1H, s), 6.75 (2H, d, J=8.5 Hz), 6.86 (2H, d, J=8.8 Hz), 7.13 (2H, d, J=8.5 Hz), 7.25 (3H, d, J=8.8 Hz).

Step 7: (1R,2S,5R)-2-Isopropyl-5-methylcyclohexyl (4S,5R)-5-(4-Bromophenyl)-4-(4-chlorophenyl)-4-methyl-2-thioxoimidazopyridine-1-carboxylate

The compound obtained in Step 6 above was reacted in the same way as in Step 6 of Reference Example 38 to give the title compound as a colorless solid.
$^1$H-NMR ($CDCl_3$) δ: 0.33 (1H, dd, J=11.7, 11.7 Hz), 0.68-0.98 (2H, m), 0.72 (3H, d, J=6.8 Hz), 0.75 (3H, d, J=6.8 Hz), 0.88 (3H, d, J=7.1 Hz), 1.19-1.32 (3H, m), 1.56-1.89 (3H, m), 1.91 (3H, s), 4.52-4.59 (1H, m), 5.23 (1H, s), 6.72 (2H, d, J=8.5 Hz), 6.98 (2H, d, J=8.5 Hz), 7.11 (2H, d, J=8.5 Hz), 7.21 (2H, d, J=8.5 Hz), 7.32 (1H, s).

Step 8: (4S,5R)-5-(4-Bromophenyl)-4-(4-chlorophenyl)-4-methylimidazopyridine-2-thione

The compound obtained in Step 7 above was reacted in the same way as in Step 7 of Reference Example 38 to give the title compound as a colorless solid.
$^1$H-NMR ($CDCl_3$) δ: 1.86 (3H, s), 4.94 (1H, s), 6.64 (1H, s), 6.75 (2H, d, J=8.3 Hz), 6.87 (2H, d, J=8.5 Hz), 7.02 (1H, s), 7.10 (2H, d, J=8.5 Hz), 7.24 (2H, d, J=8.3 Hz).

Step 9: Ethyl (5R,6S)-5-(4-Bromophenyl)-6-(4-chlorophenyl)-3-isopropyl-6-methyl-5,6-dihydroimidazo[2,1-b][1,3]thiazole-2-carboxylate

The compound obtained in Step 8 above was reacted in the same way as in Step 2 of Example 1 to give the title compound as a colorless oil.
$^1$H-NMR ($CDCl_3$) δ: 0.89 (3H, d, J=7.1 Hz), 0.99 (3H, d, J=7.1 Hz), 1.32 (3H, t, J=7.2 Hz), 1.80 (3H, s), 3.32 (1H, s), 4.24 (2H, q, J=7.3 Hz), 5.05 (1H, s), 6.65 (2H, brs), 7.03 (2H, d, J=8.8 Hz), 7.12 (2H, d, J=8.5 Hz), 7.19 (2H, d, J=8.5 Hz).

Step 10: (5R,6S)-5-(4-Bromophenyl)-6-(4-chlorophenyl)-3-isopropyl-6-methyl-5,6-dihydroimidazo[2,1-b][1,3]thiazole-2-carboxylic acid

The compound obtained in Step 9 above was reacted in the same way as in Step 3 of Example 1 to give the title compound as a colorless solid.

Reference Example 49

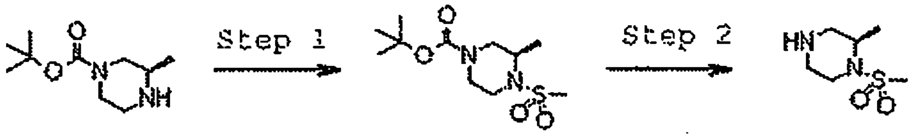


Step 1: tert-Butyl (3R)-3-Methyl-4-(methylsulfonyl)piperazine-1-carboxylate

Methanesulfonyl chloride (1.28 ml, 16.0 mmol) was added dropwise to a tetrahydrofuran (30 ml) solution of tert-butyl (3R)-3-methylpiperazine-1-carboxylate (3.0 g, 15.0 mmol) and triethylamine (3.2 ml, 23.0 mmol) under ice cooling. The resulting mixture was stirred for 1 hour and diluted with ethyl acetate. The organic layer was washed with a 10% aqueous solution of citric acid, saturated aqueous sodium bicarbonate solution, and brine and dried over anhydrous magnesium sulfate. The solvent was evaporated under reduced pressure to give the title compound (4.4 g, quantitative) as a colorless solid.

Step 2: (2R)-2-Methyl-1-(methylsulfonyl)piperazine

The compound obtained in Step 1 above was reacted in the same way as in Step 2 of Example 214 to give the title compound as a colorless solid.
$^1$H-NMR ($CDCl_3$) δ: 1. 34 (3H, d, J=6. 8 Hz), 2. 91 (1H, td, J=12.3, 3.7 Hz), 3.03 (3H, s), 3.08-3.12 (1H, m), 3.15-3.18 (1H, m), 3.29-3.33 (2H, m), 3.62 (1H, d, J=14.4 Hz), 4.10-4.14 (1H, m).
MS (ESI) m/z: 179.

Reference Example 50

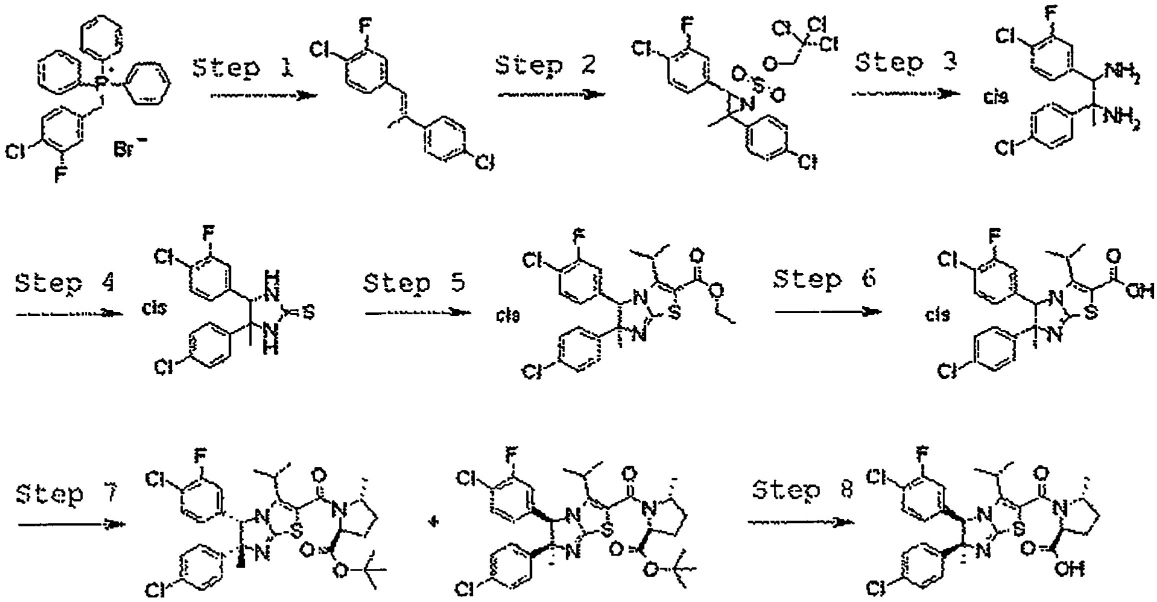


Step 1: 1-Chloro-4-[2-(4-chlorophenyl)propen-1-yl]-2-fluorobenzene

A 2.77 M solution of n-butyllithium in hexane (100 ml, 277 mmol) was added dropwise to a tetrahydrofuran (400 ml) suspension of (4-chloro-3-fluorobenzyl)triphenylphosphonium bromide (112 g, 231 mmol) at -20°C in a nitrogen atmosphere. The resulting mixture was stirred for 30 minutes, then a tetrahydrofuran solution (300 ml) of 4-chloroacetophenone (33 ml, 254 mmol) was added dropwise, and the resulting mixture was stirred at room temperature for 17 hours. An aqueous solution of saturated ammonium chloride was added to the reaction mixture, followed by extraction with ethyl acetate. The extract was washed with brine and dried over anhydrous sodium sulfate and then the solvent was evaporated. The obtained residue was purified by silica gel column chromatography (hexane:ethyl acetate = 9:1) to give the title compound (39 g, 60%; E:Z = 3:1) as a light brown solid.
$^1$H-NMR ($CDCl_3$) δ: 2.17 (0.75H, d, J=1.5 Hz, Me: Z isomer), 2.23 (2.25H, d, J=1.2 Hz, Me: E isomer), 6.39 (0.25H, s, CH=C: Z isomer), 6.64 (0.25H, dd, J=8.3, 1.7 Hz, Ar-H: Z isomer), 6.68-6.71 (1H, m), 7.04-7.14 (2.25H, m), 7.26 (0.5H, m), 7.31-7.43 (3.75H, m)

Step 2: 2,2,2-Trichloroethyl (2R*,3R*)-3-(4-Chloro-3-fluorophenyl)-2-(4-chlorophenyl)-2-methylaziridine-1-sulfamate

The compound obtained in Step 1 above was reacted in the same way as in Step 2 of Reference Example 14 to give the title compound as a colorless solid.
$^1$H-NMR ($CDCl_3$) δ: 1.43 (3H, s), 4.60 (1H, s), 4.73 (2H, dd, J=29.1, 10.9 Hz), 7.21 (1H, d, J=8.0 Hz), 7.26 (1H, dd, J=9.3, 2.0 Hz), 7.42 (2H, dt, J=8.8, 2.0 Hz), 7.48 (1H, t, J=7.8 Hz), 7.52-7.55 (2H, m).

Step 3: 1-(4-Chloro-3-fluorophenyl)-2-(4-chlorophenyl)propane-1,2-diamine

The compound obtained in Step 2 above was reacted in the same way as in Step 3 of Reference Example 14 and then subjected to the same reaction as in Step 4 of Reference Example 14 without being purified to give the title compound as a pale yellow oil.
$^1$H-NMR ($CDCl_3$) δ: 1.49 (3H, s), 1.53 (4H, brs), 4.07 (1H, s), 6.74 4 (1H, dd, J=8.2, 1.6 Hz), 6.93 (1H, dd, J=10.5, 2.0 Hz), 7.19 (1H, t, J=7.9 Hz), 7.26 (4H, s).

Step 4: (4S*,5R*)-5-(4-Chloro-3-fluorophenyl)-4-(4-chlorophenyl)-4-methylimidazopyridine-2-thione

The compound obtained in Step 3 above was reacted in the same way as in Step 1 of Example 1 to give the title compound. This compound was used in next reaction without being purified.

Step 5: Ethyl (5R*,6S*)-5-(4-Chloro-3-fluorophenyl)-6-(4-chlorophenyl)-3-isopropyl-6-methyl-5,6-dihydroimidazo[2,1-b][1,3]thiazole-2-carboxylate

The compound obtained in Step 4 above was reacted in the same way as in Step 2 of Example 1 to give the title compound as a pale yellow solid.
$^1$H-NMR ($CDCl_3$) δ: 0.90 (3H, d, J=7.3 Hz), 1.02 (3H, d, J=7.1 Hz), 1.34 (3H, t, J=7.2 Hz), 1.80 (3H, s), 3.35-3.37 (1H, m), 4.25 (2H, q, J=7.1 Hz), 5.03 (1H, s), 6.54 (2H, brs), 7.07-7.12 (5H, m).

Step 6: (5R*,6S*)-5-(4-Chloro-3-fluorophenyl)-6-(4-chlorophenyl)-3-isopropyl-6-methyl-5,6-dihydroimidazo[2,1-b][1,3]thiazole-2-carboxylic acid

The compound obtained in Step 5 above was reacted in the same way as in Step 3 of Example 1 to give the title compound as a colorless solid.
$^1$H-NMR (DMSO-$d_6$) δ: 0.91 (3H, d, J=7.3 Hz), 0.98 (3H, d, J=7.1 Hz), 1.18 (1H, td, J=7.1, 0.9 Hz), 1.89 (3H, s), 5.98 (1H, s), 7.20-7.38 (7H, m).

Step 7: tert-Butyl (5R)-1-{[(5R,6S)-5-(4-Chloro-3-fluorophenyl)-6-(4-chlorophenyl)-3-isopropyl-6-methyl-5,6-dihydroimidazo[2,1-b][1,3]thiazol-2-yl]carbonyl}-5-methyl-L-prolinate

The compound obtained in Step 6 above used instead of the compound obtained in Step 3 of Example 1 and the compound obtained in Reference Example 30 used instead of the compound obtained in Step 2 of Reference Example 18 were reacted in the same way as in Example 112 to give the title compound as a pale yellow solid.
$^1$H-NMR ($CDCl_3$) δ: 1.02-1.05 (6H, m), 1.21 (3H, d, J=6.3 Hz), 1.45 (9H, s), 1.68 (1H, brs), 1.80 (3H, s), 1.98 (1H, brs), 2.27 (2H, brs), 2.62 (1H, brs), 4.52 (2H, brs), 4.92 (1H, s), 6.53 (2H, dd, J=15.3, 7.6 Hz), 7.06-7.08 (3H, m), 7.15 (2H, d, J=8.5 Hz).

Step 8: (5R)-1-{[(5R,6S)-5-(4-Chloro-3-fluorophenyl)-6-(4-chlorophenyl)-3-isopropyl-6-methyl-5,6-dihydroimidazo[2,1-b][1,3]thiazol-2-yl]carbonyl}-5-methyl-L-proline

The compound obtained in Step 7 above was reacted in the same way as in Step 6 of Example 102 to give the title compound as a colorless solid.
$^1$H-NMR ($CDCl_3$) δ: 0.88 (3H, t, J=6.8 Hz), 0.95-0.97 (3H, m), 1.08 (3H, d, J=7.1 Hz), 1.25-1.26 (3H, m), 2.05 (3H, d, J=3.2 Hz), 2.24 (3H, m), 4.47-4.50 (2H, m), 5.33-5.35 (1H, m), 7.07-7.23 (7H, m).

Reference Example 51

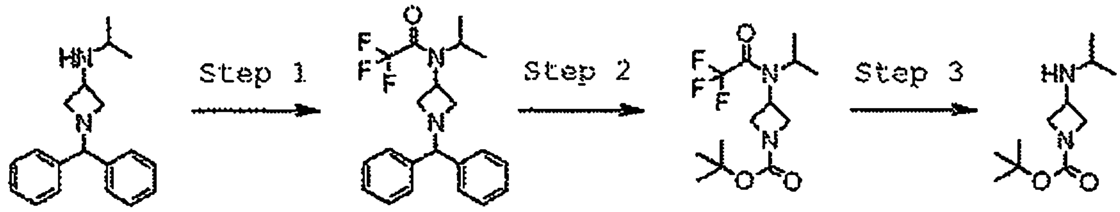


Step 1: N-[1-(Diphenylmethyl)azetidin-3-yl]-2,2,2-trifluoro-N-isopropylacetamide

Anhydrous trifluoroacetic acid (1.16 ml, 8.39 mmol) was added dropwise to a dichloromethane (40 ml) solution of 1-(diphenylmethyl)-N-isopropylazetidin-3-amine (2.14 g, 7.63 mmol) and triethylamine (2.20 ml, 15.3 mmol) under ice cooling. The resulting mixture was stirred at the same temperature for 1 hour and then an aqueous solution of saturated ammonium chloride was added, followed by extraction with chloroform. The organic layer was washed with brine and then dried over anhydrous sodium sulfate. The solvent was evaporated under reduced pressure to give a crude product (3.35 g) of the title compound. This compound was used in next reaction without being purified.

Step 2: tert-Butyl 3-[Isopropyl(trifluoroacetyl)amino]azetidine-1-carboxylate

The crude product obtained in Step 1 above was reacted in the same way as in Step 6 of Reference Example 6 and then subjected to the same reaction as in Step 4 of Reference Example 9 to give the title compound as a colorless oil. $^1$H-NMR ($CDCl_3$) δ: 1.21 (6H, d, J=6.6 Hz), 1.45 (9H, s), 3.97-4.05 (2H, m), 4.10-4.27 (2H, m), 4.51-4.83 (1H, m).

Step 3: tert-Butyl 3-(isopropylamino)azetidine-1-carboxylate

The compound obtained in Step 2 above was reacted in the same way as in Step 2 of Example 193 to give the title compound as a colorless oil.
$^1$H-NMR ($CDCl_3$) δ: 1.03 (6H, d, J=6.3 Hz), 1.42 (9H, s), 2.80 (1H, m), 3.56-3.66 (3H, m), 4.06-4.11 (2H, m). Reference Example 52

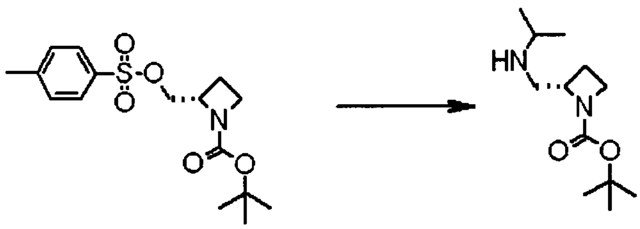

tert-Butyl (2S)-2-[(Isopropylamino)methyl]azetidine-1-carboxylate

Isopropylamine (9 ml) was added to an 2-propanol (50 ml) solution of tert-butyl (2S)-2-({[(4-methylphenyl) sulfonyl]oxy}methyl)azetidine-1-carboxylate (3.41 g, 9.98 mmol) and the resulting mixture was heated at 70°C for 16 hours. After cooling, the reaction mixture was concentrated under reduced pressure to give the title compound (1.91 g, 68%) as a yellow oil.
$^1$H-NMR ($CDCl_3$) δ: 1.05 (6H, d, J=6.2 Hz), 1.43 (9H, s), 1.94 (1H, m), 2.23 (1H, m), 2.75-2.90 (3H, m), 3.74-3.86 (2H, m), 4.32 (1H, m).

Reference Example 53

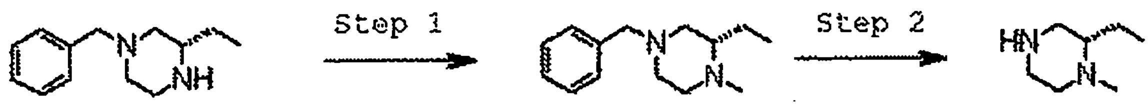


Step 1: (2S)-4-Benzyl-2-ethyl-1-methylpiperazine

(3S)-1-Benzyl-3-ethylpiperazine was reacted in the same way as in Step 3 of Example 152 to give the title compound as a pale orange oil.
$^1$H-NMR ($CDCl_3$) δ: 0.86 (3H, t, J=7.6 Hz), 1.33-1.40 (1H, m), 1.58-1.66 (1H, m), 1.94 (1H, t, J=10.2 Hz), 1.99-2.06 (1H, m), 2.19 (1H, td, J=10.9, 2.4 Hz), 2.27 (3H, s), 2.32 (1H, td, J=11.1, 2.4 Hz), 2.69-2.80 (3H, m), 3.44 (1H, d, J=13.2 Hz), 3.55 (1H, d, J=12.9 Hz), 7.24-7.26 (1H, m), 7.30-7.33 (4H, m).
MS (ESI) m/z: 219.

Step 2: (2S)-2-Ethyl-1-methylpiperazine

(2S)-4-Benzyl-2-ethyl-1-methylpiperazine was reacted in the same way as in Step 4 of Reference Example 13 to give the title compound as a pale yellow solid.
$^1$H-NMR (DMSO-$d_6$) δ: 0.91 (3H, t, J=7.6 Hz), 1.64-1.71 (1H, m), 1.93-2.00 (1H, m), 2.80 (3H, s), 3.40-3.52 (7H, m).MS (ESI) m/z: 129.

Reference Example 54

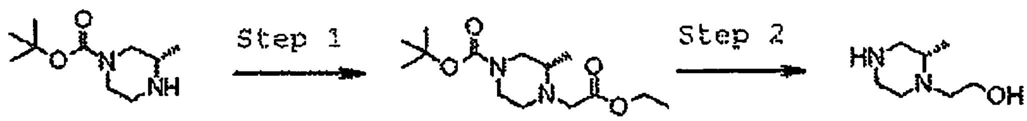


Step 1: tert-Butyl (3S)-4-(2-Ethoxy-2-oxoethyl)-3-methylpiperazine-1-carboxylate

Potassium carbonate (2.07 g, 15.0 mmol) and ethyl bromoacetate (2.16 ml, 19.5 mmol) were added to an acetonitrile (50 ml) solution of (S)-4-N-(tert-butoxycarbonyl)-2-methylpiperazine (3.00 g, 15.0 mmol) and the resulting mixture was heated and stirred at 60°C for 18 hours. Insoluble matter was removed by filtration through a celite pad and then filtrate was concentrated under reduced pressure. The obtained residue was purified by silica gel column chromatography (hexane:ethyl acetate = 1:1) to give the title compound (5.30 g, quantitative) as a pale yellow oil.
$^1$H-NMR ($CDCl_3$) δ: 1.05 (3H, d, J=6.1 Hz), 1.27 (3H, t, J=7.2 Hz), 1.46 (9H, s), 2.56-2.80 (4H, m), 3.04-3.11 (1H, m), 3.37 (2H, dd, J=35.6, 16.6 Hz), 3.63-3.94 (2H, m), 4.18 (2H, q, J=7.2 Hz).

Step 2: 2-[(2S)-2-Methylpiperazin-1-yl]ethanol

The compound obtained in Step 1 above was reacted in the same way as in Example 9. Then, the obtained alcohol form was reacted in the same way as in Step 2 of Example 214 to give the title compound as a brown oil.
$^1$HNMR (DMSO-$d_6$) δ: 1.37 (3H, d, J=6.3 Hz), 3.13-3.79 (12H, m), 10.07 (2H, brs), 11.55 (1H, brs).

Reference Example 55

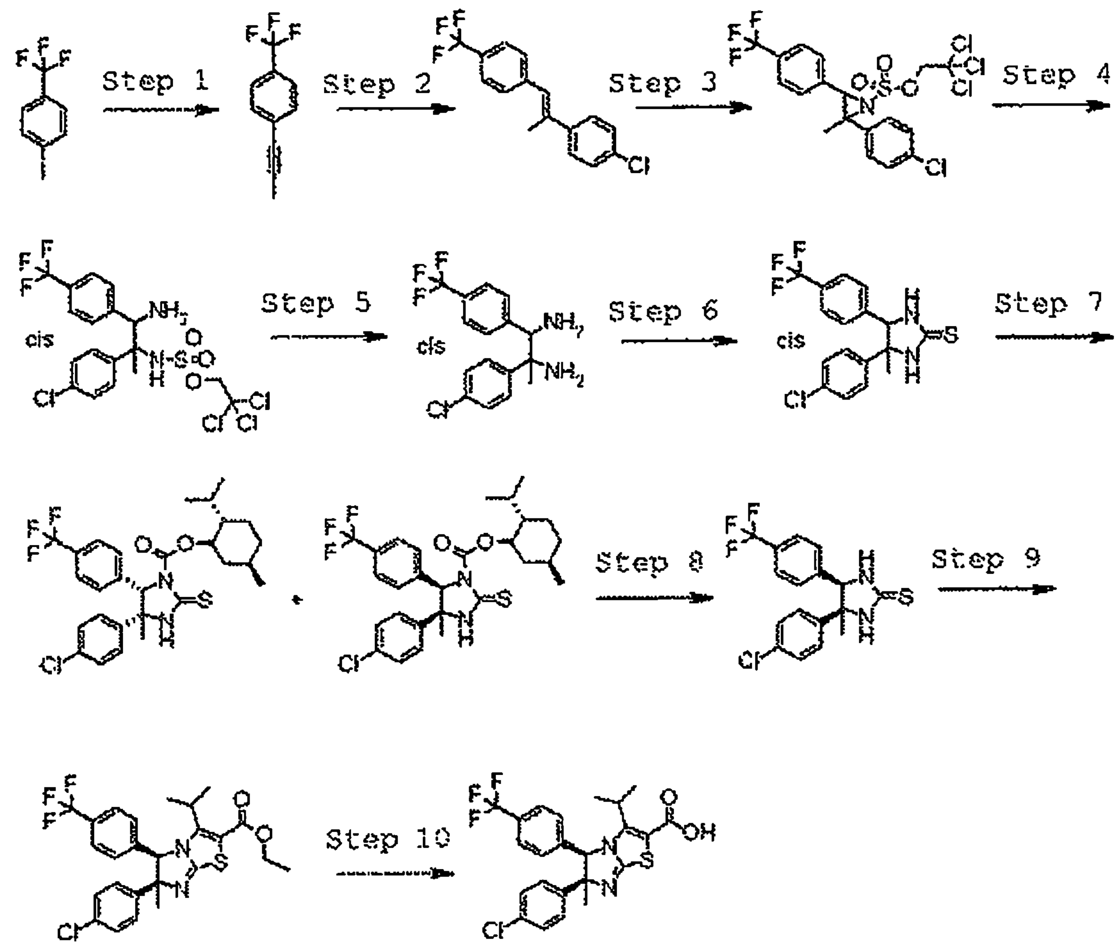


Step 1: 1-Prop-1-yn-1-yl-4-(trifluoromethyl)benzene

4-Iodobenzotrifluoride used instead of 4-chloro-2-fluoro-1-iodobenzene was reacted in the same way as in Step 1 of Reference Example 45 to give the title compound as a colorless oil.
$^1$H-NMR ($CDCl_3$) δ: 2.07 (3H, s), 7.47 (2H, d, J=8.3 Hz), 7.53 (2H, d, J=8.3 Hz).
MS (ESI) m/z: 185.

Step 2: 1-Chloro-4-{(E)-1-methyl-2-[4-(trifluoromethyl)phenyl]vinyl}benzene

The compound obtained in Step 1 above was reacted in the same way as in Step 1 of Reference Example 39 to give the title compound as a colorless solid.
$^1$H-NMR ($CDCl_3$) δ: 2.25 (3H, d, J=1.5 Hz), 6.81 (1H, s), 7.35 (2H, d, J=8.8 Hz), 7.43-7.46 (4H, m), 7.62 (2H, d, J=8.3 Hz).
MS (ESI) m/z: 297.

Step 3: 2,2,2-Trichloroethyl (2R*,3R*)-2-(4-Chlorophenyl)-2-methyl-3-[4-(trifluoromethyl)phenyl]aziridine-1-sulfonate

The compound obtained in Step 2 above was reacted in the same way as in Step 2 of Reference Example 14 to give the title compound as a colorless solid.
$^1$H-NMR ($CDCl_3$) δ: 1.42 (3H, s), 4.69 (1H, s), 4.71 (1H, d, J=11.0 Hz), 4.78 (1H, d, J=11.0 Hz), 7.42 (2H, d, J=8.5 Hz), 7.55-7.61 (4H, m), 7.71 (2H, d, J=8.5 Hz).
MS (ESI) m/z: 522.

Step 4: 2,2,2-Trichloroethyl {(1S*,2R*)-2-Amino-1-(4-chlorophenyl)-1-methyl-2-[4-(trifluoromethyl)phenyl]ethyl}sulfamate

The compound obtained in Step 3 above was reacted in the same way as in Step 3 of Reference Example 14 to give the title compound. This compound was used in next reaction without being purified.

Step 5: (1R*,2S*)-2-(4-Chlorophenyl)-1-[4-(trifluoromethyl)phenyl]propane-1,2-diamine

The compound obtained in Step 4 above was reacted in the same way as in Step 4 of Reference Example 14 to give the title compound. This compound was used in next reaction without being purified.

Step 6: (4S*,5R*)-4-(4-Chlorophenyl)-4-methyl-5-[4-(trifluoromethyl)phenyl]imidazolidine-2-thione

The compound obtained in Step 5 above was reacted in the same way as in Step 1 of Example 1 to give the title compound as a colorless solid.
$^1$H-NMR ($CDCl_3$) δ: 1.91 (3H, s), 5.04 (1H, s), 6.36 (1H, s), 6.60 (1H, s), 6.86 (2H, d, J=8.5 Hz), 7.01 (2H, d, J=8.5 Hz), 7.08 (2H, d, J=8.5 Hz), 7.38 (2H, d, J=8.5 Hz).
MS (ESI) m/z: 371.

Step 7: (2S,5R)-2-Isopropyl-5-methylcyclohexyl (4S,5R)-4-(4-Chlorophenyl)-4-methyl-2-thioxo-5-[4-(trifluoromethyl) phenyl]imidazolidine-1-carboxylate

The compound obtained in Step 6 above was reacted in the same way as in Step 6 of Reference Example 38 to give the title compound as a colorless solid.
$^1$H-NMR ($CDCl_3$) δ: 0.17-0.26 (1H, m), 0.61-0.68 (1H, m), 0.69 (3H, d, J=6.6 Hz), 0.70 (3H, d, J=6.6 Hz), 0.88 (3H, d, J=7.1 Hz), 0.92-0.99 (1H, m), 1.16-1.28 (2H, m), 1.51-1.61 (3H, m), 1.85-1.90 (1H, m), 1.94 (3H, s), 4.55 (1H, td, J=10.9, 4.3 Hz), 5.32 (1H, s), 6.96-7.00 (4H, m), 7.08 (2H, d, J=8.8 Hz), 7.35 (2H, d, J=8.3 Hz), 7.73 (1H, s).
MS (ESI) m/z: 553.

Step 8: (4S,5R)-4-(4-Chlorophenyl)-4-methyl-5-[4-(trifluoromethyl)phenyl]imidazolidine-2-thione

The compound obtained in Step 7 above was reacted in the same way as in Step 7 of Reference Example 38 to give the title compound as a colorless solid.
$^1$H-NMR ($CDCl_3$) δ: 1.92 (3H, s), 5.04 (1H, s), 6.39 (1H, s), 6.63 (1H, s), 6.86 (2H, d, J=8.8 Hz), 7.02 (2H, d, J=8.0 Hz), 7.08 (2H, d, J=8.8 Hz), 7.38 (2H, d, J=8.0 Hz).
MS (ESI) m/z: 371.

Step 9: Ethyl (5R,6S)-6-(4-Chlorophenyl)-3-isopropyl-6-methyl-5-[4-(trifluoromethyl)phenyl]-5,6-dihydroimidazo[2,1-b] [1,3]thiazole-2-carboxylate

The compound obtained in Step 8 above was reacted in the same way as in Step 2 of Example 1 to give the title compound as a colorless solid.
$^1$H-NMR ($CDCl_3$) δ: 0.87 (3H, d, J=7. 3 Hz), 0. 98 (3H, d, J=7.3 Hz), 1.33 (3H, t, J=7.1 Hz), 1.84 (3H, s), 3.29-3.37 (1H, m), 4.25 (2H, q, J=7.1 Hz), 5.13 (1H, s), 6.88 (2H, d, J=8.5 Hz), 7.00 (2H, d, J=8.5 Hz), 7.10 (2H, d, J=8.5 Hz), 7.32 (2H, d, J=8.5 Hz).
MS (ESI) m/z: 509.

Step 10: (5R,6S)-6-(4-Chlorophenyl)-3-isopropyl-6-methyl-5-[4-(trifluoromethyl)phenyl]-5,6-dihydroimidazo[2,1-b][1,3] thiazole-2-carboxylic acid

The compound obtained in Step 9 above was reacted in the same way as in Step 3 of Example 1 to give the title compound as a pale orange solid.
$^1$H-NMR ($CDCl_3$) δ: 0.86 (3H, d, J=7.1 Hz), 1.02 (3H, d, J=7.1 Hz), 2.05 (3H, s), 3.46-3.57 (1H, m), 4.81 (1H, brs), 5.40 (1H, s), 6.82-6.98 (2H, m), 7.02 (2H, d, J=8.5 Hz), 7.13 (2H, d, J=8.5 Hz), 7.36 (2H, d, J=8.5 Hz).

Reference Example 56

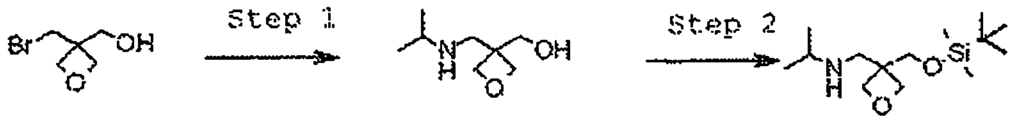


Step 1: {3-[(Isopropylamino)methyl]oxetan-3-yl}methanol

[3-(Bromomethyl)oxetan-3-yl]methanol (6.00 g, 33 mmol) was dissolved in isopropanol (60 ml), isopropylamine (28.5 ml, 0.33 mol) was added, and the resulting mixture was heated and stirred at 70°C for 16 hours. After concentration, the obtained residue was dissolved in ethanol (50 ml), potassium hydroxide (2.2 g, 33 mmol) was added, and the resulting mixture was stirred for 1 hour. Insoluble matter was removed by filtration. After extraction with dichloromethane, the extract was dried over anhydrous sodium sulfate and then the solvent was evaporated under reduced pressure to give the title compound (5.05 g, 96%) as a colorless solid.
$^1$H-NMR ($CDCl_3$) δ: 1.08 (6H, d, J=6.3 Hz), 2.72-2.82 (1H, m), 3.15 (2H, s), 4.02 (2H, s), 4.41 (2H, d, J=6.1 Hz), 4.47 (2H, d, J=6.1 Hz).

Step 2: N-{[3-({[tert-Butyl(dimethyl)silyl]oxy}methyl)oxetan-3-yl]methyl}propan-2-amine

tert-Butyldimethylsilyl chloride (102 mg, 0.68 mmol) was added to an N,N-dimethylformamide (5 ml) solution of the compound (107 mg, 0.67 mmol) obtained in Step 1 above and imidazole (91.5 mg, 1.34 mmol) and the resulting mixture was stirred at room temperature for 19 hours.
The reaction mixture was diluted with water, followed by extraction with ethyl acetate. Then, the organic layer was washed with water and brine and then dried over anhydrous sodium sulfate. The solvent was evaporated under reduced pressure to give the title compound (187 mg, quantitative) as a colorless oil.
$^1$H-NMR ($CDCl_3$) δ: 0.07 (6H, s), 0.90 (9H, s), 1.05 (6H, d, J=6.3 Hz), 2.70-2.80 (1H, m), 2.90 (2H, s), 3.81 (2H, s), 4.41 (4H, s).

Reference Example 57

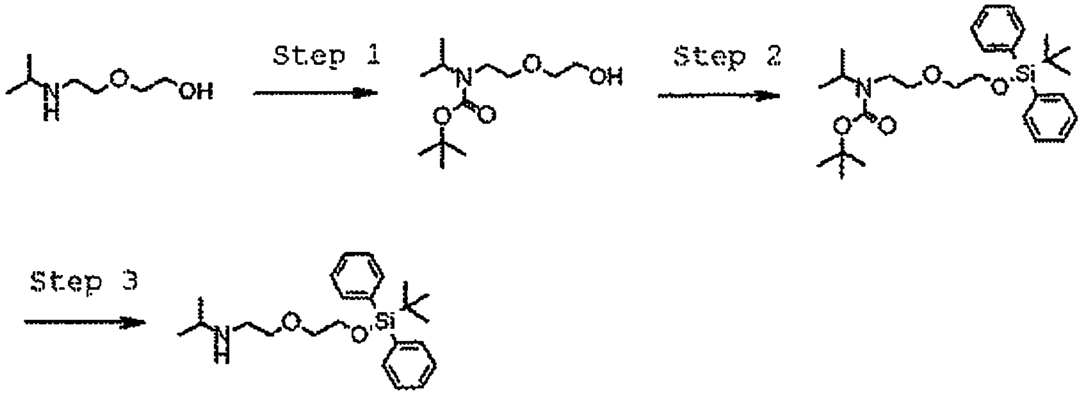


Step 1: tert-Butyl [2-(2-Hydroxyethoxy)ethyl]isopropylcarbamate

2-[2-(Dimethylamino)ethoxy]ethanol was reacted in the same way as in Step 4 of Reference Example 9 to give the title compound as a colorless oil.
$^1$H-NMR ($CDCl_3$) δ: 1.13 (6H, d, J=6.8 Hz), 1.46 (9H, s), 3.29 (2H, m), 3.55-3.59 (5H, m), 3.71-3.75 (2H, m).
MS (ESI) m/z: 270 (M+23)$^+$.

Step 2: tert-Butyl [2-(2-{[tert-Butyl(diphenyl)silyl]oxy}ethoxy)ethyl]isopropylcarbamate

The compound obtained in Step 1 above was reacted in the same way as in Step 2 of Reference Example 56

to give the title compound as a colorless oil.
$^1$H-NMR ($CDCl_3$) δ: 1.05 (9H, s), 1.06 (3H, d, J=6.8 Hz), 1.11 (3H, d, J=6.8 Hz), 1.45 (9H, s), 3.22 (2H, br), 3.54-3.58 (5H, m), 3.78 (2H, t, J=5.5 Hz), 7.35-7.44 (6H, m), 7.67-7.72 (4H, m).

Step 3: tert-Butyl [2-(2-{[tert-Butyl(diphenyl)silyl]oxy}ethoxy)ethyl]isopropylcarbamate

The compound obtained in Step 2 above was reacted in the same way as in Step 6 of Example 102 to give the title compound as a colorless oil.
$^1$H-NMR ($CDCl_3$) δ: 1.04-1.07 (15H, m), 2.74-2.82 (3H, m), 3.55-3.60 (4H, m), 3.81 (2H, t, J=5.1 Hz), 7.35-7.44 (6H, m), 7.67-7.72 (4H, m).
MS (ESI) m/z: 386.

Reference Example 58

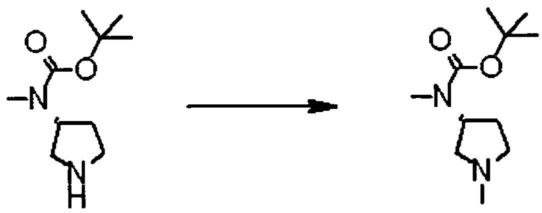

tert-Butylmethyl [(3R)-1-Methylpyrrolidin-3-yl]carbamate

tert-Butylmethyl [(3R)-pyrrolidin-3-yl]carbamate was reacted in the same way as in Step 2 of Example 168 to give the title compound as a colorless oil.
$^1$H-NMR ($CDCl_3$) δ: 1.46 (9H, s), 1.75-1.87 (1H, m), 2.09-2.23 (1H, m), 2.39 (3H, s), 2.40-2.49 (1H, m), 2.56-2.91 (3H, m), 2.82 (3H, s), 4.70-4.82 (1H, m).

Reference Example 59

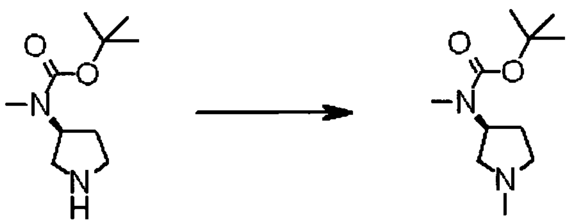

tert-Butylmethyl [(3S)-1-Methylpyrrolidin-3-yl]carbamate

tert-Butylmethyl [(3R)-pyrrolidin-3-yl]carbamate was reacted in the same way as in Step 2 of Example 168 to give the title compound as a pale yellow oil.
$^1$H-NMR ($CDCl_3$) δ: 1.46 (9H, s), 1.81 (1H, td, J=13.7, 7.5 Hz), 2.15 (1H, s), 2.39 (3H, s), 2.42-2.48 (1H, m), 2.66 (2H, s), 2.82 (4H, s), 4.76 (1H, s).

Reference Example 60

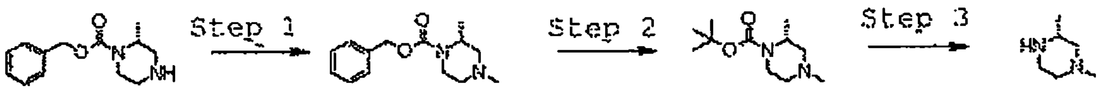


Step 1: Benzyl (2R)-2,4-Dimethylpiperazine-1-carboxylate

(R)-2-Methylpiperazine-1-carboxylic acid benzyl ester was reacted in the same way as in Step 2 of Example 168 to give the title compound as a colorless oil.
$^1$H-NMR ($CDCl_3$) δ : 1.27 (3H, d, J=6.8 Hz), 1.92 (1H, td, J=11.8, 3.4 Hz), 2.11 (1H, dd, J=11.4, 4.0 Hz), 2.24 (3H, s), 2.59 (1H, d, J=11.5 Hz), 2.73 (1H, d, J=10.0 Hz), 3.18 (1H, td, J=12.8, 3.3 Hz), 3.91 (1H, d, J=12.9 Hz), 4.30 (1H, t, J=4.9 Hz), 5.13 (2H, dd, J=15.4, 12.5 Hz), 7.28-7.38 (5H, m).

Step 2: tert-Butyl (2R)-2,4-Dimethylpiperazine-1-carboxylate

The compound obtained in Step 1 above was reacted in the same way as in Step 4 of Reference Example 13. The obtained amine form was reacted in the same way as in Step 4 of Reference Example 9 to give the title compound as a brown oil.
$^1$H-NMR ($CDCl_3$) δ: 0.88 (1H, t, J=6.8 Hz), 1.24 (3H, d, J=7.1 Hz), 1.46 (9H, d, J=0.5 Hz), 1.90 (1H, td, J=11.8, 3.5 Hz), 2.10 (1H, dd, J=11.2, 4.2 Hz), 2.24 (3H, s), 2.58 (1H, d, J=11.2 Hz), 2.72 (1H, d, J=11.2 Hz), 3.10 (1H, td, J=12.8, 3.3 Hz), 3.81 (1H, d, J=13.4 Hz), 4.20 (1H, s).

Step 3: (3R)-1,3-Dimethylpiperazine

The compound obtained in Step 2 above was reacted in the same way as in Step 2 of Example 214 to give the title compound as a brown oil.
MS (ESI) m/z: 115.

Reference Example 61

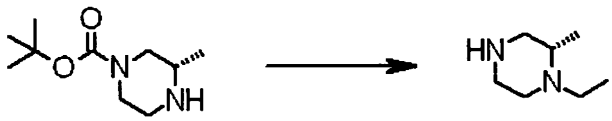

(2S)-1-Ethyl-2-methylpiperazine

(S)-4-N-(tert-Butoxycarbonyl)-2-methylpiperazine was reacted in the same way as in Step 3 of Example 152 using acetaldehyde instead of a 37% aqueous solution of formalin. Subsequently, the compound above was treated with a 30% aqueous solution of hydrochloric acid and then washed with dichloromethane. Then, the aqueous layer was concentrated under reduced pressure to give the title compound as an orange oil.
$^1$HNMR (DMSO-$d_6$) δ: 1.21 (3H, t, J=7.1 Hz), 1.36 (3H, d, J=6.6 Hz), 3.05-3.63 (9H, m), 10.04 (2H, brs), 12.00 (1H, brs).

Reference Example 62

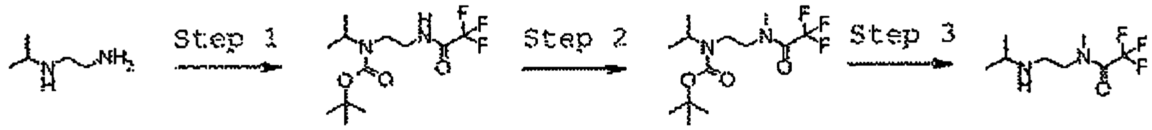


Step 1: tert-Butyl Isopropyl{2-[(trifluoroacetyl)amino]ethyl}carbamate

Ethyl trifluoroacetate (2.40 ml, 20 mmol) was added dropwise to a tetrahydrofuran (2 ml) solution of N-isopropylethylenediamine (2.04 g, 20 mmol) under ice cooling and the resulting mixture was stirred at the same temperature for 30 minutes. Subsequently, di-tert-butyl dicarbonate (4.85 ml, 21 mmol) was added and the resulting mixture was brought to room temperature and further stirred for 4 hours. The mixture was diluted with ethyl acetate, washed with water and brine, and then dried over anhydrous sodium sulfate. The drying agent was removed by filtration and the solvent was removed by concentration under reduced pressure. The obtained residue was purified by silica gel column chromatography (ethyl acetate:hexane = 1:9) to give the title compound (5.92 g, 99%) as a colorless oil.
$^1$H-NMR ($CDCl_3$) δ: 1.14 (6H, d, J=6.9 Hz), 1.47 (9H, s), 3.41-3.45 (4H, m), 4.11 1H, br), 8.45 (1H, br).
MS (ESI) m/z: 321 $(M+23)^+$.

Step 2: tert-Butyl Isopropyl{2-[methyl(trifluoroacetyl)amino]ethyl}carbamate

A tetrahydrofuran (15 ml) solution of the compound (3.00 g, 10 mmol) obtained in Step 1 above was added dropwise to an N,N-dimethylformamide (15 ml) suspension of sodium hydride (55% oil, 500 mg, 11.5 mmol) under ice cooling. After 20 minutes, methyl iodide (1.0 ml, 16 mmol) was added and the resulting mixture was brought to room temperature and stirred for 1 hour. An aqueous solution of saturated ammonium chloride was added to the reaction mixture, followed by extraction with ethyl acetate. The extract was washed with water and brine and then dried over anhydrous sodium sulfate. The drying agent was removed by filtration and the solvent was removed by concentration under reduced pressure. The obtained residue was purified by silica gel column chromatography (ethyl acetate:hexane = 1:4) to give the title compound (2.72 g, 87%) as a colorless oil.
$^1$H-NMR ($CDCl_3$) δ: 1.10-1.14 (6H, m), 1.48 (9H, s), 3.10 and 3.19 (total 3H, each brs), 3.25 (2H, br), 3.54 (2H, t, J=7.0 Hz), 4.11 and 4.37 (total 1H, each m)

Step 3: 2,2,2-Trifluoro-N-[2-(isopropylamino)ethyl]-N-methylacetamide

The compound obtained in Step 2 above was reacted in the same way as in Step 6 of Example 102 to give the title compound as a colorless oil.
$^1$H-NMR ($CDCl_3$) δ: 1.08 and 1.13 (total 6H, each d, J=6.2 Hz), 2.59 (1H, br), 2.82-2.88 (1H, m), 2.91-2.99 (2H, m), 3.07 and 3.18 (total 3H, each s), 3.52 and 3.58 (total 2H, each t, J=6.6 Hz).
MS (ESI) m/z: 213.

Reference Example 63

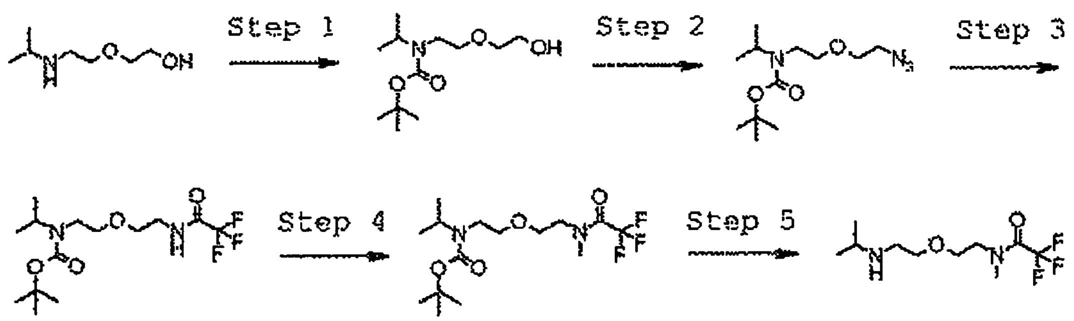

Step 1: tert-Butyl [2-(2-Hydroxyethoxy)ethyl]isopropylcarbamate

2-[2-(Dimethylamino)ethoxy]ethanol was reacted in the same way as in Step 4 of Reference Example 9 to give the title compound as a colorless oil.
$^1$H-NMR ($CDCl_3$) δ: 1.13 (6H, d, J=6.8 Hz), 1.46 (9H, s), 3.29 (2H, m), 3.55-3.59 (5H, m), 3.71-3.75 (2H, m).
MS (ESI) m/z: 270 $(M+23)^+$.

Step 2: tert-Butyl [2-(2-Azidoethoxy)ethyl]isopropylcarbamate

Methanesulfonyl chloride (0.74 ml, 9.56 mmol) was added under ice cooling to a toluene (25 ml) solution of the compound (2.35 g, 9.50 mmol) obtained in Step 1 above and triethylamine (1.35 ml, 9.70 mmol) and the resulting mixture was stirred for 15 minutes. Subsequently, an aqueous solution (20 ml) of sodium azide (4.94 g, 76 mmol) and tetra-n-butylammonium bromide (323 mg, 1.0 mmol) was added and the resulting mixture was heated and stirred at 60°C for 24 hours. The mixture was left standing to cool and then the reaction mixture was diluted with water, followed by extraction with ethyl acetate. The extract was washed with water and brine and then dried over anhydrous sodium sulfate. The drying agent was removed by filtration and the solvent was removed by concentration under reduced pressure. The obtained residue was purified by silica gel column chromatography (ethyl acetate:hexane = 1:10) to give the title compound (2.20 g, 85%) as a colorless oil.
$^1$H-NMR ($CDCl_3$) δ: 1.13 (6H, d, J=6.6 Hz), 1.46 (9H, s), 3.28 (2H, m), 3.37 (2H, t, J=5.1 Hz), 3.56 (2H, t, J=5.8 Hz), 3.64 (2H, t, J=5.1 Hz), 4.29 (1H, m).

Step 3: tert-Butyl Isopropyl(2-{2-[(trifluoroacetyl)amino]ethoxy}ethyl)carbamate

The compound obtained in Step 2 above was reacted in the same way as in Step 3 of Reference Example 9 using 5% palladium-carbon instead of a Lindlar catalyst and then subjected to the same reaction as in Step 1 of Reference Example 51 to give the title compound as a colorless oil.
$^1$H-NMR ($CDCl_3$) δ: 1.13 (6H, d, J=6.8 Hz), 1.46 (9H, s), 3.28 (2H, br), 3.54-3.59 (6H, m), 4.32 (1H, m).

Step 4: tert-Butyl Isopropyl(2-{2-[methyl(trifluoroacetyl)amino]ethoxy}ethyl)carbamate

The compound obtained in Step 3 above was reacted in the same way as in Step 2 of Reference Example 62 to give the title compound as a colorless oil.
$^1$H-NMR ($CDCl_3$) δ: 1.12 (6H, d, J=6.8 Hz), 1.46 (9H, s), 3.10 and 3.21 (total 3H, each s), 3.20-3.27 (2H, m), 3.51 (2H, m), 3.61-3.66 (4H, m), 4.25 (1H, m).

Step 5: 2,2,2-Trifluoro-N-{2-[2-(isopropylamino)ethoxy]ethyl}-N-methylacetamide

The compound obtained in Step 4 above was reacted in the same way as in Step 6 of Example 102 to give the title compound as a colorless oil.
$^1$H-NMR ($CDCl_3$) δ: 1.10 and 1.15 (total 6H, each d, J=7.4 Hz), 2.31 (1H, m), 2.81-2.98 (3H, m), 3.09 and 3.20 (total 3H, each s), 3.58-3.69 (6H, m).
MS (ESI) m/z: 257.

Reference Example 64

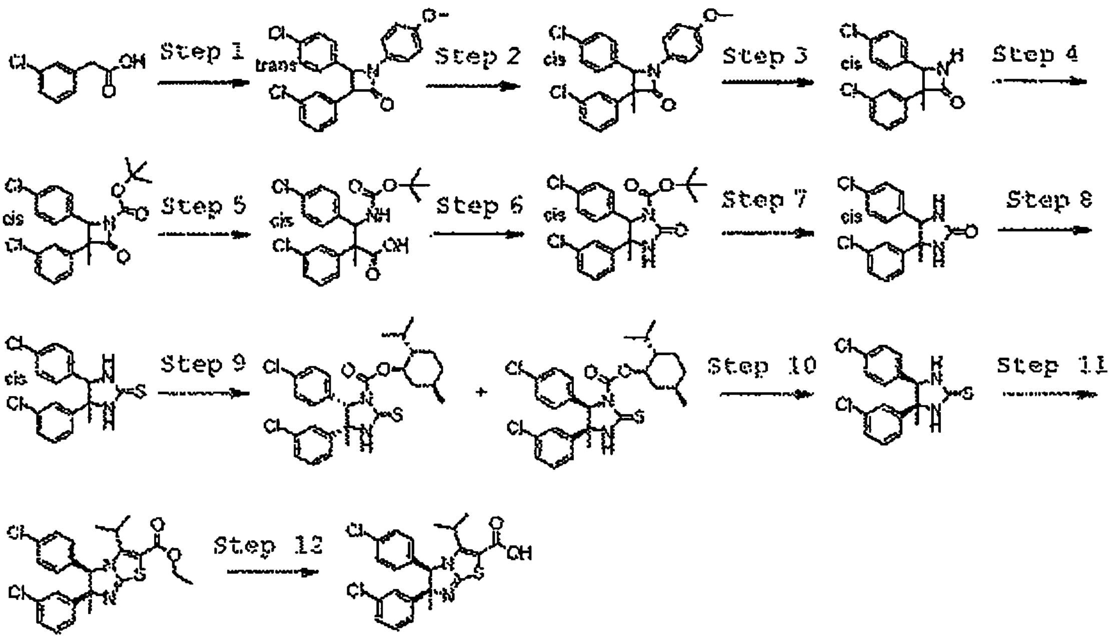


Step 1: (3R*,4S*)-3-(3-Chlorophenyl)-4-(4-chlorophenyl)-1-(4-methoxyphenyl)azetidin-2-one

3-Chlorophenylacetic acid was reacted in the same way as in Step 1 of Reference Example 37 to give the title compound as a pale yellow solid.
$^1$H-NMR ($CDCl_3$) δ: 3.76 (3H, s), 4.19 (1H, d, J=2.8 Hz), 4.87 (1H, d, J=2.3 Hz), 6.80-6.84 (2H, m), 7.20-7.40 (10H, m) .

Step 2: (3S*,4R*)-3-(3-Chlorophenyl)-4-(4-chlorophenyl)-1-(4-methoxyphenyl)-3-methylazetidin-2-one

The compound obtained in Step 1 above was reacted in the same way as in Step 2 of Reference Example 37 to give the title compound as a pale yellow solid.
$^1$H-NMR ($CDCl_3$) δ: 1.89 (3H, s), 3.75 (3H, s), 4.98 (1H, s), 6.78-6.82 (2H, m), 6.91-6.96 (3H, m), 6.99-7.07 (2H, m), 7.09-7.14 (3H, m), 7.24-7.29 (2H, m).

Step 3: (3S*,4R*)-3-(3-Chlorophenyl)-4-(4-chlorophenyl)-3-methylazetidin-2-one

The compound obtained in Step 2 above was reacted in the same way as in Step 3 of Reference Example 37 to give the title compound as a pale yellow solid.
$^1$H-NMR (DMSO-$d_6$) δ: 1.75 (3H, s), 4.77 (1H, s), 6.92-7.23 (8H, m), 8.67 (1H, s).

Step 4: tert-Butyl (2R*,3S*)-3-(3-Chlorophenyl)-2-(4-chlorophenyl)-3-methyl-4-oxoazetidine-1-carboxylate

The compound obtained in Step 3 above was reacted in the same way as in Step 4 of Reference Example 9 to give the title compound as a pale yellow oil.
$^1$H-NMR ($CDCl_3$) δ: 1.41 (9H, s), 1.85 (3H, s), 4.93 (1H, s), 6.81-6.85 (1H, m), 6.91 (2H, d, J=8.5 Hz), 6.98-7.08 (3H, m), 7.13 (2H, d, J=8.5 Hz).

Step 5: (2S*,3R*)-3-[(tert-Butoxycarbonyl)amino]-2-(3-chlorophenyl)-3-(4-chlorophenyl)-2-methylpropionic acid

The compound obtained in Step 4 above was reacted in the same way as in Step 5 of Reference Example 37 to give the title compound as a red solid.
$^1$H-NMR ($CDCl_3$) δ: 1.34 (9H, s), 1.84 (3H, s), 4.73 (1H, s), 6.30 (1H, s), 6.84-6.87 (1H, m), 6.94-7.07 (5H, m), 7.11-7.16 (2H, m).

Step 6: tert-Butyl (4S*,5R*)-4-(3-Chlorophenyl)-5-(4-chlorophenyl)-4-methyl-2-oxoimidazopyridine-1-carboxylate

The compound obtained in Step 5 above was reacted in the same way as in Step 6 of Reference Example 37 to give the title compound as a pale yellow solid.
$^{1}$H-NMR ($CDCl_3$) δ: 1.27 (9H, d, J=5.4 Hz), 1.89 (3H, s), 4.99 (1H, s), 5.27 (1H, s), 6.83 (2H, d, J=8.5 Hz), 6.89-6.93 (1H, m), 7.00-7.08 (5H, m).

Step 7: (4S*,5R*)-4-(3-Chlorophenyl)-5-(4-chlorophenyl)-4-methylimidazolidin-2-one

The compound obtained in Step 6 above was reacted in the same way as in Step 6 of Example 102 to give the title compound as a colorless solid.
$^{1}$H-NMR (DMSO-$d_6$) δ: 1.70 (3H, s), 4.72 (1H, s), 6.92-7.00 (4H, m), 7.04 (1H, s), 7.07-7.16 (5H, m).

Step 8: (4S*,5R*)-4-(3-Chlorophenyl)-5-(4-chlorophenyl)-4-methylimidazopyridine-2-thione

The compound obtained in Step 7 above was reacted in the same way as in Step 8 of Reference Example 37 to give the title compound as a yellow solid.
$^{1}$H-NMR (DMSO-$d_6$) δ: 1.72 (3H, s), 4.94 (1H, s), 6.84-7.18 (8H, m), 8.78 (1H, s), 8.94 (1H, s).

Step 9: (1R,2S,5R)-2-Isopropyl-5-methylcyclohexyl (4S,5R)-4-(3-Chlorophenyl)-5-(4-chlorophenyl)-4-methyl-2-thioxoimidazopyridine-1-carboxylate

The compound obtained in Step 8 above was reacted in the same way as in Step 6 of Reference Example 38 to give the title compound as a pale yellow solid.
$^{1}$H-NMR ($CDCl_3$) δ: 0.35 (1H, q, J=11.2 Hz), 0.73 (3H, d, J=7.1 Hz), 0.75 (3H, d, J=6.6 Hz), 0.81 (3H, d, J=6.8 Hz), 0.95-1.00 (1H, m), 1.20-1.47 (2H, m), 1.59-1.71 (4H, m), 1.89-1.99 (1H, m), 1.92 (3H, s), 4.57 (1H, td, J=10.8, 4.3 Hz), 5.24 (1H, s), 6.76-6.80 (2H, m), 6.88-6.96 (2H, m), 6.99-7.10 (5H, m).

Step 10: (4S,5R)-4-(3-Chlorophenyl)-5-(4-chlorophenyl)-4-methylimidazopyridine-2-thione

The compound obtained in Step 9 above was reacted in the same way as in Step 7 of Reference Example 38 to give the title compound as a colorless solid.
$^{1}$H-NMR ($CDCl_3$) δ: 1.89 (3H, s), 4.95 (1H, s), 6.29 (1H, s), 6.49 (1H, s), 6.81-6.85 (3H, m), 6.90 (1H, t, J=1.8 Hz), 7.04-7.13 (4H, m).

Step 11: Ethyl (5R,6S)-6-(3-Chlorophenyl)-5-(4-chlorophenyl)-3-isopropyl-6-methyl-5,6-dihydroimidazo[2,1-b][1,3]thiazole-2-carboxylate

The compound obtained in Step 10 above was reacted in the same way as in Step 2 of Example 1 to give the title compound as a colorless solid.
$^{1}$H-NMR ($CDCl_3$) δ: 1.04 (6H, t, J=7.1 Hz), 1.37 (3H, t, J=7.1 Hz), 2.12 (3H, s), 3.35-3.44 (1H, m), 4.34 (2H, q, J=7.2 Hz), 5.57 (1H, s), 7.07-7.17 (8H, m).

Step 12: (5R,6S)-6-(3-Chlorophenyl)-5-(4-chlorophenyl)-3-isopropyl-6-methyl-5,6-dihydroimidazo[2,1-b][1,3]thiazole-2-carboxylic acid

The compound obtained in Step 11 above was reacted in the same way as in Step 3 of Example 1 to give the title compound as a colorless solid.
$^{1}$H-NMR ($CDCl_3$) δ: 0.96 (6H, d, J=7.1 Hz), 2.05 (3H, s), 3.25-3.33 (1H, m), 6.26 (1H, s), 6.61-6.78 (1H, brm), 7.16-7.30 (7H, m).

Reference Example 65

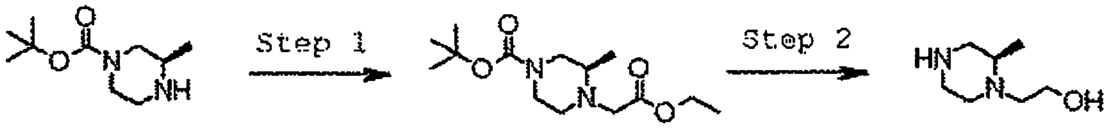


Step 1: tert-Butyl (3R)-4-(2-Ethoxy-2-oxoethyl)-3-methylpiperazine-1-carboxylate

tert-Butyl (3R)-3-methylpiperazine-1-carboxylate was reacted in the same way as in Step 1 of Reference Example 54 to give the title compound as a yellow oil.
$^1$H-NMR ($CDCl_3$) δ: 1.05 (3H, d, J=6.1 Hz), 1.27 (3H, t, J=7.2 Hz), 1.46 (9H, s), 2.55-2.65 (3H, m), 2.79 (1H, dt, J=11.4, 3.2 Hz), 3.04-3.11 (1H, m), 3.37 (2H, dd, J=35.9, 16.6 Hz), 3.83 (2H, d, J=10.7 Hz), 4.18 (2H, q, J=7.2 Hz).

Step 2: 2-[(2R)-2-Methylpiperazin-1-yl]ethanol

The compound obtained in Step 1 above was reacted in the same way as in Example 9. Then, the obtained alcohol form was reacted in the same way as in Step 2 of Example 214 to give the title compound as a pale yellow oil. $^1$H-NMR (DMSO-$d_6$) δ: 1.41 (3H, d, J=6.6 Hz), 3.11-3.19 (1H, m), 3.26-3.39 (3H, m), 3.41-3.59 (5H, m), 3.65-3.68 (1H, m), 3.78-3.85 (3H, m).

Reference Example 66

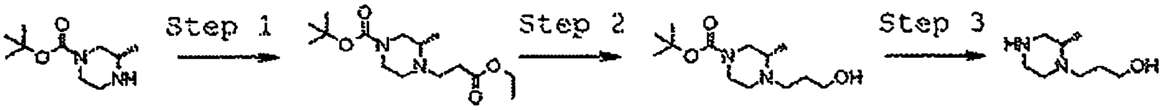


Step 1: (3S)-4-(3-Ethoxy-3-oxopropyl)-3-methylpiperazine-1-carboxylate

Ethyl acrylate (3.25 ml, 30.0 mmol) was added to an ethanol (50 ml) solution of (S)-4-N-(tert-butoxycarbonyl)-2-methylpiperazine (3.00 g, 15.0 mmol) and the resulting mixture was heated to reflux for 5 hours. The reaction mixture was concentrated under reduced pressure. The obtained residue was purified by silica gel column chromatography (hexane → ethyl acetate) to give the title compound (4.64 g, quantitative) as a pale yellow oil.
$^1$H-NMR ($CDCl_3$) δ: 1.04 (3H, d, J=6.1 Hz), 1.26 (3H, t, J=7.2 Hz), 1.45 (9H, s), 2.23-2.29 (1H, m), 2.36-2.50 (3H, m), 2.65-2.76 (3H, m), 2.98-3.15 (2H, m), 3.48-3.88 (2H, m), 4.14 (2H, q, J=7.2 Hz).

Step 2: tert-Butyl (3S)-4-(3-Hydroxypropyl)-3-methylpiperazine-1-carboxylate

The compound obtained in Step 1 above was reacted in the same way as in Example 9 to give the title compound as a pale yellow oil.
$^1$H-NMR ($CDCl_3$) δ: 1.09 (3H, d, J=6.1 Hz), 1.46 (9H, s), 1.79-1.89 (2H, m), 2.13-2.29 (1H, m), 2.38-2.51 (2H, m), 2.78-3.05 (3H, m), 3.12-3.29 (1H, m), 3.64-3.84 (5H, m).

Step 3: 3-[(2S)-2-Methylpiperazin-1-yl]propan-1-ol

The compound obtained in Step 2 above was reacted in the same way as in Reference Example 61 to give the title compound as a pale yellow oil.
$^1$HNMR (DMSO-$d_6$) δ: 1.35 (3H, d, J=6.3 Hz), 1.70-1.86 (2H, m), 2.88-3.80 (12H, m), 8.55-13.06 (3H, m).

Reference Example 67

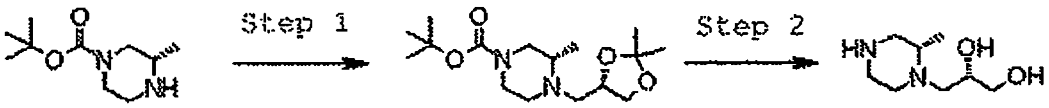

Step 1: tert-Butyl (3S)-4-{[(4S)-2,2-Dimethyl-1,3-dioxolan-4-yl]methyl}-3-methylpiperazine-1-carboxylate

(S)-4-N-(tert-Butoxycarbonyl)-2-methylpiperazine was reacted in the same way as in Step 3 of Example 152 using (4R)-2,2-dimethyl-1,3-dioxolane-4-carbaldehyde instead of a 35% aqueous solution of formalin to give the title compound as a pale yellow oil.
$^1$H-NMR ($CDCl_3$) δ: 1.04 (3H, d, J=6.3 Hz), 1.35 (3H, s), 1.41 (3H, s), 1.45 (9H, s), 2.23-2.34 (1H, m), 2.38-2.52 (2H, m), 2.66-2.84 (2H, m), 2.89 (1H, dt, J=11.6, 3.6 Hz), 3.05-3.16 (1H, m), 3.56-3.64 (1H, m), 3.69-3.83 (2H, m), 4.01-4.10 (1H, m), 4.21-4.29 (1H, m).

Step 2: (2S)-3-[(2S)-2-Methylpiperazin-1-yl]propane-1,2-diol

The compound obtained in Step 1 above was reacted in the same way as in Step 3 of Reference Example 66 to give the title compound as a pale yellow oil.
$^1$HNMR (DMSO-$d_6$, 100°C) δ: 1.40 (3H, d, J=6.6 Hz), 2.95 (1H, dd, J=13.9, 8.8 Hz), 3.20-4.02 (14H, m).

Reference Example 68

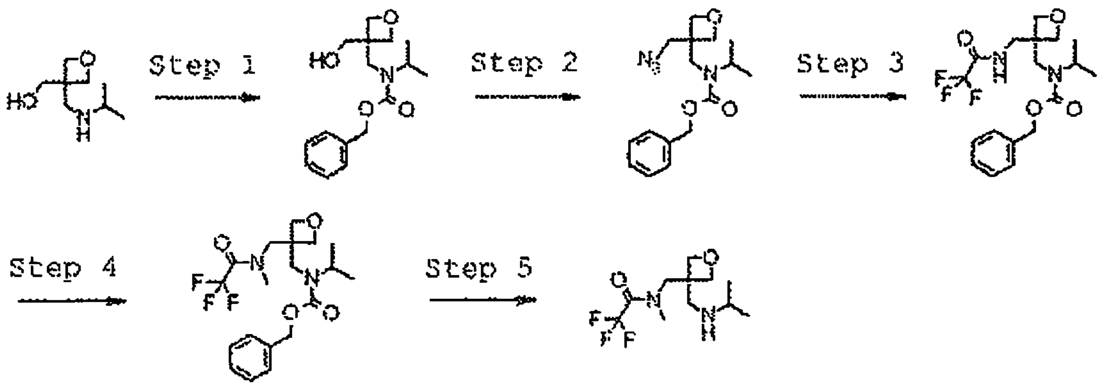


Step 1: Benzyl ([3-(Hydroxymethyl)oxetan-3-yl]methyl}isopropylcarbamate

N-(Benzyloxycarbonyloxy)succinimide (2.30 g, 9.23 mmol) was added to a solution of the compound (1.40 g, 8.79 mmol) obtained in Step 1 of Reference Example 56 in 1,4-dioxane (20 ml)/saturated aqueous sodium bicarbonate solution (20 ml) and the resulting mixture was stirred at room temperature for 20 hours. The mixture was diluted with ethyl acetate, washed with saturated aqueous sodium bicarbonate solution, an aqueous solution of saturated ammonium chloride, and brine, and then dried over anhydrous sodium sulfate. The drying agent was removed by filtration and the solvent was removed by concentration under reduced pressure. The obtained residue was purified by silica gel chromatography (ethyl acetate:hexane = 1:1) to give the title compound (1.75 g, 68%) as a colorless oil.
$^1$H-NMR ($CDCl_3$) δ: 1.19 (6H, d, J=6.8 Hz), 3.62 (2H, br), 3.77-3.83 (3H, m), 4.36 (2H, d, J=5.8 Hz), 4.48 (2H, d, J=6.3 Hz), 5.16 (2H, s), 7.33-7.40 (5H, m).

Step 2: Benzyl {[3-(Azidomethyl)oxetan-3-yl]methyl}isopropylcarbamate

The compound obtained in Step 1 above was reacted in the same way as in Step 2 of Reference Example 63 to give the title compound as a colorless oil.
$^1$H-NMR ($CDCl_3$) δ: 1.23 (6H, d, J=6.8 Hz), 3.33 (2H, s), 3.67 (2H, brs), 3.91-3.98 (1H, m), 4.27 (2H, br), 4.54 (2H, m),

5.11 (2H, s), 7.30-7.40 (5H, m).

Step 3: Benzyl Isopropyl[(3-{[(trifluoroacetyl)amino]methyl}oxetan-3-yl)methyl]carbamate

The compound obtained in Step 2 above was reacted in the same way as in Step 3 of Reference Example 9 using 5% palladium-carbon instead of a Lindlar catalyst and then subjected to the same reaction as in Step 1 of Reference Example 51 to give the title compound as a colorless oil.

$^1$H-NMR ($CDCl_3$) δ: 1.21 (6H, d, J=6.8 Hz), 3.57 (2H, s), 3.65 (2H, d, J=5.9 Hz), 3.81-3.88 (1H, m), 4.37 (2H, d, J=6.3 Hz), 4.46 (2H, d, J=6.3 Hz), 5.16 (2H, s), 7.33-7.39 (5H, m).

Step 4: Benzyl Isopropyl[(3-{[methyl(trifluoroacetyl)amino]methyl}oxetan-3-yl)methyl]-carbamate

The compound obtained in Step 3 above was reacted in the same way as in Step 2 of Reference Example 62 to give the title compound as a colorless oil.
$^1$H-NMR ($CDCl_3$) δ: 1.27 (6H, d, J=6.6 Hz), 3.16 (3H, brs), 3.33 (2H, brs), 3.66 (2H, brs), 3.91-3.98 (1H, m), 4.38 (2H, m), 4.58 (2H, d, J=6.8 Hz), 5.12 (2H, s), 7.32-7.37 (5H, m).
MS (ESI) m/z: 403.

Step 5: 2,2,2-Trifluoro-N-({3-[(isopropylamino)methyl]oxetan-3-yl}methyl)-N-methylacetamide

The compound obtained in Step 4 above was reacted in the same way as in Step 4 of Reference Example 13 to give the title compound as a colorless oil.
$^1$H-NMR ($CDCl_3$) δ: 1.10 (6H, d, J=5.6 Hz), 2.80 (1H, m), 2.89 (2H, s), 3.18 (3H, s), 3.80 (2H, s), 4.42 (2H, d, J=6.1 Hz), 4.60 (2H, d, J=6.1 Hz).

Reference Example 69

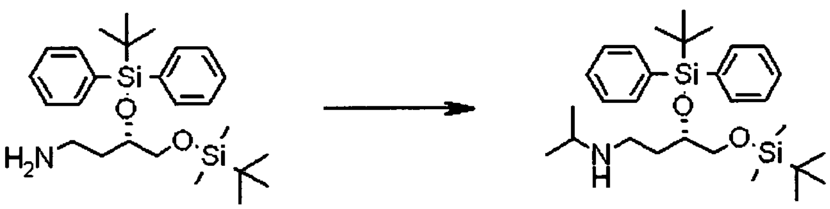

(3S)-4-{[tert-Butyl(dimethyl)silyl]oxy}-3-{[tert-butyl(diphenyl)silyl]oxy}-N-isopropylbutan-1-amine

(3S)-4-{[tert-Butyl(dimethyl)silyl]oxy}-3-{[tert-butyl(diphenyl)silyl]oxy}butan-1-amine was reacted in the same way as in Step 3 of Example 152 using acetone instead of a 35% aqueous solution of formalin to give the title compound as a colorless oil.
$^1$H-NMR ($CDCl_3$) δ: -0.13 (3H, s), -0.09 (3H, s), 0.80 (9H, s), 0.97 (3H, d, J=3.1 Hz), 0.98 (3H, d, J=3.1 Hz), 1.05 (9H, s), 1.61-1.78 (2H, m), 2.55-2.71 (3H, m), 3.39-3.48 (2H, m), 3.79 (1H, t, J=5.1 Hz), 7.34-7.43 (6H, m), 7.68 (4H, d, J=7.6 Hz).

Reference Example 70

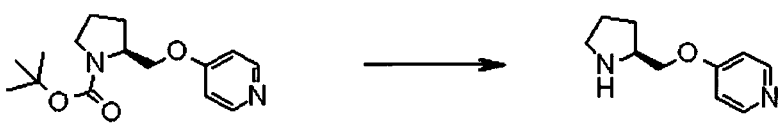

4-[(2S)-Pyrrolidin-2-ylmethoxy]pyridine

tert-Butyl (2S)-2-[(pyridin-4-yloxy)methyl]pyrrolidine-1-carboxylate was reacted in the same way as in Step 6 of Example 102 to give the title compound as a yellow oil.
$^1$H-NMR ($CDCl_3$) δ: 1.56 (1H, m), 1.73-1.89 (3H, m), 1.96 (1H, m), 2.94-3.06 (2H, m), 3.55 (1H, m), 3.89 (1H, dd, J=9.1, 7.0 Hz), 3.95 (1H, dd, J=9.1, 5.0 Hz), 6.81 (2H, dd, J=4.9, 1.7 Hz), 8.42 (2H, dd, J=4.9, 1.7 Hz).

Reference Example 71

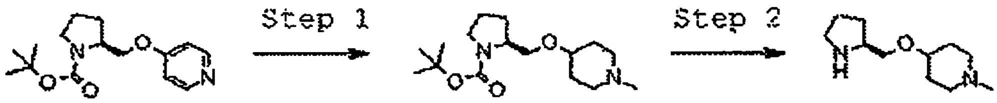


Step 1: tert-Butyl (2S)-2-{[(1-Methylpiperidin-4-yl)oxy]methyl}pyrrolidine-1-carboxylate

Methyl iodide (1.20 ml, 19.2 mmol) was added to an acetone (10 ml) solution of tert-butyl (2S)-2-[(pyridine-4-yloxy)methyl]pyrrolidine-1-carboxylate (1.00 g, 3.59 mmol) and the resulting mixture was stirred at room temperature for 18 hours. The reaction mixture was concentrated under reduced pressure to give 4-{[(2S)-1-(tert-butoxycarbonyl) pyrrolidin-2-yl]methoxy}-1-methylpyridinium iodide as a crude form. This compound was dissolved in ethanol (20 ml), platinum oxide (200 mg) was added, and the resulting mixture was stirred for 20 hours in a hydrogen atmosphere. The catalyst was removed by filtration and the filtrate was concentrated under reduced pressure. Then, the residue was purified by silica gel column chromatography (chloroform:methanol = 10:1) to give the title compound (857 mg, 80%) as a pale yellow oil.
$^1$H-NMR ($CDCl_3$, 60°C) δ: 1.46 (9H, s), 1.85-1.97 (6H, m), 2.39-2.41 (2H, m), 2.65 (3H, s), 3.08-3.11 (4H, m), 3.30 (1H, m), 3.37-3.44 (2H, m), 3.57 (1H, m), 3.68 (1H, m), 3.90 (1H, m).

Step 2: 1-Methyl-4-[(2S)-pyrrolidin-2-ylmethoxy]piperidine

The compound obtained in Step 1 above was reacted in the same way as in Step 6 of Example 102 to give the title compound as a yellow oil.
$^1$H-NMR ($CDCl_3$) δ: 1.39 (1H, m), 1.57-1.66 (2H, m), 1.68-1.90 (6H, m), 2.04-2.13 (2H, m), 2.24 (3H, s), 2.61-2.70 (2H, m), 2.84 (1H, m), 2.98 (1H, m), 3.19-3.33 (3H, m), 3.43 (1H, dd, J=9.0, 4.6 Hz).

Reference Example 72

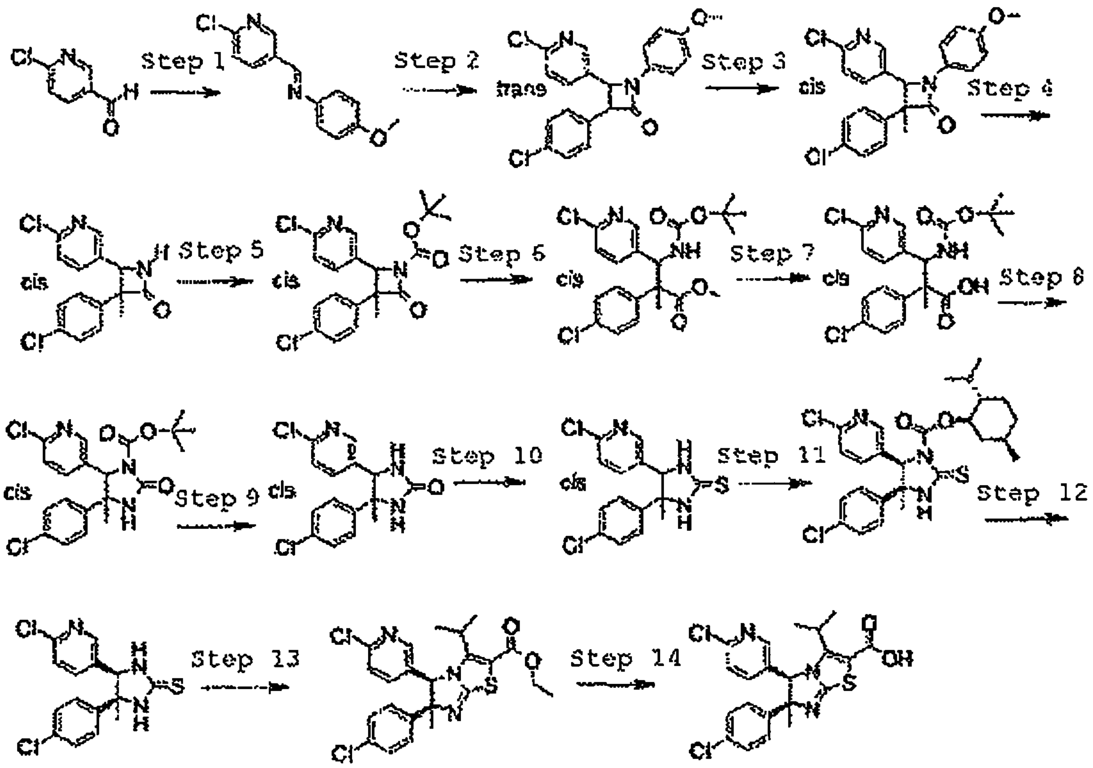


Step 1: [1-(6-Chloropyridin-3-yl)-(E)-methylidene]-(4-methoxyphenyl)amine

p-Anisidine (14.4 g, 117 mmol) was added to an ethanol (150 ml) solution of 6-chloropyridine-3-carbaldehyde (13.2 g, 93.5 mmol) and the resulting mixture was stirred at room temperature for 15 hours. The deposited matter was collected by filtration and then dried under reduced pressure to give the title compound (19.8 g, 69%) as a colorless solid. $^1$H-NMR ($CDCl_3$) δ: 3.85 (3H, s), 6.95 (2H, d, J=9.0 Hz), 7.27 (2H, d, J=8.8 Hz), 7.43 (1H, d, J=8.3 Hz), 8.28 (1H, dd, J=8.3, 2.4 Hz), 8.50 (1H, s), 8.75 (1H, d, J=2.4 Hz).

Step 2: (3R*4S*)-3-(4-Chlorophenyl)-4-(6-chloropyridin-3-yl)-1-(4-methoxyphenyl)-azetidin-2-one

The compound obtained in Step 1 above was reacted in the same way as in Step 1 of Reference Example 37 to give the title compound as a brown oil.
$^1$H-NMR ($CDCl_3$) δ: 3.77 (3H, s), 4.22 (1H, d, J=2.4 Hz), 4.90 (1H, d, J=2.4 Hz), 6.82-6.85 (2H, m), 7.20-7.40 (7H, m), 7.65 (1H, dd, J=8.2, 2.6 Hz), 8.44 (1H, d, J=2.2 Hz).

Step 3: (3S*,4R*)-3-(4-Chlorophenyl)-4-(6-chloropyridin-3-yl)-1-(4-methoxyphenyl)-3-methylazetidin-2-one

The compound obtained in Step 2 above was reacted in the same way as in Step 2 of Reference Example 37 to give the title compound as a pale yellow solid.
$^1$H-NMR ($CDCl_3$) δ: 1.91 (3H, s), 3.76 (3H, s), 5.04 (1H, s), 6.81 (2H, d, J=8.8 Hz), 6.99-7.08 (4H, m), 7.13 (2H, d, J=8.5 Hz), 7.23 (2H, d, J=8.8 Hz), 8.24 (1H, d, J=2.4 Hz).

Step 4: (3S*,4R*)-3-(4-Chlorophenyl)-4-(6-chloropyridin-3-yl)-3-methylazetidin-2-one

The compound obtained in Step 3 above was reacted in the same way as in Step 3 of Reference Example 37 to give the title compound as a pale yellow solid.
$^1$H-NMR ($CDCl_3$) δ: 1.86 (3H, s), 4.77 (1H, s), 6.22 (1H, s), 6.97 (2H, d, J=8.5 Hz), 7.07 (1H, d, J=8.5 Hz), 7.12 (2H, d, J=8.3 Hz), 7.19 (1H, dd, J=8.5, 2.4 Hz), 8.16 (1H, d, J=2.7 Hz).

Step 5: tert-Butyl (2R*,3S*)-3-(4-Chlorophenyl)-2-(6-chloropyridin-3-yl)-3-methyl-4-oxoazetidine-1-carboxylate

The compound obtained in Step 4 above was reacted in the same way as in Step 4 of Reference Example 9 to give the title compound as a pale yellow solid.
$^1$H-NMR ($CDCl_3$) δ: 1.42 (9H, s), 1.87 (3H, s), 4.96 (1H, s), 6.95 (2H, d, J=8.5 Hz), 7.07-7.09 (2H, m), 7.13 (2H, d, J=8.5 Hz), 8.15 (1H, t, J=1.5 Hz).

Step 6: Methyl (2S*,3R*)-3-(tert-Butoxycarbonylamino)-2-(4-chlorophenyl)-3-(6-chloropyridin-3-yl)-2-methylpropionate

Potassium cyanide (56 mg, 0.86 mmol) was added to a methanol (70 ml) solution of the compound (3.5 g, 8.6 mmol) obtained in Step 5 above and the resulting mixture was stirred at room temperature for 2 days. The reaction mixture was concentrated under reduced pressure and then saturated aqueous sodium bicarbonate solution (50 ml) was added, followed by extraction with ethyl acetate (50 ml X 3). The extract was washed with brine (50 ml) and then dried over anhydrous sodium sulfate. The solvent was evaporated under reduced pressure. The obtained residue was purified by silica gel column chromatography (hexane:ethyl acetate = 6:1) to give the title compound (2.6 g, 69%) as a colorless solid.
$^1$H-NMR ($CDCl_3$) δ: 1.37 (9H, s), 1.74 (3H, s), 3.69 (3H, s), 5.08 (1H, d, J=10.2 Hz), 6.98 (2H, d, J=7.8 Hz), 7.13 (1H, d, J=7.8 Hz), 7.23-7.28 (3H, m), 7.94 (1H, d, J=2.2 Hz).

Step 7: (2S*,3R*)-3-(tert-Butoxycarbonylamino)-2-(4-chlorophenyl)-3-(6-chloropyridin-3-yl)-2-methylpropionic acid

The compound obtained in Step 6 above was reacted in the same way as in Step 3 of Example 1 to give the title compound as a pale yellow solid.
$^1$H-NMR ($CDCl_3$) δ: 1.37 (9H, s), 1.84 (3H, s), 5.11 (1H, brs), 5.85 (1H, brs), 7.06-7.28 (6H, m), 8.34 (1H, brs).

Step 8: tert-Butyl (4S*,5R*)-4-(4-Chlorophenyl)-5-(6-chloropyridin-3-yl)-4-methyl-2-oxoimidazolidine-1-carboxylate

The compound obtained in Step 7 above was reacted in the same way as in Step 6 of Reference Example 37 to give the title compound as a pale yellow solid.
$^1$H-NMR ($CDCl_3$) δ: 1.30 (9H, s), 1.89 (3H, s), 5.04 (1H, s), 5.49 (1H, brs), 6.96-7.04 (3H, m), 7.09-7.24 (4H, m), 7.99 (1H, d, J=2.4 Hz).

Step 9: (4S*,5R*)-4-(4-Chlorophenyl)-5-(6-chloropyridin-3-yl)-4-methylimidazolidin-2-one

The compound obtained in Step 8 above was reacted in the same way as in Step 6 of Example 102 to give the title compound as a colorless solid.
$^1$H-NMR ($CDCl_3$) δ: 1.87 (3H, s), 4.82 (1H, s), 4.95 (1H, brs), 5.09 (1H, brs), 6.95 (2H, d, J=8.5 Hz), 7.04 (1H, d, J=8.0 Hz), 7.10-7.16 (3H, m), 8.05 (1H, d, J=2.0 Hz).

Step 10: (4S*,5R*)-4-(4-Chlorophenyl)-5-(6-chloropyridin-3-yl)-4-methylimidazolidine-2-thione

The compound obtained in Step 9 above was reacted in the same way as in Step 8 of Reference Example 37 to give the title compound as a yellow solid. This compound was used in next reaction without being purified.

Step 11: (1R,2S,5R)-2-Isopropyl-5-methylcyclohexyl (4S,5R)-4-(4-Chlorophenyl)-5-(6-chloropyridin-3-yl)-4-methyl-2-thioxoimidazolidine-1-carboxylate

The compound obtained in Step 10 above was reacted in the same way as in Step 6 of Reference Example 38 to give the title compound as a colorless solid.
$^1$H-NMR ($CDCl_3$) δ: 0.36-2.01 (9H, m), 0.73 (3H, d, J=7.1 Hz), 0.78 (3H, d, J=6.8 Hz), 0.91 (3H, d, J=7.6 Hz), 1.93 (3H, s), 4.56-4.65 (1H, m), 5.29 (1H, s), 7.00 (2H, d, J=8.8 Hz), 7.05 (1H, d, J=8.3 Hz), 7.11-7.18 (3H, m), 7.94 (1H, d, J=2.4 Hz).

Step 12: (4S,5R)-4-(4-Chlorophenyl)-5-(6-chloropyridin-3-yl)-4-methylimidazolidine-2-thione

The compound obtained in Step 11 above was reacted in the same way as in Step 7 of Reference Example 38 to give the title compound as a colorless solid.
$^1$H-NMR ($CDCl_3$) δ: 1.91 (3H, s), 5.00 (1H, s), 6.22 (1H, brs), 6.45 (1H, brs), 6.91 (2H, d, J=8.5 Hz), 7.06-7.08 (2H, m), 7.16 (2H, d, J=8.5 Hz), 8.02 (1H, d, J=2.0 Hz).

Step 13: Ethyl (5R,6S)-6-(4-Chlorophenyl)-5-(6-chloropyridin-3-yl)-3-isopropyl-6-methyl-5,6-dihydroimidazo[2,1-b][1,3]thiazole-2-carboxylate

The compound obtained in Step 12 above was reacted in the same way as in Step 2 of Example 1 to give the title compound as a colorless solid.
$^1$H-NMR ($CDCl_3$) δ: 0.90 (3H, d, J=7.1 Hz), 1.03 (3H, d, J=7.1 Hz), 1.33 (3H, t, J=7.1 Hz), 1.83 (3H, s), 3.32-3.43 (1H, m), 4.25 (2H, q, J=7.1 Hz), 5.12 (1H, s), 6.96-7.16 (6H, m), 7.92 (1H, s).

Step 14: (5R,6S)-6-(4-Chlorophenyl)-5-(6-chloropyridin-3-yl)-3-isopropyl-6-methyl-5,6-dihydroimidazo[2,1-b][1,3]thiazole-2-carboxylic acid

The compound obtained in Step 13 above was reacted in the same way as in Step 3 of Example 1 to give the title compound as a white solid.
$^1$H-NMR (DMSO-$d_6$) δ: 0.96-0.98 (9H, m), 2.01-2.08 (1H, m), 6.31 (1H, s), 7.23-7.35 (7H, m).

Reference Example 73

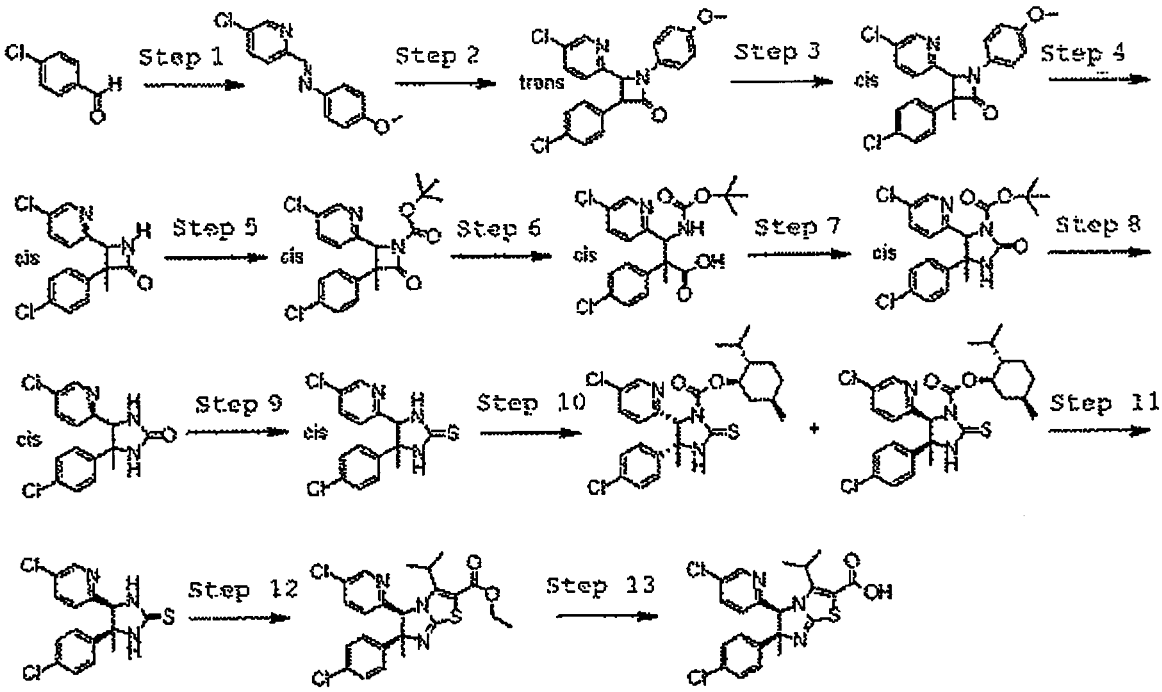


Step 1: N-[(1E)-(5-Chloropyridin-2-yl)methylidene]-4-methoxyaniline

5-Chloropyridine-2-carbaldehyde was reacted in the same way as in Step 1 of Reference Example 72 to give the title compound.

Step 2: (3R*4S*)-4-(5-Bromopyridin-2-yl)-3-(4-chlorophenyl)-1-(4-methoxyphenyl)azetidin-2-one

The compound obtained in Step 1 above was reacted in the same way as in Step 1 of Reference Example 37 to give the title compound as a red solid.
$^1$H-NMR ($CDCl_3$) δ: 3.76 (3H, s), 4.36 (1H, d, J=2.3 Hz), 5.03 (1H, d, J=2.8 Hz), 6.80-6.84 (2H, m), 7.23-7.28 (3H, m), 7.35 (4H, s), 7.84 (1H, dd, J=8.5, 2.5 Hz), 8.71 (1H, d, J=2.3 Hz).

Step 3: (3S*,4R*)-4-(5-Bromopyridin-2-yl)-3-(4-chlorophenyl)-1-(4-methoxyphenyl)-3-methylazetidin-2-one

The compound obtained in Step 2 above was reacted in the same way as in Step 2 of Reference Example 37 to give the title compound as a pale yellow solid.
$^1$H-NMR ($CDCl_3$) δ: 1.89 (3H, s), 3.75 (3H, s), 4.98 (1H, s), 6.78-6.82 (2H, m), 6.91-6.96 (3H, m), 6.99-7.07 (2H, m), 7.09-7.14 (3H, m), 7.24-7.29 (2H, m).

Step 4: (3S*,4R*)-4-(5-Bromopyridin-2-yl)-3-(4-chlorophenyl)-3-methylazetidin-2-one

The compound obtained in Step 3 above was reacted in the same way as in Step 3 of Reference Example 37 to give the title compound as a red solid.
$^1$H-NMR ($CDCl_3$) δ: 1.88 (3H, s), 4.90 (1H, s), 6.18 (1H, brs), 6.86 (1H, d, J=8.3 Hz), 6.99-7.04 (2H, m), 7.05-7.09 (2H, m), 7.54 (1H, dd, J=8.4, 2.3 Hz), 8.51 (1H, d, J=2.2 Hz).

Step 5: tert-Butyl (2R*,3S*)-2-(5-Bromopyridin-2-yl)-3-(4-chlorophenyl)-3-methyl-4-oxoazetidine-1-carboxylate

The compound obtained in Step 4 above was reacted in the same way as in Step 4 of Reference Example 9 to give the title compound as a pale yellow solid.
$^1$H-NMR ($CDCl_3$) δ: 1.43 (9H, s), 1.89 (3H, s), 5.09 (1H, s), 6.77 (1H, d, J=8.3 Hz), 6.99 (2H, d, J=8.5 Hz), 7.07 (2H, d, J=8.5 Hz), 7.56 (1H, dd, J=8.4, 2.3 Hz), 8.49 (1H, d, J=2.2 Hz).

Step 6: (2S*,3R*)-3-[(tert-Butoxycarbonyl)amino]-2-(3-chlorophenyl)-3-(4-chlorophenyl)-2-methylpropionic acid

The compound obtained in Step 5 above was reacted in the same way as in Step 6 of Reference Example 37 to give the title compound as a yellow solid.
$^1$H-NMR ($CDCl_3$) δ: 1.39 (9H, s), 1.72 (3H, s), 5.32 (1H, d, J=10.0 Hz), 5.52 (1H, d, J=10.0 Hz), 7.10-7.18 (4H, m), 7.33 (1H, d, J=8.1 Hz), 7.84 (1H, dd, J=8.3, 2.2 Hz), 8.37-8.39 (1H, m).

Step 7: tert-Butyl (4S*,5R*)-5-(5-Bromopyridin-2-yl)-4-(4-chlorophenyl)-4-methyl-2-oxoimidazopyridine-1-carboxylate

The compound obtained in Step 6 above was reacted in the same way as in Step 6 of Reference Example 37 to give the title compound as a pale yellow solid.
$^1$H-NMR ($CDCl_3$) δ: 1.26 (9H, d, J=2.4 Hz), 2.05 (3H, d, J=2.4 Hz), 5.19 (1H, d, J=2.2 Hz), 5.68 (1H, s), 6.74 (1H, dd, J=8.3, 2.0 Hz), 6.99-7.10 (4H, m), 7.51 (1H, dt, J=8.3, 2.2 Hz), 8.38 (1H, s).

Step 8: (4S*,5R*)-5-(5-Bromopyridin-2-yl)-4-(4-chlorophenyl)-4-methylimidazolidin-2-one

The compound obtained in Step 7 above was reacted in the same way as in Step 6 of Example 102 to give the title compound as a pale yellow solid.
$^1$H-NMR ($CDCl_3$) δ: 1.95 (3H, s), 4.95 (1H, s), 4.96 (1H, s), 5.03 (1H, s), 6.80 (1H, d, J=8.3 Hz), 6.95-7.00 (2H, m), 7.06-7.10 (2H, m), 7.50 (1H, dd, J=8.3, 2.2 Hz), 8.44 (1H, d, J=2.2 Hz).

Step 9: (4S*,5R*)-5-(5-Bromopyridin-2-yl)-4-(4-chlorophenyl)-4-methylimidazopyridine-2-thione

The compound obtained in Step 8 above was reacted in the same way as in Step 8 of Reference Example 37 to give the title compound as a yellow solid.
$^1$H-NMR ($CDCl_3$) δ: 1.98 (3H, s), 5.14 (1H, s), 6.48 (1H, s), 6.63 (1H, s), 6.77 (1H, d, J=8.3 Hz), 6.91-6.95 (2H, m), 7.07-7.11 (2H, m), 7.26 (2H, s), 7.52 (1H, dd, J=8.3, 2.2 Hz), 8.44 (1H, d, J=2.2 Hz).

Step 10: (1R,2S,5R)-2-Isopropyl-5-methylcyclohexyl (4S,5S)-5-(5-Bromopyridin-2-yl)-4-(4-chlorophenyl)-4-methyl-2-thioxoimidazopyridine-1-carboxylate

The compound obtained in Step 9 above was reacted in the same way as in Step 6 of Reference Example 38 to give the title compound as a colorless solid.
$^1$H-NMR ($CDCl_3$) δ: 0.24 (1H, q, J=11.7 Hz), 0.63-0.68 (1H, m), 0.72 (3H, d, J=7.1 Hz), 0.74 (3H, d, J=6.6 Hz), 0.88 (3H, d, J=7.1 Hz), 0.91-1.01 (1H, m), 1.15-1.31 (2H, m), 1.51-1.63 (2H, m), 1.86-1.94 (2H, m), 1.96 (3H, s), 4.55 (1H, td, J=10.9, 4.2 Hz), 5.43 (1H, s), 6.74 (1H, d, J=8.3 Hz), 7.05-7.11 (4H, m), 7.52 (1H, dd, J=8.3, 2.2 Hz), 8.35 (1H, d, J=2.2 Hz), 8.42 (1H, brs).

Step 11: (4S,5S)-5-(5-Bromopyridin-2-yl)-4-(4-chlorophenyl)-4-methylimidazopyridine-2-thione

The compound obtained in Step 10 above was reacted in the same way as in Step 7 of Reference Example 38 to give the title compound as a colorless solid.
$^1$H-NMR ($CDCl_3$) δ: 1.96 (3H, s), 5.13 (1H, s), 6.78 (1H, d, J=8.3 Hz), 6.90-6.95 (2H, m), 7.05-7.09 (2H, m), 7.15 (1H, s), 7.39 (1H, s), 7.50 (1H, dd, J=8.3, 2.2 Hz), 8.41 (1H, d, J=2.2 Hz).

Step 12: Ethyl (5S,6S)-5-(5-Bromopyridin-2-yl)-6-(4-chlorophenyl)-3-isopropyl-6-methyl-5,6-dihydroimidazo[2,1-b][1,3] thiazole-2-carboxylate

The compound obtained in Step 11 above was reacted in the same way as in Step 2 of Example 1 to give the title compound as a pale yellow solid.
$^1$H-NMR ($CDCl_3$) δ: 0.80 (3H, d, J=7.1 Hz), 1.04 (3H, d, J=7.1 Hz), 1.33 (3H, t, J=7.1 Hz), 1.83 (3H, s), 3.41-3.50 (1H, m), 4.25 (2H, q, J=7.2 Hz), 5.31 (1H, s), 6.60 (1H, d, J=8.5 Hz), 7.02-7.06 (2H, m), 7.16-7.20 (2H, m), 7.48 (1H, dd, J=8.4, 2.3 Hz), 8.38 (1H, d, J=1.7 Hz).

Step 13: (5S,6S)-5-(5-Bromopyridin-2-yl)-6-(4-chlorophenyl)-3-isopropyl-6-methyl-5,6-dihydroimidazo[2,1-b][1,3]thiazole-2-carboxylic acid

The compound obtained in Step 12 above was reacted in the same way as in Step 3 of Example 1 to give the title compound as a colorless solid.
$^1$H-NMR ($CDCl_3$) δ: 0.79 (3H, d, J=7.1 Hz), 1.08 (3H, d, J=7.1 Hz), 2.07 (3H, s), 3.58-3.67 (1H, m), 5.58 (1H, s), 6.69 (1H, s), 7.03-7.09 (3H, m), 7.20 (2H, d, J=8.3 Hz), 7.54 (1H, dd, J=8.3, 2.2 Hz), 8.37 (1H, d, J=2.2 Hz).

Reference Example 74

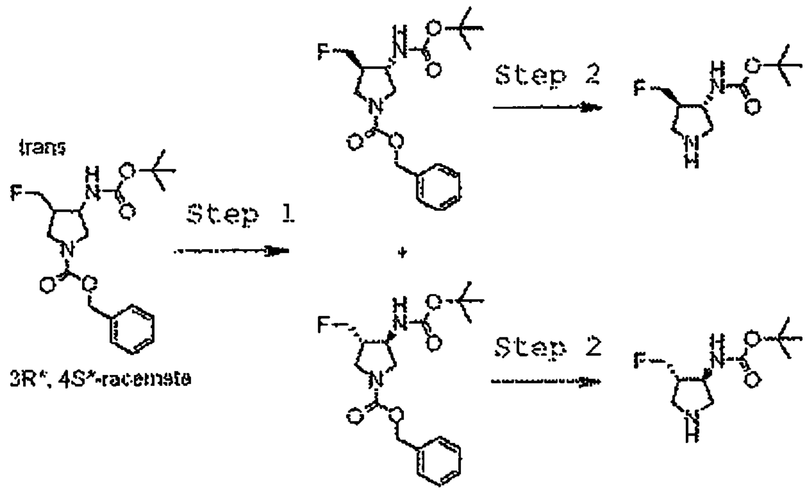


Step 1: Benzyl (3S,4R)-3-[(tert-Butoxycarbonyl)amino]-4-(fluoromethyl)pyrrolidine-1-carboxylate and benzyl (3R,4S)-3-[(tert-Butoxycarbonyl)amino]-4-(fluoromethyl)pyrrolidine-1-carboxylate

Benzyl (3R*,4S*)-3-[(tert-butoxycarbonyl)amino]-4-(fluoromethyl)pyrrolidine-1-carboxylate (1.0 g, 2.83 mmol) was resolved using an optically active column to respectively give the title compounds ((3S,4R) form: 0.47 g, 47%; and (3R,4S) form: 0.46 g, 46%) as a colorless solid.
(3S,4R) form:
$^1$H-NMR ($CDCl_3$) δ: 1.44 (9H, s), 2.34-2.52 (1H, m), 3.17-3.28 (1H, m), 3.34 (1H, dd, J=11.2, 7.6 Hz), 3.68-3.77 (1H, m), 3.81-3.89 (1H, m), 4.01-4.14 (1H, m), 4.35-4.64 (3H, m), 5.13 (2H, s), 7.29-7.38 (5H, m).
(3R, 4S) form:
$^1$H-NMR ($CDCl_3$) δ: 1.44 (9H, s), 2.33-2.53 (1H, m), 3.16-3.28 (1H, m), 3.34 (1H, dd, J=11.5, 7.6 Hz), 3.67-3.78 (1H,

m), 3.80-3.88 (1H, m), 4.02-4.12 (1H, m), 4.34-4.66 (3H, m), 5.13 (2H, s), 7.29-7.39 (5H, m).

Step 2: tert-Butyl [(3S,4R)-4-(fluoromethyl)pyrrolidin-3-yl]carbamate and tert-butyl[(3R,4S)-4-(fluoromethyl)pyrrolidin-3-yl]carbamate

The (3S,4R) and (3R,4S) forms obtained in Step 1 above were reacted in the same way as in Step 4 of Reference Example 13 to respectively give the title compounds. These compounds were separately used in next reaction without being purified.

Reference Example 75

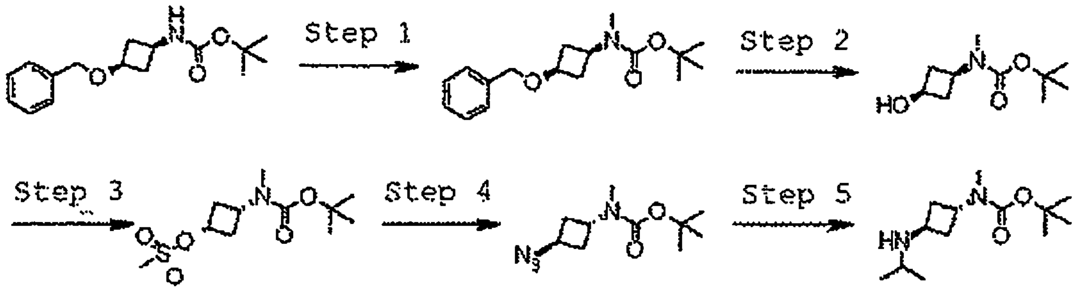


Step 1: tert-Butyl cis-3-(Benzyloxy)cyclobutyl(methyl)carbamate

Lithium aluminium hydride (600 mg, 15.8 mmol) was added in small moieties to a tetrahydrofuran (55 ml) solution of tert-butyl cis-3-(benzyloxy)cyclobutylcarbamate (2.75 g, 9.92 mmol) under ice cooling and the resulting mixture was brought to room temperature and then further heated to reflux for 1 hour. The mixture was left standing to cool and then the reaction mixture was cooled on ice. Water (0.6 ml), a 15% aqueous solution of sodium hydroxide (0.6 ml), and water (1.8 ml) were added and the resulting mixture was stirred at room temperature for 1 hour. Insoluble matter was removed by filtration, then di-tert-butyl dicarbonate (2.50 ml, 10.9 mmol) was added to the filtrate, and the resulting mixture was stirred for 24 hours. The solvent was removed by concentration under reduced pressure. The obtained residue was purified by silica gel column chromatography (ethyl acetate:hexane = 1:9 → 1:4) to give the title compound (2.32 g, 80%) as a colorless oil.
$^1$H-NMR ($CDCl_3$) δ: 1.45 (9H, s), 2.02-2.10 (2H, m), 2.47-2.54 (2H, m), 2.80 (3H, s), 3.70-3.77 (1H, m), 4.02 (1H, m), 4.42 (2H, s), 7.28-7.35 (5H, m).
MS (ESI) m/z: 314 $(M+23)^+$.

Step 2: tert-Butyl cis-3-Hydroxycyclobutyl(methyl)carbamate

The compound obtained in Step 1 above was reacted in the same way as in Step 4 of Reference Example 13 to give the title compound as a colorless oil.
$^1$H-NMR ($CDCl_3$) δ: 1.45 (9H, s), 1.84 (1H, m), 1.98-2.06 (2H, m), 2.54-2.61 (2H, m), 2.81 (3H, s), 3.97-4.05 (1H, m), 4.07 (1H, m).

Step 3: cis-3-[(tert-Butoxycarbonyl)(methyl)amino]cyclobutylmethane sulfonate

Methanesulfonyl chloride (0.42 ml, 5.43 mmol) was added under ice cooling to a dichloromethane (20 ml) solution of the compound (900 mg, 4.47 mmol) obtained in Step 2 above and triethylamine (0.95 ml, 6.81 mmol) and the resulting mixture was stirred for 60 minutes. The reaction mixture was diluted with ethyl acetate, washed with saturated aqueous sodium bicarbonate solution, an aqueous solution of saturated ammonium chloride, and brine, and then dried over anhydrous sodium sulfate. The drying agent was removed by filtration and the solvent was removed by concentration under reduced pressure. The obtained residue was purified by silica gel column chromatography (ethyl acetate:hexane = 1:4) to give the title compound (1.15 g, 92%) as a colorless solid.

$^1$H-NMR ($CDCl_3$) δ: 1.46 (9H, s), 2.37-2.46 (2H, m), 2.61-2.76 (2H, m), 2.82 (3H, s), 3.00 (3H, s), 4.11 (1H, m), 4.67-4.74 (1H, m).

Step 4: tert-Butyl (trans-3-Azidocyclobutyl)methylcarbamate

The compound obtained in Step 3 above was reacted in the same way as in Step 2 of Reference Example 9 to give the title compound as a colorless oil.
$^1$H-NMR ($CDCl_3$) δ: 1.46 (9H, s), 2.26-2.32 (2H, m), 2.45-2.52 (2H, m), 2.81 (3H, s), 4.00-4.04 (1H, m), 4.69 (1H, m).

Step 5: tert-Butyl [trans-3-(Isopropylamino)cyclobutyl]methylcarbamate

The compound obtained in Step 4 above was reacted in the same way as in Step 3 of Reference Example 9 using 5% palladium-carbon instead of a Lindlar catalyst. The obtained amine form was reacted in the same way as in Step 3 of Example 152 using acetone instead of a 37% aqueous solution of formalin to give the title compound as a colorless oil.
$^1$H-NMR ($CDCl_3$) δ: 1.03 (6H, d, J=6.3 Hz), 1.45 (9H, s), 2.02-2.09 (2H, m), 2.31-2.39 (2H, m), 2.76-2.82 (1H, m), 2.84 (3H, s), 3.36-3.41 (1H, m), 4.65 (1H, m).
MS (ESI) m/z: 242.

Reference Example 76

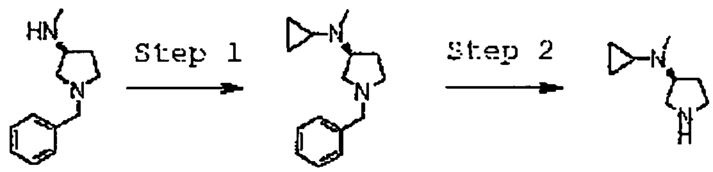


Step 1: (3S)-1-Benzyl-N-cyclopropyl-N-methylpyrrolidin-3-amine

(3S)-1-Benzyl-N-methylpyrrolidin-3-amine was reacted in the same way as in Example 197 to give the title compound as a colorless oil.
$^1$H-NMR ($CDCl_3$) δ: 0.39-0.48 (4H, m), 1.57-1.62 (1H, m), 1.82 (1H, m), 1.94-2.04 (1H, m), 2.29 (3H, s), 2.43-2.52 (2H, m), 2.69 (1H, m), 2.82 (1H, m), 3.21 (1H, m), 3.53 (1H, d, J=12.9 Hz), 3.66 (1H, d, J=12.9 Hz), 7.21-7.33 (5H, m).

Step 2: (3S)-N-Cyclopropyl-N-methylpyrrolidin-3-amine

The compound obtained in Step 1 above was reacted in the same way as in Step 6 of Reference Example 6 to give the title compound as a yellow oil.
$^1$H-NMR ($CDCl_3$) δ: 0.42-0.53 (4H, m), 1.62-1.67 (1H, m), 1.78-1.83 (1H, m), 1.97-2.05 (1H, m), 2.34 (3H, s), 2.91 (1H, dd, J=10.7, 7.8 Hz), 3.01-3.16 (3H, m), 3.22 (1H, dd, J=10.7, 6.7 Hz), 5.16 (1H, s).

Reference Example 77

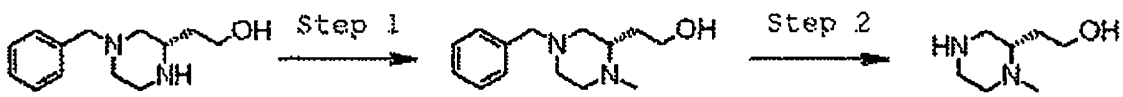

Step 1: 2-[(2S)-4-Benzyl-1-methylpiperazin-2-yl]ethanol

2-[(2S)-4-Benzylpiperazin-2-yl]ethanol was reacted in the same way as in Step 3 of Example 152 to give the title compound as a pale yellow oil.
$^1$H-NMR ($CDCl_3$) δ: 1.45-1.57 (1H, m), 2.02 (1H, m), 2.15-2.52 (5H, m), 2.36 (3H, s), 2.62-2.72 (2H, m), 2.81 (1H, m), 3.48 (2H, s), 3.63-3.71 (1H, m), 3.87 (1H, m), 7.18-7.32 (5H, m).

Step 2: 2-[(2S)-1-Methylpiperazin-2-yl]ethanol

The compound obtained in Step 1 above was reacted in the same way as in Step 6 of Reference Example 6 to give the title compound as a pale yellow oil.
$^1$HNMR (DMSO-$d_6$) δ: 1.69-1.85 (1H, m), 2.10 (1H, brs), 2.81 (3H, s), 3.18-3.75 (10H, m), 10.00 (2H, brs), 11.89 (1H, brs).
MS (ESI) m/z: 145.

Reference Example 78

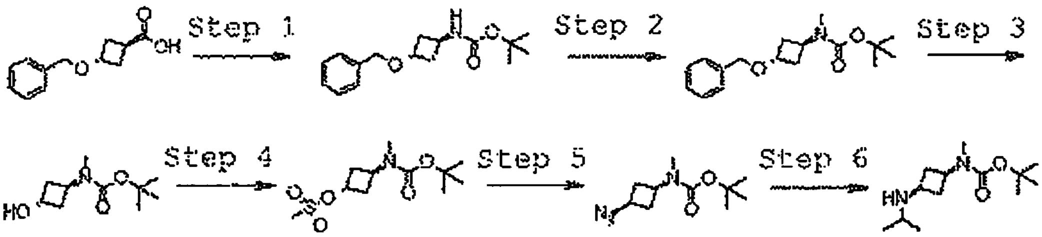


Step 1: tert-Butyl trans-3-(Benzyloxy)cyclobutylcarbamate

trans-3-(Benzyloxy)cyclobutanecarboxylate was reacted in the same way as in Step 3 of Reference Example 4 using tert-butanol instead of benzyl alcohol to give the title compound as a colorless solid.
$^1$H-NMR ($CDCl_3$) δ: 1.44 (9H, s), 2.11-2.14 (2H, m), 2.43 (2H, m), 4.15-4.21 (2H, m), 4.40 (2H, s), 4.66 (1H, m), 7.27-7.36 (5H, m).

Step 2: tert-Butyl trans-3-(Benzyloxy)cyclobutyl(methyl)carbamate

The compound obtained in Step 1 above was reacted in the same way as in Step 2 of Reference Example 62 to give the title compound as a colorless oil.
$^1$H-NMR ($CDCl_3$) δ: 1.45 (9H, s), 2.32-2.36 (4H, m), 2.82 (3H, s), 4.08-4.12 (1H, m), 4.42 (2H, s), 4.73 (1H, m), 7.28-7.35 (5H, m).
MS (ESI) m/z: 314 $(M+23)^+$.

Step 3: tert-Butyl trans-3-Hydroxycyclobutyl(methyl)carbamate

The compound obtained in Step 2 above was reacted in the same way as in Step 4 of Reference Example 13 to give the title compound as a colorless solid.
$^1$H-NMR ($CDCl_3$) δ: 1.45 (9H, s), 1.96 (1H, m), 2.17-2.24 (2H, m), 2.38-2.45 (2H, m), 2.81 (3H, s), 4.40 (1H, m), 4.78 (1H, m).

Step 4: trans-3-[(tert-Butoxycarbonyl)(methyl)amino]cyclobutylmethane sulfamate

The compound obtained in Step 3 above was reacted in the same way as in Step 3 of Reference Example 75 to give the title compound as a colorless oil.
$^1$H-NMR ($CDCl_3$) δ: 1.46 (9H, s), 2.55-2.68 (4H, m), 2.82 (3H, s), 3.01 (3H, s), 4.76 (1H, m), 5.09-5.12 (1H, m).

Step 5: tert-Butyl (cis-3-Azidocyclobutyl)methylcarbamate

The compound obtained in Step 4 above was reacted in the same way as in Step 2 of Reference Example 9 to give the title compound as a colorless oil.
$^1$H-NMR ($CDCl_3$) δ: 1.46 (9H, s), 2.11-2.17 (2H, m), 2.52-2.59 (2H, m), 2.81 (3H, s), 3.51-3.60 (1H, m), 4.21 (1H, m).

Step 6: tert-Butyl [cis-3-(Isopropylamino)cyclobutyl]methylcarbamate

The compound obtained in Step 5 above was reacted in the same way as in Step 3 of Reference Example 9 using 5% palladium-carbon instead of a Lindlar catalyst. The obtained amine form was reacted in the same way as in Step 3 of Example 152 using acetone instead of a 37% aqueous solution of formalin to give the title compound as a colorless oil.
$^1$H-NMR ($CDCl_3$) δ: 1.03 (6H, d, J=6.3 Hz), 1.45 (9H, s), 1.68-1.77 (2H, m), 2.46-2.54 (2H, m), 2.79 (3H, s), 2.80-2.87 (1H, m), 2.93-3.00 (1H, m), 4.06 (1H, m).
MS (ESI) m/z: 242.

Reference Example 79

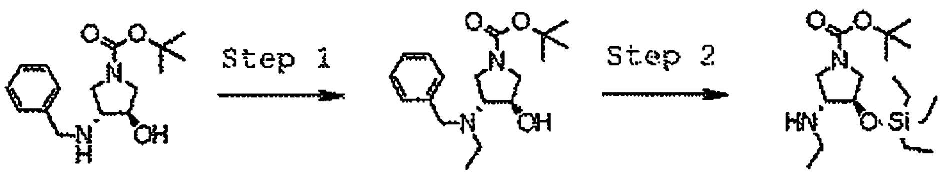


Step 1: tert-Butyl (3R,4R)-3-[Benzyl(ethyl)amino]-4-hydroxypyrrolidine-1-carboxylate

tert-Butyl (3R,4R)-3-(benzylamino)-4-hydroxypyrrolidine-1-carboxylate was reacted in the same way as in Step 3 of Example 152 using acetaldehyde instead of a 37% aqueous solution of formalin to give the title compound as a pale yellow oil.
$^1$H-NMR ($CDCl_3$) δ: 1.07 (3H, t, J=7.1 Hz), 1.45 (9H, s), 1.95 (1H, br), 2.55-2.70 (2H, m), 3.08-3.26 (2H, m), 3.52-3.81 (4H, m), 4.12 (1H, m), 7.25-7.34 (5H, m).
MS (ESI) m/z: 321.

Step 2: tert-Butyl (3R,4R)-3-(Ethylamino)-4-[(triethylsilyl)oxy]pyrrolidine-1-carboxylate

The compound obtained in Step 1 above was reacted in the same way as in Step 6 of Reference Example 6 using 5% palladium-carbon instead of 20% palladium hydroxide-carbon and then subjected to the same reaction as in Step 2 of Reference Example 56 using triethylsilyl chloride instead of tert-butyldimethylsilyl chloride to give the title compound as a colorless oil.
$^1$H-NMR ($CDCl_3$) δ: 0.57-0.63 (6H, m), 0.93-0.97 (9H, m), 1.10 (3H, t, J=7.1 Hz), 1.46 (9H, s), 2.63-2.69 (2H, m), 3.07-3.22 (3H, m), 3.51-3.66 (2H, m), 4.03 (1H, brs).

Reference Example 80

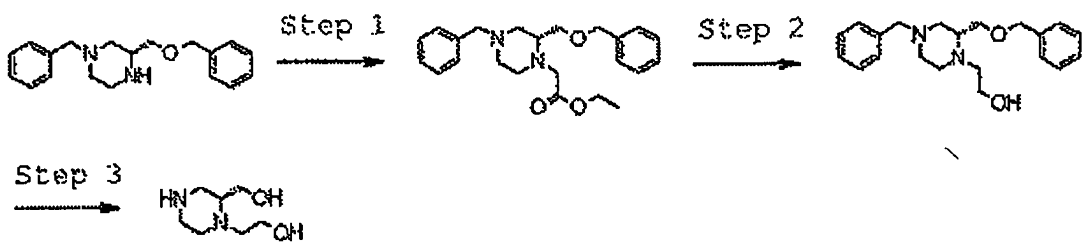


Step 1: Ethyl {(2R)-4-Benzyl-2-[(benzyloxy)methyl]piperazin-1-yl}acetate

(3R)-1-Benzyl-3-[(benzyloxy)methyl]piperazine was reacted in the same way as in Step 1 of Reference Example 54 to give the title compound as a pale yellow oil.
$^{1}$H-NMR ($CDCl_3$) δ: 1.18 (3H, t, J=7.1 Hz), 2.12 (1H, t, J=9.8 Hz), 2.24-2.30 (1H, m), 2.59-2.71 (2H, m), 2.74-2.82 (2H, m), 2.93-3.03 (1H, m), 3.37-3.55 (6H, m), 4.07 (2H, q, J=7.1 Hz), 4.43 (2H, dd, J=14.5, 12.1 Hz), 7.20-7.32 (10H, m).

Step 2: 2-{(2R)-4-Benzyl-2-[(benzyloxy)methyl]piperazin-1-yl}ethanol

The compound obtained in Step 1 above was reacted in the same way as in Example 9 to give the title compound as a pale yellow oil.
$^{1}$H-NMR ($CDCl_3$, 60°C) δ: 1.45 (1H, brs), 2.27-3.07 (9H, m), 3.40-3.73 (6H, m), 4.46 (2H, s), 7.05-7.48 (10H, m).

Step 3: 2-[(2R)-2-(Hydroxymethyl)piperazin-1-yl]ethanol

The compound obtained in Step 2 above was reacted in the same way as in Step 6 of Reference Example 6 to give the title compound as a yellow oil.
$^{1}$HNMR (DMSO-$d_6$, 100°C) δ: 3.26 (1H, dt, J=14.0, 5.1 Hz), 3.35 (1H, dd, J=14.0, 10.3 Hz), 3.42-3.56 (4H, m), 3.57-3.65 (1H, m), 3.71 (1H, dt, J=14.0, 3.4 Hz), 3.77-3.91 (6H, m).
MS (ESI) m/z: 131.

Reference Example 81

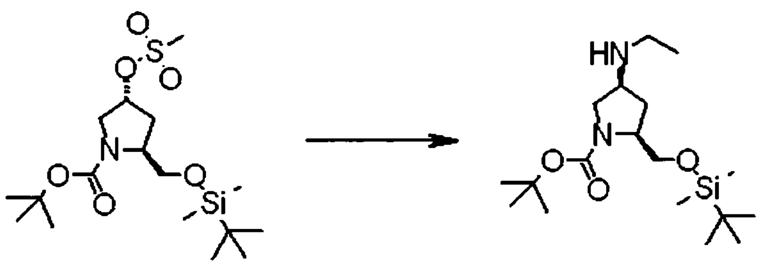

tert-Butyl (2S,4S)-2-({[tert-Butyl(dimethyl)silyl]oxy}methyl)-4-(ethylamino)pyrrolidine-1-carboxylate

A 70% aqueous solution of ethylamine (10 ml) was added to an 2-propanol (50 ml) solution of tert-butyl (2S, 4R)-2-({[tert-butyl(dimethyl)silyl]oxy}methyl)-4-[(methylsulfonyl)oxy]pyrrolidine-1-carboxylate (4.50 g, 10.9 mmol) and the resulting mixture was heated at 60°C for 22 hours. The reaction mixture solution was concentrated under reduced pressure. Then, the residue was neutralized by the addition of saturated aqueous sodium bicarbonate solution, followed by extraction with chloroform. The organic layer was washed with brine and then dried over anhydrous sodium sulfate. The solvent was evaporated under reduced pressure. The obtained residue was purified by silica gel column chromatography (chloroform:methanol = 95:5) to give the title compound (2.20 g, 56%) as a colorless oil.
$^{1}$H-NMR ($CDCl_3$, 60°C) δ: 0.05 (6H, s), 0.89 (9H, s), 1.10 (3H, t, J=7.0 Hz), 1.45 (9H, s), 1.78 (1H, m), 2.24 (1H, m),

2.63-2.65 (2H, m), 2.96 (1H, m), 3.23 (1H, m), 3.73-3.81 (4H, m).

Reference Example 82

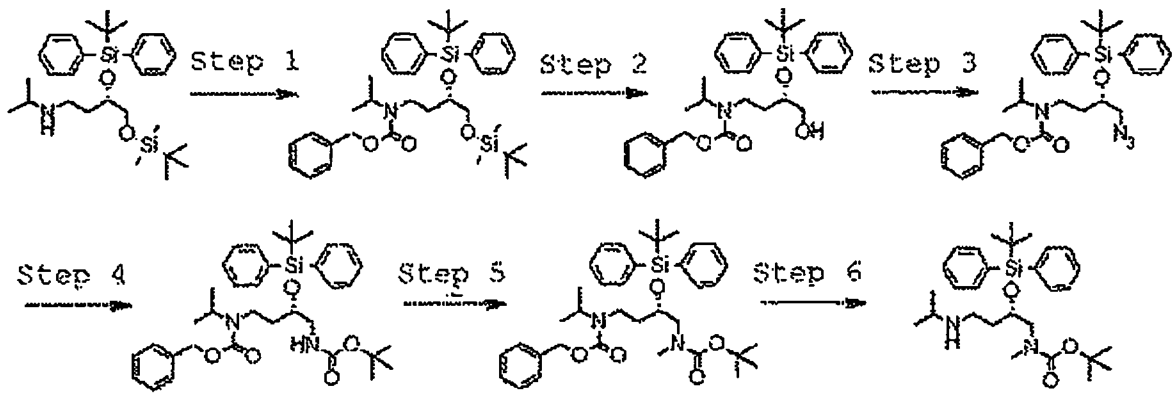


Step 1: Benzyl [(3S)-4-{[tert-Butyl(dimethyl)silyl]oxy}-3-{[tert-butyl(diphenyl)silyl]oxy}butyl]isopropylcarbamate

(3S)-4-{[tert-Butyl(dimethyl)silyl]oxy]-3-{[tert-butyl(diphenyl)silyl]oxy}-N-isopropylbutan-1-amine was reacted in the same way as in Step 1 of Reference Example 68 to give the title compound as a colorless oil.
$^1$H-NMR ($CDCl_3$) δ: -0.18 (3H, s), -0.13 (3H, s), 0.78 (9H, s), 1.04 (9H, s), 1.06 (6H, d, J=7.2 Hz), 1.77-1.85 (2H, m), 3.09-3.24 (2H, m), 3.39 (2H, m), 3.71 (1H, m), 4.11-4.25 (1H, m), 5.10 (2H, s), 7.24-7.66 (15H, m).

Step 2: Benzyl [(3S)-3-{[tert-Butyl(diphenyl)silyl]oxy}-4-hydroxybutyl]isopropylcarbamate

The compound (2.90 g, 4.57 mmol) obtained in Step 1 above was dissolved in methanol (60 ml), p-toluenesulfonic acid monohydrate (1.00 g, 5.26 mmol) was added, and the resulting mixture was stirred at room temperature for 2 hours. The reaction mixture was diluted with saturated aqueous sodium bicarbonate solution, followed by extraction with ethyl acetate. The extract was washed with water and brine and then dried over anhydrous sodium sulfate. The drying agent was removed by filtration and the solvent was removed by concentration under reduced pressure. The obtained residue was purified by silica gel column chromatography (ethyl acetate:hexane = 1:4) to give the title compound (2.20 g, 93%) as a colorless oil.
$^1$H-NMR ($CDCl_3$) δ: 1.02 (6H, d, J=6.9 Hz), 1.07 (9H, s), 1.24-1.28 (2H, m), 2.98-4.25 (6H, m), 5.07 (2H, s), 7.30-7.68 (15H, m).

Step 3: Benzyl [(3S)-4-Azido-3-{[tert-butyl(diphenyl)silyl]oxy}butyl]isopropylcarbamate

The compound obtained in Step 2 above was reacted in the same way as in Step 2 of Reference Example 68 to give the title compound as a colorless oil.
$^1$H-NMR ($CDCl_3$) δ: 1.01 (6H, d, J=6.8 Hz), 1.07 (9H, s), 1.67-1.81 (2H, m), 2.90-3.25 (4H, m), 3.80 (2H, m), 5.07 (2H, s), 7.31-7.52 (11H, m), 7.67-7.73 (4H, m).

Step 4: Benzyl [(3S)-4-[(tert-Butoxycarbonyl)amino]-3-{[tert-butyl(diphenyl)silyl]oxy}butyl]-isopropylcarbamate

The compound obtained in Step 3 above was reacted in the same way as in Step 3 of Reference Example 9. Then, the obtained amine form was reacted in the same way as in Step 4 of Reference Example 9 to give the title compound as a colorless oil.
$^1$H-NMR ($CDCl_3$) δ: 1.00 (6H, d, J=6.8 Hz), 1.05 (9H, s), 1.39 (9H, s), 1.71 (2H, m), 3.09 (4H, m), 3.79-4.50 (3H, m), 5.08 (2H, s), 7.30-7.64 (15H, m).
MS (ESI) m/z: 641 $(M+23)^+$.

Step 5: Benzyl [(3S)-4-[(tert-Butoxycarbonyl)(methyl)amino]-3-{[tert-butyl(diphenyl)silyl]oxy}butyl]isopropylcarbamate

The compound obtained in Step 4 above was reacted in the same way as in Step 2 of Reference Example 62 to give the title compound as a colorless oil.
$^1$H-NMR ($CDCl_3$) δ: 0.98 (6H, brs), 1.05 (9H, s), 1.40 (9H, s), 1.62 (2H, m), 2.54 (3H, brs), 2.88-4.17 (6H, m), 5.06 (2H, s), 7.24-7.65 (15H, m).
MS (ESI) m/z: 655 $(M+23)^+$.

Step 6: tert-Butyl [(2S)-2-{[tert-Butyl(diphenyl)silyl]oxy}-4-(isopropylamino)butyl]methylcarbamate

The compound obtained in Step 5 above was reacted in the same way as in Step 4 of Reference Example 13 to give the title compound as a colorless oil.
$^1$H-NMR ($CDCl_3$) δ: 1.06 (9H, s), 1.39 (9H, s), 1.41 (6H, d, J=6.8 Hz), 1.71 (1H, m), 2.25 (1H, m), 2.44 (3H, s), 2.66 (1H, m), 2.97 (1H, m), 3.13 (2H, m), 3.58 (1H, m), 3.90 (1H, m), 7.36-7.65 (10H, m).
MS (ESI) m/z: 499.

Reference Example 83

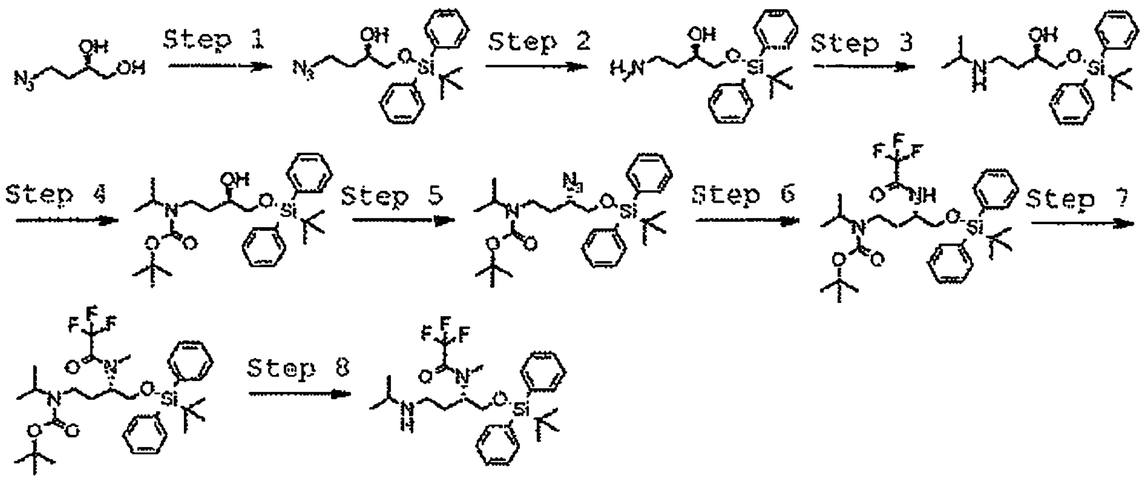


Step 1: (2R)-4-Azido-1-{[tert-butyl(diphenyl)silyl]oxy}butan-2-ol

tert-Butyldiphenylsilyl chloride (4.86 ml, 19.0 mmol) was added to a dichloromethane (23 ml) solution of (2R)-4-azidobutane-1,2-diol (2.30 g, 17.5 mmol), triethylamine (2.65 ml, 19.0 mmol), and 4-dimethylaminopyridine (85 mg) and the resulting mixture was stirred at room temperature for 75 hours. The reaction mixture was diluted with ethyl acetate, washed with saturated aqueous ammonium chloride solution, saturated aqueous sodium bicarbonate solution, and brine, and then dried over anhydrous sodium sulfate. The solvent was evaporated under reduced pressure. The obtained residue was purified by silica gel column chromatography (ethyl acetate:hexane = 1:10) to give the title compound (6.45 g, quantitative) as a colorless oil.
$^1$H-NMR ($CDCl_3$) δ: 1.07 (9H, s), 1.62-1.71 (2H, m), 2.49 (1H, d, J=3.9 Hz), 3.44 (2H, t, J=6. Hz), 3.49-3.53 (1H, m), 3.65-3.68 (1H, m), 3.82-3.86 (1H, m), 7.38-7.47 (6H, m), 7.64-7.67 (4H, m).

Step 2: (2R)-4-Amino-1-{[tert-butyl(diphenyl)silyl]oxy}butan-2-ol

The compound obtained in Step 1 above was reacted in the same way as in Step 3 of Reference Example 9 using 5% palladium-carbon instead of a Lindlar catalyst to give the title compound as a brown oil.
MS (ESI) m/z: 344.

Step 3: (2R)-4-Amino-1-{[tert-butyl(diphenyl)silyl]oxy}butan-2-ol

The compound obtained in Step 2 above was reacted in the same way as in Step 3 of Example 152 using acetone instead of a 37% aqueous solution of formalin to give the title compound as a brown oil.
MS (ESI) m/z: 386.

Step 4: tert-Butyl [(3R)-4-{[tert-Butyl(diphenyl)silyl]oxy}-3-hydroxybutyl]isopropylcarbamate

The compound obtained in Step 3 above was reacted in the same way as in Step 4 of Reference Example 9 to give the title compound as a colorless oil.
$^1$H-NMR ($CDCl_3$) δ: 1.06 (9H, s), 1.13 (6H, brs), 1.44 (9H, s), 1.62 (1H, m), 3.13 (2H, m), 3.61 (2H, brs), 3.69 (2H, brs), 4.10 (1H, m), 7.36-7.44 (6H, m), 7.64-7.67 (4H, m). MS (ESI) m/z: 508 $(M+23)^+$.

Step 5: tert-Butyl [(3S)-3-Azido-4-{[tert-butyl(diphenyl)silyl]oxy}butyl]isopropylcarbamate

The compound obtained in Step 4 above was reacted in the same way as in Step 2 of Reference Example 63 to give the title compound as a colorless oil.
$^1$H-NMR ($CDCl_3$) δ: 1.07 (9H, s), 1.10 (6H, d, J=6.8 Hz), 1.43 (9H, s), 1.67 (1H, br), 3.13 (2H, br), 3.41 (2H, br), 3.63-3.74 (3H, m), 7.38-7.46 (6H, m), 7.66-7.69 (4H, m).

Step 6: tert-Butyl {(3S)-4-{[tert-Butyl(diphenyl)silyl]oxy}-3-[(trifluoroacetyl)amino]butyl}-isopropylcarbamate

The compound obtained in Step 5 above was reacted in the same way as in Step 3 of Reference Example 9 using 5% palladium-carbon instead of a Lindlar catalyst and then subjected to the same reaction as in Step 1 of Reference Example 51 to give the title compound as a colorless oil.
$^1$H-NMR ($CDCl_3$) δ: 1.06 (9H, s), 1.08 (3H, d, J=5.6 Hz), 1.10 (3H, d, J=5.6 Hz), 1.44 (9H, s), 1.90 (1H, br), 1.92-1.98 (1H, m), 3.06-3.12 (2H, m), 3.70-3.72 (2H, m), 3.97-4.01 (2H, m), 7.37-7.64 (10H, m).
MS (ESI) m/z: 603 $(M+23)^+$.

Step 7: tert-Butyl {(3S)-4-{[tert-Butyl(diphenyl)silyl]oxy}-3-[methyl(trifluoroacetyl)amino]-butyl}isopropylcarbamate

The compound obtained in Step 6 above was reacted in the same way as in Step 2 of Reference Example 62 to give the title compound as a colorless oil.
$^1$H-NMR ($CDCl_3$) δ: 1.04 (9H, s), 1.07 (6H, d, J=5.9 Hz), 1.43 (9H, s), 1.76 (1H, m), 2.97 (2H, m), 3.03 (3H, s), 3.67 (2H, d, J=6.6 Hz), 4.05-4.63 (3H, m), 7.38-7.64 (10H, m).
MS (ESI) m/z: 617 $(M+23)^+$.

Step 8: N-[(1S)-1-({[tert-Butyl(diphenyl)silyl]oxy}methyl)-3-(isopropylamino)propyl]-2,2,2-trifluoro-N-methylacetamide

The compound obtained in Step 7 above was reacted in the same way as in Step 4 of Reference Example 9 to give the title compound as a colorless oil.
$^1$H-NMR ($CDCl_3$) δ: 1.02 (9H, s), 1.14 (6H, d, J=7.1 Hz), 1.74-1.79 (2H, m), 2.59-2.71 (2H, m), 2.83-2.86 (2H, m), 2.98 (3H, s), 3.67-3.71 (2H, m), 4.62-4.66 (1H, m), 7.37-7.62 (10H, m).
MS (ESI) m/z: 495.

Reference Example 84

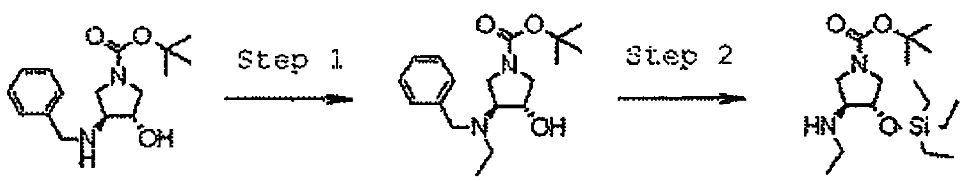

Step 1: tert-Butyl (3S,4S)-3-[Benzyl(ethyl)amino]-4-hydroxypyrrolidine-1-carboxylate

tert-Butyl (3S,4S)-3-(benzylamino)-4-hydroxypyrrolidine-1-carboxylate was reacted in the same way as in Step 3 of Example 152 using acetaldehyde instead of a 37% aqueous solution of formalin to give the title compound as a pale yellow oil.
$^1$H-NMR ($CDCl_3$) δ: 1.07 (3H, t, J=7.1 Hz), 1.45 (9H, s), 1.95 (1H, m), 2.56-2.71 (2H, m), 3.08-3.25 (2H, m), 3.52-3.80 (4H, m), 4.13 (1H, m), 7.23-7.34 (5H, m).
MS (ESI) m/z: 321.

Step 2: tert-Butyl (3S,4S)-3-(Ethylamino)-4-[(triethylsilyl)oxy]pyrrolidine-1-carboxylate

The compound obtained in Step 1 above was reacted in the same way as in Step 6 of Reference Example 6 using 5% palladium-carbon instead of 20% palladium hydroxide-carbon and then subjected to the same reaction as in Step 2 of Reference Example 56 using triethylsilyl chloride instead of tert-butyldimethylsilyl chloride to give the title compound as a colorless oil.
$^1$H-NMR ($CDCl_3$) δ: 0.56-0.62 (6H, m), 0.92-0.98 (9H, m), 1.11 (3H, t, J=7.1 Hz), 1.47 (9H, s), 2.63-2.68 (2H, m), 3.07-3.21 (3H, m), 3.51-3.67 (2H, m), 4.01 (1H, brs).

Reference Example 85

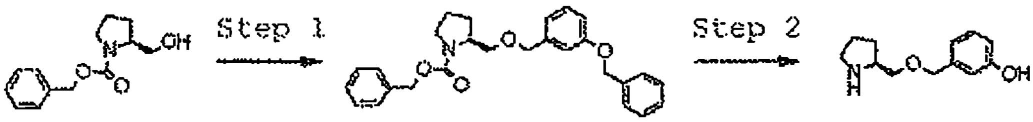


Step 1: Benzyl (2S)-2-{[3-(Benzyloxy)phenoxy]methyl}pyrrolidine-1-carboxylate

Triphenylphosphine (4.01 g, 15.3 mmol), 3-(benzyloxy)phenol (2.81 g, 14.0 mmol), and diisopropyl azodicarboxylate (3.17 ml, 15.0 mmol) were added to a toluene (50 ml) solution of benzyl (2S)-2-(hydroxymethyl)pyrrolidine-1-carboxylate (3.00 g, 12.8 mmol) under ice cooling and the resulting mixture was stirred for 4 days while gradually brought to room temperature. The reaction mixture was concentrated under reduced pressure. Then, a hexane:diethyl ether (1:1) mixed solvent was added to the obtained residue and the resulting mixture was stirred. Then, the deposited insoluble matter was removed by filtration. The obtained filtrate was washed with 1 N aqueous sodium hydroxide solution and brine. Then, the organic layer was dried over anhydrous magnesium sulfate. The solvent was evaporated under reduced pressure. The obtained residue was purified by silica gel column chromatography (hexane → hexane:ethyl acetate = 2:1) to give the title compound (2.80 g, 53%) as a pale yellow oil.
$^1$H-NMR ($CDCl_3$) δ: 1.80-2.14 (4H, m), 3.36-3.56 (2H, m), 3.69-4.01 (1H, m), 4.02-4.28 (2H, m), 5.03 (2H, d, J=7.8 Hz), 5.08-5.20 (2H, m), 6.39-6.63 (3H, m), 7.09-7.18 (1H, m), 7.30-7.43 (10H, m).
MS (ESI) m/z: 418.

Step 2: 3-[(2S)-Pyrrolidin-2-ylmethoxy]phenol

The compound obtained in Step 1 above was reacted in the same way as in Step 4 of Reference Example 13 to give the title compound as a slightly purple solid.
$^1$HNMR (DMSO-$d_6$) δ: 1.65-1.77 (1H, m), 1.82-2.01 (2H, m), 2.05-2.13 (1H, m), 3.11-3.24 (2H, m), 3.84 (1H, dq, J=16.1, 3.9 Hz), 4.06 (1H, t, J=9.4 Hz), 4.15 (1H, dd, J=10.4, 3.9 Hz), 6.35-6.45 (3H, m), 7.07 (1H, t, J=7.9 Hz), 9.32 (1H, brs), 9.54 (1H, s).

Reference Example 86

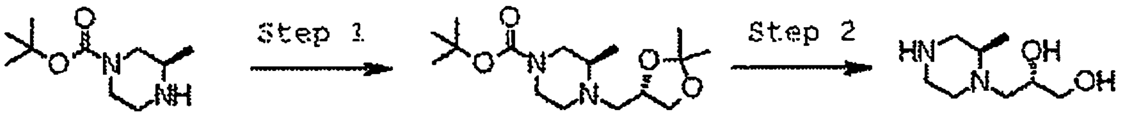


Step 1: tert-Butyl (3R)-4-{[(4S)-2,2-Dimethyl-1,3-dioxolan-4-yl]methyl}-3-methylpiperazine-1-carboxylate

tert-Butyl (3R)-3-methylpiperazine-1-carboxylate was reacted in the same way as in Step 3 of Example 152 using (4R)-2,2-dimethyl-1,3-dioxolane-4-carbaldehyde instead of a 37% aqueous solution of formalin to give the title compound as a colorless solid.
$^1$H-NMR ($CDCl_3$) δ: 1. 03 (3H, d, J=6.3 Hz), 1.37 (4H, d, J=11.0 Hz), 1.41 (4H, d, J=11.0 Hz), 1.46 (9H, s), 2.38 (2H, m), 2.46 (1H, m), 2.84 (2H, m), 3.22 (1H, s), 3.61-3.64 (2H, m), 4.06 (1H, dd, J=7.9, 6.5 Hz), 4.21-4.28 (1H, m).

Step 2: (2S)-3-[(2R)-2-Methylpiperazin-1-yl]propane-1,2-diol

The compound obtained in Step 1 above was reacted in the same way as in Step 3 of Reference Example 66 to give the title compound as a pale yellow solid.
MS (ESI) m/z: 175.

Reference Example 87

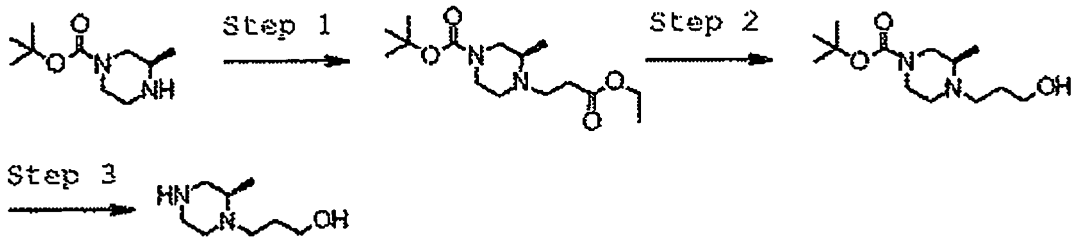


Step 1: tert-Butyl (3R)-4-(3-Ethoxy-3-oxopropyl)-3-methylpiperazine-1-carboxylate

tert-Butyl (3R)-3-methylpiperazine-1-carboxylate was reacted in the same way as in Step 1 of Reference Example 66 to give the title compound as a colorless solid.
$^1$H-NMR ($CDCl_3$) δ: 1.04 (3H, d, J=6.3 Hz), 1.26 (3H, t, J=7.1 Hz), 1.45 (9H, s), 2.23-2.29 (1H, m), 2.37-2.40 (1H, m), 2.44 (2H, t, J=7.4 Hz), 2.65-2.76 (3H, m), 3.02-3.10 (2H, m), 3.72 (2H, d, J=12.9 Hz), 4.14 (2H, q, J=7.2 Hz).

Step 2: tert-Butyl (3R)-4-(3-Hydroxypropyl)-3-methylpiperazine-1-carboxylate

The compound obtained in Step 1 above was reacted in the same way as in Example 9 to give the title compound as a colorless oil.
$^1$H-NMR ($CDCl_3$) δ: 1.09 (3H, d, J=6.3 Hz), 1.46 (9H, s), 1.59-1.63 (1H, m), 1.79-1.89 (1H, m), 2.21 (1H, brs), 2.40-2.43 (2H, m), 2.93-2.96 (3H, m), 3.20 (1H, brs), 3.64-3.77 (2H, m), 3.80 (2H, dd, J=6.5, 4.0 Hz), 5.01 (1H, brs).

Step 3: 3-[(2R)-2-Methylpiperazin-1-yl]propan-1-ol

The compound obtained in Step 2 above was reacted in the same way as in Step 2 of Example 214 to give the title compound as a colorless solid.
MS (ESI) m/z: 158.

Reference Example 88

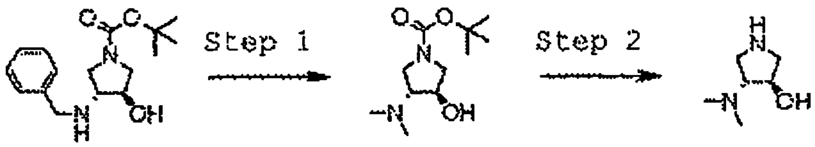


Step 1: tert-Butyl (3R,4R)-3-(Dimethylamino)-4-hydroxypyrrolidine-1-carboxylate

20% Palladium hydroxide-carbon (0.2 g) was added to an ethanol (20 ml) solution of tert-butyl (3R,4R)-3-(benzylamino)-4-hydroxypyrrolidine-1-carboxylate (500 mg, 1.71 mmol) and the resulting mixture was stirred at room temperature for 3 hours in a hydrogen atmosphere. Subsequently, a 37% aqueous solution of formalin (2.0 ml, 23.3 mmol) was added and the resulting mixture was further stirred for 3 hours in a hydrogen atmosphere. Insoluble matter was removed by filtration through celite and then the filtrate was concentrated under reduced pressure. The obtained residue was purified by silica gel column chromatography (chloroform:methanol = 10:1) to give the title compound (405 mg, quantitative) as a colorless oil.
$^1$H-NMR ($CDCl_3$) δ: 1.46 (9H, s), 2.33 (6H, s), 2.77 (1H, m), 3.19 (1H, m), 3.26 (1H, m), 3.59 (1H, m), 3.72 (1H, m), 4.27 (1H, q, J=6.3 Hz).

Step 2: (3R,4R)-4-(Dimethylamino)pyrrolidin-3-ol

The compound obtained in Step 1 above was reacted in the same way as in Step 6 of Example 102 to give the title compound as a colorless solid.
$^1$H-NMR ($D_2O$) δ: 2.90 (6H, s), 3.21 (1H, s), 3.28 (1H, dd, J=12.7, 5.4 Hz), 3.42-3.48 (1H, m), 3.57 (1H, dd, J=12.7, 6.8 Hz), 3.81-3.94 (2H, m), 4.69-4.73 (1H, m).

Reference Example 89

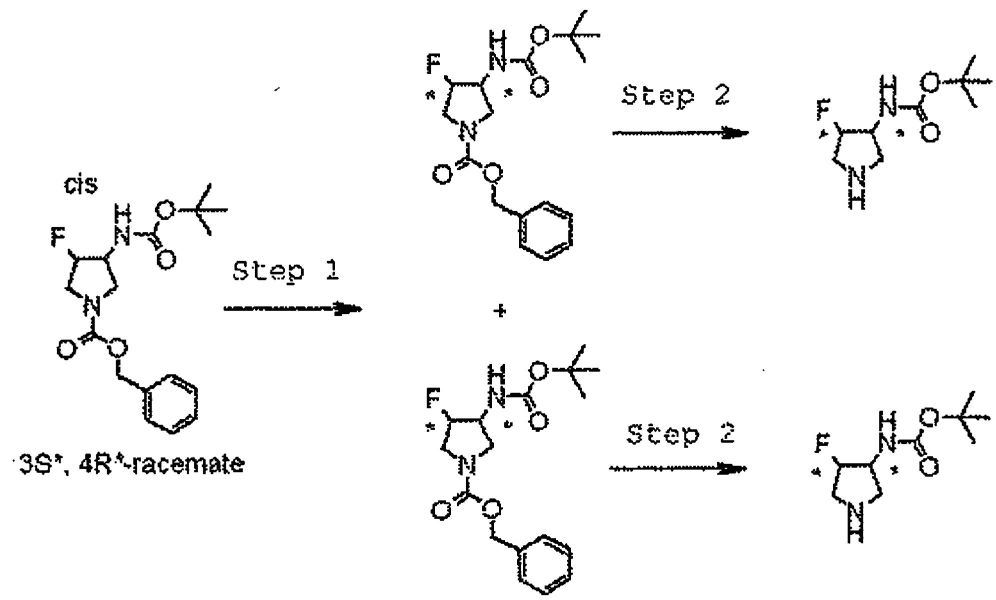

Step 1: Benzyl 3,4-cis-3-[(tert-Butoxycarbonyl)amino]-4-fluoropyrrolidine-1-carboxylate (Isomer A) and Benzyl 3,4-cis-3-[(tert-Butoxycarbonyl)amino]-4-fluoropyrrolidine-1-carboxylate (Isomer B)

Benzyl (3S*,4R*)-3-[(tert-butoxycarbonyl)amino]-4-fluoropyrrolidine-1-carboxylate (2.60 g) was resolved using an optically active column to respectively give the title compounds (Isomer A: 0.98 g, 28%; and Isomer B: 0.86 g, 25%) as a colorless solid.

Isomer A:

$^1$H-NMR ($CDCl_3$) δ: 1.44-1.48 (9H, m), 3.15 (1H, q, J=10.7 Hz), 3.52-4.00 (3H, m), 4.23-4.44 (1H, m), 4.82-5.01 (1H, m), 5.08-5.19 (3H, m), 7.30-7.40 (5H, m).

Isomer B:

$^1$H-NMR ($CDCl_3$) δ: 1.43-1.47 (9H, m), 3.15 (1H, q, J=10.6 Hz), 3.52-3.99 (3H, m), 4.23-4.42 (1H, m), 4.83-5.01 (1H, m), 5.08-5.18 (3H, m), 7.29-7.40 (5H, m).

Step 2: tert-Butyl (3,4-cis-4-Fluoropyrrolidin-3-yl)carbamate (Isomer A) and tert-Butyl (3,4-cis-4-Fluoropyrrolidin-3-yl) carbamate (Isomer B)

The compounds (Isomers A and B) obtained in Step 1 above were separately subjected to the same reaction as in Step 4 of Reference Example 13 to respectively give the title compounds as a colorless solid.

Reference Example 90

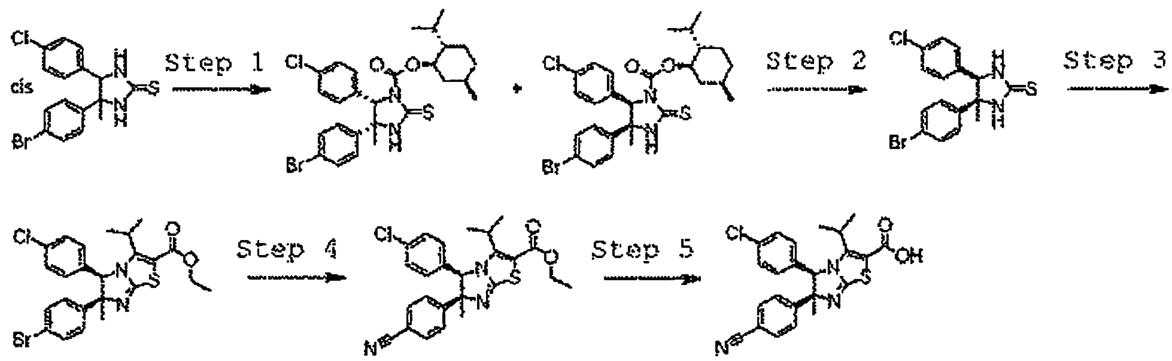


Step 1: (1R,2S,5R)-2-Isopropyl-5-methylcyclohexyl (4S,5R)-4-(4-Bromophenyl)-5-(4-chlorophenyl)-4-methyl-2-thioxoimidazopyridine-1-carboxylate

The compound obtained in Step 8 of Reference Example 37 was reacted in the same way as in Step 6 of Reference Example 38 to give the title compound as a colorless solid.
$^1$H-NMR ($CDCl_3$) δ: 0.35 (1H, q, J=11.7 Hz), 0.68 (1H, m), 0.72 (3H, d, J=7.1 Hz), 0.75 (3H, d, J=6.8 Hz), 0.90 (3H, d, J=6.8 Hz), 0.98 (1H, m), 1.23-1.29 (2H, m), 1.56-1.61 (4H, m), 1.88 (1H, m), 1.90 (3H, s), 4.57 (1H, td, J=10.9, 4.3 Hz), 5.24 (1H, s), 6.78 (2H, d, J=8.5 Hz), 6.92 (2H, d, J=8.8 Hz), 7.07 (2H, d, J=8.5 Hz), 7.26 (5H, d, J=8.8 Hz).

Step 2: (4S,5R)-4-(4-Bromophenyl)-5-(4-chlorophenyl)-4-methylimidazopyridine-2-thione

The compound obtained in Step 1 above was reacted in the same way as in Step 7 of Reference Example 38 to give the title compound as a colorless solid.
$^1$H-NMR ($CDCl_3$) δ: 1.87 (3H, s), 4.96 (1H, s), 6.11 (1H, brs), 6.33 (1H, brs), 6.78-6.83 (4H, m), 7.10 (2H, d, J=8.9 Hz), 7.25-7.28 (2H, m).

Step 3: Ethyl 6-(4-Bromophenyl)-5-(4-chlorophenyl)-3-isopropyl-6-methyl-5,6-dihydroimidazo[2,1-b][1,3]thiazole-2-carboxylate

The compound obtained in Step 2 above was reacted in the same way as in Step 2 of Example 1 to give the title compound as a pale yellow solid.
$^1$H-NMR ($CDCl_3$) δ: 1.02 (3H, d, J=7.1 Hz), 1.04 (3H, d, J=7.1 Hz), 1.37 (3H, t, J=7.2 Hz), 2.10 (3H, s), 3.37 (1H, m), 4.34 (2H, q, J=7.2 Hz), 5.50 (1H, brs), 6.50-6.85 (2H, m), 7.07 (2H, d, J=8.5 Hz), 7.14 (2H, d, J=8.5 Hz), 7.29 (3H, d, J=8.5 Hz).

Step 4: Ethyl (5R,6S)-5-(4-Chlorophenyl)-6-(4-cyanophenyl)-3-isopropyl-6-methyl-5,6-dihydroimidazo[2,1-b][1,3]thiazole-2-carboxylate

The compound obtained in Step 3 above was reacted in the same way as in Step 2 of Example 137 to give the title compound as a pale yellow oil.
$^1$H-NMR ($CDCl_3$) δ: 0.87 (3H, d, J=7.1 Hz), 0.97 (3H, d, J=7.1 Hz), 1.32 (3H, t, J=7.2 Hz), 1.78 (3H, s), 3.31 (1H, m), 4.23 (2H, q, J=7.2 Hz), 5.04 (1H, s), 6.70 (2H, brs), 6.99-7.18 (6H, m).
MS (ESI) m/z: 466, 468.

Step 5: (5R,6S)-5-(4-Chlorophenyl)-6-(4-cyanophenyl)-3-isopropyl-6-methyl-5,6-dihydroimidazo[2,1-b][1,3]thiazole-2-carboxylic acid

The compound obtained in Step 4 above was reacted in the same way as in Step 3 of Example 1 to give the title compound as a colorless solid.
$^1$H-NMR (DMSO-$d_6$) δ: 0.93 (6H, d, J=7.1 Hz), 2.01 (3H, s), 3.26 (1H, m), 6.22 (1H, brs), 7.18-7.21 (4H, m), 7.41 (2H, d, J=8.5 Hz), 7.64 (2H, d, J=8.3 Hz).

Reference Example 91

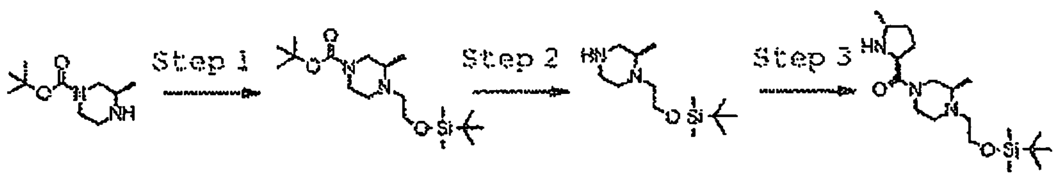


Step 1: tert-Butyl (3R)-4-(2-{[tert-Butyl(dimethyl)silyl]oxy}ethyl)-3-methylpiperazine-1-carboxylate

tert-Butyl (3R)-3-methylpiperazine-1-carboxylate was reacted in the same way as in Step 3 of Example 152 using {[tert-butyl(dimethyl)silyl]oxy}acetaldehyde instead of a 37% aqueous solution of formalin to give the title compound as a colorless oil.
$^1$H-NMR ($CDCl_3$, 60°C) δ: 0.06 (6H, s), 0.91 (9H, s), 1.04 (3H, d, J=6.3 Hz), 1.46 (9H, s), 2.38 (1H, m), 2.43-2.54 (2H, m), 2.75-2.82 (3H, m), 3.12 (1H, m), 3.66-3.76 (4H, m).

Step 2: (2R)-1-(2-{[tert-Butyl(dimethyl)silyl]oxy}ethyl)-2-methylpiperazine

The compound obtained in Step 1 above was reacted in the same way as in Step 6 of Example 102 under ice cooling and then subjected to the same reaction as in Step 2 of Reference Example 56 to give the title compound as a yellow oil.
$^1$H-NMR ($CDCl_3$) δ: 0.06 (6H, s), 0.90 (9H, s), 1.06 (3H, d, J=5.4 Hz), 2.50-2.56 (4H, m), 2.83-2.90 (5H, m), 3.69-3.75 (2H, m).

Step 3: (2R)-1-(2-{[tert-Butyl(dimethyl)silyl]oxy}ethyl)-2-methyl-4-[(5R)-5-methyl-L-prolyl]piperazine

The compound obtained in Step 1 of Reference Example 31 used instead of (5R,6S)-5,6-bis(4-chlorophenyl)-3-isopropyl-6-methyl-5,6-dihydroimidazo[2,1-b][1,3]thiazole-2-carboxylic acid and the compound obtained in Step 2 above used instead of piperazin-2-one were reacted in the same way as in Step 4 of Example 1 to give the title compound as a yellow oil.
$^1$H-NMR ($CDCl_3$, 65°C) δ: 0.05 (6H, s), 0.90 (9H, s), 1.05 (3H, d, J=6.1 Hz), 1.12 (3H, d, J=6.1 Hz), 1.36-1.49 (2H, m), 1.69 (1H, m), 1.89 (1H, m), 2.17 (1H, m), 2.40 (1H, m), 2.48-2.54 (2H, m), 2.77-2.87 (3H, m), 3.43 (1H, m), 3.68-3.71 (2H, m), 3.98 (1H, m).

Reference Example 92

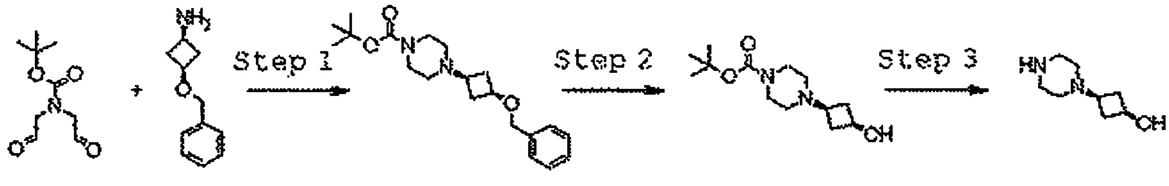


Step 1: tert-Butyl 4-[cis-3-(Benzyloxy)cyclobutyl]piperazine-1-carboxylate

cis-3-(Benzyloxy)cyclobutanamine (872 mg, 4.92 mmol), acetic acid (0.847 ml, 14.8 mmol), and 1 M solution of sodium cyanoborohydride in tetrahydrofuran (14.8 ml, 14.8 mmol) were sequentially added to a methanol (10 ml) solution of tert-butyl bis(2-oxoethyl)carbamate (0.830 g, 4.12 mmol) and the resulting mixture was stirred at room temperature for 18 hours. The reaction mixture was diluted with ethyl acetate and washed with saturated aqueous sodium bicarbonate solution and brine. Then, the organic layer was dried over anhydrous magnesium sulfate. The solvent was evaporated under reduced pressure. The obtained residue was purified by silica gel column chromatography (ethyl acetate) to give the title compound (1.04 g, 61%) as a pale yellow oil.
$^1$H-NMR ($CDCl_3$) δ: 1.46 (9H, s), 1.86 (2H, ddd, J=16.6, 8.4, 2.8 Hz), 2.26-2.35 (5H, m), 2.40-2.46 (2H, m), 3.43 (4H, t, J=4.9 Hz), 3.75-3.82 (1H, m), 4.42 (2H, s), 7.26-7.36 (5H, m).

Step 2: tert-Butyl 4-(cis-3-Hydroxycyclobutyl)piperazine-1-carboxylate

The compound obtained in Step 1 above was reacted in the same way as in Step 4 of Reference Example 13 to give the title compound as a colorless solid.
$^1$H-NMR (DMSO-$d_6$) δ: 1.40 (9H, s), 1.98-2.17 (2H, m), 2.69-2.88 (2H, m), 3.08-3.48 (6H, m), 3.77-4.09 (3H, m), 5.34-5.55 (1H, m), 10.75 (1H, brs).

Step 3: cis-3-Piperazin-1-ylcyclobutanol

The compound obtained in Step 2 above was reacted in the same way as in Step 2 of Example 214 to give the title compound as a pale yellow oil.
$^1$H-NMR (DMSO-$d_6$) δ: 2.06-2.26 (2H, m), 3.00-3.67 (12H, m), 3.79-3.86 (1H, m), 9.85 (2H, brs), 12.21 (1H, brs).

Reference Example 93

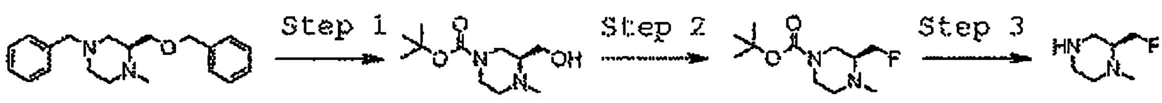


Step 1: tert-Butyl (3S)-3-(Hydroxymethyl)-4-methylpiperazine-1-carboxylate

(2S)-4-Benzyl-2-[(benzyloxy)methyl]-1-methylpiperazine was reacted in the same way as in Step 6 of Reference

Example 6 to give the title compound as a colorless oil.
$^1$H-NMR ($CDCl_3$) δ: 1.46 (9H, s), 2.14-2.18 (2H, m), 2.27 (2H, td, J=11.0, 3.3 Hz), 2.35 (3H, s), 2.79 (1H, d, J=11.0 Hz), 3.05 (2H, m), 3.50 (1H, dd, J=11.4, 2.1 Hz), 3.84 (2H, dd, J=11.4, 4.5 Hz).

Step 2: tert-Butyl (3S)-3-(Fluoromethyl)-4-methylpiperazine-1-carboxylate

Triethylamine (122 μl, 0.88 mmol) and methanesulfonyl chloride (59 μl, 0.76 mmol) were added under ice cooling to a dichloromethane (6 ml) solution of the compound (135 mg, 0.59 mmol) obtained in Step 1 above and the resulting mixture was stirred for 1 hour. The mixture was stirred at room temperature for 30 minutes and then an aqueous solution of saturated ammonium chloride was added, followed by extraction with ethyl acetate. The organic layer was washed with brine and dried over anhydrous sodium sulfate and then the solvent was evaporated. The obtained mesylate was dissolved in tetrahydrofuran (6 ml), 1 M solution of tetra-n-butylammonium fluoride in tetrahydrofuran (1.76 ml, 1.76 mmol) was added under ice cooling, and the resulting mixture was stirred at room temperature for 3 hours. The solvent was evaporated and ethyl acetate was added. The organic layer was washed with brine and dried over anhydrous sodium sulfate and then the solvent was evaporated. The residue was purified by silica gel column chromatography (hexane:ethyl acetate = 100:0 → 30:1) to give the title compound (109 mg, 80%) as a pale yellow oil.
$^1$H-NMR ($CDCl_3$) δ: 1.46 (9H, s), 2.21-2.34 (3H, m), 2.38 (3H, s), 2.99-3.11 (1H, m), 3.63-3.69 (1H, m), 3.85-3.90 (2H, m), 4.43 (1H, d, J=3.4 Hz), 4.55 (1H, d, J=3.7 Hz). MS (ESI) m/z: 233.

Step 3: (2S)-2-(Fluoromethyl)-1-methylpiperazine

The compound obtained in Step 2 above was reacted in the same way as in Step 2 of Example 214 to give the title compound as a brown oil.

Reference Example 94

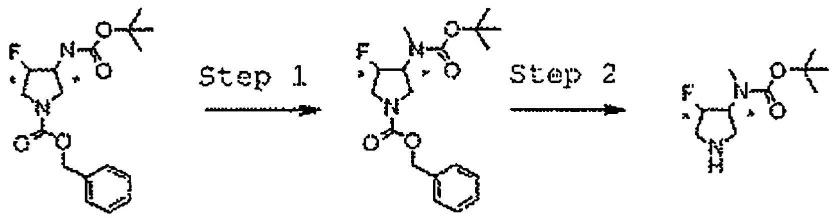


Step 1: Benzyl 3,4-cis-3-[(tert-Butoxycarbonyl)(methyl)amino]-4-fluoropyrrolidine-1-carboxylate (Isomer A)

Benzyl 3,4-cis-3-[(tert-butoxycarbonyl)amino]-4-fluoropyrrolidine-1-carboxylate (Isomer A) was reacted in the same way as in Step 4 of Reference Example 62 to give the title compound as a colorless oil.
$^1$H-NMR ($CDCl_3$) δ: 1.47 (9H, d, J=2.0 Hz), 2.91 (3H, d, J=5.4 Hz), 3.48-3.83 (4H, m), 4.30-4.89 (1H, m), 5.05-5.24 (3H, m), 7.29-7.39 (5H, m).

Step 2: tert-Butyl (3,4-cis-4-Fluoropyrrolidin-3-yl)methylcarbamate (Isomer A)

The compound obtained in Step 1 above was reacted in the same way as in Step 4 of Reference Example 13 to give the title compound as a pale orange oil.

Reference Example 95

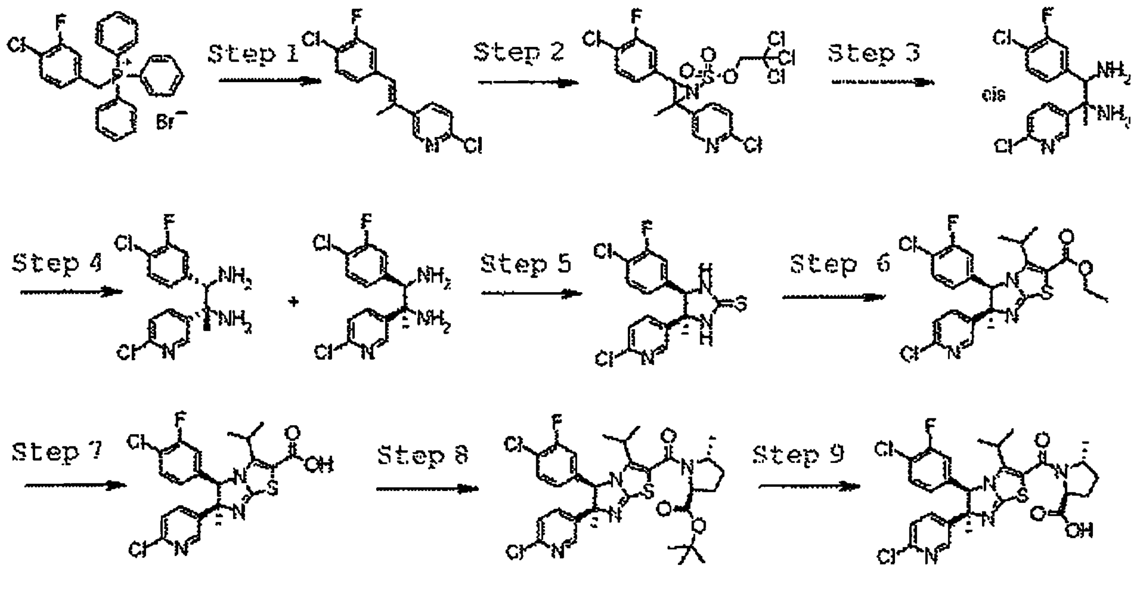


Step 1: 2-Chloro-5-[(E)-2-(4-chloro-3-fluorophenyl)-1-methylvinyl]pyridine

Bromo(4-chloro-3-fluorobenzyl)triphenylphosphorane was reacted in the same way as in Step 1 of Reference Example 50 to give the title compound as a colorless solid.
$^1$H-NMR ($CDCl_3$) δ: 2.26 (3H, d, J=1.5 Hz), 6.73 (1H, s), 7.07 (1H, dd, J=8.3, 2.0 Hz), 7.15 (1H, dd, J=10.3, 2.0 Hz), 7.34 (1H, dd, J=8.4, 0.6 Hz), 7.40 (1H, t, J=8.1 Hz), 7.75 (1H, dd, J=8.3, 2.7 Hz), 8.51 (1H, d, J=2.7 Hz).
MS (ESI) m/z: 282.

Step 2: 2,2,2-Trichloroethyl (2R*,3R*)-3-(4-Chloro-3-fluorophenyl)-2-(6-chloropyridin-3-yl)-2-methylaziridine-1-sulfamate

The compound obtained in Step 1 above was reacted in the same way as in Step 2 of Reference Example 14 to give the title compound as a colorless solid.
$^1$H-NMR ($CDCl_3$) δ: 1.46 (3H, s), 4.62 (1H, s), 4.80 (2H, s), 7.21 (1H, d, J=8.3 Hz), 7.26 (1H, dd, J=9.3, 2.0 Hz), 7.42 (1H, d, J=8.5 Hz), 7.50 (1H, t, J=7.8 Hz), 7.92 (1H, dd, J=8.3, 2.4 Hz), 8.60 (1H, d, J=2.4 Hz).
MS (ESI) m/z: 508.

Step 3: (1R*,2S*)-1-(4-Chloro-3-fluorophenyl)-2-(6-chloropyridin-3-yl)propane-1,2-diamine

The compound obtained in Step 2 above was reacted in the same way as in Step 3 of Reference Example 14 and then subjected to the same reaction as in Step 4 of Reference Example 14 without being purified to give the title compound as a colorless solid.
$^1$H-NMR ($CDCl_3$) δ: 1.54 (3H, s), 1.58 (4H, brs), 4.08 (1H, s), 6.72 (1H, dd, J=8.3, 2.0 Hz), 6.96 (1H, dd, J=10.3, 2.0 Hz), 7.21 (1H, t, J=7.9 Hz), 7.23 (1H, dd, J=8.3, 0.7 Hz), 7.59 (1H, dd, J=8.5, 2.7 Hz), 8.36 (1H, dd, J=2.7, 0.7 Hz).
MS (ESI) m/z: 314.

Step 4: (1R,2S)-1-(4-Chloro-3-fluorophenyl)-2-(6-chloropyridin-3-yl)propane-1,2-diamine

The compound obtained in Step 3 above was reacted in the same way as in Step 5 of Reference Example 1 to give the title compound as a colorless solid.
$^1$H-NMR ($CDCl_3$) δ: 1.54 (3H, s), 1.56 (4H, brs), 4.08 (1H, s), 6.72 (1H, dd, J=8.3, 2.0 Hz), 6.96 (1H, dd, J=10.4, 2.1 Hz), 7.20-7.24 (2H, m), 7.59 (1H, dd, J=8.4, 2.6 Hz), 8.36 (1H, dd, J=2.7, 0.7 Hz).
MS (ESI) m/z: 314.

Step 5: (4S,5R)-5-(4-Chloro-3-fluorophenyl)-4-(6-chloropyridin-3-yl)-4-methylimidazopyridine-2-thione

The compound obtained in Step 4 above was reacted in the same way as in Step 1 of Example 1 to give the title compound as a colorless solid.
$^1$H-NMR (DMSO-$d_6$) δ: 1.75 (3H, s), 5.02 (1H, s), 6.77 (1H, d, J=8.3 Hz), 6.93 (1H, dd, J=10.4, 1.8 Hz), 7.31 (1H, d, J=8.5 Hz), 7.38 (1H, t, J=8.1 Hz), 7.44 (1H, dd, J=8.2, 2.6 Hz), 8.02 (1H, d, J=2.7 Hz), 8.95 (1H, brs), 9.06 (1H, brs).

Step 6: Ethyl (5R,6S)-5-(4-Chloro-3-fluorophenyl)-6-(6-chloropyridin-3-yl)-3-isopropyl-6-methyl-5,6-dihydroimidazo [2,1-b][1,3]thiazole-2-carboxylate

The compound obtained in Step 5 above was reacted in the same way as in Step 2 of Example 1 to give the title compound as a colorless solid.
$^1$H-NMR ($CDCl_3$) δ: 0.90 (3H, d, J=7.1 Hz), 1.04 (3H, d, J=7.1 Hz), 1.34 (3H, t, J=7.2 Hz), 1.83 (3H, s), 3.35-3.45 (1H, m), 4.26 (2H, q, J=7.1 Hz), 5.10 (1H, s), 6.51-6.59 (2H, m), 7.05 (1H, d, J=8.5 Hz), 7.16 (1H, t, J=7.8 Hz), 7.51-7.55 (1H, m), 8.21-8.22 (1H, m).
MS (ESI) m/z: 494.

Step 7: (5R,6S)-5-(4-Chloro-3-fluorophenyl)-6-(6-chloropyridin-3-yl)-3-isopropyl-6-methyl-5,6-dihydroimidazo[2,1-b] [1,3]thiazole-2-carboxylic acid

The compound obtained in Step 6 above was reacted in the same way as in Step 3 of Example 1 to give the title compound as a colorless solid.
$^1$H-NMR (DMSO-$d_6$ ) δ: 0.82 (3H, d, J=7.1 Hz), 0.99 (3H, d, J=7.1 Hz), 1.79 (3H, s), 3.39-3.44 (1H, m), 5.80 (1H, s), 6.41-6.45 (1H, m), 7.26 (1H, d, J=8.3 Hz), 7.36-7.44 (2H, m), 7.67 (1H, dd, J=8.4, 2.6 Hz), 8.28 (1H, d, J=2.4 Hz).
MS (ESI) m/z: 466.

Step 8: tert-Butyl (5R)-1-{[(5R,6S)-5-(4-Chloro-3-fluorophenyl)-6-(6-chloropyridin-3-yl)-3-isopropyl-6-methyl-5,6-dihydroimidazo[2,1-b][1,3]thiazol-2-yl]carbonyl}-5-methyl-L-prolinate

The compound obtained in Step 7 above was reacted in the same way as in Example 112 using the compound obtained in Reference Example 30 instead of the compound obtained in 2 of Reference Example 18 to give the title compound as a pale yellow solid.
$^1$H-NMR ($CDCl_3$) δ: 1.01 (3H, d, J=7.1 Hz), 1. 07 (3H, d, J=6.8 Hz), 1.21 (3H, d, J=6.3 Hz), 1.45 (9H, s), 1.65-1.68 (1H, m), 1.82 (3H, s), 1.97-2.01 (1H, m), 2.23-2.27 (2H, m), 2.67-2.71 (1H, m), 4.47-4.51 (1H, m), 4.54-4.57 (1H, m), 4.97 (1H, s), 6.52-6.57 (2H, m), 7.02 (1H, d, J=8.3 Hz), 7.12 (1H, t, J=7.8 Hz), 7.52 (1H, dd, J=8.3, 2.7 Hz), 8.23 (1H, d, J=2.4 Hz).
MS (ESI) m/z: 633.

Step 9: (5R)-1-{[(5R,6S)-5-(4-Chloro-3-fluorophenyl)-6-(6-chloropyridin-3-yl)-3-isopropyl-6-methyl-5,6-dihydroimidazo [2,1-b][1,3]thiazol-2-yl]carbonyl}-5-methyl-L-proline

The compound obtained in Step 8 above was reacted in the same way as in Step 6 of Example 102 to give the title compound as a colorless solid.
$^1$H-NMR (DMSO-$d_6$) δ: 0.91 (3H, d, J=7.1 Hz), 1.00 (3H, d, J=7.1 Hz), 1.17 (3H, d, J=6.3 Hz), 1.62-1.67 (1H, m), 1.88 (3H, s), 1.92-1.97 (1H, m), 2.06-2.12 (1H, m), 2.32-2.37 (1H, m), 2.67-2.76 (1H, m), 4.30-4.33 (1H, m), 4.52-4.55 (1H, m), 5.77 (1H, s), 6.72-6.76 (1H, m), 6.95-7.00 (1H, m), 7.25 (1H, d, J=8.3 Hz), 7.37 (1H, t, J=7.9 Hz), 7.68 (1H, dd, J=8.3, 2.2 Hz), 8.28 (1H, d, J=2.2 Hz).
MS (ESI) m/z: 577.

Reference Example 96

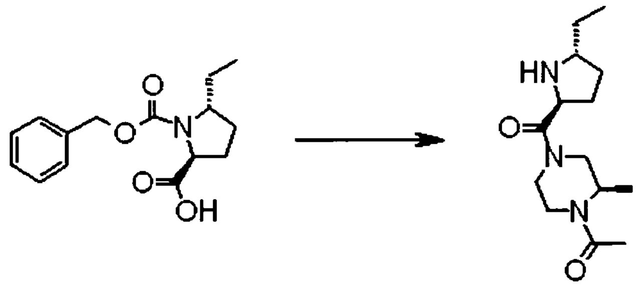

(2R)-1-Acetyl-4-[(5R)-5-ethyl-L-prolyl]-2-methylpiperazine

The compound obtained in Step 2 of Reference Example 13 used instead of (5R,6S)-5,6-bis(4-chlorophenyl)-3-isopropyl-6-methyl-5,6-dihydroimidazo[2,1-b][1,3]thiazole-2-carboxylic acid and (2R)-1-acetyl-2-methylpiperazine used instead of piperazin-2-one were reacted in the same way as in Step 4 of Example 1. Then, the obtained amide form was reacted in the same way as in Step 4 of Reference Example 13 to give the title compound.
$^1$H-NMR (DMSO-$d_6$) δ: 0.92 and 0.94 (total 3H, each d, J=7.6 Hz), 1.04 (3H, t, J=7.0 Hz), 1.57-1.77 (2H, m), 1.81-1.90 (1H, m), 2.00 and 2.02 (total 3H, each s), 2.04-2.14 (1H, m), 2.38-2.49 (1H, m), 2.81-2.98 (1H, m), 3.30-3.37 (2H, m), 3.38-3.49 (3H, m), 3.71 and 3.89 (total 1H, each m), 4.11-4.20 (1H, m), 4.50-4.57 (1H, m), 4.77-4.83 (1H, m).

(Test Example 1 Mdm2/p53 binding assay)

A protein dilution containing 6.25 nM each of His-p53 (fusion protein of a p53 partial protein having p53 amino acids at positions 1 to 132, with a histidine protein) and GST-Mdm2 (fusion protein of a Mdm2 partial protein, having Mdm2 amino acids at positions 25 to 108 with leucine residue 33 substituted by glutamic acid, with glutathione transferase) proteins was prepared using a protein buffer solution (20 mM HEPES pH 7.4, 150 mM NaCl, 0.1% BSA). This protein dilution was added in an amount of 8 μL/well to a 384-well plate (384-well low volume NBC, Corning Inc., catalog No: 3676).
Next, a test compound was diluted with protein buffer solution containing 10% DMSO, and this test compound dilution was added in an amount of 4 μL/well to the plate.
Subsequently, a dilution containing an XL665-labeled anti-His antibody (HTRF monoclonal anti-6HIS antibody labeled with XL665 (catalog No: 61HISXL), Schering-Plough Corp.) and a europium (Eu)-labeled anti-GST antibody (HTRF monoclonal anti-GST antibody labeled with europium cryptate, Schering-Plough Corp., catalog No: 61GSTKL) at concentrations of 2.5 μg/mL and 0.325 μg/mL, respectively, was prepared using an antibody diluting buffer solution (20 mM HEPES pH 7.4, 150 mM NaCl, 0.1% BSA, 0.5 M KF). These dilutions were added in an amount of 8 μL/well (total reaction solution volume: 20 μl/well) Then, the plate was left at 25°C for 1 hour.
Time-resolved fluorescence at 620 and 665 nm was measured at an excitation wavelength of 320 nm using a plate reader (ARVOsx, PerkinElmer Co., Ltd.). Ratio (R) was calculated using the measured values (cps 620 nm and cps 665 nm) according to the following formula:

$$R = (\text{cps } 665 \text{ nm} - BI - C \times \text{cps } 620 \text{ nm}) / \text{cps } 620 \text{ nm}$$

BI: measured value at 665 nm of reaction solution (only each buffer solution) nonsupplemented with each protein, the compound, and the antibodies

$$C \text{ (correction factor)} = (A - BI)/D$$

A and D: each measured value at 665 nm and 620 nm of reaction solution supplemented with only Eu-labeled anti-GST antibody solution.
The R value calculated from the well supplemented with His-p53, GST-Mdm2, the test compound, and each antibody was defined as R (sample). The R value calculated from the well supplemented with His-p53, GST-Mdm2, and each antibody but without the test compound was defined as R (control). The R value calculated from the well supplemented

with GST-Mdm2, the test compound, and each antibody but without His-p53 was defined as R (background). T/C was calculated from the formula shown below. An $IC_{50}$ value for Mdm2/p53 binding was calculated by sigmoid fitting using Prism (GraphPad Software). The results are shown in Table 1.

T/C = (R (sample)-R (background))/(R (control)-R (background))

[Table 1]

| | $IC_{50}$ value (μM) |
|---|---|
| Compound of Example 18 | 0.087 |
| Compound of Example 39 | 0.051 |
| Compound of Example 68 | 0.046 |
| Compound of Example 73 | 0.018 |
| Compound of Example 74<br>Compound of Example 88 | 0.023<br>0.029 |
| Compound of Example 89 | 0.143 |
| Compound of Example 93 | 0.135 |
| Compound of Example 95 | 0.018 |
| Compound (Isomer B) of Example 97 | 0.010 |
| Compound (Cis-Isomer) of Example 99 | 0.194 |
| Compound of Example 102 | 0.080 |
| Compound of Example 105 | 0.018 |
| Compound of Example 111 | 0.058 |
| Compound of Example 112 | 0.014 |
| Compound of Example 126 | 0.020 |
| Compound of Example 155 | 0.030 |
| Compound of Example 164 | 0.084 |
| Compound of Example 167 | 0.010 |
| Compound of Example 180 | 0.013 |
| Compound of Example 204 | 0.019 |
| Compound of Example 211 | 0.012 |
| Compound of Example 219 | 0.004 |
| Compound of Example 220 | 0.076 |
| Compound of Example 240 | 0.013 |
| Compound of Example 260 | 0.015 |

(Test Example 2 Cell growth inhibition assay)

An anti-cell test was conducted using two cells (human lung cancer-derived cell line NCI-H460 having wild-type p53 and human colon cancer-derived cell line DLD-1 having mutant p53).

Cells of the respective cell lines were suspended in a medium (RPMI1640 medium containing 10% fetal bovine serum) and these suspensions were inoculated in amounts of 500 cells/150 μL/well and 1000 cells/150 μL/well, respectively, to a 96-well multiwell plate. A test compound was dissolved in DMSO and this solution was diluted with medium to prepare a sample solution (DMSO concentration: 1% or lower). On the next day of inoculation, medium containing DMSO nonsupplemented with the test compound (hereinafter, referred to as a DMSO dilution, DMSO concentration: 1% or lower) or the sample solution was added in an amount of 50 μL/well. MTT assay was conducted immediately after addition of the sample solution or the DMSO dilution to the cells and after culture of the cells (after addition of the sample solution or the DMSO dilution) at 37°C for 72 hours in a 5% $CO_2$ atmosphere. The MTT assay was conducted as shown below.

A 5 mg/mL MTT (3-(4,5-dimethylthiazol-2-yl)-2,5-diphenyltetrazolium bromide, Sigma-Aldrich Co., M-2128) solution was prepared using phosphate buffer solutions (Dulbecco's Phosphate-buffered Salines). This MTT solution was added in an amount of 20 μL/well. Then, the plate was cultured at 37°C for 4 hours in a 5% $CO_2$ atmosphere. The plate was centrifuged at 1200 rpm for 5 minutes and then the culture supernatant was removed by aspiration using a dispenser. DMSO was added in an amount of 150 μL/well to dissolve generated formazan. The plate was stirred using a plate mixer for uniform color development from each well. The absorbance of each well was measured under conditions of OD 540 nm and reference 660 nm using a plate reader (SpectraMax PLUS384, Molecular Devices, CA, USA).

The OD value measured immediately after addition of the sample solution was defined as S. The OD value measured 72 hours after addition of the sample solution was defined as T. The OD value measured 72 hours after addition of the DMSO dilution was defined as C. T/C (%) was determined at each concentration according to the calculation formula shown below to prepare a dose response curve, from which 50% growth inhibition concentration ($GI_{50}$ value) was calculated.

$$T/C\ (\%) = (T-S)/(C-S) \times 100$$

The results are shown in Table 2.

[Table 2]

| | $GI_{50}$ value (μM) | |
|---|---|---|
| | NCI-H460 cell | DLD-1 cell |
| Compound of Example 73 | 1.75 | >50 |
| Compound (Isomer B) of Example 97 | 2.81 | >50 |
| Compound of Example 112 | 1.38 | 40.7 |

## Claims

1. A compound represented by formula (1), a salt of the compound, or a solvate of the compound or the salt:

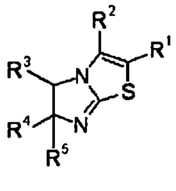

(1)

wherein
$R^1$ represents a hydrogen atom, $-V_1-V_2$, -CO-W, or $-COX_1-CO-X_2$, wherein
$V_1$ represents a $C_1$-$C_6$ alkylene group,
$V_2$, W and $X_2$ each independently represents a group selected from the group consisting of a hydrogen atom, a hydroxy group, a $C_1$-$C_6$ alkoxy group, an amino group which may be substituted with one or two 4- to 7-membered saturated or unsaturated heterocyclic groups (said 4- to 7-membered saturated or unsaturated heterocyclic group

may have one or more $C_1$-$C_6$ alkyl groups and/or oxo groups), a $C_1$-$C_6$ alkylamino group which may have one or more substituents selected from the following Group A, an amino $C_1$-$C_6$ alkylamino group which may have one or more substituents selected from the following Group B, a 4- to 7-membered saturated or unsaturated nitrogen-containing heterocyclic group which may have one or more substituents selected from the following Group C, and an 8- to 11-membered bicyclic condensed nitrogen-containing heterocyclic group which may have one or more substituents selected from the following Group D,
$X_1$ represents a group selected from the group consisting of an NH-$C_1$-$C_6$ alkylene group (said NH-$C_1$-$C_6$ alkylene group may be substituted on NH with a $C_1$-$C_6$ alkyl group), a divalent 4- to 7-membered saturated or unsaturated nitrogen-containing heterocyclic group which may have one or more substituents selected from the following Group E, and a divalent 8- to 11-membered bicyclic condensed nitrogen-containing heterocyclic group which may have one or more substituents selected from the following Group F,
$R^2$ represents a group selected from the group consisting of a hydrogen atom, a phenyl group, and a $C_1$-$C_6$ alkyl group which may have one or more substituents selected from the following Group G,
$R^3$ and $R^4$ each independently represents a group selected from the group consisting of a $C_1$-$C_6$ alkyl group which may have one or more substituents selected from the following Group H, a phenyl group which may have one or more substituents selected from the following Group I, and a 5- to 6-membered aromatic heterocyclic group which may have one or more substituents selected from the following Group I (provided that $R^3$ and $R^4$ do not both represent a C1-C6 alkyl group which may have a substituent(s)),
$R^5$ represents a hydrogen atom or a $C_1$-$C_6$ alkyl group, and
furthermore, $R^4$ and $R^5$ together with the carbon atom on the ring to which $R^4$ and $R^5$ are bonded may form a 3-to 7-membered spiro ring:

Group A: a $C_1$-$C_6$ alkylamino $C_1$-$C_6$ alkoxy group, a hydroxy $C_1$-$C_6$ alkoxy group, a $C_1$-$C_6$ alkoxy group, a $C_1$-$C_6$ alkoxycarbonyl group, a $C_1$-$C_6$ alkylamino group, a 4- to 7-membered saturated or unsaturated heterocyclic group (said 4- to 7-membered saturated or unsaturated heterocyclic group may have one or more substituents selected from the group consisting of a $C_1$-$C_6$ alkyl group which may be substituted with one or more hydroxy groups, a $C_2$-$C_6$ alkanoyl group, a hydroxy group, a $C_1$-$C_6$ alkoxycarbonyl group, a $C_1$-$C_6$ alkylamino $C_1$-$C_6$ alkyl group, a $C_1$-$C_6$ alkylaminocarbonyl group, a $C_1$-$C_6$ alkylamino $C_1$-$C_6$ alkylcarbonyl group, and an oxo group), a hydroxy group, and a carboxy group;
Group B: a $C_1$-$C_6$ alkyl group (said $C_1$-$C_6$ alkyl group being a substituent on an amino group of the amino $C_1$-$C_6$ alkylamino group), a formyl group, and a $C_2$-$C_6$ alkanoyl group;
Group C: a halogen atom, a hydroxy group, a $C_1$-$C_6$ alkyl group which may be substituted with one or more hydroxy groups, a $C_1$-$C_6$ alkoxy group, a $C_1$-$C_6$ alkoxy $C_1$-$C_6$ alkyl group (said $C_1$-$C_6$ alkoxy $C_1$-$C_6$ alkyl group may have one or more substituents selected from the group consisting of a hydroxyphenyl group, a carboxy group, and a 4- to 7-membered saturated or unsaturated heterocyclic group which may have a substituent(s)), a halogeno $C_1$-$C_6$ alkyl group, a carboxy $C_1$-$C_6$ alkyl group, a $C_1$-$C_6$ alkoxycarbonyl $C_1$-$C_6$ alkyl group, a $C_1$-$C_6$ alkylamino $C_1$-$C_6$ alkyl group, a carbamoyl $C_1$-$C_6$ alkyl group, a carboxy group, a formyl group, a $C_2$-$C_6$ alkanoyl group, a $C_1$-$C_6$ alkoxycarbonyl group, an amino group, a $C_1$-$C_6$ alkylamino group, a $C_1$-$C_6$ alkylsulfonyl group, an oxo group, a phenyl group, a 4- to 7-membered saturated or unsaturated heterocyclic group (said 4- to 7-membered saturated or unsaturated heterocyclic group may have one or more substituents selected from a $C_1$-$C_6$ alkyl group, a $C_2$-$C_6$ alkanoyl group, and an oxo group), and a $C_1$-$C_6$ alkylene-4- to 7-membered saturated or unsaturated heterocyclic group (said 4- to 7-membered saturated or unsaturated heterocyclic group may have one or more substituents selected from a $C_1$-$C_6$ alkyl group, a $C_2$-$C_6$ alkanoyl group, and an oxo group);
Group D: a $C_1$-$C_6$ alkyl group and an oxo group;
Group E: a halogen atom, a hydroxy group, a $C_1$-$C_6$ alkyl group, a $C_1$-$C_6$ alkoxy group, an amino group, a $C_1$-$C_6$ alkylamino group, a cyano group, a $C_1$-$C_6$ alkylamino $C_1$-$C_6$ alkyl group, and an oxo group;
Group F: a $C_1$-$C_6$ alkyl group and an oxo group;
Group G: a $C_1$-$C_6$ alkoxy group, an oxo group, a 4- to 7-membered saturated or unsaturated heterocyclic group (said 4- to 7-membered saturated or unsaturated heterocyclic group may be substituted with a $C_1$-$C_6$ alkyl group and/or an oxo group), a hydroxy group, and a $C_3$-$C_8$ cycloalkyl group;
Group H: a phenyl group and a $C_3$-$C_8$ cycloalkyl group; and
Group I: a halogen atom, an amino group, a $C_1$-$C_6$ alkyl group, a $C_1$-$C_6$ alkoxy group, a $C_1$-$C_6$ alkylamino group, a halogeno $C_1$-$C_6$ alkyl group, and a cyano group.

**2.** The compound according to claim 1, a salt of the compound, or a solvate of the compound or the salt, wherein $R^1$ in the formula (1) represents -$V_1$-$V_2$, wherein
$V_1$ represents a $C_1$-$C_6$ alkylene group, and
$V_2$ represents a group selected from the group consisting of a hydrogen atom, a hydroxy group, and a 4-to 7-membered saturated or unsaturated nitrogen-containing heterocyclic group which may have one or more substituents selected from Group C (where, Group C has the same meaning as defined above).

**3.** The compound according to claim 2, a salt of the compound, or a solvate of the compound or the salt, wherein $V_1$ represents a methylene group or an ethylidene group ($-CH(CH_3)-$), and
$V_2$ represents a 4- to 7-membered saturated or unsaturated nitrogen-containing heterocyclic group which may have one or more substituents selected from Group C (where, Group C has the same meaning as defined above).

**4.** The compound according to claim 3, a salt of the compound, or a solvate of the compound or the salt, wherein $V_1$ represents a methylene group or an ethylidene group ($-CH(CH_3)-$), and
$V_2$ represents a piperazinyl group or a pyrrolidinyl group (said piperazinyl group or pyrrolidinyl group may have one or more substituents selected from the group consisting of an oxo group, a methyl group, and an ethyl group).

**5.** The compound according to claim 1, a salt of the compound, or a solvate of the compound or the salt, wherein $R^1$ in the formula (1) represents -CO-W, wherein
W represents a group selected from the group consisting of a hydroxy group, a $C_1$-$C_6$ alkoxy group, an amino group which may be substituted with one or two 4-to 7-membered saturated or unsaturated heterocyclic groups (said 4- to 7-membered saturated or unsaturated heterocyclic group may have one or more $C_1$-$C_6$ alkyl groups and/or oxo groups), a $C_1$-$C_6$ alkylamino group which may have one or more substituents selected from Group A, an amino $C_1$-$C_6$ alkylamino group which may have one or more substituents selected from Group B, a 4- to 7-membered saturated or unsaturated nitrogen-containing heterocyclic group which may have one or more substituents selected from Group C, and an 8- to 11-membered bicyclic condensed nitrogen-containing heterocyclic group which may have one or more substituents selected from Group D (where, Groups A, B, C, and D have the same meanings as defined above).

**6.** The compound according to claim 5, a salt of the compound, or a solvate of the compound or the salt, wherein W represents a $C_1$-$C_6$ alkylamino group which may have one or more substituents selected from Group A (where, Group A has the same meaning as defined above).

**7.** The compound according to claim 6, a salt of the compound, or a solvate of the compound or the salt, wherein W represents a methylamino group, a dimethylamino group, an ethylmethylamino group, or an isopropylmethylamino group (these groups may be substituted with an azetidinyl group, a pyrrolidinyl group, or a cyclobutyl group (said azetidinyl group, pyrrolidinyl group, or cyclobutyl group may have one or more $C_1$-$C_6$ alkyl groups and/or oxo groups)).

**8.** The compound according to claim 5, a salt of the compound, or a solvate of the compound or the salt, wherein W represents an amino $C_1$-$C_6$ alkylamino group which may have one or more substituents selected from Group B (where, Group B has the same meaning as defined above).

**9.** The compound according to claim 8, a salt of the compound, or a solvate of the compound or the salt, wherein W represents a (2-aminoethyl) amino group, a (1-aminopropyl-2-yl)amino group, or a (2-aminopropyl)amino group (said (2-aminoethyl)amino group, (1-aminopropyl-2-yl) amino group, or (2-aminopropyl)amino group may be substituted with one or more methyl groups (however, said methyl group being a substituent on an amino group of each group) or acetyl groups).

**10.** The compound according to claim 5, a salt of the compound, or a solvate of the compound or the salt, wherein W represents a 4- to 7-membered saturated or unsaturated nitrogen-containing heterocyclic group which may have one or more substituents selected from Group C (where, Group C has the same meaning as defined above).

**11.** The compound according to claim 10, a salt of the compound, or a solvate of the compound or the salt, wherein W represents a piperazinyl group or a pyrrolidinyl group (said piperazinyl group or pyrrolidinyl group may have one or more substituents selected from the group consisting of an oxo group, a methyl group, an ethyl group, an acetyl group, a dimethylaminomethyl group, a (morpholin-4-yl)methyl group, and a carbamoyl methyl group).

**12.** The compound according to claim 1, a salt of the compound, or a solvate of the compound or the salt, wherein $R^1$ in the formula (1) represents -CO-$X_1$-CO-$X_2$, wherein
$X_1$ represents a group selected from the group consisting of an NH-$C_1$-$C_6$ alkylene group (said NH-$C_1$-$C_6$ alkylene group may be substituted on NH with a $C_1$-$C_6$ alkyl group), a divalent 4- to 7-membered saturated or unsaturated nitrogen-containing heterocyclic group which may have one or more substituents selected from Group E, and a

divalent 8- to 11-membered bicyclic condensed nitrogen-containing heterocyclic group which may have one or more substituents selected from Group F (where, Groups E and F have the same meanings as defined above), and $X_2$ represents a group selected from the group consisting of a hydroxy group, a $C_1$-$C_6$ alkoxy group, an amino group which may be substituted with one or two 4-to 7-membered saturated or unsaturated heterocyclic groups (said 4- to 7-membered saturated or unsaturated heterocyclic group may have one or more $C_1$-$C_6$ alkyl groups and/or oxo groups), a $C_1$-$C_6$ alkylamino group which may have one or more substituents selected from Group A, an amino $C_1$-$C_6$ alkylamino group which may have one or more substituents selected from Group B, a 4- to 7-membered saturated or unsaturated nitrogen-containing heterocyclic group which may have one or more substituents selected from Group C, and an 8- to 11-membered bicyclic condensed nitrogen-containing heterocyclic group which may have one or more substituents selected from Group D (where, Groups A, B, C, and D have the same meanings as defined above).

**13.** The compound according to claim 12, a salt of the compound, or a solvate of the compound or the salt, wherein $X_1$ represents an NH-$C_1$-$C_6$ alkylene group (said NH-$C_1$-$C_6$ alkylene group may be substituted on NH with a $C_1$-$C_6$ alkyl group).

**14.** The compound according to claim 13, a salt of the compound, or a solvate of the compound or the salt, wherein $X_1$ represents an NH-methylene group (said NH-methylene group may be substituted on NH with a substituent selected from the group consisting of an ethyl group, a propyl group, an isopropyl group, a butyl group, an isobutyl group, and a sec-butyl group).

**15.** The compound according to claim 12, a salt of the compound, or a solvate of the compound or the salt, wherein $X_1$ represents a divalent 4- to 7-membered saturated or unsaturated nitrogen-containing heterocyclic group which may have one or more substituents selected from Group E (where, Group E has the same meaning as defined above).

**16.** The compound according to claim 15, a salt of the compound, or a solvate of the compound or the salt, wherein $X_1$ represents a pyrrolidin-1,2-diyl group which may have one or more substituents selected from the group consisting of a $C_1$-$C_3$ alkyl group, a hydroxy group, and a methoxy group.

**17.** The compound according to claim 12, a salt of the compound, or a solvate of the compound or the salt, wherein $X_2$ represents a $C_1$-$C_6$ alkylamino group which may have one or more substituents selected from Group A (where, Group A has the same meaning as defined above).

**18.** The compound according to claim 17, a salt of the compound, or a solvate of the compound or the salt, wherein $X_2$ represents a dimethylamino group, an ethylmethylamino group, or a diethylamino group (said dimethylamino group, ethylmethylamino group, or diethylamino group may have one or more substituents selected from the group consisting of a hydroxy group, a methoxy group, and a carboxy group).

**19.** The compound according to claim 12, a salt of the compound, or a solvate of the compound or the salt, wherein $X_2$ represents an amino $C_1$-$C_6$ alkylamino group which may have one or more substituents selected from Group B (where, Group B has the same meaning as defined above).

**20.** The compound according to claim 19, a salt of the compound, or a solvate of the compound or the salt, wherein $X_2$ represents a (2-aminoethyl)amino group, a (1-aminopropyl-2-yl)amino group, or a (2-aminopropyl)amino group (said (2-aminoethyl)amino group, (1-aminopropyl-2-yl)amino group, or (2-aminopropyl)amino group may be substituted with one or more methyl groups (however, said methyl group being a substituent on an amino group of each group) or acetyl groups).

**21.** The compound according to claim 12, a salt of the compound, or a solvate of the compound or the salt, wherein $X_2$ represents a 4- to 7-membered saturated or unsaturated nitrogen-containing heterocyclic group which may have one or more substituents selected from Group C (where, Group C has the same meaning as defined above).

**22.** The compound according to claim 21, a salt of the compound, or a solvate of the compound or the salt, wherein $X_2$ represents a piperazinyl group, a pyrrolidinyl group, or a morpholino group (said piperazinyl group, pyrrolidinyl group, or morpholino group may have one or more substituents selected from the group consisting of an oxo group, a methyl group, an ethyl group, a cyclopropyl group, an acetyl group, a hydroxy group, a carboxy group, a carbamoyl group, a dimethylamino group, a hydroxymethyl group, a hydroxyethyl group, an amino group, a fluoro group, and a fluoromethyl group).

**23.** The compound according to claim 12, a salt of the compound, or a solvate of the compound or the salt, wherein $X_2$ represents an 8- to 11-membered bicyclic condensed nitrogen-containing heterocyclic group which may have one or more substituents selected from Group D (where, Group D has the same meaning as defined above).

**24.** The compound according to claim 23, a salt of the compound, or a solvate of the compound or the salt, wherein $X_2$ represents an octahydropyrrolopyrazyl group (said octahydropyrrolopyrazyl group may have one or more methyl groups or oxo groups).

**25.** The compound according to claim 12 , a salt of the compound, or a solvate of the compound or the salt, wherein $X_1$ represents an NH-$C_1$-$C_6$ alkylene group (said NH-$C_1$-$C_6$ alkylene group may be substituted on NH with a $C_1$-$C_6$ alkyl group) or a divalent 4- to 7-membered saturated or unsaturated nitrogen-containing heterocyclic group which may have one or more substituents selected from Group E (where, Group E has the same meaning as defined above), and
$X_2$ represents a $C_1$-$C_6$ alkylamino group which may have one or more substituents selected from Group A, an amino $C_1$-$C_6$ alkylamino group which may have one or more substituents selected from Group B, a 4- to 7-membered saturated or unsaturated nitrogen-containing heterocyclic group which may have one or more substituents selected from Group C, or an 8- to 11-membered bicyclic condensed nitrogen-containing heterocyclic group which may have one or more substituents selected from Group D (where, Groups A, B, C, and D each have the same meanings as defined above).

**26.** The compound according to any one of claims 1 to 25, a salt of the compound, or a solvate of the compound or the salt, wherein
$R^2$ in the formula (1) represents an alkyl group which may have one or more substituents selected from Group G (where, Group G has the same meaning as defined above).

**27.** The compound according to any one of claims 1 to 26, a salt of the compound, or a solvate of the compound or the salt, wherein
$R^2$ in the formula (1) represents a $C_1$-$C_4$ alkyl group.

**28.** The compound according to any one of claims 1 to 27, a salt of the compound, or a solvate of the compound or the salt, wherein
$R^3$ in the formula (1) represents a phenyl group which may have one or more substituents selected from Group I or a 5- to 6-membered aromatic heterocyclic group which may have one or more substituents selected from Group I (where, Group I has the same meaning as defined above).

**29.** The compound according to any one of claims 1 to 28, a salt of the compound, or a solvate of the compound or the salt, wherein
$R^3$ in the formula (1) represents a 4-chlorophenyl group, a 6-chloropyridin-3-yl group, a 3-fluoro-4-chlorophenyl group, a 2-fluoro-4-chlorophenyl group, a 5-bromopyridin-2-yl group, a 4-trifluoromethylphenyl group, or a 4-bromophenyl group.

**30.** The compound according to any one of claims 1 to 29, a salt of the compound, or a solvate of the compound or the salt, wherein
$R^4$ in the formula (1) represents a phenyl group which may have one or more substituents selected from Group I or a 5- to 6-membered aromatic heterocyclic group which may have one or more substituents selected from Group I (where, Group I has the same meaning as defined above).

**31.** The compound according to any one of claims 1 to 30, a salt of the compound, or a solvate of the compound or the salt, wherein
$R^4$ in the formula (1) represents a 4-chlorophenyl group, a 6-chloropyridin-3-yl group, a 4-chloro-3-methylaminophenyl group, a 3-fluoro-4-chlorophenyl group, a 2-fluoro-4-chlorophenyl group, or a 3,4-difluoro-phenyl group.

**32.** The compound according to any one of claims 1 to 31, a salt of the compound, or a solvate of the compound or the salt, wherein,
in the formula (1), both $R^3$ and $R^4$ represent a 4-chlorophenyl group, $R^3$ represents 3-fluoro-4-chlorophenyl group and $R^4$ represents 4-chlorophenyl group, or $R^3$ represents a 3-fluoro-4-chlorophenyl group and $R^4$ represents a 6-chloropyridin-3-yl group.

**33.** The compound according to any one of claims 1 to 32, a salt of the compound, or a solvate of the compound or the salt, wherein
$R^5$ in the formula (1) represents a $C_1$-$C_3$ alkyl group.

**34.** A compound represented by formula (1-A), a salt of the compound, or a solvate of the compound or the salt:

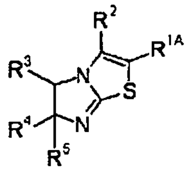
(1-A)

wherein
$R^{1A}$ represents $-V_1-V_2$, wherein
$V_1$ represents a $C_1$-$C_6$ alkylene group,
$V_2$ represents a group selected from the group consisting of a hydrogen atom, a hydroxy group, and a 4-to 7-membered saturated or unsaturated nitrogen-containing heterocyclic group which may have one or more substituents selected from the following Group C,
$R^2$ represents a $C_1$-$C_6$ alkyl group which may have one or more substituents selected from the following Group G,
$R^3$ and $R^4$ each independently represents a phenyl group which may have one or more substituents selected from the following Group I, and
$R^5$ represents a hydrogen atom or a $C_1$-$C_6$ alkyl group:

Group C: a halogen atom, a hydroxy group, a $C_1$-$C_6$ alkyl group which may be substituted with one or more hydroxy groups, a $C_1$-$C_6$ alkoxy group, a $C_1$-$C_6$ alkoxy $C_1$-$C_6$ alkyl group (said $C_1$-$C_6$ alkoxy $C_1$-$C_6$ alkyl group may have one or more substituents selected from the group consisting of a hydroxyphenyl group, a carboxy group, and a 4- to 7-membered saturated or unsaturated heterocyclic group which may have a substituent(s)), a halogeno $C_1$-$C_6$ alkyl group, a carboxy $C_1$-$C_6$ alkyl group, a $C_1$-$C_6$ alkoxycarbonyl $C_1$-$C_6$ alkyl group, a $C_1$-$C_6$ alkylamino $C_1$-$C_6$ alkyl group, a carbamoyl $C_1$-$C_6$ alkyl group, a carboxy group, a formyl group, a $C_2$-$C_6$ alkanoyl group, a $C_1$-$C_6$ alkoxycarbonyl group, an amino group, a $C_1$-$C_6$ alkylamino group, a $C_1$-$C_6$ alkylsulfonyl group, an oxo group, a phenyl group, a 4- to 7-membered saturated or unsaturated heterocyclic group (said 4- to 7-membered saturated or unsaturated heterocyclic group may have one or more substituents selected from a $C_1$-$C_6$ alkyl group, a $C_2$-$C_6$ alkanoyl group, and an oxo group), and a $C_1$-$C_6$ alkylene-4- to 7-membered saturated or unsaturated heterocyclic group (said 4- to 7-membered saturated or unsaturated heterocyclic group may have one or more substituents selected from a $C_1$-$C_6$ alkyl group, a $C_2$-$C_6$ alkanoyl group, and an oxo group);
Group G: a $C_1$-$C_6$ alkoxy group, an oxo group, a 4- to 7-membered saturated or unsaturated heterocyclic group (said 4- to 7-membered saturated or unsaturated heterocyclic group may have one or more $C_1$-$C_6$ alkyl groups and/or oxo groups), a hydroxy group, and a $C_3$-$C_8$ cycloalkyl group; and
Group I: a halogen atom, an amino group, a $C_1$-$C_6$ alkyl group, a $C_1$-$C_6$ alkoxy group, a $C_1$-$C_6$ alkylamino group, a halogeno $C_1$-$C_6$ alkyl group, and a cyano group.

**35.** A compound represented by formula (1-B), a salt of the compound, or a solvate of the compound or the salt:

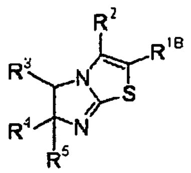
(1-B)

wherein
$R^{1B}$ represents -CO-W, wherein
W represents a group selected from the group consisting of a hydroxy group, a $C_1$-$C_6$ alkoxy group, an amino group which may be substituted with one or two 4-to 7-membered saturated or unsaturated heterocyclic groups (said 4-

to 7-membered saturated or unsaturated heterocyclic group may have one or more $C_1$-$C_6$ alkyl groups and/or oxo groups), a $C_1$-$C_6$ alkylamino group which may have one or more substituents selected from the following Group A, an amino $C_1$-$C_6$ alkylamino group which may have one or more substituents selected from the following Group B, a 4- to 7-membered saturated or unsaturated nitrogen-containing heterocyclic group which may have one or more substituents selected from the following Group C, and an 8- to 11-membered bicyclic condensed nitrogen-containing heterocyclic group which may have one or more substituents selected from the following Group D,
$R^2$ represents a $C_1$-$C_6$ alkyl group which may have one or more substituents selected from the following Group G,
$R^3$ and $R^4$ each independently represents a phenyl group which may have one or more substituents selected from the following Group I or a 5- to 6-membered aromatic heterocyclic group which may have one or more substituents selected from the following Group I, and
$R^5$ represents a hydrogen atom or a $C_1$-$C_6$ alkyl group:

Group A: a $C_1$-$C_6$ alkylamino $C_1$-$C_6$ alkoxy group, a hydroxy $C_1$-$C_6$ alkoxy group, a $C_1$-$C_6$ alkoxy group, a $C_1$-$C_6$ alkoxycarbonyl group, a $C_1$-$C_6$ alkylamino group, a 4- to 7-membered saturated or unsaturated heterocyclic group (said 4- to 7-membered saturated or unsaturated heterocyclic group may have one or more substituents selected from the group consisting of a $C_1$-$C_6$ alkyl group which may be substituted with one or more hydroxy groups, a $C_2$-$C_6$ alkanoyl group, a hydroxy group, a $C_1$-$C_6$ alkoxycarbonyl group, a $C_1$-$C_6$ alkylamino $C_1$-$C_6$ alkyl group, a $C_1$-$C_6$ alkylaminocarbonyl group, a $C_1$-$C_6$ alkylamino $C_1$-$C_6$ alkylcarbonyl group, and an oxo group), a hydroxy group, and carboxy group;
Group B: a $C_1$-$C_6$ alkyl group (said $C_1$-$C_6$ alkyl group being a substituent on an amino group of the amino $C_1$-$C_6$ alkylamino group), a formyl group, and a $C_2$-$C_6$ alkanoyl group;
Group C: a halogen atom, a hydroxy group, a $C_1$-$C_6$ alkyl group which may be substituted with one or more hydroxy groups, a $C_1$-$C_6$ alkoxy group, a $C_1$-$C_6$ alkoxy $C_1$-$C_6$ alkyl group (said $C_1$-$C_6$ alkoxy $C_1$-$C_6$ alkyl group may have one or more substituents selected from the group consisting of a hydroxyphenyl group, a carboxy group, and a 4- to 7-membered saturated or unsaturated heterocyclic group which may have a substituent(s)), a halogeno $C_1$-$C_6$ alkyl group, a carboxy $C_1$-$C_6$ alkyl group, a $C_1$-$C_6$ alkoxycarbonyl $C_1$-$C_6$ alkyl group, a $C_1$-$C_6$ alkylamino $C_1$-$C_6$ alkyl group, a carbamoyl $C_1$-$C_6$ alkyl group, a carboxy group, a formyl group, a $C_2$-$C_6$ alkanoyl group, a $C_1$-$C_6$ alkoxycarbonyl group, an amino group, a $C_1$-$C_6$ alkylamino group, a $C_1$-$C_6$ alkylsulfonyl group, an oxo group, a phenyl group, a 4- to 7-membered saturated or unsaturated heterocyclic group (said 4- to 7-membered saturated or unsaturated heterocyclic group may have one or more substituents selected from a $C_1$-$C_6$ alkyl group, a $C_2$-$C_6$ alkanoyl group, and an oxo group), and a $C_1$-$C_6$ alkylene-4- to 7-membered saturated or unsaturated heterocyclic group (said 4- to 7-membered saturated or unsaturated heterocyclic group may have one or more substituents selected from a $C_1$-$C_6$ alkyl group, a $C_2$-$C_6$ alkanoyl group, and an oxo group);
Group D: a $C_1$-$C_6$ alkyl group and an oxo group;
Group G: a $C_1$-$C_6$ alkoxy group, an oxo group, a 4- to 7-membered saturated or unsaturated heterocyclic group (said 4- to 7-membered saturated or unsaturated heterocyclic group may have one or more $C_1$-$C_6$ alkyl groups and/or oxo groups), a hydroxy group, and a $C_3$-$C_8$ cycloalkyl group; and
Group I: a halogen atom, an amino group, a $C_1$-$C_6$ alkyl group, a $C_1$-$C_6$ alkoxy group, a $C_1$-$C_6$ alkylamino group, a halogeno $C_1$-$C_6$ alkyl group, and a cyano group.

**36.** A compound represented by formula (1-C), a salt of the compound, or a solvate of the compound or the salt:

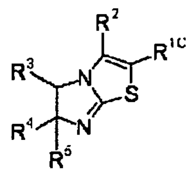
(1-C)

wherein
$R^{1C}$ represents -CO-$X_1$-CO-$X_2$, wherein
$X_1$ represents a group selected from the group consisting of an NH-$C_1$-$C_6$ alkylene group (said NH-$C_1$-$C_6$ alkylene group may be substituted on NH with a $C_1$-$C_6$ alkyl group), a divalent 4- to 7-membered saturated or unsaturated nitrogen-containing heterocyclic group which may have one or more substituents selected from the following Group E, and a divalent 8- to 11-membered bicyclic condensed nitrogen-containing heterocyclic group which may have one or more substituents selected from the following Group F,
$X_2$ represents a group selected from the group consisting of a hydroxy group, a $C_1$-$C_6$ alkoxy group, an amino group

which may be substituted with one or two 4-to 7-membered saturated or unsaturated heterocyclic groups (said 4- to 7-membered saturated or unsaturated heterocyclic group may have one or more $C_1$-$C_6$ alkyl groups and/or oxo groups), a $C_1$-$C_6$ alkylamino group which may have one or more substituents selected from the following Group A, an amino $C_1$-$C_6$ alkylamino group which may have one or more substituents selected from the following Group B, a 4- to 7-membered saturated or unsaturated nitrogen-containing heterocyclic group which may have one or more substituents selected from the following Group C, and an 8- to 11-membered bicyclic condensed nitrogen-containing heterocyclic group which may have one or more substituents selected from the following Group D,
$R^2$ represents a group selected from the group consisting of a $C_1$-$C_6$ alkyl group which may have one or more substituents selected from the following Group G,
$R^3$ represents a group selected from the group consisting of a phenyl group which may have one or more substituents selected from the following Group I and a 5-to 6-membered aromatic heterocyclic group which may have one or more substituents selected from the following Group I,
$R^4$ represents a group selected from the group consisting of a $C_1$-$C_6$ alkyl group which may have one or more substituents selected from the following Group H, a phenyl group which may have one or more substituents selected from the following Group I, and a 5- to 6-membered aromatic heterocyclic group which may have one or more substituents selected from the following Group I,
$R^5$ represents a hydrogen atom or a $C_1$-$C_6$ alkyl group, and
furthermore, $R^4$ and $R^5$ together with the carbon atom on the ring to which $R^4$ and $R^5$ are bonded may form a 3-to 7-membered spiro ring:

Group A: a $C_1$-$C_6$ alkylamino $C_1$-$C_6$ alkoxy group, a hydroxy $C_1$-$C_6$ alkoxy group, a $C_1$-$C_6$ alkoxy group, a $C_1$-$C_6$ alkoxycarbonyl group, a $C_1$-$C_6$ alkylamino group, a 4- to 7-membered saturated or unsaturated heterocyclic group (said 4- to 7-membered saturated or unsaturated heterocyclic group may have one or more substituents selected from the group consisting of a $C_1$-$C_6$ alkyl group which may be substituted with one or more hydroxy groups, a $C_2$-$C_6$ alkanoyl group, a hydroxy group, a $C_1$-$C_6$ alkoxycarbonyl group, a $C_1$-$C_6$ alkylamino $C_1$-$C_6$ alkyl group, a $C_1$-$C_6$ alkylaminocarbonyl group, a $C_1$-$C_6$ alkylamino $C_1$-$C_6$ alkylcarbonyl group, and an oxo group), a hydroxy group, and a carboxy group;
Group B: a $C_1$-$C_6$ alkyl group (said $C_1$-$C_6$ alkyl group being a substituent on an amino group of the amino $C_1$-$C_6$ alkylamino group), a formyl group, and a $C_2$-$C_6$ alkanoyl group;
Group C: a halogen atom, a hydroxy group, a $C_1$-$C_6$ alkyl group which may be substituted with one or more hydroxy groups, a $C_1$-$C_6$ alkoxy group, a $C_1$-$C_6$ alkoxy $C_1$-$C_6$ alkyl group (said $C_1$-$C_6$ alkoxy $C_1$-$C_6$ alkyl group may have one or more substituents selected from the group consisting of a hydroxyphenyl group, a carboxy group, and a 4- to 7-membered saturated or unsaturated heterocyclic group which may have a substituent(s)), a halogeno $C_1$-$C_6$ alkyl group, a carboxy $C_1$-$C_6$ alkyl group, a $C_1$-$C_6$ alkoxycarbonyl $C_1$-$C_6$ alkyl group, a $C_1$-$C_6$ alkylamino $C_1$-$C_6$ alkyl group, a carbamoyl $C_1$-$C_6$ alkyl group, a carboxy group, a formyl group, a $C_2$-$C_6$ alkanoyl group, a $C_1$-$C_6$ alkoxycarbonyl group, an amino group, a $C_1$-$C_6$ alkylamino group, a $C_1$-$C_6$ alkylsulfonyl group, an oxo group, a phenyl group, a 4- to 7-membered saturated or unsaturated heterocyclic group (said 4- to 7-membered saturated or unsaturated heterocyclic group may have one or more substituents selected from a $C_1$-$C_6$ alkyl group, a $C_2$-$C_6$ alkanoyl group, and an oxo group), and a $C_1$-$C_6$ alkylene-4- to 7-membered saturated or unsaturated heterocyclic group (said 4- to 7-membered saturated or unsaturated heterocyclic group may have one or more substituents selected from a $C_1$-$C_6$ alkyl group, a $C_2$-$C_6$ alkanoyl group, and an oxo group);
Group D: a $C_1$-$C_6$ alkyl group and an oxo group;
Group E: a halogen atom, a hydroxy group, a $C_1$-$C_6$ alkyl group, a $C_1$-$C_6$ alkoxy group, an amino group, a $C_1$-$C_6$ alkylamino group, a cyano group, a $C_1$-$C_6$ alkylamino $C_1$-$C_6$ alkyl group, and an oxo group;
Group F: a $C_1$-$C_6$ alkyl group and an oxo group;
Group G: a $C_1$-$C_6$ alkoxy group, an oxo group, a 4- to 7-membered saturated or unsaturated heterocyclic group (said 4- to 7-membered saturated or unsaturated heterocyclic group may have one or more $C_1$-$C_6$ alkyl groups and/or oxo groups), a hydroxy group, and a $C_3$-$C_8$ cycloalkyl group;
Group H: a phenyl group and a $C_3$-$C_8$ cycloalkyl group; and
Group I: a halogen atom, an amino group, a $C_1$-$C_6$ alkyl group, a $C_1$-$C_6$ alkoxy group, a $C_1$-$C_6$ alkylamino group, a halogeno $C_1$-$C_6$ alkyl group, and a cyano group.

**37.** An inhibitor of Mdm2 comprising the compound according to any one of claims 1 to 36, a salt of the compound, or a solvate of the compound or the salt.

**38.** An inhibitor of Mdm2 ubiquitin ligase comprising the compound according to any one of claims 1 to 36, a salt of the compound, or a solvate of the compound or the salt.

**39.** An inhibitor of p53-Mdm2 binding comprising the compound according to any one of claims 1 to 36, a salt of the compound, or a solvate of the compound or the salt.

**40.** An inhibitor of suppression of p53 transcription activity comprising the compound according to any one of claims 1 to 36, a salt of the compound, or a solvate of the compound or the salt.

**41.** An inhibitor of p53 degradation comprising the compound according to any one of claims 1 to 36, a salt of the compound, or a solvate of the compound or the salt.

**42.** A medicament comprising the compound according to any one of claims 1 to 36, a salt of the compound, or a solvate of the compound or the salt as an active ingredient.

**43.** An anti-tumor agent comprising the compound according to any one of claims 1 to 36, a salt of the compound, or a solvate of the compound or the salt as an active ingredient.

**44.** A pharmaceutical composition comprising the compound according to any one of claims 1 to 36, a salt of the compound, or a solvate of the compound or the salt and a pharmaceutically acceptable carrier.

**45.** A method for treating cancer, **characterized by** administering the compound according to any one of claims 1 to 36, a salt of the compound, or a solvate of the compound or the salt.

**46.** Use of the compound according to any one of claims 1 to 36, a salt of the compound, or a solvate of the compound or the salt for the manufacture of a medicament.

**47.** Use of the compound according to any one of claims 1 to 36, a salt of the compound, or a solvate of the compound or the salt for the manufacture of an anti-tumor agent.

## INTERNATIONAL SEARCH REPORT

International application No.
PCT/JP2007/073930

A. CLASSIFICATION OF SUBJECT MATTER
*C07D513/04*(2006.01)i, *A61K31/429*(2006.01)i, *A61K31/4439*(2006.01)i, *A61K31/454*(2006.01)i, *A61K31/496*(2006.01)i, *A61K31/4985*(2006.01)i, *A61K31/5377*(2006.01)i, *A61K31/551*(2006.01)i, *A61P35/00*(2006.01)i,

According to International Patent Classification (IPC) or to both national classification and IPC

B. FIELDS SEARCHED

Minimum documentation searched (classification system followed by classification symbols)
C07D513/04, A61K31/429, A61K31/4439, A61K31/454, A61K31/496, A61K31/4985, A61K31/5377, A61K31/551, A61P35/00, A61P43/00, C07D513/10, C07D519/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
BIOSIS(STN), CAplus(STN), EMBASE(STN), MEDLINE(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | SHARAF, Mohie A. F. et al., Reactions with 4,5-di(p-chlorophenyl)imidazolidine-2-thione, Phosphorus, Sulfur and Silicon and the Related Elements, 1994, vol.92, no.1-4, p.19-27, full text, particularly, compounds 15a, 15c | 1,26-32<br>2-25,33-44, 46,47 |
| X<br>A | HAMMOUDA, Hamdy Ali et al., Reactions with 4,5-diphenylimidazolidine-2-thione. I. Synthesis of some imidazo[2,1-b]thiazole and imidazo [2,1-b][1,3]thiazinone derivatives, Gazzetta Chimica Italiana, 1984, vol.114, no.5-6, p.201-4, full text, particularly, compound 5 | 1,28,30<br>2-27,29, 31-44,46,47 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 February, 2008 (19.02.08) | 26 February, 2008 (26.02.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2007/073930

C (Continuation). DOCUMENTS CONSIDERED TO BE RELEVANT

| Category* | Citation of document, with indication, where appropriate, of the relevant passages | Relevant to claim No. |
|---|---|---|
| A | JP 2006-508906 A (Cyclacel Ltd.), 16 March, 2006 (16.03.06), & WO 2004/005278 A1 & EP 1519932 A1 & US 2005/215548 A1 & CN 1665802 A & NZ 536494 A & EP 1519932 B1 & DE 60316686 E | 1-44,46,47 |
| A | JP 2002-535357 A (The Board of Trustees of the University of Illinois), 22 October, 2002 (22.10.02), & WO 2000/044364 A2 & WO 2000/044364 A3 & CA 2360671 A1 & EP 1143943 A2 & EP 1143943 A3 & EP 1143943 B1 & NZ 513487 A & US 6593353 B1 & EP 1797880 A2 & AT 380548 T & US 2002/006941 A1 & US 7012087 B2 & ZA 2001005823 A & US 2002/019425 A1 & US 2003/144331 A1 & US 6982277 B2 & US 2003/176318 A1 & US 7008956 B2 & US 2005/222224 A1 | 1-44,46,47 |
| A | JP 2002-105085 A (Yamanouchi Pharmaceutical Co., Ltd.), 10 April, 2002 (10.04.02), (Family: none) | 1-44,46,47 |
| A | JP 06-336484 A (Daiichi Pharmaceutical Co., Ltd.), 06 December, 1994 (06.12.94), & EP 618208 A1 & NO 9401135 A & FI 9401487 A & CA 2120395 A1 & AU 9459252 A & AU 672675 B2 & JP 3670309 B2 & CN 1100425 A & US 5599813 A | 1-44,46,47 |
| A | JP 2006-527713 A (F. Hoffmann-LA Roche AG.), 07 December, 2006 (07.12.06), & US 2004/259867 A1 & US 7132421 B2 & AU 2004253245 A1 & CA 2528096 A1 & WO 2005/002575 A1 & EP 1643989 A1 & CN 1805742 A & BR 2004011597 A & KR 748424 B1 | 1-44,46,47 |
| A | VASSILEV, Lyubomir T. et al., In Vivo Activation of the p53 Pathway by Small-Molecule Antagonists of MDM2, Science, 2004, vol.303, no.5659, p.844-848 | 1-44,46,47 |

Form PCT/ISA/210 (continuation of second sheet) (April 2007)

**INTERNATIONAL SEARCH REPORT**

International application No.
PCT/JP2007/073930

Continuation of A. CLASSIFICATION OF SUBJECT MATTER
(International Patent Classification (IPC))

*A61P43/00*(2006.01)i, *C07D513/10*(2006.01)i, *C07D519/00*(2006.01)i

(According to International Patent Classification (IPC) or to both national classification and IPC)

**INTERNATIONAL SEARCH REPORT**

International application No.
PCT/JP2007/073930

**Box No. II Observations where certain claims were found unsearchable (Continuation of item 2 of first sheet)**

This international search report has not been established in respect of certain claims under Article 17(2)(a) for the following reasons:

1. [X] Claims Nos.: 45
because they relate to subject matter not required to be searched by this Authority, namely:
Claim 45 pertains to methods for treatment of the human body by therapy and thus relates to a subject matter which this International Searching Authority is not required, under the provisions of the PCT Rule 39.1(iv), to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying additional fees, this Authority did not invite payment of additional fees.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- WO 199949065 A
- WO 200015657 A
- WO 200624837 A
- WO 200480460 A
- WO 200351359 A
- WO 200351360 A
- WO 20053097 A
- WO 20052575 A
- WO 2005110996 A
- WO 2005123691 A
- WO 200697261 A
- WO 200341715 A
- WO 200395625 A
- US 2004197893 A
- US 2004220179 A
- WO 200496134 A
- WO 200691646 A
- US 2004259867 A
- US 2004259884 A
- WO 200175145 A
- WO 200376608 A

### Non-patent literature cited in the description

- *Science,* 2004, vol. 303, 844-848
- *Proceedings of the National Academy of Sciences of the United States of America,* 2006, vol. 103, 1888-1893
- *Analytical Biochemistry,* 2004, vol. 331, 138-146
- *Bioorganic & Medicinal Chemistry Letters,* 2005, vol. 15, 765-770
- *Journal of Medicinal Chemistry,* 2005, vol. 48, 909-912
- *Chemical Biology & Drug Design,* 2006, vol. 67, 201-205
- *Bioorganic & Medicinal Chemistry Letters,* 2005, vol. 15, 1857-1861
- *Molecular Cancer Therapeutics,* 2006, vol. 5 (1), 160-169
- *Bioorganic & Medicinal Chemistry Letters,* 2006, vol. 16, 3115-3120
- *Bioorganic & Medicinal Chemistry Letters,* 2006, vol. 16, 3310-3314
- *Bioorganic & Medicinal Chemistry Letters,* 2006, vol. 16, 3463-3468
- *Journal of the American Chemical Society,* 2005, vol. 127, 10130-10131
- *Tetrahedron Letters,* 2005, vol. 46, 5949-5951
- *Journal of Medicinal Chemistry,* 2006, vol. 49, 3432-3435
- *Synlett,* 1998, 623
- *Tetrahedron Lett.,* 2005, vol. 46, 4031
- *J. Am. Chem. Soc.,* 2002, vol. 124, 136672
- *J. Am. Chem. Soc.,* 2001, vol. 123, 7707